(19) Europäisches Patentamt
European Patent Office
Office européen des brevets

(11) EP 2 627 631 B1

(12) **EUROPÄISCHE PATENTSCHRIFT**

(45) Veröffentlichungstag und Bekanntmachung des Hinweises auf die Patenterteilung:
**16.12.2015 Patentblatt 2015/51**

(21) Anmeldenummer: **11761005.5**

(22) Anmeldetag: **14.09.2011**

(51) Int Cl.:
***C07D 207/277*** *(2006.01)* ***A61K 31/4015*** *(2006.01)*
***A61K 31/402*** *(2006.01)* ***A61K 31/4025*** *(2006.01)*
***A61P 35/00*** *(2006.01)* ***C07D 401/04*** *(2006.01)*
***C07D 401/06*** *(2006.01)* ***C07D 401/12*** *(2006.01)*
***C07D 403/04*** *(2006.01)* ***C07D 403/12*** *(2006.01)*
***C07D 405/04*** *(2006.01)* ***C07D 405/12*** *(2006.01)*
***C07D 405/14*** *(2006.01)* ***C07D 409/12*** *(2006.01)*
***C07D 413/10*** *(2006.01)* ***C07D 413/12*** *(2006.01)*

(86) Internationale Anmeldenummer:
**PCT/EP2011/004608**

(87) Internationale Veröffentlichungsnummer:
**WO 2012/048775 (19.04.2012 Gazette 2012/16)**

(54) **PYRROLIDINONE ALS METAP-2 INHIBITOREN**

PYRROLIDINONES AS METAP2 INHIBITORS

PYRROLIDINONES EN TANT QU'INHIBITEURS DE METAP-2

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.10.2010 DE 102010048374**

(43) Veröffentlichungstag der Anmeldung:
**21.08.2013 Patentblatt 2013/34**

(73) Patentinhaber: **Merck Patent GmbH**
**64293 Darmstadt (DE)**

(72) Erfinder:
- **HEINRICH, Timo**
**64823 Gross-Umstadt (DE)**
- **ZENKE, Frank**
**64291 Darmstadt (DE)**
- **CALDERINI, Michel**
**64291 Darmstadt (DE)**
- **MUSIL, Djordje**
**64342 Seeheim-Jugenheim (DE)**

(56) Entgegenhaltungen:
**EP-A1- 1 864 971**
**WO-A1-2004/037787**
**WO-A1-2006/127396**
**WO-A2-2010/007114**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 627 631 B1

## Beschreibung

Die Erfindung betrifft Verbindungen der Formel I

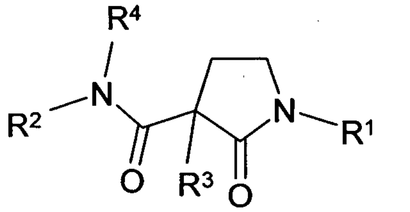

I

worin

$R^1$ unsubstituiertes oder ein-, zwei-, drei-, vier- oder fünffach durch Hal, CN, NHCOA, $NHSO_2A$, $SO_2A$ und/oder $CONH_2$ substituiertes Phenyl, Benzyl, Naphthyl oder Biphenyl; A oder $(CH_2)_nHet$,

$R^2$ $[C(R^4)_2]_nAr^2$, $(CH_2)_nCyc$, $CH[B(OH)_2]CH_2Het$,

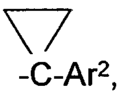

CH(C=CH)phenyl, A oder $(CH_2)_nHet$,

$R^3$ OH, $N_3$, $NH_2$ oder F,

$R^4$ H oder Alkyl mit 1, 2, 3 oder 4 C-Atomen,

$R^2$ und $R^4$ zusammen auch Alkylen mit 2, 3, 4 oder 5 C-Atomen, wobei eine $CH_2$-Gruppe auch durch NH, NA, N-COA, $N-(CH_2)_nAr^3$, $N-(CH_2)_nHet^2$, CH-A, $CH-O-(CH_2)_nAr^3$, $N-SO_2A$ oder O ersetzt sein kann und/oder durch A substituiert sein kann,

Het unsubstituiertes oder ein-, zwei- oder dreifach durch A, OA, COOA, COA, CHO, $(CH_2)_nCONH_2$, $(CH_2)_nCONHA$, $(CH_2)_nCONA_2$, $SO_2A$, $NHSO_2A$, =O und/oder $Het^1$ substituiertes Pyridazinyl, Pyrazolyl, Benzimidazolyl, Pyridyl, Dibenzofuranyl, Carbazolyl, Indolyl, Dihydro-indolyl, Benzofuranyl, Dihydrobenzofuranyl, 2,3-Dihydro-benzo[1,4]dioxin-yl, Chromanyl, Piperazinyl, Morpholinyl, Tetrahydropyranyl, Chinolinyl, Isochinolinyl, Isoindolyl, Dihydrochinolinyl, Dihydroisochinolinyl, Tetrahydrochinolinyl, Tetrahydroisochinolinyl, Chinazolinyl, Chinoxalinyl, Phthalazinyl, Purinyl, Naphthyridinyl, Pyrimidinyl, Indazolyl, Furyl, Thienyl, Imidazolyl, Pyrrolyl, Oxazolyl, Oxadiazolyl, Isoxazolyl, Thiazolyl, Triazolyl, Tetrazolyl, Thiadiazol, Benzothiazolyl, Imidazo[1,2-a]pyridinyl, 1,3-Benzo-dioxolyl, Benzoxazolyl, Piperidin-1-yl, Pyrrolidin-1-yl, [1,2]Oxazinan-2-yl, [1,2,5]Oxadiazinan-2-yl, [1,3]Oxazinan-3-yl oder Hexahydropyrimidinyl,

$Het^1$ unsubstituiertes oder ein-, zwei- oder dreifach durch A und/oder OA substituiertes Pyridazinyl, Pyrazolyl, Pyridyl, Piperazinyl, Morpholinyl, Pyrimidinyl, Furyl, Thienyl, Imidazolyl, Pyrrolyl, Oxazolyl, Isoxazolyl, Thiazolyl, Triazolyl, Tetrazolyl, Thiadiazol, Piperidin-1-yl, Pyrrolidin-1-yl, Tetrahydropyranyl, [1,2]Oxazinan-2-yl, [1,2,5]Oxadiazinan-2-yl, [1,3]Oxazinan-3-yl oder Hexahydropyrimidinyl,

$Het^2$ Pyridyl, Pyrimidinyl, Furyl, Thienyl, Imidazolyl, Pyrrolyl, Oxazolyl, Oxadiazolyl, Isoxazolyl, Thiazolyl, Triazolyl, Tetrazolyl oder Thiadiazol,

A unverzweigtes oder verzweigtes Alkyl mit 1-10 C-Atomen, worin 1-7 H-Atome durch F, Cl, Br, OH, CHO, COA, COOA, CN, $CONA_2$, CONHA und/oder $CONH_2$ ersetzt sein können, und/oder worin eine oder zwei nicht-benachbarte CH- und/oder $CH_2$-Gruppen durch O, N und/oder $NR^4$ ersetzt sein können, oder Cyc,

$Ar^2$ unsubstituiertes oder ein-, zwei-, drei-, vier- oder fünffach durch A, Hal, CN, OH und/oder OA substituiertes Phenyl,

$Ar^3$ unsubstituiertes oder ein-, zwei- oder dreifach durch Hal, OH, OA und/oder A substituiertes Phenyl,

Cyc cyclisches Alkyl mit 3-7 C-Atomen,

Hal F, Cl, Br oder I,

n 0, 1, 2, 3 oder 4,

bedeuten, sowie ihre pharmazeutisch verwendbaren Salze, Tautomere und Stereoisomere, einschließlich deren Mischungen in allen Verhältnissen.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle

pharmakologische Eigenschaften besitzen.

Insbesondere zeigen sie eine regulatorische, modulatorische und/oder inhibierende Wirkung auf Metallproteasen, vorzugsweise auf die Methionin-Amino-Peptidase (MetAP), besonders auf den Subtyp MetAP-2.

Sie können als Arzneimittel gegen Krebs aber auch als Arzneimittel, die den Fett-Stoffwechsel positiv beeinflussen, aber auch als Arzneimittel gegen Entzündungen verwendet werden.

Es wurde gefunden, dass das S-Enantiomer der erfindungsgemäßen Verbindungen deutlich aktiver gegen MetAP-2 ist, als das Spiegelbild (R-Enantiomer).

Andere hydroxy-substituierte Pyrrolidinone kennt man aus:

Zeitschrift für Naturforschung, B: Chemical Sciences (1994), 49(11), 1586-95;
Analytica Chimica Acta (1987), 202, 167-74;
Journal of Electroanalytical Chemistry and Interfacial Electrochemistry (1988), 239(1-2), 161-73;
Zeitschrift fuer Naturfor.Teil B: Anorg. Chem. Org. Chem (1978), 33B(12), 1540-6;
J. Chem. Soc. (1965), (Oct.), 5556-62;
J. Chem. Soc. (1965), (Oct.), 5551-6.

In der WO 01/79157 sind substituierte Hydrazide und N-Alkoxyamide beschrieben, die MetAP-2 inhibitorische Aktivität aufweisen und zur Inhibierung von Angiogenese verwendet werden können, insbesondere zur Behandlung von Krankheiten, wie z.B. Krebs, deren Entwicklung von Angiogenese abhängt.

In der WO 02/081415 sind MetAP-2 Inhibitoren beschrieben, die zur Behandlung von Krebs, Hämangiom, proliferativer Retinopathie, rheumatoider Arthritis, atherosklerotischer Neovaskularisation, Psoriasis, okularer Neovaskularisation und Fettleibigkeit verwendet werden können.

In der WO 2008/011114 sind Verbindungen als Angiogenese-Inhibitoren und MetAP-2-Inhibitoren beschrieben, die zur Behandlung von lymphoider Leukämie und Lymphom verwendet werden können.

Die Wirkung der erfindungsgemäßen Verbindungen gegen Krebs liegt besonders in ihrer Wirkung gegen Angiogenese. Angiogenese-Hemmung hat sich bei über 70 Krankheiten als hilfreich erwiesen, wie z. B. Eierstockkrebs (F. Spinella et al. J. Cardiovasc. Pharmacol. 2004, 44, S140), Brustkrebs (A. Morabito et al. Crit. Rev. Oncol./Hematol. 2004, 49, 91), Prostatakrebs (B. Nicholson et al. Cancer Metastas. Rev. 2001, 20, 297), diabetische Erblindung, Schuppenflechte und Makuladegeneration (E. Ng et al. Can. J. Ophthalmol. 2005, 23, 3706).

Proteasen regulieren viele unterschiedliche Zell-Prozesse, besonders die Modulation von Peptiden und Proteinen, besonders den Protein-Umsatz, die Protein-Reifung und Signalpeptid-Prozessierung, den Abbau von anormalen Proteinen sowie die In-/Aktivierung von regulatorischen Proteinen. Besonders die Amino-terminale Modifikation von naszierenden Polypeptiden stellt die häufigste Modulation dar. Aminoproteasen sind Metalloproteasen, die Aminosäuren vom ungeschützten N-Terminus von Peptiden oder Proteinen abspalten, was sowohl co- als auch posttranslatorisch erfolgen kann. Methionin Aminopeptidase (MetAP) spaltet terminales Methionin naszierender Peptide besonders, wenn die vorletzte Aminosäure klein und ungeladen ist (z. B. Gly, Ala, Ser, Thr, Val, Pro oder Cys).

Bei vielen Krankheitsprozessen steht die Angiogenese entweder ursächlich im Mittelpunkt der Erkrankung oder wirkt sich verschlimmernd auf die Progression der Erkrankung aus. Beispielsweise im Krebsgeschehen führt die Angiogenese dazu, dass der Tumor sich vergrößern und in andere Organe übertreten kann. Weitere Erkrankungen, bei denen Angiogenese eine wichtige Rolle spielt sind Psoriasis, Arthrose, Arteriosklerose sowie Augenerkrankungen wie diabetische Retinopathie, altersbedingte makulare Degeneration, Rubeosis iridis oder neovasculäres Glaukom, ferner bei Entzündungen. Die dieser Erfindung zugrunde liegenden Verbindungen der Formel I, Zusammensetzungen, die diese Verbindungen enthalten, sowie die beschriebenen Verfahren können somit zur Behandlung dieser Krankheiten eingesetzt werden.

Dementsprechend werden die erfindungsgemäßen Verbindungen oder ein pharmazeutisch unbedenkliches Salz davon für die Behandlung von Krebs verabreicht, einschließlich solider Karzinome, wie zum Beispiel Karzinome der Lungen, des Pankreas, der Schilddrüse, der Harnblase oder des Kolons, myeloische Erkrankungen (z. B. myeloische Leukämie) oder Adenome (z. B. villöses Kolonadenom).

Zu den Tumoren zählen weiterhin die Monozytenleukämie, Hirn-, Urogenital-, Lymphsystem-, Magen-, Kehlkopf- und Lungenkarzinom, darunter Lungenadenokarzinom und kleinzelliges Lungenkarzinom, Bauchspeicheldrüsen- und/oder Brustkarzinom.

Gegenstand der vorliegenden Erfindung sind deshalb erfindungsgemäße Verbindungen als Arzneimittel und/oder Arzneimittelwirkstoffe bei der Behandlung und/oder Prophylaxe der genannten Erkrankungen und die Verwendung von erfindungsgemäßen Verbindungen zur Herstellung eines Pharmazeutikums für die Behandlung und/oder Prophylaxe der genannten Erkrankungen. Beschrieben wird auch ein Verfahren zur Behandlung der genannten Erkrankungen umfassend die Verabreichung eines oder mehrerer erfindungsgemäßer Verbindungen an einen Patienten mit Bedarf an einer derartigen Verabreichung.

Es kann gezeigt werden, dass die erfindungsgemäßen Verbindungen anticancerogene Wirkung aufweisen.

Die erfindungsgemäßen Verbindungen werden an einen Patienten mit einer Erkrankung verabreicht, z. B. zur Inhibierung des Tumorwachstums, zur Verminderung der mit einer lymphoproliferativen Erkrankung einhergehenden Entzündung, zur Inhibition der Transplantatabstoßung oder neurologischer Schädigung aufgrund von Gewebereparatur usw. Die vorliegenden Verbindungen sind nützlich für prophylaktische oder therapeutische Zwecke. Wie hierin verwendet, wird der Begriff "Behandeln" als Bezugnahme sowohl auf die Verhinderung von Krankheiten als auch die Behandlung vorbestehender Leiden verwendet. Die Verhinderung von Proliferation/ Vitalität wird durch Verabreichung der erfindungsgemäßen Verbindungen vor Entwicklung der evidenten Krankheit erreicht, z. B. zur Verhinderung des Tumorwachstums. Als Alternative werden die Verbindungen zur Behandlung andauernder Krankheiten durch Stabilisation oder Verbesserung der klinischen Symptome des Patienten verwendet.

Der Wirt oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Die Suszeptibilität einer bestimmten Zelle gegenüber der Behandlung mit den erfindungsgemäßen Verbindungen kann durch Testen in vitro bestimmt werden. Typischerweise wird eine Kultur der Zelle mit einer erfindungsgemäßen Verbindung bei verschiedenen Konzentrationen für eine Zeitdauer inkubiert, die ausreicht, um den aktiven Mitteln zu ermöglichen, Zelltod zu induzieren oder Zellproliferation, Zellvitalität oder Migration zu inhibieren, gewöhnlich zwischen ungefähr einer Stunde und einer Woche. Zum Testen in vitro können kultivierte Zellen aus einer Biopsieprobe verwendet werden. Die Menge nach der Behandlung zurückbleibenden Zellen werden dann bestimmt. Die Dosis variiert abhängig von der verwendeten spezifischen Verbindung, der spezifischen Erkrankung, dem Patientenstatus usw.. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die Behandlung wird im Allgemeinen fortgesetzt, bis eine erhebliche Reduktion vorliegt, z. B. mindestens ca. 50 % Verminderung der Zelllast und kann fortgesetzt werden, bis im Wesentlichen keine unerwünschten Zellen mehr im Körper nachgewiesen werden.

Es wurde gefunden, dass die erfindungsgemäßen Verbindungen eine spezifische Inhibierung der MetAP-2 bewirken. Die erfindungsgemäßen Verbindungen zeigen bevorzugt eine vorteilhafte biologische Aktivität, die in den, zum Beispiel hierin beschriebenen Tests nachweisbar ist. In derartigen Tests zeigen und bewirken die erfindungsgemäßen Verbindungen einen inhibierenden Effekt, der gewöhnlich durch $IC_{50}$-Werte in einem geeigneten Bereich, bevorzugt im mikromolaren Bereich und bevorzugter im nanomolaren Bereich dokumentiert wird.

Zudem können die erfindungsgemäßen Verbindungen verwendet werden, um bei gewissen existierenden Krebs-Chemotherapien und -bestrahlungen additive oder synergistische Effekte zu erzielen und/oder, um die Wirksamkeit gewisser existierender Krebs-Chemotherapien und -bestrahlungen wiederherzustellen.

Die erfindungsgemäßen Verbindungen können auch zur Behandlung von Fettleibigkeit (Obesitas) verwendet werden. Henri R. Lijnen et al. beschreibt in Obesity, Vor. 18 no.12, 2241-2246 (2010) die Verwendung von Fumagillin, einem Met-AP2-Inhibitor, bei der Reduzierung von adipösem Gewebe.
Die Verwendung von Met-AP2-Inhibitoren (Verbindungen vom Fumagillin-Typ) zur Behandlung von Obesitas ist auch in der WO 2011/085201 A1 beschrieben.

Die erfindungsgemäßen Verbindungen können auch zur Behandlung von Malaria verwendet werden. X. Chem et al. beschreibt in Chemistry & Biology, Vol.16, 193-202 (2009) die Verwendung von Fumagillin, einem Met-AP2-Inhibitor, zur Behandlung von Malaria.

Die erfindungsgemäßen Verbindungen können auch zur Behandlung von gutartiger Prostatahypertrophie verwendet werden.
Die Verwendung von Met-AP2-Inhibitoren (Verbindungen vom Fumagillin-Typ) zur Behandlung von gutartiger Prostatahypertrophie ist in der WO 2011/085198 A1 beschrieben.

Unter Verbindungen der Formel I versteht man auch die Hydrate und Solvate dieser Verbindungen.
Gegenstand der Erfindung sind auch die optisch aktiven Formen (Stereoisomeren), Salze, die Enantiomeren, die Racemate, die Diastereomeren sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.
Unter pharmazeutisch verwendbaren Derivaten versteht man z.B. die Salze der erfindungsgemäßen Verbindungen als auch sogenannte Prodrug-Verbindungen.

Unter Prodrug-Derivaten versteht man mit z. B. Alkyl- oder Acylgruppen, Zuckern oder Oligopeptiden abgewandelte Verbindungen der Formel I, die im Organismus rasch zu den wirksamen erfindungsgemäßen Verbindungen gespalten werden.
Hierzu gehören auch bioabbaubare Polymerderivate der erfindungsgemäßen Verbindungen, wie dies z. B. in Int. J. Pharm. 115, 61-67 (1995) beschrieben ist.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die

z.B. von einem Forscher oder Mediziner gesucht oder erstrebt wird.
Darüberhinaus bedeutet der Ausdruck "therapeutisch wirksame Menge" eine Menge, die, verglichen zu einem entsprechenden Subjekt, das diese Menge nicht erhalten hat, folgendes zur Folge hat:

verbesserte Heilbehandlung, Heilung, Prävention oder Beseitigung einer Krankheit, eines Krankheitsbildes, eines Krankheitszustandes, eines Leidens,
einer Störung oder von Nebenwirkungen oder auch die Verminderung des Fortschreitens einer Krankheit, eines Leidens oder einer Störung.

Die Bezeichnung "therapeutisch wirksame Menge" umfaßt auch die Mengen, die wirkungsvoll sind, die normale physiologische Funktion zu erhöhen.

Gegenstand der Erfindung ist auch die Verwendung von Mischungen der Verbindungen der Formel I, z.B. Gemische zweier Diastereomerer z.B. im Verhältnis 1:1, 1:2, 1:3, 1:4, 1:5, 1:10, 1:100 oder 1:1000.
Besonders bevorzugt handelt es sich dabei um Mischungen stereoisomerer Verbindungen.

Gegenstand der Erfindung sind die Verbindungen der Formel I und ihre Salze sowie ein Verfahren zur Herstellung von Verbindungen der Formel I sowie ihrer pharmazeutisch verwendbaren Salze, Tautomeren und Stereoisomeren, dadurch gekennzeichnet, daß man

a) eine Verbindung der Formel II

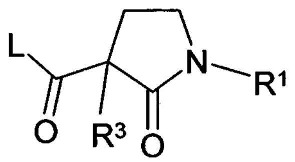

II

worin $R^1$ und $R^3$ die in Anspruch 1 angegebenen Bedeutungen haben, und L Cl, Br, I oder eine freie oder reaktionsfähig funktionell abgewandelte OH-Gruppe bedeutet,
mit einer Verbindung der Formel III

$R^2$-$NHR^4$ III

worin $R^2$ und $R^4$ die in Anspruch 1 angegebenen Bedeutungen haben,
umsetzt,
oder
b) zur Herstellung von Verbindungen der Formel I, worin $R^3$ OH bedeutet,
eine Verbindung der Formel IV

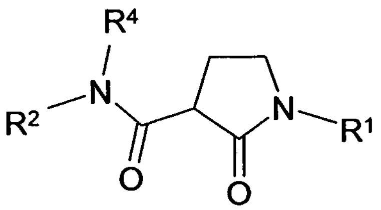

IV

worin $R^1$, $R^2$ und $R^4$ die in Anspruch 1 angegebenen Bedeutungen haben,
oxidiert,
oder
c) einen Rest $R^3$ in einen anderen Rest $R^3$ umwandelt, indem man eine OH-Gruppe gegen ein Halogenatom austauscht,
oder ein Halogenatom gegen $N_3$ austauscht,

und/oder eine Base oder Säure der Formel I in eines ihrer Salze umwandelt.

Vor- und nachstehend haben die Reste $R^1$, $R^2$ und $R^3$ die bei der Formel I angegebenen Bedeutungen, sofern nicht ausdrücklich etwas anderes angegeben ist.

A bedeutet Alkyl, ist unverzweigt (linear) oder verzweigt, und hat 1, 2, 3, 4, 5, 6, 7, 8, 9 oder 10 C-Atome. A

bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2-, 3- oder 4-Methylpentyl, 1,1- , 1,2- , 1,3- , 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.

Vorzugsweise bedeutet A unverzweigtes oder verzweigtes Alkyl mit 1-6 C-Atomen, worin 1-7 H-Atome durch F, Cl, Br und/oder OH ersetzt sein können, oder Cyc.

Weiterhin bedeutet A vorzugsweise unverzweigtes oder verzweigtes Alkyl mit 1-10 C-Atomen, worin 1-7 H-Atome durch F, Cl, Br, OH, CHO, COA, COOA, CN, $CONA_2$, CONHA und/oder $CONH_2$ ersetzt sein können,

und/oder worin eine oder zwei nicht-benachbarte CH- und/oder $CH_2$-Gruppen durch O, N und/oder $NR^4$ ersetzt sein können,

oder Cyc.

A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl.

Cyclisches Alkyl bedeutet vorzugsweise Cyclopropyl, Cyclobutyl, Cylopentyl, Cyclohexyl oder Cycloheptyl.

$R^1$ bedeutet vorzugsweise unsubstituiertes oder ein-, zwei-, drei-, vier- oder fünffach durch Hal, CN, NHCOA, $NHSO_2A$, $SO_2A$ und/oder $CONH_2$ substituiertes Phenyl, Benzyl, Naphthyl oder Biphenyl;

A oder $(CH_2)_n$Het.

$R^2$ bedeutet vorzugsweise $[C(R^4)_2]_nAr^2$, $(CH_2)_n$Cyc,

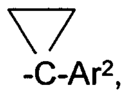

CH(C≡CH)phenyl, A oder $(CH_2)_n$Het.

$R^3$ bedeutet vorzugsweise OH, $N_3$, $NH_2$ oder F.

$R^4$ bedeutet vorzugsweise H, Methyl, Ethyl oder Propyl.

$Ar^2$ bedeutet z.B. Phenyl, o-, m- oder p-Tolyl, o-, m- oder p-Ethylphenyl, o-, m-oder p-Propylphenyl, o-, m- oder p-Isopropylphenyl, o-, m- oder p-tert.-Butylphenyl, o-, m- oder p-Trifluormethylphenyl, o-, m- oder p-Fluorphenyl, o-, m- oder p-Bromphenyl, o-, m- oder p-Chlorphenyl, o-, m- oder p-Hydroxyphenyl, o-, m- oder p-Methoxyphenyl, o-, m- oder p-Methylsulfonylphenyl, o-, m- oder p-Nitrophenyl, o-, m- oder p-Aminophenyl, o-, m- oder p-Methylaminophenyl, o-, m- oder p-Dimethylaminophenyl, o-, m- oder p-Aminosulfonylphenyl, o-, m- oder p-Aminocarbonylphenyl, o-, m- oder p-Carboxyphenyl, o-, m- oder p-Methoxycarbonylphenyl, o-, m- oder p-Ethoxycarbonylphenyl, o-, m-oder p-Acetylphenyl, o-, m- oder p-Formylphenyl, o-, m- oder p-Cyanphenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,3,4-, 2,3,5-, 2,3,6-, 2,4,6- oder 3,4,5-Trichlorphenyl, p-Iodphenyl, 4-Fluor-3-chlorphenyl, 2-Fluor-4-bromphenyl, 2,5-Difluor-4.-brom-phenyl oder 2,5-Dimethyl-4-chlorphenyl; ferner Naphthyl oder Biphenyl.

$Ar^2$ bedeutet weiterhin vorzugsweise unsubstituiertes oder ein-, zwei-, drei-, vier- oder fünffach durch A, Hal, CN, OH und/oder OA substituiertes Phenyl.

Het bedeutet, ungeachtetet weiterer Substitutionen, z.B. 2- oder 3-Furyl, 2-oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4-oder-5-yl, 1,2,4-Triazol-1-, -3- oder 5-yl, 1- oder 5-Tetrazolyl, 1,2,3-Oxadiazol-4- oder -5-yl, 1,2,4-Oxadiazol-3-oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, 1-, 2-, 4- oder 5-Benzimidazolyl, 1-, 2-, 3-, 4-, 5-, 6- oder 7-Indazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]-oxazinyl, weiter bevorzugt 1,3-Benzodioxol-5-yl, 1,4-Benzodioxan-6-yl, 2,1,3-Benzothiadiazol-4- oder -5-yl oder 2,1,3-Benzoxadiazol-5-yl.

Die heterocyclischen Reste können auch teilweise oder vollständig hydriert sein.

Unsubstituiertes Het kann also z. B. auch bedeuten 2,3-Dihydro-2-, -3-, -4-oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4-oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4-oder -5-pyrazolyl, Tetrahydro-1-, -3- oder -4-pyrazolyl, 1,4-Dihydro-1-, -2-, -3-oder -4-pyridyl, 1,2,3,4-Tetrahydro-1-, -2-, -3-, -4-, -5- oder -6-pyridyl, 1-, 2-, 3-oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl,

1,2,3,4-Tetrahydro-1-, -2-, -3-, -4-, -5-, -6-, -7- oder -8-isochinolyl, 2-, 3-, 5-, 6-, 7- oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydro-benzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydro-benzofuranyl oder 2,3-Dihydro-2-oxo-furanyl.

Het bedeutet weiterhin vorzugsweise unsubstituiertes oder ein-, zwei- oder dreifach durch A, OA, COOA, COA, CHO, $(CH_2)_nCONH_2$, $SO_2A$, $NHSO_2A$, =O und/oder $Het^1$ substituiertes Pyridazinyl, Pyrazolyl, Benzimidazolyl, Pyridyl, Dibenzofuranyl, Carbazolyl, Indolyl, Dihydro-indolyl, Benzofuranyl, Dihydro-benzofuranyl, 2,3-Dihydro-benzo[1,4]dioxinyl, Chromanyl, Piperazinyl, Morpholinyl, Tetrahydropyranyl, Chinolinyl, Isochinolinyl, Isoindolyl, Dihydrochinolinyl, Dihydroisochinolinyl, Tetrahydrochinolinyl, Tetrahydroisochinolinyl, Chinazolinyl, Chinoxalinyl, Phthalazinyl, Purinyl, Naphthyridinyl, Pyrimidinyl, Indazolyl, Furyl, Thienyl, Imidazolyl, Pyrrolyl, Oxazolyl, Oxadiazolyl, Isoxazolyl, Thiazolyl, Triazolyl, Tetrazolyl, Thiadiazol, Benzothiazolyl, Imidazo[1,2-a]pyridinyl, 1,3-Benzodioxolyl, Benzoxazolyl, Piperidin-1-yl, Pyrrolidin-1-yl, [1,2]Oxazinan-2-yl, [1,2,5]Oxadiazinan-2-yl, [1,3]Oxazinan-3-yl oder Hexahydropyrimidinyl.

Het bedeutet besonders bevorzugt unsubstituiertes oder ein- oder zweifach durch A, COOA, COA, CHO, $(CH_2)_nCONH_2$, $(CH_2)_nCONHA$, $(CH_2)_nCONA_2$, $SO_2A$, $NHSO_2A$, =O und/oder Pyrrolidinyl substituiertes Dihydro-indolyl, Benzofuranyl, Dihydro-benzofuranyl, 2,3-Dihydro-benzo[1,4]dioxin-yl, Chromanyl, Oxazolyl, Oxadiazolyl, Tetrazolyl, Oxadiazolyl, Thiazolyl, Thiadiazolyl, Thienyl, Furanyl, Tetrahydropyranyl, Pyrazolyl, Pyridyl, Benzothiazolyl, 2,3-Dihydrobenzo[1,4]dioxin-yl, Chinolyl, Isochinolyl oder Pyrrolidinyl.

$Het^1$ bedeutet, ungeachtetet weiterer Substitutionen, z.B. 2- oder 3-Furyl, 2-oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder 5-yl, 1- oder 5-Tetrazolyl, 1,2,3-Oxadiazol-4- oder -5-yl, 1,2,4-Oxadiazol-3-oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl oder Pyrazinyl.

Die heterocyclischen Reste können auch teilweise oder vollständig hydriert sein.

Unsubstituiertes $Het^1$ kann also z. B. auch bedeuten 2,3-Dihydro-2-, -3-, -4-oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4-oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4-oder -5-pyrazolyl, Tetrahydro-1-, -3- oder -4-pyrazolyl, 1,4-Dihydro-1-, -2-, -3-oder -4-pyridyl, 1,2,3,4-Tetrahydro-1-, -2-, -3-, -4-, -5- oder -6-pyridyl, 1-, 2-, 3-oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl oder 1-, 2- oder 3-Piperazinyl.

$Het^1$ bedeutet weiterhin vorzugsweise unsubstituiertes oder ein-, zwei- oder dreifach durch A und/oder OA substituiertes Pyridazinyl, Pyrazolyl, Pyridyl, Piperazinyl, Morpholinyl, Pyrimidinyl, Furyl, Thienyl, Imidazolyl, Pyrrolyl, Oxazolyl, Isoxazolyl, Thiazolyl, Triazolyl, Tetrazolyl, Thiadiazol, Piperidin-1-yl, Pyrrolidin-1-yl, Tetrahydropyranyl, [1,2]Oxazinan-2-yl, [1,2,5]Oxadiazinan-2-yl, [1,3]Oxazinan-3-yl oder Hexahydropyrimidinyl.

$Het^1$ bedeutet besonders bevorzugt unsubstituiertes oder einfach durch A substituiertes Imidazolyl, Thiazolyl oder Pyrrolidinyl.

Hal bedeutet vorzugsweise F, Cl oder Br, aber auch I, besonders bevorzugt F oder Cl.

Für die gesamte Erfindung gilt, daß sämtliche Reste, die mehrfach auftreten, gleich oder verschieden sein können, d.h. unabhängig voneinander sind.

Die Verbindungen der Formel I können ein oder mehrere chirale Zentren besitzen und daher in verschiedenen stereoisomeren Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis If ausgedrückt werden, die der Formel I entsprechen und worin die nicht näher bezeichneten Reste die bei der Formel I angegebene Bedeutung haben, worin jedoch

in Ia

$R^1$ unsubstituiertes oder ein-, zwei-, drei-, vier- oder fünffach durch Hal, CN, NHCOA, $NHSO_2A$, $SO_2A$ und/oder $CONH_2$ substituiertes Phenyl, Benzyl, Naphthyl oder Biphenyl; A oder $(CH_2)_nHet$

bedeutet;

in Ib

$R^2$ $[C(R^4)_2]_nAr^2$, $(CH_2)_nCyc$, $CH[B(OH)_2]CH_2Het$,

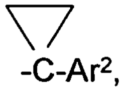

CH(C≡CH)phenyl, A oder $(CH_2)_n$Het

bedeutet;
in Ic

$R^3$ OH, $N_3$, $NH_2$ oder F

bedeutet;
in Id

Het unsubstituiertes oder ein-, zwei- oder dreifach durch A, OA, COOA, COA, CHO, $(CH_2)_nCONH_2$, $(CH_2)_n$CONHA, $(CH_2)_nCONA_2$, $SO_2A$, $NHSO_2A$, =O und/oder $Het^1$ substituiertes Pyridazinyl, Pyrazolyl, Benzimidazolyl, Pyridyl, Dibenzofuranyl, Carbazolyl, Indolyl, Dihydro-indolyl, Benzofuranyl, Dihydro-benzofuranyl, 2,3-Dihydro-benzo[1,4]dioxin-yl, Chromanyl, Piperazinyl, Morpholinyl, Tetrahydropyranyl, Chinolinyl, Isochinolinyl, Isoindolyl, Dihydrochinolinyl, Dihydroisochinolinyl, Tetrahydrochinolinyl, Tetrahydroisochinolinyl, Chinazolinyl, Chinoxalinyl, Phthalazinyl, Purinyl, Naphthyridinyl, Pyrimidinyl, Indazolyl, Furyl, Thienyl, Imidazolyl, Pyrrolyl, Oxazolyl, Oxadiazolyl, Isoxazolyl, Thiazolyl, Triazolyl, Tetrazolyl, Thiadiazol, Benzothiazolyl, Imidazo[1,2-a]pyridinyl, 1,3-Benzodioxolyl, Benzoxazolyl, Piperidin-1-yl, Pyrrolidin-1-yl, [1,2]Oxazinan-2-yl, [1,2,5]Oxadiazinan-2-yl, [1,3]Oxazinan-3-yl oder Hexahydropyrimidinyl

bedeutet;
in Ie

$Het^1$ unsubstituiertes oder ein-, zwei- oder dreifach durch A und/oder OA substituiertes Pyridazinyl, Pyrazolyl, Pyridyl, Piperazinyl, Morpholinyl, Pyrimidinyl, Furyl, Thienyl, Imidazolyl, Pyrrolyl, Oxazolyl, Isoxazolyl, Thiazolyl, Triazolyl, Tetrazolyl, Thiadiazol, Piperidin-1-yl, Pyrrolidin-1-yl, Tetrahydropyranyl, [1,2]Oxazinan-2-yl, [1,2,5]Oxadiazinan-2-yl, [1,3]Oxazinan-3-yl oder Hexahydropyrimidinyl

bedeutet;
in If

$R^1$ unsubstituiertes oder ein-, zwei-, drei-, vier- oder fünffach durch Hal, CN, NHCOA, $NHSO_2A$, $SO_2A$ und/oder $CONH_2$ substituiertes Phenyl, Benzyl, Naphthyl oder Biphenyl; A oder $(CH_2)_n$Het,

$R^2$ $[C(R^4)_2]_nAr^2$, $CH[B(OH)_2]CH_2$Het,
$(CH_2)_n$Cyc,

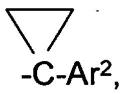

CH(C≡CH)phenyl, A oder $(CH_2)_n$Het,

$R^3$ OH, $N_3$, $NH_2$ oder F,

$R^4$ H oder Alkyl mit 1, 2, 3 oder 4 C-Atomen,

$R^2$ und $R^4$ zusammen auch Alkylen mit 2, 3, 4 oder 5 C-Atomen, wobei eine $CH_2$-Gruppe auch durch NH, NA, N-COA, $N-(CH_2)_nAr^3$, $N-(CH_2)_nHet^2$, CH-A, $CH-O-(CH_2)_nAr^3$, $N-SO_2A$ oder O ersetzt sein kann und/oder durch A substituiert sein kann,

Het unsubstituiertes oder ein-, zwei- oder dreifach durch A, OA, COOA, COA, CHO, $(CH_2)_nCONH_2$, $(CH_2)_n$CONHA, $(CH_2)_nCONA_2$, $SO_2A$, $NHSO_2A$, =O und/oder $Het^1$ substituiertes Pyridazinyl, Pyrazolyl, Benzimidazolyl, Pyridyl, Dibenzofuranyl, Carbazolyl, Indolyl, Dihydro-indolyl, Benzofuranyl, Dihydrobenzofuranyl, 2,3-Dihydrobenzo[1,4]dioxin-yl, Chromanyl, Piperazinyl, Morpholinyl, Tetrahydropyranyl, Chinolinyl, Isochinolinyl, Isoindolyl, Dihydrochinolinyl, Dihydroisochinolinyl, Tetrahydrochinolinyl, Tetrahydroisochinolinyl, Chinazolinyl, Chinoxalinyl, Phthalazinyl, Purinyl, Naphthyridinyl, Pyrimidinyl, Indazolyl, Furyl, Thienyl, Imidazolyl, Pyrrolyl, Oxazolyl, Oxadiazolyl, Isoxazolyl, Thiazolyl, Triazolyl, Tetrazolyl, Thiadiazol, Benzothiazolyl, Imidazo[1,2-a]pyridinyl, 1,3-Benzodioxolyl, Benzoxazolyl, Piperidin-1-yl, Pyrrolidin-1-yl, [1,2]Oxazinan-2-yl, [1,2,5]Oxadiazinan-2-yl, [1,3]Oxazinan-3-yl oder Hexahydropyrimidinyl,

| | |
|---|---|
| $Het^1$ | unsubstituiertes oder ein-, zwei- oder dreifach durch A und/oder OA substituiertes Pyridazinyl, Pyrazolyl, Pyridyl, Piperazinyl, Morpholinyl, Pyrimidinyl, Furyl, Thienyl, Imidazolyl, Pyrrolyl, Oxazolyl, Isoxazolyl, Thiazolyl, Triazolyl, Tetrazolyl, Thiadiazol, Piperidin-1-yl, Pyrrolidin-1-yl, Tetrahydropyranyl, [1,2]Oxazinan-2-yl, [1,2,5]Oxadiazinan-2-yl, [1,3]Oxazinan-3-yl oder Hexahydropyrimidinyl, |
| $Het^2$ | Pyridyl, Pyrimidinyl, Furyl, Thienyl, Imidazolyl, Pyrrolyl, Oxazolyl, Oxadiazolyl, Isoxazolyl, Thiazolyl, Triazolyl, Tetrazolyl oder Thiadiazol, |
| A | unverzweigtes oder verzweigtes Alkyl mit 1-10 C-Atomen, worin 1-7 H-Atome durch F, Cl, Br, OH, CHO, COA, COOA, CN, $CONA_2$, CONHA und/oder $CONH_2$ ersetzt sein können, und/oder worin eine oder zwei nicht-benachbarte CH- und/oder $CH_2$-Gruppen durch O, N und/oder $NR^4$ ersetzt sein können, oder Cyc, |
| $Ar^2$ | unsubstituiertes oder ein-, zwei-, drei-, vier- oder fünffach durch A, Hal, CN, OH und/oder OA substituiertes Phenyl, |
| $Ar^3$ | unsubstituiertes oder ein-, zwei- oder dreifach durch Hal, OH, OA und/oder A substituiertes Phenyl, |
| Cyc | cyclisches Alkyl mit 3-7 C-Atomen, |
| Hal | F, Cl, Br oder I, |
| n | 0, 1, 2, 3 oder 4, |

bedeuten; sowie ihre pharmazeutisch verwendbaren Salze, Tautomere und Stereoisomere, einschließlich deren Mischungen in allen Verhältnissen.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Verbindungen der Formel I können vorzugsweise erhalten werden, indem man Verbindungen der Formel II mit einer Verbindung der Formel III umsetzt.
Die Verbindungen der Formel II und der Formel III sind in der Regel bekannt. Sind sie neu, so können sie aber nach an sich bekannten Methoden hergestellt werden.

In den Verbindungen der Formel II bedeutet L vorzugsweise Cl, Br, I oder eine freie oder eine reaktionsfähig abgewandelte OH-Gruppe wie z.B. ein aktivierter Ester, ein Imidazolid oder Alkylsulfonyloxy mit 1-6 C-Atomen (bevorzugt Methylsulfonyloxy oder Trifluormethylsulfonyloxy) oder Arylsulfonyloxy mit 6-10 C-Atomen (bevorzugt Phenyl- oder p-Tolylsulfonyloxy).
Die Reaktion gelingt vorzugsweise in Gegenwart eines Dehydratisierungsmittels, wie z.B. eines Carbodiimids wie N,N'-Dicyclohexylcarbodiimid ("DCCI"), 1,1'-Carbonyl-diimidazol oder N-3-Dimethylaminopropyl-N'-ethyl-carbodiimid ("DAPECI"), ferner Propanphosphonsäureanhydrid T3P (vgl. Angew. Chem. 92, 129 (1980)), Diphenylphosphorylazid oder 2-Ethoxy-N-ethoxycarbonyl-1,2-dihydrochinolin, gegebenenfalls in Gegenwart von N-Hydroxybenzotriazol;

T3P = 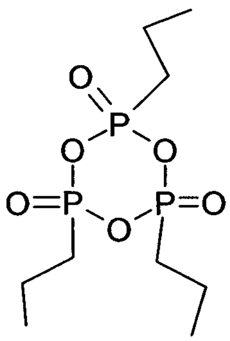 .

Die Umsetzung erfolgt in einem inerten Lösungsmittel und erfolgt in der Regel in Gegenwart eines säurebindenden Mittels vorzugsweise einer organischen Base wie DIPEA, Triethylamin, Dimethylanilin, Pyridin oder Chinolin. Auch der Zusatz eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums kann günstig sein.

Die Reaktionszeit liegt je nach den angewendeten Bedingungen zwischen einigen Minuten und 14 Tagen, die Reaktionstemperatur zwischen etwa -15° und 150°, normalerweise zwischen 40° und 130°, besonders bevorzugt zwischen 60° und 110°C.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylen-glykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.
Besonders bevorzugt sind Glykolether, wie Ethylenglycolmonomethylether, THF, Dichlormethan und/oder DMF.

Verbindungen der Formel I können weiterhin vorzugsweise erhalten werden, indem man Verbindungen der Formel IV oxidiert.
Die Oxidation erfolgt vorzugsweise mit tert.-Butylhydroperoxid.
Die Reaktionszeit liegt je nach den angewendeten Bedingungen zwischen einigen Minuten und 14 Tagen, die Reaktionstemperatur zwischen etwa -15° und 150°, normalerweise zwischen 40° und 130°, besonders bevorzugt zwischen 60° und 110°C.
Als Lösungsmittel ist Wasser bevorzugt, wobei der Zusatz eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums, bevorzugt ist.

<u>Pharmazeutische Salze und andere Formen</u>

Die genannten erfindungsgemäßen Verbindungen lassen sich in ihrer endgültigen Nichtsalzform verwenden. Andererseits umfaßt die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der Verbindungen der Formel I werden größtenteils konventionell hergestellt. Sofern die Verbindung der Formel I eine Carbonsäuregruppe enthält, läßt sich eines ihrer geeigneten Salze dadurch bilden, daß man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und N-Methylglutamin. Die Aluminiumsalze der Verbindungen der Formel I zählen ebenfalls dazu. Bei bestimmten Verbindungen der Formel I lassen sich Säureadditionssalze dadurch bilden, daß man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen der Formel I die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, Iodid, Isethionat, Isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der erfindungsgemäßen Verbindungen Aluminium-, Ammonium-, Calcium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium,sowie die Erdalkalimetalsalze Calcium und Magnesium. Zu Salzen der Verbindungen der Formel I, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer Ionenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der vorliegenden Erfindung, die basische stickstoffhaltige Gruppen enthalten, lassen sich mit Mitteln wie ($C_1$-$C_4$) Alkylhalogeniden, z.B. Methyl-, Ethyl-, Isopropyl- und tert.-Butylchlorid, -bromid und -iodid; Di($C_1$-$C_4$)Alkylsulfaten, z.B. Dimethyl-, Diethyl- und Diamylsulfat; ($C_{10}$-$C_{18}$)Alkylhalogeniden, z.B. Decyl-, Dodecyl-, Lauryl-, Myristyl- und Stearylchlorid, -bromid und -iodid; sowie Aryl-($C_1$-$C_4$)Alkylhalogeniden, z.B. Benzylchlorid und Phenethylbromid, quarternisieren. Mit solchen Salzen können sowohl wasserals auch öllösliche erfindungsgemäße Verbindungen hergestellt werden.

Zu den oben genannten pharmazeutischen Salzen, die bevorzugt sind, zählen Acetat, Trifluoracetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Die Säureadditionssalze basischer Verbindungen der Formel I werden dadurch hergestellt, daß man die freie Basenform mit einer ausreichenden Menge der gewünschten Säure in Kontakt bringt, wodurch man auf übliche Weise das Salz darstellt. Die freie Base läßt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.

Wie erwähnt werden die pharmazeutisch unbedenklichen Basenadditionssalze der Verbindungen der Formel I mit Metallen oder Aminen wie Alkalimetallen und Erdalkalimetallen oder organischen Aminen gebildet. Bevorzugte Metalle sind Natrium, Kalium, Magnesium und Calcium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von erfindungsgemäßen sauren Verbindungen werden dadurch hergestellt, daß man die freie Säureform mit einer ausreichenden Menge der gewünschten Base in Kontakt bringt, wodurch man das Salz auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine erfindungsgemäße Verbindung mehr als eine Gruppe, die solche pharmazeutisch unbedenklichen Salze bilden kann, so umfaßt die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, daß unter dem Ausdruck "pharmazeutisch unbedenkliches Salz" im vorliegenden Zusammenhang ein Wirkstoff zu verstehen ist, der eine Verbindung der Formel I in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Gegenstand der Erfindung sind ferner Arzneimittel, enthaltend mindestens eine Verbindung der Formel I und/oder ihre pharmazeutisch verwendbaren Derivate, Solvate und Stereoisomere, einschließlich deren Mischungen in allen Verhältnissen, sowie gegebenenfalls Träger- und/oder Hilfsstoffe.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepaßte pharmazeutische Formulierungen können als separate Einheiten, wie z.B. Kapseln oder Tabletten; Pulver oder Granulate; Lösungen oder Suspensionen in wäßrigen oder nichtwäßrigen Flüssigkeiten; eßbare Schäume oder Schaumspeisen; oder Öl-in-Wasser-Flüssigemulsionen oder Wasser-in-Öl-Flüs-

sigemulsionen dargereicht werden.

So läßt sich beispielsweise bei der oralen Verabreichung in Form einer Tablette oder Kapsel die Wirkstoffkomponente mit einem oralen, nichttoxischen und pharmazeutisch unbedenklichen inerten Trägerstoff, wie z.B. Ethanol, Glyzerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem eßbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpreßt wird, ein Schmiermittel und ein Sprengmittel zugegeben werden und das Ganze zu Tabletten verpreßt wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch läßt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepreßt wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengußformen zu verhindern. Das gefettete Gemisch wird dann zu Tabletten verpreßt. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpreßt werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so daß eine gegebene Quantität eine vorgegebene Menge der Verbindung enthält. Sirupe lassen sich herstellen, indem die Verbindung in einer wäßrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nichttoxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung läßt sich auch so herstellen, daß die Freisetzung verlängert oder retardiert wird, wie beispielsweise durch Beschichtung oder Einbettung von partikulärem Material in Polymere, Wachs u.ä.

Die Verbindungen der Formel I sowie Salze, Solvate und physiologisch funktionelle Derivate davon lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die Verbindungen der Formel I sowie die Salze, Solvate und physiologisch funktionellen Derivate davon können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepaßte pharmazeutische Formulierungen können als eigenständige Pflaster für längeren, engen Kontakt mit der Epidermis des Empfängers dargereicht werden. So kann beispielsweise der Wirkstoff aus dem Pflaster mittels Iontophorese zugeführt werden, wie in Pharmaceutical Research, 3(6), 318 (1986) allgemein beschrieben.

An die topische Verabreichung angepaßte pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepaßten pharmazeutischen Formulierungen gehören Augentropfen, wobei der Wirkstoff in einem geeigneten Träger, insbesondere einem wäßrigen Lösungsmittel, gelöst oder suspendiert ist.

An die topische Applikation im Mund angepaßte pharmazeutische Formulierungen umfassen Lutschtabletten, Pastillen und Mundspülmittel.

An die rektale Verabreichung angepaßte pharmazeutische Formulierungen können in Form von Zäpfchen oder Einläufen dargereicht werden.

An die nasale Verabreichung angepaßte pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepaßte pharmazeutische Formulierungen umfassen feinpartikuläre Stäube oder Nebel, die mittels verschiedener Arten von unter Druck stehenden Dosierspendern mit Aerosolen, Verneblern oder Insufflatoren erzeugt werden können.

An die vaginale Verabreichung angepaßte pharmazeutische Formulierungen können als Pessare, Tampons, Cremes, Gele, Pasten, Schäume oder Sprayformulierungen dargereicht werden.

Zu den an die parenterale Verabreichung angepaßten pharmazeutischen Formulierungen gehören wäßrige und nichtwäßrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wäßrige und nichtwäßrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so daß nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, daß die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer Verbindung der Formel I hängt von einer Reihe von Faktoren ab, einschließlich z.B. dem Alter und Gewicht des Tiers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer erfindungsgemäßen Verbindung für die Behandlung von neoplastischem Wachstum, z.B. Dickdarm- oder Brustkarzinom, im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so daß die Gesamttagesdosis die gleiche ist. Eine wirksame Menge eines Salzes davon kann als Anteil der wirksamen Menge der erfindungsgemäßen Verbindung *per se* bestimmt werden. Es läßt sich annehmen, daß ähnliche Dosierungen für die Behandlung der anderen, obenerwähnten Krankheitszustände geeignet sind.

Gegenstand der Erfindung sind ferner Arzneimittel enthaltend mindestens eine Verbindung der Formel I und/oder ihre pharmazeutisch verwendbaren Salze und Stereoisomere, einschließlich deren Mischungen in allen Verhältnissen, und mindestens einen weiteren Arzneimittelwirkstoff.

Gegenstand der Erfindung ist auch ein Set (Kit), bestehend aus getrennten Packungen von

(a) einer wirksamen Menge an einer Verbindung der Formel I und/oder ihrer pharmazeutisch verwendbaren Salze

und Stereoisomere, einschließlich deren Mischungen in allen Verhältnissen,
und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formel I und/oder ihrer pharmazeutisch verwendbaren Salze und Stereoisomere, einschließlich deren Mischungen in allen Verhältnissen,
und einer wirksamen Menge eines weiteren Arzneimittelwirkstoffs gelöst oder in lyophilisierter Form vorliegt.

Gegenstand der Erfindung sind die Verbindungen der Formel I gemäß Anspruch 1-8, sowie ihrer pharmazeutisch verwendbaren Salze, Tautomeren und Stereoisomeren, einschließlich deren Mischungen in allen Verhältnissen, zur Verwendung zur Behandlung von Tumoren, Tumormetastasen, proliferativer Erkrankungen der Mesangiumzellen, Hämangiom, proliferativer Retinopathie, rheumatoider Arthritis, atherosklerotischer Neovaskularisation, Psoriasis, okularer Neovaskularisation, Osteoporose, Diabetes und Fettleibigkeit, lymphoider Leukämie, Lymphom, Malaria und Prostatahypertrophie

VERWENDUNG

Die vorliegenden Verbindungen eignen sich als pharmazeutische Wirkstoffe für Säugetiere, insbesondere für den Menschen, bei der Behandlung und Bekämpfung von Krankheiten. Zu diesen Krankheiten zählen die Proliferation von Tumorzellen, die pathologische Gefäßneubildung (oder Angiogenese), die das Wachstum fester Tumoren fördert, die Gefäßneubildung im Auge (diabetische Retinopathie, altersbedingte Makula-Degeneration und dergleichen) sowie Entzündung (Schuppenflechte, rheumatoide Arthritis und dergleichen), sowie proliferative Erkrankungen der Mesangiumzellen.

Die vorliegende Erfindung umfasst die Verwendung der Verbindungen der Formel I und/oder ihre physiologisch unbedenklichen Salze und Solvate zur Herstellung eines Arzneimittels zur Behandlung oder Vorbeugung von Tumoren, Tumorerkrankungen und/oder Tumormetastasen.
Die Tumorerkrankung ist vorzugsweise ausgewählt aus der Gruppe Tumor des Plattenepithel, der Blase, des Magens, der Nieren, von Kopf und Hals, des Ösophagus, des Gebärmutterhalses, der Schilddrüse, des Darms, der Leber, des Gehirns, der Prostata, des Urogenitaltrakts, des lymphatischen Systems, des Magens, des Kehlkopfs, der Lunge, der Haut, Monozytenleukämie, Lungenadenokarzinom, kleinzelliges Lungenkarzinom, Bauchspeicheldrüsenkrebs, Glioblastom, Brustkarzinom, akute myelotische Leukämie, chronische myelotische Leukämie, akute lymphatische Leukämie, chronische lymphatische Leukämie, Hodgkin-Lymphom, non-Hodgkin-Lymphom.

Ebensfalls umfasst ist die Verwendung der erfindungsgemäßen Verbindungen nach Anspruch 1 und/oder ihre physiologisch unbedenklichen Salze und Solvate zur Herstellung eines Arzneimittels zur Behandlung von Osteoporose, Diabetes und Fettleibigkeit.

Ebensfalls umfasst ist die Verwendung der erfindungsgemäßen Verbindungen nach Anspruch 1 und/oder ihre physiologisch unbedenklichen Salze und Solvate zur Herstellung eines Arzneimittels zur Behandlung oder Vorbeugung einer Krankheit, an der Angiogenese beteiligt ist.
Eine derartige Krankheit, an der Angiogenese beteiligt ist, ist eine Augenkrankheit, wie Retina-Vaskularisierung, diabetische Retinopathie, altersbedingte Makula-Degeneration und dergleichen.
Die angiogene Erkrankung ist vorzugsweise ausgewählt aus der Gruppe diabetische Retinopathie, Arthritis, Krebs, Psoriasis, Kaposi Sarkom, Hemangioma, myocardiale Angiogenesis, atherosklerotische Plaque-Neovaskularisation, angiogene Augenerkrankungen, choroidale Neovaskularisation, retrolentale Fibroplasie, makulare Degeneration, corneale Transplantatabstossung, Rubeosis iridis, neurosculares Glaukom, Oster Webber Syndrom.

Die proliferative Erkrankung der Mesangiumzellen ist vorzugsweise ausgewählt aus der Gruppe
Glomerulonephritis, diabetische Nephropathie, maligne Nephrosclerose, thrombotisches Mikroangiopathie-Syndrom, Transplantatabstossung, Glomerulopathie.

Die Verwendung von Verbindungen der Formel I und/oder ihre physiologisch unbedenklichen Salze und Solvate zur Herstellung eines Arzneimittels zur Behandlung oder Vorbeugung von Entzündungskrankheiten, fällt ebenfalls unter den Umfang der vorliegenden Erfindung. Zu solchen Entzündungskrankheiten zählen zum Beispiel rheumatoide Arthritis, Schuppenflechte, Kontaktdermatitis, Spät-Typ der Überempfindlichkeitsreaktion und dergleichen.
Die inflammatorische Erkrankung ist vorzugsweise ausgewählt aus der Gruppe
entzündliche Darmerkrankung, Arthritis, Atherosclersose, Asthma, Allergien, entzündliche Nierenerkrankungen, multiple Sklerose, chronisch obstruktive Lungenerkrankung, entzündliche Hauterkrankungen, Pardontalerkrankungen, Psoriasis, durch T-Zellen - vermittelte Immunerkrankung.

Die entzündliche Darmerkrankung ist vorzugsweise ausgewählt aus der Gruppe
ulcerative Colitis, Morbus Crohn, unbestimmte Colitis.

Die durch T-Zellen - vermittelte Immunerkrankung ist vorzugsweise ausgewählt aus der Gruppe allergische Encephalomyelitis, allergische Neuritis, Transplantatabstossung, Graft-versus-Host-Reaktion, Myocarditis, Thyroiditis, Nephritis, systemischer Lupus erythematodes, insulinabhängiger Diabetes mellitus.

Die Arthritis-Erkrankung ist vorzugsweise ausgewählt aus der Gruppe rheumatoide Arthritis, Osteoarthritis, Caplan Syndrom, Felty Syndrom, Sjogren Syndrom, Spondylitis ankylosans, Morbus Still, Chondrocalcinosis, metabolische Arthritis, rheumatisches Fieber, Morbus Reiter, Wissler Syndrom.

Die entzündliche Nierenerkrankung ist vorzugsweise ausgewählt aus der Gruppe Glomerulonephritis, glomeruläre Verletzung, nephrotisches Syndrom, interstitielle Nephritis, Lupus nephritis, Goodpasture-Syndrom, Wegener-Granulomatose, Nierenvaskulitis, IgA-Nephropathie, idiopatische glomeruläre Erkrankung.

Die entzündliche Hauterkrankung ist vorzugsweise ausgewählt aus der Gruppe Psoriasis, atopische Dermatitis, Kontaktempfindlichkeit, Akne.

Ebenfalls umfasst ist die Verwendung der Verbindungen der Formel I und/oder ihre physiologisch unbedenklichen Salze und Solvate zur Herstellung eines Arzneimittels zur Behandlung oder Vorbeugung einer Krankheit bzw. eines Leidens bei einem Säugetier, wobei man diesem Verfahren einem kranken Säugetier, das einer derartigen Behandlung bedarf, eine therapeutisch wirksame Menge einer erfindungsgemäßen Verbindung verabreicht. Die therapeutische Menge hängt von der jeweiligen Krankheit ab und kann vom Fachmann ohne allen großen Aufwand bestimmt werden.

Die vorliegende Erfindung umfasst auch die Verwendung Verbindungen der Formel I und/oder ihre physiologisch unbedenklichen Salze und Solvate zur Herstellung eines Arzneimittels zur Behandlung oder Vorbeugung von Retina-Vaskularisierung.

Ebenfalls umfasst ist die Verwendung der Verbindungen der Formel I und/oder ihre physiologisch unbedenklichen Salze zur Herstellung eines Arzneimittels zur Behandlung und/oder Bekämpfung einer durch Tumore bedingten Krankheit bei einem Säugetier, wobei man diesem Verfahren einem kranken Säugetier, das einer derartigen Behandlung bedarf, eine therapeutisch wirksame Menge einer erfindungsgemäßen Verbindung verabreicht. Die therapeutische Menge hängt von der jeweiligen Krankheit ab und kann vom Fachmann ohne allen großen Aufwand bestimmt werden.

Die offenbarten Verbindungen der Formel I können in Verbindung mit anderen Therapeutika, einschließlich Antikrebsmitteln, verabreicht werden. Wie hier verwendet, betrifft der Begriff "Antikrebsmittel" jedes Mittel, das einem Patienten mit Krebs zum Zweck der Behandlung des Krebses verabreicht wird.

Die Verbindungen der Formel I können auch gemeinsam mit anderen gut bekannten Therapeutika, die aufgrund ihrer jeweiligen Eignung für das behandelte Leiden ausgewählt werden, verabreicht werden.

Die vorliegenden Verbindungen eignen sich auch zur Kombination mit bekannten Antikrebsmitteln. Zu diesen bekannten Antikrebsmitteln zählen die folgenden: Östrogenrezeptormodulatoren, Androgenrezeptormodulatoren, Retinoidrezeptormodulatoren, Zytotoxika, antiproliferative Mittel, Prenyl-Proteintransferasehemmer, HMG-CoA-Reduktase-Hemmer, HIV-ProteaseHemmer, Reverse-Transkriptase-Hemmer sowie weitere Angiogenesehemmer. Die vorliegenden Verbindungen eignen sich insbesondere zur gemeinsamen Anwendung mit Radiotherapie. "Östrogenrezeptormodulatoren" bezieht sich auf Verbindungen, die die Bindung von Östrogen an den Rezeptor stören oder diese hemmen, und zwar unabhängig davon, wie dies geschieht. Zu den Östrogenrezeptormodulatoren zählen zum Beispiel Tamoxifen, Raloxifen, Idoxifen, LY353381, LY 117081, Toremifen, Fulvestrant, 4-[7-(2,2-Dimethyl-1-oxopropoxy-4-methyl-2-[4-[2-(1-piperidinyl)ethoxy]phenyl]-2H-1-benzopyran-3-yl]phenyl-2,2-dimethyl-propanoat, 4,4'-Dihydroxybenzophenon-2,4-dinitrophenylhydrazon und SH646, was jedoch keine Einschränkung darstellen soll.

"Androgenrezeptormodulatoren" bezieht sich auf Verbindungen, die die Bindung von Androgenen an den Rezeptor stören oder diese hemmen, und zwar unabhängig davon, wie dies geschieht. Zu den Androgenrezeptormodulatoren zählen zum Beispiel Finasterid und andere 5a-Reduktase-Hemmer, Nilutamid, Flutamid, Bicalutamid, Liarozol und Abirateron-acetat.

"Retinoidrezeptormodulatoren" bezieht sich auf Verbindungen, die die Bindung von Retinoiden an den Rezeptor stören oder diese hemmen, und zwar unabhängig davon, wie dies geschieht. Zu solchen Retinoidrezeptormodulatoren zählen zum Beispiel Bexaroten, Tretinoin, 13-cis-Retinsäure, 9-cis-Retinsäure, $\alpha$-Difluormethylornithin, ILX23-7553, trans-N-(4'-Hydroxy-phenyl)retinamid und N-4-Carboxyphenylretinamid.

"Zytotoxika" bezieht sich auf Verbindungen, die in erster Linie durch direkte Einwirkung auf die Zellfunktion zum Zelltod führen oder die die Zellmyose hemmen oder diese stören, darunter Alkylierungsmittel, Tumornekrosefaktoren, interkaliernde Mittel, Mikrotubulin-Hemmer und Topoisomerase-Hemmer.

Zu den Zytotoxika zählen zum Beispiel Tirapazimin, Sertenef, Cachectin, Ifosfamid, Tasonermin, Lonidamin, Carboplatin, Altretamin, Prednimustin, Dibromdulcit, Ranimustin, Fotemustin, Nedaplatin, Oxaliplatin, Temozolomid, Heptaplatin, Estramustin, Improsulfan-tosylat, Trofosfamid, Nimustin, Dibrospidium-chlorid, Pumitepa, Lobaplatin, Satraplatin, Profiromycin, Cisplatin, Irofulven, Dexifosfamid, cis-Amindichlor(2-methylpyridin)platin, Benzylguanin, Glufosfamid, GPX100, (trans,trans,trans)-bis-mu-(hexan-1,6-diamin)-mu-[diamin-platin(II)]bis[diamin(chlor)platin(II)]-tetrachlorid, Diarizidinylspermin, Arsentrioxid, 1-(11-Dodecylamino-10-hydroxyundecyl)-3,7-dimethylxanthin, Zorubicin, Idarubicin, Daunorubicin, Bisantren, Mitoxantron, Pirarubicin, Pinafid, Valrubicin, Amrubicin, Antineoplaston, 3'-Desamino-3'-morpholino-13-desoxo-10-hydroxycarminomycin, Annamycin, Galarubicin, Elinafid, MEN10755 und 4-Desmethoxy-3-desamino-3-aziridinyl-4-methylsulfonyl-daunorubicin (siehe WO 00/50032), was jedoch keine Einschränkung darstellen soll. Zu den Mikrotubulin-Hemmern zählen zum Beispiel Paclitaxel, Vindesin-sulfat, 3',4'-Dideshydro-4'-desoxy-8'-norvincaleukoblastin, Docetaxol, Rhizoxin, Dolastatin, Mivobulin-isethionat, Auristatin, Cemadotin, RPR109881, BMS184476, Vinflunin, Cryptophycin, 2,3,4,5,6-pentafluor-N-(3-fluor-4-methoxyphenyl)benzolsulfonamid, Anhydrovinblastin, N,N-dimethyl-L-valyl-L-valyl-N-methyl-L-valyl-L-prolyl-L-prolin-t-butylamid, TDX258 und BMS188797.
Topoisomerase-Hemmer sind zum Beispiel Topotecan, Hycaptamin, Irinotecan, Rubitecan, 6-Ethoxypropionyl-3',4'-O-exo-benzyliden-chartreusin, 9-Methoxy-N,N-dimethyl-5-nitropyrazolo[3,4,5-kl]acridin-2-(6H)propanamin, 1-Amino-9-ethyl-5-fluor-2,3-dihydro-9-hydroxy-4-methyl-1H,12H-benzo[de]-pyrano[3',4':b,7]indolizino[1,2b]chinolin-10,13(9H,15H)-dion, Lurtotecan, 7-[2-(N-Isopropylamino)ethyl]-(20S)camptothecin, BNP1350, BNPI1100, BN80915, BN80942, Etoposid-phosphat, Teniposid, Sobuzoxan, 2'-Dimethylamino-2'-desoxy-etoposid, GL331, N-[2-(Dimethylamino)ethyl]-9-hydroxy-5,6-dimethyl-6H-pyrido[4,3-b]carbazol-1-carboxamid, Asulacrin, (5a,5aB,8aa,9b)-9-[2-[N-[2-(Dimethylamino)ethyl]-N-methylamino]ethyl]-5-[4-hydroxy-3,5-dimethoxy-phenyl]-5,5a,6,8,8a,9-hexohydrofuro(3',4':6,7)naphtho(2,3-d)-1,3-dioxol-6-on, 2,3-(Methylendioxy)-5-methyl-7-hydroxy-8-methoxybenzo[c]phenanthridinium, 6,9-Bis[(2-aminoethyl)amino]benzo[g]isochinolin-5,10-dion, 5-(3-Aminopropyl-amino)-7,10-dihydroxy-2-(2-hydroxyethylaminomethyl)-6H-pyrazolo[4,5,1-de]-acridin-6-on, N-[1-[2(Diethylamino)ethylamino]-7-methoxy-9-oxo-9H-thioxan-then-4-ylmethyl]formamid, N-(2-(Dimethyl-amino)-ethyl)acridin-4-carboxamid, 6-[[2-(Dimethylamino)-ethyl]amino]-3-hydroxy-7H-indeno[2,1-c]chinolin-7-on und Dimesna.
Zu den "antiproliferativen Mitteln" zählen Antisense-RNA- und -DNA-Oligonucleotide wie G3139, ODN698, RVASKRAS, GEM231 und INX3001, sowie Antimetaboliten wie Enocitabin, Carmofur, Tegafur, Pentostatin, Doxifluridin, Trimetrexat, Fludarabin, Capecitabin, Galocitabin, Cytarabinocfosfat, Fosteabin-Natriumhydrat, Raltitrexed, Paltitrexid, Emitefur, Tiazofurin, Decitabin, Nolatrexed, Pemetrexed, Nelzarabin, 2'-Desoxy-2'-methylidencytidin, 2'-Fluormethylen-2'-desoxycytidin, N-[5-(2,3-Dihydrobenzo-furyl)sulfonyl]-N'-(3,4-dichlorphenyl)harnstoff, N6-[4-Desoxy-4-[N2-[2(E),4(E)-tetradecadienoyl]glycylamino]-L-glycero-B-L-manno-heptopyranosyl]adenin, Aplidin, Ecteinascidin, Troxacitabine, 4-[2-Amino-4-oxo-4,6,7,8-tetrahydro-3H-pyrimidino[5,4-b][1,4]thiazin-6-yl-(S)-ethyl]-2,5-thienoyl-L-glutaminsäure, Aminopterin, 5-Flurouracil, Alanosin, 11-Acetyl-8-(carbamoyloxymethyl)-4-formyl-6-methoxy-14-oxa-1,11-diazatetracyclo(7.4.1.0.0)-tetradeca-2,4,6-trien-9-ylessigsäureester, Swainsonin, Lometrexol, Dexrazoxan, Methioninase, 2'-cyan-2'-desoxy-N4-palmitoyl-1-B-D-Arabinofuranosylcytosin und 3-Amino-pyridin-2-carboxaldehyd-thiosemicarbazon. Die "antiproliferativen Mittel" beinhalten auch andere monoklonale Antikörper gegen Wachstumsfaktoren als bereits unter den "Angiogenese-Hemmern" angeführt wurden, wie Trastuzumab, sowie Tumorsuppressorgene, wie p53, die über rekombinanten virusvermittelten Gentransfer abgegeben werden können (siehe z.B. US-Patent Nr. 6,069,134).

**Wirkungsnachweis von pharmakologischen Inhibitoren auf die Proliferation/Vitalität von Tumorzellen *in vitro***

**1.0 Hintergrund**

In der vorliegenden Versuchsbeschreibung wird die Hemmung der Tumorzellproliferation/ Tumorzellvitalität durch Wirkstoffe beschrieben.
Die Zellen werden in geeigneter Zelldichte in Mikrotiterplatten (96-well Format) ausgesät und die Testsubstanzen werden in Form einer Konzentrationreihe zugegeben. Nach vier weiteren Tagen der Kultivierung in serumhaltigem Medium kann die Tumorzellproliferation/ Tumorzellvitalität mittels eines Alamarblue-Testsystem bestimmt werden.

**2.0 Versuchsdurchführung**

**2.1 Zellkultur**

Beispielsweise käuflich erhältliche Colon-Carcinom-Zelllinien, Zelllinien des Eierstocks, Zelllinien der Prostata oder Zelllinien der Brust etc.
Die Zellen werden in Medium kultiviert. In Abständen von mehreren Tagen werden die Zellen mit Hilfe von Trypsin-Lösung von den Kulturschalen abgelöst und in geeigneten Verdünnung in frischem Medium ausgesät. Die Zellen werden bei 37° Celsius und 10% $CO_2$ kultiviert.

**2.2. Aussaat der Zellen**

Eine definierte Zellzahl (z.B. 2000 Zellen) werden pro Kultur/ well in einem Volumen von 180µl Kulturmedium in Mikrotiterplatten (96 well Zellkulturplatten) mit einer Mehrkanalpipette ausgesät. Die Zellen werden anschließend in einem CO2-Brutschrank (37°C und 10% CO2) kultiviert.

**2.3. Zugabe der Testsubstanzen**

Die Testsubstanzen werden beispielsweise in DMSO gelöst und anschließend in entsprechender Konzentration (gegebenenfalls einer Verdünnungsreihe) im Zellkulturmedium eingesetzt. Die Verdünnungs-stufen können je nach Effizienz der Wirkstoffe und gewünschter Spreizung der Konzentrationen angepasst werden. Die Testsubstanzen werden in entsprechenden Konzentrationen mit Zellkulturmedium versetzt. Die Zugabe der Testsubstanzen zu den Zellen kann am selben Tag wie die Aussat der Zellen erfolgen. Dazu wird aus der Vorverdünnungsplatte jeweils 20µl Substanzlösung in die Kulturen/wells gegeben. Die Zellen werden für weitere 4 Tage bei 37°Celsius und 10% $CO_2$ kultiviert.

**2.4. Messung der Farbreaktion**

Pro well werden jeweils 20µl AlamarBlue Reagenz gegeben und die Microtiterplatten werden beispielsweise für weitere sieben Stunden in einem CO2-Brutschrank (bei 37°C und 10% CO2) inkubiert. Die Platten werden an einem Reader mit einem Fluoreszenzfilter bei einer Wellenlänge von 540nm gemessen. Die Platten können direkt vor der Messung leicht geschüttelt werden.

**3. Ausweitung**

Der Extinktionswert der Mediumkontrolle (keine Verwendung von Zellen und Testsubstanzen) wird von allen anderen Extinktionswerten subtrahiert. Die Kontrollen (Zellen ohne Testsubstanz) werden gleich 100 Prozent gesetzt und alle anderen Extinktionswerte hierzu in Beziehung gesetzt (beispielsweise in % der Kontrolle) ausgedrückt:

Rechnung:

$$\frac{100 * (\text{Wert mit Zellen und Testsubstanz - Wert der Mediumkontrolle})}{(\text{Wert mit Zellen - Wert der Mediumkontrolle})}$$

Die Bestimmung von $IC_{50}$ Werten (50%ige Hemmung) erfolgt mit Hilfe von Statistikprogrammen wie z.B. RS1. $IC_{50}$-Daten erfindungsgemäßer Verbindungen sind in Tabelle 1 angegeben.

| Material | Best. Nr. | Hersteller |
|---|---|---|
| ------ | | |
| Mikrotiterplatten für die Zellkultur (Nunclon Surface 96well Plate) | | 167008 Nunc |
| DMEM | P04-03550 | Pan Biotech |
| PBS (10x) Dulbecco | 14200-067 | Gibco |
| 96well Platten (Polypropylen) | | 267334 Nunc |
| AlamarBlue™ | BUF012B | Serotec |
| FCS | 1302 | Pan Biotech GmbH |
| Trypsin/EDTA Solution 10x | L 2153 | Biochrom AG |
| 75cm2 Kulturflaschen | 353136 | BD Falcon |
| A2780 | 93112519 | ECACC |
| Colo205 | CCL222 | ATCC |
| MCF7 | HTB22 | ATCC |
| PC3 | CRL-1435 | ATCC |

**Bestimmung der Proliferationshemmung durch Inhibitoren der Methioninaminopeptidase 2 im BrdU Proliferationstest (zellulärer Assay)**

Die Hemmung der Proliferation wird durch Inkorporation von Bromodesoxyuridin (BrdU) in humanen Endothelzellen aus der Nabelschnur (HUVEC, PromoCell, C-12200) bestimmt. Die HUVEC werden bei 37°C und 5% $CO_2$ in Basalmedium (PromoCell, C-22200) mit Supplementmix (PromoCell, C-39225) kultiviert. Nach Ablösung der Zellen mittels Trypsin/EDTA wird die Lebendzellzahl bestimmt und die Zellen in einer Dichte von 1000 Zellen pro Kavität in

einem Gesamtvolumen von 175 μl ausgesät (Kavitäten werden zuvor entweder mit supplementiertem Kulturmedium für 1-2 Stunden bei 37°C oder mit 1,5% Gelatine für 0,5 - 2 Stunden bei 37°C beschichtet). Nach 24 stündiger Kultivierung werden die Testsubstanzen in verschiedenen Konzentrationen (z.B. finale Konzentrationen 30 μM bis 0,03 nM in 10-fach Verdünnungsschritten) und einem Volumen von 25 μl hinzugegeben. Die DMSO Konzentration wird mit 0,3% konstant gehalten. Nach insgesamt 48 oder 72 stündiger Kultivierung werden 20 μl Bromdesoxyuridin (Roche, # 11647229001 1:1000 verdünnt in Kulturmedium, Endkonzentration 10μM) hinzugegeben und für weitere 20 bis 24 Stunden kultiviert. Nach insgesamt 72 bzw. 96 Stunden Inkubation mit Testsubstanzen wird das Kulturmedium entfernt und ein immunhistochemischer Nachweis zur Detektion der BrdU-Inkorporation durchgeführt (BrdU ELISA, Roche, # 11647229001). Hierzu werden die Zellen für 30 min bei Raumtemperatur mit einem Fixativ behandelt und anschließend mit einem Peroxidase-markiertem anti-BrdU Antikörper (1:100 verdünnt in Antikörperverdünnungspuffer) für 60 min bei Raumtemperatur inkubiert. Nach dreimaligem Waschen mit 1-fach konzentriertem DPBS-Puffer (Gibco, # 14200) wird die enzymatische Umsetzung in TMB-Substratlösung initiiert. Die Farbentwicklung wird nach 15 min durch Zugabe von 25 μl einer 1 M Schwefelsäurelösung abgestoppt. Eine Bestimmung der optischen Dichte erfolgt innerhalb von 5 min durch Messung bei einer Wellenlänge von 450 nM. Als Kontrollen dienen Kavitäten mit DMSO-behandelten Zellen (100% Kontrolle) oder leere Kavitäten (Leerwert). Die Sensitivität dieses Tests gegenüber Inhibitoren der Methioninaminopeptidase wird durch Verwendung des Inhibitors Fumagillin überprüft und bestätigt.

**MetAP-2 Aktivitätsmessung**

Die MetAP-2 Aktivität wird durch eine Kopplung von enzymatischen Reaktionen nachgewiesen. Das Tripeptid Met-Arg-Ser (MAS) wird als Substrat eingesetzt. Das freigesetzte Methionin wird zunächst durch die L-Aminooxidase (AAO) zu $Met_{ox}$ und $H_2O_2$ umgesetzt. Im zweiten Schritt katalysiert die Peroxidase (POD) mt Hilfe des $H_2O_2$ die Oxidation des Leukofarbstoffs Dianisidin zu $Dianisidin_{ox}$, dessen Zunahme photometrisch bei 450 nm detektiert wird.
MetAP-2 Aktivität kann als Kinetik kontinuierlich aufgezeichnet werden. Das Reaktionsschema verdeutlicht, dass pro mol Methionen ein mol $Dianisidin_{ox}$ gebildet wird. Die MetAP-2 Enzymaktivität lässt sich deshalb direkt als Δ Absorption pro Zeiteinheit berechnen. Eine Qualifizierung der MetAP-2 Aktivität (mol Met/Zeiteinheit) ist mit Hilfe des $Dianisidin_{ox}$-Extinktionskoeffizienten möglich.
Die Extinktionsänderung pro Zeiteinheit wird graphisch dargestellt und eine Steigungsberechnung im visuell linearen Bereich der Reaktion durchgeführt. Die Aktivitäten der verbindungen sind in Tabelle 1 zusammengefasst.

Löslichkeitsmessung

Bestimmung nach "Shake flask solubility measurement"

Eluentenherstellung:

| | |
|---|---|
| Eluent A: | 2 ml Diethylamin, zur Synthese +<br>1000 ml Methanol, LiChrosolv |
| Eluent B: | 5 g Ammoniumacetat, zur Analyse +<br>5 ml Methanol, LiChrosolv +<br>995 ml Reinstwasser |

Probenlösungsmittel:

Puffer: 3,954 g Natriumdihydrogenphosphat-Monohydrat + 6,024 g
Natriumchlorid + 950 ml Reinstwasser mit 0,1 M NaOH oder 0,1 M HCl wird der pH-Wert eingestellt.

Probenvorbereitung:

Die Proben werden bei 37°C und 450 rpm 24 h lang geschüttelt.
Nach ca. 7h wird der pH Wert der Proben überprüft und gegebenenfalls nachgestellt.

Es wird auch kontrolliert, ob die Probe noch im Überschuss vorhanden ist.

Kurz vor Ende der 24h-Schüttelzeit werden die Proben nochmals auf pH-Wert und auf einen Niederschlag überprüft.

Reinstwasser Anlage: MilliQ Gradient, Millipore, Gerät: F3PN37462D
Schüttler: TiMix control, Bühler
Inkubationshaube: TH 15 Bühler
pH Meter: 766 Calimatic Knick Gerät: pH 1
pH Elektrode: InLab 423 Mettler
APCI-MS (atmospheric pressure chemical ionization - mass spectrometry) $(M+H)^+$.

Die racemischen Endprodukte der erfindungsgemäßen Verbindungen oder die racemischen Intermediate lassen sich über eine chirale HPLC oder SFC-Säule einfach und sowohl im analytischen als auch im präparativen Maßstab trennen. §

NMR Spektrum nach Zugabe von deuterierter Trifluoressigsäure

Methoden:

%

HPLC-MS Analysis Bedingungen:

1. Säule : Acquity BEH C-18 (2.1 x 100mm , 1.7 μm)
2. Mobile Phase : A - 5 mM Ammonium Acetat in Wasser B -Acetonitril
3. Flow Mode : Gradient

| % | A | % | B |
|---|---|---|---|
| 0.0 | 0.3 | 90 | 10 |
| 1.0 | 0.3 | 90 | 10 |
| 2.0 | 0.3 | 85 | 15 |
| 4.5 | 0.3 | 45 | 55 |
| 6.0 | 0.3 | 10 | 90 |
| 8.0 | 0.3 | 10 | 90 |
| 9.0 | 0.3 | 90 | 10 |
| 10.0 | 0.3 | 90 | 10 |

4. Fluß : 0.3 ml/min.
5. UV max. : 254.0 nm
6. Column Temp. : 30.0 deg.
7. Sample Preparation: Acetonitril + Wasser

*HPLC: La Chrom Anlage

Chromolite Performance RP18-e 100-4,6mm
Gradient: Acetonitril/$_{H2O}$ mit 0,01% Ameisensäure
Methode: Chromolith/Chromolith (extended)
Fluß: 3mL/min

$

Säule: XBridge C8, 3.5 μm, 4.6 x 50 mm;

Solvent A: Wasser + 0.1 % TFA;
Solvent B: Acetonitril + 0.1 % TFA;
Fluß: 2 ml/min;
Gradient: 0 min: 5 % B, 8 min: 100 % B, 8.1 min: 10 %;
Detektion bei 254 nm

1) Enantiomerentrennung:

Trennung auf Chiralcel OD-H mit n-Heptan/Ethanol = 70/30.
Die Substanz wird in 10 ml n-Heptan/EtOH = 1/1 gelöst und über 5x25cm Chiralcel OD- Säule mit 20 μm Material bei einem Fluß von 100mL/min n-Heptan/Ethanol = 70/30 getrennt.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation.
F.: Schmelzpunkt
Massenspektrometrie (MS): EI (Elektronenstoß-Ionisation) $M^+$
FAB (Fast Atom Bombardment) $(M+H)^+$
ESI (Electrospray Ionization) $(M+H)^+$
APCI-MS (atmospheric pressure chemical ionization - mass spectrometry) $(M+H)^+$.

Syntheseschemata zur Herstellung von Verbindungen der Formel I:

a)

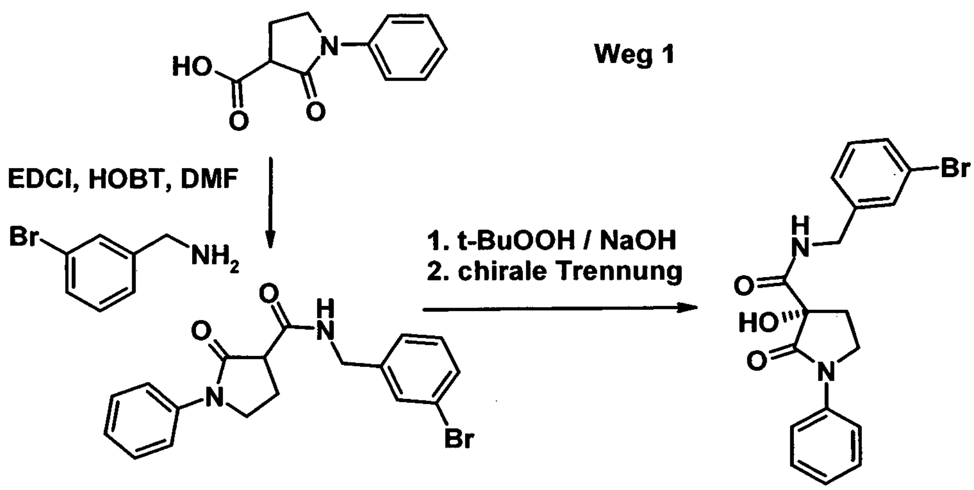


b)

**Weg 2**

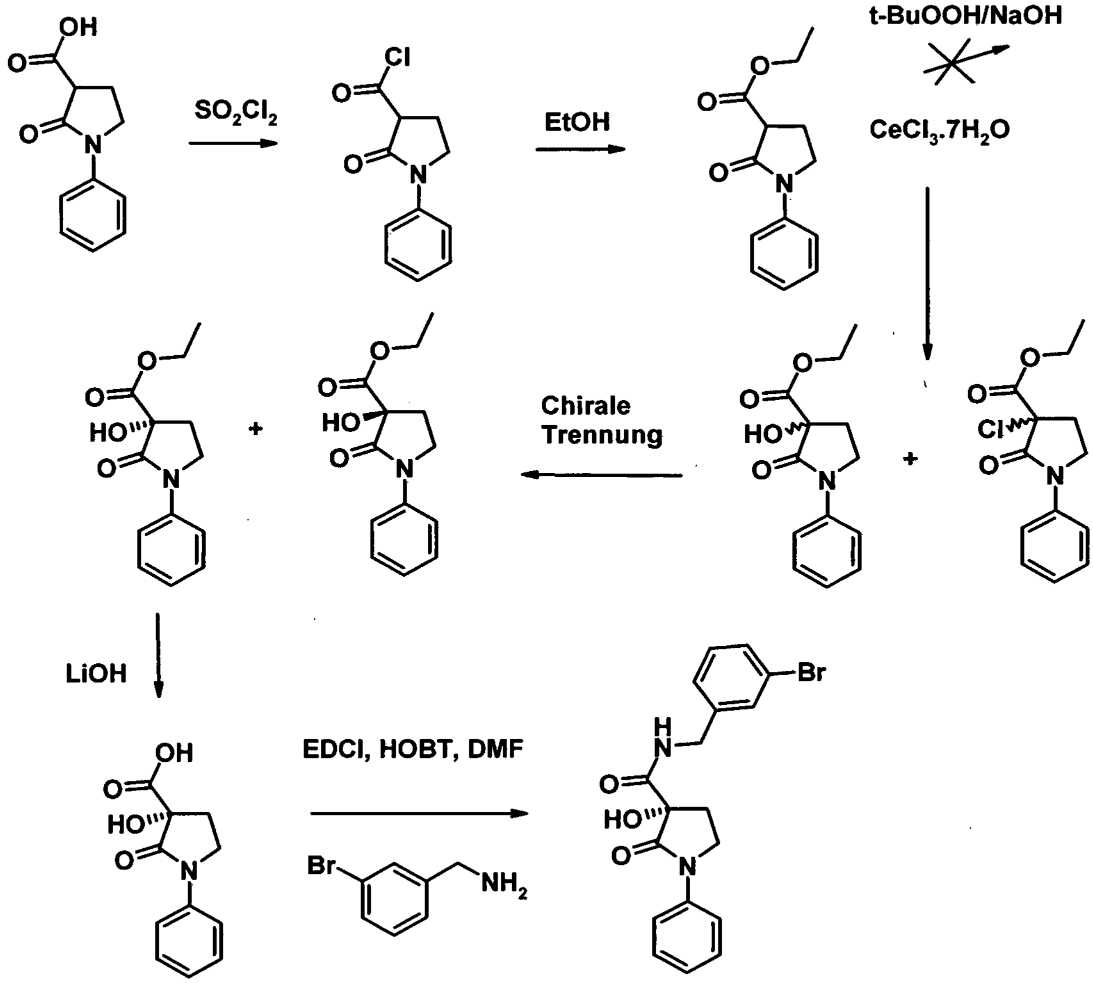


c)

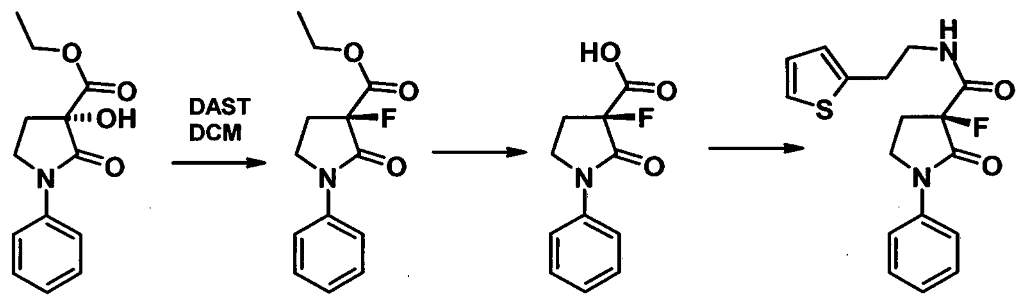


a) Weg 1: Die Säure wird in einer Amidkupplung zum entsprechenden Amid umgesetzt und dann mit tertiärem Butylhydroperoxid in einer basischen Umgebung am Kohlenstoff zwischen den beiden Carboxylgruppen zum Alkohol oxidiert (gefolgt von einer Enantiomerentrennung über Chromatographie).

b) Weg 2: Alternativ wurde gefunden, dass unter den gleichen oxidativen Bedingungen wie in Weg 1 für das Amid beschrieben, sich der Ethylester der Ausgangssäure nicht oxidieren lässt. Jedoch gelingt dies mit Cerchlorid. Das dabei mit 30% erhaltene Chlorderivat lässt sich analog den Alkoholen in die Enantiomere trennen und dann weiter derivatisieren. (weitere Umsetzungen hier nicht gezeigt, Produkte sind dann aber z.B. "A39", "A43" und "A61").

c) Die Verwendung von Cerfluorid ergibt jedoch nicht das analoge Fluorderivat. Die Umsetzung der Alkoholverbindung mit DAST (Diethylaminosulfurtrifluorid) in Dichlormethan führt zum gewünschten Fluor-Analogon. Die Testung bestätigt die aus mechanistischen Betrachtungen der Fluorierung von Alkoholen mit DAST erwartete Inversion am Asymmetriezentrum.

d)

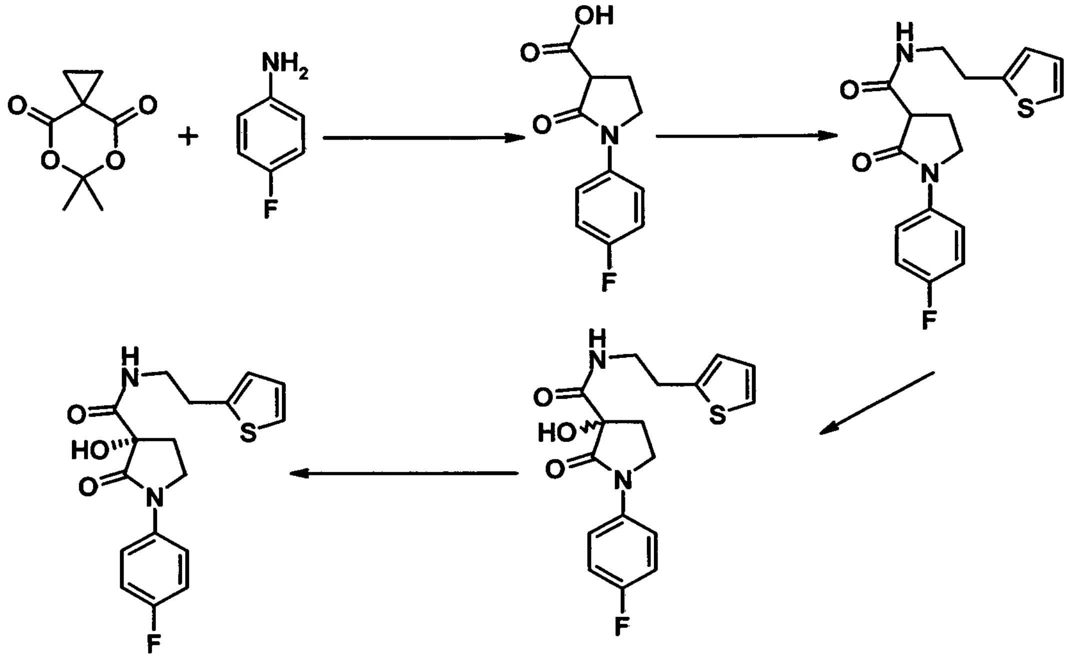

In d) ist gezeigt, wie sich N-Aryl-lactam-carbonsäuren herstellen lassen. Dieser Weg lässt sich dann leicht durchführen, wenn die Stickstoffverbindung eine Flüssigkeit ist. Es zeigt sich, dass das gemeinsame Schmelzen der Edukte, wenn die Stickstoff-Arylverbindung nicht flüssig ist, nicht vorteilhaft ist. Man erhält jedoch das gewünschte Produkt, wenn man die Stickstoff-Arylverbindung schmilzt und zu dieser Schmelze den Dicarbonsäureester (Meldrum's acid) gibt.

e)

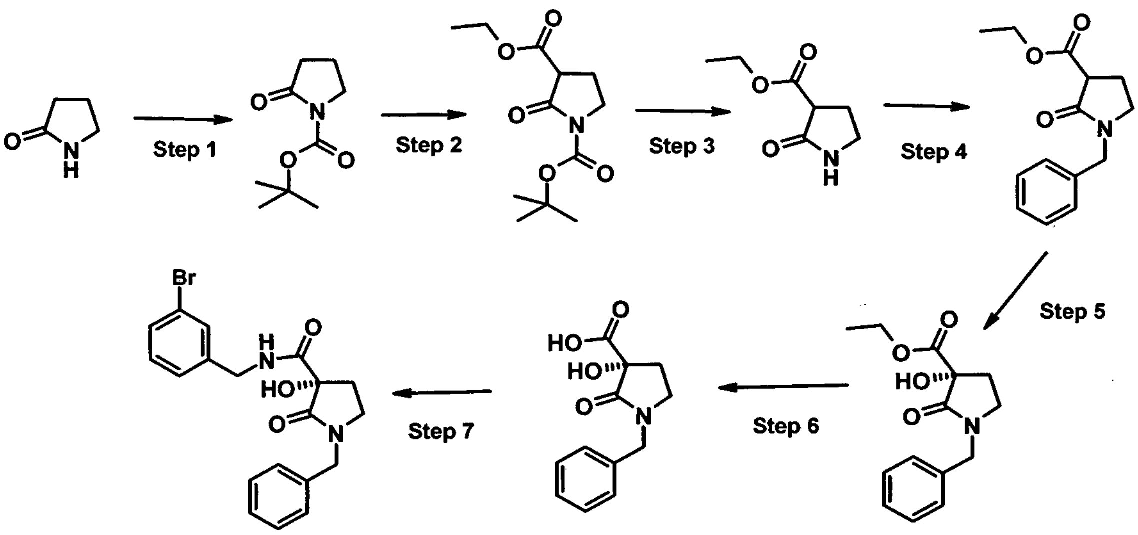


In e) ist ein Weg gezeigt, um die N-Alkaryl-lactam-carbonsäure herzustellen.

f)

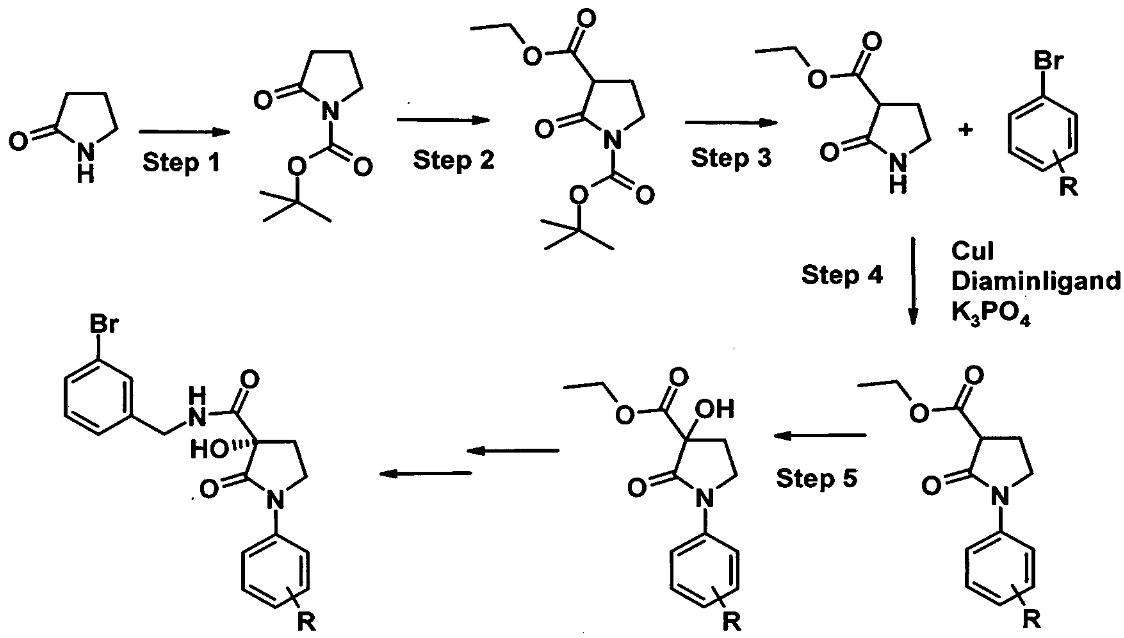


In f) ist eine weitere Alternative gezeigt, die durchgeführt wird, um solche Arylreste am Lactam-Stickstoff verfügbar zu machen, bei denen die analoge Stickstoffverbindung keine Flüssigkeit ist. Hier wird der Lactam-Stickstoff an ein Arylhalogenid gekuppelt, ein Schritt, der bevorzugt durch Übergangsmetallverbindungen katalysiert wird.

Beispiel 1

Synthese von 3-Hydroxy-2-oxo-1-phenyl-pyrrolidin-3-carbonsäure-(2-thiophen-2-yl-ethyl)-amid ("A65") [analog Syntheseschema a); Weg 1]

a) 2-Oxo-1-phenyl-pyrrolidin-3-carbonsäure-(2-thiophen-2-yl-ethyl)-amid

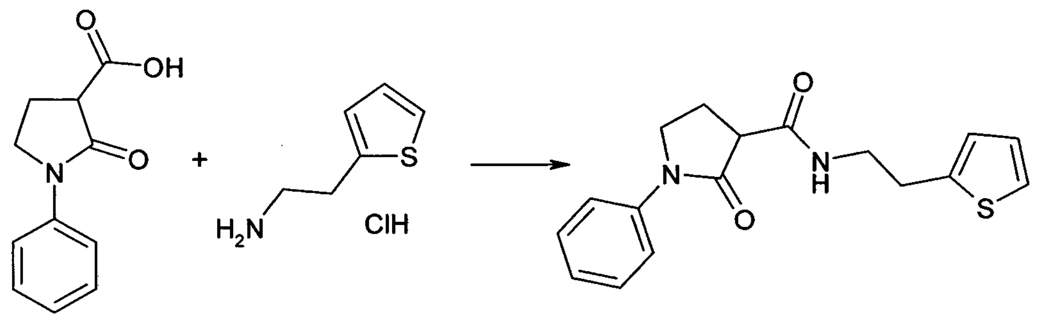

Man vereint in einem Mikrowellengefäß 200 mg 1-Phenyl-2-oxo-3-pyrrolidin-carbonsäure, 155 mg 2-Thiophenethylamin Hydrochlorid, 550 mg N-(3-Diemethylaminopyroyl)-N'-ethylcarbodiimid Hydrochlorid, 190 mg Hydroxybenzotriazol, 132 μl Triethylamin und 5 ml DMF. Nach dem Verschließen des Gefäßes mit einem Septum wird mittels Mikrowelle (Emrys Optimizer, PersonalChemistry) erhitzt (160°C, 5 min). Der Ansatz wird mit Wasser versetzt. Es entsteht ein Niederschlag. Es wird solange Wasser zugegeben bis kein weiterer Niederschlag mehr entsteht. Der Niederschlag wird abgesaugt und getrocknet.

Ausbeute: 239 mg;
Aussehen: weisser Feststoff;
Reinheit: 100% (220nm, UV-Spur des LC-MS);
LC-MS: 315(M+H);

HPLC: 3,03 (Rt/min);
HPLC-Methode: 1_100_2 (Gerät: LaChrom)
Säule: Chromolith Performance RP18e 100-3mm
Flussrate: 2 ml/min (Pumpe: L-7100)
Solvent A: Wasser + 0,05% HCOOH
Solvent B: Acetonitril + 0,04% HCOOH
Wellenlänge: 220 nm (Detektor: L-7455)
Gradient: 0 - 0,2 min: 99% A, 0,2 - 3,8 min: 99% A --> 100% B, 3,8 - 4,4 min: 100% B, 4,4 - 4,5 min: 100% B --> 99% A, 4,5 - 5,1 min: 99% A;

LC-MS-Methode: (Gerät: Agilent 1100 Series)

Säule: Chromolith Speed Rod RP18e-50-4.6
Flussrate: 2.4ml/min
Solvent A: Wasser + 0,05% HCOOH
Solvent B: Acetonitril + 0,04% HCOOH
WL: 220 nm
Gradient: 0-2.8min: 4%B auf 100%B, 2.8-3.3min:100%B.

b) 3-Hydroxy-2-oxo-1-phenyl-pyrrolidin-3-carbonsöure (2-thiophen-2-yl-ethyl)-amid ("A65")

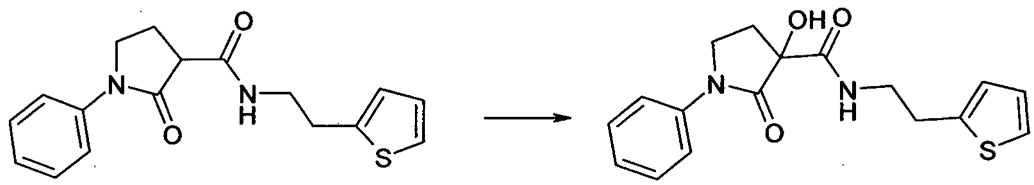

Man löst 100 mg des unter a) hergestellten Amids in 10 ml tert.-Butanol und gibt 60 mg tert.-Butylhydroperoxid (70%ig in Wasser) und 0.2 ml Natriumethylat (20%ig in Ethanol) hinzu, Dann wird für 4h bei 90°C gerührt. Der Ansatz wird bis fast zum Rückstand eingeengt. Der Rückstand wird mit Wasser und EE (Essigsäurethylester) versetzt und ausgeschüttelt. Die organische Phase wird über $Na_2SO_4$ getrocknet, abgesaugt und im Vakuum zum Rückstand eingeengt: Man erhält 80 mg. Der Rückstand wird in DMSO gelöst und über präparative HPLC aufgereinigt. Die Produktfraktionen werden vereinigt und im Vakuum zum Rückstand eingeengt. Man erhält 57 mg (54%) "A65";

Aussehen: weisser Feststoff;
Reinheit: 100% (220nm, UV-Spur des LC-MS);
LC-MS: 331 (M+H);
HPLC: 2,88 (Rt/min);
HPLC-Methode: 1_100_2 (Gerät: LaChrom)
Säule: Chromolith Performance RP18e 100-3mm
Flussrate: 2 ml/min (Pumpe: L-7100)
Solvent A: Wasser + 0,05% HCOOH
Solvent B: Acetonitril + 0,04% HCOOH
Wellenlänge: 220 nm (Detektor: L-7455)
Gradient: 0 - 0,2 min: 99% A, 0,2 - 3,8 min: 99% A --> 100% B, 3,8 - 4,4 min: 100% B, 4,4 - 4,5 min: 100% B --> 99% A, 4,5 - 5,1 min: 99% A;
LC-MS-Methode: (Gerät: Agilent 1100 Series)
Säule: Chromolith Speed Rod RP18e-50-4.6
Flussrate: 2.4ml/min
Solvent A: Wasser + 0,05% HCOOH
Solvent B: Acetonitril + 0,04% HCOOH
WL: 220 nm
Gradient: 0-2.8min: 4%B auf 100%B, 2.8-3.3min:100%B;

Präp.-HPLC-Methode: 15_35_10_50ml_empfind_o_equi.M (Gerät: Agilent 1100 Series)
Säule: Chromolith Prep Rod RP18e
Flussrate: 50m1/min
Solvent A: Acetonitril + 0,1% TFA
Solvent B: Wasser + 0,1% TFA
WL: 220 nm
Gradient: von 1 - 15% ACN in 2 min, von 15 - 35% ACN in 8 min, sammeln ab 2 min bis 11 min.

Trennung des Racemats in die Enantiomere:

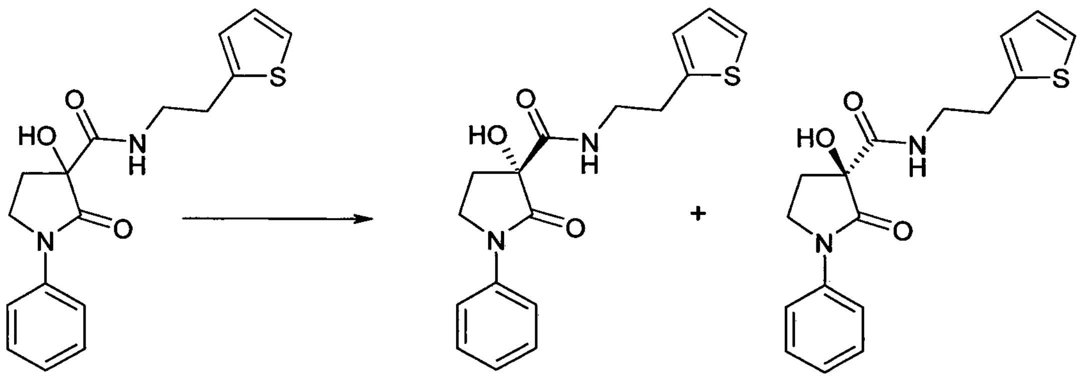

Analytische Trennung auf Chiralcel OD-H mit n-Heptan/Ethanol = 70:30.
40 mg des Racemats werden in 10 ml n-Heptan/EtOH = 1:1 gelöst und über 5x25cm Chiralcel OD-Säule mit 20 µm Material bei einem Fluß von 100 ml/min n-Heptan/Ethanol = 70:30 getrennt.

2 Fraktionen wurden gesammelt und eingeengt.

Fraktion 1: m=20 mg Enantiomer 1 :
Enantiomerenrein

Fraktion 2: m=20 mg Enantiomer 2:
Enantiomerenverhältnis:
Enantiomer 1: 0,6% : 99,4% Enantiomer 2

Beispiel 2

Herstellung von 3-Hydroxy-2-oxo-1-phenyl-pyrrolidin-3-carbonsäure [Zwischenprodukt für Syntheseschema b); Weg 2]

a)

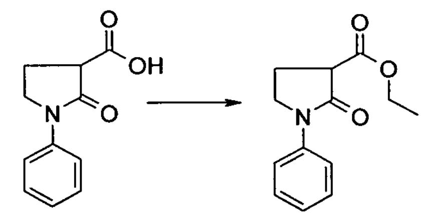

Zu einer Lösung von 2-Oxo-1-phenyl-pyrrolidin-3-carbonsäure (5 g) in 5 ml Ethanol wird bei 0°C Thionylchlorid (5.8 g) zugetropft und für 4 h bei 80°C gerührt.

Nach vollständiger Reaktion wird das Lösungsmittel im Vakuum entfernt und der Rückstand zwischen Wasser und Essigester verteilt. Die organische Phase wird mit gesättigter Natriumhydrogencarbonat-Lösung und gesättigter Natriumchlorid-Lösung gewaschen und über Natriumsulfat getrocknet. Nach dem Entfernen von Lösungsmittel und Trocknungsmittel erhält man für die weiteren Umsetzungen ausreichend reinen 2-Oxo-1-phenyl-pyrrolidin-3-carbonsäureethylester;

$^1$H NMR (400 MHz, DMSO-$d_6$) δ [ppm] 7.82-7.81 (t, J=2 Hz, 1H), 7.56-7.54 (m, 1H), 7.43-7.39 (t, J= 8 Hz, 1H), 7.24-7.21 (m, 1H), 4.17-4.12 (m, 2H), 3.3.92-3.73 (m, 3H), 2.39-2.27 (m, 2H), 1.22-1.14 (t, J= 7 Hz, 3H);

LCMS: Masse gefunden (M+1, 234);

Methode: A- 0.1% TFA in $H_2O$, B- 0.1% TFA in Acetonitril: Fluß-2.0ml/min; Säule: X Bridge C8 (50x4.6mm.3.5u) + ve mode;

Rt (min) : 3.360 Area % 98.577.

b)

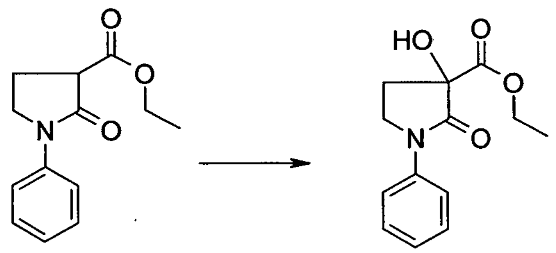

Zu einer Lösung von 2-Oxo-1-phenyl-pyrrolidin-3-carbobonsäureethylester (5 g) in 10 ml Isopropanol gibt man Cerchlorid Heptahydrat (1.59 g) und begast mit $O_2$ für 15 min, dann lässt man die Mischung 14 h rühren.

Dann werden alle flüchtigen Bestandteile im Vakuum entfernt und der Ansatz durch Chromatographie aufgereinigt. Der erhaltene 3-Hydroxy-2-oxo-1-phenyl-pyrrolidin-3-carbobonsäureethylester wird mit chiraler HPLC analysiert und nachfolgend in die Enantiomere aufgetrennt.

Das 'R'-Enantiomer eluiert nach Rt(min) 8.197.

Das 'S'-Enantiomer eluiert nach Rt(min) 11.240;

(Chiralpak AD-H(250x4.6)mm, 5μm; n-Hexane/IPA = 60:40).

$^1$H NMR (400 MHz, DMSO-$d_6$) δ [ppm] 7.67-7.65 (d, J=8 Hz, 2H), 7.42-7.38 (m, 2H), 7.20-7.17 (m, 1H), 6.53 (s, 1H), 4.13 (m, 2H), 3.91-3.80 (m, 2H), 3.92-3.79 (m, 2H), 2.58-2.51 (m, 1H), 2.17-2.10 (m, 1H), 1.19-1.16 (t, J=8 Hz, 2H); LCMS: Masse gefunden (M+1, 250);

Methode: A- 0.1% TFA in $H_2O$, B- 0.1% TFA in Acetonitril: Fluß - 2.0ml/min; Säule: X Bridge C8 (50x4.6mm.3.5u) + ve mode;

Rt (min): 2.973 Area % 98.698.

c)

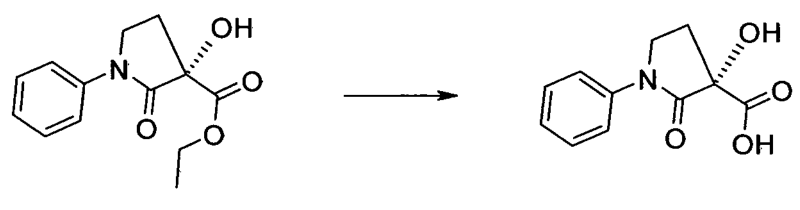

Zu einer Lösung von (S)-3-Hydroxy-2-oxo-1-phenyl-pyrrolidin-3-carbonsäureethylester (1.5 g) in THF/$H_2O$ = 3:1 gibt man LiOH/$H_2O$ (750 mg) und rührt für 4 h.

Dann wird das Lösungsmittel im Vakuum entfernt und der Ansatz mit 1.5N HCl angesäuert. Die wässrige Phase wird mit Essigester extrahiert und die vereinigten organischen Phasen werden über Natriumsulfat getrocknet. Man erhält (S)-3-Hydroxy-2-oxo-1-phenyl-pyrrolidin-3-carbonsäure;

$^1$H NMR (400 MHz, DMSO-$d_6$) δ [ppm] 7.68-7.65 (m, 2H), 7.42-7.37 (m, 2H), 7.20-7.15 (m, 1 H), 6.53 (s, 1 H), 3.87-3.81 (m, 2H), 2.56-2.51 (m, 1 H), 2.13-2.07 (m, 1 H);

LCMS: Masse gefunden (M+1, 222);

Methode: A- 0.1% TFA in $H_2O$, B- 0.1% TFA in Acetonitril: Fluß - 2.0ml/min; Säule: X Bridge C8 (50x4.6mm.3.5u) + ve mode;

Rt (min): 1.970 Area % 98.63;

Chirale HPLC:

Methode: n-Hexan:IPA : 60:40; Fluß - 1.0ml/min;
Säule: Chiralpak AD-H(250x4.6)mm, 5μm;
Rt (min): 9.794 Area % 98.698.

Amidkupplung

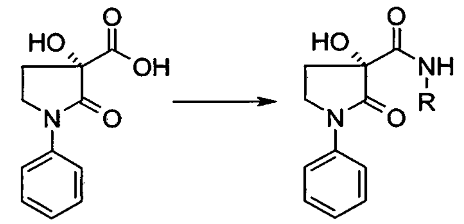

Zu einer Lösung der zuvor hergestellten Säure (1eq) und Amin (1.2 eq) in trockenen DMF (3mml / mmol) gibt man EDCI (3 eq) und HOBT (1.5 eq) und erwärmt für 20 min auf 160 °C in der Mikrowelle.
Nach beendeter Reaktion wird das Lösungsmittel im Vakuum entfernt und über Chromatographie aufgereinigt.

Beispiel 3

Herstellung von (S)-1-(3-Chlor-phenyl)-3-hydroxy-2-oxo-pyrrolidin-3-carbonsäure-(2-thiophen-2-yl-ethyl)-amid ("A37")

a)

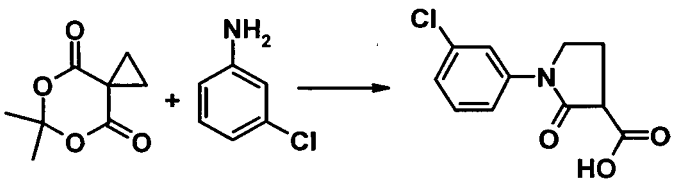

3-Chloroanilin (10.45 g) wird zu Cyclopropyl-Meldrum's Säure (6,6-Diemthyl-5,7-dioxa-spiro[2.5]octan-4,8-dion; 7g) in trockenem Dichlormethan gegeben und 14 h bei RT gerührt.
Dann wird der Ansatz mit 10%iger NaOH-Lösung gewaschen und die wässrige Phase mit 1 N HCl angesäuert, bis es zur Bildung eines farblosen Niederschlags kommt. Dieser wird abfiltriert und im Vakuum getrocknet;
$^1$H NMR (400 MHz, DMSO-$d_6$) δ [ppm] 12.89 (s, 1H), 7.83-7.82 (t, J=2 Hz, 1H), 7.57-7.54 (m, 1 H), 7.43-7.39 (t, J=8 Hz, 1 H), 7.23-7.20 (m, 1 H), 3.92-3.79 (m, 2H), 3.63-3.59 (m, 1H), 2.36-2.25 (m, 2H);
LGMS: Masse gefunden (M+1, 240);
Methode: A- 0.1 % TFA in $H_2O$, B- 0.1 % TFA in Acetonitril: Fluß - 2.0ml/min; Säule: X Bridge C8 (50x4.6mm.3.5 μ) + ve mode;
Rt (min): 3.073 Area % 98.91.
b)

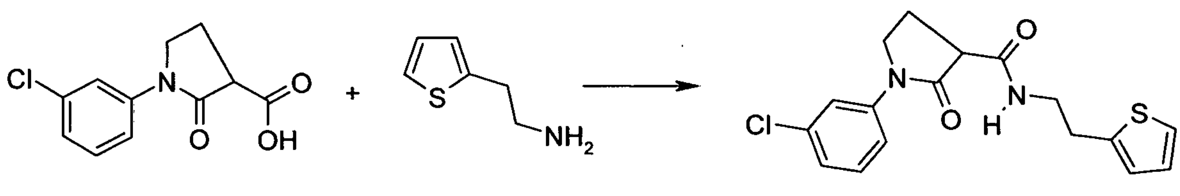

Die unter a) hergestellte Säure (0.2 g) wird in trockenem Dichlormethan gelöst. Nachfolgend werden 2-Thiopenethylamin (127 mg), Triethylamin (25 mg) und T3P (399 mg) zugegeben und der Ansatz 14 h gerührt. Man arbeitet wässrig auf und extrahiert mit Dichlormethan.

Die organische Phase wird mit Wasser, gesättigter Natriumhydrogencarbonatlösung und gesättigter Natriumchlorid-Lösung gewaschen, über Natriumsulfat getrocknet, eingedampft und an Kieselgel chromatographiert;
$^1$H NMR (400 MHz, DMSO-$d_6$) δ [ppm] 8.36-8.35 (t, J=5.4 Hz, 1H), 7.85-7.84 (d, J=2 Hz, 1 H), 7.56-7.54 (dd, J=2, 6.2 Hz, 1 H), 7.43-7.39 (t, J=8 Hz, 1 H), 7.33-7.32 (dd, J=2, 4 Hz, 1H), 7.22-7.20 (dd, J=2, 6 Hz, 1 H), 6.95-6.92 (m, 2H), 3.90-3.79 (m, 2H), 3.54-3.50 (m, 1 H), 3.38 (m, 2H), 2.96-2.93 (t, J=6.8 Hz, 1H),2.29-2.23 (m, 2H);
LCMS: Masse gefunden (M+1, 349);
Methode: A- 0.1% TFA in $H_2O$, B- 0.1% TFA in Acetonitril: Fluß - 2.0ml/min; Säule: X Bridge C8 (50x4.6mm.3.5 μ) + ve mode;
Rt (min): 4.417 Area % 99.65.
c)

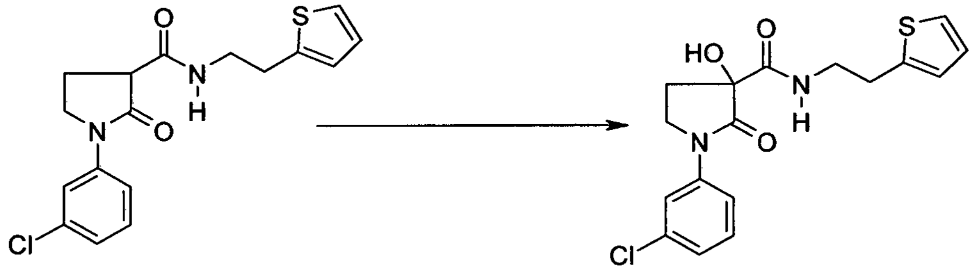

Das unter b) hergestellte Amid wird in tert.-Butanol (100 mg) gelöst. Tert.-Butylhydrogenperoxid (70% aq.)-Lösung (0.051 g) und Natriumethoxid (20%ig in Ethanol; 39 mg) werden zugefügt und für 1 h bei 70°C gerührt.
Nach beendeter Reaktion wird das Lösungsmittel im Vakuum entfernt und Wasser zugegeben bis ein farbloser Niederschlag entsteht. Dieser wird abfiltriert und chromatographiert.
Die Enantiomere werden durch chirale präparative HPLC erhalten.
Das nach Rt(min) 5.419 eluierende Enantiomer, ist die aktive S-konfigurierte Verbindung "A37"; (Chiralpak AD-H(250x4.6)mm, 5μm; n-Hexan/IPA = 60:40);
$^1$H NMR (400 MHz, DMSO-$d_6$) δ [ppm] 8.13 (t, J = 5.8, 1H), 7.89 (t, J = 2.0, 1 H), 7.61 (d, J = 8.2, 1 H), 7.43 (t, J = 8.1, 1 H), 7.33 (dd, J = 5.0, 1.0, 1 H), 7.25 (d, J = 8.0, 1 H), 6.94 (dd, J = 5.0, 3.5, 1 H), 6.88 (d, J = 3.5, 1 H), 6.69 (s, 1 H), 3.91 - 3.75 (m, 2H), 3.46 - 3.33 (m, 2H), 2.95 (t, J = 7.4, 2H), 2.08 (dt, J = 13.2, 8.2, 1 H);
LCMS: Masse gefunden (M+1, 365);
Method: A- 0.1% TFA in $H_2O$, B- 0.1% TFA in Acetonitril: Fluß - 2.0ml/min; Säule: X Bridge C8 (50x4.6mm.3.5μ) + ve mode;
Rt (min): 4.055 Area % 99.02;
HPLC > 99.25%:
Methode: A- 0.1 % TFA in $H_2O$, B- 0.1 % TFA in Acetonitril: Fluß - 2.0ml/min;
Säule: X Bridge C8 (50x4.6mm.3.5 μ);
Rt (min): 4.102;
Chirale HPLC:
Methode : n-Hexan:IPA = 60:40; Fluß - 1.0ml/min
Säule : Chiralpak AD-H(250x4.6)mm, 5 μm;
Rt (min): 5.419 Area % 100.

Beispiel 4

Herstellung von (S)-3-Fluor-2-oxo-1-phenyl-pyrrolidin-3-carbonsäure-(2-thiophen-2-yl-ethyl)-amid ("A86")

a)

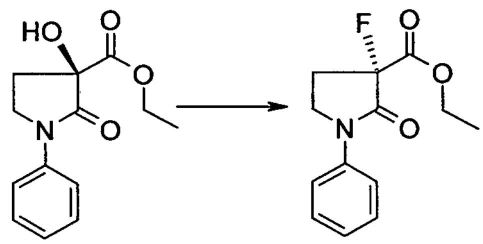

Man löst 300 mg (R)-3-Hydroxy-2-oxo-1-phenyl-pyrrolidin-3-carbonsäureethylester in trockenem Dichlormethan, gibt bei -78 °C 244 mg DAST langsam zu und rührt noch 4 h nach.

Nach beendeter Reaktion wird der Ansatz auf gesättigte Natriumhydrogencarbonat-Lösung gegossen, die wässrige Phase mit Dichlormethan extrahiert und die vereinigten organischen Phasen über Natriumsulfat getrocknet. Trocknungsmittel und Lösungsmittel werden entfernt und der Rückstand (180 mg) in der nachfolgenden Reaktion ohne weitere Aufreinigung weiter umgesetzt.

LCMS: Masse gefunden (M+1, 252);

Methode: A- 0.1% TFA in $H_2O$, B- 0.1% TFA in Acetonitril: Fluß - 2.0ml/min; Säule: X Bridge C8 (50x4.6mm.3.5u) + ve mode;

Rt (min): 3.745 Area % 93.764.

b)

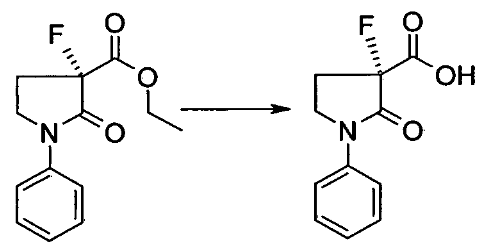

Zu einer Lösung von 180 mg (S)-3-Fluor-2-oxo-1-phenyl-pyrrolidin-3-carbonsäureethylester in THF/$H_2O$ = 3:1 gibt man 84 mg LiOH/$H_2O$ und rührt bei RT für 4 h. Nach vollständiger Reaktion wird der Ansatz mit 1.5 N HCl neutralisiert. Man extrahiert die wässrige Phase mit Ethylacetat und trocknet die organische Phase über Natriumsulfat. Nach dem Entfernen des Lösungsmittels im Vakuum erhält man 90 mg (S)-3-Fluor-2-oxo-1-phenyl-pyrrolidin-3-carbonsäure;

LCMS: Masse gefunden (M+1, 222);

Methode: A- 0.1% HCOOH, B- MeOH: Fluß =1.0 ml/min;

Säule: Atlantis DC18 (50x4.6mm.5$\mu$) + ve mode;

Rt (min): 1.948 Area % 90.84.

c)

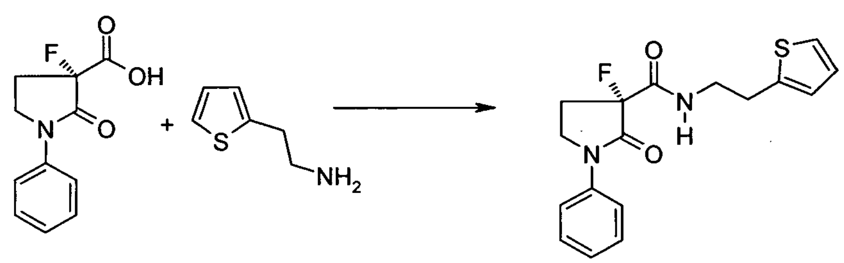

Zu einer Lösung von 90 mg (S)-3-Fluor-2-oxo-1-phenyl-pyrrolidin-3-carbonsäure und 61 mg 2-Thiophen-2-yl-ethylamin in trockenem DMF gibt man 230 mg EDCI, 65 mg HOBt und erwärmt für 20 min auf 160 °C in der Mikrowelle. Nach beendeter Reaktion entfernt man alle flüchtigen Bestandteile im Vakuum und reinigt den Rückstand chromatographisch auf. Man erhält 23 mg "A86";

$^1H$ NMR (400 MHz, DMSO-$d_6$) $\delta$ [ppm] 8.72 (s, 1 H), 7.69 (d, $J$ = 7.7, 2H), 7.50 - 7.40 (m, 2H), 7.33 (dd, $J$ = 5.1, 1.2, 1 H), 7.23 (t, $J$ = 7.4, 1 H), 6.94 (dd, $J$ = 5.1, 3.4, 1 H), 6.89 (d, $J$ = 3.4, 1 H), 3.97 (dd, $J$ = 9.2, 3.3, 1 H), 3.92 (dd, $J$ = 6.2, 3.4, 1 H), 3.48 - 3.37 (m, 2H), 2.98 (t, $J$ = 7.3, 2H), 2.77 - 2.62 (m, 1 H), 2.47 - 2.35 (m, 2H);

$^{19}F$ NMR (377 MHz, DMSO-$d_6$) δ [ppm] - 158.16.
LCMS: Masse gefunden (M+1, 333);
Methode: A- 0.1 % TFA in $H_2O$, B- 0.1 % TFA in Acetonitril: Fluß - 2.0ml/min; Säule: X Bridge C8 (50x4.6mm.3.5 μ) + ve mode;
Rt (min): 4.147 Area % 97.65;
HPLC > 97%:
Methode: A- 0.1% TFA in $H_2O$, B- 0.1% TFA in Acetonitril: Flow - 2.0ml/min;
Säule: X Bridge C8 (50x4.6mm.3.5 p);
Rt (min): 4.209.

Beispiel 5

Herstellung von (S)-3-Amino-2-oxo-1-phenyl-pyrrolidin-3-carbonsäure-(3-chlor-5-fluor-benzyl)-amid ("A91a")

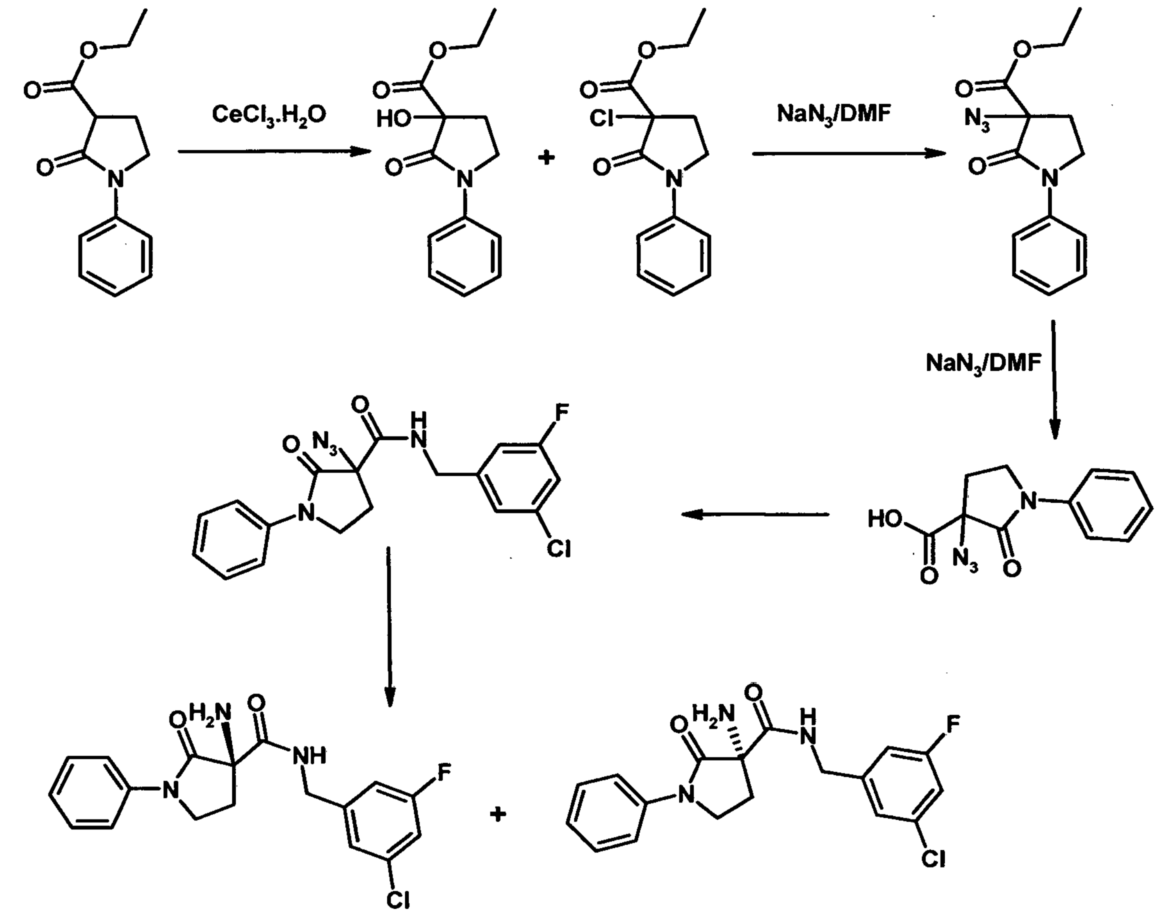


Beispiel 6

6.1 Synthese von 2-Oxo-1-phenyl-pyrrolidin-3-carbonsäureethylester

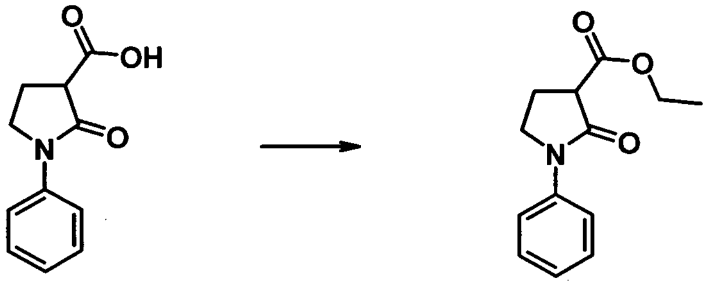

Zu einer Lösung von 2-Oxo-1-phenyl-pyrrolidin-3-carbonsäure (5 g) in Ethanol gibt man Thionylchlorid bei 0 °C (5.8 g) und rührt den Ansatz für 4 h bei 80 °C. Nach beendeter Reaktion wird der Ansatz im Vakuum aufkonzentriert, der Rückstand in Wasser aufgenommen und mit Ethylacetat extrahiert. Die organische Phase wird mit gesättigter Natriumbicarbonatlösung sowie gesättigter Natriumchlorid Lösung gewaschen und über Natriumsulfat getrocknet. Man erhält 2-Oxo-1-phenyl-pyrrolidin-3-carbonsäureethylester (4.5 g, 79%);
$^1$H NMR (400 MHz, DMSO-$d_6$) δ [ppm] 7.82 - 7.81 (t, *J* = 2 Hz, 1H), 7.56 - 7.54 (m, 1H), 7.43 - 7.39 (t, *J* = 8 Hz, 1H), 7.24 - 7.21 (m, 1H), 4.17-4.12 (m, 2H), 3.92 - 3.73 (m, 3H), 2.39 - 2.27 (m, 2H), 1.22 - 1.14 (t, *J* = 7 Hz, 3H);
LC/MS: 233.0 (M+H bei 1, 234 min)
HPLC:
Method: A- 0.1% TFA in $H_2O$, B- 0.1% TFA in ACN: Flow - 2.0ml/min.
Column: X Bridge C8 (50x4.6mm.3.5u) + ve mode
Rt (min): 3.360 Area % 98.577.

6.2 Synthese von (S)- und (R)-3-Hydroxy-2-oxo-1-phenyl-pyrrolidin-3-carbonsäureethylester und 3-Chlor-2-oxo-1-phenyl-pyrrolidin-3-carbonsäureethylester

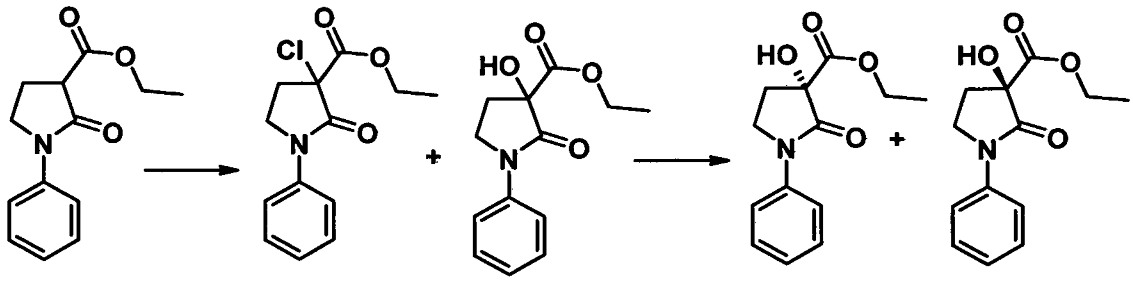

Zu einer Lösung von 2-Oxo-1-phenyl-pyrrolidin-3-carbonsäureethylester (5 g) in Isopropanol gibt man Cer(III)chlorid-heptahydrat (1.59 g) und leitet $O_2$ für 15 min ein. Der Ansatz wird für 12 h bei RT gerührt. Nach beendeter Reaktion wird das Lösungsmittel im Vakuum entfernt und der Rückstand an Kieselgel chromatographisch aufgereinigt. 3-Hydroxy-2-oxo-1-phenyl-pyrrolidin-3-carbonsäureethylester (2.7 g) wird mittels chiraler HPLC analysiert und mit dieser Methode aufgereinigt. Die Substanz, die bei Rt (min) 8.197 eluiert, ist das "R" Enantiomer (1.2 g) und die Substanz, die bei Rt (min) 11.240 eluiert, das "S"-Enantiomer (1.2 g);
(Chiralpak AD-H (250x4.6) mm, 5 μm; n-Hexan/Isopropanol = 60:40).
Das erforderliche S-Enantiomer wird weiter für die Synthese von Alkoholderivaten verwendet.

Bei dieser Oxidation entsteht mit 20% Ausbeute auch 3-Chlor-2-oxo-1-phenyl-pyrrolidin-3-carbonsäureethylester als Racemat. Dieses wird vor der Enantiomerentrennung der Alkohole über Kieselgel Chromatographie abgetrennt (1 g) und für die nächste Stufe auch racemisch eingesetzt.

6.3 Synthese von 3-Azido-2-oxo-1-phenylpyrrolidin-3-carbonsäureethylester

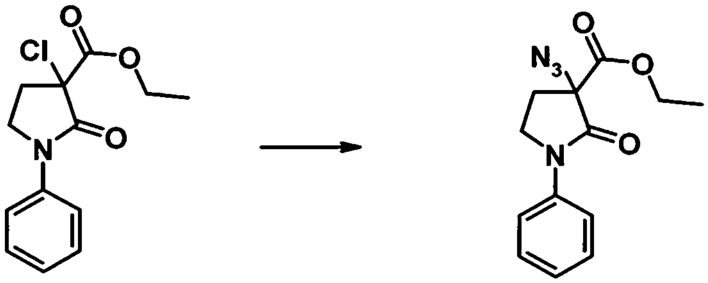

Zu einer Lösung von 3-Chlor-2-oxo-1-phenylpyrrolidin-3-carbonsäureethylester (0.2 g, 0.74 mmol) in trockenem DME (5 ml) gibt man $NaN_3$ (58 mg, 0.89 mmol). Die Reaktionslösung wird unter Sticktoff für 14 h bei 75°C gerührt. Nach Vervollständigung wir der Ansatz mit Essigester verdünnt und mit Wasser extrahiert. Die organische Phase wird über Natriumsulfat getrocknet, aufkonzentriert und der Rückstand direkt im nächsten Schritt weiter eingesetzt (200 mg, 97%);
Dünnschicht-Chromatogramm: Hexan/Ethylacetat = 7:3 $R_f$: 0.4;
LC/MS: 275.0 (M+H bei 1,275min);
$^1$H NMR (DMSO-$d_6$, 400MHz): δ [ppm] 7.67 - 7.65 (m, 2H), 7.46 - 7.42 (m, 2H), 7.26 - 7.23 (m, 1H), 4.30 - 4.22 (m, 2H), 3.95 - 3.92 (m, 2H), 2.68 - 2.63 (m, 1 H), 2.16 - 2.10 (m, 1 H), 1.22 (t, J = 7.08 Hz, 3H).

6.4 Synthese von 3-Azido-2-oxo-1-phenylpyrrolidin-3-carbonsäure

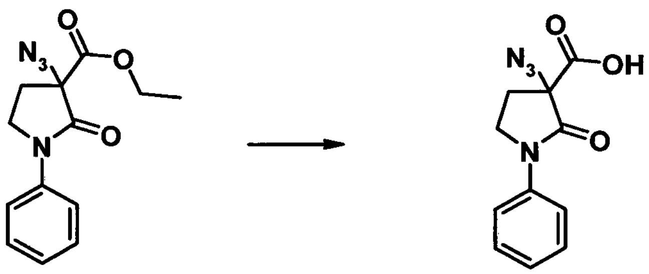

Zu einer Lösung von 3-Azido-2-oxo-1-phenylpyrrolidin-3-carbonsäureethylester (0.2 g, 0.7 mmol) in THF (2 ml) und $H_2O$ (1 ml) gibt man LiOH (91 mg, 2.1 mmol) und rührt den Ansatz bei RT für 20 Stunden. Nach vollständiger Reaktion werden alle flüchtigen Bestandteile im Vakuum entfernt, der Rückstand mit Wasser verdünnt und mit 1.5 N HCl-Lösung angesäuert. Der erhaltene Niederschlag wird abfiltriert und mit Wasser gewaschen. Nach dem Trocknen im Vakuum erhält man einen farblosen Feststoff als Produkt. (150 mg, 84%);
Dünnschicht-Chromatogramm: Hexan /Ethylacetat (7/3): $R_f$: 0.4;
LC/MS: 247.0 (M+H bei 1, 247 min);
$^1$H NMR (DMSO-$d_6$,400 MHz): δ [ppm] 7.68-7.66 (m, 2H), 7.45-7.41 (m, 2H), 7.25-7.21 (m, 1H), 3.95-3.89 (m, 2H), 2.64-2.59 (m, 1H), 2.13-2.05 (m, 1H).

6.5 Synthese von 3-Azido-2-oxo-1-phenyl-pyrrolidin-3-carbonsäure-(3-chlor-5-fluor-benzyl)amid ("A150")

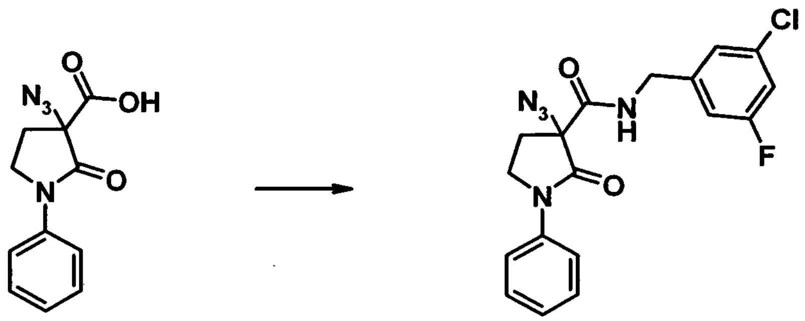

Zu einer Lösung von 3-Azido-2-oxo-1-phenylpyrrolidin-3-carbonsäure (150 mg, 0.61 mmol) in 5 ml Dichloromethan gibt man 3-Chlor-5-fluorbenzylamin (97 mg, 0.61 mmol) und $Et_3N$ (0.24 ml, 1.8 mmol) gefolgt von T3P (0.6 ml, 1.8 mmol) bei 0°C. Die Reaktionsmischung wird unter Stickstoff für 4 Stunden bei Raumtemperatur gerührt. Nach beendeter Reaktion gibt man Wasser hinzu und extrahiert mit Dichlormethan erschöpfend. Nachfolgend werden die vereinigten organischen Phasen mit Wasser und gesättigter Natriumchlorid-lösung gewaschen und über Natriumsulfat getrocknet. Nach dem Abfiltrieren und Eindampfen wird der Rückstand an Kieselgel chromatographisch aufgereinigt. Man erhält das Produkt als farblosen Feststoff (150 mg, 63% yield);
Dünnschicht-Chromatogramm: Chloroform/Methanol = 9.5:0.5), $R_f$: 0.3 LC/MS: 388.1 (M+H bei 1, 388 min);
HPLC : Rt 5.12 min (HPLC purity 95.15 %, 95.46 %);
$^1$H NMR (DMSO-$d_6$, 400MHz): $\delta$ [ppm] 9.13 (t, J = 6.08 Hz, 1H), 7.70 - 7.68 (m, 2H), 7.43 (t, J = 8.64 Hz, 2H), 7.32 - 7.29 (m, 1 H), 7.24 - 7.20 (m, 2H), 7.09 (d, J = 9.80 Hz, 1 H), 4.42 - 4.34 (m, 2H), 3.95 - 3.89 (m, 2H), 2.61 - 2.58 (m, 1 H), 2.28 - 2.22 (m, 1H).

6.6 Synthese von 3-Amino-2-oxo-1-phenyl-pyrrolidin-3-carbonsäure-(3-chlor-5-fluor-benzyl)amid ("A151")

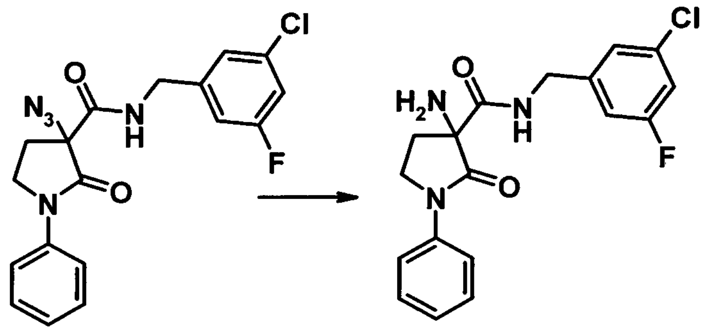

Zu einer Lösung von 3-Azido-2-oxo-1-phenyl-pyrrolidin-3-carbonsäure-(3-chlor-5-fluor-benzyl)amid (100 mg, 0.25 mmol) in 2 ml Methanol gibt man $Et_3N$ (0.1 ml, 0.75 mmol) gefolgt von Propandithiol (0.08 ml, 0.75 mmol). Der Ansatz wird für 12 Stunden bei RT gerührt. Nach beendeter Reaktion werden alle flüchtigen Bestandteile im Vakuum entfernt und der Rückstand an Kieselgel aufgereinigt.

Analog werden die nachstehenden Verbindungen erhalten

| Nr. | Struktur / Name | $^1$H NMR (400 MHz, DMSO-$d_6$) δ [ppm] | LC-MS; rt; [M+H$^+$]* |
|---|---|---|---|
| "A1" | 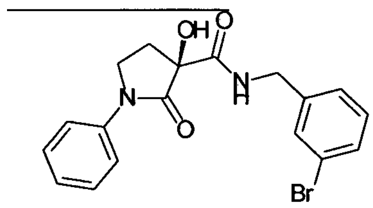 (S)-3-Hydroxy-2-oxo-1-phenyl-pyrrolidin-3-carbonsäure-3-brom-benzylamid | 8.65 (t, *J* = 6.1, 1H), 7.70 (d, *J* = 8.3, 2H), 7.47 (s, 1H), 7.40 (t, *J* = 7.8, 3H), 7.26 (d, *J* = 4.8, 2H), 7.17 (t, *J* = 7.3, 1H), 6.73 (s, 1H), 4.34 (dd, *J* = 15.2, 6.5, 1H), 4.24 (dd, *J* = 15.3, 6.0, 1H), 3.85 (t, *J* = 6.8, 2H), 2.62 - 2.52 (m, 1H), 2.11 (dt, *J* = 12.9, 7.8, 1H) | 4.005 min [389.0 + 392.0] % |
| "A2" | 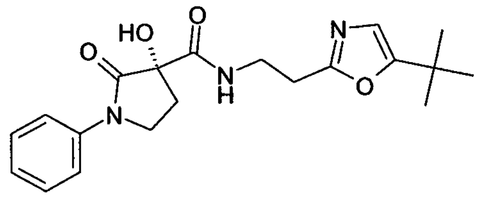 (S)-3-Hydroxy-2-oxo-1-phenyl-pyrrolidin-3-carbonsäure-[2-(5-tert.-butyl-oxazol-2-yl)-ethyl]-amid | 8.16-8.11 (m, 1H), 7.68 (d, *J* = 7.7, 2H), 7.43 - 7.33 (m, 2H), 7.18 (d, *J* = 7.5, 1H), 6.68 (s, 1H), 6.66 (s, 1H), 3.83 (m, 2H), 3.56 - 3.47 (m, 2H), 2.85 (m, 2H), 2.55 (m, 1H), 2.13 - 2.01 (m, 1H), 1.22 (s, 9H) | 3.513 min [372.3] % |
| "A3" | 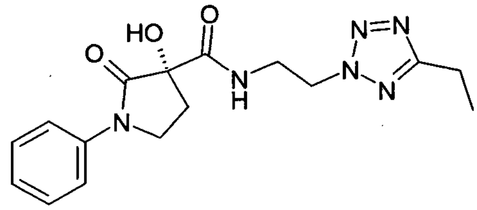 (S)-3-Hydroxy-2-oxo-1-phenyl-pyrrolidin-3-carbonsäure-[2-(5-ethyl-tetrazol-2-yl)-ethyl]-amid | 8.22 (t, *J* = 6.0, 1H), 7.68 (d, *J* = 1.1, 1H), 7.66 (d, *J* = 1.0, 1H), 7.42 - 7.36 (m, 2H), 7.21 - 7.12 (m, 1H), 6.65 (s, 1H), 4.78 - 4.60 (m, 2H), 3.80 (ddd, *J* = 15.3, 8.7, 5.2, 2H), 3.76-3.65 (m, 1H), 3.56 - 3.42 (m, 1H), 2.81 (q, *J* = 7.6, 2H), 2.42 (ddd, *J* = 12.8, 7.1, 3.8, 1H), 2.05 (dt, *J* = 12.9, 8.0, 1H), 1.25 (t, *J* = 7.6, 3H) | 2.679 min [345.3] % |
| "A4" | 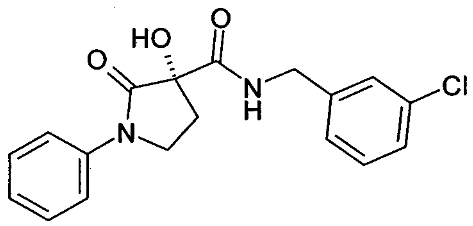 (S)-3-Hydroxy-2-oxo-1-phenyl-pyrrolidin-3-carbonsäure-3-chlor-benzylamid | 8.65 (t, *J* = 6.2, 1H), 7.69 (dd, *J* = 8.7, 1.1, 2H), 7.43 - 7.37 (m, 2H), 7.33 (t, *J* = 7.6, 2H), 7.27 (dd, *J* = 6.6, 2.1, 1H), 7.22 (d, *J* = 7.5, 1H), 7.18 (dd, *J* = 10.5, 4.2, 1H), 6.73 (s, 1H), 4.35 (dd, *J* = 15.2, 6.6, 1H), 4.25 (dd, *J* = 15.4, 6.1, 1H), 3.90 - 3.80 (m, 2H), 2.61 - 2.54 (m, 1H), 2.11 (dt, *J* = 13.0, 7.8, 1H) | 3.886 min [345.0] % |
| "A5" | 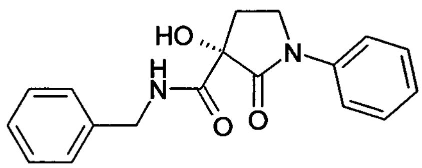 (S)-3-Hydroxy-2-oxo-1-phenyl-pyrrolidin-3-carbonsäure-benzylamid | 8.51 (t, *J* = 6.0, 1H), 7.69 (dd, *J* = 8.6, 0.9, 2H), 7.45 - 7.34 (m, 2H), 7.32 - 7.24 (m, 4H), 7.20 (dt, *J* = 14.8, 4.6, 2H), 6.68 (s, 1H), 4.33 (dd, *J* = 15.1, 6.5, 1H), 4.27 (dd, *J* = 15.1, 6.2, 1H), 3.92 - 3.74 (m, 2H), 2.63 - 2.52 (m, 2H), 2.11 (dt, *J* = 12.9, 7.9, 1H) | 3.309 min [311.3]% |

(fortgesetzt)

| Nr. | Struktur / Name | $^1H$ NMR (400 MHz, DMSO-$d_6$) δ [ppm] | LC-MS; rt; [M+H+]* |
|---|---|---|---|
| "A6" | 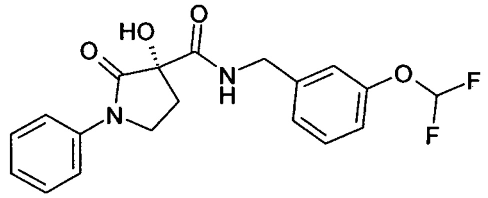 (S)-3-Hydroxy-2-oxo-1-phenyl-pyrrolidin-3-carbonsäure-3-difluormethoxy-benzylamid | 8.63 (t, J = 6.3, 1H), 7.77 - 7.60 (m, 2H), 7.45 - 7.30 (m, 3H), 7.22 - 7.10 (m, 3H), 7.07 (s, 1H), 7.02 (d, J = 9.8, 1H), 6.72 (s, 1H), 4.35 (dd, J = 15.4, 6.5, 1H), 4.27 (dd, J = 15.4, 6.2, 1H), 3.85 (dd, J = 8.7, 5.5, 2H), 2.64 - 2.52 (m, 1H), 2.20 - 2.05 (m, 1H) | 3.927 min [377.0] % |
| "A7" | 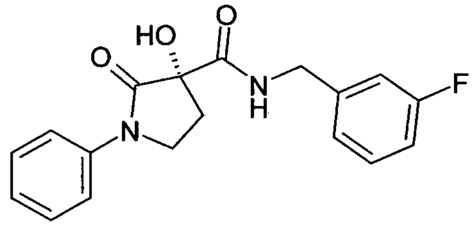 (S)-3-Hydroxy-2-oxo-1-phenyl-pyrrolidin-3-carbonsäure-3-fluor-benzylamid | 8.64 (t, J = 6.4, 1H), 7.69 (dd, J = 8.7, 1.1, 2H), 7.43 - 7.37 (m, 2H), 7.33 (td, J = 7.9, 6.1, 1H), 7.21 - 7.14 (m, 1H), 7.10 (d, J = 7.4, 2H), 7.08 - 6.99 (m, 2H), 6.73 (s, 1H), 4.37 (dd, J = 15.5, 6.6, 1H), 4.26 (dd, J = 15.4, 6.1, 1H), 3.92 - 3.75 (m, 2H), 2.63 - 2.54 (m, 1H), 2.12 (dt, J = 12.9, 7.7, 1H) | 3.522 min [329.0} % |
| "A8" | 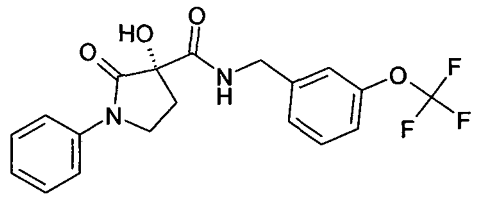 (S)-3-Hydroxy-2-oxo-1-phenyl-pyrrolidin-3-carbonsäure-3-trifluormethoxy-benzylamid | 8.70 (t, J = 6.4, 1H), 7.70 (d, J = 1.1, 1H), 7.68 (d, J = 1.0, 1H), 7.46 - 7.36 (m, 3H), 7.29 (d, J = 7.9, 1H), 7.25 (s, 1H), 7.22-7.14 (m, 2H), 6.73 (s, 1H), 4.38 (dd, J = 15.5, 6.6, 1H), 4.30 (dd, J = 15.5, 6.1, 1H), 3.90 - 3.82 (m, 2H), 2.56 (dt, J = 11.5, 5.6, 1H), 2.12 (dt, J = 12.9, 7.7, 1H) | 4.362 min [395.0] % |
| "A9" | 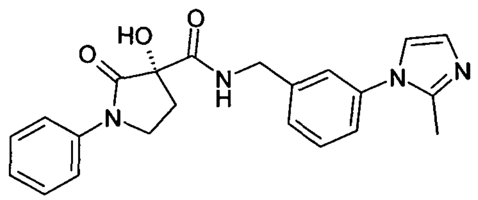 (S)-3-Hydroxy-2-oxo-1-phenyl-pyrrolidin-3-carbonsäure-3-(2-methyl-imidazol-1-yl)-benzylamid | 8.69 (t, J = 6.3, 1H), 7.68 (d, J = 7.9, 2H), 7.43 (dt, J = 22.3, 8.0, 3H), 7.30 (dd, J = 16.9, 8.3, 3H), 7.23 (d, J = 1.0, 1H), 7.17 (t, J = 7.3, 1H), 6.89 (d, J = 1.0, 1H), 6.73 (s, 1H), 4.44 (dd, J = 15.4, 6.7, 1H), 4.31 (dd, J = 15.4, 6.0, 1H), 3.91 - 3.77 (m, 2H), 2.63 - 2.54 (m, 1H), 2.25 (s, 3H), 2.11 (dt, J = 13.0, 7.8, 1H) | 2.357 min [391.3] % |

(fortgesetzt)

| Nr. | Struktur / Name | $^1$H NMR (400 MHz, DMSO-$d_6$) δ [ppm] | LC-MS; rt; [M+H+]* |
|---|---|---|---|
| "A10" | 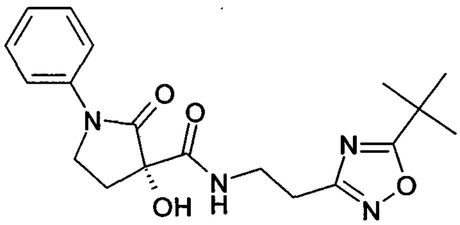 (S)-3-Hydroxy-2-oxo-1-phenyl-pyrrolidin-3-carbonsäure-[2-(5-tert.-butyl-[1,2,4]oxadiazol-3-yl)-ethyl]-amid | (400 MHz, $CDCl_3$) δ [ppm] 7.66 (dd, *J* = 8.7, 1.0, 2H), 7.61 (br. s, 1H), 7.45-7.36 (m, 2H), 7.21 (t, *J* = 7.4, 1H), 4.18 (td, *J* = 9.2, 7.0, 1H), 3.84 (td, *J* = 9.2, 1.2, 1H), 3.78 - 3.60 (m, 2H), 3.07 - 2.88 (m, 2H), 2.74 (ddd, *J* = 12.7, 6.9, 1.3, 1H), 2.26 (dt, *J* = 12.7, 9.3, 1H), 1.45 (s, 9H), 1.33 - 1.18 (m, 2H), 0.89 (s, 1H) | 3.549 min [373.3] % |
| "A11" | 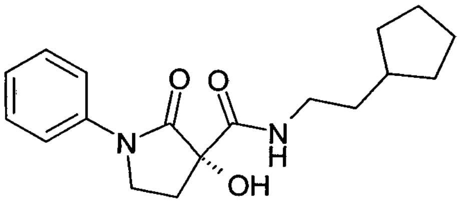 (S)-3-Hydroxy-2-oxo-1-phenyl-pyrrolidin-3-carbonsäure-(2-cyclopentyl-ethyl)-amid | 7.89 (s, 1H), 7.72 - 7.66 (m, 2H), 7.39 (t, *J* = 8.0, 2H), 7.17 (t, *J* = 7.4, 1H), 6.57 (s, 1H), 5.70 (s, 1H), 3.94 - 3.74 (m, 2H), 3.08 (m, 2H), 2.11 - 1.96 (m, 1H), 1.72 (m, 4H), 1.55 (m, 2H), 1.50 - 1.38 (m, 4H), 1.02 (m, 3H) | HPLC 5.529 min |
| "A12" | 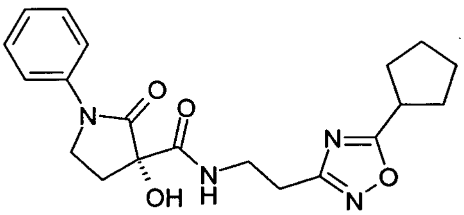 (S)-3-Hydroxy-2-oxo-1-phenyl-pyrrolidin-3-carbonsäure-[2-(5-cyclopentyl-[1,2,4]oxadiazol-3-yl)-ethyl]-amid | (400 MHz, $CDCl_3$) δ 7.66 (d, *J* = 7.7, 1H), 7.55 (s, 1H), 7.40 (t, *J* = 8.0, 1H), 7.21 (t, *J* = 7.4, 1H), 4.18 (td, *J* = 9.2, 7.0, 1H), 3.84 (td, *J* = 9.2, 1.2, 1H), 3.77 - 3.62 (m, 1H), 3.39 - 3.27 (m, 1H), 3.04 - 2.86 (m, 1H), 2.73 (ddd, *J* = 12.7, 6.9, 1.2, 1H), 2.26 (dt, *J* = 12.7, 9.3, 1H), 2.14 (m, 1H), 2.01 -1.89 (m, 1H), 1.88 -1.77 (m, 1H), 1.77 - 1.68 (m, 1H) | 3.706 min [385.3] % |
| "A13" | 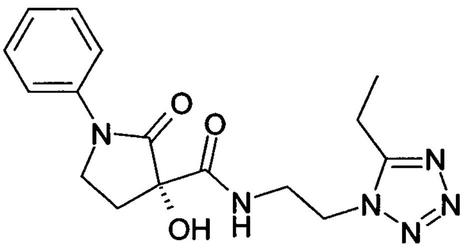 (S)-3-Hydroxy-2-oxo-1-phenyl-pyrrolidin-3-carbonsäure-[2-(5-ethyl-tetrazol-1-yl)-ethyl]-amid | 8.28 (t, *J* = 6.1, 1H), 7.67 (d, *J* = 7.7, 2H), 7.45-7.34 (m, 2H), 7.17 (t, *J* = 7.4, 1H), 6.66 (s, 1H), 4.52 - 4.33 (m, 2H), 3.89 - 3.72 (m, 2H), 3.60 (m, 1H), 3.41 (m, 1H), 2.86 (q, *J* = 7.5, 2H), 2.41 (ddd, *J* = 12.7, 7.0, 4.1, 1H), 2.04 (dt, *J* = 12.9, 8.1, 1H), 1.28 (t, *J* = 7.5, 3H) | 2.407 min [345.3] % |

(fortgesetzt)

| Nr. | Struktur / Name | $^1H$ NMR (400 MHz, DMSO-$d_6$) δ [ppm] | LC-MS; rt; $[M+H^+]^*$ |
|---|---|---|---|
| "A14" | 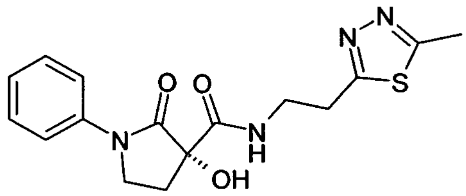<br>(S)-3-Hydroxy-2-oxo-1-phenyl-pyrrolidin-3-carbonsäure-[2-(5-methyl-[1,3,4]thiadiazol-2-yl)-ethyl]-amid | 8.23 (t, *J* = 6.2, 1H), 7.69 (d, *J* = 8.0, 2H), 7.40 (t, *J* = 7.8, 2H), 7.17 (t, *J* = 7.4, 1H), 6.66 (s, 1H), 3.82 (dd, *J* = 8.4, 5.2, 2H), 3.55 - 3.47 (m, 2H), 3.46 - 3.38 (m, 2H), 3.20 (t, *J* = 7.0, 2H), 2.66 (s, 3H), 2.08 (dt, *J* = 12.3, 8.0, 1H) | 2.329 min [347.0] % |
| "A15" | 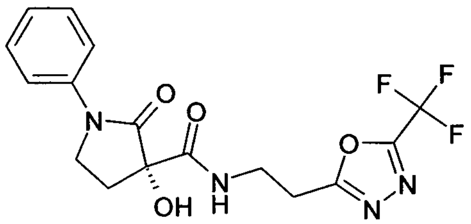<br>(S)-3-Hydroxy-2-oxo-1-phenyl-pyrrolidin-3-carbonsäure-[2-(5-trifluormethyl-[1,3,4]oxadiazol-2-yl)-ethyl]-amid | 8.32 (s, 1H), 7.67 (d, *J* = 7.9, 2H), 7.39 (t, *J* = 8.0, 2H), 7.17 (t, *J* = 7.4, 1H), 6.66 (s, 1H), 3.86 - 3.75 (m, 2H), 3.58 (td, *J* = 13.4, 6.8, 1H), 3.43 (dt, *J* = 13.4, 6.6, 2H), 3.23 - 3.04 (m, 2H), 2.47 - 2.40 (m, 1H), 2.06 (dt, *J* = 13.1, 7.7, 1H) | 3.342 min [385.3]% |
| "A16" | 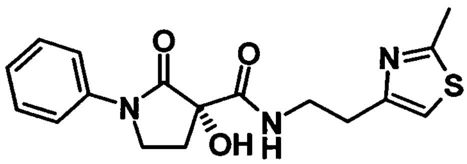<br>(S)-3-Hydroxy-2-oxo-1-phenyl-pyrrolidin-3-carbonsäure-[2-(2-methyl-thiazol-4-yl)-ethyl]-amid | 8.10 (t, *J* = 5.9, 1H), 7.69 (d, *J* = 7.8, 2H), 7.40 (t, *J* = 8.0, 2H), 7.17 (t, *J* = 7.4, 1H), 7.14 (s, 1H), 6.62 (s, 1H), 3.83 (dd, *J* = 12.2, 5.5, 2H), 3.50 - 3.35 (m, 2H), 2.82 (t, *J* = 7.2, 2H), 2.61 (s, 3H), 2.53 (m, 1H), 2.07 (dt, *J* = 12.9, 8.0, 1H) | HPLC 7.206 min |
| "A17" | 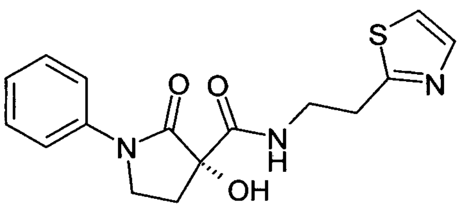<br>(S)-3-Hydroxy-2-oxo-1-phenyl-pyrrolidin-3-carbonsäure-(2-thiazol-2-yl-ethyl)-amid | 8.20 (t, *J* = 6.0, 1H), 7.71 (d, *J* = 3.3, 1H), 7.69 (d, *J* = 7.8, 2H), 7.59 (d, *J* = 3.3, 1H), 7.45 - 7.36 (m, 2H), 7.17 (t, *J* = 7.4, 1H), 6.65 (s, 1H), 3.83 (t, *J* = 6.3, 2H), 3.52 (dt, *J* = 13.7, 6.8, 1H), 3.47-3.35 (m, 2H), 3.15 (t, *J* = 7.1, 2H), 2.56 - 2.51 (m, 1H), 2.07 (dt, *J* = 12.9, 7.9, 1H) | 2.143 min [332.0] % |
| "A18" | 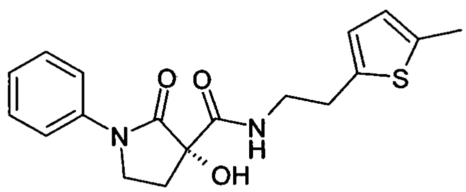<br>(S)-3-Hydroxy-2-oxo-1-phenyl-pyrrolidin-3-carbonsäure-[2-(5-methyl-thiophen-2-yl)-ethyl]-amid | 8.07 (t, *J* = 5.9, 1H), 7.73 - 7.65 (m, 2H), 7.40 (dd, J = 10.8, 5.3, 2H), 7.17 (t, *J* = 7.4, 1H), 6.64 (d, *J* = 3.3, 1H), 6.63 (s, 1H), 6.59 (dd, *J* = 3.3, 1.1, 1H), 3.91 - 3.75 (m, 2H), 3.30 - 3.33 (m, 1H), 3.15 (m, 1H), 2.85 (t, *J* = 7.4, 2H), 2.53 (m, 1H), 2.34 (s, 3H), 2.07 (dt, *J* = 12.9, 8.0, 1H) | 3.887 min [345.3] % |

(fortgesetzt)

| Nr. | Struktur / Name | $^1$H NMR (400 MHz, DMSO-$d_6$) δ [ppm] | LC-MS; rt; [M+H$^+$]* |
|---|---|---|---|
| "A19" | 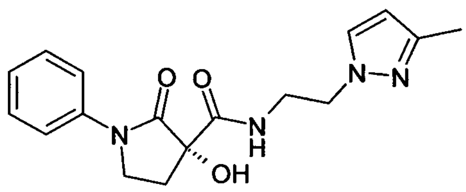 (S)-3-Hydroxy-2-oxo-1-phenyl-pyrrolidin-3-carbonsäure-[2-(3-methyl-pyrazol-1-yl)-ethyl]-amid | 8.10 (t, *J* = 5.7, 1H), 7.79 - 7.65 (m, 2H), 7.53 (d, *J* = 2.1, 1H), 7.40 (t, *J* = 8.0, 2H), 7.17 (t, *J* = 7.4, 1H), 6.65 (s, 1H), 5.97 (d, *J* = 2.1, 1H), 4.09 (t, *J* = 6.4, 2H), 3.82 (dd, *J* = 8.5, 5.4, 2H), 3.48 (m, 1H), 3.40 (m, 1H), 2.52 (m, 1H), 2.14 (s, 3H), 2.06 (dt, *J* = 12.9, 7.9, 1H) | HPLC: 6.550 min |
| "A20" | 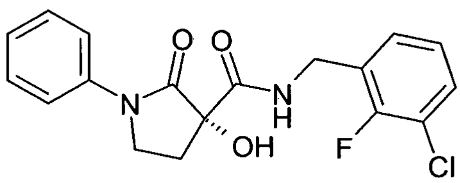 (S)-3-Hydroxy-2-oxo-1-phenyl-pyrrolidin-3-carbonsäure-3-chlor-2-fluor-benzylamid | 8.64 (t, *J* = 6.0, 1H), 7.69 (dd, *J* = 8.7, 1.0, 1H), 7.45 (td, *J* = 7.9, 1.6, 1H), 7.43 - 7.37 (m, 1H), 7.31 (td, *J* = 7.5, 1.5, 1H), 7.18 (td, *J* = 7.7, 1.8, 1H), 6.75 (s, 1H), 4.41 (dd, *J* = 15.6, 6.0, 1H), 4.33 (dd, *J* = 15.5, 6.0, 1H), 3.90 - 3.80 (m, 1H), 2.62 - 2.55 (m, 1H), 2.12 (dt, *J* = 12.9, 8.0, 1H) | 4.002 min [363.0] % |
| "A21" | 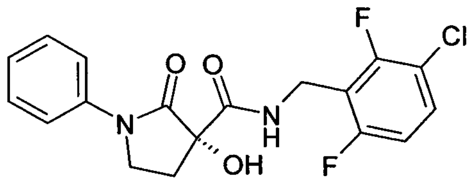 (S)-3-Hydroxy-2-oxo-1-phenyl-pyrrolidin-3-carbonsäure-3-chlor-2,6-difluor-benzylamid | (400 MHz, $CDCl_3$) δ [ppm] 7.68 - 7.59 (m, 2H), 7.42 - 7.36 (m, 2H), 7.36 - 7.29 (m, 2H), 7.24 - 7.18 (m, 1H), 6.88 (td, *J* = 8.8, 1.8, 1H), 4.65 (dd, *J* = 14.6, 6.5, 1H), 4.50 (dd, *J* = 14.6, 5.6, 1H), 4.20 (td, *J* = 9.2, 6.9, 1H), 3.81 (td, *J* = 9.2, 1.2, 1H), 2.72 (ddd, *J* = 12.7, 6.9, 1.2, 1H), 2.25 (dt, *J* = 12.7, 9.3, 1H) | |
| "A22" | 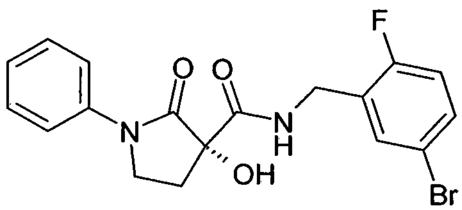 (S)-3-Hydroxy-2-oxo-1-phenyl-pyrrolidin-3-carbonsäure-5-brom-2-fluor-benzylamid | 8.66 (t, *J* = 6.3, 1H), 7.78 - 7.67 (m, 2H), 7.53-7.44 (m, 2H), 7.40 (t, *J* = 8.0, 2H), 7.16 (dd, *J* = 16.7, 8.2, 2H), 6.78 (s, 1H), 4.39 (dd, *J* = 15.8, 6.6, 1H), 4.26 (dd, *J* = 15.9, 6.0, 1H), 3.86 (dd, *J* = 8.0, 5.1, 2H), 2.64 - 2.54 (m, 1H), 2.13 (dt, *J* = 13.0, 7.6, 1H) | HPLC 7.301 min |
| "A23" | 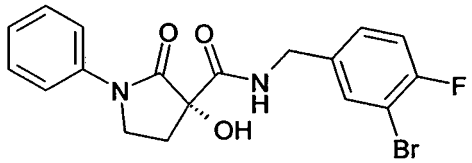 (S)-3-Hydroxy-2-oxo-1-phenyl-pyrrolidin-3-carbonsäure-3-brom-4-fluor-benzylamid | 8.67 (t, *J* = 6.3, 1H), 7.69 (d, *J* = 8.4, 2H), 7.59 (d, *J* = 6.9, 1H), 7.40 (t, *J* = 7.8, 2H), 7.31 (d, *J* = 7.3, 2H), 7.18 (t, *J* = 7.4, 1H), 6.72 (s, 1H), 4.33 (dd, *J* = 15.1, 6.7, 1H), 4.22 (dd, *J* = 15.2, 6.0, 1H), 3.85 (t, *J* = 6.8, 2H), 2.62 - 2.52 (m, 1H), 2.11 (dt, *J* = 13.3, 7.8, 1H) | 4.094 min [409.0 + 410.0] % |

(fortgesetzt)

| Nr. | Struktur / Name | $^1$H NMR (400 MHz, DMSO-$d_6$) δ [ppm] | LC-MS; rt; [M+H$^+$]* |
|---|---|---|---|
| "A24" | 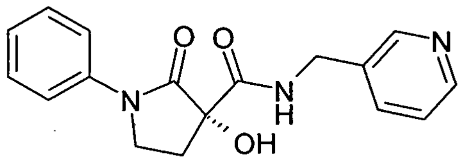<br>(S)-3-Hydroxy-2-oxo-1-phenyl-pyrrolidin-3-carbonsäure-(pyridin-3-ylmethyl)-amid | 8.67 (t, *J* = 6.3, 1H), 8.48 (d, *J* = 1.8, 1H), 8.42 (dd, *J* = 4.7, 1.5, 1H), 7.69 (d, *J* = 7.9, 2H), 7.65 (dd, *J* = 12.9, 5.0, 1H), 7.46 - 7.36 (m, 2H), 7.32 (dd, *J* = 7.8, 4.8, 1H), 7.17 (t, *J* = 7.4, 1H), 7.00 (s, 1H), 6.71 (s, 1H), 4.37 (dd, *J* = 15.2, 6.5, 1H), 4.28 (dd, *J* = 15.2, 6.1, 1H), 3.94-3.78 (m, 2H), 2.63 - 2.53 (m, 1H), 2.11 (dt, *J* = 12.9, 7.8, 1H) | 1.872 min [312.3] % |
| "A25" | 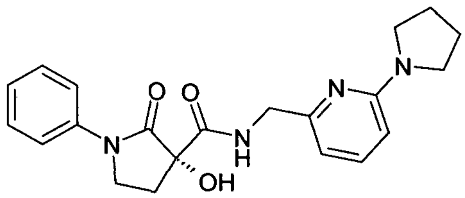<br>(S)-3-Hydroxy-2-oxo-1-phenyl-pyrrolidin-3-carbonsäure-(6-pyrrolidin-1-yl-pyridin-2-ylmethyl)-amid | 8.44 (t, *J* = 5.6, 1H), 7.70 (d, *J* = 8.1, 2H), 7.41 (dd, *J* = 15.5, 7.4, 3H), 7.17 (t, *J* = 7.4, 1H), 6.75 (s, 1H), 6.47 (d, *J* = 7.3, 1H), 6.28 (d, *J* = 8.4, 1H), 4.31 - 4.17 (m, 2H), 3.93 - 3.77 (m, 2H), 3.37 (m, 4H), 3.03 - 2.95 (m, 1H), 2.64 -2.57 (m, 1H), 2.12 (dt, *J* = 13.0,8.0, 1H), 2.01 - 1.83 (m, 4H), 1.09 - 0.89 (m, 1H) | HPLC 9.533 |
| "A26" | 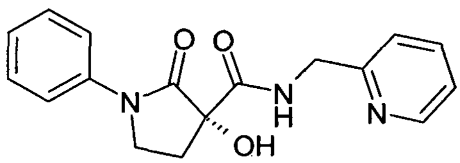<br>(S)-3-Hydroxy-2-oxo-1-phenyl-pyrrolidin-3-carbonsäure-(pyridin-2-ylmethyl)-amid | 8.63 (t, *J* = 5.2, 1H), 8.51 (d, *J* = 3.8, 1 H), 7.77 (t, *J* = 7.5, 1H), 7.71 (d, *J* = 8.5, 1H), 7.42 (t, *J* = 7.6, 2H), 7.33 (d, *J* = 7.7, 1H), 7.30 - 7.24 (m, 1H), 7.19 (t, *J* = 7.2, 1H), 6.79 (s, 1H), 4.46 (dd, *J* = 16.5, 6.4, 1H), 4.38 (dd, *J* = 16.1, 6.0, 1H), 3.88 (m, 2H), 2.62 (m, 1H), 2.16 (m, 1H) | 1.959 min [312.3] |
| "A27" | 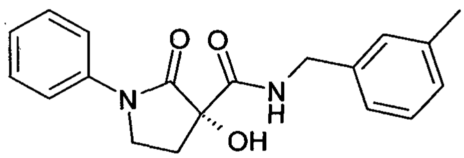<br>(S)-3-Hydroxy-2-oxo-1-phenyl-pyrrolidin-3-carbonsäure-3-methylbenzylamid | 8.46 (t, *J* = 6.2, 1H), 7.70 (d, *J* = 7.8, 2H), 7.46 - 7.35 (m, 2H), 7.17 (t, *J* = 7.3, 2H), 7.07 (d, *J* = 4.4, 2H), 7.02 (d, *J* = 7.8, 1H), 6.68 (s, 1H), 4.29 (dd, *J* = 15.0, 6.5, 1H), 4.23 (dd, *J* = 15.1, 6.2, 1H), 3.96-3.74 (m, 2H), 2.64 - 2.52 (m, 1H), 2.26 (s, 3H), 2.11 (dt, *J* = 12.8, 7.8, 1H) | 3.763 min [325.3] |

(fortgesetzt)

| Nr. | Struktur / Name | $^1$H NMR (400 MHz, DMSO-$d_6$) δ [ppm] | LC-MS; rt; [M+H$^+$]* |
|---|---|---|---|
| "A28" | 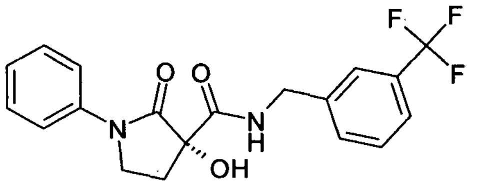 (S)-3-Hydroxy-2-oxo-1-phenyl-pyrrolidin-3-carbonsäure-3-trifluormethyl-benzylamid | 8.74 (t, *J* = 6.3, 1H), 7.69 (d, *J* = 7.8, 2H), 7.63 (s, 1H), 7.54 (dd, *J* = 13.3, 6.1, 3H), 7.40 (t, *J* = 8.0, 2H), 7.17 (t, *J* = 7.4, 1H), 6.74 (s, 1H), 4.43 (dd, *J* = 15.5, 6.5, 1H), 4.33 (dd, *J* = 15.4, 6.1, 1H), 3.94-3.76 (m, 2H), 2.57 (dt, *J* = 11.8, 5.7, 1H), 2.12 (dt, *J* = 13.0, 7.8, 1H) | 4.260 min [379.0] % |
| "A29" | 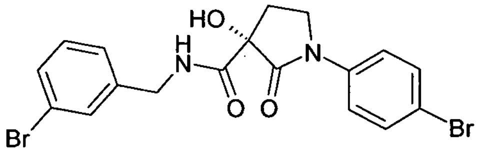 (S)-1-(4-Brom-phenyl)-3-hydroxy-2-oxo-pyrrolidin-3-carbonsäure-3-brom-benzylamid | (400 MHz, $CDCl_3$) δ [ppm] 7.58 (d, *J* = 9.1, 1H), 7.52 (d, *J* = 9.1, 1H), 7.42 (d, *J* = 4.6, 1H), 7.22 (dd, *J* = 4.8, 1.4, 1H), 4.50 - 4.36 (m, 1H), 4.20 (dd, *J* = 16.1, 9.2, 1H), 3.83 (t, *J* = 9.2, 1H), 3.66 (s, 1H), 2.78 (dd, *J* = 12.8, 6.9, 1H), 2.29 (dt, *J* = 12.8, 9.4, 1H) | 4.583 min [467.0 + 469.0 + 472.0] % |
| "A30" | 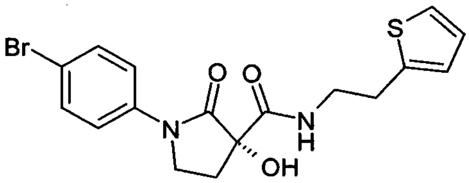 (S)-1-(4-Bromo-phenyl)-3-hydroxy-2-oxo-pyrrolidine-3-carboxylic acid (2-thiophen-2-yl-ethyl)-amide | 8.11 (s, 1H), 7.68 (d, *J* = 9.0, 2H), 7.58 (d, *J* = 9.0, 2H), 7.32 (d, *J* = 5.1, 1H), 6.98 - 6.90 (m, 1H), 6.89 (s, 1H), 6.66 (s, 1H), 3.88 - 3.76 (m, 2H), 3.36 (d, *J* = 5.9, 2H), 2.95 (t, *J* = 7.3, 2H), 2.53 (s, 1H), 2.07 (dt, *J* = 12.4, 7.6, 2H) | 4.127 min [409.0 + 410.0] % |
| "A31" | 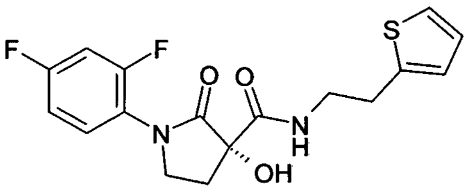 (S)-1-(2,4-Difluor-phenyl)-3-hydroxy-2-oxo-pyrrolidin-3-carbonsäure-(2-thiophen-2-yl-ethyl)-amid | 8.08 (t, *J* = 6.0, 1H), 7.50 - 7.41 (m, 1H), 7.41 - 7.36 (m, 1H), 7.33 (dd, *J* = 5.1, 1.2, 1H), 7.20-7.13 (m, 1H), 6.94 (dd, *J* = 5.1, 3.4, 1H), 6.89 (d, *J* = 2.4, 1H), 6.66 (s, 1H), 3.81 - 3.65 (m, 2H), 3.49 - 3.35 (m, 2H), 2.96 (t, *J* = 7.4, 2H), 2.57 - 2.52 (m, 1H), 2.13 (ddd, *J* = 12.9, 8.4, 7.3, 1H) | 3.539 min [367.0] % |
| ' i432" | 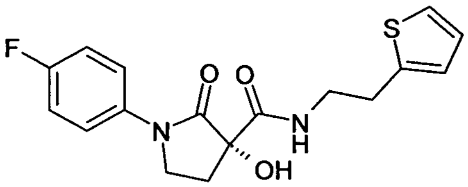 (S)-1-(4-Fluor-phenyl)-3-hydroxy-2-oxo-pyrrolidin-3-carbonsäure-(2-thiophen-2-yl-ethyl)-amid | (400 MHz, $CDCl_3$) δ [ppm] 7.67 - 7.59 (m, 1H), 7.18 (dd, *J* = 5.1, 1.1, 1H), 7.10 (dd, *J* = 11.7, 5.7, 1H), 6.96 (dd, *J* = 5.1, 3.4, 0H), 6.87 (d, *J* = 3.4, 1H), 4.17 (td, *J* = 9.2, 6.9, 1H), 3.80 (t, *J* = 9.2, 1H), 3.55 (dd, *J* = 13.1, 6.7, 2H), 3.07 (t, *J* = 6.7, 1H), 2.71 (dd, *J* = 13.4, 6.3, 1H), 2.25 (dt, *J* = 12.7, 9.3, 1H) | 3.623 min [349.0] % |

(fortgesetzt)

| Nr. | Struktur / Name | $^1H$ NMR (400 MHz, DMSO-$d_6$) δ [ppm] | LC-MS; rt; [M+H$^+$]* |
|---|---|---|---|
| "A33" | 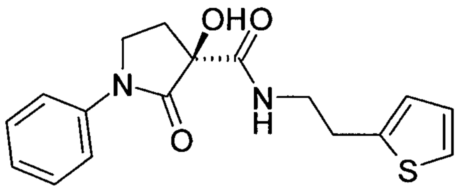 (S)-3-Hydroxy-2-oxo-1-phenyl-pyrrolidin-3-carbonsäure-(2-thiophen-2-yl-ethyl)-amid | | 1) HPLC 7.19 min |
| "A34" | 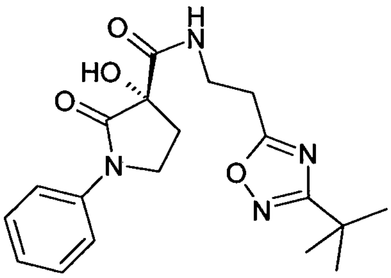 (S)-3-Hydroxy-2-oxo-1-phenyl-pyrrolidin-3-carbonsäure-[2-(3-tert.-butyl-[1,2,4]oxadiazol-5-yl)-ethyl]-amid | 8.24 (t, *J* = 6.1, 1H), 7.68 (d, *J* = 7.8, 2H), 7.39 (t, *J* = 8.0, 2H), 7.17 (t, *J* = 7.4, 1H), 6.66 (s, 1H), 3.82 (dd, *J* = 7.8, 5.7, 2H), 3.64 - 3.49 (m, 1H), 3.41 (td, *J* = 13.0, 6.6, 2H), 3.12 - 3.01 (m, 2H), 2.06 (dt, *J* = 12.8, 7.9, 1H), 1.28 (s, 9H) | 3.564 min [373.3] % |
| "A35" | 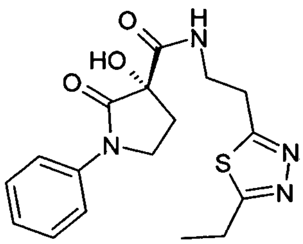 (S)-3-Hydroxy-2-oxo-1-phenyl-pyrrolidin-3-carbonsäure-[2-(5-ethyl-[1,3,4]thiadiazol-2-yl)-ethyl]-amid | 8.23 (t, *J* = 6.0, 1H), 7.70 (d, *J* = 1.1, 1H), 7.68 (d, *J* = 0.8, 1H), 7.46 - 7.35 (m, 2H), 7.17 (t, *J* = 7.4, 1 H), 6.66 (s, 1H), 3.90 - 3.74 (m, 2H), 3.51 (dt, *J* = 13.4, 6.7, 1H), 3.46 - 3.36 (m, 1H), 3.21 (t, *J* = 6.9, 2H), 3.02 (q, *J* = 7.5, 2H), 2.07 (dt, *J* = 12.9, 8.0, 1H), 1.27 (t, *J* = 7.5, 3H) | 2.631 min [361.0] % |
| "A36" | 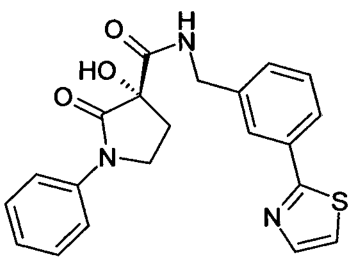 (S)-3-Hydroxy-2-oxo-1-phenyl-pyrrolidin-3-carbonsäure-3-thiazol-2-yl-benzylamid | 8.68 (t, *J* = 6.4, 1H), 7.92 (d, *J* = 3.2, 1H), 7.85 (s, 1H), 7.81 (dt, *J* = 7.6, 1.4, 1H), 7.78 (d, *J* = 3.2, 1H), 7.70 (dd, *J* = 8.7, 1.0, 2H), 7.47 - 7.35 (m, 4H), 7.24 - 7.13 (m, 1H), 6.72 (s, 1H), 4.45 - 4.29 (m, 2H), 3.95 - 3.72 (m, 2H), 2.64 2.55 (m, 1H), 2.13 (dt, *J* = 12.9, 7.8, 1H) | 3.457 min [394.0] % |

(fortgesetzt)

| Nr. | Struktur / Name | $^1$H NMR (400 MHz, DMSO-$d_6$) $\delta$ [ppm] | LC-MS; rt; [M+H$^+$]* |
|---|---|---|---|
| "A37" | 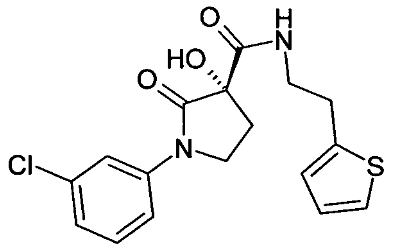 (S)-1-(3-Chlor-phenyl)-3-hydroxy-2-oxo-pyrrolidin-3-carbonsäure-(2-thiophen-2-yl-ethyl)-amid | 8.13 (t, *J* = 5.8, 1H), 7.89 (t, *J* = 2.0, 1 H), 7.61 (d, *J* = 8.2, 1H), 7.43 (t, *J* = 8.1, 1H), 7.33 (dd, *J* = 5.0, 1.0, 1H), 7.25 (d, *J* = 8.0, 1H), 6.94 (dd, *J* = 5.0, 3.5, 1H), 6.88 (d, *J* = 3.5, 1H), 6.69 (s, 1H), 3.91 - 3.75 (m, 2H), 3.46 - 3.33 (m, 2H), 2.95 (t, *J* = 7.4, 2H), 2.08 (dt, *J* = 13.2, 8.2, 1H) | |
| "A38" | 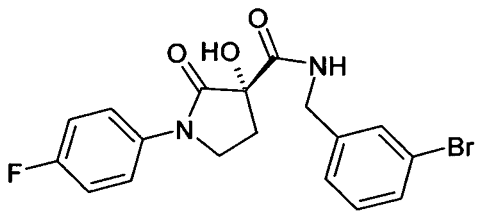 (S)-1-(4-Fluor-phenyl)-3-hydroxy-2-oxo-pyrrolidin-3-carbonsäure-3-brom-benzylamid | 8.66 (t, *J* = 6.4, 1H), 7.77 - 7.69 (m, 2H), 7.46 (s, 1H), 7.44 - 7.36 (m, 1H), 7.29 - 7.21 (m, 4H), 6.73 (s, 1H), 4.34 (dd, *J* = 15.3, 6.6, 1H), 4.24 (dd, *J* = 15.4, 6.1, 1H), 3.84 (t, *J* = 6.9, 2H), 2.61 - 2.52 (m, 2H), 2.11 (dt, *J* = 12.8, 7.7, 1H) | 4.130 min [409.0 + 410.0] % |
| "A39" | 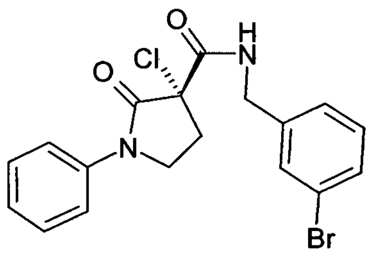 (S)-3-Chlor-2-oxo-1-phenyl-pyrrolidin-3-carbonsäure-3-brom-benzylamid | 9.03 (t, *J* = 6.0, 1H), 7.68 (s, 1H), 7.66 (s, 1H), 7.49 (s, 1H), 7.46 - 7.39 (m, 3H), 7.33 - 7.27 (m, 2H), 7.24 (dd, *J* = 13.6, 6.2, 1H), 4.41 (dd, *J* = 15.4, 6.3, 1H), 4.33 (dd, *J* = 15.4, 6.0, 1H), 4.07 - 3.87 (m, 2H), 3.04 (dt, *J* = 14.8, 7.6, 1H), 2.57 - 2.51 (m, 1H) | 5.006 min [407.0 + 409.0] |
| "A40" | 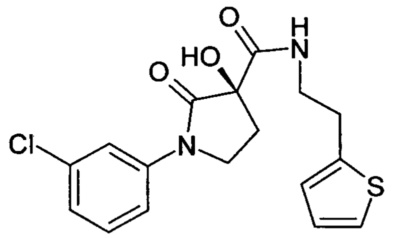 (R)-1-(3-Chlor-phenyl)-3-hydroxy-2-oxo-pyrrolidin-3-carbonsäure-(2-thiophen-2-yl-ethyl)-amid | 8.13 (t, *J* = 5.9, 1H), 7.89 (t, *J* = 2.0, 1H), 7.61 (ddd, *J* = 8.3, 2.1, 0.7, 1H), 7.43 (t, *J* = 8.2, 1H), 7.33 (dd, *J* = 5.1, 1.2, 1H), 7.27 - 7.21 (m, 1H), 6.94 (dd, *J* = 5.1, 3.4, 1H), 6.88 (d, *J* = 3.4, 1H), 6.69 (s, 1H), 3.89 - 3.80 (m, 2H), 3.45 - 3.34 (m, 2H), 2.95 (t, *J* = 7.2, 2H), 2.08 (dt, *J* = 13.0, 8.0, 2H) | 4.045 min [365.0]% |

(fortgesetzt)

| Nr. | Struktur / Name | $^1H$ NMR (400 MHz, DMSO-$d_6$) δ [ppm] | LC-MS; rt; [M+H+]* |
|---|---|---|---|
| "A41" | 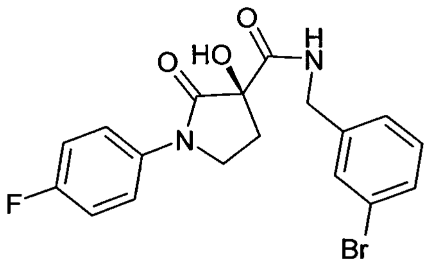 (R)-1-(4-Fluor-phenyl)-3-hydroxy-2-oxo-pyrrolidin-3-carbonsäure-3-brom-benzylamid | 8.66 (t, *J* = 6.3, 1H), 7.81 - 7.65 (m, 2H), 7.46 (s, 1H), 7.41 (td, *J* = 4.8, 2.0, 1H), 7.31 - 7.21 (m, 4H), 6.74 (s, 1H), 4.34 (dd, *J* = 15.3, 6.6, 1H), 4.24 (dd, *J* = 15.3, 6.1, 1H), 3.84 (dd, *J* = 8.2, 5.5, 2H), 2.56 (dt, *J* = 11.6, 5.5, 1H), 2.11 (dt, *J* = 13.0, 7.7, 1H) | 4.133 min [409.0 + 410.0] % |
| "A42" | 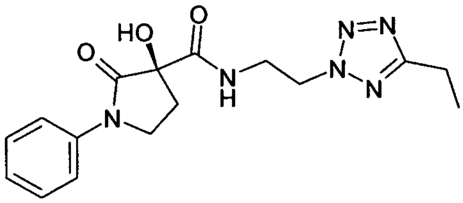 (R)-3-Hydroxy-2-oxo-1-phenyl-pyrrolidin-3-carbonsäure-[2-(5-ethyl-tetrazol-2-yl)-ethyl]-amid | 8.22 (t, *J* = 6.0, 1H), 7.67 (dd, *J* = 8.7, 1.0, 2H), 7.44 - 7.34 (m, 2H), 7.17 (dd, *J* = 10.5, 4.2, 1H), 6.65 (s, 1H), 4.78 - 4.57 (m, 2H), 3.84 - 3.74 (m, 2H), 3.74 - 3.66 (m, 1H), 3.54 - 3.39 (m, 1H), 2.81 (q, *J* = 7.6, 2H), 2.42 (ddd, *J* = 12.8, 7.1, 3.7, 1H), 2.05 (dt, *J* = 12.9, 8.1, 1H), 1.25 (t, *J* = 7.6, 3H) | |
| "A43" | 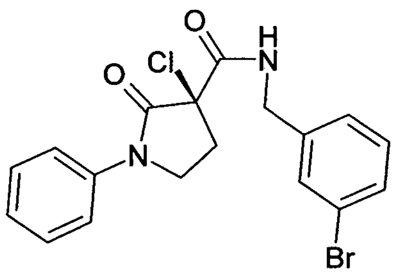 (R)-3-Chlor-2-oxo-1-phenyl-pyrrolidin-3-carbonsäure-3-brom-benzylamid | 9.03 (t, *J* = 6.1, 1H), 7.68 (d, *J* = 1.1, 1H), 7.66 (d, *J* = 0.8, 1H), 7.49 (s, 1 H), 7.48 - 7.40 (m, 3H), 7.29 (dd, *J* = 4.1, 1.6, 2H), 7.26 - 7.19 (m, 1H), 4.41 (dd, *J* = 15.4, 6.3, 1H), 4.33 (dd, *J* = 15.4, 6.0, 1H), 4.06 - 3.90 (m, 2H), 3.04 (dt, *J* = 14.8, 7.5, 1H), 2.59 - 2.51 (m, 1H) | 5.002 min [407.0 + 409.0] % |
| "A44" | 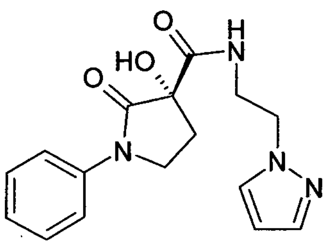 (S)-3-Hydroxy-2-oxo-1-phenyl-pyrrolidin-3-carbonsäure-(2-pyrazol-1-yl-ethyl)-amid | 8.11 (t, *J* = 5.9, 1H), 7.74 - 7.63 (m, 3H), 7.44 (d, *J* = 1.4, 1H), 7.43-7.37 (m, 2H), 7.18 (dd, *J* = 10.6, 4.2, 1H), 6.65 (s, 1H), 6.21 (t, *J* = 2.0, 1H), 4.20 (t, J = 6.4, 2H), 3.82 (dd, *J* = 8.4, 5.4, 2H), 3.52 (m, , 1H), 3.42 (m, 1H), 2.06 (m, 1H) | 2.376 min [315.0] % |

(fortgesetzt)

| Nr. | Struktur / Name | $^1$H NMR (400 MHz, DMSO-$d_6$) δ [ppm] | LC-MS; rt; [M+H$^+$]* |
|---|---|---|---|
| "A45" | 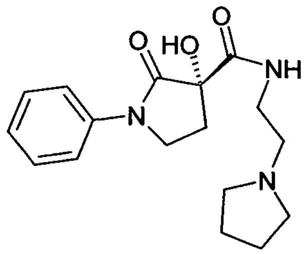 (S)-3-Hydroxy-2-oxo-1-phenyl-pyrrolidin-3-carbonsäure-(2-pyrrolidin-1-yl-ethyl)-amid | 7.85 (s, 1H), 7.68 (dd, *J* = 8.7, 1.0, 2H), 7.44 - 7.34 (m, 2H), 7.17 (dd, *J* = 10.6, 4.2, 1H), 6.65 (s, 1H), 3.93 - 3.76 (m, 2H), 3.20 (qd, *J* = 13.1, 6.3, 2H), 2.52 (m, 3H), 2.46 (m, 4H), 2.07 (dt, *J* = 12.9, 8.0, 1H), 1.67 (m, 4H) | 1.990 min [318.3] % |
| "A46" | 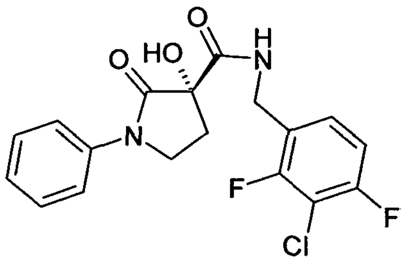 (S)-3-Hydroxy-2-oxo-1-phenyl-pyrrolidin-3-carbonsäure-3-chlor-2,4-difluor-benzylamid | 8.66 (t, *J* = 6.2, 1H), 7.69 (dd, *J* = 8.7, 1.0, 2H), 7.43 - 7.33 (m, 3H), 7.30 (td, *J* = 8.8, 1.5, 1H), 7.18 (t, *J* = 7.4, 1H), 6.74 (s, 1H), 4.38 (dd, *J* = 15.3, 6.2, 1H), 4.30 (dd, *J* = 15.6, 6.2, 1H), 3.90 - 3.80 (m, 2H), 2.63 - 2.52 (m, 1H), 2.11 (dt, *J* = 13.0, 7.7, 1H) | 4.169 min [381.0] % |
| "A47" | 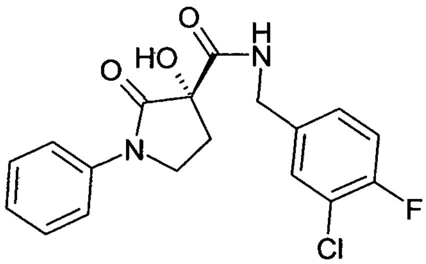 (S)-3-Hydroxy-2-oxo-1-phenyl-pyrrolidin-3-carbonsäure-3-chlor-4-fluor-benzylamid | 8.68 (t, *J* = 6.4, 1H), 7.69 (dd, *J* = 8.7, 1.0, 2H), 7.46 (dd, *J* = 7.3, 2.1, 1H), 7.43 - 7.36 (m, 2H), 7.34 (d, *J* = 9.3, 1H), 7.30 - 7.22 (m, 1H), 7.17 (t, *J* = 7.4, 1H), 6.73 (s, 1H), 4.33 (dd, *J* = 15.2, 6.8, 1H), 4.22 (dd, *J* = 15.3, 6.1, 1H), 3.85 (dd, *J* = 8.6, 5.6, 2H), 2.63 - 2.52 (m, 1H), 2.11 (dt, *J* = 13.0, 7.6, 1H) | 4.128 min [363.0] % |
| "A48" | 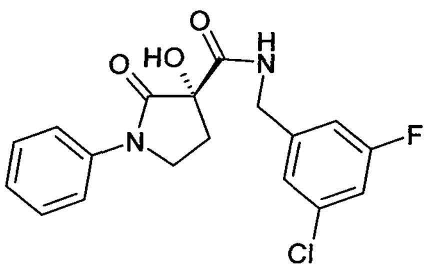 (S)-3-Hydroxy-2-oxo-1-phenyl-pyrrolidin-3-carbonsäure-3-chlor-5-fluor-benzylamid | 8.72 (t, *J* = 6.4, 1H), 7.69 (dd, *J* = 8.7, 1.0, 2H), 7.45 - 7.33 (m, 2H), 7.27 (dt, *J* = 8.8, 2.1, 1H), 7.18 (dd, *J* = 13.0, 4.5, 2H), 7.10 (d, *J* = 9.0, 1H), 6.76 (s, 1H), 4.38 (dd, *J* = 15.7, 6.6, 1H), 4.24 (dd, *J* = 15.9, 6.0, 1H), 3.89 - 3.82 (m, 2H), 2.62 - 2.54 (m, 1H), 2.12 (dt, *J* = 12.9, 7.6, 1H) | 4.052 min [363.0] % |

(fortgesetzt)

| Nr. | Struktur / Name | $^1$H NMR (400 MHz, DMSO-$d_6$) δ [ppm] | LC-MS; rt; [M+H$^+$]* |
|---|---|---|---|
| "A49" | 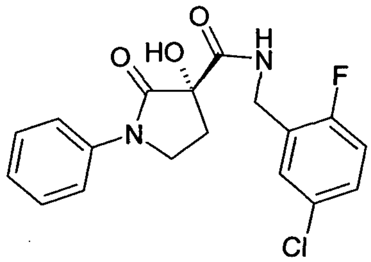 (S)-3-Hydroxy-2-oxo-1-phenyl-pyrrolidin-3-carbonsäure-5-chlor-2-fluor-benzylamid | 8.67 (t, *J* = 6.3, 1H), 7.70 (dd, *J* = 8.7, 1.1, 2H), 7.44 - 7.31 (m, 4H), 7.26 - 7.14 (m, 2H), 6.78 (s, 1H), 4.39 (dd, *J* = 15.7, 6.6, 1H), 4.26 (dd, *J* = 15.7, 5.8, 1H), 3.91 - 3.81 (m, 2H), 2.63 - 2.54 (m, 1H), 2.13 (dt, *J* = 13.0, 7.7, 1H) | 3.985 min [363.0] % |
| "A50" | 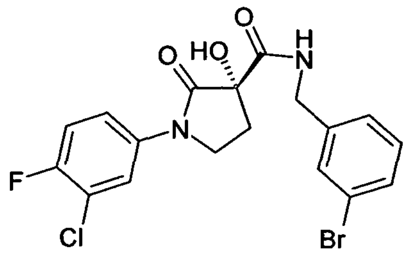 (S)-1-(3-Chlor-4-fluor-phenyl)-3-hydroxy-2-oxo-pyrrolidin-3-carbonsäure-3-brom-benzylamid | 8.68 (t, *J* = 6.4, 1H), 8.00 (dd, *J* = 6.7, 2.7, 1H), 7.68 (ddd, *J* = 9.1, 4.2, 2.8, 1H), 7.47 (dd, *J* = 12.1, 6.1, 2H), 7.44 - 7.39 (m, 1H), 7.32 - 7.19 (m, 2H), 6.78 (s, 1H), 4.34 (dd, *J* = 15.4, 6.6, 1H), 4.24 (dd, *J* =15.3,6.1, 1H), 3.92 - 3.77 (m, 2H), 2.61 - 2.52 (m, 1H), 2.10 (ddd, *J* = 19.9, 12.4, 6.8, 1H) | 4.587 min [441.0] % |
| "A51" | 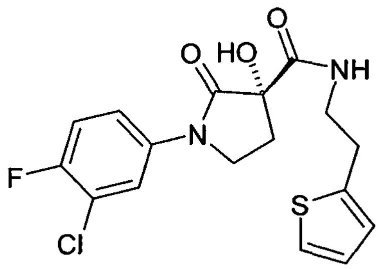 (S)-1-(3-Chlor-4-fluor-phenyl)-3-hydroxy-2-oxo-pyrrolidin-3-carbonsäure-(2-thiophen-2-yl-ethyl)-amid | 8.13 (t, *J* = 6.0, 1H), 7.99 (dd, *J* = 6.7, 2.7, 1 H), 7.75 - 7.63 (m, 1H), 7.48 (d, *J* = 9.1, 1H), 7.33 (dd, J = 5.1, 1.1, 1H), 6.94 (dd, *J* = 5.1, 3.4, 1H), 6.88 (d, *J* = 3.3, 1H), 6.69 (s, 1H), 3.89 - 3.75 (m, 2H), 3.46 - 3.35 (m, 1H), 2.95 (t, *J* = 7.3, 2H), 2.07 (dt, *J* = 13.0, 7.9, 1H) | 4.148 min [383.0]% |
| "A52" | 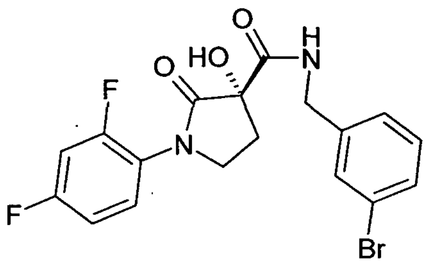 (S)-1-(2,4-Difluor-phenyl)-3-hydroxy-2-oxo-pyrrolidin-3-carbonsäure-3-brom-benzylamid | 8.63 (t, *J* = 6.4, 1H), 7.50 - 7.44 (m, 2H), 7.44 - 7.36 (m, 2H), 7.30 - 7.24 (m, 2H), 7.16 (ddd, *J* = 8.2, 2.9, 1.5, 1H), 6.75 (s, 1H), 4.35 (dd, *J* = 15.3, 6.6, 1H), 4.26 (dd, *J* = 15.4, 6.1, 1H), 3.83 - 3.66 (m, 2H), 2.64 - 2.55 (m, 1H), 2.23 - 2.13 (m, 2H) | 4.207 min [427.0 + 428.0] % |

(fortgesetzt)

| Nr. | Struktur / Name | $^1$H NMR (400 MHz, DMSO-$d_6$) δ [ppm] | LC-MS; rt; [M+H$^+$]$^*$ |
|---|---|---|---|
| "A53" | 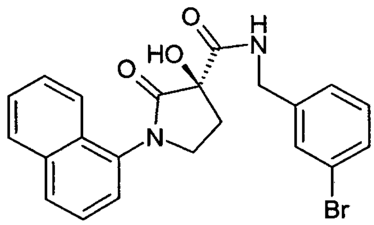 (R)-3-Hydroxy-1-naphthalin-1-yl-2-oxo-pyrrolidin-3-carbonsäure-3-brom-benzylamid | 8.69 (t, *J* = 6.3, 1H), 8.00 (d, *J* = 7.5, 1H), 7.95 (t, *J* = 7.2, 2H), 7.62 - 7.49 (m, 4H), 7.43 (dd, *J* = 12.9, 7.3, 2H), 7.31 (d, *J* = 7.7, 1H), 7.26 (t, *J* = 7.7, 1H), 6.84 (s, 1H), 4.40 (dd, *J* = 15.4, 6.6, 1H), 4.33 (dd, *J* = 15.4, 6.2, 1H), 3.91 - 3.75 (m, 2H), 2.69 (ddd, *J* = 12.7, 7.4, 3.2, 1H), 2.40 - 2.29 (m, 1H) | 4.364 min [439.0 + 442.0] % |
| "A54" | 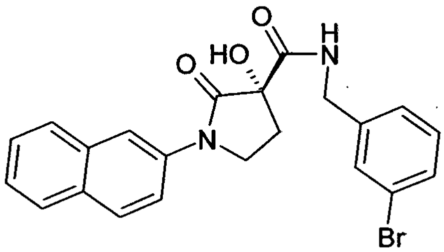 (S)-3-Hydroxy-1-naphthalin-2-yl-2-oxo-pyrrolidin-3-carbonsäure-3-brom-benzylamid | 8.69 (t, *J* = 6.4, 1H), 8.10 - 8.04 (m, 2H), 7.95 (d, *J* = 9.7, 1H), 7.90 (dd, *J* = 8.1, 2.9, 2H), 7.54 - 7.43 (m, 3H), 7.43 - 7.39 (m, 1H), 7.28 (t, *J* = 1.9, 1H), 6.78 (s, 1H), 4.36 (dd, *J* = 15.4, 6.6, 1H), 4.26 (dd, *J* = 15.4, 6.2, 1H), 3.99 (dd, *J* = 8.5, 5.6, 2H), 2.70 - 2.57 (m, 2H), 2.18 (dt, *J* = 12.9, 7.6, 1H) | 4.622 min [441.0 + 442.0] % |
| "A55" | 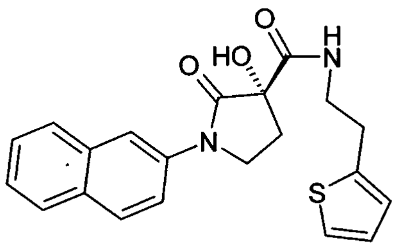 (S)-3-Hydroxy-1-naphthalin-2-yl-2-oxo-pyrrolidin-3-carbonsäure-(2-thiophen-2-yl-ethyl)-amid | 8.14 (t, *J* = 5.9, 1H), 8.08 - 8.02 (m, 2H), 7.94 (d, *J* = 8.7, 1H), 7.93 - 7.87 (m, 2H), 7.54 - 7.49 (m, 1H), 7.49 - 7.44 (m, 1H), 7.33 (dd, *J* = 5.1, 1.2, 1H), 6.95 (dd, *J* = 5.1, 3.4, 1H), 6.90 (d, *J* = 2.6, 1H), 6.69 (s, 1H), 4.01 - 3.93 (m, 2H), 3.38 (dd, *J* = 17.0, 10.5, 2H), 3.30 - 3.22 (m, 1H), 2.97 (t, *J* = 7.4, 2H), 2.61 - 2.52 (m, 2H), 2.13 (dt, *J* = 12.8, 7.8, 1H) | 4.264 min [381.0] % |
| "A56" | 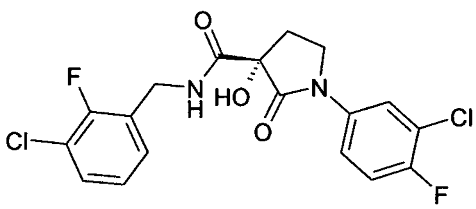 (S)-1-(3-Chlor-4-fluor-phenyl)-3-hydroxy-2-oxo-pyrrolidin-3-carbonsäure-3-chlor-2-fluor-benzylamid | 8.68 (t, *J* = 6.2, 1H), 7.99 (dd, *J* = 6.7, 2.7, 1H), 7.75 - 7.63 (m, 1H), 7.48 (d, *J* = 9.1, 1H), 7.46 - 7.42 (m, 1H), 7.30 (t, *J* = 6.5, 1H), 7.18 (t, *J* = 8.0, 1H), 6.83 (s, 1H), 4.40 (dd, *J* = 15.6, 6.4, 1H), 4.32 (dd, *J* = 15.6, 6.2, 1H), 3.90 - 3.81 (m, 2H), 2.63 - 2.53 (m, 1H), 2.12 (dt, *J* = 13.0, 7.7, 1H) | 4.602 min [415.0 + 417.0] % |

(fortgesetzt)

| Nr. | Struktur / Name | $^1$H NMR (400 MHz, DMSO-$d_6$) δ [ppm] | LC-MS; rt; [M+H$^+$]* |
|---|---|---|---|
| "A57" | 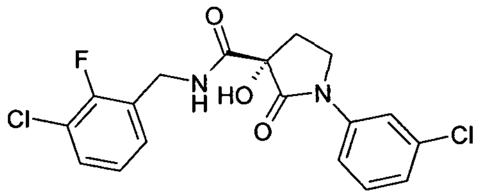 (S)-1-(3-Chlor-phenyl)-3-hydroxy-2-oxo-pyrrolidin-3-carbonsäure-3-chlor-2-fluor-benzylamid | 8.67 (t, *J* = 6.2, 1H), 7.88 (t, *J* = 2.0, 1H), 7.61 (dd, *J* = 8.3, 1.3, 1H), 7.48-7.41 (m, 1H), 7.32 - 7.27 (m, 1H), 7.25 (dd, *J* = 7.9, 1.3, 1H), 7.19 (dt, *J* = 8.3, 4.2, 1H), 6.80 (s, 1H), 4.40 (dd, *J* = 15.7, 6.7, 1H), 4.32 (dd, *J* = 15.6, 5.8, 1H), 3.86 (t, *J* = 6.8, 2H), 2.63 - 2.51 (m, 1H), 2.12 (dt, *J* = 13.0, 7.7, 1H) | 4.525 min [397.0 + 398.0 + 399.0] % |
| "A58" | 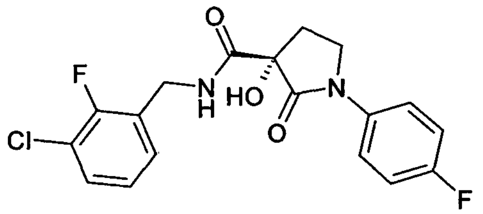 (S)-1-(4-Fluor-phenyl)-3-hydroxy-2-oxo-pyrrolidin-3-carbonsäure-3-chlor-2-fluor-benzylamid | 8.65 (t, *J* = 6.2, 1H), 7.76 - 7.65 (m, 2H), 7.52 - 7.36 (m, 1H), 7.33 - 7.28 (m, 1H), 7.27 - 7.22 (m, 2H), 7.18 (td, *J* = 7.9, 0.9, 1H), 6.76 (s, 1H), 4.40 (dd, *J* = 15.7, 6.3, 1H), 4.33 (dd, *J* = 15.3, 6.2, 1H), 3.84 (dd, *J* = 8.7, 5.6, 2H), 2.63 - 2.51 (m, 2H), 2.11 (dt, *J* = 12.9, 7.7, 1H) | 4.129 min [381.0] % |
| "A59" | 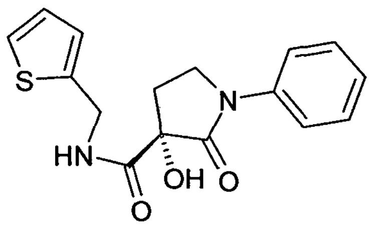 (S)-3-Hydroxy-2-oxo-1-phenyl-pyrrolidin-3-carbonsäure-(thiophen-2-ylmethyl)-amid | 8.58 (t, *J* = 6.2, 1H), 7.70 (d, *J* = 1.1, 1H), 7.68 (d, *J* = 1.0, 1H), 7.45 - 7.33 (m, 3H), 7.22 - 7.14 (m, 1H), 6.99 - 6.87 (m, 2H), 6.66 (s, 1H), 4.44 (d, *J* = 6.4, 2H), 3.91 - 3.78 (m, 2H), 2.59 - 2.52 (m, 1H), 2.09 (dt, *J* = 12.9, 8.5, 1H) | 3.373 min [317.0] % |
| "A60" | 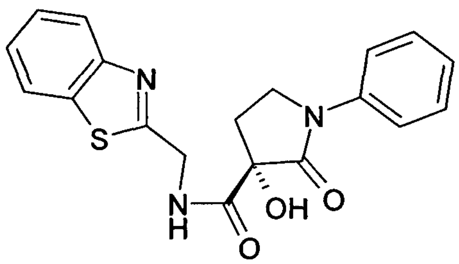 (S)-3-Hydroxy-2-oxo-1-phenyl-pyrrolidin-3-carbonsäure-(benzothiazol-2-ylmethyl)-amid | 9.03 (t, *J* = 6.2, 1H), 8.05 (d, *J* = 8.0, 1H), 7.93 (d, *J* = 7.9, 1H), 7.70 (d, *J* = 7.7, 2H), 7.53 - 7.46 (m, 1H), 7.44 - 7.35 (m, 3H), 7.18 (t, *J* = 7.4, 1H), 6.81 (s, 1H), 4.72 (dd, *J* = 16.2, 6.2, 1H), 4.65 (dd, *J* = 16.2, 6.1, 1H), 3.86 (dd, *J* = 9.7, 5.5, 2H), 2.60 (dd, *J* = 12.1, 7.6, 1H), 2.16 (dt, *J* = 12.7, 7.8, 1H) | 3.462 min [368.0] % |

(fortgesetzt)

| Nr. | Struktur / Name | $^1$H NMR (400 MHz, DMSO-$d_6$) δ [ppm] | LC-MS; rt; [M+H$^+$]* |
|---|---|---|---|
| "A61" | 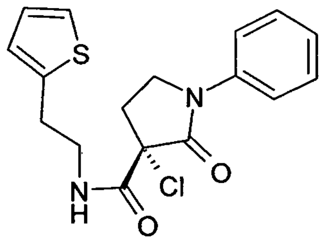 (S)-3-Chlor-2-oxo-1-phenyl-pyrrolidin-3-carbonsäure-(2-thiophen-2-yl-ethyl)-amid | 8.51 (s, 1H), 7.67 (d, *J* = 1.1, 1H), 7.65 (d, *J* = 0.8, 1H), 7.43 (t, *J* = 8.0, 2H), 7.32 (dd, *J* = 5.1, 1.2, 1H), 7.23 (t, *J* = 7.4, 1H), 6.94 (dd, *J* = 5.0, 3.4, 1H), 6.89 (d, *J* = 3.3, 1H), 4.06 - 3.82 (m, 2H), 3.54 - 3.35 (m, 3H), 2.97 (dt, *J* = 20.8, 7.4, 3H) | 4.522 min [349.0] % |
| "A62" | 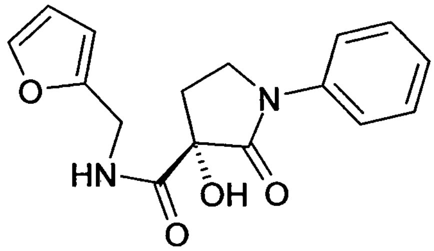 (S)-3-Hydroxy-2-oxo-1-phenyl-pyrrolidin-3-carbonsäure-(furan-2-ylmethyl)-amid | 8.37 (s, 1H), 7.70 (d, *J* = 1.1, 1H), 7.68 (d, *J* = 0.9, 1H), 7.54 (dd, *J* = 1.8, 0.8, 1H), 7.44 - 7.37 (m, 2H), 7.17 (t, *J* = 7.4, 1H), 6.68 (s, 1H), 6.37 (dd, *J* = 3.2, 1.8, 1H), 6.21 (d, *J* = 2.4, 1H), 4.28 (d, *J* = 6.6, 2H), 3.89 - 3.82 (m, 2H), 2.60 - 2.53 (m, 1H), 2.09 (dt, *J* = 12.9, 8.4, 1H) | 2.863 min [301.0] % |
| "A63" | 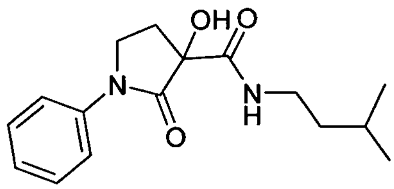 3-Hydroxy-2-oxo-1-phenyl-pyrrolidin-3-carbonsäure-(3-methylbutyl)-amid | 7.86 (br. t, *J* = 5.9, 1H), 7.69 (dt, *J* = 9.0, 1.7, 2H), 7.44 - 7.37 (m, 2H), 7.22 - 7.12 (m, 1H), 6.56 (s, 1H), 3.96 - 3.72 (m, 2H), 3.11 (dd, *J* = 14.4, 6.2, 2H), 2.57 - 2.52 (m, 1H), 2.14 - 1.98 (m, 1H), 1.56 (dp, *J* = 13.3, 6.7, 1H), 1.33 (dd, *J* = 14.5, 7.0, 2H), 0.87 (d, *J* = 2.2, 3H), 0.86 (d, *J* = 2.2, 3H) | 1.978 min [291.10] |
| "A64" | 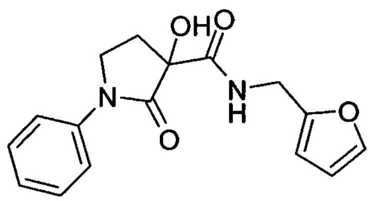 3-Hydroxy-2-oxo-1-phenyl-pyrrolidin-3-carbonsäure-(furan-2-ylmethyl)-amid | 8.35 (t, *J* = 6.0, 1H), 7.70 (dd, *J* = 8.7, 1.0, 2H), 7.55 (dd, *J* = 1.7, 0.8, 1H), 7.47 - 7.35 (m, 2H), 7.18 (t, *J* = 7.4, 1H), 6.67 (s, 1H), 6.38 (dd, *J* = 3.1, 1.9, 1H), 6.22 (dd, *J* = 3.1, 0.7, 1H), 4.29 (d, *J* = 6.2, 2H), 3.93 - 3.80 (m, 2H), 2.56 (ddd, *J* = 12.8, 7.3, 4.1, 1H), 2.18 - 2.04 (m, 1H) | 1.716 min [301.10] |

(fortgesetzt)

| Nr. | Struktur / Name | $^1$H NMR (400 MHz, DMSO-$d_6$) δ [ppm] | LC-MS; rt; [M+H$^+$]* |
|---|---|---|---|
| "A65" | 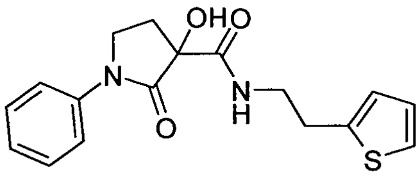 3-Hydroxy-2-oxo-1-phenyl-pyrrolidin-3-carbonsäure-(2-thiophen-2-yl-ethyl)-amid | 8.08 (t, *J* = 5.9, 1H), 7.71 (s, 1H), 7.69 (d, *J* = 8.8, 1H), 7.48 - 7.37 (m, 2H), 7.33 (dt, *J* = 10.3, 5.1, 1H), 7.23 - 7.13 (m, 1H), 6.96 (dd, *J* = 5.0, 3.5, 1H), 6.90 (t, *J* = 7.1, 1H), 6.61 (d, *J* = 17.5, 1H), 3.91 - 3.80 (m, 2H), 3.48 - 3.38 (m, 1H), 3.33 - 3.22 (m, 3H), 2.98 (t, *J* = 7.3, 2H), 2.58 - 2.52 (m, 1 H), 2.10 (m, 1H) | |
| "A66" | 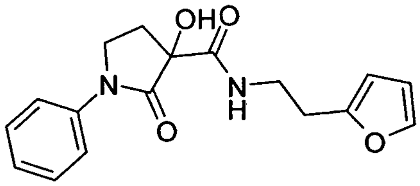 3-Hydroxy-2-oxo-1-phenyl-pyrrolidin-3-carbonsäure-(2-furan-2-yl-ethyl)-amid | 8.06 (t, *J* = 5.9, 1H), 7.70 (t, *J* = 1.5, 1H), 7.68 (t, *J* = 1.6, 1H), 7.52 (dd, *J* = 1.8, 0.7, 1H), 7.44 - 7.37 (m, 2H), 7.22 - 7.14 (m, 1H), 6.62 (s, 1H), 6.35 (dd, *J* = 3.1, 1.9, 1H), 6.16 (dd, *J* = 3.1, 0.7, 1H), 3.92 - 3.76 (m, 2H), 3.40 (td, *J* = 13.7, 7.4, 1H), 2.79 (t, *J* = 7.3, 2H), 2.53 (dd, *J* = 6.7, 3.6, 1H), 2.15 - 2.00 (m, 1H) | 1.814 min [315.10] |
| "A67" | 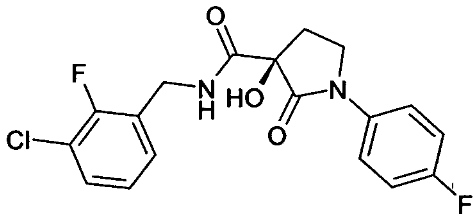 (R)-1-(4-Fluor-phenyl)-3-hydroxy-2-oxo-pyrrolidin-3-carbonsäure-3-chlor-2-fluor-benzylamid | 8.65 (t, *J* = 6.3, 1H), 7.78 - 7.68 (m, 2H), 7.45 (td, *J* = 8.0, 1.6, 1H), 7.33 - 7.28 (m, 1H), 7.27 - 7.22 (m, 2H), 7.18 (td, *J* = 7.9, 0.8, 1H), 6.75 (d, *J* = 4.2, 1H), 4.40 (dd, *J* = 15.6, 6.3, 1H), 4.33 (dd, *J* = 15.6, 5.9, 1H), 3.89 - 3.79 (m, 2H), 2.62 - 2.52 (m, 1H), 2.11 (dt, *J* = 13.0, 7.7, 1H) | 4.138 min [381.0] % |
| "A68" | 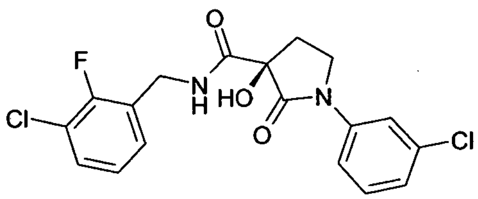 (R)-1-(3-Chlor-phenyl)-3-hydroxy-2-oxo-pyrrolidin-3-carbonsäure-3-chlor-2-fluor-benzylamid | 8.67 (t, *J* = 6.3, 1H), 7.88 (t, *J* = 2.1, 1H), 7.61 (dd, *J* = 8.3, 1.3, 1H), 750 - 7.40 (m, 2H), 7.32 - 7.27 (m, 1H), 7.25 (ddd, *J* = 8.0, 2.0, 0.8, 1H), 7.19 (td, *J* = 8.0, 0.9, 1H), 6.80 (s, 1H), 4.40 (dd, *J* = 15.5, 6.4, 1H), 4.32 (dd, *J* = 15.7, 5.9, 1H), 3.86 (t, *J* = 6.9, 2H), 2.62 - 2.53 (m, 1H), 2.12 (dt, *J* = 13.0, 7.7, 2H) | 4.523 min [397.0] % |

(fortgesetzt)

| Nr. | Struktur / Name | $^1H$ NMR (400 MHz, DMSO-$d_6$) δ [ppm] | LC-MS; rt; [M+H$^+$]* |
|---|---|---|---|
| "A69" | 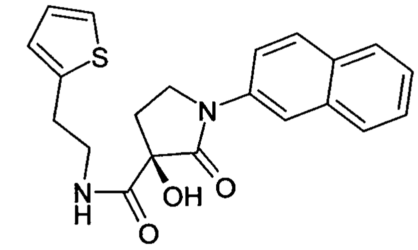 (R)-3-Hydroxy-1-naphthalin-2-yl-2-oxo-pyrrolidin-3-carbonsäure-(2-thiophen-2-yl-ethyl)-amid | 8.14 (t, *J* = 5.9, 1H), 8.09 - 8.04 (m, 2H), 7.94 (d, *J* = 8.7, 1H), 7.92 - 7.87 (m, 2H), 7.51 (dd, *J* = 10.9, 4.2, 1H), 7.47 (dd, *J* = 10.8, 4.2, 1H), 7.33 (dd, *J* = 5.1, 1.2, 1H), 6.95 (dd, *J* = 5.1, 3.4, 1H), 6.90 (d, *J* = 2.5, 1H), 6.69 (s, 1H), 4.04 - 3.88 (m, 2H), 3.48 - 3.35 (m, 1H), 3.29 (d, *J* = 5.9, 1H), 2.97 (t, *J* = 7.3, 2H), 2.63 - 2.52 (m, 1H), 2.13 (dt, *J* = 12.8, 7.9, 1H) | 4.214 min [381.0] % |
| "A70" | 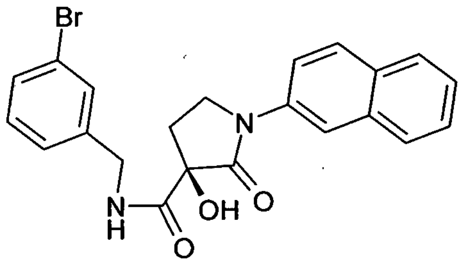 (R)-3-Hydroxy-1-naphthalin-2-yl-2-oxo-pyrrolidin-3-carbonsäure-3-brom-benzylamid | 8.69 (t, *J* = 6.3, 1H), 8.05 (s, 1 H), 7.95 (d, *J* = 9.7, 1H), 7.90 (dd, *J* = 8.0, 3.1, 2H), 7.55 - 7.44 (m, 3H), 7.44 - 7.39 (m, 1H), 7.28 (s, 1H), 6.78 (s, 1H), 4.36 (dd, *J* = 15.4, 6.6, 1H), 4.26 (dd, *J* = 15.4, 6.2, 1H), 3.99 (t, *J* = 7.0, 2H), 2.70 - 2.56 (m, 1H), 2.18 (dt, *J* = 13.1, 7.8, 1H) | 4.629 min [441.0] % |
| "A71" | 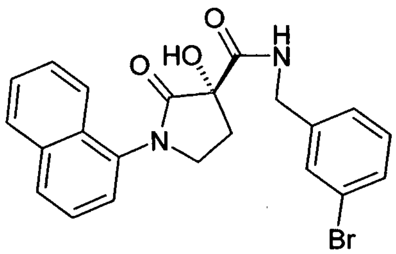 (S)-3-Hydroxy-1-naphthalin-1-yl-2-oxo-pyrrolidin-3-carbonsäure-3-brom-benzylamid | 8.69 (t, *J* = 6.4, 1H), 8.02 - 7.98 (m, 1H), 7.95 (t, *J* = 7.5, 2H), 7.61 - 7.49 (m, 4H), 7.45 (d, *J* = 7.3, 1H), 7.41 (dd, *J* = 5.5, 4.2, 1H), 7.31 (d, *J* = 7.8, 1H), 7.26 (t, *J* = 7.7, 1H), 6.82 (s, 1H), 4.40 (dd, *J* = 15.1, 6.6, 1H), 4.33 (dd, *J* = 15.3, 6.3, 1H), 3.86 (dd, *J* = 16.3, 7.4, 1H), 3.79 (td, *J* = 8.8, 3.3, 1H), 2.69 (ddd, *J* = 12.9, 7.5, 3.1, 1H), 2.34 (dt, *J* = 12.9, 8.5, 1H) | 4.394 min [439.0 + 442.0] % |
| "A72" | 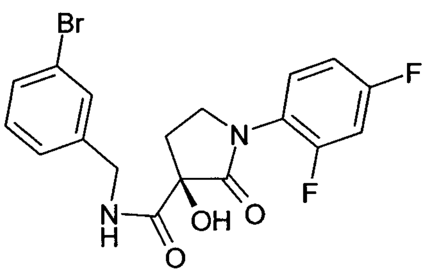 (R)-1-(2,4-Difluor-phenyl)-3-hydroxy-2-oxo-pyrrolidin-3-carbonsäure-3-brom-benzylamid | 8.64 (t, *J* = 6.5, 1H), 7.50 - 7.44 (m, 2H), 7.44-7.37 (m, 2H), 7.26 (dd, *J* = 4.1, 2.2, 2H), 7.17 (t, *J* = 8.6, 1H), 6.75 (s, 1H), 4.35 (dd, *J* = 15.4, 6.5, 1H), 4.26 (dd, *J* = 15.4, 6.1, 1H), 3.81 - 3.66 (m, 2H), 2.64 - 2.56 (m, 1H), 2.22 - 2.13 (m, 1H) | 4.055 min [425.0 + 427.0] % |

(fortgesetzt)

| Nr. | Struktur / Name | $^{1}H$ NMR (400 MHz, DMSO-$d_6$) δ [ppm] | LC-MS; rt; [M+H$^+$]* |
|---|---|---|---|
| "A73" | 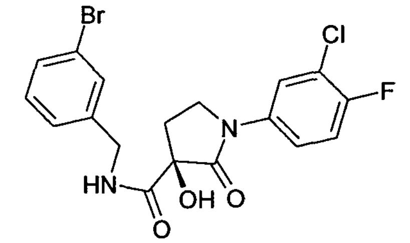<br>(R)-1-(3-Chlor-4-fluor-phenyl)-3-hydroxy-2-oxo-pyrrolidin-3-carbonsäure-3-brom-benzylamid | 8.68 (t, *J* = 6.4, 1H), 8.00 (dd, *J* = 6.7, 2.7, 1H), 7.68 (ddd, *J* = 9.1, 4.2, 2.8, 1H), 7.47 (dd, *J* = 12.2, 6.0, 2H), 7.45 - 7.35 (m, 1H), 7.33 - 7.22 (m, 2H), 6.78 (s, 1H), 4.34 (dd, *J* = 15.3, 6.6, 1H), 4.24 (dd, *J* = 15.4, 6.1, 1H), 3.93 - 3.82 (m, 2H), 2.62 - 2.51 (m, 2H), 2.11 (dt, *J* = 12.9, 7.7, 1H) | 4.586 min [441.0] % |
| "A74" | 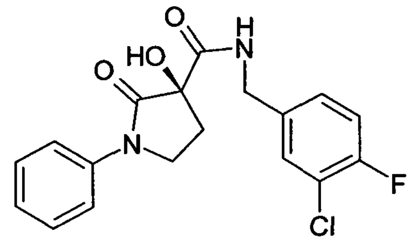<br>(R)-3-Hydroxy-2-oxo-1-phenyl-pyrrolidin-3-carbonsäure-3-chlor-4-fluor-benzylamid | 8.68 (t, *J* = 6.4, 1H), 7.74 - 7.65 (m, 2H), 7.47 (dd, *J* = 7.3, 2.1, 1H), 7.40 (dtd, *J* = 6.3, 4.2, 2.1, 2H), 7.34 (d, *J* = 9.3, 1H), 7.27 (ddd, *J* = 8.5, 4.8, 2.1, 1H), 7.17 (t, *J* = 7.4, 1H), 6.73 (s, 1H), 4.33 (dd, *J* = 15.1, 6.7, 1H), 4.22 (dd, *J* = 15.2, 6.3, 1H), 3.85 (dd *J* = 8.5, 5.6, 2H), 2.62 - 2.53 (m, 1H), 2.18 - 2.04 (m, 1H). | 4.124 min [363.0] % |
| "A75" | 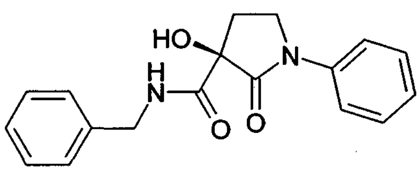<br>(R)-3-Hydroxy-2-oxo-1-phenyl-pyrrolidin-3-carbonsäure-benzylamid | 8.52 (t, *J* = 6.2, 1H), 7.75 - 7.64 (m, 2H), 7.45 - 7.35 (m, 2H), 7.33 - 7.14 (m, 6H), 6.68 (s, 1H), 4.33 (dd, *J* = 15.0, 6.5, 1H), 4.27 (dd, *J* = 15.0, 6.2, 1H), 3.92 - 3.79 (m, 2H), 2.62 - 2.53 (m, 1H), 2.17 - 2.05 (m, 1H) | |
| "A76" | 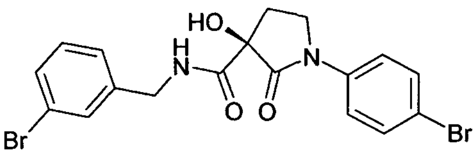<br>(R)-1-(4-Brom-phenyl)-3-hydroxy-2-oxo-pyrrolidin-3-carbonsäure-3-brom-benzylamid | (400 MHz, $CDCl_3$) δ [ppm] 7.60 - 7.55 (m, 1H), 7.55 -7.47 (m, 1H), 7.42 (t, *J* = 3.5, 1H), 7.23 - 7.17 (m, 1H), 4.51 - 4.33 (m, 1H), 4.19 (td, *J* = 9.1, 7.0, 1H), 3.82 (t, *J* = 8.7, 1H), 3.77 (s, 1H), 2.78 (dd, *J* = 12.8, 5.9, 1H), 2.29 (dt, *J* = 12.8, 9.3, 1H) | 4.596 min [467.0 + 469.0 + 472.0] % |
| "A77" | 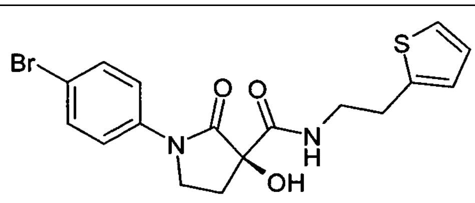<br>(R)-1-(4-Brom-phenyl)-3-hydroxy-2-oxo-pyrrolidin-3-carbonsäure-(2-thiophen-2-yl-ethyl)-amid | (400 MHz, $CDCl_3$) δ [ppm] 7.61 - 7.54 (m, 2H), 7.54 - 7.49 (m, 2H), 7.18 (dd, *J* = 5.1, 1.2, 1H), 7.07 (s, 1H), 6.96 (dd, *J* = 5.1, 3.4, 1H), 6.87 (dd, *J* = 3.4, 1.0, 1H), 4.16 (td, *J* = 9.2, 6.9, 1H), 3.80 (td, *J* = 9.2, 1.2, 1H), 3.55 (dd, *J* = 13.0, 6.6, 3H), 3.07 (t, *J* = 6.9, 2H), 2.71 (ddd, *J* = 12.8, 6.9, 1.2, 1H), 2.25 (dt, *J* = 12.7, 9.4, 2H) | 4.133 min [409.0 + 412.0] % |

(fortgesetzt)

| Nr. | Struktur / Name | $^1$H NMR (400 MHz, DMSO-$d_6$) δ [ppm] | LC-MS; rt; [M+H$^+$]* |
|---|---|---|---|
| "A78" | 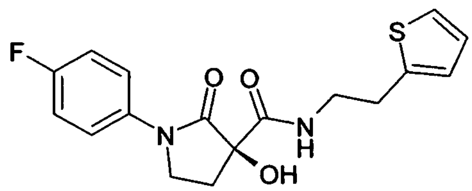 (R)-1-(4-Fluor-phenyl)-3-hydroxy-2-oxo-pyrrolidin-3-carbonsäure-(2-thiophen-2-yl-ethyl)-amid | (400 MHz, $CDCl_3$) δ [ppm] 7.69 - 7.58 (m, 2H), 7.18 (dd, *J* = 5.1, 1.1, 1H), 7.15 - 7.04 (m, 2H), 6.96 (dd, *J* = 5.1, 3.4, 1H), 6.87 (d, *J* = 2.6, 1H), 4.17 (td, *J* = 9.2, 7.0, 1H), 3.79 (dd, *J* = 9.2, 8.1, 1H), 3.58 (s, 1H), 3.55 (q, *J* = 6.6, 2H), 3.07 (t, *J* = 6.8, 2H), 2.80 - 2.64 (m, 1H), 2.25 (dt, *J* = 12.8, 9.3, 1H) | 3.624 min [349.0] % |
| "A79" | 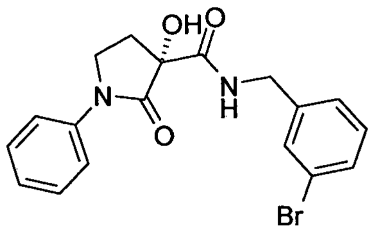 (R)-3-Hydroxy-2-oxo-1-phenyl-pyrrolidin-3-carbonsäure-3-brom-benzylamid | 8.65 (s, 1H), 7.70 (d, *J* = 8.2, 2H), 7.45 (d, *J* = 12.1, 1H), 7.43 (s, 3H), 7.26 (d, *J* = 3.8, 2H), 7.17 (t, *J* = 7.1, 1H), 6.73 (s, 1H), 4.34 (dd, *J* = 15.1, 6.5, 1H), 4.24 (dd, *J* = 15.5, 6.1, 1H), 3.85 (t, *J* = 6.7, 2H), 2.56 *(dd, J* = 13.0, 6.4, 1H), 2.19 - 2.05 (m, 1H) | |
| "A80" | 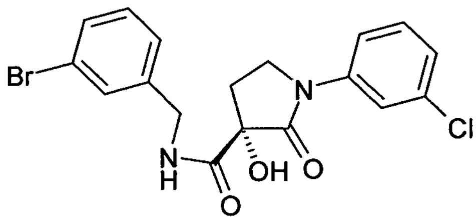 (S)-1-(3-Chlor-phenyl)-3-hydroxy-2-oxo-pyrrolidin-3-carbonsäure-3-brom-benzylamid | 8.68 (t, *J* = 6.4, 1H), 7.90 (t, *J* = 2.1, 1H), 7.62 (dd, *J* = 8.0, 1.7, 1H), 7.42 (ddd, *J* = 7.9, 7.2, 2.1, 3H), 7.30 - 7.22 (m, 3H), 6.77 (s, 1H), 4.34 (dd, *J* = 15.4, 6.6, 1H), 4.24 (dd, *J* = 15.4, 6.1, 1H), 3.92 - 3.79 (m, 2H), 2.63 - 2.52 (m, 1H), 2.12 (dt, *J*= 13.0, 7.8, 1H) | 4.547 min [423.0 + 424.0] % |
| "A81" | 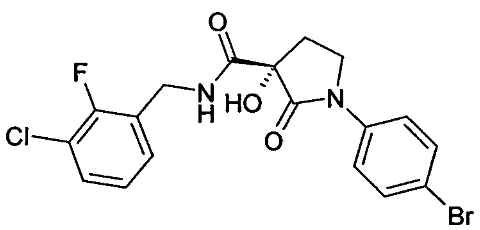 (S)-1-(4-Brom-phenyl)-3-hydroxy-2-oxo-pyrrolidin-3-carbonsäure-3-chlor-2-fluor-benzylamid | 8.66 (t, *J* = 6.2, 1H), 7.74 - 7.64 (m, 2H), 7.64 - 7.56 (m, 2H), 7.52 - 7.41 (m, 1H), 7.30 (t, *J* = 7.3, 1H), 7.18 (t, *J* = 7.9, 1H), 6.78 (s, 1H), 4.40 (dd, *J* = 15.4, 6.5, 1H), 4.32 (dd, *J* =15.5,6.2, 1H), 3.91-3.76 (m, 2H), 2.63 - 2.52 (m, 1H), 2.12 (dt, *J* = 12.9, 7.7, 1H) | 5.804 min [438.7 + 440.0] % |
| "A82" | 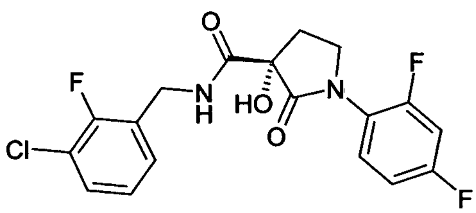 (S)-1-(2,4-Difluor-phenyl)-3-hydroxy-2-oxo-pyrrolidin-3-carbonsäure-3-chlor-2-fluor-benzylamid | 8.63 (t, *J* = 6.3, 1H), 7.51 - 7.36 (m, 3H), 7.30 (t, *J* = 6.6, 1H), 7.16 (dd, *J* = 13.5, 5.4, 2H), 6.77 (s, 1H), 4.42 (dd, *J* = 15.6, 6.4, 1H), 4.34 (dd, *J* = 15.5, 6.0, 1H), 3.84 - 3.67 (m, 2H), 2.72 - 2.56 (m, 1H), 2.17 (dt, *J* = 13.0, 7.6, 1 H) | 4.073 min [399.0] % |

(fortgesetzt)

| Nr. | Struktur / Name | $^1H$ NMR (400 MHz, DMSO-$d_6$) δ [ppm] | LC-MS; rt; [M+H$^+$]* |
|---|---|---|---|
| "A83" | 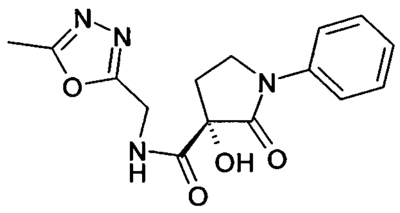 (S)-3-Hydroxy-2-oxo-1-phenyl-pyrrolidin-3-carbonsäure-(5-methyl-[1,3,4]oxadiazol-2-ylmethyl)-amid | 8.69 (t, *J* = 6.0, 1H), 7.73 - 7.63 (m, 2H), 7.40 (dd, *J* = 10.8, 5.3, 2H), 7.17 (t, *J* = 7.4, 1H), 6.75 (s, 1H), 4.51 (dd, *J* = 16.1, 6.1, 1H), 4.42 (dd, *J* = 16.1, 5.8, 1H), 3.92 - 3.76 (m, 2H), 2.59 - 2.52 (m, 1H), 2.45 (s, 3H), 2.12 (dt, *J* = 13.0, 7.9, 1H) | 2.188 min [317.0]% |
| "A84" | 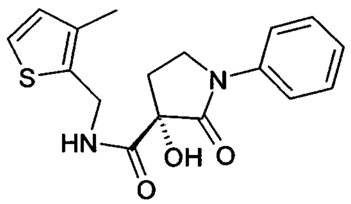 (S)-3-Hydroxy-2-oxo-1-phenyl-pyrrolidin-3-carbonsäure-(3-methyl-thiophen-2-ylmethyl)-amid | 8.45 (t, *J* = 6.1, 1H), 7.74 - 7.66 (m, 2H), 7.46 - 7.37 (m, 2H), 7.25 *(d, J* = 5.1, 1H), 7.22 - 7.14 (m, 1H), 6.79 (d, *J* = 5.1, 1H), 6.64 (s, 1H), 4.39 (dd, *J* = 15.2, 6.4, 1H), 4.33 (dd, *J* = 15.4, 6.4, 1H), 3.84 (ddd, *J* = 14.2, 9.2, 3.7, 2H), 2.60 - 2.52 (m, 1H), 2.16 (s, 3H), 2.09 (dt, *J* = 12.9, 8.5, 1H) | 3.541 min [331.0] % |
| "A85" | 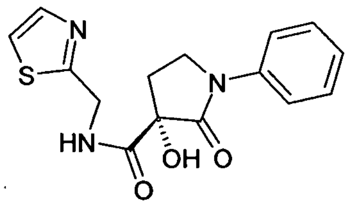 (S)-3-Hydroxy-2-oxo-1-phenyl-pyrrolidin-3-carbonsäure-(thiazol-2-ylmethyl)-amid | 8.89 (t, *J* = 6.2, 1H), 7.70 (td, *J* = 3.2, 1.0, 3H), 7.60 (d, *J* = 3.3, 1H), 7.40 (dd, *J* = 10.7, 5.3, 2H), 7.18 (t, *J* = 7.4, 1H), 6.76 (s, 1H), 4.59 (dd, *J* = 16.0, 6.4, 1H), 4.54 (dd, *J* = 16.0, 6.3, 1H), 3.92 - 3.80 (m, 2H), 2.61 - 2.52 (m, 1H), 2.13 (dt, *J* = 12.9, 8.0, 1H) | 2.356 min [318.0] |
| "A86" | 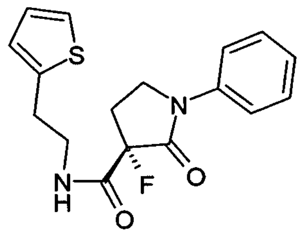 (S)-3-Fluor-2-oxo-1-phenyl-pyrrolidin-3-carbonsäure-(2-thiophen-2-yl-ethyl)-amid | $^{19}F$ NMR (377 MHz, DMSO) δ [ppm] -158.16; $^1H$ NMR 400 MHz, DMSO-$d_6$) δ [ppm] 8.72 (s, 1H), 7.69 (d, J = 7.7, 2H), 7.50 - 7.40 (m, 2H), 7.33 (dd, J = 5.1, 1.2, 1H), 7.23 (t, J = 7.4, 1H), 6.94 (dd, J = 5.1, 3.4, 1H), 6.89 (d, J = 3.4, 1H), 3.97 (dd, J = 9.2, 3.3, 1H), 3.92 (dd, J = 6.2, 3.4, 1H), 3.48 - 3.37 (m, 2H), 2.98 (t, J = 7.3, 2H), 2.77 - 2.62 (m, 1H), 2.47 - 2.35 (m, 2H) | |

(fortgesetzt)

| Nr. | Struktur / Name | $^1H$ NMR (400 MHz, DMSO-$d_6$) δ [ppm] | LC-MS; rt; [M+H+]* |
|---|---|---|---|
| "A87" | 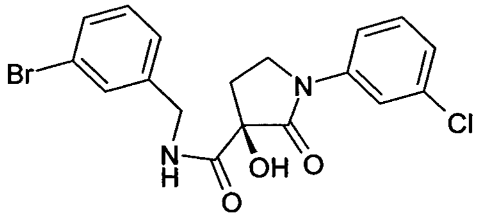<br>(R)-1-(3-Chlor-phenyl)-3-hydroxy-2-oxo-pyrrolidin-3-carbonsäure-3-brom-benzylamid | 8.68 (t, *J* = 6.3, 1H), 7.90 (t, *J* = 2.1, 1H), 7.62 (ddd, *J* = 8.3, 2.1, 0.8, 1H), 7.48 - 7.44 (m, 1H), 7.44 - 7.38 (m, 2H), 7.31 - 7.20 (m, 3H), 6.78 (s, 1H), 4.34 (dd, *J* = 15.3, 6.6, 1H), 4.24 (dd, *J* = 15.3, 6.1, 1H), 3.92 - 3.77 (m, 2H), 2.64 - 2.53 (m, 1H), 2.12 (dt, *J* = 13.0, 7.8, 1H) | 4.487 min [422.8 + 424.8] % |
| "A88" | 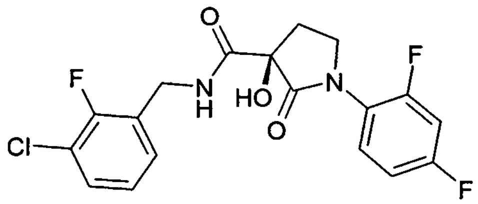<br>(R)-1-(2,4-Difluor-phenyl)-3-hydroxy-2-oxo-pyrrolidin-3-carbonsäure-3-chlor-2-fluor-benzylamid | 8.63 (t, *J* = 6.3, 1H), 7.51 - 7.36 (m, 3H), 7.30 (dd, *J* = 10.7, 3.9, 1H), 7.20-7.13 (m, 2H), 6.78 (s, 1H), 4.41 (dd, *J* = 15.6, 6.4, 1H), 4.34 (dd, *J* = 15.6, 6.1, 1H), 3.84 - 3.66 (m, 2H), 2.61 (ddd, *J* = 12.8, 7.4, 4.2, 1H), 2.17 (ddd, *J* = 13.0, 8.2, 7.0, 1H) | 4.075 min [399.0] % |
| "A89" | 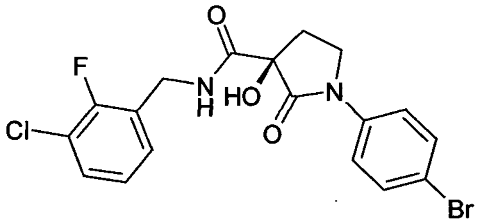<br>(R)-1-(4-Brom-phenyl)-3-hydroxy-2-oxo-pyrrolidin-3-carbonsäure-3-chlor-2-fluor-benzylamid | 8.66 (t, *J* = 6.3, 1H), 7.73 - 7.63 (m, 2H), 7.63 - 7.56 (m, 2H), 7.45 (t, *J* = 6.8, 1H), 7.30 (t, *J* = 6.5, 1H), 7.18 (t, *J* = 7.9, 1H), 6.78 (s, 1H), 4.40 (dd, *J* = 15.5, 6.3, 1H), 4.32 (dd, *J* = 15.6, 5.8, 1H), 3.87 - 3.80 (m, 2H), 2.62 - 2.53 (m, 2H), 2.12 (dt, *J* = 13.0, 7.7, 1H) | 5.804 min [438.7+440.0]<br>% |
| "A90" | 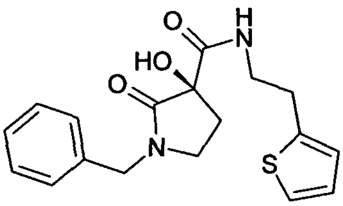<br>(R)-1-Benzyl-3-hydroxy-2-oxo-pyrrolidin-3-carbonsäure-(2-thiophen-2-yl-ethyl)-amid | 8.02 (t, *J* = 6.0, 1 H), 7.38 - 7.31 (m, 3H), 7.26 (t, *J* = 6.8, 3H), 6.94 (dd, *J* = 5.1, 3.4, 1H), 6.90 (dd, *J* = 3.4, 1.0, 1H), 6.45 (s, 1H), 4.48 (d, *J* = 15.2, 1H), 4.36 (d, *J* = 15.2, 1H), 3.40 (ddd, *J* = 14.0, 10.2, 4.9, 1H), 3.30 - 3.16 (m, 3H), 2.95 (t, *J* = 7.4, 2H), 2.37 (ddd, *J* = 12.9, 7.7, 3.5, 1H), 1.93 (ddd, *J* = 13.0, 8.7, 7.0, 1H) | |

(fortgesetzt)

| Nr. | Struktur / Name | $^1$H NMR (400 MHz, DMSO-$d_6$) δ [ppm] | LC-MS; rt; [M+H$^+$]* |
|---|---|---|---|
| "A91" | 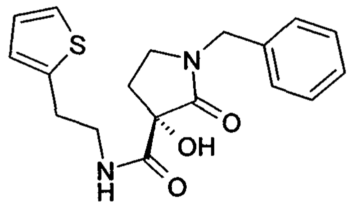 (S)-1-Benzyl-3-hydroxy-2-oxo-pyrrolidin-3-carbonsäure-(2-thiophen-2-yl-ethyl)-amid | 8.02 (t, *J* = 6.0, 1H), 7.37 - 7.31 (m, 3H), 7.27 (dd, *J* = 10.2, 4.6, 3H), 6.94 (dd, *J* = 5.1, 3.4, 1H), 6.89 (dd, *J* = 3.3, 1.0, 1H), 6.45 (s, 1H), 4.48 (d, *J* = 15.3, 1H), 4.36 (d, *J* = 15.2, 1H), 3.46 - 3.38 (m, 2H), 3.30 - 3.18 (m, 4H), 2.95 (t, *J* = 7.4, 2H), 2.37 (ddd, *J* = 12.8, 7.6, 3.4, 1H), 1.93 (ddd, *J* = 12.9, 8.7, 7.0, 1H) | |
| "A91a" | (S)-3-Amino-2-oxo-1-phenyl-pyrrolidin-3-carbonsäure-(3-chlor-5-fluor-benzyl)-amid | | |
| | | | 1) HPLC 2) LC-MS; rt; [M+H+] |
| "A92" | 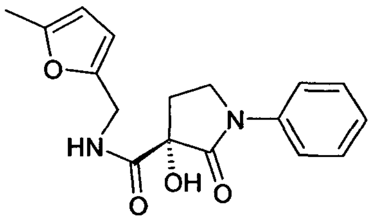 (S)-3-Hydroxy-2-oxo-1-phenyl-pyrrolidin-3-carbonsäure-(5-methyl-furan-2-ylmethyl)-amid | 8.27 (t, *J* = 6.1, 1H), 7.69 (dd, *J* = 8.7, 1.0, 2H), 7.47 - 7.33 (m, 2H), 7.17 (t, *J* = 7.4, 1H), 6.67 (s, 1H), 6.07 (d, *J* = 3.0, 1H), 5.96 (dd, *J* = 3.0, 1.0, 1H), 4.22 (d, *J* = 6.0, 3H), 3.94 - 3.73 (m, 2H), 2.60 - 2.55 (m, 1H), 2.21 (s, 3H), 2.15 - 2.03 (m, 1H) | 1) 3.342 min 2) [313.1] |
| "A93" | 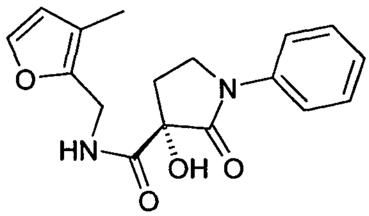 (S)-3-Hydroxy-2-oxo-1-phenyl-pyrrolidin-3-carbonsäure-(3-methyl-furan-2-ylmethyl)-amid | 8.23 (d, *J* = 5.4, 1H), 7.69 (dd, *J* = 8.7, 1.0, 2H), 7.45 (d, *J* = 1.8, 1H), 7.44 - 7.37 (m, 2H), 7.17 (*t, J* = 7.4, 1H), 6.63 (s, 1H), 6.24 (d, *J* = 1.8, 1H), 4.24 (dd, *J* = 5.9, 2.6, 2H), 3.90 - 3.78 (m, 2H), 2.56 - 2.53 (m, 1H), 2.13 - 2.03 (m, 1H), 1.97 (s, 3H) | 1) 3.326 min 2) [313] |
| "A94" | 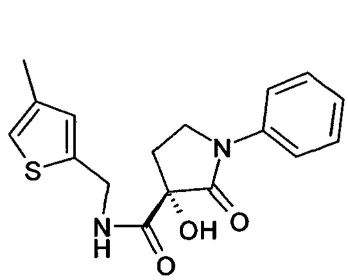 (S)-3-Hydroxy-2-oxo-1-phenyl-pyrrolidin-3-carbonsäure-(4-methyl-thiophen-2-ylmethyl)-amid | 8.52 (t, *J* = 6.2, 1H), 7.70 (dd, *J* = 5.4, 3.4, 2H), 7.44 - 7.37 (m, 2H), 7.22 - 7.14 (m, 1H), 6.94 - 6.90 (m, 1H), 6.76 (s, 1H), 6.66 (s, 1H), 4.45 - 4.30 (m, 2H), 3.93 - 3.78 (m, 2H), 2.56 - 2.51 (m, 1H), 2.13 (s, 3H), 2.12 - 2.05 (m, 1H) | 1) 3.666 min 2) 3.762 min [331.0] |

(fortgesetzt)

| Nr. | Struktur / Name | $^1$H NMR (400 MHz, DMSO-$d_6$) δ [ppm] | LC-MS; rt; [M+H$^+$]* |
|---|---|---|---|
| "A95" | 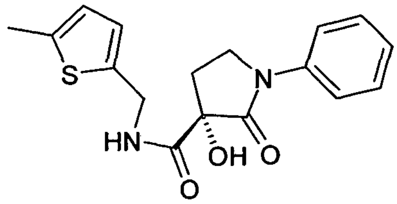 (S)-3-Hydroxy-2-oxo-1-phenyl-pyrrolidin-3-carbonsäure-(5-methyl-thiophen-2-ylmethyl)-amid | 8.48 (t, *J* = 6.2, 1H), 7.69 (dd, *J* = 8.7, 1.1, 2H), 7.44 - 7.36 (m, 2H), 7.23 - 7.09 (m, 1H), 6.71 (d, *J* = 3.3, 1H), 6.65 (s, 1H), 6.63 - 6.54 (m, 1H), 4.44 - 4.28 (m, 2H), 3.92 - 3.76 (m, 2H), 2.57 - 2.51 (m, 1H), 2.36 (d, *J* = 0.8, 3H), 2.15 - 2.04 (m, 1H) | 1) 3.653 min 2) 2.553 [331.2] |
| "A96" | 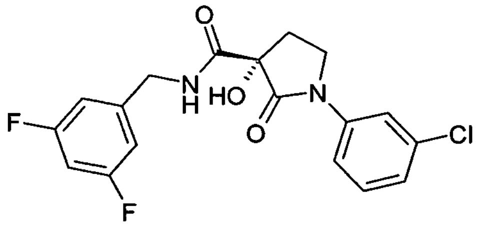 (S)-1-(3-Chlor-phenyl)-3-hydroxy-2-oxo-pyrrolidin-3-carbonsäure-3,5-difluor-benzylamid | 8.75 (t, *J* = 6.3, 1H), 7.88 (t, *J* = 2.1, 1H), 7.62 (ddd, *J* = 8.4, 2.1, 0.8, 1H), 7.44 (t, *J* = 8.2, 1H), 7.25 (ddd, *J* = 8.1, 2.0, 0.8, 1H), 7.07 (tt, *J* = 9.4, 2.4, 1H), 6.97 (dd, *J* = 8.6, 2.1, 2H), 6.82 (s, 1H), 4.38 (dd, *J* = 15.8, 6.7, 1H), 4.24 (dd, *J* = 15.8, 6.1, 1H), 3.92 - 3.80 (m, 2H), 2.63 - 2.54 (m, 1H), 2.13 (dt, *J* = 13.0, 7.6, 1H) | 1) 4.352 min 2) 4.344 min [381.0] |
| "A97" | 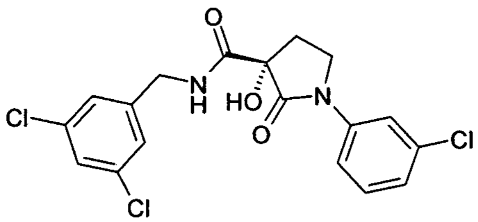 (S)-1-(3-Chlor-phenyl)-3-hydroxy-2-oxo-pyrrolidin-3-carbonsäure-3,5-dichlor-benzylamid | 8.77 (t, *J* = 6.3, 1H), 7.90 (t, *J* = 2.1, 1H), 7.62 (ddd, *J* = 8.3, 2.1, 0.8, 1H), 7.45 (d, *J* = 2.0, 1H), 7.43 (t, *J* = 6.1, 1H), 7.31 (d, *J* = 1.9, 2H), 7.25 (ddd, *J* = 8.0, 2.0, 0.8, 1H), 6.82 (s, 1H), 4.36 (dd, *J* = 15.7, 6.7, 1H), 4.23 (dd, *J* = 15.7, 6.0, 1H), 3.94 - 3.79 (m, 2H), 2.62 - 2.54 (m, 1H), 2.21 - 2.08 (m, 1H) | 1) 4.888 min 2) 3.373 min [415.0 + 416.0] |
| "A98" | 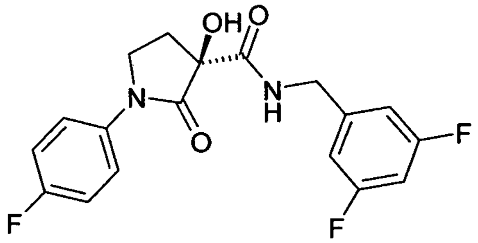 (S)-1-(4-Fluor-phenyl)-3-hydroxy-2-oxo-pyrrolidin-3-carbonsäure-3,5-difluor-benzylamid | 8.73 (t, *J* = 6.4, 1H), 7.76 -7.67 (m, 2H), 7.30 - 7.19 (m, 2H), 7.06 (tt, *J* = 9.4, 2.3, 1H), 7.02 - 6.93 (m, 2H), 6.78 (s, 1H), 4.39 (dd, *J* = 15.8, 6.7, 1H), 4.24 (dd, *J* = 15.9, 6.0, 1H), 3.84 (dd, *J* = 8.0, 5.7, 2H), 2.59 (dt, *J* = 11.9, 5.7, 1H), 2.12 (dt, *J* = 13.0, 7.6, 1H) | 1) 3.930 min 2) 3.935 min [365.0] |

(fortgesetzt)

| Nr. | Struktur / Name | $^1$H NMR (400 MHz, DMSO-$d_6$) δ [ppm] | LC-MS; rt; [M+H+]* |
|---|---|---|---|
| "A99" | 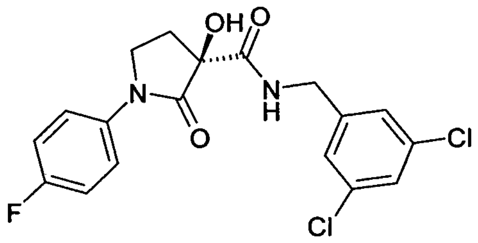 (S)-1-(4-Fluor-phenyl)-3-hydroxy-2-oxo-pyrrolidin-3-carbonsäure-3,5-dichlor-benzylamid | 8.74 (t, *J* = 6.4, 1H), 7.77 - 7.68 (m, 2H), 7.45 (t, *J* = 1.9, 1H), 7.32 (d, *J* = 1.9, 2H), 7.28 - 7.22 (m, 2H), 6.78 (s, 1H), 4.36 (dd, *J* = 15.7, 6.7, 1H), 4.23 (dd, *J* = 15.7, 6.0, 1H), 3.90 - 3.79 (m, 2H), 2.61 - 2.52 (m, 1H), 2.11 (dt, *J* = 13.0, 7.6, 1H) | 1) 4.566 min 2) 4.563 min [397.0 + 399.0] |
| "A100" | 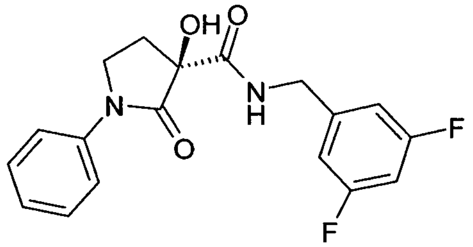 (S)-3-Hydroxy-2-oxo-1-phenyl-pyrrolidin-3-carbonsäure-3,5-difluor-benzylamid | 8.72 (t, *J* = 6.4, 1H), 7.73 - 7.67 (m, 2H), 7.43 - 7.37 (m, 2H), 7.18 (dd, *J* = 10.5, 4.2, 1H), 7.06 (tt, *J* = 9.4, 2.4, 1H), 7.00 - 6.95 (m, 2H), 6.77 (s, 1H), 4.39 (dd, *J* = 15.9, 6.8, 1H), 4.24 (dd, *J* = 15.8, 6.0, 1H), 3.86 (dd, *J* = 8.0, 5.7, 2H), 2.63 - 2.55 (m, 1H), 2.18 - 2.07 (m, 1H). | 1) 3.829 min 2) 3.472 min [347.2] |
| "A101" | 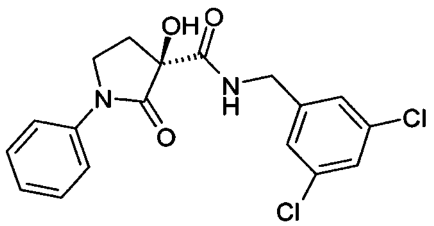 (S)-3-Hydroxy-2-oxo-1-phenyl-pyrrolidin-3-carbonsäure-3,5-dichlor-benzylamid | 8.74 (t, *J* = 6.4, 1H), 7.70 (dd, *J* = 8.7, 1.0, 2H), 7.45 (t, *J* = 1.9, 1H), 7.44 - 7.36 (m, 2H), 7.32 (d, *J* = 1.9, 2H), 7.18 (t, *J* = 7.4, 1H), 6.77 (s, 1H), 4.37 (dd, *J* = 15.6, 6.7, 1H), 4.23 (dd, *J* = 15.7, 6.0, 1H), 3.90 - 3.78 (m, 2H), 2.63 - 2.53 (m, 1H), 2.12 (dt, *J* = 13.0, 7.7, 1H) | 1) 4.431 min 2) 4.451 min [379.0 + 381.3] |
| "A102" | 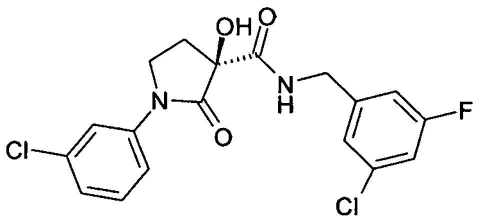 (S)-1-(3-Chlor-phenyl)-3-hydroxy-2-oxo-pyrrolidin-3-carbonsäure-3-chlor-5-fluor-benzylamid | 8.76 (t, *J* = 6.3, 1H), 7.89 (t, *J* = 2.1, 1H), 7.62 (ddd, *J* = 8.3, 2.1, 0.7, 1H), 7.44 (t, *J* = 8.2, 1H), 7.30 - 7.22 (m, 2H), 7.20 (s, 1H), 7.09 *(d, J* = 9.6, 1H), 6.82 (s, 1H), 4.37 (dd, *J* = 15.8, 6.7, 1H), 4.24 (dd, *J* = 15.8, 6.0, 1H), 3.93 - 3.81 (m, 2H), 2.63 - 2.54 (m, 1H), 2.18 - 2.08 (m, 1H) | 1) 4.630 min 2) 4.624 min [397.0 + 399.0] |
| "A103" | 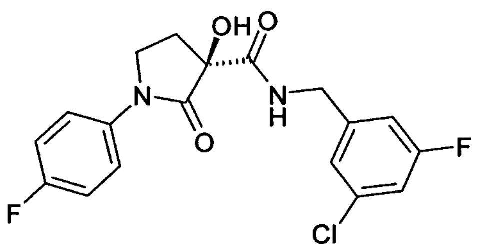 (S)-1-(4-Fluor-phenyl)-3-hydroxy-2-oxo-pyrrolidin-3-carbonsäure-3-chlor-5-fluor-benzylamid | 8.73 (t, *J* = 6.4, 1H), 7.79 - 7.64 (m, 2H), 7.26 (ddd, *J* = 12.3, 7.1, 2.1, 3H), 7.20 (s, 1H), 7.10 (d, *J* = 9.3, 1H), 6.77 (s, 1H), 4.38 (dd, *J* = 15.8, 6.7, 1H), 4.24 (dd, *J* = 15.8, 6.1, 1H), 3.94 - 3.73 (m, 2H), 2.64 - 2.53 (m, 1H), 2.12 (dt, *J* = 13.1, 7.7, 1H) | 1) 4.245 min 2) 4.354 min [381.0] |

(fortgesetzt)

| Nr. | Struktur / Name | $^1$H NMR (400 MHz, DMSO-d$_6$) δ [ppm] | LC-MS; rt; [M+H$^+$]* |
|---|---|---|---|
| "A104" | 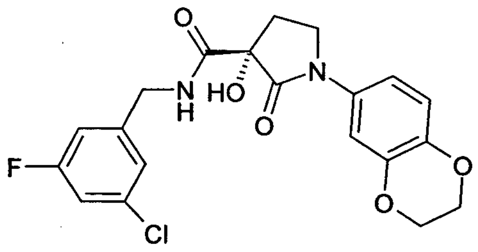<br>(S)-1-(2,3-Dihydro-benzo[1,4]dioxin-6-yl)-3-hydroxy-2-oxo-pyrrolidin-3-carbonsäure-3-chlor-5-fluor-benzylamid | 8.70 (t, *J* = 6.4, 1H), 7.27 (dt, *J* = 6.2, 2.9, 2H), 7.20 (s, 1H), 7.11 (d, *J* = 2.5, 1H), 7.09 (d, *J* = 2.6, 1H), 6.87 (d, *J* = 8.8, 1H), 6.72 (s, 1H), 4.37 (dd, *J* = 15.9, 6.7, 1H), 4.27 - 4.19 (m, 5H), 3.82 - 3.74 (m, 2H), 2.59 - 2.51 (m, 1H), 2.07 (dt, *J* = 12.9, 7.6, 1H) | 1) 4.066 min 2) 4.019 min [421.0] |
| "A105" | 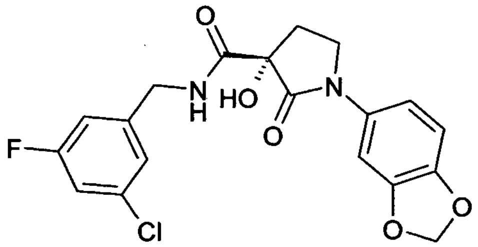<br>(S)-1-Benzo[1,3]dioxol-5-yl-3-hydroxy-2-oxo-pyrrolidin-3-carbonsäure-3-chlor-5-fluor-benzylamid | 8.71 (t, *J* = 6.4, 1H), 7.40 (d, *J* = 2.2, 1H), 7.27 (dt, *J* = 8.7, 2.1, 1H), 7.20 (s, 1H), 7.09 (d, *J* = 8.9, 1H), 7.04 (dd, *J* = 8.5, 2.2, 1H), 6.94 (d, *J* = 8.5, 1H), 6.73 (s, 1H), 6.02 (s, 2H), 4.37 (dd, *J* = 15.8, 6.8, 1H), 4.23 (dd, *J* = 15.8, 5.9, 1H), 3.79 (dd, *J* = 7.5, 6.1, 2H), 2.61 - 2.52 (m, 1H), 2.08 *(dt, J* = 12.9, 7.6, 1H) | 1) 4.062 min 2) 4.044 min [407.0] |
| "A106" | 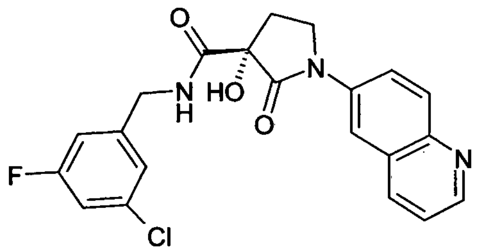<br>(S)-3-Hydroxy-2-oxo-1-chinolin-6-yl-pyrrolidin-3-carbonsäure-3-chlor-5-fluor-benzylamid | 8.85 (dd, *J* = 4.2, 1.7, 1H), 8.79 (t, *J* = 6.4, 1H), 8.36 (d, *J* = 7.6, 1H), 8.32 (dd, *J* = 9.2, 2.5, 1H), 8.12 (d, *J* = 2.5, 1H), 8.05 (d, *J* = 9.2, 1H), 7.53 (dd, *J* = 8.3, 4.2, 1H), 7.28 (dt, *J* = 8.8, 2.1, 1H), 7.22 (s, 1H), 7.11 (d, *J* = 9.7, 1H), 6.87 (s, 1H), 4.39 (dd, *J* = 15.8, 6.6, 1H), 4.26 (dd, *J* = 15.8, 6.1, 1H), 4.05 - 3.96 (m, 2H), 2.64 (dt, *J* = 6.9, 5.8, 1H), 2.20 (dt, *J* = 13.0, 7.6, 1H) | 1) 3.027 min 2) 3.099 min [414.0] |
| "A107" | 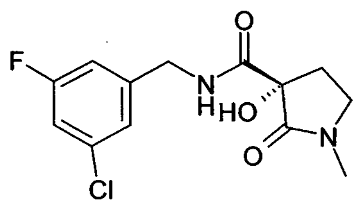<br>(S)-3-Hydroxy-1-methyl-2-oxo-pyrrolidin-3-carbonsäure-3-chlor-5-fluor-benzylamid | 8.59 (t, *J* = 6.4, 1H), 7.31 - 7.23 (m, 1H), 7.19 (s, 1H), 7.09 (d, *J* = 9.8, 1H), 6.46 (s, 1H), 4.36 (dd, *J* = 15.7, 6.8, 1H), 4.21 (dd, *J* = 15.8, 5.8, 1H), 2.76 (s, 3H), 2.45 - 2.40 (m, 1H), 2.00 - 1.88 (m, 1H) | 1)2.869 min 2) 2.950 min [301.0] |

(fortgesetzt)

| Nr. | Struktur / Name | $^{1}H$ NMR (400 MHz, DMSO-$d_6$) δ [ppm] | LC-MS; rt; [M+H$^+$]* |
|---|---|---|---|
| "A108" | 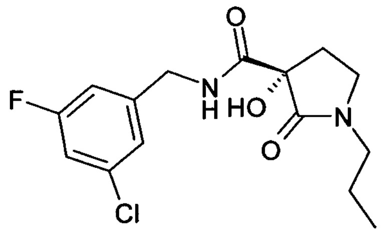<br>(S)-3-Hydroxy-2-oxo-1-propyl-pyrrolidin-3-carbonsäure-3-chlor-5-fluor-benzylamid | 8.57 (t, *J* = 6.4, 1H), 7.26 (dt, *J* = 8.8, 2.2, 1H), 7.18 (s, 1H), 7.13 - 7.01 (m, 1H), 6.44 (s, 1H), 4.36 *(dd, J* = 15.8, 6.8, 1H), 4.21 (dd, *J* = 15.8, 6.0, 1H), 3.27 - 3.16 (m, 1H), 3.11 (dt, *J* = 13.5, 6.8, 1H), 2.47 - 2.39 (m, 1H), 1.94 (ddd, *J* = 13.0, 8.3, 6.9, 1H), 1.47 (h, *J* = 7.3, 2H), 0.80 (t, *J* = 7.4, 3H) | 1) 3.482 min<br>2) 3.504 min<br>[329.0] |
| "A109" | 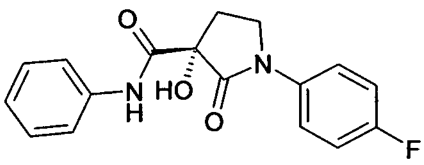<br>(S)-1-(4-Fluor-phenyl)-3-hydroxy-2-oxo-pyrrolidin-3-carbonsäure-phenylamid | 9.79 (s, 1H), 7.80 - 7.69 (m, 4H), 7.34 - 7.24 (m, 4H), 7.08 (dd, *J* = 10.6, 4.2, 1H), 7.02 (s, 1H), 3.89 (dd, *J* = 8.2, 5.5, 2H), 2.76 - 2.61 (m, 1H), 2.19 (dt, *J*= 13.1, 7.7, 1H) | 1) 3.689 min<br>2) 3.653 min<br>[315.0] |
| "A110" | 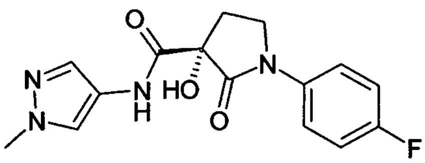<br>(S)-1-(4-Fluor-phenyl)-3-hydroxy-2-oxo-pyrrolidin-3-carbonsäure-(1-methyl-1 H-pyrazol-4-yl)-amid | 10.11 (s, 1H), 7.90 (s, 1H), 7.73 (dd, *J* = 9.1, 4.9, 2H), 7.59 (s, 1H), 7.25 (t, *J* = 8.9, 2H), 6.87 (s, 1H), 3.88 (dd, *J* = 12.2, 5.5, 2H), 3.76 (s, 3H), 2.72 - 2.58 (m, 2H), 2.23 - 2.07 (m, 1H) | 1)2.516 min<br>2) 2.576 min<br>[319.2] |
| "A111" | 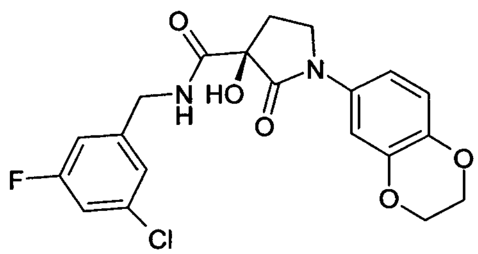<br>(R)-1-(2,3-Dihydro-benzo[1,4]dioxin-6-yl)-3-hydroxy-2-oxo-pyrrolidin-3-carbonsäure-3-chlor-5-fluor-benzylamid | 8.70 (t, *J* = 6.4, 1H), 7.28 (dd, *J* = 4.6, 2.4, 1H), 7.26 (d, *J* = 3.5, 1H), 7.20 (s, 1H), 7.11 (d, *J* = 2.5, 1H), 7.09 *(d, J* = 2.6, 1H), 6.86 (d, *J* = 8.8, 1H), 6.72 (s, 1H), 4.37 (dd, *J* = 15.8, 6.8, 1H), 4.27 - 4.18 (m, 5H), 3.84 - 3.72 (m, 2H), 2.55 (dd, *J* = 13.1, 6.1, 1H), 2.07 (dt, *J* = 13.0, 7.6, 1H) | 1) 4.069 min<br>2) 4.025 min<br>[421.0] |
| "A112" | 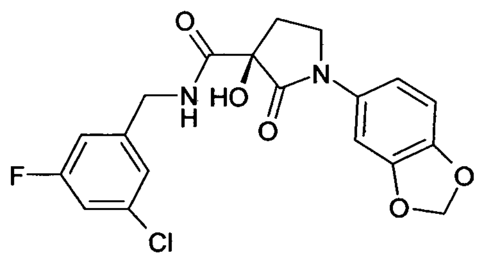<br>(R)-1-Benzo[1,3]dioxol-5-yl-3-hydroxy-2-oxo-pyrrolidin-3-carbonsäure-3-chlor-5-fluor-benzylamid | 8.71 (t, *J* = 6.3, 1H), 7.40 (d, J = 2.1, 1H), 7.27 (d, *J* = 8.7, 1H), 7.20 (s, 1H), 7.09 (d, *J* = 9.4, 1H), 7.04 (dd, *J* = 8.5, 2.1, 1H), 6.94 (d, *J* = 8.5, 1H), 6.74 (s, 1H), 6.02 (s, 2H), 4.37 (dd, *J* = 15.7, 6.7, 1H), 4.23 (dd, *J* = 15.7, 6.0, 1H), 3.79 (t, *J* = 6.8, 2H), 2.63 - 2.52 (m, 1H), 2.15 - 2.02 (m, 1H) | 1) 4.060 min<br>2) 4.199 min<br>[407.0] |

(fortgesetzt)

| Nr. | Struktur / Name | $^1$H NMR (400 MHz, DMSO-$d_6$) $\delta$ [ppm] | LC-MS; rt; [M+H$^+$]* |
|---|---|---|---|
| "A113" | 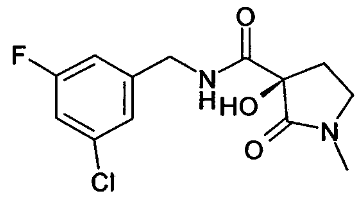<br>(R)-3-Hydroxy-1-methyl-2-oxo-pyrrolidin-3-carbonsäure-3-chlor-5-fluor-benzylamid | 8.59 (t, *J* = 6.3, 1H), 7.26 (dt, *J* = 8.8, 2.2, 1H), 7.19 (s, 1H), 7.09 (d, *J* = 9.7, 1H), 6.46 (s, 1H), 4.36 *(dd, J* = 15.8, 6.8, 1H), 4.21 (dd, *J* = 15.8, 6.0, 1H), 3.37 - 3.28 (m, 5H), 2.47 - 2.41 (m, 1H), 2.01 - 1.85 (m, 1H) | 1)2.867 min<br>2) 2.950 min<br>[301.0] |
| "A114" | 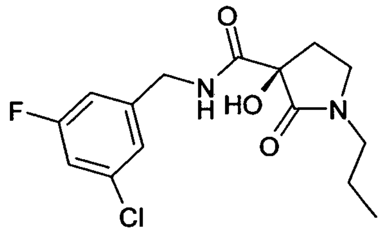<br>(R)-3-Hydroxy-2-oxo-1-propyl-pyrrolidin-3-carbonsäure-3-chlor-5-fluor-benzylamid | 8.57 (t, *J* = 6.4, 1H), 7.26 (dt, *J* = 8.8, 2.2, 1H), 7.18 (s, 1H), 7.08 (d, *J* = 9.7, 1H), 6.44 (s, 1H), 4.36 (dd, *J* = 15.8, 6.8, 1H), 4.21 (dd, *J* = 15.8, 6.0, 1H), 3.26 - 3.17 (m, 1H), 3.11 (dt, *J* = 13.4, 6.8, 1H), 2.47 - 2.39 (m, 1H), 2.02 - 1.86 (m, 1H), 1.47 (h, *J* = 7.3, 2H), 0.80 (t, *J* = 7.4, 3H) | 1) 3.482 min<br>2) 3.505 min<br>[329.0] |
| "A115" | 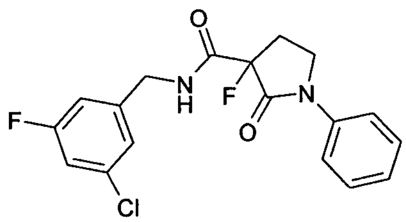<br>3-Fluor-2-oxo-1-phenyl-pyrrolidin-3-carbonsäure-3-chlor-5-fluor-benzylamid | 9.26 (t, *J* = 5.6, 1H), 7.70 (dd, *J* = 8.7, 1.0, 2H), 7.49 - 7.41 (m, 2H), 7.30 (dt, *J* = 8.8, 2.1, 1H), 7.28-7.23 (m, 1H), 7.21 (d, *J* = 4.1, 1H), 7.10 (d, *J* = 9.6, 1H), 4.44 (dd, *J* = 15.8, 6.5, 1H), 4.29 (dd, *J* = 15.8, 5.9, 1H), 4.05 - 3.89 (m, 2H), 2.79 (tdd, *J* = 14.1, 7.5, 3.8, 1H), 2.57 - 2.39 (m, 7H) | 1) 4.729 min<br>2) 4.799 min<br>[365.0] |
| "A116" | 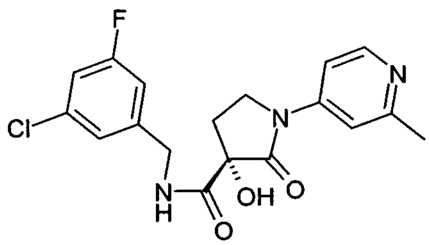<br>(S)-3-Hydroxy-1-(2-methyl-pyridin-4-yl)-2-oxo-pyrrolidin-3-carbonsäure-3-chlor-5-fluor-benzylamid | | 1)2.958 min<br>2)2.911 min<br>[378.0] |
| "A117" | 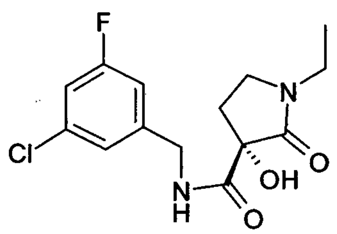<br>(S)-1-Ethyl-3-hydroxy-2-oxo-pyrrolidin-3-carbonsäure-3-chlor-5-fluor-benzylamid | | 1) 3.123 min<br>2) 3.218 min<br>[315.0] |

(fortgesetzt)

| Nr. | Struktur / Name | $^1H$ NMR (400 MHz, DMSO-$d_6$) δ [ppm] | LC-MS; rt; [M+H$^+$]* |
|---|---|---|---|
| "A118" | 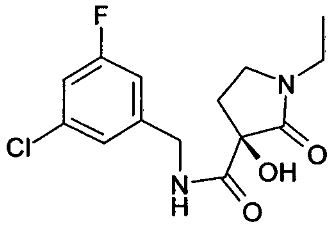 (R)-1-Ethyl-3-hydroxy-2-oxo-pyrrolidin-3-carbonsäure-3-chlor-5-fluor-benzylamid | | 1) 3.122 min 2) 3.218 min [315.0] |
| "A119" | 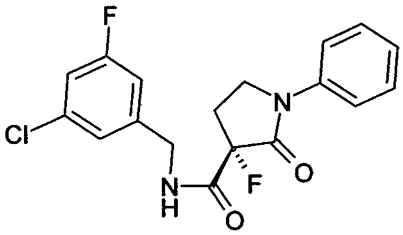 (S)-3-Fluor-2-oxo-1-phenyl-pyrrolidin-3-carbonsäure-3-chlor-5-fluor-benzylamid | | 1) 4.729 min 2) 4.819 min [365.0] |
| "A120" | 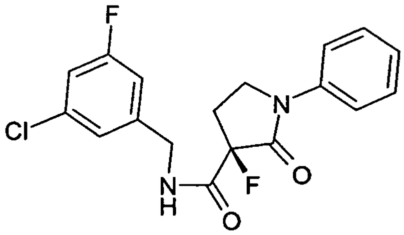 (R)-3-Fluor-2-oxo-1-phenyl-pyrrolidin-3-carbonsäure-3-chlor-5-fluor-benzylamid | | 1) 4.718 min 2) 4.799 min [365.0] |
| | | | |

Analog werden die nachstehenden Verbindungen hergestellt:

(S)-3-Hydroxy-1-isobutyl-2-oxo-pyrrolidin-3-carbonsäure-3-chlor-5-fluor-benzylamid ("A121")

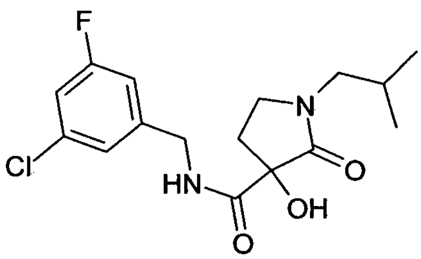

,

(S)-1-(2-Amino-1-methyl-ethyl)-3-hydroxy-2-oxo-pyrrolidin-3-carbonsäure-3-chlor-5-fluor-benzylamid ("A122")

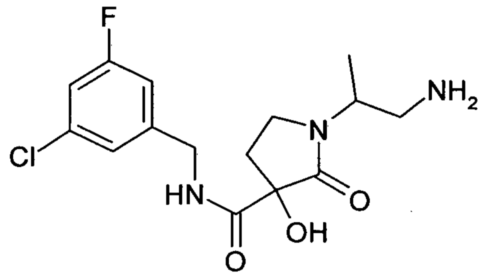

,

(S)-1-(2-Dimethylamino-1-methyl-ethyl)-3-hydroxy-2-oxo-pyrrolidin-3-carbonsäure-3-chlor-5-fluor-benzylamid ("A123")

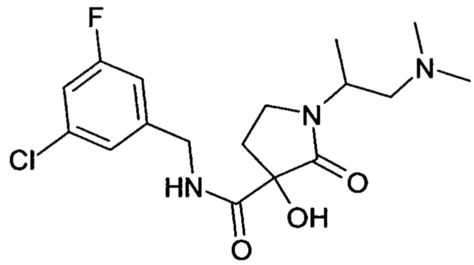

,

(S)-1-(2-Amino-ethyl)-3-hydroxy-2-oxo-pyrrolidin-3-carbonsäure-3-chlor-5-fluor-benzylamid ("A124")

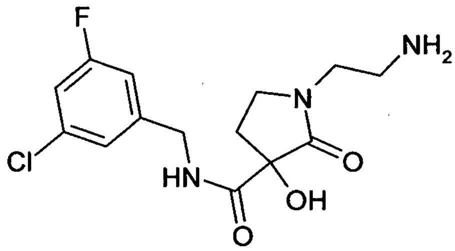

,

(S)-1-(2-Dimethylamino-ethyl)-3-hydroxy-2-oxo-pyrrolidin-3-carbonsäure-3-chlor-5-fluor-benzylamid ("A125")

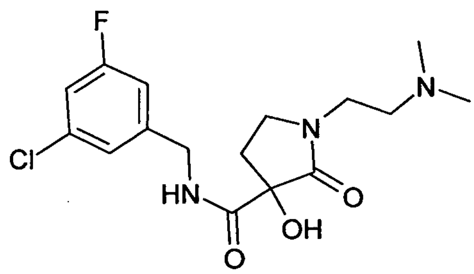

,

(S)-1-(3-Dimethylamino-propyl)-3-hydroxy-2-oxo-pyrrolidin-3-carbonsäure-3-chlor-5-fluor-benzylamid ("A126")

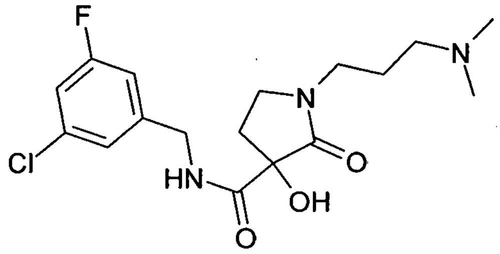

,

(S)-3-Hydroxy-1-(3-hydroxy-propyl)-2-oxo-pyrrolidin-3-carbonsäure-3-chlor-5-fluor-benzylamid ("A127")

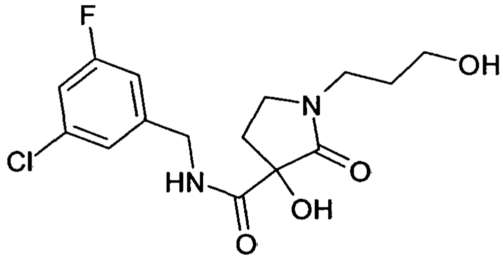

,

(S)-3-Hydroxy-1-(3-methoxy-propyl)-2-oxo-pyrrolidin-3-carbonsäure-3-chlor-5-fluor-benzylamid ("A128")

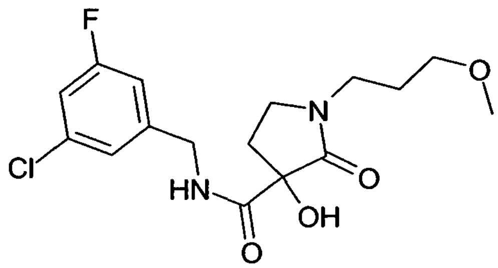

,

(S)-3-Hydroxy-1-(2-methoxy-ethyl)-2-oxo-pyrrolidin-3-carbonsäure-3-chlor-5-fluor-benzylamid ("A129")

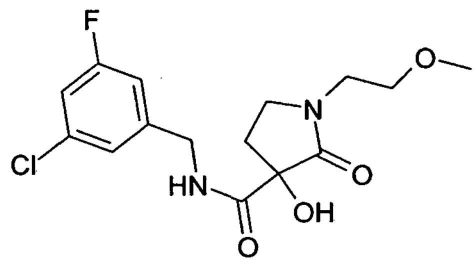

,

(S)-1-(2-Formylamino-ethyl)-3-hydroxy-2-oxo-pyrrolidin-3-carbonsäure-3-chlor-5-fluor-benzylamid ("A130")

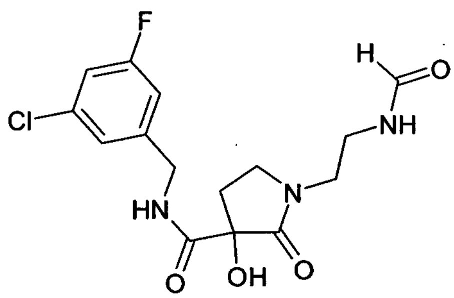

,

(S)-1-(2-Acetylamino-ethyl)-3-hydroxy-2-oxo-pyrrolidin-3-carbonsäure-3-chlor-5-fluor-benzylamid ("A131")

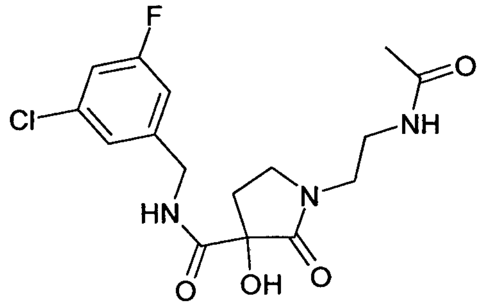

,

(S)-1-[1-(Acetylamino-methyl)-cyclopropyl]-3-hydroxy-2-oxo-pyrrolidin-3-carbonsäure-3-chlor-5-fluor-benzylamid ("A132")

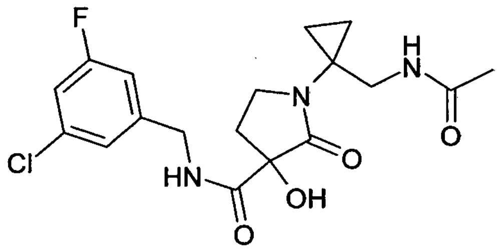

,

(S)-3-Hydroxy-2-oxo-1-(2-ureido-ethyl)-pyrrolidin-3-carbonsäure-3-chlor-5-fluor-benzylamid ("A133")

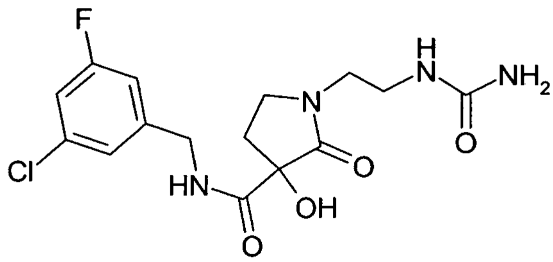

,

(S)-1-(1H-Benzimidazol-5-yl)-3-hydroxy-2-oxo-pyrrolidin-3-carbonsäure-3-chlor-5-fluor-benzylamid ("A134")

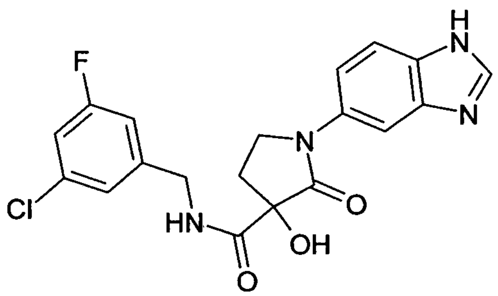

,

(S)-3-Hydroxy-2-oxo-1-(2-oxo-2,3-dihydro-1H-indol-5-yl)-pyrrolidin-3-carbonsäure-3-chlor-5-fluor-benzylamid ("A135")

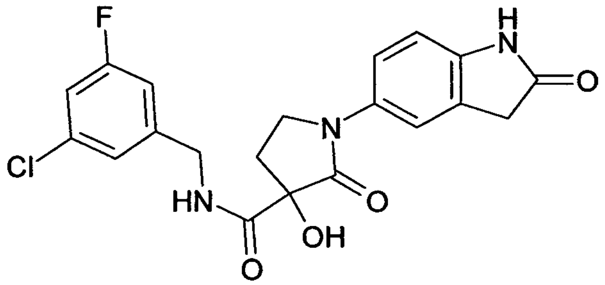

,

(S)-3-Hydroxy-2-oxo-1-((S)-2,2,2-trifluor-1-formylaminomethyl-ethyl)-pyrrolidin-3-carbonsäure-3-chlor-5-fluor-benzylamid ("A136")

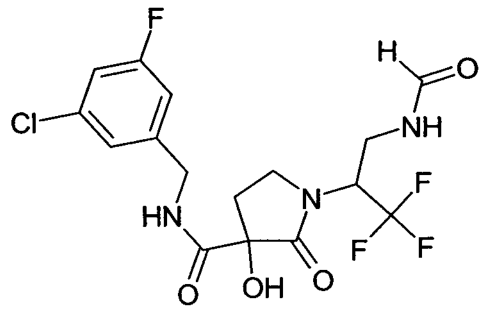

,

1-(3-Carbamoyl-phenyl)-3-hydroxy-2-oxo-pyrrolidine-3-carbonsäure-3-chlor-5-fluor-benzylamid ("A137")

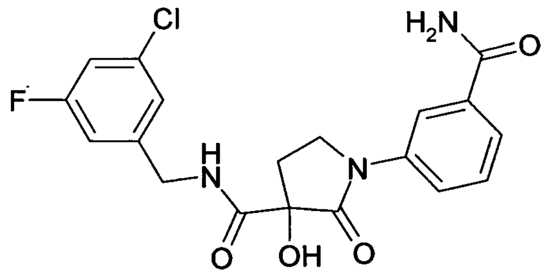

,

1-(2-Carbamoyl-phenyl)-3-hydroxy-2-oxo-pyrrolidine-3-carbonsäure-3-chlor-5-fluor-benzylamid ("A138")

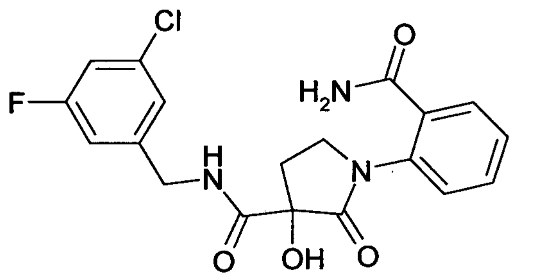

,

{2-[(S)-3-(3-Chlor-5-fluor-benzylcarbamoyl)-3-hydroxy-2-oxo-pyrrolidin-1-yl]-ethyl}-carbaminsäure-tert.-butylester ("A140")

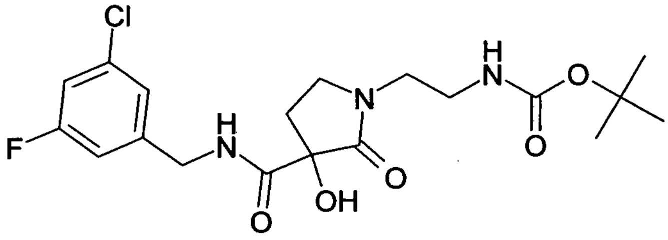

,

1-(3-Cyan-phenyl)-3-hydroxy-2-oxo-pyrrolidine-3-carbonsäure-3-chlor-5-fluor-benzylamid ("A141")

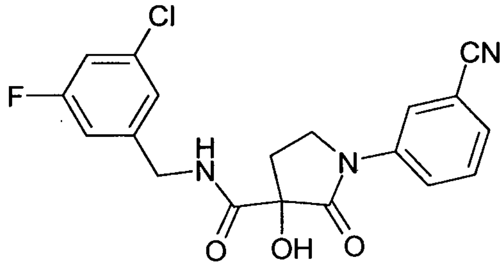

,

1-(2-Cyan-phenyl)-3-hydroxy-2-oxo-pyrrolidine-3-carbonsäure-3-chlor-5-fluor-benzylamid ("A142"),

1-(4-Cyan-phenyl)-3-hydroxy-2-oxo-pyrrolidine-3-carbonsäure-3-chlor-5-fluor-benzylamid ("A143"),

1-(3-Amino-3-oxo-propyl)-N-[(3-chlor-5-fluor-phenyl)methyl]-3-hydroxy-2-oxo-pyrrolidin-3-carboxamid ("A144")

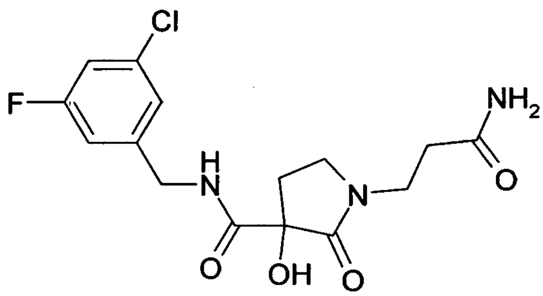

,

N-[(3-Chlor-5-fluor-phenyl)methyl]-3-hydroxy-1-[3-(methylamino)-3-oxo-propyl]-2-oxo-pyrrolidin-3-carboxamid ("A145")

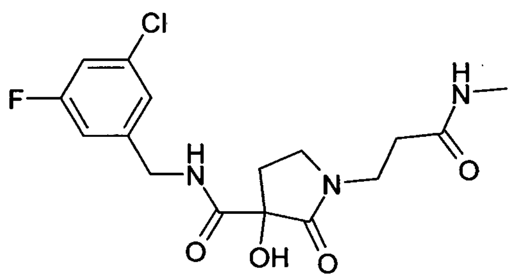

,

1-(4-Amino-4-oxo-butyl)-N-[(3-chlor-5-fluor-phenyl)methyl]-3-hydroxy-2-oxo-pyrrolidin-3-carboxamid ("A146"),

N-[(3-Chlor-5-fluor-phenyl)methyl]-3-hydroxy-1-[4-(methylamino)-4-oxo-butyl]-2-oxo-pyrrolidin-3-carboxamid ("A147"),

1-(2-Cyan-ethyl)-3-hydroxy-2-oxo-pyrrolidin-3-carbonsäure-3-chlor-5-fluor-benzylamid ("A148")

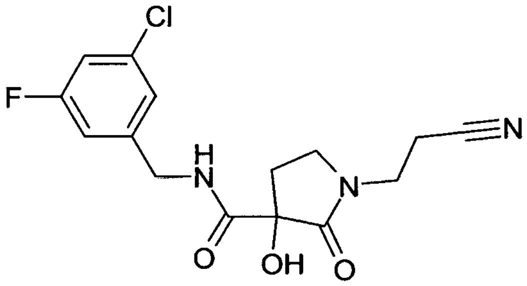

,

1-(3-Cyan-propyl)-3-hydroxy-2-oxo-pyrrolidin-3-carbonsäure-3-chlor-5-fluor-benzylamid ("A149")

3-Hydroxy-1-(6-methyl-pyridin-3-yl)-2-oxo-pyrrolidin-3-carbonsäure-3-chlor-5-fluor-benzylamid ("A152")

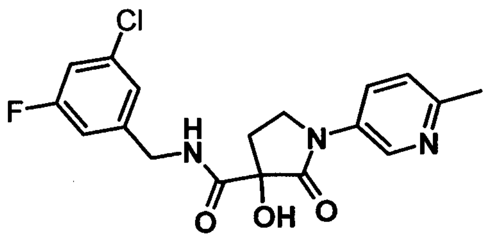

,

3-Hydroxy-2-oxo-pyrrolidin-1,3-dicarbonsäure-3-(3-chlor-5-fluor-benzylamid)-1-ethylamid ("A153")

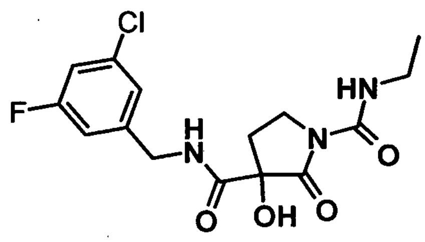

,

3-Hydroxy-2-oxo-pyrrolidin-1,3-dicarbonsäure-3-(3-chlor-5-fluor-benzylamid)-1-phenylamid ("A154")

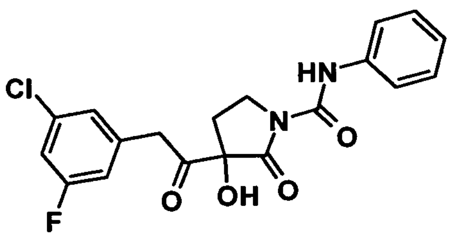

,

3-Azido-N-[(3-chlor-5-fluor-phenyl)-methyl]-2-oxo-1-phenyl-pyrrolidin-3-carboxamid ("A155")

3-Hydroxy-2-oxo-1-p-tolyl-pyrrolidin-3-carbonsäure-3-chlor-5-fluor-benzylamid ("A156")

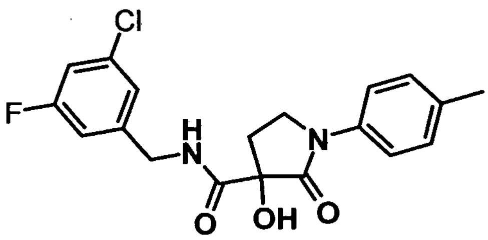

,

1-Carbamoylmethyl-3-hydroxy-2-oxo-pyrrollidin-3-carbonsäure-3-chlor-5-fluor-benzylamid ("A157")

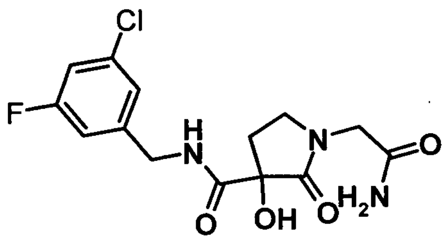

,

(3S)-1-[(1 S)-2-Amino-1-methyl-2-oxo-ethyl]-N-[(3-chlor-5-fluor-phenyl)methyl]-3-hydroxy-2-oxo-pyrrolidin-3-carboxamid ("A158")

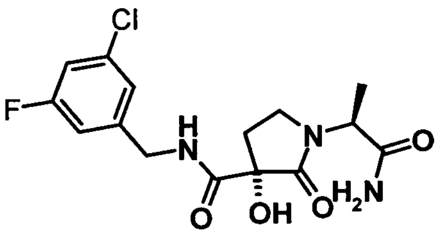

,

(3S)-1-[(1R)-2-Amino-1-methyl-2-oxo-ethyl]-N-[(3-chlor-5-fluor-phenyl)methyl]-3-hydroxy-2-oxo-pyrrolidin-3-carboxamid ("A159"),

3-Hydroxy-2-oxo-1-(4-fluorphenyl)-pyrrolidin-3-carbonsäure-3-trifluormethyl-5-fluor-benzylamid ("A160"),

3-Hydroxy-2-oxo-1-phenyl-pyrrolidin-3-carbonsäure-3,5-(bis-trifluormethyl)-benzylamid ("A161").

Analog werden die nachstehenden Verbindungen erhalten

| Nr. | Struktur / Name | $^1H$ NMR (400 MHz, DMSO-$d_6$) δ [ppm] | LC-MS; rt; [M+H$^+$]* |
|---|---|---|---|
| "A162" | 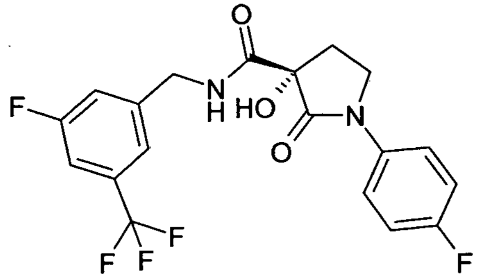 (S)-1-(4-Fluor-phenyl)-3-hydroxy-2-oxo-pyrrolidin-3-carbonsäure-3-fluor-5-trifluormethyl-benzylamid | 8.82 (t, *J* = 6.3, 1H), 7.76 - 7.70 (m, 2H), 7.53 (s, 1H), 7.51 (s, 2H), 7.43 (d, *J* = 9.5, 1H), 7.28 - 7.22 (m, 2H), 6.79 (s, 1H), 4.46 (dd, *J* = 15.8, 6.7, 1H), 4.32 (dd, *J* = 15.9, 6.0, 1H), 3.84 (t, *J* = 6.8, 2H), 2.63 - 2.54 (m, 1H), 2.12 (dt, *J* = 13.1, 7.6, 1H). | 4.57 min [415] |
| "A163" | 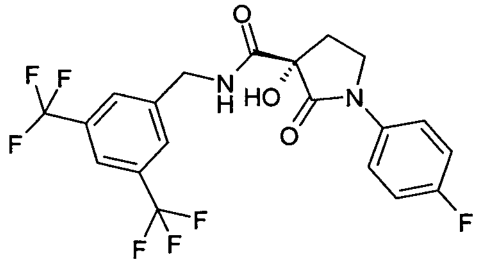 (S)-1-(4-Fluor-phenyl)-3-hydroxy-2-oxo-pyrrolidin-3-carbonsäure-3,5-bis-trifluormethyl-benzylamid | 8.90 (t, *J* = 6.3, 1H), 7.96 (d, *J* = 4.1, 3H), 7.76 - 7.70 (m, 2H), 7.30 - 7.20 (m, 2H), 6.81 (s, 1H), 4.55 (dd, *J* = 15.9, 6.7, 1H), 4.40 (dd, *J* = 16.0, 6.0, 1H), 3.90 - 3.79 (m, 2H), 2.64 - 2.54 (m, 1H), 2.12 (dt, *J* = 13.0, 7.5, 1H). | 5.09 min [465] |
| "A164" | 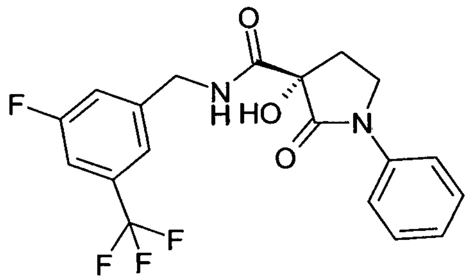 (S)-3-Hydroxy-2-oxo-1-phenyl-pyrrolidin-3-carbonsäure-3-fluor-5-trifluormethyl-benzylamid | 8.81 (t, *J* = 6.3, 1H), 7.70 (t, *J* = 1.6, 1H), 7.68 (d, *J* = 1.0, 1H), 7.52 (d, *J* = 7.8, 2H), 7.43 (d, *J* = 8.8, 2H), 7.21 - 7.12 (m, 1H), 6.78 (s, 1H), 4.47 (dd, *J* = 16.0, 6.8, 1H), 4.32 (dd, *J* = 15.8, 6.0, 1H), 3.86 (dd, *J* = 8.3, 5.7, 2H), 2.58 (dt, *J* = 6.7, 5.8, 1H), 2.12 (dt, *J* = 13.1, 7.6, 1H). | 4.48 min; [397] |

(fortgesetzt)

| Nr. | Struktur / Name | $^1H$ NMR (400 MHz, DMSO-$d_6$) δ [ppm] | LC-MS; rt; [M+H+]* |
|---|---|---|---|
| "A165" | 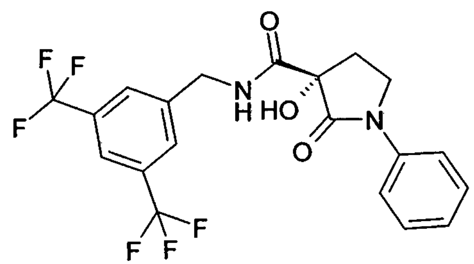 (S)-3-Hydroxy-2-oxo-1-phenyl-pyrrolidin-3-carbonsäure-3,5-bis-trifluormethyl-benzylamid | 8.90 (t, *J* = 6.4, 1H), 7.96 (d, *J* = 6.2, 3H), 7.69 (dd, *J* = 8.7, 1.0, 2H), 7.40 (dd, *J* = 10.7, 5.3, 2H), 7.17 (t, *J* = 7.4, 1H), 6.80 (s, 1H), 4.55 (dd, *J* = 15.9, 6.6, 1 H), 4.40 (dd, *J* = 15.9, 5.9, 1H), 3.92 - 3.77 (m, 2H), 2.58 (ddd, *J* = 12.0, 7.4, 4.4, 1H), 2.12 (dt, *J* = 13.0, 7.6, 1H). | 5.02 min [447] |
| "A166" | 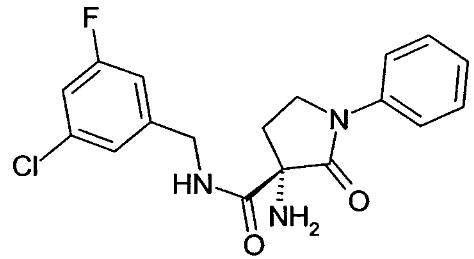 (S)-3-Amino-2-oxo-1-phenyl-pyrrolidin-3-carbonsäure-3-chlor-5-fluor-benzylamid | 8.74 (s, 1H), 7.69 (d, *J* = 8.0, 2H), 7.39 (t, *J* = 7.9, 2H), 7.28 (d, *J* = 8.7, 1H), 7.17 (dd, *J* = 14.5, 7.2, 2H), 7.09 (d, *J* = 9.3, 1H), 4.35 (dd, *J* = 15.7, 6.2, 1H), 4.26 (dd, *J* = 15.7, 5.8, 1H), 3.89 - 3.78 (m, 2H), 2.57 (br. s, 2H), 2.06 (dt, *J* = 12.7, 7.8, 1H). | 3.53 min [362] |
| "A167" | 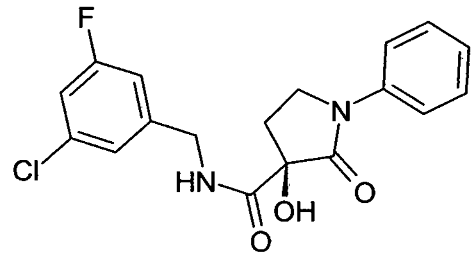 (R)-3-Hydroxy-2-oxo-1-phenyl-pyrrolidin-3-carbonsäure-3-chlor-5-fluor-benzylamid | 8.73 (t, *J* = 6.3, 1H), 7.69 (dd, *J* = 8.7, 1.0, 2H), 7.43 - 7.38 (m, 2H), 7.27 (dt, *J* = 8.8,2.1, 1H), 7.21-7.15 (m, 2H), 7.12 - 7.08 (m, 1H), 6.77 (s, 1H), 4.38 (dd, *J* = 15.7, 6.7, 1H), 4.24 (dd, *J* = 15.7, 6.1, 1H), 3.89 - 3.83 (m, 2H), 2.63 - 2.54 (m, 1H), 2.12 (dt, *J* = 13.1, 7.7, 1H). | 4.15 min [363] |

(fortgesetzt)

| Nr. | Struktur / Name | $^1$H NMR (400 MHz, DMSO-$d_6$) δ [ppm] | LC-MS; rt; [M+H$^+$]* |
|---|---|---|---|
| "A168" | 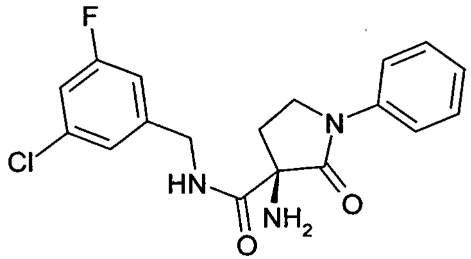 (R)-3-Amino-2-oxo-1-phenyl-pyrrolidin-3-carbonsäure-3-chlor-5-fluor-benzylamid | 8.77 (s, 1H), 7.69 (d, *J* = 7.8, 2H), 7.40 (t, *J* = 8.0, 2H), 7.30 - 7.25 (m, 1H), 7.22 - 7.13 (m, 2H), 7.09 (d, *J* = 9.3, 1H), 4.35 (dd, *J* = 15.6, 6.0, 1H), 4.27 (dd, *J* = 15.9, 5.9, 1H), 3.89 - 3.81 (m, 2H), 2.14 - 2.03 (m, 1H). | 3.53 min [362] |
| "A169" | 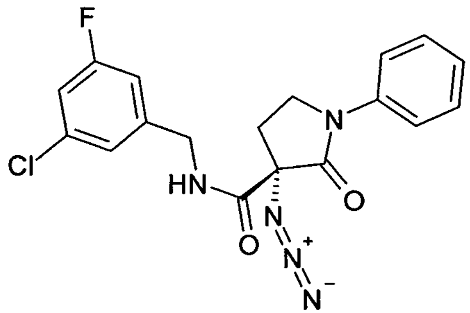 (S)-3-Azido-2-oxo-1-phenyl-pyrrolidin-3-carbonsäure-3-chlor-5-fluor-benzylamid | 9.13 (s, 1H), 7.69 (d, *J* = 7.7, 2H), 7.46 - 7.40 (m, 2H), 7.31 (d, *J* = 9.1, 1H), 7.26 - 7.18 (m, 2H), 7.09 (d, *J* = 8.6, 1H), 4.36 (dd, *J* = 11.5, 6.2, 2H), 3.92 (d, *J* = 7.5, 3H), 1.23 (s, 3H). | 5.11 min [388] |
| "A170" | 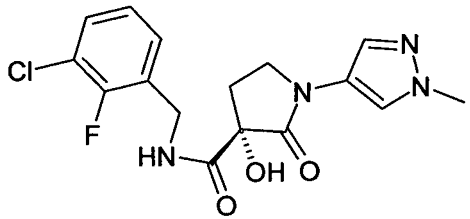 (S)-3-Hydroxy-1-(1-methyl-1 H-pyrazol-4-yl)-2-oxo-pyrrolidin-3-carbonsäure-3-chlor-2-fluor-benzylamid | 8.62 (t, *J* = 6.0, 1H), 8.01 (s, 0H), 7.63 (s, 1H), 7.45 (t, *J* = 7.0, 1H), 7.30 (t, *J* = 6.7, 1H), 7.18 (t, *J* = 7.9, 1H), 6.69 (s, 1H), 4.39 (dd, *J* = 15.2, 6.1, 1H), 4.31 (dd, *J* = 15.6, 6.2, 1H), 3.68 (dd, *J* = 14.7, 5.9, 2H), 2.59 (dd, *J* = 8.9, 4.3, 1H), 2.17 - 2.07 (m, 1H). | 3.12 min [367] |

(fortgesetzt)

| Nr. | Struktur / Name | $^1H$ NMR (400 MHz, DMSO-$d_6$) δ [ppm] | LC-MS; rt; [M+H$^+$]* |
|---|---|---|---|
| "A171" | 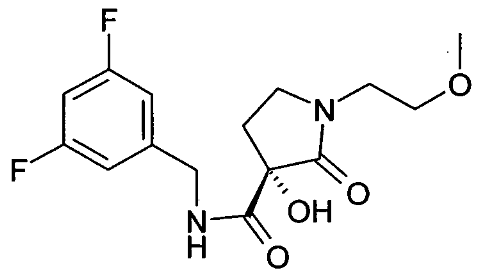 (S)-3-Hydroxy-1-(2-methoxy-ethyl)-2-oxo-pyrrolidin-3-carbonsäure-3,5-difluor-benzylamid | 8.57 (t, *J* = 6.3, 1H), 7.09 - 7.02 (m, 1H), 6.97 (d, *J* = 6.7, 1H), 6.47 (s, 1H), 4.37 (dd, *J* = 15.9, 6.8, 1H), 4.21 (dd, *J* = 15.9, 5.9, 1H), 3.39 (ddd, *J* = 13.4, 10.5, 5.4, 4H), 3.23 (s, 3H), 2.48 - 2.38 (m, 1H), 1.98 - 1.89 (m, 1H). | 2.70 min [329] |
| "A172" | 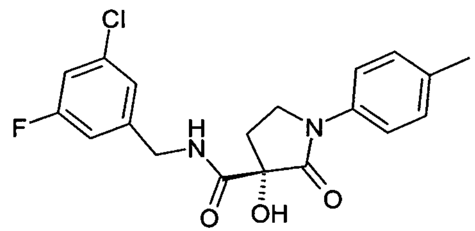 (S)-3-Hydroxy-2-oxo-1-p-tolyl-pyrrolidin-3-carbonsäure-3-chlor-5-fluor-benzylamid | 8.72 (t, *J* = 6.4, 1H), 7.60 - 7.55 (m, 2H), 7.27 (dt, *J* = 8.8, 2.2, 1H), 7.20 (dd, *J* = 4.9, 3.3, 3H), 7.10 (d, *J* = 9.7, 1H), 6.73 (s, 1H), 4.37 (dd, *J* = 15.8, 6.7, 1H), 4.23 (dd, *J* = 15.8, 6.0, 1H), 3.82 (dd, *J* = 7.5, 6.1, 2H), 2.63 - 2.54 (m, 1H), 2.28 (s, 3H), 2.10 (dt, *J* = 13.0, 7.6, 1H). | 4.42 min [377.3] |
| "A173" | 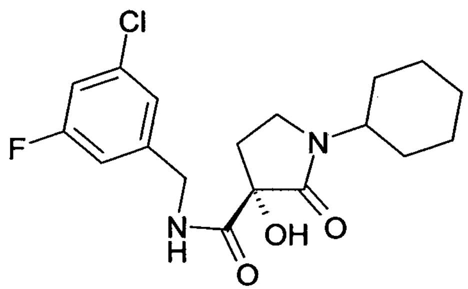 (S)-1-Cyclohexyl-3-hydroxy-2-oxo-pyrrolidin-3-carbonsäure-3-chlor-5-fluor-benzylamid | 8.55 (t, *J* = 6.4, 1H), 7.26 (dt, *J* = 8.8, 2.2, 1H), 7.18 (s, 1H), 7.09 (d, *J* = 9.7, 1H), 6.42 (s, 1H), 4.35 (dd, *J* = 15.8, 6.7, 1H), 4.20 (dd, *J* = 15.8, 6.0, 1H), 3.68 (ddd, *J* = 11.8, 8.0, 3.8, 1H), 3.32 - 3.21 (m, 3H), 2.46 - 2.36 (m, 1H), 1.98-1.83 (m, 1H), 1.80 - 1.67 (m, 2H), 1.66 - 1.50 (m, 3H), 1.50 - 1.21 (m, 4H), 1.19 - 0.99 (m, 2H). | 4.19 min [369.3] |

(fortgesetzt)

| Nr. | Struktur / Name | $^1H$ NMR (400 MHz, DMSO-$d_6$) δ [ppm] | LC-MS; rt; $[M+H^+]$* |
|---|---|---|---|
| "A174" | 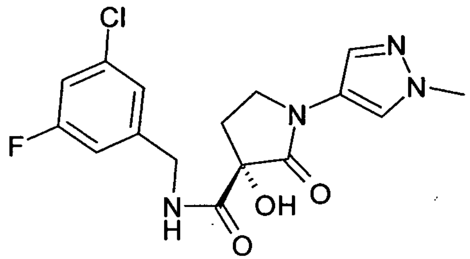 (S)-3-Hydroxy-1-(1-methyl-1H-pyrazol-4-yl)-2-oxo-pyrrolidin-3-carbonsäure-3-chlor-5-fluor-benzylamid | 8.71 (t, *J* = 6.4, 1H), 8.01 (s, 1H), 7.63 (d, *J* = 0.6, 1H), 7.27 (dt, *J* = 8.8, 2.2, 1H), 7.19 (s, 1H), 7.09 (d, *J* = 9.6, 1H), 6.70 (s, 1H), 4.36 (dd, *J* = 15.8, 6.7, 1H), 4.23 (dd, *J* = 15.7, 6.1, 1H), 3.75 - 3.62 (m, 2H), 2.60 (ddd, *J* = 12.2, 7.7, 4.3, 1H), 2.18 - 2.05 (m, 1H). | 3.17 min [367] |
| "A175" | 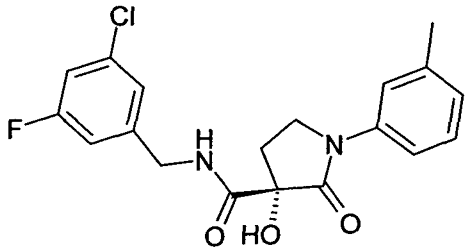 (S)-3-Hydroxy-2-oxo-1-m-tolyl-pyrrolidin-3-carbonsäure-3-chlor-5-fluor-benzylamid | 8.72 (t, *J* = 6.3, 1H), 7.50 (d, *J* = 6.5, 2H), 7.31 - 7.24 (m, 2H), 7.20 (s, 1H), 7.10 (d, *J* = 9.7, 1H), 7.00 (d, *J* = 7.3, 1H), 6.74 (s, 1H), 4.38 (dd, *J* = 15.8, 6.7, 1H), 4.23 (dd, *J* = 15.7, 6.0, 1H), 3.83 (dd, *J* = 8.0, 5.6, 2H), 2.63 - 2.53 (m, 1H), 2.11 (dt, *J* = 13.0, 7.6, 1H). | 4.45 min [377] |
| "A176" | 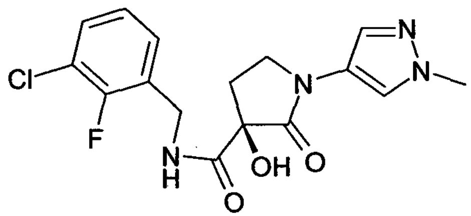 (R)-3-Hydroxy-1-(1-methyl-1H-pyrazol-4-yl)-2-oxo-pyrrolidin-3-carbonsäure-3-chlor-2-fluor-benzylamid | 8.62 (t, *J* = 6.1, 1H), 8.01 (s, 1H), 7.63 (s, 1H), 7.45 (t, *J* = 7.4, 1H), 7.30 (t, *J* = 6.9, 1H), 7.18 (t, *J* = 7.9, 1H), 6.69 (s, 1H), 4.39 (dd, *J* = 15.6, 6.8, 1H), 4.31 (dd, *J* = 15.6, 6.2, 1H), 3.82 (s, 3H), 3.74-3.60 (m, 2H), 2.64 - 2.57 (m, 1H), 2.13 (dt, *J* = 13.0, 7.5, 1H). | 3.11 min [367] |

(fortgesetzt)

| Nr. | Struktur / Name | $^1$H NMR (400 MHz, DMSO-$d_6$) δ [ppm] | LC-MS; rt; [M+H$^+$]* |
|---|---|---|---|
| "A177" | 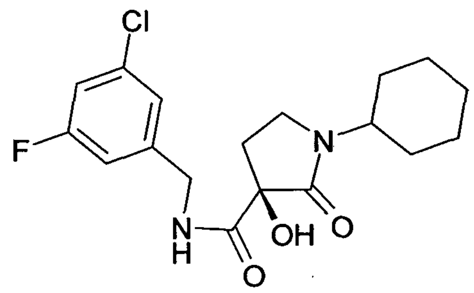 (R)-1-Cyclohexyl-3-hydroxy-2-oxo-pyrrolidin-3-carbonsäure-3-chlor-5-fluor-benzylamid | 8.55 (t, *J* = 6.3, 1H), 7.26 (dt, *J* = 8.8, 2.1, 1H), 7.18 (s, 1H), 7.09 (d, *J* = 9.6, 1H), 6.42 (s, 1H), 4.35 (dd, *J* = 15.7, 6.7, 1H), 4.20 (dd, *J* = 15.7, 5.9, 1H), 3.68 (tt, *J* = 11.8, 3.7, 1H), 3.31 - 3.21 (m, 2H), 2.45 - 2.36 (m, 1H), 1.98 -1.84 (m, 1H), 1.80-1.68 (m, 2H), 1.66 - 1.50 (m, 3H), 1.50 - 1.20 (m, 5H), 1.15 - 1.01 (m, 1H). | 4.19 min [369] |
| "A178" | 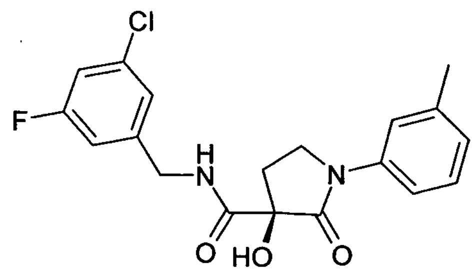 (R)-3-Hydroxy-2-oxo-1-m-tolyl-pyrrolidin-3-carbonsäure-3-chlor-5-fluor-benzylamid | 8.72 (t, *J* = 6.4, 1H), 7.50 (d, *J* = 6.4, 2H), 7.31 - 7.24 (m, 2H), 7.20 (s, 1H), 7.10 (d, *J* = 9.7, 1H), 7.00 (d, *J* = 7.6, 1H), 6.74 (s, 1H), 4.38 (dd, *J* = 15.8, 6.8, 1H), 4.23 (dd, *J* = 15.7, 6.0, 1H), 3.83 (t, *J* = 6.8, 2H), 2.62 - 2.52 (m, 1H), 2.31 (s, 3H), 2.11 (dt, *J* = 13.0, 7.6, 1H). | 4.45 min [377] |
| "A179" | 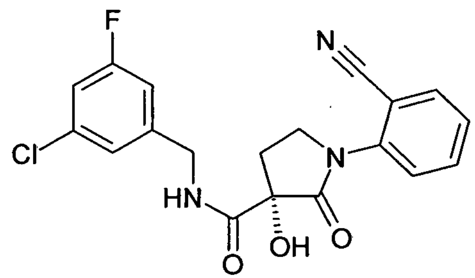 (S)-1-(2-Cyan-phenyl)-3-hydroxy-2-oxo-pyrrolidin-3-carbonsäure-3-chlor-5-fluor-benzylamid | 8.74 (t, *J* = 6.5, 1H), 7.92 (dd, *J* = 8.0, 1.5, 1H), 7.84 - 7.74 (m, 1H), 7.51 (ddd, *J* = 6.3, 3.7, 1.1, 2H), 7.27 (dd, *J* = 8.8, 2.2, 1H), 7.21 (s, 1H), 7.11 (d, *J* = 8.8, 1H), 6.87 (s, 1H), 4.39 (dd, *J* = 15.7, 7.0, 1H), 4.26 (dd, *J* = 15.8, 6.2, 1H), 3.97 - 3.76 (m, 3H), 2.69 - 2.56 (m, 2H), 2.28 - 2.14 (m, 1H). | 3.87 min [388] |

(fortgesetzt)

| Nr. | Struktur / Name | $^1H$ NMR (400 MHz, DMSO-$d_6$) δ [ppm] | LC-MS; rt; [M+H+]* |
|---|---|---|---|
| "A180" | 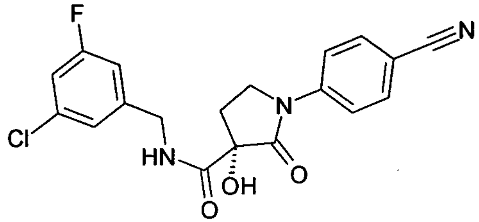 (S)-1-(4-Cyan-phenyl)-3-hydroxy-2-oxo-pyrrolidin-3-carbonsäure-3-chlor-5-fluor-benzylamid | 8.79 (s, 1H), 7.91 (q, *J* = 9.1, 4H), 7.27 (d, *J* = 8.7, 1H), 7.19 (s, 1H), 7.08 (s, 1H), 6.88 (s, 1H), 4.37 (dd, *J* = 15.6, 6.2, 2H), 4.23 (dd, *J* = 15.7, 5.8, 2H), 3.90 (dd, *J* = 11.5, 5.7, 2H), 2.63 - 2.57 (m, 2H), 2.15 (dt, *J* = 11.6, 7.4, 2H). | 4.15 min [386.3] |
| "A181" | 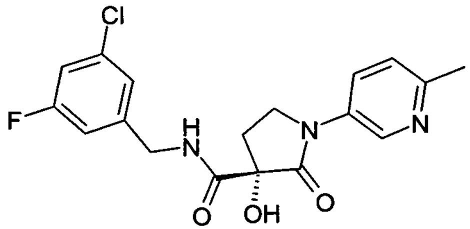 (S)-3-Hydroxy-1-(6-methyl-pyridin-3-yl)-2-oxo-pyrrolidin-3-carbonsäure-3-chlor-5-fluor-benzylamid | 8.76 (q, *J* = 5.2, 1H), 8.01 (dd, *J* = 8.5, 2.7, 1H), 7.32 -7.24 (m, 1H), 7.20 (s, 1H), 7.09 (d, *J* = 8.7, 1H), 6.81 (s, 1H), 4.37 (dd, *J* = 15.8, 6.7, 1H), 4.24 (dd, *J* = 15.7, 6.0, 1H), 3.86 (dd, *J* = 8.5, 5.7, 1H), 2.60 (dt, *J* = 6.8, 5.8, 1H), 2.45 (s, 3H), 2.14 (dt, *J* = 12.9, 7.4, 1H). | 2.88 min [378] |
| "A182" | 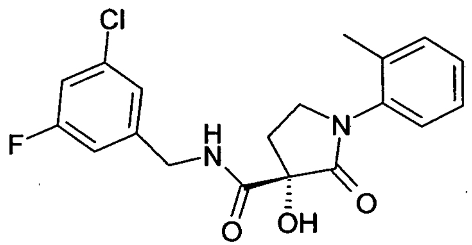 (S)-3-Hydroxy-2-oxo-1-o-tolyl-pyrrolidin-3-carbonsäure-3-chlor-5-fluor-benzylamid | 8.69 (t, *J* = 6.4, 1H), 7.32 - 7.17 (m, 6H), 7.11 (*d, J* = 9.7, 1H), 6.70 (s, 1H), 4.41 (dd, *J* = 15.9, 6.7, 1H), 4.24 (dd, *J* = 15.8, 6.0, 1H), 3.68 (dd, *J* = 8.3, 5.3, 2H), 2.65 - 2.57 (m, 1H), 2.19 (dt, *J* = 13.0, 7.6, 1H), 2.11 (s, 3H). | 4.17 min [377] |

(fortgesetzt)

| Nr. | Struktur / Name | $^1H$ NMR (400 MHz, DMSO-$d_6$) δ [ppm] | LC-MS; rt; $[M+H^+]$* |
|---|---|---|---|
| "A183" | 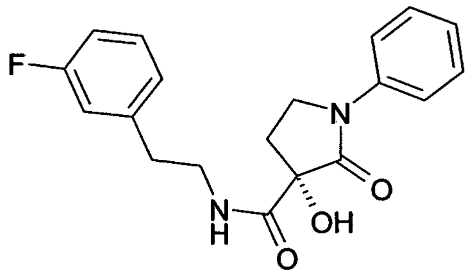 (S)-3-Hydroxy-2-oxo-1-phenyl-pyrrolidin-3-carbonsäure-[2-(3-fluor-phenyl)-ethyl]-amid | 8.02 (t, *J* = 5.9, 1H), 7.69 (dd, *J* = 8.7, 1.0, 2H), 7.44 - 7.37 (m, 2H), 7.35 - 7.28 (m, 1H), 7.17 (t, *J* = 7.4, 1H), 7.09 - 6.97 (m, 3H), 6.62 (s, 1H), 3.86 - 3.79 (m, 2H), 3.42 - 3.34 (m, 1H), 2.77 (t, *J* = 7.3, 2H), 2.47 - 2.37 (m, 2H), 2.05 (dt, *J* = 12.8, 7.9, 1H). | 3.79 min [343.3] |
| "A184" | 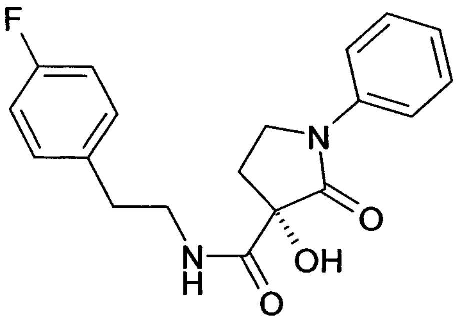 (S)-3-Hydroxy-2-oxo-1-phenyl-pyrrolidin-3-carbonsäure-[2-(4-fluor-phenyl)-ethyl]-amid | 8.00 (t, *J* = 5.8, 1H), 7.72 - 7.66 (m, 2H), 7.43 - 7.37 (m, 2H), 7.23 (dd, *J* = 8.6, 5.7, 2H), 7.17 (t, *J* = 7.4, 1H), 7.08 (t, *J* = 8.9, 2H), 6.61 (s, 1H), 3.86 - 3.67 (m, 2H), 3.31 - 3.13 (m, 2H), 2.73 (t, *J* = 7.4, 2H), 2.47 - 2.41 (m 1H), 2.05 (dt, *J* = 12.8, 7.9, 1H). | 3.78 min [343] |
| "A185" | 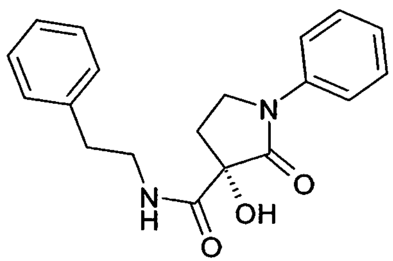 (S)-3-Hydroxy-2-oxo-1-phenyl-pyrrolidin-3-carbonsäure-phenethyl-amid | 8.00 (t, *J* = 6.0, 1H), 7.69 (dd, *J* = 8.7, 1.0, 2H), 7.44 - 7.37 (m, 2H), 7.33 - 7.24 (m, 2H), 7.18 (ddd, *J* = 13.8, 7.8, 1.7, 4H), 6.62 (s, 1H), 3.89 - 3.77 (m, 2H), 3.40 - 3.34 (m, 2H), 2.74 (t, *J* = 7.5, 2H), 2.47 - 2.40 (m, 1H), 2.06 (dt, *J* = 12.9, 7.9, 1H). | 3.64 min [325.3] |

(fortgesetzt)

| Nr. | Struktur / Name | $^1$H NMR (400 MHz, DMSO-$d_6$) δ [ppm] | LC-MS; rt; [M+H$^+$]* |
|---|---|---|---|
| "A186" | 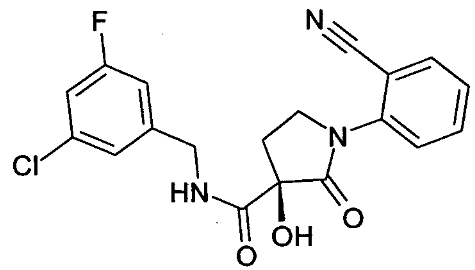 (R)-1-(2-Cyan-phenyl)-3-hydroxy-2-oxo-pyrrolidin-3-carbonsäure-3-chlor-5-fluor-benzylamid | 8.74 (t, *J* = 6.3, 1H), 7.92 (dd, *J* = 8.0, 1.5, 1H), 7.82 - 7.76 (m, 1H), 7.55 - 7.48 (m, 2H), 7.27 (d, *J* = 8.8, 1H), 7.21 (s, 1H), 7.11 (*d, J* = 9.7, 1H), 6.87 (s, 1H), 4.39 (dd, *J* = 15.8, 6.8, 1H), 4.26 (dd, *J* = 15.7, 5.9, 1H), 3.96 - 3.83 (m, 2H), 2.62 (m, 1H), 2.27 - 2.17 (m, 1H). | 3.87 min [388] |
| "A187" | 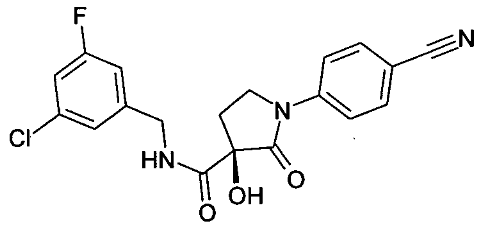 (R)-1-(4-Cyan-phenyl)-3-hydroxy-2-oxo-pyrrolidin-3-carbonsäure-3-chlor-5-fluor-benzylamid | 8.79 (t, *J* = 6.5, 1H), 7.91 (q, *J* = 9.1, 4H), 7.27 (*d, J* = 8.7, 1H), 7.19 (s, 1H), 7.09 (d, *J* = 8.8, 1H), 6.89 (s, 1H), 4.37 (dd, *J* = 15.8, 6.7, 1H), 4.24 (dd, *J* = 15.9, 6.0, 1H), 3.90 (*t, J* = 6.5, 2H), 2.64 - 2.56 (m, 1H), 2.20 - 2.10 (m, 1H). | 4.14 min [386.3] |
| "A188" | 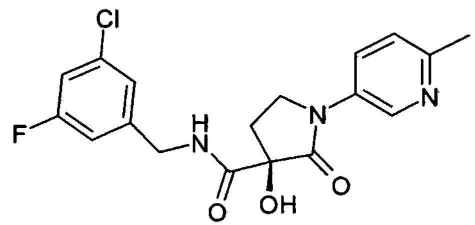 (R)-3-Hydroxy-1-(6-methyl-pyridin-3-yl)-2-oxo-pyrrolidin-3-carbonsäure-3-chlor-5-fluor-benzylamid | 8.76 (q, *J* = 5.2, 1H), 8.01 (dd, *J* = 8.5, 2.7, 1H), 7.32 -7.24 (m, 1H), 7.20 (s, 0H), 7.09 (d, *J* = 8.9, 1H), 6.81 (s, 1H), 4.37 (dd, *J* = 15.8, 6.7, 1H), 4.24 (dd, *J* = 15.7, 6.1, 1H), 3.86 (dd, *J* = 8.5, 5.7, 1H), 2.60 (dt, *J* = 6.8, 5.7, 1H), 2.45 (s, 3H), 2.14 (dt, *J*= 12.9, 7.4, 1H). | 2.88 min [378] |

(fortgesetzt)

| Nr. | Struktur / Name | $^1H$ NMR (400 MHz, DMSO-$d_6$) δ [ppm] | LC-MS; rt; [M+H$^+$]* |
|---|---|---|---|
| "A189" | 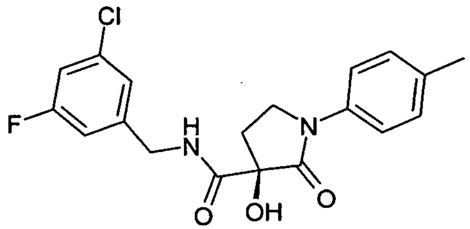 (R)-3-Hydroxy-2-oxo-1-p-tolyl-pyrrolidin-3-carbonsäure-3-chlor-5-fluor-benzylamid | 8.72 (t, $J$ = 6.3, 1H), 7.57 (d, $J$ = 8.5, 2H), 7.27 (dt, $J$ = 8.8, 2.1, 1H), 7.23 - 7.18 (m, 3H), 7.10 (d, $J$ = 9.8, 1H), 6.73 (s, 1H), 4.38 (dd, $J$ = 15.8, 6.8, 1H), 4.23 (dd, $J$ = 15.7, 6.1, 1H), 3.86 - 3.79 (m, 2H), 2.62 - 2.53 (m, 1H), 2.28 (s, 3H), 2.10 (dt, $J$ = 13.0, 7.6, 1H). | 4.44 min [377] |
| "A190" | 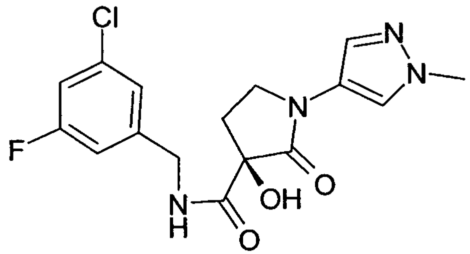 (R)-3-Hydroxy-1-(1-methyl-1H-pyrazol-4-yl)-2-oxo-pyrrolidin-3-carbonsäure-3-chlor-5-fluor-benzylamid | 8.71 (t, $J$ = 6.4, 1H), 8.01 (s, 1H), 7.63 (d, $J$ = 0.5, 1H), 7.31 - 7.23 (m, 1H), 7.19 (s, 1H), 7.09 (d, $J$ = 9.7, 1H), 6.70 (s, 1H), 4.37 (dd, $J$ = 15.8, 6.6, 1H), 4.23 (dd, $J$ = 15.9, 5.9, 1H), 3.82 (s, 3H), 3.74 - 3.62 (m, 2H), 2.60 (ddd, $J$ = 12.1, 7.6, 4.2, 1H), 2.13 (ddd, $J$ = 13.1, 8.5, 6.7, 1H). | 3.19 min [367] |
| "A191" | 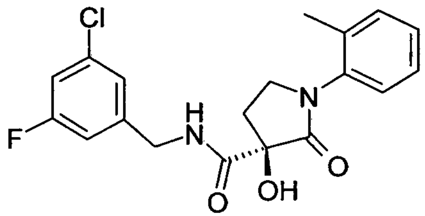 (R)-3-Hydroxy-2-oxo-1-o-tolyl-pyrrolidin-3-carbonsäure-3-chlor-5-fluor-benzylamid | 8.69 (t, $J$ = 6.4, 1H), 7.31 - 7.17 (m, 6H), 7.11 (d, $J$ = 9.7, 1H), 6.70 (s, 1H), 4.41 (dd, $J$ = 15.9, 6.7, 1H), 4.24 (dd, $J$ = 15.8, 6.0, 1H), 3.68 (dd, $J$ = 8.3, 5.3, 2H), 2.66 - 2.55 (m, 1H), 2.19 (dt, $J$ = 13.0, 7.6, 1H), 2.11 (s, 3H). | 4.17 min [377] |

(fortgesetzt)

| Nr. | Struktur / Name | $^1H$ NMR (400 MHz, DMSO-$d_6$) δ [ppm] | LC-MS; rt; [M+H$^+$]* |
|---|---|---|---|
| "A192" | 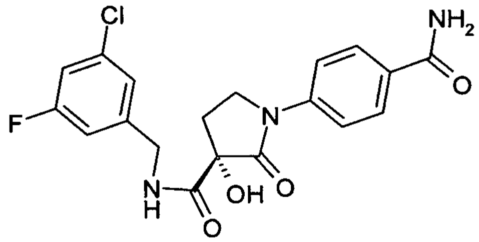 (S)-1-(4-Carbamoyl-phenyl)-3-hydroxy-2-oxo-pyrrolidin-3-carbonsäure-3-chlor-5-fluor-benzylamid | 8.76 (t, *J* = 6.4, 1H), 7.95 (br. s, 1H), 7.91 (d, *J* = 8.8, 2H), 7.79 (d, *J* = 8.9, 2H), 7.33 (br. s, 1H), 7.27 (d, *J* = 8.7, 1H), 7.20 (s, 1H), 7.10 (d, *J* = 9.2, 1H), 6.82 (s, 1H), 4.38 (dd, *J* = 15.8, 6.9, 1H), 4.24 (dd, *J* = 15.8, 6.0, 1H), 3.89 (dd, *J* = 9.3, 5.1, 2H), 3.37 (m, 1H), 2.64 - 2.55 (m, 2H), 2.19 - 2.08 (m, 1H). | 3.2 min [406] |
| "A193" | 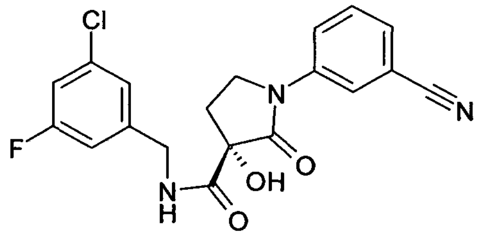 (S)-1-(3-Cyan-phenyl)-3-hydroxy-2-oxo-pyrrolidin-3-carbonsäure-3-chlor-5-fluor-benzylamid | 8.78 (t, *J* = 6.3, 1H), 8.16 (s, 1H), 8.08 (dt, *J* = 7.4, 2.0, 1H), 7.68 - 7.59 (m, 2H), 7.27 (dd, *J* = 8.8, 2.0, 1H), 7.19 (s, 1H), 7.09 (d, *J* = 9.5, 1H), 6.86 (s, 1H), 4.38 (dd, *J* = 15.6, 6.7, 1H), 4.24 (dd, *J* = 15.7, 6.0, 1H), 3.96 - 3.84 (m, 2H), 2.59 (ddd, *J* = 11.8, 7.1,4.5, 1H), 2.14 (dt, *J* = 13.1, 7.9, 1H). | 4.16 min [388.3] |
| "A194" | 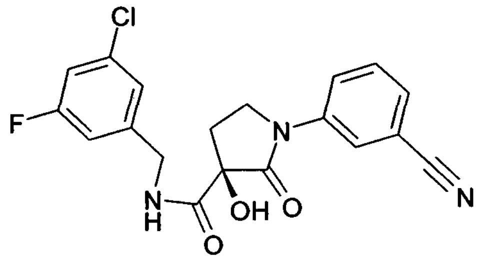 (R)-1-(3-Cyan-phenyl)-3-hydroxy-2-oxo-pyrrolidin-3-carbonsäure-3-chlor-5-fluor-benzylamid | 8.78 (t, *J* = 6.4, 1H), 8.16 (d, *J* = 1.5, 1H), 8.08 (dt, *J* = 7.5, 2.1, 1H), 7.68-7.59 (m, 2H), 7.27 (dt, *J* = 8.8, 2.1, 1H), 7.19 (s, 1H), 7.09 (d, *J* = 9.6, 1H), 6.86 (s, 1H), 4.38 (dd, *J* = 15.8, 6.7, 1H), 4.24 (dd, *J* = 15.7, 6.0, 1H), 3.96 - 3.83 (m, 2H), 2.59 (ddd, *J* = 11.8,7.1,4.6, 1H), 2.14 (dt, *J* = 13.0, 7.7, 1H). | 4.14 min [388.1] |

(fortgesetzt)

| Nr. | Struktur / Name | $^1H$ NMR (400 MHz, DMSO-$d_6$) δ [ppm] | LC-MS; rt; [M+H$^+$]* |
|---|---|---|---|
| "A195" | 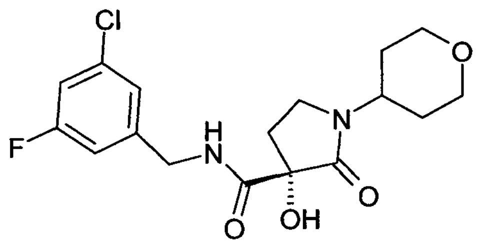 (S)-3-Hydroxy-2-oxo-1-(tetrahydro-pyran-4-yl)-pyrrolidin-3-carbonsäure-3-chlor-5-fluor-benzylamid | 8.58 (t, *J* = 6.3, 1H), 7.26 (dt, *J* = 8.7, 2.1, 1H), 7.18 (s, 1H), 7.09 (d, J = 9.7, 1H), 6.47 (s, 1H), 4.35 (dd, *J* = 15.8, 6.7, 1H), 4.21 (dd, *J* = 15.8, 6.0, 1H), 4.02 - 3.83 (m, 3H), 3.38 (dd, *J* = 11.9, 9.7, 2H), 2.46 - 2.38 (m, 1H), 1.92 (dt, *J* = 13.0, 7.4, 1H), 1.69 (m, 2H), 1.57 - 1.40 (m, 2H). | 3.1 min [371] |
| "A196" | 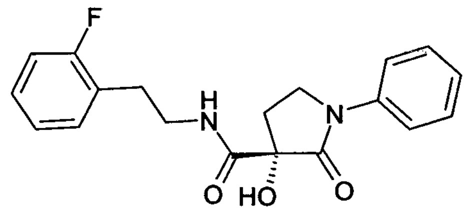 (S)-3-Hydroxy-2-oxo-1-phenyl-pyrrolidin-3-carbonsäure-[2-(2-fluor-phenyl)-ethyl]-amid | 8.08 (t, *J* = 5.9, 1H), 7.68 (d, *J* = 7.7, 2H), 7.43 - 7.36 (m, 2H), 7.25 (dt, *J* = 7.3, 4.6, 2H), 7.13 (ddd, *J* = 15.4, 11.1, 4.2, 3H), 6.60 (s, 1H), 3.86 - 3.76 (m, 2H), 3.45 -3.19 (m, 2H), 2.85 - 2.71 (m, 2H), 2.48 - 2.39 (m, 1H), 2.05 (dt, *J* = 12.9, 8.0, 1H). | 3.74 min [343] |
| "A197" | 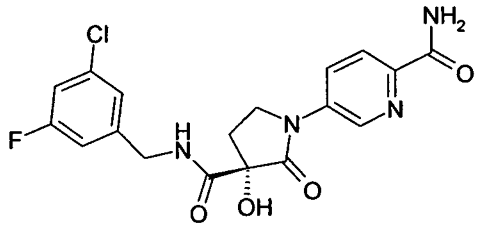 5-[(S)-3-(3-Chlor-5-fluor-benzylcarbamoyl)-3-hydroxy-2-oxo-pyrrolidin-1-yl]-pyridin-2-carbonsäure-amid | 9.00 (d, *J* = 2.4, 1H), 8.80 (t, *J* = 6.3, 1H), 8.28 (dd, *J* = 8.7, 2.5, 1H), 8.07 (d, *J* = 8.6, 2H), 7.60 (s, 1H), 7.28 (d, *J* = 8.8, 1H), 7.20 (s, 1H), 7.10 (d, *J* = 9.2, 1H), 6.91 (s, 1H), 4.38 (dd, *J* = 15.8, 6.7, 1H), 4.25 (dd, *J* = 15.7, 6.0, 1H), 4.03 - 3.88 (m, 2H), 2.69 - 2.58 (m, 1H), 2.24 - 2.12 (m, 1H). | 3.18 min [407] |

(fortgesetzt)

| Nr. | Struktur / Name | $^1$H NMR (400 MHz, DMSO-$d_6$) δ [ppm] | LC-MS; rt; [M+H$^+$]* |
|---|---|---|---|
| "A198" | 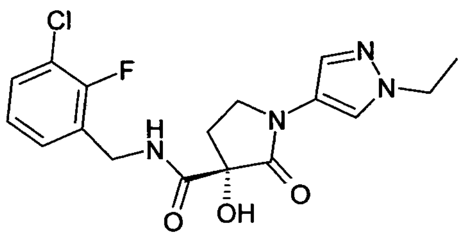 (S)-1-(1-Ethyl-1H-pyrazol-4-yl)-3-hydroxy-2-oxo-pyrrolidin-3-carbonsäure-3-chlor-2-fluor-benzylamid | 8.62 (t, *J* = 6.3, 1H), 8.04 (s, 1H), 7.65 (d, *J* = 0.4, 1H), 7.50-7.42 (m, 1H), 7.30 (t, *J* = 6.5, 1H), 7.18 (t, *J* = 7.9, 1H), 6.68 (s, 1H), 4.39 (dd, *J* = 15.6, 6.3, 1H), 4.31 (dd, *J* = 15.6, 5.9, 1H), 4.11 (q, *J* = 7.3, 2H), 3.75 - 3.62 (m, 2H), 2.60 (ddd, *J* = 12.0, 7.6, 4.2, 1H), 2.18 - 2.06 (m, 1H), 1.33 (t, *J* = 7.3, 3H). | 3.36 min [381.3] |
| "A199" | 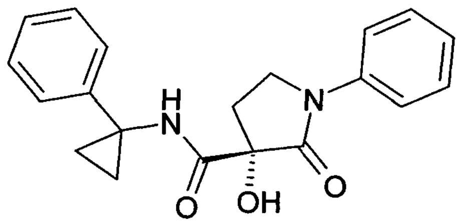 (S)-3-Hydroxy-2-oxo-1-phenyl-pyrrolidin-3-carbonsäure-(1-phenyl-cyclopropyl)-amid | 8.64 (s, 1H), 7.69 (dd, *J* = 8.7, 1.0, 2H), 7.44 - 7.36 (m, 2H), 7.26 - 7.18 (m, 2H), 7.18 - 7.09 (m, 3H), 6.67 (s, 1H), 3.88 - 3.76 (m, 2H), 2.61 - 2.51 (m, 1H), 2.10 (dt, *J* = 13.0, 7.8, 1H), 1.29 - 1.18 (m, 4H). | 3.61 min [337] |
| "A200" | 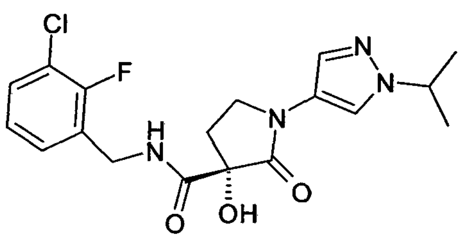 (S)-3-Hydroxy-1-(1-isopropyl-1 H-pyrazol-4-yl)-2-oxo-pyrrolidin-3-carbonsäure-3-chlor-2-fluor-benzylamid | 8.63 (t, *J* = 6.2, 1H), 8.03 (s, 1H), 7.66 (s, 1H), 7.49 - 7.42 (m, 1H), 7.30 (dd, *J* = 10.4, 4.1, 1H), 7.19 (dt, *J* = 8.6, 4.3, 1H), 6.69 (s, 1H), 4.49 (dq, *J* = 13.3, 6.6, 1H), 4.39 (dd, *J* = 15.6, 6.4, 1H), 4.31 (dd, *J* = 15.5, 6.1, 1H), 3.76 - 3.62 (m, 2H), 2.59 (ddd, *J* = 12.0, 7.6, 4.2, 1H), 2.12 (ddd, *J* = 18.7, 11.3, 6.3, 1H), 1.38 (d, *J* = 6.7, 6H). | 3.61 min [395.3] |

(fortgesetzt)

| Nr. | Struktur / Name | $^1H$ NMR (400 MHz, DMSO-$d_6$) δ [ppm] | LC-MS; rt; [M+H+]* |
|---|---|---|---|
| "A201" | 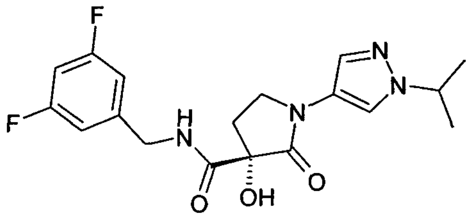 (S)-3-Hydroxy-1-(1-isopropyl-1H-pyrazol-4-yl)-2-oxo-pyrrolidin-3-carbonsäure-3,5-difluor-benzylamid | 8.71 (t, *J* = 6.4, 1H), 8.03 (d, *J* = 0.5, 1H), 7.66 (d, *J* = 0.5, 1H), 7.07 (tt, *J* = 9.4, 2.4, 1H), 7.01 - 6.93 (m, 2H), 6.69 (s, 1H), 4.55 - 4.43 (m, 1H), 4.38 (dd, *J* = 15.9, 6.8, 1H), 4.23 (dd, *J* = 15.8, 6.0, 1H), 3.76 - 3.61 (m, 2H), 2.61 (ddd, *J* = 12.2, 7.6, 4.3, 1H), 2.13 (ddd, *J* = 13.1, 8.5, 6.8, 1 H), 1.38 (d, *J* = 6.7, 6H). | 3.37 min [379.2] |
| "A202" | 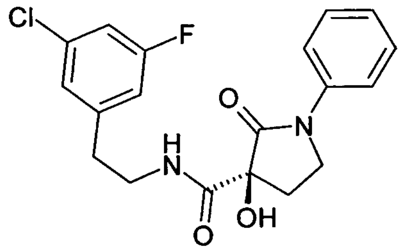 (S)-3-Hydroxy-2-oxo-1-phenyl-pyrrolidin-3-carbonsäure-[2-(3-chlor-5-fluor-phenyl)-ethyl]-amid | 8.06 (t, *J* = 6.0, 1H), 7.68 (dd, *J* = 8.7, 1.0, 2H), 7.44 - 7.35 (m, 2H), 7.24 (dt, *J* = 8.9, 2.2, 1H), 7.20 - 7.13 (m, 2H), 7.08 (dd, *J* = 9.7, 1.4, 1H), 6.63 (s, 1H), 3.81 (dd, *J* = 7.8, 5.8, 2H), 3.45 - 3.35 (m, 2H), 2.78 (t, *J* = 6.9, 2H), 2.43 (dt, *J* = 12.8, 5.6, 1H), 2.05 (dt, *J* = 12.9, 7.8, 1H). | 4.31 min [377] |
| "A203" | 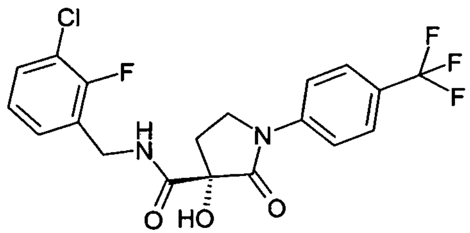 (S)-3-Hydroxy-2-oxo-1-(4-trifluoromethyl-phenyl)-pyrrolidin-3-carbonsäure-3-chlor-2-fluor-benzylamid | 8.70 (t, *J* = 6.2, 1H), 7.94 (d, *J* = 8.6, 2H), 7.77 (d, *J* = 8.7, 2H), 7.49 - 7.42 (m, 1H), 7.30 (t, *J* = 6.5, 1H), 7.18 (dd, *J* = 8.2, 7.5, 1H), 6.85 (s, 1H), 4.40 (dd, *J* = 15.6, 6.5, 1H), 4.33 (dd, *J* = 15.6, 6.0, 1H), 3.91 (dd, *J* = 8.6, 5.6, 2H), 2.60 (dt, *J* = 6.7, 5.6, 1H), 2.15 (dt, *J*= 13.1, 7.8, 1H). | 4.89 min [431] |

(fortgesetzt)

| Nr. | Struktur / Name | $^1$H NMR (400 MHz, DMSO-$d_6$) δ [ppm] | LC-MS; rt; [M+H$^+$]* |
|---|---|---|---|
| "A204" | 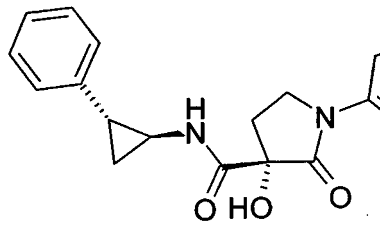 (S)-3-Hydroxy-2-oxo-1-phenyl-pyrrolidin-3-carbonsäure-((1 S,2R)-2-phenyl-cyclopropyl)-amid | 8.25 (d, *J* = 5.2, 1H), 7.69 (dd, *J* = 8.7, 1.0, 2H), 7.44 - 7.37 (m, 2H), 7.24 (dd, *J* = 10.3, 4.6, 2H), 7.20 - 7.06 (m, 4H), 6.62 (s, 1H), 3.87 - 3.81 (m, 2H), 2.88 (td, *J* = 8.3, 4.9, 1H), 2.61 - 2.51 (m, 1H), 2.13 - 2.00 (m, 2H), 1.40 -1.32 (m, 1H), 1.13 (dt, *J* = 7.9, 5.8, 1H). | 3.85 min [337.3] |
| "A205" | 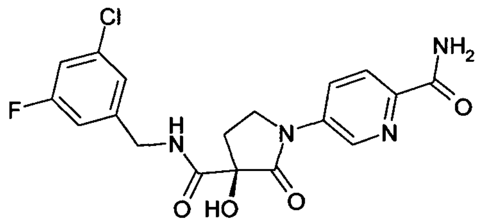 5-[(R)-3-(3-Chlor-5-fluor-benzylcarbamoyl)-3-hydroxy-2-oxo-pyrrolidin-1-yl]-pyridin-2-carbonsäure-amid | 9.00 (d, *J* = 2.1, 1H), 8.80 (t, *J* = 6.3, 1H), 8.28 (dd, *J* = 8.7, 2.6, 1H), 8.07 (d, *J* = 8.6, 2H), 7.60 (s, 1H), 7.28 (dt, *J* = 8.8, 2.1, 1H), 7.20 (s, 1H), 7.10 (d, *J* = 9.6, 1H), 6.90 (s, 1H), 4.38 (dd, *J* = 15.8, 6.7, 1H), 4.25 (dd, *J* = 15.6, 6.0, 1H), 4.01 - 3.90 (m, 2H), 2.68 - 2.58 (m, 1H), 2.23 - 2.13 (m, 1H). | 3.18 min [407] |
| "A206" | 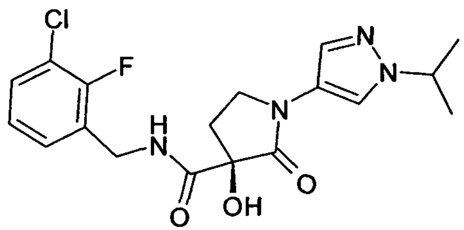 (R)-3-Hydroxy-1-(1-isopropyl-1H-pyrazol-4-yl)-2-oxo-pyrrolidin-3-carbonsäure-3-chlor-2-fluor-benzylamid | 8.63 (t, *J* = 6.3, 1H), 8.03 (d, *J* = 0.5, 1H), 7.66 (*d, J* = 0.5, 1H), 7.49 - 7.42 (m, 1H), 7.34 - 7.26 (m, 1H), 7.18 (td, *J* = 7.9, 0.8, 1H), 6.68 (s, 1H), 4.54 - 4.43 (m, 1H), 4.39 (dd, *J* = 15.6, 6.4, 1H), 4.31 (dd, *J* = 15.6, 6.0, 1H), 3.75 - 3.62 (m, 2H), 2.60 (ddd, *J* = 11.9, 7.5, 4.2, 1H), 2.20 - 2.07 (m, 1H), 1.38 (d, *J* = 6.7, 6H). | 3.62 min [395.3] |

(fortgesetzt)

| Nr. | Struktur / Name | $^1$H NMR (400 MHz, DMSO-$d_6$) δ [ppm] | LC-MS; rt; [M+H$^+$]* |
|---|---|---|---|
| "A207" | 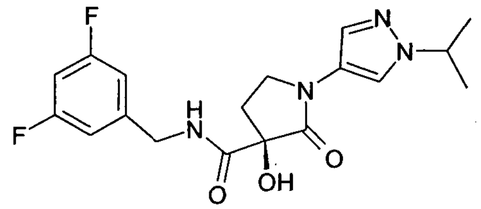 (R)-3-Hydroxy-1-(1-isopropyl-1H-pyrazol-4-yl)-2-oxo-pyrrolidin-3-carbonsäure-3,5-difluor-benzylamid | 8.71 (t, *J* = 6.4, 1H), 8.03 (s, 1H), 7.66 (s, 1H), 7.29 - 6.83 (m, 3H), 6.67 (d, *J* = 14.3, 1H), 4.55 - 4.43 (m, 1H), 4.38 (dd, *J* = 15.8, 6.7, 1H), 4.23 (dd, *J* = 15.8, 6.0, 1H), 3.77 - 3.62 (m, 2H), 2.61 (ddd, *J* = 12.2, 7.6, 4.4, 1H), 2.12 (ddd, *J* = 19.8, 11.8, 6.7, 1H), 1.38 (d, *J* = 6.7, 6H). | 3.38 min [379.2] |
| "A208" | 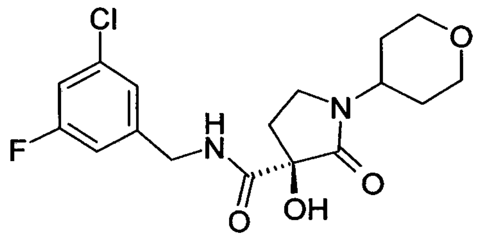 (R)-3-Hydroxy-2-oxo-1-(tetrahydro-pyran-4-yl)-pyrrolidin-3-carbonsäure- 3-chlor-5-fluor-benzylamid | 8.58 (t, *J* = 6.5, 1H), 7.26 (dt, *J* = 8.7, 2.1, 1H), 7.18 (s, 1H), 7.09 (d, *J* = 9.7, 1H), 6.47 (s, 1H), 4.35 (dd, *J* = 15.9, 6.9, 1H), 4.21 (dd, *J* = 15.7, 6.0, 1H), 4.00 - 3.84 (m, 3H), 3.44 - 3.34 (m, 3H), 2.47 - 2.38 (m, 1H), 1.96 - 1.87 (m, 1H), 1.78 - 1.61 (m, 2H), 1.53 - 1.41 (m, 2H). | 3.1 min [371] |
| "A209" | 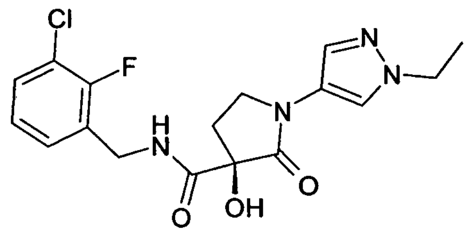 (R)-1-(1-Ethyl-1H-pyrazol-4-yl)-3-hydroxy-2-oxo-pyrrolidin-3-carbonsäure-3-chlor-2-fluor-benzylamid | 8.62 (t, *J* = 6.3, 1H), 8.04 (s, 1H), 7.65 (d, *J* = 0.4, 1H), 7.50 - 7.41 (m, 1H), 7.30 (t, *J* = 6.5, 1H), 7.18 (t, *J* = 7.9, 1H), 6.68 (s, 1H), 4.39 (dd, *J* = 15.6, 6.3, 1H), 4.31 (dd, *J* = 15.6, 5.9, 1H), 4.11 (q, *J* = 7.3, 2H), 3.76 - 3.62 (m, 2H), 2.60 (ddd, *J* = 12.0, 7.6, 4.2, 1H), 2.20 - 2.06 (m, 1H), 1.33 (t, *J* = 7.3, 3H). | 3.35 min [381] |

(fortgesetzt)

| Nr. | Struktur / Name | $^1$H NMR (400 MHz, DMSO-$d_6$) δ [ppm] | LC-MS; rt; [M+H$^+$]* |
|---|---|---|---|
| "A210" | 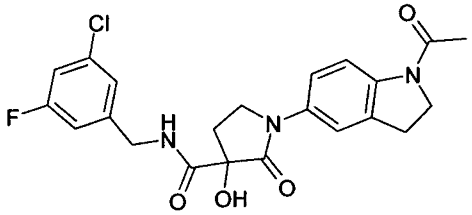 1-(1-Acetyl-2,3-dihydro-1H-indol-5-yl)-3-hydroxy-2-oxo-pyrrolidin-3-carbonsäure-3-chlor-5-fluor-benzylamid | 8.71 (t, *J* = 6.4, 1H), 8.01 (d, *J* = 8.8, 1H), 7.62 (s, 1H), 7.39 (d, *J* = 8.7, 1H), 7.30 - 7.24 (m, 1H), 7.20 (s, 1H), 7.10 (d, *J* = 10.0, 1H), 6.72 (s, 1H), 4.38 (dd, *J* = 15.8, 6.7, 1H), 4.23 (dd, *J* = 15.8, 6.0, 1H), 4.09 (t, *J* = 8.6, 2H), 3.81 (t, *J* = 6.9, 2H), 3.15 (t, *J* = 8.4, 2H), 2.63 - 2.52 (m, 1H), 2.14 (s, 3H), 2.09 (dd, *J* = 13.8, 6.2, 1H). | 3.66 min [446] |
| "A211" | 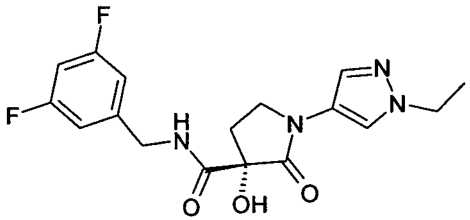 (S)-1-(1-Ethyl-1 H-pyrazol-4-yl)-3-hydroxy-2-oxo-pyrrolidin-3-carbonsäure-3,5-difluor-benzylamid | 8.70 (t, *J* = 6.2, 1H), 8.04 (s, 1H), 7.65 (s, 1H), 7.06 (t, *J* = 9.4, 1H), 6.97 (d, *J* = 6.5, 2H), 6.69 (s, 1H), 4.38 (dd, *J* = 15.8, 6.8, 1H), 4.23 (dd, *J* = 15.8, 5.9, 1H), 4.11 (q, *J* = 7.2, 2H), 3.77 - 3.60 (m, 2H), 2.61 (ddd, *J* = 12.3, 7.6, 4.4, 1H), 2.21 - 2.07 (m, 1H), 1.33 (t, *J* = 7.3, 3H). | 3.06 min [365] |
| "A212" | 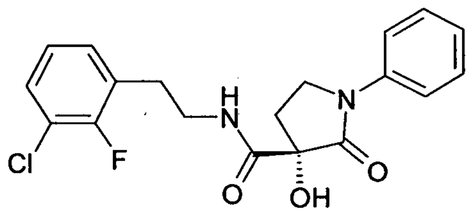 (S)-3-Hydroxy-2-oxo-1-phenyl-pyrrolidin-3-carbonsäure-[2-(3-chlor-2-fluor-phenyl)-ethyl]-amid | 8.12 (t, *J* = 6.0, 1H), 7.68 (dd, *J* = 8.7, 1.0, 2H), 7.45 - 7.36 (m, 3H), 7.28-7.22 (m, 1H), 7.15 (dt, *J* = 16.5, 7.6, 2H), 6.60 (s, 1H), 3.80 (dd, *J* = 7.9, 5.7, 2H), 3.39 (td, *J* = 13.8, 7.2, 1H), 3.31 - 3.20 (m, 1H), 2.82 (dd, *J* = 10.6, 6.7, 2H), 2.47 - 2.37 (m, 1H), 2.04 (dt, *J* = 12.9, 7.9, 1H). | 4.18 min [3773.] |

(fortgesetzt)

| Nr. | Struktur / Name | $^1$H NMR (400 MHz, DMSO-$d_6$) δ [ppm] | LC-MS; rt; [M+H$^+$]* |
|---|---|---|---|
| "A213" | 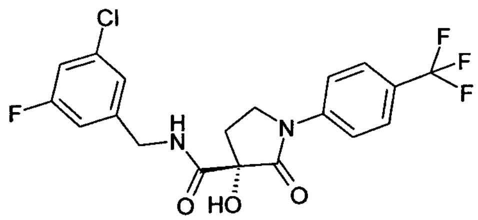 (S)-3-Hydroxy-2-oxo-1-(4-trifluormethyl-phenyl)-pyrrolidin-3-carbonsäure-3-chlor-5-fluor-benzylamid | 8.78 (t, *J* = 6.3, 1H), 7.94 (d, *J* = 8.6, 2H), 7.78 (d, *J* = 8.7, 2H), 7.27 (dt, *J* = 8.8, 2.2, 1H), 7.20 (s, 1H), 7.09 (d, *J* = 8.9, 1H), 6.86 (s, 1H), 4.38 (dd, *J* = 15.8, 6.7, 1H), 4.24 (dd, *J* = 15.8, 6.1, 1H), 3.97 - 3.86 (m, 2H), 2.64 - 2.55 (m, 1H), 2.15 (dt, *J* = 13.1, 7.7, 1H). | $$ 6.299 min [429.0] |
| "A214" | 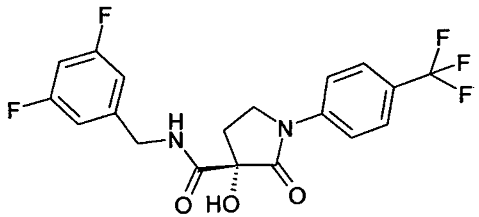 (S)-3-Hydroxy-2-oxo-1-(4-trifluormethyl-phenyl)-pyrrolidin-3-carbonsäure-3,5-difluor-benzylamid | 8.77 (t, *J* = 6.4, 1H), 7.94 (d, *J* = 8.6, 2H), 7.78 (d, *J* = 8.8, 2H), 7.12 - 7.02 (m, 1H), 6.98 (d, *J* = 6.6, 2H), 6.86 (s, 1H), 4.39 (dd, *J* = 15.7, 6.8, 1H), 4.25 (dd, *J* = 15.9, 6.1, 1H), 3.92 (dd, *J* = 8.7, 5.7, 2H), 2.61 (dt, *J* = 6.7, 5.7, 1H), 2.16 (dt, *J* = 13.0, 7.6, 1H). | $$ 6.055 min [413.0] |
| "A215" | 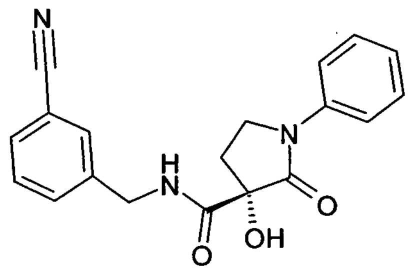 (S)-3-Hydroxy-2-oxo-1-phenyl-pyrrolidin-3-carbonsäure-3-cyan-benzylamid | 8.73 (t, *J* = 6.4, 1H), 7.75 - 7.66 (m, 4H), 7.61 (d, *J* = 7.9, 1H), 7.56 - 7.48 (m, 1H), 7.45 - 7.36 (m, 2H), 7.18 (t, *J* = 7.4, 1H), 6.76 (s, 1H), 4.41 (dd, *J* = 15.5, 6.7, 1H), 4.29 (dd, *J* = 15.6, 6.1, 1H), 3.85 (dd, *J* = 7.6, 6.0, 2H), 2.64-2.54 (m, 1H), 2.12 (dt, *J* = 13.0, 7.7, 1H). | 3.352 min [336.2] |

(fortgesetzt)

| Nr. | Struktur / Name | $^1$H NMR (400 MHz, DMSO-$d_6$) δ [ppm] | LC-MS; rt; [M+H$^+$]* |
|---|---|---|---|
| "A216" | 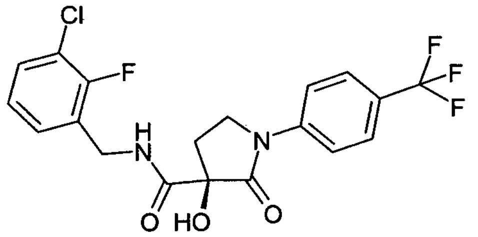 (R)-3-Hydroxy-2-oxo-1-(4-trifluormethyl-phenyl)-pyrrolidin-3-carbonsäure-3-chlor-2-fluor-benzylamid | 8.70 (t, *J* = 6.2, 1H), 7.94 (d, *J* = 8.6, 2H), 7.77 (d, *J* = 8.8, 2H), 7.51 - 7.42 (m, 1H), 7.30 (t, *J* = 6.5, 1H), 7.18 (t, *J* = 7.9, 1H), 6.85 (s, 1H), 4.40 (dd, *J* = 15.6, 6.3, 1H), 4.33 (dd, *J* = 15.5, 6.1, 1H), 3.91 (dd, *J* = 8.6, 5.6, 2H), 2.60 (dt, *J* = 6.6, 5.7, 1H), 2.15 (dt, *J* = 13.0, 7.8, 1H). | 4.879 min [431.0] |
| "A217" | 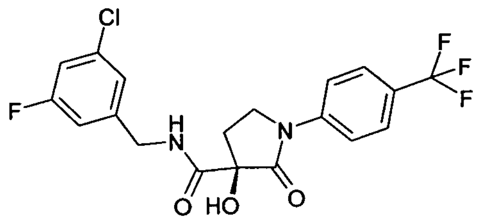 (R)-3-Hydroxy-2-oxo-1-(4-trifluormethyl-phenyl)-pyrrolidin-3-carbonsäure-3-chlor-5-fluor-benzylamid | 8.78 (t, *J* = 6.3, 1H), 7.94 (d, *J* = 8.6, 2H), 7.78 (d, *J* = 8.7, 2H), 7.27 (dt, *J* = 8.8, 2.1, 1H), 7.20 (s, 1H), 7.09 (d, *J* = 9.6, 1H), 6.86 (s, 1H), 4.38 (dd, *J* = 15.7, 6.7, 1H), 4.24 (dd, *J* = 15.8, 6.1, 1H), 3.97-3.84 (m, 2H), 2.65 - 2.56 (m, 1H), 2.15 (dt, *J*= 13.1, 7.7, 1H). | $$ 6.301 min [429.0] |
| "A218" | 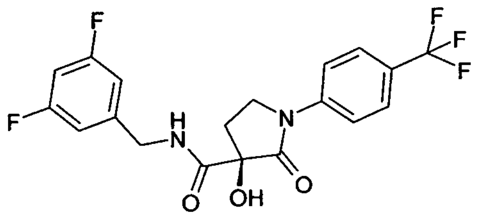 (R)-3-Hydroxy-2-oxo-1-(4-trifluormethyl-phenyl)-pyrrolidin-3-carbonsäure-3,5-difluor-benzylamid | 8.77 (t, *J* = 6.4, 1H), 7.94 (d, *J* = 8.7, 2H), 7.78 (d, *J* = 8.8, 2H), 7.11 - 7.03 (m, 1H), 6.98 (d, *J* = 6.6, 2H), 6.86 (s, 1H), 4.39 (dd, *J* = 15.8, 6.7, 1H), 4.24 (dd, *J* = 15.8, 6.0, 1H), 3.91 (dd, *J* = 8.7, 5.7, 2H), 2.61 (dt, *J* = 6.8, 5.8, 1H), 2.16 (dt, *J* = 13.0, 7.6, 1H). | 4.69 min [415] |

(fortgesetzt)

| Nr. | Struktur / Name | $^1$H NMR (400 MHz, DMSO-$d_6$) δ [ppm] | LC-MS; rt; [M+H$^+$]* |
|---|---|---|---|
| "A219" | 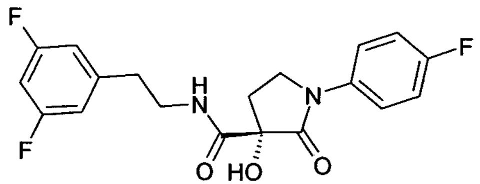 (S)-1-(4-Fluor-phenyl)-3-hydroxy-2-oxo-pyrrolidin-3-carbonsäure-[2-(3,5-difluorphenyl)-ethyl]-amid | 8.05 (t, *J* = 6.0, 1H), 7.75 - 7.68 (m, 2H), 7.28-7.20 (m, 2H), 7.03 (tt, *J* = 9.5, 2.3, 1H), 6.98 - 6.92 (m, 2H), 6.63 (s, 1H), 3.80 (dd, *J* = 7.8, 5.7, 2H), 3.42 - 3.25 (m, 2H), 2.78 (t, *J* = 7.0, 2H), 2.44 (dt, *J* = 12.8, 5.6, 1H), 2.04 (dt, *J* = 12.9, 7.8, 1H). | 4.22 min [379.3] |
| "A220" | 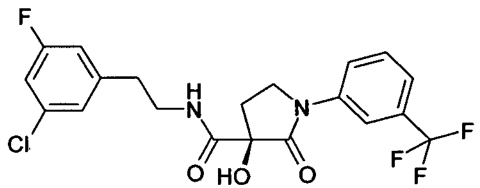 (R)-3-Hydroxy-2-oxo-1-(3-trifluormethyl-phenyl)-pyrrolidin-3-carbonsäure-[2-(3-chlor-5-fluorphenyl)-ethyl]-amid | 8.21 (s, 1H), 8.11 (t, *J* = 6.0, 1H), 7.87 (d, *J* = 8.1, 1H), 7.65 (t, *J* = 8.0, 1H), 7.54 (d, *J* = 7.8, 1H), 7.23 (dt, *J* = 8.9, 2.2, 1H), 7.15 (s, 1H), 7.07 (d, *J* = 9.5, 1H), 6.70 (s, 1H), 3.94-3.82 (m, 2H), 3.43 - 3.34 (m, 2H), 3.30 (s, 1H), 2.78 (t, *J* = 6.9, 2H), 2.47 - 2.41 (m, 1H), 2.08 (dt, *J* = 13.2, 8.5, 1H). | 5.09 min [445] |
| "A221" | 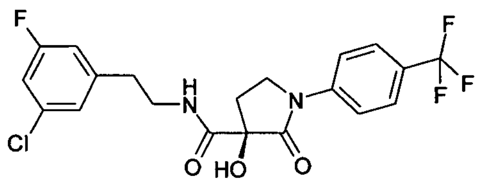 (R)-3-Hydroxy-2-oxo-1-(4-trifluormethyl-phenyl)-pyrrolidin-3-carbonsäure-[2-(3-chlor-5-fluor-phenyl)-ethyl]-amid | 8.10 (t, *J* = 6.0, 1H), 7.93 (d, *J* = 8.6, 2H), 7.77 (d, *J* = 8.8, 2H), 7.24 (dt, *J* = 8.9, 2.1, 1H), 7.15 (s, 1H), 7.07 (d, *J* = 9.0, 1H), 6.72 (s, 1 H), 3.93 - 3.81 (m, 2H), 3.43 - 3.33 (m, 2H), 2.78 (t, *J* = 6.9, 2H), 2.48 - 2.41 (m, 1H), 2.08 (dt, *J* = 12.8, 8.0, 1H). | HPLC: 5.066 Min 99.06% purity |
| "A222" | 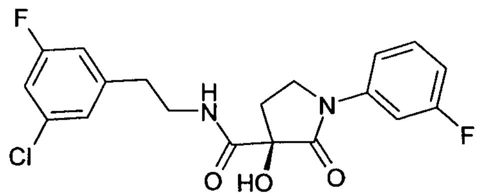 (R)-1-(3-Fluor-phenyl)-3-hydroxy-2-oxo-pyrrolidin-3-carbonsäure-[2-(3-chlor-5-fluor-phenyl)-ethyl]-amid | 8.08 (t, *J* = 6.0, 1H), 7.69 (dt, *J* = 12.0, 2.2, 1H), 7.51 - 7.39 (m, 2H), 7.23 (dt, *J* = 8.9, 2.2, 1H), 7.15 (d, *J* = 1.5, 1H), 7.10-7.05 (m, 1H), 7.04 - 6.98 (m, 1H), 6.68 (s, 1H), 3.87 - 3.76 (m, 2H), 3.45-3.34 (m, 1H), 2.78 (t, *J* = 6.9, 2H), 2.43 (ddd, J = 12.8, 7.2, 4.1, 1H), 2.05 (dt, *J* = 12.9, 7.9, 1H). | 4.58 min [395] |

(fortgesetzt)

| Nr. | Struktur / Name | $^1H$ NMR (400 MHz, DMSO-$d_6$) δ [ppm] | LC-MS; rt; [M+H$^+$]* |
|---|---|---|---|
| "A223" | 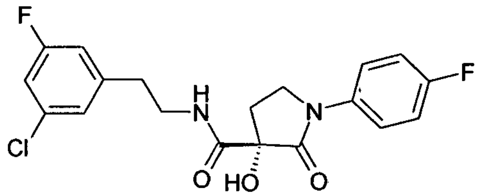 (S)-1-(4-Fluor-phenyl)-3-hydroxy-2-oxo-pyrrolidin-3-carbonsäure-[2-(3-chlor-5-fluor-phenyl)-ethyl]-amid | 8.06 (t, *J* = 6.0, 1H), 7.75 - 7.68 (m, 2H), 7.28-7.21 (m, 3H), 7.16 (s, 1H), 7.08 (dd, *J* = 9.7, 1.4, 1H), 6.63 (s, 1H), 3.80 (dd, *J* = 7.6, 6.0, 2H), 3.44 - 3.34 (m, 1H), 2.78 (t, *J* = 7.0, 2H), 2.43 (dt, *J* = 12.7, 5.5, 1H), 2.04 (dt, *J* = 12.9, 7.8, 1H). | 4.4 min [395] |
| "A224" | 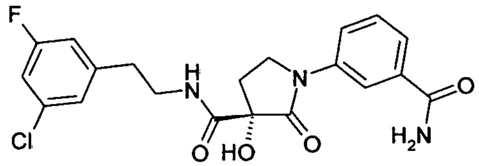 (S)-1-(3-Carbamoyl-phenyl)-3-hydroxy-2-oxo-pyrrolidin-3-carbonsäure-[2-(3-chlor-5-fluor-phenyl)-ethyl]-amid | 8.08 (t, *J* = 6.0, 1H), 8.05 - 7.99 (m, 2H), 7.94 (dd, *J* = 8.2, 1.4, 1H), 7.67 (d, *J* = 7.8, 1H), 7.47 (t, *J* = 8.0, 1H), 7.42 (s, 1H), 7.24 (dt, *J* = 8.9, 2.1, 1H), 7.16 (s, 1H), 7.08 (dd, *J* = 9.7, 1.4, 1H), 6.66 (s, 1H), 3.89-3.83 (m, 2H), 3.42 - 3.34 (m, 1H), 2.78 (t, *J* = 7.0, 2H), 2.44 (dd, *J* = 12.3, 6.0, 1H), 2.07 (dt, *J* = 12.9, 7.8, 1H). | 3.44 min [420] |
| "A225" | 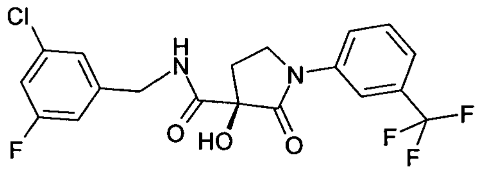 (R)-3-Hydroxy-2-oxo-1-(3-trifluormethyl-phenyl)-pyrrolidin-3-carbonsäure-3-chlor-5-fluor-benzylamid | 8.80 (t, *J* = 6.4, 1H), 8.21 (s, 1H), 7.89 (d, *J* = 8.3, 1H), 7.66 (t, *J* = 8.0, 1H), 7.54 (d, *J* = 7.8, 1H), 7.27 (dt, *J* = 8.7, 2.1, 1H), 7.20 (s, 1H), 7.09 (d, *J* = 9.6, 1H), 6.89 (s, 1H), 4.38 (dd, *J* = 15.8, 6.7, 1H), 4.24 (dd, *J* = 15.8, 6.0, 1H), 4.00 - 3.84 (m, 2H), 2.60 (ddd, *J* = 11.8, 7.2, 4.4, 1H), 2.15 (dt, *J* = 13.1, 7.7, 1H). | 4.89 min [431] |
| "A226" | 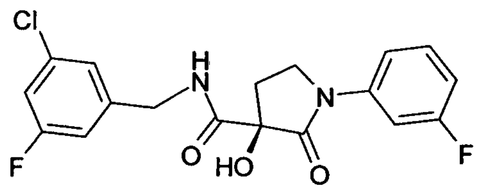 (R)-1-(3-Fluor-phenyl)-3-hydroxy-2-oxo-pyrrolidin-3-carbonsäure-3-chlor-5-fluor-benzylamid | 8.75 (t, *J* = 6.5, 1H), 7.69 (dt, *J* = 11.9, 2.2, 1H), 7.46 (dt, *J* = 15.0, 8.6, 2H), 7.27 (dd, *J* = 8.8, 2.1, 1H), 7.20 (s, 1H), 7.09 (d, *J* = 8.8, 1H), 7.03 (t, *J* = 7.9, 1H), 6.82 (s, 1H), 4.37 (dd, *J* = 15.4, 6.6, 1H), 4.24 (dd, *J* = 15.8, 5.7, 1H), 3.92 - 3.79 (m, 2H), 2.58 (dt, *J* = 6.8, 5.7, 1H), 2.13 (dt, *J* = 13.1, 7.7, 1H). | 4.39 min [381] |

(fortgesetzt)

| Nr. | Struktur / Name | $^1H$ NMR (400 MHz, DMSO-$d_6$) δ [ppm] | LC-MS; rt; $[M+H^+]$* |
|---|---|---|---|
| "A227" | 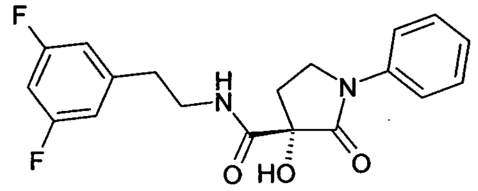 (S)-3-Hydroxy-2-oxo-1-phenyl-pyrrolidin-3-carbonsäure-[2-(3,5-difluor-phenyl)-ethyl]-amid | 8.05 (t, *J* = 6.0, 1H), 7.68 (dt, *J* = 9.0, 1.7, 2H), 7.43 - 7.36 (m, 2H), 7.21-7.13 (m, 1H), 7.03 (tt, *J* = 9.5, 2.3, 1H), 6.99 - 6.92 (m, 2H), 6.63 (s, 1H), 3.82 (dd, *J* = 7.7, 5.9, 2H), 3.41 - 3.34 (m, 2H), 2.78 (t, *J* = 7.0, 2H), 2.44 (dt, *J* = 12.8, 5.5, 1H), 2.05 (dt, *J* = 12.9, 7.8, 1H). | 3.97 min [361.3] |
| "A228" | 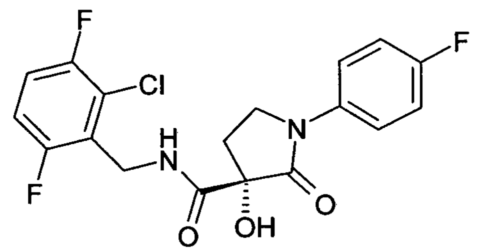 (S)-1-(4-Fluor-phenyl)-3-hydroxy-2-oxo-pyrrolidin-3-carbonsäure-2-chlor-3,6-difluor-benzylamid | 8.22 (t, *J* = 5.5, 1H), 7.70 (ddd, *J* = 10.7, 5.4, 3.0, 2H), 7.43 (td, *J* = 9.0, 4.7, 1H), 7.33 - 7.20 (m, 3H), 6.65 (s, 1H), 4.50 (dd, *J* = 15.0, 5.1, 1H), 4.43 (dd, *J* = 14.5, 4.5, 1H), 3.89-3.76 (m, 2H), 2.52 (m, 1H), 2.07 (dt, *J* = 12.9, 8.4, 1H). | 4.01 min [399] |
| "A229" | 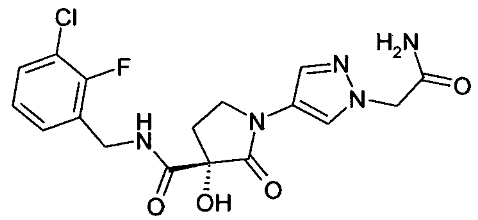 (S)-1-(1-Carbamoylmethyl-1H-pyrazol-4-yl)-3-hydroxy-2-oxo-pyrrolidin-3-carbonsäure-3-chlor-2-fluor-benzylamid | 8.62 (t, *J* = 6.2, 1H), 8.04 (s, 1H), 7.66 (s, 1H), 7.51 - 7.43 (m, 2H), 7.31 (t, *J* = 6.5, 1H), 7.23 (s, 1H), 7.18 (t, *J* = 7.9, 1H), 6.70 (s, 1H), 4.74 (s, 2H), 4.40 (dd, *J* = 15.7, 6.2, 1H), 4.36 - 4.29 (m, 1H), 3.77 - 3.64 (m, 2H), 3.49 - 3.39 (m, 1H), 3.16 (d, *J* = 5.2, 1H), 2.61 (ddd, *J* = 12.1, 7.5, 4.3, 1H), 2.20 - 2.09 (m, 1H). | HPLC 2.866 min 96.09 % purity |

(fortgesetzt)

| Nr. | Struktur / Name | $^1H$ NMR (400 MHz, DMSO-$d_6$) δ [ppm] | LC-MS; rt; [M+H$^+$]* |
|---|---|---|---|
| "A230" | 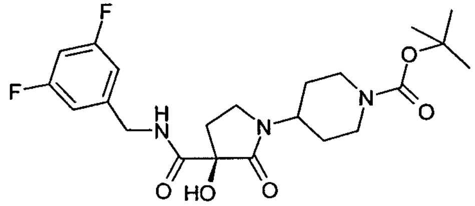 4-[(R)-3-(3,5-Difluor-benzylcarbamoyl)-3-hydroxy-2-oxo-pyrrolidin-1-yl]-piperidin-1-carbonsäure-tert.-butylester | | 4.17 min [354.3] |
| "A231" | 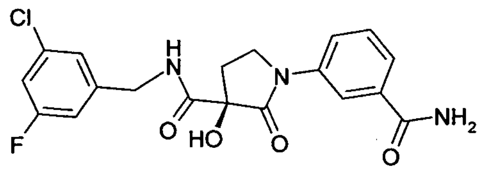 (R)-1-(3-Carbamoyl-phenyl)-3-hydroxy-2-oxo-pyrrolidin-3-carbonsäure-3-chlor-5-fluor-benzylamid | 8.75 (t, $J$ = 6.4, 1H), 8.07 - 8.00 (m, 2H), 7.99-7.92 (m, 1H), 7.67 (d, $J$ = 7.9, 1H), 7.48 (t, $J$ = 8.0, 1H), 7.42 (s, 1H), 7.27 (dt, $J$ = 8.8, 2.2, 1H), 7.20 (s, 1H), 7.10 (d, $J$ = 9.7, 1H), 6.80 (s, 1H), 4.38 (dd, $J$ = 15.8, 6.7, 1H), 4.25 (dd, $J$ = 15.7, 6.0, 1H), 3.94-3.84 (m, 2H), 2.65 - 2.54 (m, 1H), 2.14 (dt, J = 13.0, 7.6, 1H). | 3.32 min [406] |
| "A232" | 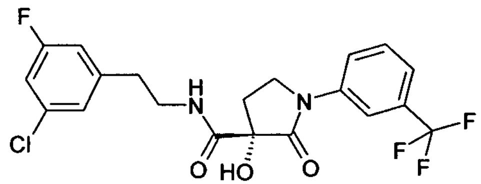 (S)-3-Hydroxy-2-oxo-1-(3-trifluormethyl-phenyl)-pyrrolidin-3-carbonsäure-[2-(3-chlor-5-fluor-phenyl)-ethyl]-amid | 11.12 (s, 1H), 8.69 (d, $J$ = 6.7, 1H), 7.70 (s, 1H), 7.41 - 7.38 (m, 2H), 7.38 - 7.35 (m, 1H), 7.27 (d, $J$ = 8.8, 1H), 7.22 (s, 1H), 7.12 (d, $J$ = 9.5, 1H), 6.69 (s, 1H), 6.42 (s, 1H), 4.40 (dd, $J$ = 15.8, 7.1, 2H), 4.25 (dd, $J$ = 15.8, 5.8, 2H), 3.91 - 3.84 (m, 2H), 2.64 - 2.55 (m, 2H), 2.14 (t, $J$ = 6.6, 1H), 2.12-2.08 (m, 1H). | 5.09 min [445] |
| "A233" | 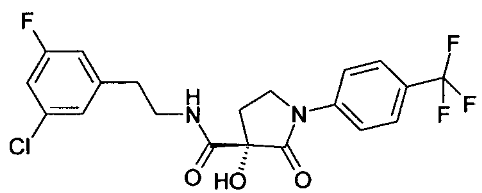 (S)-3-Hydroxy-2-oxo-1-(4-trifluormethyl-phenyl)-pyrrolidin-3-carbonsäure-[2-(3-chlor-5-fluor-phenyl)-ethyl]-amid | 8.11 (t, $J$ = 6.0, 1H), 7.93 (d, $J$ = 8.6, 2H), 7.77 (d, $J$ = 8.7, 2H), 7.24 (dt, $J$ = 8.9, 2.1, 1H), 7.15 (s, 1H), 7.08 (dd, $J$ = 9.3, 1.8, 1H), 6.75 (s, 1H), 3.94 - 3.81 (m, 2H), 3.44 - 3.34 (m, 2H), 2.78 (t, $J$ = 6.9, 2H), 2.47 - 2.41 (m, 1H), 2.08 (dt, $J$ = 12.8, 7.9, 1H). | HPLC 5.068 min 99.04 % purity |

(fortgesetzt)

| Nr. | Struktur / Name | $^1$H NMR (400 MHz, DMSO-$d_6$) δ [ppm] | LC-MS; rt; [M+H$^+$]* |
|---|---|---|---|
| "A234" | 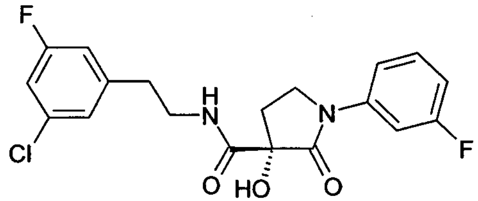 (S)-1-(3-Fluor-phenyl)-3-hydroxy-2-oxo-pyrrolidin-3-carbonsäure-[2-(3-chlor-5-fluor-phenyl)-ethyl]-amid | 8.08 (t, *J* = 6.0, 1H), 7.69 (dt, *J* = 12.0, 2.2, 1H), 7.46 (tdd, *J* = 16.3, 11.0, 5.1, 2H), 7.23 (dt, *J* = 8.9, 2.1, 1H), 7.15 (s, 1H), 7.08 (dd, *J* = 9.3, 1.8, 1H), 7.05 - 6.98 (m, 1H), 6.68 (s, 1H), 3.89 - 3.70 (m, 2H), 3.44 - 3.33 (m, 2H), 2.78 (t, *J* = 6.9, 2H), 2.46 - 2.38 (m, 1H), 2.05 (dt, *J* = 13.0, 8.0, 1H). | 4.59 min [395] |
| "A235" | 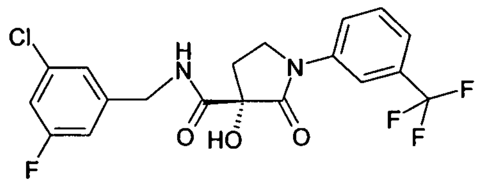 (S)-3-Hydroxy-2-oxo-1-(3-trifluormethyl-phenyl)-pyrrolidin-3-carbonsäure-3-chlor-5-fluor-benzylamid | 8.80 (t, *J* = 6.1, 1H), 8.21 (s, 1H), 7.89 (d, *J* = 8.2, 1H), 7.66 (t, *J* = 8.0, 1H), 7.55 (d, *J* = 7.7, 1H), 7.27 (d, *J* = 8.7, 1H), 7.20 (s, 1H), 7.09 (d, J = 9.6, 1H), 6.90 (s, 1H), 4.38 (dd, *J* = 15.7, 6.7, 1H), 4.24 (dd, *J* = 15.8, 5.8, 1H), 3.98-3.86 (m, 2H), 2.64 - 2.55 (m, 1H), 2.22 - 2.07 (m, 1H). | 4.89 min [431] |
| "A236" | 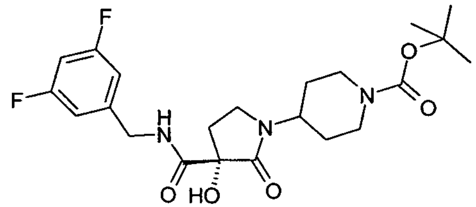 4-[(S)-3-(3,5-Difluor-benzyl-carbamoyl)-3-hydroxy-2-oxo-pyrrolidin-1-yl]-piperidin-1-carbonsäure-tert.-butylester | 8.57 (d, *J* = 6.6, 1H), 7.07 (d, *J* = 9.2, 1H), 6.97 (d, *J* = 6.6, 2H), 6.47 (s, 1H), 4.36 (dd, *J* = 15.9, 6.7, 2H), 4.21 (dd, *J* = 16.0, 6.0, 1H), 4.00 (dd, *J* = 17.8, 12.9, 3H), 3.94-3.84 (m, 2H), 1.97 - 1.86 (m, 2H), 1.59 - 1.48 (m, 4H), 1.39 (s, 9H), 1.23 (s, 1H), 1.14 (t, *J* = 7.5, 2H). | 4.17 min [354.3] |
| "A237" | 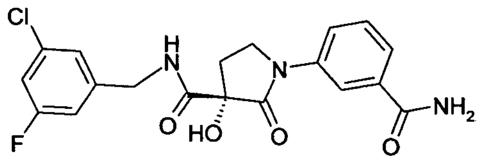 (S)-1-(3-Carbamoyl-phenyl)-3-hydroxy-2-oxo-pyrrolidin-3-carbonsäure-3-chloro-5-fluor-benzylamid | 8.75 (t, *J* = 6.4, 1H), 8.07 - 8.00 (m, 2H), 7.99-7.93 (m, 1H), 7.67 (d, *J* = 7.9, 1H), 7.48 (t, *J* = 8.0, 1H), 7.42 (s, 1H), 7.27 (dt, *J* = 8.8, 2.2, 1H), 7.20 (s, 1H), 7.10 (d, *J* = 9.7, 1H), 6.80 (s, 1H), 4.38 (dd, *J* = 15.8, 6.7, 1H), 4.25 (dd, *J* = 15.7, 6.0, 1H), 3.94-3.86 (m, 2H), 2.64 - 2.56 (m, 1H), 2.14 (dt, *J* = 13.0, 7.6, 1H). | 3.25 min [406] |

(fortgesetzt)

| Nr. | Struktur / Name | $^1$H NMR (400 MHz, DMSO-$d_6$) δ [ppm] | LC-MS; rt; [M+H$^+$]* |
|---|---|---|---|
| "A238" | 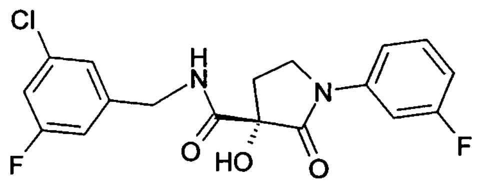 (S)-1-(3-Fluor-phenyl)-3-hydroxy-2-oxo-pyrrolidin-3-carbonsäure-3-chlor-5-fluor-benzylamid | 8.77 (t, *J* = 6.3, 1H), 7.70 (dt, *J* = 11.9, 2.2, 1H), 7.55 - 7.42 (m, 2H), 7.29 (dt, *J* = 8.8, 2.1, 1H), 7.21 (s, 1H), 7.11 (d, *J* = 9.6, 1H), 7.04 (td, *J* = 8.1, 1.6, 1H), 6.83 (s, 1H), 4.39 (dd, *J* = 15.7, 6.7, 1H), 4.25 (dd, *J* = 15.7, 6.0, 1H), 3.93 - 3.83 (m, 2H), 2.64 - 2.55 (m, 1H), 2.14 (dt, *J* = 13.1, 7.7, 1H). | 4.4 min [381] |
| "A239" | 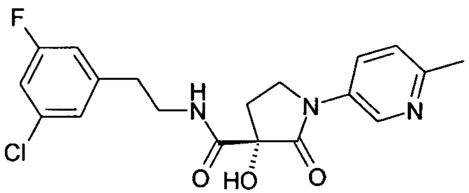 (S)-3-Hydroxy-1-(6-methyl-pyridin-3-yl)-2-oxo-pyrrolidin-3-carbon-säure-[2-(3-chlor-5-fluor-phenyl)-ethyl]-amid | 8.75 (d, *J* = 2.6, 1H), 8.08 (t, *J* = 5.9, 1H), 8.00 (dd, *J* = 8.5, 2.7, 1H), 7.29 (d, *J* = 8.5, 1H), 7.23 (dt, *J* = 8.9, 2.2, 1H), 7.15 (s, 1H), 7.08 (dd, *J* = 9.3, 1.8, 1H), 6.67 (s, 1H), 3.88 - 3.77 (m, 2H), 3.42 - 3.33 (m, 2H), 2.78 (t, *J* = 7.0, 2H), 2.44 (s, 3H), 2.07 (dt, *J* = 12.9, 7.8, 1H). | 3.09 min [392.3] |
| "A240" | 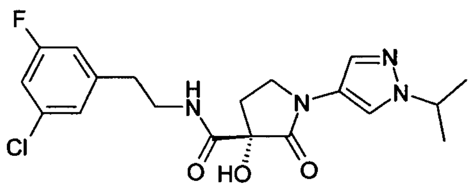 (S)-3-Hydroxy-1-(1-isopropyl-1 H-pyrazol-4-yl)-2-oxo-pyrrolidin-3-carbonsäure-[2-(3-chlor-5-fluorphenyl)-ethyl]-amid | 8.06 - 7.97 (m, 2H), 7.65 (s, 1H), 7.24 (dt, *J* = 8.9, 2.1, 1H), 7.15 (s, 1H), 7.07 (d, *J* = 9.2, 1 H), 6.54 (d, *J* = 8.0, 1H), 4.48 (dt, *J* = 13.3, 6.7, 1H), 3.71 - 3.59 (m, 2H), 3.42 - 3.33 (m, 2H), 2.77 (t, *J* = 7.0, 2H), 2.44 (dd, *J* = 7.8, 3.9, 1H), 2.12 - 1.99 (m, 1H), 1.38 (d, *J* = 6.7, 6H). | 3.94 min [409.3] |
| "A241" | 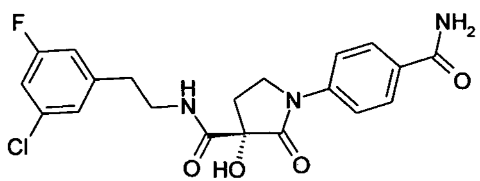 (S)-1-(4-Carbamoyl-phenyl)-3-hydroxy-2-oxo-pyrrolidin-3-carbonsäure-[2-(3-chlor-5-fluorphenyl)-ethyl]-amid | 8.09 (t, *J* = 6.0, 1H), 7.95 (s, 1H), 7.91 (d, *J* = 8.9, 2H), 7.77 (d, *J* = 8.9, 2H), 7.33 (s, 1H), 7.24 (dt, *J* = 8.8, 2.1, 1H), 7.16 (s, 1H), 7.08 (d, *J* = 9.0, 1H), 6.68 (s, 1H), 3.90 - 3.80 (m, 2H), 3.44 - 3.34 (m, 2H), 2.78 (t, *J* = 6.9, 2H), 2.47 - 2.38 (m, 1H), 2.06 (dt, *J* = 12.8, 7.9, 1H). | 3.42 min [420] |

(fortgesetzt)

| Nr. | Struktur / Name | $^1$H NMR (400 MHz, DMSO-$d_6$) δ [ppm] | LC-MS; rt; [M+H$^+$]* |
|---|---|---|---|
| "A242" | 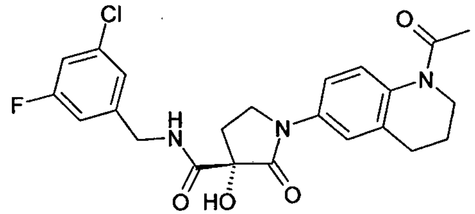 (S)-1-(1-Acetyl-1,2,3,4-tetrahydro-chinolin-6-yl)-3-hydroxy-2-oxo-pyrrolidin-3-carbonsäure-3-chlor-5-fluor-benzylamid | 8.72 (t, *J* = 6.3, 1H), 7.49 (s, 1H), 7.27 (dd, *J* = 8.8, 2.2, 1H), 7.20 (s, 1H), 7.09 (d, *J* = 9.2, 1H), 6.75 (s, 1H), 4.38 (dd, *J* = 15.7, 6.7, 1H), 4.23 (dd, *J* = 15.7, 5.8, 1H), 3.83 (t, *J* = 6.8, 1H), 3.65 (t, *J* = 6.3, 2H), 2.70 (t, *J* = 6.6, 1H), 2.56 (dd, *J* = 12.7, 6.0, 1H), 2.19 - 2.05 (m, 3H), 1.91 - 1.81 (m, 2H). | 3.89 min [460.3] |
| "A243" | 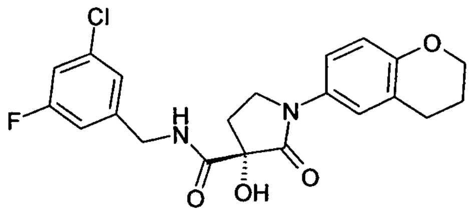 (S)-1-Chroman-6-yl-3-hydroxy-2-oxo-pyrrolidin-3-carbonsäure-3-chlor-5-fluor-benzylamid | 8.69 (t, *J* = 6.4, 1H), 7.36 (dd, *J* = 8.8, 2.7, 1H), 7.31 (d, *J* = 2.6, 1H), 7.27 (dt, *J* = 8.8, 2.1, 1H), 7.20 (s, 1H), 7.09 (d, *J* = 9.0, 1H), 6.74 (d, *J* = 8.8, 1H), 6.68 (s, 1H), 4.37 (dd, *J* = 15.8, 6.7, 1H), 4.23 (dd, *J* = 15.8, 6.0, 1H), 4.14 - 4.08 (m, 2H), 3.81 - 3.74 (m, 2H), 2.73 (t, *J* = 6.4, 2H), 2.60 - 2.51 (m, 1H), 2.08 (dt, *J* = 13.0, 7.6, 1H), 1.95 -1.86 (m, 2H). | 4.41 min [419.3] |
| "A244" | 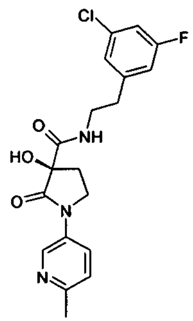 (R)-3-Hydroxy-1-(6-methyl-pyridin-3-yl)-2-oxo-pyrrolidin-3-carbonsäure-[2-(3-chlor-5-fluor-phenyl)-ethyl]-amid | 8.75 (d, *J* = 2.6, 1H), 8.08 (t, *J* = 5.9, 1H), 8.00 (dd, *J* = 8.5, 2.7, 1H), 7.29 (d, *J* = 8.5, 1H), 7.23 (dt, *J* = 8.9, 2.1, 1H), 7.15 (s, 1H), 7.08 (dd, *J* = 9.7, 1.4, 1H), 6.67 (s, 1H), 3.88 - 3.77 (m, 2H), 3.44 - 3.35 (m, 1H), 2.78 (t, *J* = 6.9, 2H), 2.44 (s, 3H),2.43 (m, 1H), 2.07 (dt, *J* = 12.9, 7.8, 1H). | 3.09 min [392.3] |

(fortgesetzt)

| Nr. | Struktur / Name | $^1$H NMR (400 MHz, DMSO-$d_6$) δ [ppm] | LC-MS; rt; [M+H$^+$]* |
|---|---|---|---|
| "A245" | 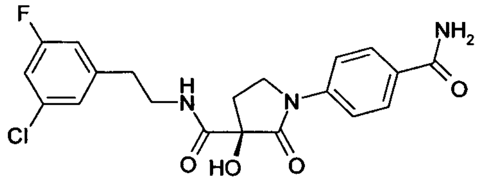 (R)-1 -(4-Carbamoyl-phenyl)-3-hydroxy-2-oxo-pyrrolidin-3-carbonsäure-[2-(3-chlor-5-fluor-phenyl)-ethyl]-amid | 8.09 (t, *J* = 6.0, 1H), 7.95 (s, 1H), 7.91 (d, *J* = 8.9, 2H), 7.77 (d, *J* = 8.9, 2H), 7.33 (s, 1H), 7.24 (dt, *J* = 8.8, 2.1, 1H), 7.16 (s, 1H), 7.08 (d, *J* = 9.0, 1H), 6.68 (s, 1H), 3.91 - 3.79 (m, 2H), 3.43 - 3.35 (m, 2H), 2.78 (t, *J* = 6.9, 2H), 2.47 - 2.38 (m, 1H), 2.06 (dt, *J* = 12.8, 7.9, 1H). | 3.42 min [420] |
| "A246" | 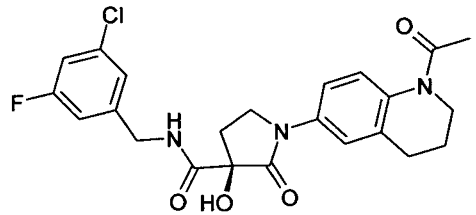 (R)-1-(1-Acetyl-1,2,3,4-tetrahydro-chinolin-6-yl)-3-hydroxy-2-oxo-pyrrolidin-3-carbonsäure-3-chlor-5-fluor-benzylamid | 8.72 (t, *J* = 6.2, 1H), 7.49 (s, 2H), 7.30 - 7.24 (m, 1H), 7.20 (s, 1H), 7.09 (d, *J* = 9.4, 1H), 6.75 (s, 1H), 4.38 (dd, *J* = 15.9, 6.7, 1H), 4.23 (dd, *J* = 15.7, 6.3, 1H), 3.83 (t, *J* = 6.7, 2H), 3.65 (t, *J* = 6.4, 2H), 2.70 (t, *J* = 6.5, 2H), 2.56 (dd, *J* = 12.7, 5.9, 1H), 2.14 (s, 3H), 2.10 (dd, *J* = 12.9, 7.8, 2H), 1.90 - 1.81 (m, 2H). | 3.89 min [460.3] |
| "A247" | 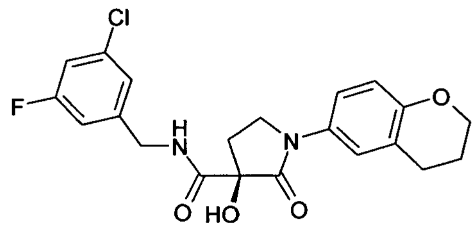 (R)-1-Chroman-6-yl-3-hydroxy-2-oxo-pyrrolidin-3-carbonsäure-3-chlor-5-fluor-benzylamid | 8.69 (t, *J* = 6.4, 1H), 7.36 (dd, *J* = 8.8, 2.7, 1H), 7.31 (d, *J* = 2.6, 1H), 7.27 (dt, *J* = 8.8, 2.1, 1H), 7.20 (s, 1H), 7.09 (d, *J* = 9.6, 1H), 6.74 (d, *J* = 8.8, 1H), 6.68 (s, 1H), 4.37 (dd, *J* = 16.0, 6.9, 1H), 4.23 (dd, *J* = 15.8, 6.0, 1H), 4.14 - 4.07 (m, 2H), 3.81 - 3.74 (m, 2H), 2.73 (t, *J* = 6.4, 2H), 2.60 - 2.51 (m, 1H), 2.08 (dt, *J* = 13.0, 7.6, 1H), 1.95 - 1.86 (m, 2H). | 4.41 min [419.3] |

(fortgesetzt)

| Nr. | Struktur / Name | $^1$H NMR (400 MHz, DMSO-$d_6$) δ [ppm] | LC-MS; rt; [M+H$^+$]* |
|---|---|---|---|
| "A248" | 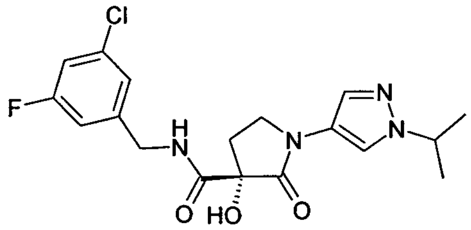 (S)-3-Hydroxy-1-(1-isopropyl-1 H-pyrazol-4-yl)-2-oxo-pyrrolidin-3-carbonsäure-3-chlor-5-fluor-benzylamid | 8.70 (t, J = 6.4, 1H), 8.03 (s, 1H), 7.66 (s, 1H), 7.27 (dt, *J* = 8.8, 2.1, 1H), 7.19 (s, 1H), 7.09 (d, *J* = 9.6, 1H), 6.69 (s, 1H), 4.48 (dt, *J* = 13.3, 6.7, 1H), 4.36 (dd, *J* = 15.7, 6.7, 1H), 4.23 (dd, *J* = 15.7, 6.0, 1H), 3.74 - 3.63 (m, 2H), 2.60 (ddd, *J* = 12.1, 7.6, 4.2, 1H), 2.17 - 2.09 (m, 1 H), 1.38 (d, *J* = 6.7, 6H). | 3.78 min [395] |
| "A249" | 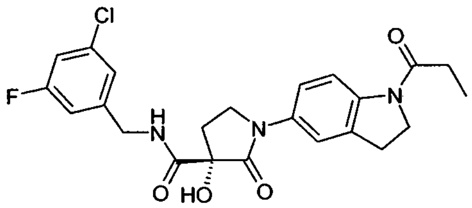 (S)-3-Hydroxy-2-oxo-1-(1-propionyl-2,3-dihydro-1H-indol-5-yl)-pyrrolidin-3-carbonsäure-3-chlor-5-fluor-benzylamid | 8.71 (t, *J* = 6.4, 1H), 8.05 (d, *J* = 8.7, 1H), 7.62 (s, 1H), 7.40 (d, *J* = 8.4, 1H), 7.27 (dd, *J* = 8.7, 2.2, 1H), 7.20 (s, 1H), 7.10 (d, *J* = 8.8, 1H), 6.72 (s, 1 H), 4.38 (dd, *J* = 15.5, 6.7, 1H), 4.23 (dd, *J* = 15.8, 5.9, 1H), 4.08 (t, *J* = 8.5, 2H), 3.84 - 3.79 (m, 2H), 3.15 (t, *J*= 8.5, 2H), 2.61 - 2.52 (m, 2H), 2.46 - 2.41 (m, 2H), 2.10 (dt, *J* = 13.0, 7.4, 1H), 1.07 - 1.02 (m, 3H). | 4.07 min [460] |
| "A250" | 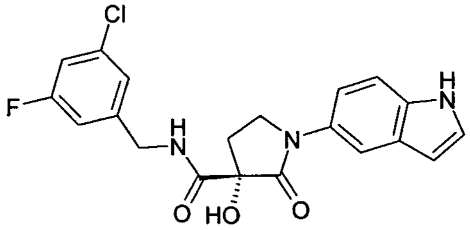 (S)-3-Hydroxy-1-(1H-indol-5-yl)-2-oxo-pyrrolidin-3-carbonsäure-3-chlor-5-fluor-benzylamid | 11.12 (s, 1H), 8.69 (d, *J* = 6.7, 1H), 7.70 (s, 1H), 7.44 - 7.34 (m, 3H), 7.27 (d, *J* = 8.8, 1H), 7.22 (s, 1H), 7.12 (d, *J* = 9.5, 1H), 6.69 (s, 1H), 6.42 (s, 1H), 4.40 (dd, *J* = 15.8, 7.1, 1H), 4.25 (dd, *J* = 15.8, 5.8, 1H), 3.90 - 3.84 (m, 2H), 2.64 - 2.55 (m, 2H), 2.12 (dt, *J* = 12.9, 7.6, 1H). | 3.94 min [402] |

(fortgesetzt)

| Nr. | Struktur / Name | $^1$H NMR (400 MHz, DMSO-$d_6$) δ [ppm] | LC-MS; rt; [M+H$^+$]* |
|---|---|---|---|
| "A251" | 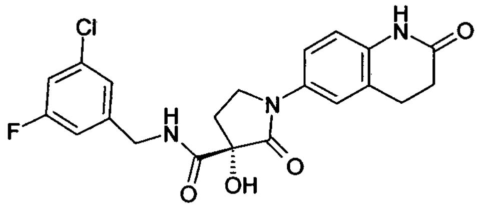 (S)-3-Hydroxy-2-oxo-1-(2-oxo-1,2,3,4-tetrahydro-chinolin-6-yl)-pyrrolidin-3-carbonsäure-3-chlor-5-fluor-benzylamid | 12.06 (s, 1H), 10.10 (s, 1H), 8.70 (t, *J* = 6.3, 1H), 7.65 (s, 1H), 7.47 (s, 1H), 7.43 (dd, *J* = 8.6, 2.4, 1H), 7.27 (dt, *J* = 8.7, 2.1, 1H), 7.20 (s, 1H), 7.09 (d, *J* = 8.9, 1H), 7.03 (s, 2H), 6.85 (d, *J* = 8.6, 1H), 6.72 (s, 1H), 4.37 (dd, *J* = 15.7, 6.8, 1H), 4.23 (dd, *J* = 15.7, 6.1, 1H), 3.79 (t, *J* = 6.8, 1H), 2.87 (t, *J* = 7.6, 1H), 2.61 - 2.52 (m, 1H), 2.43 (dd, *J* = 8.3, 6.7, 2H), 2.09 (dt, *J* = 12.9, 7.6, 1H). | 3.38 min [432] |
| "A252" | 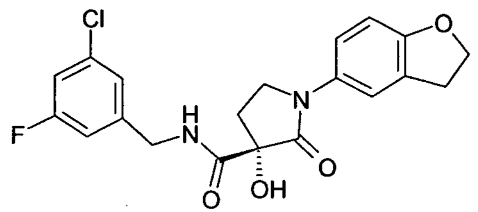 (S)-1-(2,3-Dihydro-benzofuran-5-yl)-3-hydroxy-2-oxo-pyrrolidin-3-carbonsäure-3-chlor-5-fluor-benzylamid | 8.69 (t, *J* = 6.3, 1H), 7.53 (s, 1H), 7.32 (dd, *J* = 8.6, 2.2, 1H), 7.27 (d, *J* = 8.7, 1H), 7.20 (s, 1H), 7.10 (d, *J* = 9.2, 1H), 6.77 (d, *J* = 8.6, 1H), 6.69 (s, 1H), 4.52 (t, *J* = 8.7, 2H), 4.38 (dd, *J* = 15.7, 6.7, 1H), 4.23 (dd, *J* = 15.7, 6.1, 1H), 3.82 - 3.76 (m, 2H), 3.18 (t, *J* = 8.7, 2H), 2.62 - 2.52 (m, 1H), 2.09 (dt, *J* = 12.9, 7.6, 1H). | 4.06 min [405] |
| "A253" | 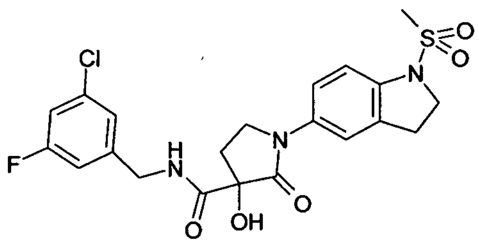 3-Hydroxy-1-(1-methansulfonyl-2,3-dihydro-1H-indol-5-yl)-2-oxo-pyrrolidin-3-carbonsäure-3-chlor-5-fluor-benzylamid | 8.71 (t, *J* = 6.3, 1H), 7.64 (d, *J* = 2.0, 1H), 7.46 (dd, *J* = 8.6, 2.1, 1H), 7.29-7.23 (m, 2H), 7.20 (s, 1H), 7.09 (d, *J* = 9.6, 1H), 6.74 (s, 1H), 4.38 (dd, *J* = 15.7, 6.6, 1H), 4.23 (dd, *J* = 15.8, 6.0, 1H), 3.94 (t, *J* = 8.5, 2H), 3.85 - 3.78 (m, 2H), 3.13 (t, *J* = 8.4, 2H), 2.96 (s, 2H), 2.61 - 2.53 (m, 1H), 2.11 (dt, *J* = 13.0, 7.7, 1H). | 3.99 min [482] |

(fortgesetzt)

| Nr. | Struktur / Name | $^1$H NMR (400 MHz, DMSO-$d_6$) δ [ppm] | LC-MS; rt; $[M+H^+]$* |
|---|---|---|---|
| "A254" | 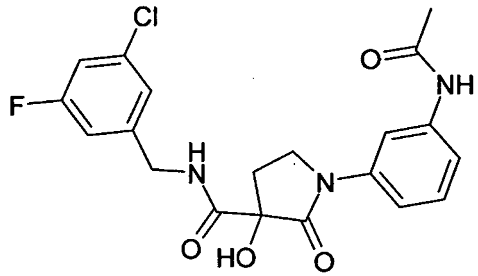<br>1-(3-Acetylamino-phenyl)-3-hydroxy-2-oxo-pyrrolidin-3-carbonsäure-3-chlor-5-fluor-benzylamid | 10.02 (s, 1H), 8.73 (s, 1H), 7.93 (s, 1H), 7.48-7.43 (m, 1H), 7.34 - 7.24 (m, 3H), 7.20 (s, 1H), 7.09 (d, $J$ = 9.7, 1H), 6.78 (s, 1H), 4.38 (dd, $J$ = 15.7, 6.2, 1H), 4.24 (dd, $J$ = 15.7, 5.3, 1H), 3.81 (dd, $J$ = 8.3, 5.6, 2H), 2.62 - 2.53 (m, 1H), 2.12 (m, 1H), 2.03 (s, 3H). | 3.53 min [420] |
| "A255" | 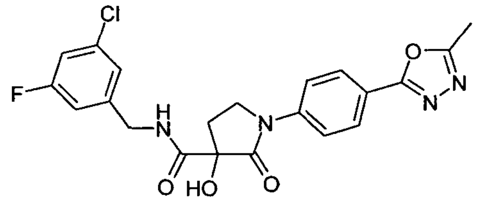<br>3-Hydroxy-1-[4-(5-methyl-[1,3,4]oxadiazol-2-yl)-phenyl]-2-oxo-pyrrolidin-3-carbonsäure-3-chlor-5-fluor-benzylamid | 8.78 (t, $J$ = 6.4, 1H), 8.03 - 7.98 (m, 2H), 7.97-7.92 (m, 2H), 7.27 (dt, $J$ = 8.8, 2.1, 1H), 7.20 (s, 1H), 7.10 (d, $J$ = 9.6, 1H), 6.86 (s, 1H), 4.38 (dd, $J$ = 15.7, 6.7, 1H), 4.24 (dd, $J$ = 15.7, 6.0, 1H), 3.97 - 3.87 (m, 2H), 2.65 - 2.58 (m, 1H), 2.16 (m, 1H). | 3.76 min [445] |
| "A256" | 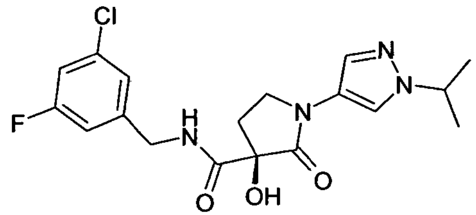<br>(R)-3-Hydroxy-1-(1-isopropyl-1H-pyrazol-4-yl)-2-oxo-pyrrolidin-3-carbonsäure-3-chlor-5-fluor-benzylamid | | 3.71 min [395] |

(fortgesetzt)

| Nr. | Struktur / Name | $^1$H NMR (400 MHz, DMSO-$d_6$) δ [ppm] | LC-MS; rt; [M+H$^+$]* |
|---|---|---|---|
| "A257" | 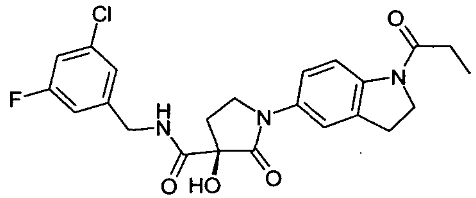 (R)-3-Hydroxy-2-oxo-1-(1-propionyl-2,3-dihydro-1H-indol-5-yl)-pyrrolidin-3-carbonsäure-3-chlor-5-fluor-benzylamid | 8.71 (t, $J$ = 6.4, 1H), 8.05 (d, $J$ = 8.6, 1H), 7.62 (s, 1H), 7.40 (d, $J$ = 8.5, 1H), 7.27 (dt, $J$ = 8.9, 2.2, 1H), 7.20 (s, 1H), 7.10 (d, $J$ = 9.5, 1H), 6.72 (s, 1H), 4.38 (dd, $J$ = 15.7, 6.7, 1H), 4.23 (dd, $J$ = 15.6, 6.0, 1H), 4.08 (t, $J$ = 8.6, 2H), 3.84 - 3.79 (m, 2H), 3.15 (t, $J$ = 8.5, 2H), 2.61 - 2.52 (m, 2H), 2.45 (d, $J$ = 7.4, 2H), 2.15 - 2.06 (m, 1H), 1.07 - 1.02 (m, 3H). | 4.00 min [460] |
| "A258" | 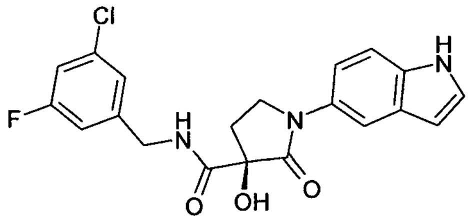 (R)-3-Hydroxy-1-(1H-indol-5-yl)-2-oxo-pyrrolidin-3-carbonsäure-3-chlor-5-fluor-benzylamid | 11.12 (s, 1H), 8.70 (t, $J$ = 6.4, 1H), 7.70 (s, 1H), 7.45 - 7.34 (m, 3H), 7.27 (d, $J$ = 8.6, 1H), 7.22 (s, 1H), 7.12 (d, $J$ = 9.6, 1H), 6.69 (s, 1H), 6.42 (s, 1H), 4.40 (dd, $J$ = 15.4, 6.6, 1H), 4.30 - 4.23 (m, 1H), 3.87 (t, $J$ = 6.7, 2H), 2.63 - 2.56 (m, 1H), 2.12 (dt, $J$ = 12.9, 7.6, 1H). | 3.94 min [402] |
| "A259" | 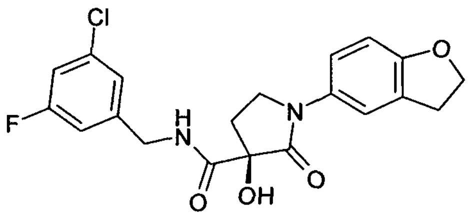 (R)-1-(2,3-Dihydro-benzofuran-5-yl)-3-hydroxy-2-oxo-pyrrolidin-3-carbonsäure-3-chlor-5-fluor-benzylamid | 8.69 (t, $J$ = 6.4, 1H), 7.55 -7.52 (m, 1H), 7.32 (dd, $J$ = 8.6, 2.4, 1H), 7.27 (dt, $J$ = 8.8, 2.2, 1H), 7.20 (s, 1H), 7.10 (d, $J$ = 9.7, 1H), 6.77 (d, $J$ = 8.6, 1H), 6.69 (s, 1H), 4.52 (t, $J$ = 8.7, 2H), 4.38 (dd, $J$ = 15.7, 6.7, 1H), 4.23 (dd, $J$ = 15.8, 6.0, 1H), 3.78 (dd, $J$ = 7.5, 6.1, 2H), 3.18 (t, $J$ = 8.7, 2H), 2.60 - 2.52 (m, 1H), 2.09 (dt, $J$ = 12.9, 7.6, 1H). | 4.05 min [405] |

(fortgesetzt)

| Nr. | Struktur / Name | $^1$H NMR (400 MHz, DMSO-$d_6$) δ [ppm] | LC-MS; rt; [M+H$^+$]* |
|---|---|---|---|
| "A260" | 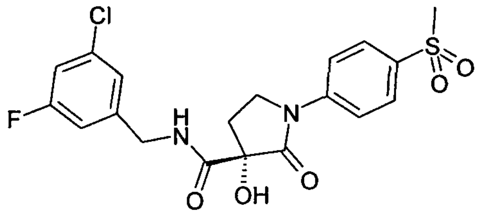 (S)-3-Hydroxy-1-(4-methansulfonyl-phenyl)-2-oxo-pyrrolidin-3-carbonsäure-3-chlor-5-fluor-benzylamid | 8.78 (t, *J* = 6.3, 1H), 8.00 - 7.93 (m, 3H), 7.27 (dt, *J* = 8.8, 2.1, 1H), 7.20 (s, 1H), 7.09 (d, *J* = 9.8, 1H), 6.88 (s, 1H), 4.38 (dd, *J* = 15.4, 6.6, 1H), 4.24 (dd, *J* = 15.8, 5.9, 1H), 3.92 (t, *J* = 6.1, 2H), 2.60 (dt, *J* = 7.1, 5.8, 1H), 2.21 - 2.12 (m, 1H). | 4.98 min [439] |
| "A261" | 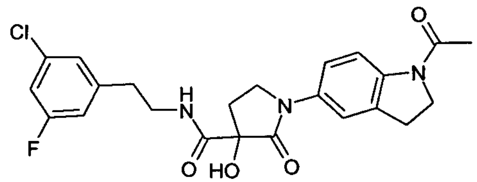 1-(1-Acetyl-2,3-dihydro-1H-indol-5-yl)-3-hydroxy-2-oxo-pyrrolidin-3-carbonsäure-[2-(3-chlor-5-fluorphenyl)-ethyl]-amid | 8.05 (t, *J* = 6.0, 1H), 8.00 (d, *J* = 8.8, 1H), 7.61 (s, 1H), 7.42 - 7.34 (m, 1H), 7.24 (dt, J = 8.8, 2.1, 1H), 7.16 (s, 1H), 7.08 (d, *J* = 9.1, 1H), 4.09 (t, *J* = 8.6, 2H), 3.81 - 3.74 (m, 2H), 3.43 - 3.23 (m, 4H), 3.15 (t, *J* = 8.5, 2H), 2.77 (t, *J* = 7.0, 2H), 2.46 - 2.37 (m, 1H), 2.14 (s, 3H), 2.03 (dt, *J* = 12.7, 7.8, 1H). | 3.83 min [460.3] |
| "A262" | 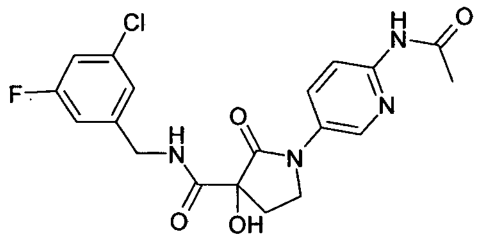 1-(6-Acetylamino-pyridin-3-yl)-3-hydroxy-2-oxo-pyrrolidin-3-carbonsäure-3-chlor-5-fluor-benzylamid | 10.54 (s, 1H), 8.76 (t, *J* = 6.4, 1H), 8.66 - 8.63 (m, 1H), 8.13-8.05 (m, 2H), 7.27 (dt, *J* = 8.8, 2.1, 1H), 7.20 (s, 1H), 7.10 (d, *J* = 9.6, 1H), 6.80 (s, 1H), 4.38 (dd, *J* = 15.7, 6.8, 1H), 4.24 (dd, *J* = 15.8, 6.1, 1H), 3.92 - 3.81 (m, 2H), 2.60 (ddd, *J* = 12.0, 7.1,4.8, 1H), 2.14 (dt, *J* = 13.0, 7.6, 1H), 2.07 (s, 3H). | 3.03 min [421] |

(fortgesetzt)

| Nr. | Struktur / Name | $^1$H NMR (400 MHz, DMSO-$d_6$) δ [ppm] | LC-MS; rt; [M+H$^+$]* |
|---|---|---|---|
| "A263" | 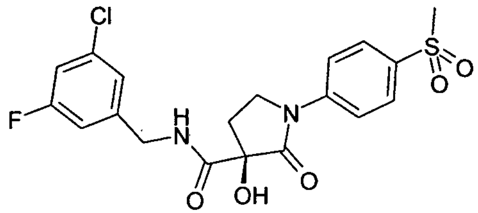 (R)-3-Hydroxy-1-(4-methan-sulfonyl-phenyl)-2-oxo-pyrrolidin-3-carbonsäure-3-chlor-5-fluor-benzylamid | 8.78 (t, *J* = 6.4, 1H), 8.00 - 7.92 (m, 3H), 7.27 (dt, *J* = 8.8, 2.1, 1H), 7.20 (s, 1H), 7.09 (d, *J* = 8.8, 1H), 6.88 (s, 1H), 4.38 (dd, *J* = 16.1,6.7, 1H), 4.24 (dd, *J* = 15.8, 6.0, 1H), 3.92 (t, *J* = 6.1, 2H), 3.19 (s, 3H), 2.65 - 2.57 (m, 1 H), 2.16 (dt, *J* = 13.1, 7.7, 1H). | 5.01 min [439] |
| "A264" | 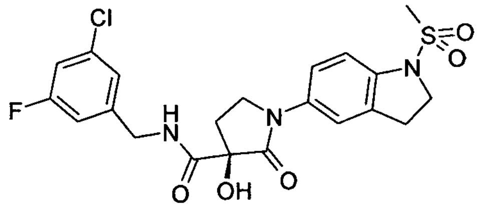 (R)-3-Hydroxy-1-(1-methan-sulfonyl-2,3-dihydro-1H-indol-5-yl)-2-oxo-pyrrolidin-3-carbonsäure-3-chlor-5-fluor-benzylamid | 8.70 (t, *J* = 6.3, 1H), 7.64 (d, *J* = 2.0, 1H), 7.46 (dd, *J* = 8.6, 2.1, 1H), 7.29-7.23 (m, 2H), 7.20 (s, 1H), 7.09 (d, *J* = 9.6, 1H), 6.74 , (s, 1H), 4.38 (dd, *J* = 15.8, 6.6, 1H), 4.23 (dd, *J* = 15.8, 6.0, 1H), 3.94 (t, *J* = 8.5, 2H), 3.85 - 3.78 (m, 2H), 3.13 (t, *J* = 8.4, 2H), 2.96 (s, 2H), 2.61 - 2.53 (m, 1H), 2.11 (dt, *J* = 13.0, 7.7, 1H). | $$$ 10.55 min 100% purity |
| "A265" | 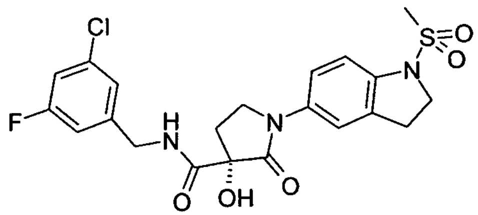 (S)-3-Hydroxy-1-(1-methansulfonyl-2,3-dihydro-1H-indol-5-yl)-2-oxo-pyrrolidin-3-carbonsäure-3-chlor-5-fluor-benzylamid | 8.72 (t, J = 6.3, 1H), 7.64 (d, *J* = 2.0, 1H), 7.46 (dd, *J* = 8.6, 2.1, 1H), 7.29-7.23 (m, 2H), 7.21 (s, 1H), 7.09 (d, *J* = 9.6, 1H), 6.74 (s, 1H), 4.38 (dd, *J* = 15.7, 6.6, 1H), 4.23 (dd, *J* = 15.7, 6.0, 1H), 3.94 (t, *J* = 8.5, 2H), 3.85 - 3.78 (m, 2H), 3.13 (t, *J* = 8.4, 2H), 2.96 (s, 2H), 2.61 - 2.53 (m, 1H), 2.11 (dt, *J* = 13.0, 7.7, 1H). | $$$ 6.50 min 100% purity |

(fortgesetzt)

| Nr. | Struktur / Name | $^1H$ NMR (400 MHz, DMSO-$d_6$) δ [ppm] | LC-MS; rt; $[M+H^+]$* |
|---|---|---|---|
| "A266" | 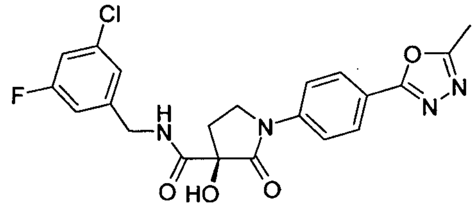 (R)-3-Hydroxy-1-[4-(5-methyl-[1,3,4]oxadiazol-2-yl)-phenyl]-2-oxo-pyrrolidin-3-carbonsäure-3-chlor-5-fluor-benzylamid | 8.78 (t, *J* = 6.4, 1H), 8.04 - 7.98 (m, 2H), 7.97-7.91 (m, 2H), 7.27 (dt, *J* = 8.8, 2.1, 1H), 7.20 (s, 1H), 7.10 (d, *J* = 9.6, 1H), 6.86 (s, 1H), 4.38 (dd, *J* = 15.7, 6.7, 1H), 4.24 (dd, *J* = 15.7, 6.0, 1H), 3.97 - 3.87 (m, 2H), 2.65 - 2.58 (m, 1H), 2.17 - 2.15 (m, 1H). | $$$ 14.31 min 100% purity |
| "A267" | 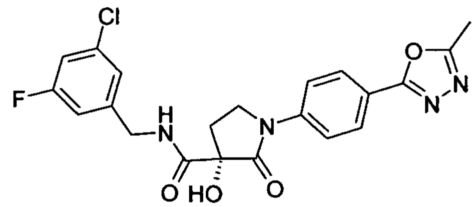 (S)-3-Hydroxy-1-(1-methansulfonyl-2,3-dihydro-1H-indol-5-yl)-2-oxo-pyrrolidin-3-carbonsäure-3-chlor-5-fluor-benzylamid | 8.79 (t, *J* = 6.4, 1H), 8.00 (m, 2H), 7.97 - 7.92 (m, 2H), 7.27 (dt, *J* = 8.8, 2.1, 1H), 7.20 (s, 1H), 7.10 (d, *J* = 9.6, 1H), 6.86 (s,1H), 4.38 (dd, *J* = 15.7, 6.7, 1H), 4.24 (dd, *J* = 15.7, 6.0, 1H), 3.97 - 3.87 (m, 2H), 2.65 - 2.58 (m, 1H), 2.16 (m, 1H). | $$$ 5.54 min 89% purity |
| "A268" | 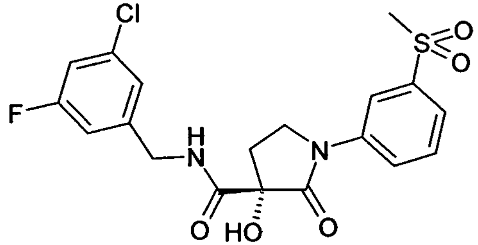 (S)-3-Hydroxy-1-(3-methansulfonyl-phenyl)-2-oxo-pyrrolidin-3-carbonsäure-3-chlor-5-fluor-benzylamid | 8.79 (t, *J* = 6.4, 1H), 8.32 (s, 1H), 7.99 (d, *J* = 7.7, 1H), 7.71 (dt, *J* = 15.6, 7.8, 2H), 7.27 (d, *J* = 8.9, 1H), 7.20 (s, 1H), 7.10 (d, *J* = 9.6, 1H), 6.87 (s, 1H), 4.37 (dd, *J* = 16.1, 6.7, 1H), 4.25 (dd, *J* = 15.7, 6.1, 1H), 3.93 (t, *J* = 6.9, 2H), 3.23 (s, 3H), 2.64-2.57 (m, 2H), 2.45 - 2.38 (m, 1H), 2.16 (dt, *J* = 13.3, 7.9, 1H). | 4.99 min [441] |

(fortgesetzt)

| Nr. | Struktur / Name | $^1$H NMR (400 MHz, DMSO-$d_6$) δ [ppm] | LC-MS; rt; [M+H$^+$]* |
|---|---|---|---|
| "A269" | 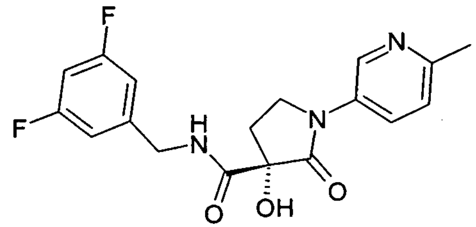 (S)-3-Hydroxy-1-(6-methyl-pyridin-3-yl)-2-oxo-pyrrolidin-3-carbonsäure-3,5-difluor-benzylamid | 8.74 (dd, *J* = 7.5, 4.6, 2H), 8.01 (dd, *J* = 8.5, 2.7, 1H), 7.30 (d, *J* = 8.5, 1H), 7.07 (tt, *J* = 9.4, 2.4, 1H), 6.99 (t, *J* = 6.4, 2H), 6.81 (s, 1H), 4.38 (dd, *J* = 15.8, 6.8, 1H), 4.25 (dd, *J* = 15.9, 6.1, 1H), 3.87 (t, *J* = 6.8, 2H), 2.64 - 2.55 (m, 1H), 2.45 (s, 3H), 2.14 (dt, *J* = 13.0, 7.5, 1H). | 2.49 min [362] |
| "A270" | 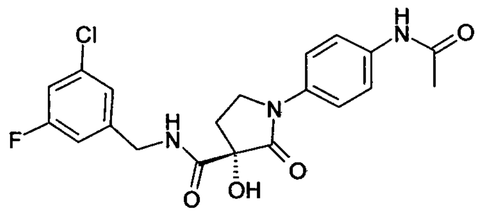 (S)-1-(4-Acetylamino-phenyl)-3-hydroxy-2-oxo-pyrrolidin-3-carbonsäure-3-chlor-5-fluor-benzylamid | 9.96 (s, 1H), 8.71 (t, *J* = 6.5, 1H), 7.66 - 7.55 (m, 4H), 7.27 (d, *J* = 8.5, 1H), 7.20 (s, 1H), 7.09 (d, *J* = 9.2, 1H), 6.73 (s, 1H), 4.38 (dd, *J* = 15.4, 6.6, 1H), 4.23 (dd, *J* = 15.7, 5.9, 1H), 3.81 (t, *J* = 6.8, 2H), 2.62 - 2.53 (m, 2H), 2.16 - 2.05 (m, 2H), 2.02 (s, 3H). | 3.37 min [420] |
| "A271" | 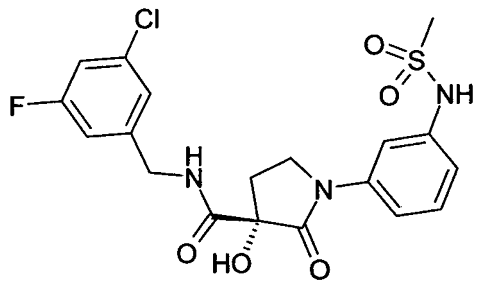 (S)-3-Hydroxy-1-(3-methansulfonyl-amino-phenyl)-2-oxo-pyrrolidin-3-carbonsäure-3-chlor-5-fluor-benzylamid | 9.84 (s, 1H), 8.73 (t, *J* = 6.4, 1H), 7.67 (s, 1H), 7.35 (dd, *J* = 3.9, 2.1, 2H), 7.27 (dt, *J* = 8.8, 2.2, 1H), 7.20 (s, 1H), 7.09 (d, *J* = 8.9, 1H), 7.05 - 7.00 (m, 1H), 6.79 (s, 1H), 4.37 (dd, *J* = 15.7, 6.7, 1H), 4.24 (dd, *J* = 15.7, 6.0, 1H), 3.87 - 3.77 (m, 2H), 2.99 (s, 3H), 2.63 - 2.53 (m, 1H), 2.12 (dt, *J* = 13.0, 7.8, 1H). | 3.72 min [456] |

(fortgesetzt)

| Nr. | Struktur / Name | $^1$H NMR (400 MHz, DMSO-$d_6$) δ [ppm] | LC-MS; rt; [M+H$^+$]* |
|---|---|---|---|
| "A272" | 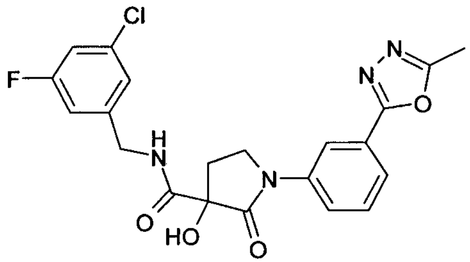 3-Hydroxy-1-[3-(5-methyl-[1,3,4]oxadiazol-2-yl)-phenyl]-2-oxo-pyrrolidin-3-carbonsäure-3-chlor-5-fluor-benzylamid | 8.79 (t, *J* = 6.4, 1H), 8.44 (t, *J* = 1.8, 1H), 7.84-7.76 (m, 2H), 7.63 (t, *J* = 8.0, 1H), 7.27 (dt, *J* = 8.7, 2.1, 1H), 7.21 (s, 1H), 7.10 (d, *J* = 9.5, 1H), 6.86 (s, 1H), 4.38 (dd, *J* = 15.8, 6.7, 1H), 4.25 (dd, *J* = 15.7, 6.0, 1H), 3.99 - 3.84 (m, 2H), 2.65 - 2.60 (m, 1H), 2.59 (s, 3H), 2.22-2.10 (m, 1H). | 3.80 min [445] |
| "A273" | 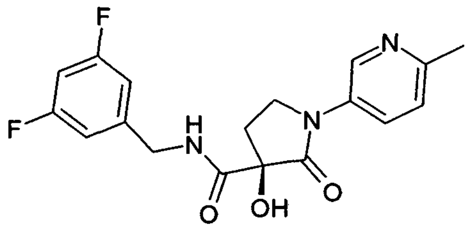 (R)-3-Hydroxy-1-(6-methyl-pyridin-3-yl)-2-oxo-pyrrolidin-3-carbonsäure-3,5-difluor-benzylamid | 8.74 (dd, *J* = 7.6, 4.7, 2H), 8.01 (dd, *J* = 8.5, 2.7, 1H), 7.30 (d, *J* = 8.5, 1H), 7.07 (tt, *J* = 9.3, 2.3, 1H), 6.99 (t, *J* = 6.4, 2H), 6.81 (s, 1H), 4.38 (dd, *J* = 15.9, 6.7, 1H), 4.25 (dd, *J* = 15.8, 6.0, 1H), 3.87 (t, *J* = 6.8, 2H), 2.65 - 2.56 (m, 1H), 2.45 (s, 3H), 2.14 (dt, *J* = 13.1, 7.5, 1H). | 2.48 min [362] |
| "A274" | 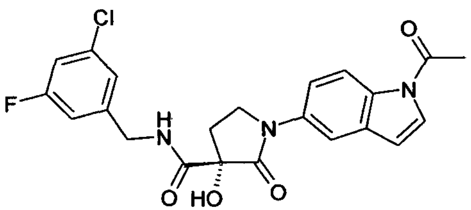 (S)-1-(1-Acetyl-1H-indol-5-yl)-3-hydroxy-2-oxo-pyrrolidin-3-carbonsäure-3-chlor-5-fluor-benzylamid | 8.73 (t, *J* = 6.4, 1H), 8.31 (d, *J* = 9.0, 1H), 7.92 (d, *J* = 2.1, 1H), 7.88 (d, *J* = 3.8, 1H), 7.65 (dd, *J* = 9.0, 2.2, 1H), 7.27 (dt, *J* = 8.8, 2.1, 1H), 7.21 (s, 1H), 7.11 (d, *J* = 9.6, 1H), 6.76 (d, *J* = 4.0, 2H), 4.39 (dd, *J* = 15.7, 6.7, 1H), 4.25 (dd, *J* = 15.7, 6.1, 1H), 3.91 (t, *J* = 6.8, 2H), 2.64 (s, 3H), 2.62 - 2.55 (m, 1H), 2.14 (dt, *J* = 12.9, 7.5, 1H). | 4.10 min [444] |

(fortgesetzt)

| Nr. | Struktur / Name | $^1$H NMR (400 MHz, DMSO-$d_6$) δ [ppm] | LC-MS; rt; [M+H$^+$]* |
|---|---|---|---|
| "A275" | 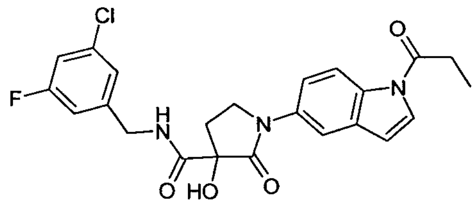 3-Hydroxy-2-oxo-1-(1-propionyl-1H-indol-5-yl)-pyrrolidin-3-carbonsäure-3-chlor-5-fluor-benzylamid | 8.72 (t, *J* = 6.4, 1H), 8.34 (d, *J* = 9.0, 1H), 7.94-7.90 (m, 2H), 7.66 (dd, *J* = 9.0, 2.2, 1H), 7.27 (dt, *J* = 8.8, 2.1, 1H), 7.21 (s, 1H), 7.11 (d, *J* = 9.7, 1H), 6.76 (d, *J* = 4.3, 2H), 4.39 (dd, *J* = 15.7, 6.7, 1H), 4.25 (dd, *J* = 15.7, 6.0, 1H), 3.91 (t, *J* = 6.8, 2H), 3.06 (q, *J* = 7.3, 2H), 2.61 (dt, *J* = 11.9, 5.7, 1H), 2.14 (dt, *J* = 13.0, 7.6, 1H), 1.17 (t, *J* = 7.3, 3H). | 4.42 min [458] |
| "A276" | 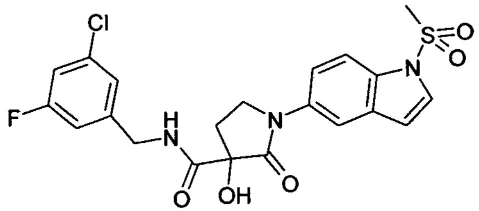 3-Hydroxy-1-(1-methansulfonyl-1H-indol-5-yl)-2-oxo-pyrrolidin-3-carbonsäure-3-chlor-5-fluor-benzylamid | 8.72 (t, *J* = 6.4, 1H), 7.94 (d, *J* = 2.0, 1H), 7.84 (d, *J* = 9.0, 1H), 7.71 (dd, *J* = 9.1, 2.1, 1H), 7.59 (d, *J* = 3.6, 1H), 7.30 - 7.25 (m, 1H), 7.21 (s, 1H), 7.11 (d, *J* = 8.9, 1H), 6.86 (d, *J* = 3.6, 1H), 6.76 (s, 1H), 4.39 (dd, *J* = 15.7, 6.6, 1H), 4.25 (dd, *J* = 15.7, 5.9, 1H), 3.95 - 3.85 (m, 2H), 3.41 (s, 3H), 2.65-2.57 (m, 1H), 2.15 (dt, *J* = 13.1, 7.6, 1H). | 4.24 min [480.0] |
| "A277" | 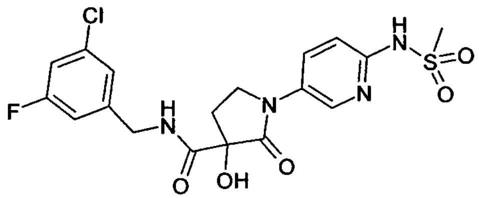 3-Hydroxy-1-(6-methansulfonyl-amino-pyridin-3-yl)-2-oxo-pyrrolidin-3-carbonsäure-3-chlor-5-fluor-benzylamid | 10.56 (s, 1H), 8.74 (t, *J* = 6.5, 1H), 8.57 (s, 1H), 8.11 (dd, *J* = 9.0, 2.7, 1H), 7.27 (d, *J* = 8.7, 1H), 7.20 (s, 1H), 7.10 (d, *J* = 9.8, 1H), 7.02 (d, *J* = 8.9, 1H), 6.80 (s, 1H), 4.38 (dd, *J* = 15.9, 6.7, 2H), 4.24 (dd, *J* = 15.7, 5.8, 1H), 3.88-3.82 (m, 2H), 3.28 (s, 3H), 2.60 (dd, *J* = 13.0, 6.2, 2H), 2.15 (dd, *J* = 14.3, 6.5, 2H). | 3.26 min [457] |

(fortgesetzt)

| Nr. | Struktur / Name | $^1$H NMR (400 MHz, DMSO-$d_6$) δ [ppm] | LC-MS; rt; [M+H$^+$]* |
|---|---|---|---|
| "A278" | 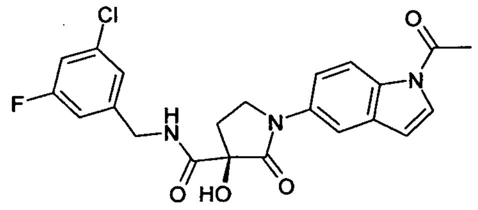 (R)-1-(1-Acetyl-1H-indol-5-yl)-3-hydroxy-2-oxo-pyrrolidin-3-carbonsäure-3-chlor-5-fluor-benzylamid | 8.72 (t, *J* = 6.3, 1H), 8.31 (d, *J* = 9.0, 1H), 7.92 (d, *J* = 2.0, 1H), 7.88 (d, *J* = 3.8, 1H), 7.65 (dd, *J* = 9.0, 2.1, 1H), 7.30 - 7.24 (m, 1H), 7.21 (s, 1H), 7.11 (d, *J* = 9.7, 1H), 6.76 (d, *J* = 5.1, 2H), 4.39 (dd, *J* = 15.8, 6.7, 1H), 4.25 (dd, *J* = 15.7, 6.0, 1H), 3.91 (t, *J* = 6.8, 2H), 2.64 (s, 3H), 2.60 (dd, *J* = 12.6, 6.2, 1H), 2.14 (dt, *J* = 13.1, 7.6, 1H). | 4.09 min [444] |
| "A279" | 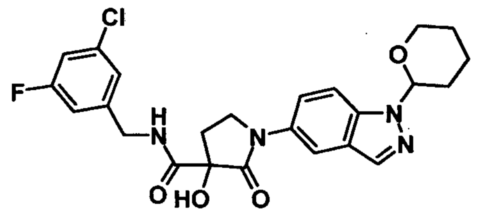 3-Hydroxy-2-oxo-1-[1-(tetrahydro-pyran-2-yl)-1H-indazol-5-yl]-pyrrolidin-3-carbonsäure-3-chlor-5-fluor-benzylamid | 8.73 (t, *J* = 6.4, 1H), 8.12 (s, 1H), 7.94 (dd, *J* = 10.1, 1.4, 1H), 7.82 (ddd, *J* = 12.4, 9.1, 2.0, 1H), 7.75 (d, *J* = 9.2, 1H), 7.27 (dt, *J* = 8.8, 2.1, 1H), 7.21 (s, 1H), 7.11 (d, *J* = 9.6, 1H), 6.76 (d, *J* = 0.7, 1H), 5.84 (dd, *J* = 9.6, 2.4, 1H), 4.39 (dd, *J* = 15.8, 6.7, 1H), 4.25 (dd, *J* = 15.7, 6.0, 1H), 3.90 (dd, *J* = 17.8, 11.2, 3H), 3.74 (ddd, *J* = 11.5, 9.3, 5.3, 1H), 2.68-2.56 (m, 1H), 2.47 - 2.33 (m, 1H), 2.15 (dt, *J* = 13.0, 7.5, 1H), 2.09 - 1.92 (m, 2H), 1.86 - 1.67 (m, 1H), 1.57 (dd, *J* = 8.0, 4.6, 2H). | 4.34 min [486.1] |
| "A280" | 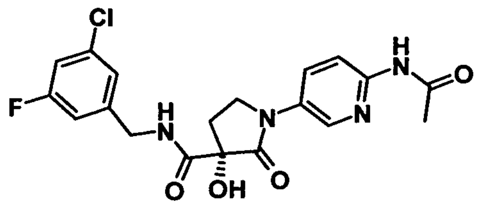 (S)-1-(6-Acetylamino-pyridin-3-yl)-3-hydroxy-2-oxo-pyrrolidin-3-carbonsäure-3-chlor-5-fluor-benzylamid | 10.54 (s, 1H), 8.76 (t, *J* = 6.4, 1H), 8.66 - 8.63 (m, 1H), 8.13 - 8.05 (m, 2H), 7.27 (dt, *J* = 8.8, 2.1, 1H), 7.20 (s, 1H), 7.10 (d, *J* = 9.6, 1H), 6.80 (s, 1H), 4.38 (dd, *J* = 15.7, 6.8, 1H), 4.24 (dd, *J* = 15.8, 6.1, 1H), 3.92 - 3.81 (m, 2H), 2.60 (ddd, *J* = 12.0, 7.1, 4.8, 1H), 2.14 (dt, *J* = 13.0, 7.6, 1H), 2.07 (s, 3H). | 1.873 min [421.1] $ |

(fortgesetzt)

| Nr. | Struktur / Name | $^1H$ NMR (400 MHz, DMSO-$d_6$) δ [ppm] | LC-MS; rt; [M+H$^+$]* |
|---|---|---|---|
| "A281" | 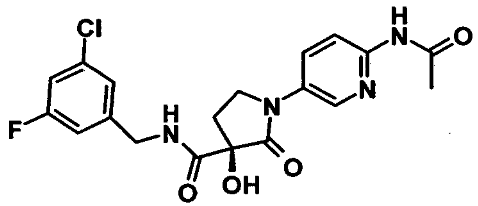 (R)-1-(6-Acetylamino-pyridin-3-yl)-3-hydroxy-2-oxo-pyrrolidin-3-carbonsäure-3-chlor-5-fluor-benzylamid | 10.55 (s, 1H), 8.77 (t, J = 6.4, 1H), 8.66 - 8.63 (m, 1H), 8.13 - 8.05 (m, 2H), 7.27 (dt, J = 8.8, 2.1, 1H), 7.21 (s, 1H), 7.10 (d, J = 9.6, 1H), 6.80 (s, 1H), 4.38 (dd, J = 15.7, 6.8, 1H), 4.24 (dd, J = 15.8, 6.1, 1H), 3.92 - 3.81 (m, 2H), 2.60 (ddd, J = 12.0, 7.1,4.8, 1H), 2.14 (dt, J = 13.0, 7.6, 1H), 2.08 (s, 3H). | 1.874 min [421.0] $ |
| "A282" | 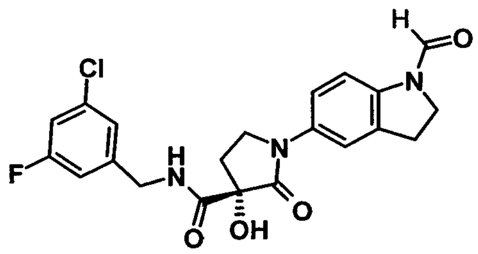 (S)-1-(1-Formyl-2,3-dihydro-1H-indol-5-yl)-3-hydroxy-2-oxo-pyrrolidin-3-carbonsäure-3-chlor-5-fluor-benzylamid | 9.00 (s, 1H), 8.71 (t, J = 6.4, 1H), 7.63 (d, J = 13.1, 1H), 7.45 (s, 1H), 7.31-7.24 (m, 1H), 7.20 (s, 1H), 7.10 (d, J = 9.6, 1H), 6.73 (s, 1H), 4.38 (dd, J = 15.8, 6.7, 1H), 4.24 (dd, J = 15.7, 6.0, 1H), 3.82 (t, J = 6.8, 2H), 3.13 (dt, J = 17.1, 8.6, 2H), 2.62 - 2.53 (m, 1H), 2.11 (dt, J = 12.9, 7.5, 1H). | 3.51 min [432.0] |
| "A283" | 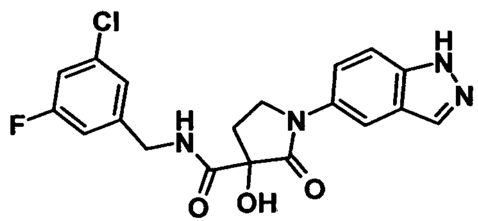 3-Hydroxy-1-(1H-indazol-5-yl)-2-oxo-pyrrolidin-3-carbonsäure-3-chlor-5-fluor-benzylamid | 13.09 (s, 1H), 8.72 (t, J = 6.4, 1H), 8.08 (s, 1H), 7.91 (d, J = 1.5, 1H), 7.76 (dd, J = 9.0, 1.9, 1H), 7.56 (d, J = 9.0, 1H), 7.27 (dt, J = 8.8, 2.1, 1H), 7.21 (s, 1H), 7.11 (d, J = 9.7, 1H), 6.74 (s, 1H), 4.39 (dd, J = 15.7, 6.8, 1H), 4.25 (dd, J = 15.8, 6.0, 1H), 3.91 (t, J = 6.8, 2H), 2.65 - 2.57 (m, 1H), 2.14 (dt, J = 12.9, 7.5, 1H). | 3.41 min [403.0] |

(fortgesetzt)

| Nr. | Struktur / Name | $^1$H NMR (400 MHz, DMSO-$d_6$) δ [ppm] | LC-MS; rt; [M+H$^+$]* |
|---|---|---|---|
| "A284" | 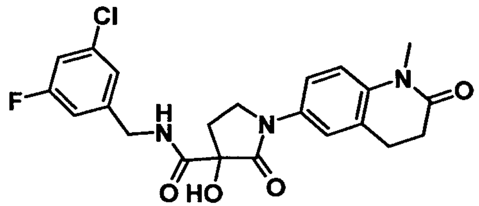 3-Hydroxy-1-(1-methyl-2-oxo-1,2,3,4-tetrahydro-chinolin-6-yl)-2-oxo-pyrrolidin-3-carbonsäure-3-chlor-5-fluor-benzylamid | 8.71 (t, *J* = 6.3, 1H), 7.57 (dd, *J* = 8.8, 2.4, 1H), 7.54 (s, 1H), 7.30 - 7.24 (m, 1H), 7.20 (s, 1H), 7.11 (d, *J* = 8.7, 2H), 6.73 (s, 1H), 4.38 (dd, *J* = 15.8, 6.7, 1H), 4.24 (dd, *J* = 15.7, 6.0, 1H), 3.83 (t, *J* = 6.8, 2H), 3.24 (s, 3H), 2.87 (t, *J* = 7.3, 2H), 2.63 - 2.51 (m, 3H), 2.12 (dt, *J* = 13.0, 7.6, 1H). | 3.67 min [446.0] |
| "A285" | 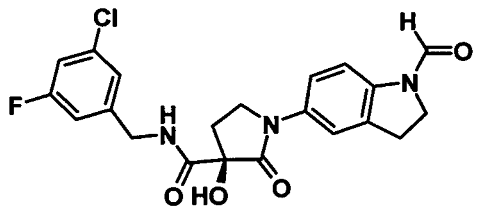 (R)-1-(1-Formyl-2,3-dihydro-1H-indol-5-yl)-3-hydroxy-2-oxo-pyrrolidin-3-carbonsäure-3-chlor-5-fluor-benzylamid | 9.00 (s, 1H), 8.71 (t, *J* = 6.4, 1H), 7.63 (d, *J* = 12.9, 1H), 7.45 (t, *J* = 4.7, 1H), 7.27 (dt, *J* = 8.8, 2.1, 1H), 7.20 (s, 1H), 7.10 (d, *J* = 9.1, 1H), 6.73 (s, 1H), 4.38 (dd, *J* = 15.7, 6.7, 1H), 4.24 (dd, *J* = 15.7, 6.0, 1H), 3.82 (dd, *J* = 8.2, 5.6, 2H), 3.14 (dt, *J* = 17.1, 5.1, 2H), 2.57 (dt, *J* = 6.6, 5.6, 1H), 2.11 (dt, *J* = 13.0, 7.6, 1H). | 3.52 min [432.0] |
| "A286" | 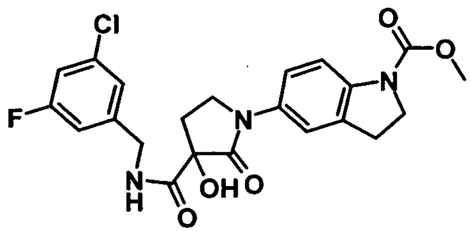 5-[3-(3-Chlor-5-fluor-benzylcarbamoyl)-3-hydroxy-2-oxo-pyrrolidin-1-yl]-2,3-dihydro-indol-1-carbonsäure-methylester | 8.71 (t, *J* = 6.4, 1H), 7.68 (br. s, 1H), 7.59 (s, 1H), 7.45 - 7.40 (m, 1H), 7.27 (dt, *J* = 8.8, 2.2, 1H), 7.20 (s, 1H), 7.10 (d, *J* = 9.6, 1H), 6.73 (s, 1H), 4.38 (dd, *J* = 15.7, 6.7, 1H), 4.24 (dd, *J* = 15.8, 6.0, 1H), 3.96 (t, *J* = 8.7, 2H), 3.81 (t, *J* = 6.9, 2H), 3.73 (s, 3H), 3.11 (t, *J* = 8.6, 2H), 2.56 (dt, *J* = 17.8, 5.5, 1H), 2.10 (dt, *J* = 12.9, 7.6, 1H). | 4.198 min [462] |

(fortgesetzt)

| Nr. | Struktur / Name | $^1$H NMR (400 MHz, DMSO-$d_6$) δ [ppm] | LC-MS; rt; [M+H$^+$]* |
|---|---|---|---|
| "A287" | 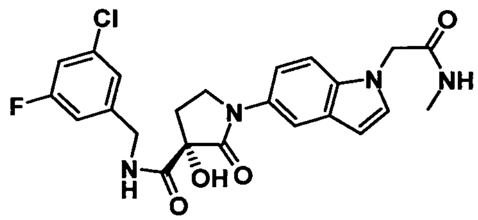 (S)-3-Hydroxy-1-(1-methylcarbamoyl-methyl-1 H-indol-5-yl)-2-oxo-pyrrolidin-3-carbonsäure-3-chlor-5-fluor-benzylamid | δ 8.70 (t, *J* = 6.4, 1H), 8.02 (d, *J* = 4.6, 1H), 7.72 (d, *J* = 1.8, 1H), 7.43 (dd, *J* = 8.9, 2.0, 1H), 7.36 (d, *J* = 8.9, 1H), 7.34 (d, *J* = 3.1, 1H), 7.27 (dt, *J* = 8.7, 2.1, 1H), 7.22 (s, 1H), 7.11 (d, *J* = 9.7, 1H), 6.74 (s, 1H), 6.44 (dd, *J* = 3.1, 0.6, 1H), 4.79 (s, 2H), 4.39 (dd, *J* = 15.8, 6.8, 1H), 4.25 (dd, *J* = 15.7, 6.0, 1H), 3.87 (t, *J* = 6.8, 2H), 2.64 - 2.55 (m, 4H), 2.13 (dt, *J* = 12.9, 7.5, 1H). | 3.56 min [473] |
| "A288" | 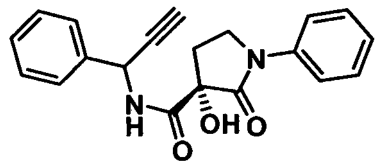 (S)-3-Hydroxy-2-oxo-1-phenyl-pyrrolidin-3-carbonsäure-(1-phenyl-prop-2-ynyl)-amid | 8.64 (d, *J* = 8.7, 1H), 7.70 (dd, *J* = 8.7, 1.0, 2H), 7.47 (d, *J* = 7.2, 2H), 7.44-7.33 (m, 5H), 7.33 - 7.27 (m, 1H), 7.22 - 7.14 (m, 1H), 6.71 (s, 1H), 6.02-5.63 (m, 1H), 3.85 (td, *J* = 8.8, 5.2, 2H), 3.50 (d, *J* = 2.5, 1H), 2.55 - 2.50 (m, 1H), 2.09 (dt, *J* = 13.0, 7.8, 1H). | 3.91 min [335] |
| "A289" | 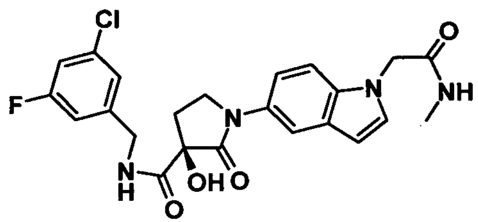 (R)-3-Hydroxy-1-(1-methyl-carbamoylmethyl-1H-indol-5-yl)-2-oxo-pyrrolidin-3-carbonsäure-3-chlor-5-fluor-benzylamid | 8.69 (t, *J* = 6.3, 1H), 8.00 (d, *J* = 4.5, 1H), 7.72 (d, *J* = 2.0, 1H), 7.43 (dd, *J* = 8.9, 2.0, 1H), 7.36 (d, *J* = 9.0, 1H), 7.34 (d, *J* = 3.1, 1H), 7.27 (dt, *J* = 8.7, 2.1, 1H), 7.22 (s, 1H), 7.11 (d, *J* = 9.0, 1H), 6.71 (s, 1H), 6.44 (d, *J* = 3.1, 1H), 4.79 (s, 2H), 4.39 (dd, *J* = 15.8, 6.8, 1H), 4.25 (dd, *J* = 15.6, 5.9, 1H), 3.87 (t, *J* = 6.7, 2H), 2.65 - 2.55 (m, 4H), 2.13 (dt, *J* = 12.9, 7.5, 1H). | 3.56 min [473] |

(fortgesetzt)

| Nr. | Struktur / Name | $^{1}H$ NMR (400 MHz, DMSO-$d_6$) δ [ppm] | LC-MS; rt; [M+H$^{+}$]* |
|---|---|---|---|
| "A290" | 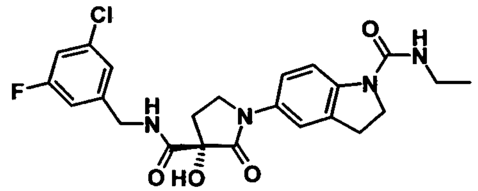 5-[(S)-3-(3-Chlor-5-fluor-benzylcarbamoyl)-3-hydroxy-2-oxo-pyrrolidin-1-yl]-2,3-dihydro-indol-1-carbonsäure-ethylamid | 8.69 (t, *J* = 6.3, 1H), 7.80 (d, *J* = 8.8, 1H), 7.51 (d, *J* = 2.1, 1H), 7.32 - 7.24 (m, 2H), 7.20 (s, 1H), 7.10 (d, *J* = 8.8, 1H), 6.68 (s, 1H), 6.61 (t, *J* = 5.5, 1H), 4.38 (dd, *J* = 15.7, 6.7, 1H), 4.23 (dd, *J* = 16.0, 6.1, 1H), 3.86 (t, *J* = 8.7, 2H), 3.79 (dd, *J* = 8.6, 5.7, 2H), 3.18 - 3.08 (m, 4H), 2.60 - 2.51 (m, 2H), 2.09 (dt, *J* = 13.0, 7.6, 1H), 1.07 (t, *J* = 7.1, 3H). | 3.78 min [475] |
| "A291" | 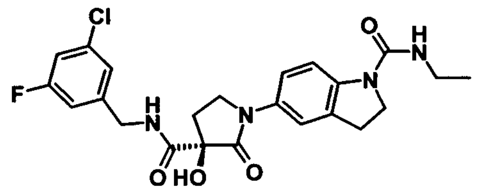 5-[(R)-3-(3-Chlor-5-fluor-benzylcarbamoyl)-3-hydroxy-2-oxo-pyrrolidin-1-yl]-2,3-dihydro-indol-1-carbonsäure-ethylamid | δ 8.69 (t, *J* = 6.4, 1H), 7.80 (d, *J* = 8.8, 1H), 7.51 (d, *J* = 2.0, 1H), 7.32-7.23 (m, 2H), 7.20 (s, 1H), 7.10 (d, *J* = 9.7, 1H), 6.68 (s, 1H), 6.61 (t, *J* = 5.5, 1H), 4.38 (dd, *J* = 15.8, 6.8, 1H), 4.23 (dd, *J* = 15.8, 6.0, 1H), 3.86 (t, *J* = 8.7, 2H), 3.79 (dd, *J* = 8.6, 5.7, 2H), 3.20 - 3.06 (m, 4H), 2.55 (dt, *J* = 6.8, 5.7, 1H), 2.09 (dt, *J* = 12.9, 7.6, 1H), 1.07 (t, *J* = 7.1, 3H). | 3.78 min [475] |
| "A292" | 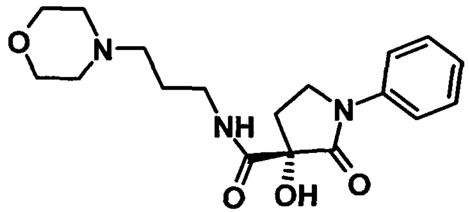 (S)-3-Hydroxy-2-oxo-1-phenyl-pyrrolidin-3-carbonsäure-(3-morpholin-4-yl-propyl)-amid | 8.17 (t, *J* = 5.7, 1H), 7.68 (dt, *J* = 9.0, 1.7, 2H), 7.44 - 7.35 (m, 2H), 7.22-7.13 (m, 1H), 6.60 (s, 1H), 3.89 - 3.77 (m, 2H), 3.63 - 3.51 (m, 4H), 3.14 (dd, *J* = 12.8, 6.8, 2H), 2.58-2.51 (m, 1H), 2.40 - 2.20 (m, 6H), 2.07 (m, 1H), 1.58 (p, *J* = 6.8, 2H). | 1.94 min [348] |

(fortgesetzt)

| Nr. | Struktur / Name | $^1H$ NMR (400 MHz, DMSO-$d_6$) δ [ppm] | LC-MS; rt; [M+H+]* |
|---|---|---|---|
| "A293" | 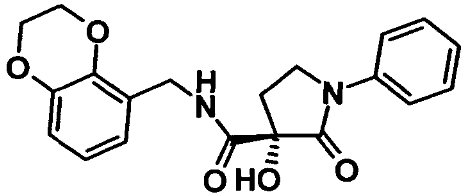 (S)-3-Hydroxy-2-oxo-1-phenyl-pyrrolidin-3-carbonsäure-(2,3-dihydro-benzo[1,4]dioxin-5-ylmethyl)-amid | | 3.47 min [369] |
| "A294" | 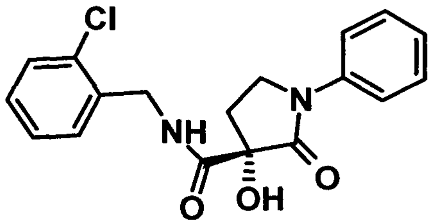 (S)-3-Hydroxy-2-oxo-1-phenyl-pyrrolidin-3-carbonsäure-2-chlor-benzylamid | 8.59 (t, *J* = 6.3, 1H), 7.70 (dt, *J* = 9.0, 1.7, 2H), 7.44 - 7.24 (m, 6H), 7.21-7.14 (m, 1H), 6.79 (s, 1H), 4.41 (dd, *J* = 16.2, 6.5, 1H), 4.31 (dd, *J* = 16.1, 6.1, 1H), 3.86 (dd, *J* = 8.5, 5.6, 2H), 2.65 - 2.57 (m, 1H), 2.14 (dt, *J* = 12.9, 7.7, 1H). | 3.82 min [345] |
| "A295" | 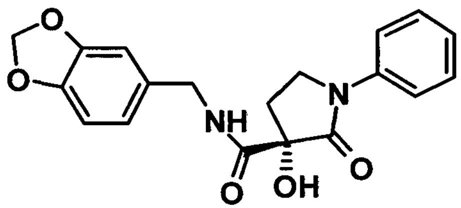 (S)-3-Hydroxy-2-oxo-1-phenyl-pyrrolidin-3-carbonsäure-(benzo[1,3]dioxol-5-ylmethyl)-amid | | 3.36 min [355] |

(fortgesetzt)

| Nr. | Struktur / Name | $^{1}$H NMR (400 MHz, DMSO-$d_6$) δ [ppm] | LC-MS; rt; [M+H$^+$]* |
|---|---|---|---|
| "A296" | 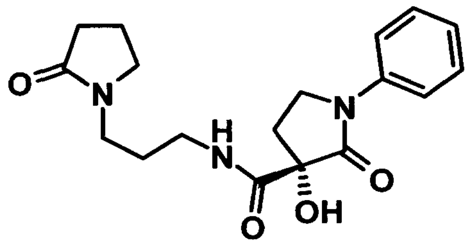 (S)-3-Hydroxy-2-oxo-1-phenyl-pyrrolidin-3-carbonsäure-[3-(2-oxo-pyrrolidin-1-yl)-propyl]-amid | 8.01 (t, J = 6.1, 1H), 7.69 (dd, *J* = 8.7, 1.0, 2H), 7.40 (dd, *J* = 10.8, 5.3, 2H), 7.17 (t, *J* = 7.4, 1H), 6.61 (s, 1H), 3.90 - 3.77 (m, 2H), 3.32 - 3.29 (m, 2H), 3.16 (m, 2H), 3.05 (m, 2H), 2.53 (ddd, *J* = 11.8, 6.6, 3.5, 2H), 2.20 (t, *J* = 8.1, 2H), 2.07 (m, 1H), 1.95 - 1.85 (m, 2H), 1.59 (p, *J* = 6.9, 2H). | 2.35 min [346] |
| "A297" | 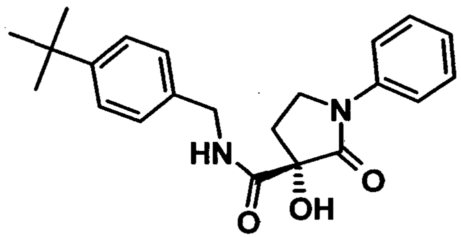 (S)-3-Hydroxy-2-oxo-1-phenyl-pyrrolidin-3-carbonsäure-4-tert.-butyl-benzylamid | 8.44 (t, *J* = 6.3, 1H), 7.69 (dd, *J* = 8.7, 1.1, 2H), 7.44 - 7.36 (m, 2H), 7.34-7.28 (m, 2H), 7.18 (d, *J* = 7.7, 3H), 6.66 (s, 1H), 4.27 (d, *J* = 6.4, 1H), 4.24 (d, *J* = 6.3, 1H), 3.86 (m, 3H), 2.61 - 2.52 (m, 1H), 2.10 (dt, *J* = 12.9, 7.8, 1H), 1.25 (s, 9H). | 4.76 min [367] |
| "A298" | 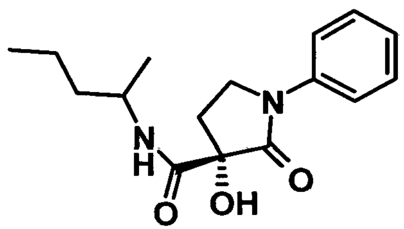 (S)-3-Hydroxy-2-oxo-1-phenyl-pyrrolidin-3-carbonsäure-(1-methylbutyl)-amid | 7.69 (dt, *J* = 9.0, 1.7, 2H), 7.52 (d, *J* = 8.7, 1H), 7.44 - 7.36 (m, 2H), 7.19-7.14 (m, 1H), 6.59 (d, *J* = 1.0, 1H), 3.92 - 3.70 (m, 3H), 2.58 - 2.50 (m, 1H), 2.07 (m, 1H), 1.54 - 1.14 (m, 5H), 1.06 (dd, *J* = 6.6, 4.2, 3H), 0.84 (dt, *J* = 11.5, 7.2, 3H). | 3.43 min [291] |

(fortgesetzt)

| Nr. | Struktur / Name | $^1$H NMR (400 MHz, DMSO-$d_6$) δ [ppm] | LC-MS; rt; [M+H$^+$]* |
|---|---|---|---|
| "A299" | 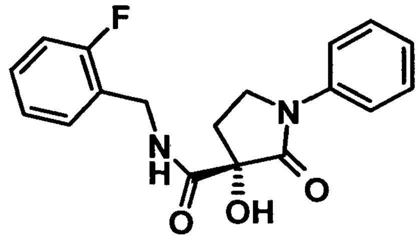 (S)-3-Hydroxy-2-oxo-1-phenyl-pyrrolidine-3-carbonsäure-2-fluor-benzylamid | 8.53 (t, *J* = 6.3, 1H), 7.73 - 7.66 (m, 2H), 7.43-7.37 (m, 2H), 7.34 (t, *J* = 7.8, 1H), 7.31 - 7.25 (m, 1H), 7.20-7.11 (m, 3H), 6.73 (s, 1H), 4.38 (dd, *J* = 15.6, 6.4, 1H), 4.31 (dd, *J* = 15.5, 5.9, 1H), 3.91 - 3.80 (m, 2H), 2.58 (ddd, J = 12.9, 7.0, 4.5, 1H), 2.12 (dt, *J* = 13.0, 7.7, 1H). | 3.51 min [329] |
| "A300" | 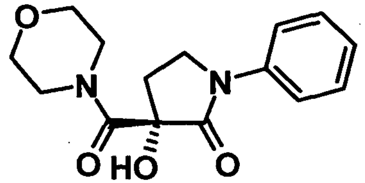 (S)-3-Hydroxy-3-(morpholin-4-carbonyl)-1-phenyl-pyrrolidin-2-on | 7.71 - 7.65 (m, 2H), 7.40 (dd, *J* = 10.8, 5.3, 2H), 7.17 (t, *J* = 7.4, 1H), 6.77 (s, 1H), 4.09 (m, 1H), 3.78 (m, 2H), 3.73 - 3.62 (m, 2H), 3.59 (m, 5H), 2.72-2.63 (m, 1H), 2.11 (dt, *J* = 12.6, 8.9, 1H). | 2.38 min [291] |
| "A301" | 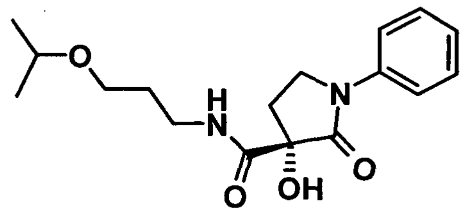 (S)-3-Hydroxy-2-oxo-1-phenyl-pyrrolidin-3-carbonsäure-(3-isopropoxy-propyl)-amid | 7.94 (t, *J* = 5.8, 1H), 7.68 (dt, *J* = 9.0, 1.7, 2H), 7.43 - 7.34 (m, 2H), 7.22-7.12 (m, 1H), 6.59 (s, 1H), 3.89 - 3.76 (m, 2H), 3.49 (hept, *J* = 6.1, 1H), 3.36 (t, *J* = 6.2, 2H), 3.14 (p, *J* = 6.6, 2H), 2.56 - 2.50 (m, 1H), 2.12 - 1.98 (m, 1H), 1.63 (p, *J* = 6.5, 2H), 1.06 (dd, *J* = 6.1, 3.0, 6H). | 3.05 min [321] |
| "A302" | 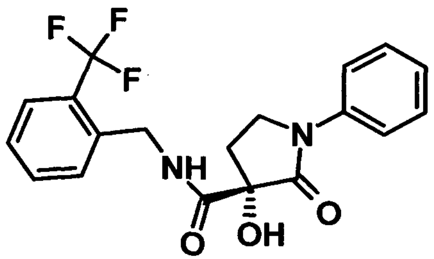 | | 4.20 min [379] |

(fortgesetzt)

| Nr. | Struktur / Name | $^{1}H$ NMR (400 MHz, DMSO-$d_6$) δ [ppm] | LC-MS; rt; [M+H$^+$]* |
|---|---|---|---|
| | (S)-3-Hydroxy-2-oxo-1-phenyl-pyrrolidin-3-carbonsäure-2-trifluoromethyl-benzylamid | | |
| "A303" | 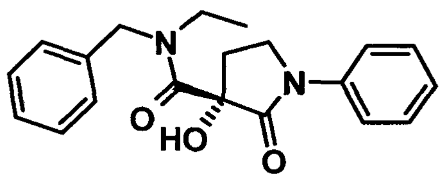 (S)-3-Hydroxy-2-oxo-1-phenyl-pyrrolidin-3-carbonsäure-benzyl-ethyl-amid | 7.70 (d, $J$ = 8.0, 2H), 7.43 - 7.37 (m, 2H), 7.37-7.26 (m, 3H), 7.25 - 7.14 (m, 3H), 6.75 (s, 1H), 4.98 (dd, $J$ = 78.2, 15.8, 1H), 4.49 (dd, $J$ = 48.6, 15.3, 1H), 3.83 (dd, $J$ = 19.3, 9.9, 1H), 3.75 - 3.56 (m, 2H), 3.10 (ddd, $J$ = 23.0, 13.8, 6.1, 1H), 2.77 - 2.61 (m, 1H), 2.24 - 2.09 (m, 1H), 1.15 (t, $J$ = 6.8, 2H), 0.90 (t, J = 6.9, 1H). | 4.22 min [339] |
| "A304" | 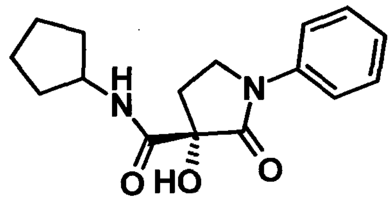 (S)-3-Hydroxy-2-oxo-1-phenyl-pyrrolidine-3-carbonsäure-cyclopentylamid | 7.72 - 7.66 (m, 2H), 7.63 (d, $J$ = 7.8, 1H), 7.43-7.36 (m, 2H), 7.20 - 7.13 (m, 1H), 6.59 (s, 1H), 4.06 - 3.95 (m, 1H), 3.89-3.77 (m, 2H), 2.56 - 2.51 (m, 1H), 2.11 - 2.01 (m, 1H), 1.79 (m, 2H), 1.70-1.58 (m, 2H), 1.54 - 1.37 (m, 4H). | 3.07 min [289] |
| "A305" | 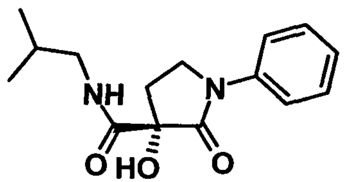 (S)-3-Hydroxy-2-oxo-1-phenyl-pyrrolidine-3-carboxylic acid isobutyl-amid | 7.87 (t, $J$ = 6.1, 1H), 7.68 (dt, $J$ = 9.0, 1.8, 2H), 7.43 - 7.36 (m, 2H), 7.20 - 7.14 (m, 1H), 6.61 (s, 1H), 3.94 - 3.74 (m, 2H), 2.98 - 2.85 (m, 2H), 2.53 (ddd, $J$ = 12.3, 7.1, 3.7, 1H), 2.15 - 2.03 (m, 1H), 1.75 (dp, $J$ = *13.5*, 6.8, 1H), 0.82 (dd, $J$ = 6.7, 3.2, 6H). | 3.00 min [277] |
| "A306" | 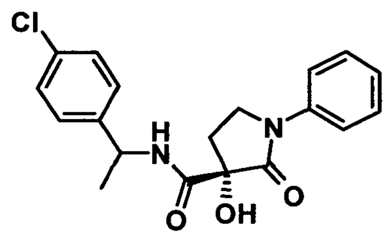 (S)-3-Hydroxy-2-oxo-1-phenyl-pyrrolidine-3-carboxylic acid [1-(4-chloro-phenyl)-ethyl]-amid | 8.28 (t, $J$ = 7.6, 1H), 7.72 - 7.64 (m, 2H), 7.43-7.31 (m, 6H), 7.17 (td, $J$ = 7.4, 4.4, 1H), 6.69 (d, $J$ = 9.2, 1H), 4.98 - 4.86 (m, 1H), 3.87 - 3.75 (m, 2H), 2.58 (ddd, $J$ = 22.2, 11.9, 7.8, 1H), 2.47 - 2.37 (m, 1H), 2.09 (ddd, $J$ = 15.9, 13.8, 8.0, 1H), 1.40 (dd, $J$ = 7.0, 2.2, 3H). | 4.18 min [359] |

(fortgesetzt)

| Nr. | Struktur / Name | $^1$H NMR (400 MHz, DMSO-$d_6$) δ [ppm] | LC-MS; rt; [M+H$^+$]* |
|---|---|---|---|
| "A307" | 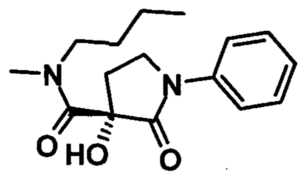 (S)-3-Hydroxy-2-oxo-1-phenyl-pyrrolidin-3-carbonsäure-butylmethyl-amid | 7.68 (dd, *J* = 8.7, 1.0, 2H), 7.43 - 7.35 (m, 2H), 7.20 - 7.12 (m, 1H), 6.57 (s, 1H), 3.79 (t, *J* = 8.6, 1H), 3.71 - 3.45 (m, 2H), 3.22 (m, 1H), 3.18 (m, 2H), 2.77 (m, 1H), 2.60 (m, 1H), 2.11 (m, 1H), 1.52 (m, 1H), 1.49 - 1.38 (m, 1H), 1.23 (m, 2H), 0.87 (m, 3H). | 3.64 min [291] |
| "A308" | 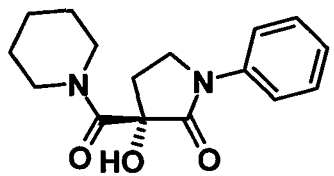 (S)-3-Hydroxy-1-phenyl-3-(piperidin-1-carbonyl)-pyrrolidin-2-on | 7.71 - 7.64 (m, 2H), 7.39 (dd, *J* = 10.8, 5.3, 2H), 7.16 (t, *J* = 7.4, 1H), 6.64 (s, 1H), 3.88 (s, 1H), 3.81 - 3.74 (m, 1H), 3.64 (m, 2H), 3.52 (m, 1H), 3.26 (m, 1H), 2.60 (m, 1H), 2.11 (m, 1H), 1.53 (m, 6H). | 3.18 min [289] |
| "A309" | 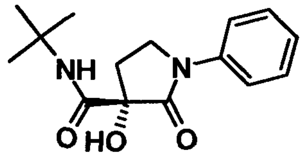 (S)-3-Hydroxy-2-oxo-1-phenyl-pyrrolidine-3-carbonsäure-tert.-butylamid | 7.72 - 7.65 (m, 2H), 7.43 - 7.36 (m, 2H), 7.21-7.14 (m, 1H), 7.03 (s, 1H), 6.70 (s, 1H), 3.90 - 3.75 (m, 2H), 2.55 - 2.50 (m, 1 H), 2.05 (ddd, *J* = 12.9, 8.7, 7.6, 1H), 1.28 (s, 9H). | 3.10 min [277] |
| "A310" | 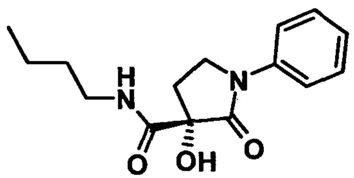 (S)-3-Hydroxy-2-oxo-1-phenyl-pyrrolidin-3-carbonsäure-butylamid | 7.89 (t, *J* = 5.9, 1H), 7.68 (dt, *J* = 9.0, 1.7, 2H), 7.44 - 7.36 (m, 2H), 7.20 - 7.13 (m, 1H), 6.58 (s, 1H), 3.92 - 3.77 (m, 2H), 3.08 (dd, *J* = 13.2, 6.9, 2H), 2.57 - 2.50 (m, 1H), 2.12 - 2.01 (m, 1H), 1.45-1.34 (m, 2H), 1.25 (dq, *J* = 14.1, 7.2, 3H), 0.85 (t, *J* = 7.3, 3H). | 3.07 min [277] |

(fortgesetzt)

| Nr. | Struktur / Name | $^1$H NMR (400 MHz, DMSO-$d_6$) δ [ppm] | LC-MS; rt; [M+H$^+$]* |
|---|---|---|---|
| "A311" | 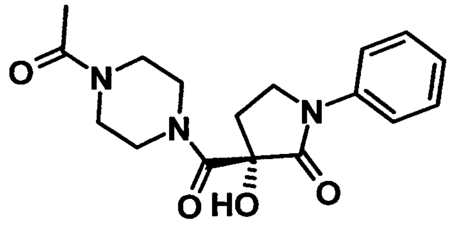<br>(S)-3-(4-Acetyl-piperazin-1-carbonyl)-3-hydroxy-1-phenyl-pyrrolidin-2-on | 7.68 (dd, *J* = 8.7, 1.0, 2H), 7.43 - 7.36 (m, 2H), 7.21 - 7.13 (m, 1 H), 7.02 (d, *J* = 15.9, 1H), 6.82 (s, 1H), 4.09 (m, 1H), 3.79 (m, 1H), 3.72 - 3.53 (m, 4H), 3.44 (m, 2H), 3.24 (m, 1H), 3.16 (d, *J* = 5.2, 1H), 2.69 (dd, *J* = 12.5, 5.9, 1H), 2.13 (dt, *J* = 12.6, 9.0, 1H), 2.01 (s, 3H). | 2.30 min [332] |
| "A312" | 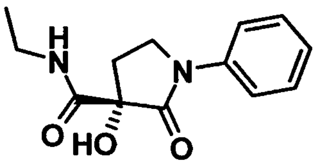<br>(S)-3-Hydroxy-2-oxo-1-phenyl-pyrrolidin-3-carbonsäure-ethylamid | 7.94 (t, *J* = 5.7, 1H), 7.72 - 7.65 (m, 2H), 7.43 - 7.34 (m, 2H), 7.21 - 7.13 (m, 1H), 6.58 (s, 1H), 3.90 - 3.75 (m, 2H), 3.17-3.05 (m, 2H), 2.57 - 2.51 (m, 1H), 2.07 (ddd, *J* = 12.9, 8.5, 7.4, 1H), 1.02 (t, *J* = 7.2, 3H). | 2.15 min [249] |
| "A313" | 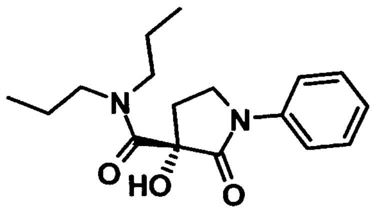<br>(S)-3-Hydroxy-2-oxo-1-phenyl-pyrrolidin-3-carbonsäure-dipropylamid | 7.68 (dt, *J* = 9.0, 1.7, 2H), 7.42 - 7.34 (m, 2H), 7.19 - 7.13 (m, 1H), 6.55 (s, 1H), 3.79 (td, *J* = 9.2, 1.9, 1H), 3.62 (dt, *J* = 9.3, 6.8, 1H), 3.51 (t, *J* = 7.9, 2H), 3.21 - 3.05 (m, 2H), 2.58 (ddd, *J* = 12.6, 6.6, 1.9, 1H), 2.13 (dt, *J* = 12.7, 8.8, 1H), 1.61 (pd, *J* = 13.2, 7.4, 2H), 1.51-1.38 (m, 2H), 0.81 (dt, *J* = 12.3, 7.4, 6H). | 4.04 min [305] |
| "A314" | 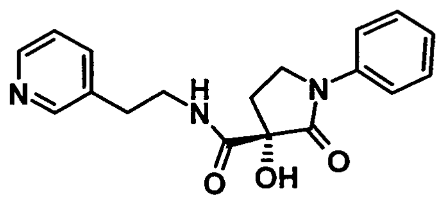<br>(S)-3-Hydroxy-2-oxo-1-phenyl-pyrrolidin-3-carbonsäure-(2-pyridin-3-yl-ethyl)-amid | 8.42 (d, *J* = 1.7, 1H), 8.40 (dd, *J* = 4.8, 1.6, 1H), 8.07 (t, *J* = 6.0, 1H), 7.68 (dd, *J* = 8.7, 1.0, 2H), 7.65-7.57 (m, 2H), 7.43 - 7.35 (m, 2H), 7.29 (ddd, *J* = 7.8, 4.8, 0.7, 1H), 7.22 - 7.13 (m, 1H), 6.62 (s, 1H), 3.87 - 3.75 (m, 2H), 3.44 - 3.20 (m, 2H), 2.77 (t, *J* = 7.1, 2H), 2.44 (m, 1H), 2.10 -1.99 (m, 1H). | 1.88 min [326] |

(fortgesetzt)

| Nr. | Struktur / Name | $^1H$ NMR (400 MHz, DMSO-$d_6$) δ [ppm] | LC-MS; rt; [M+H$^+$]* |
|---|---|---|---|
| "A315" | 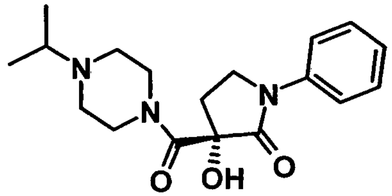 (S)-3-Hydroxy-3-(4-isopropyl-piperazin-1-carbonyl)-1-phenyl-pyrrolidin-2-on | 7.70 - 7.64 (m, 2H), 7.42 - 7.36 (m, 2H), 7.19-7.12 (m, 1H), 6.67 (s, 1H), 3.98 (m, 1H), 3.77 (m, 1H), 3.65 (m, 2H), 3.59 - 3.37 (m, 2H), 2.63 (m, 2H), 2.46 - 2.30 (m, 4H), 2.11 (dt, *J* = 12.7, 8.9, 1H), 0.96 (d, *J* = 6.5, 6H). | 2.18 min [332] |
| "A316" | 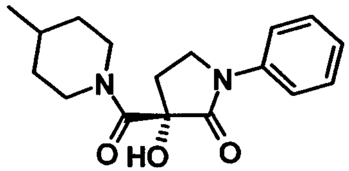 (S)-3-Hydroxy-3-(4-methyl-piperidin-1-carbonyl)-1-phenyl-pyrrolidin-2-on | 7.67 (dt, *J* = 12.0, 6.1, 2H), 7.43 - 7.35 (m, 2H), 7.19-7.12 (m, 1H), 6.64 (s, 1H), 4.61 (m, 1H), 4.26 (m, 1H), 3.84 - 3.72 (m, 1H), 3.65 (m, 1H), 2.92 (m, 1H), 2.60 (m, 2H), 2.11 (dt, *J* = 12.6, 8.8, 1H), 1.62 (m, 3H), 1.19 (m, 2H), 0.89 (d, *J* = 6.1, 3H). | 3.69 min [303] |
| "A317" | 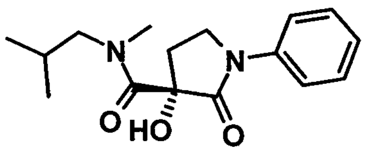 (S)-3-Hydroxy-2-oxo-1-phenyl-pyrrolidin-3-carbonsäure-isobutyl-methyl-amid | 7.71 - 7.64 (m, 2H), 7.42 - 7.35 (m, 2H), 7.19 - 7.12 (m, 1H), 6.58 (s, 1H), 3.81 (m, 1H), 3.62 (m, 1H), 3.17 (s, 3H), 3.10 (m, 1H), 2.77 (s, 1H), 2.67 - 2.57 (m, 1H), 2.12 (dt, *J* = 12.8, 8.7, 1H), 2.03 - 1.80 (m, 1H), 0.81 (d, *J* = 6.0, 6H). | 3.55 min [291] |
| "A318" | 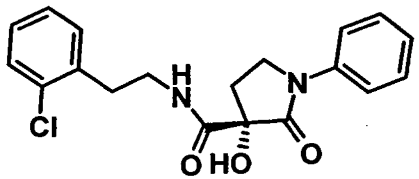 (S)-3-Hydroxy-2-oxo-1-phenyl-pyrrolidin-3-carbonsäure-[2-(2-chlor-phenyl)-ethyl]-amid | 8.09 (t, *J* = 6.0, 1H), 7.72 - 7.66 (m, 2H), 7.43 - 7.37 (m, 3H), 7.34 - 7.30 (m, 1H), 7.28 - 7.21 (m, 2H), 7.20 - 7.14 (m, 1H), 6.60 (s, 1H), 3.85 - 3.77 (m, 2H), 3.46 - 3.34 (m, 1H), 3.28 (m, 1H), 2.93 - 2.78 (m, 2H), 2.47 - 2.41 (m, 1H), 2.06 (dt, *J* = 12.9, 7.9, 1H) | 4.00 min [359] |

(fortgesetzt)

| Nr. | Struktur / Name | $^1H$ NMR (400 MHz, DMSO-$d_6$) δ [ppm] | LC-MS; rt; [M+H$^+$]* |
|---|---|---|---|
| "A319" | 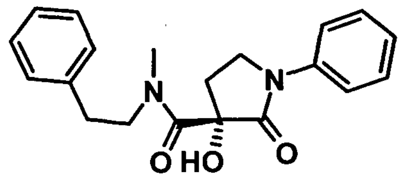 (S)-3-Hydroxy-2-oxo-1-phenyl-pyrrolidin-3-carbonsäure-methyl-phenethyl-amid | 7.72 - 7.66 (m, 2H), 7.39 (t, *J* = 7.9, 2H), 7.34 - 7.12 (m, 6H), 6.68 (s, 1H), 3.88 (m, 1H), 3.77 (m, 1H), 3.69 - 3.47 (m, 1H), 3.40 - 3.33 (m, 1H), 3.16 (m, 1H), 3.03 - 2.88 (m, 1H), 2.89 (s, 3H), 2.80 - 2.72 (m, 1H), 2.51 (m, 1H), 2.09 (dt, *J* = 12.7, 8.8, 1H). | 4.10 min [339] |
| "A320" | 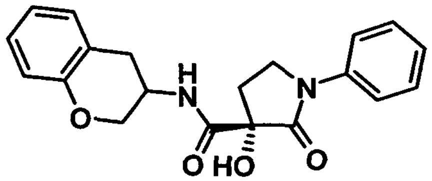 (S)-3-Hydroxy-2-oxo-1-phenyl-pyrrolidin-3-carbonsäure-chroman-3-ylamid | 7.88 (dd, *J* = 17.7, 8.2, 1H), 7.73-7.66 (m, 2H), 7.44 - 7.36 (m, 2H), 7.18 (td, *J* = 7.3, 1.1, 1H), 7.13 - 7.05 (m, 2H), 6.86 (t, *J* = 7.4, 1H), 6.81 - 6.77 (m, 1 H), 6.75 (d, *J* = 1.6, 1H), 4.24 - 4.11 (m, 1H), 4.11 - 4.01 (m, 1H), 3.97 - 3.79 (m, 3H), 3.00 - 2.78 (m, 2H), 2.57 (m, 1H), 2.09 (m, 1H). | 3.74 min [353] |
| "A321" | 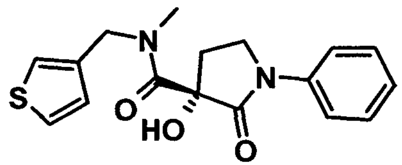 (S)-3-Hydroxy-2-oxo-1-phenyl-pyrrolidin-3-carbonsäure-methyl-thiophen-3-ylmethyl-amid | 7.73 - 7.67 (m, 2H), 7.50 (s, 1H), 7.40 (dd, *J* = 10.8, 5.3, 2H), 7.33 (s, 1H), 7.17 (t, *J* = 7.4, 1H), 6.98 (d, J = 4.4,, 1H), 6.75 (m, 1H), 4.95 (m, 1H), 4.45 (m, 1H), 3.81 (m, 1H), 3.67 (m, 1H), 3.23 - 3.11 (m, 2H), 2.69 (s, 3H), 2.15 (dt, *J* = 12.7, 8.8, 1H). | 3.71 min [331] |
| "A322" | 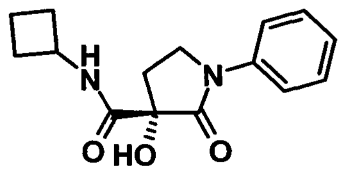 (S)-3-Hydroxy-2-oxo-1-phenyl-pyrrolidin-3-carbonsäure-cyclobutylamid | 8.08 (d, *J* = 8.4, 1H), 7.68 (dd, *J* = 8.7, 1.0, 2H), 7.43 - 7.36 (m, 2H), 7.17 (t, *J* = 7.4, 1H), 6.59 (s, 1H), 4.29 - 4.13 (m, 1H), 3.89 - 3.75 (m, 2H), 2.57 - 2.50 (m, 1H), 2.16 - 1.99 5H), 1.65 - 1.52 (m, 2H). | 2.73 min [275] |

(fortgesetzt)

| Nr. | Struktur / Name | $^1$H NMR (400 MHz, DMSO-$d_6$) δ [ppm] | LC-MS; rt; [M+H$^+$]* |
|---|---|---|---|
| "A323" | 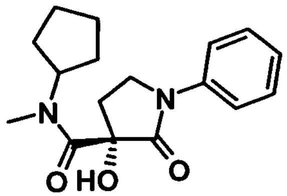 (S)-3-Hydroxy-2-oxo-1-phenyl-pyrrolidin-3-carbonsäure-cyclopentyl-methyl-amid | 7.67 (d, $J$ = 7.9, 2H), 7.39 (dd, $J$ = 10.7, 5.3, 2H), 7.16 (t, $J$ = 7.4, 1H), 6.61 (m, 1H), 3.78 (m, 1H), 3.65 (m, 1H), 3.05 (m, 1H), 2.65 (s, 3H), 2.59 (dd, $J$ = 12.8, 4.9, 1H), 2.14 (m, 1H), 1.80 (m, 1H), 1.61 (m, 3H), 1.50 (m, 4H). | 3.62 min [303] |
| "A324" | 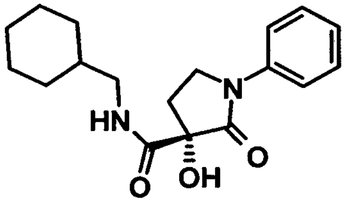 (S)-3-Hydroxy-2-oxo-1-phenyl-pyrrolidin-3-carbonsäure-cyclohexylmethyl-amid | 7.84 (t, $J$ = 6.2, 1H), 7.68 (dt, $J$ = 3.1, 1.7, 2H), 7.43 - 7.35 (m, 2H), 7.16 (dd, $J$ = 10.6, 4.2, 1H), 6.60 (s, 1H), 3.90-3.75 (m, 2H), 3.01 - 2.86 (m, 2H), 2.58 - 2.50 (m, 1H), 2.06 (ddd, $J$ = 23.3, 17.6, 11.7, 1H), 1.64 (m, 5H), 1.44 (ddd, $J$ = 10.9, 9.2, 5.5, 1H), 1.21 - 0.99 (m, 3H), 0.93 - 0.75 (m, 2H). | 3.97 min [317] |
| "A325" | 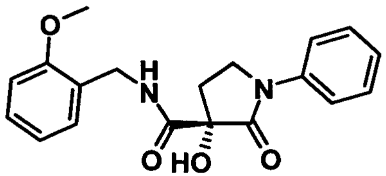 (S)-3-Hydroxy-2-oxo-1-phenyl-pyrrolidin-3-carbonsäure-2-methoxy-benzylamid | 8.25 (t, $J$ = 6.2, 1H), 7.72 - 7.66 (m, 2H), 7.44 - 7.35 (m, 2H), 7.25 - 7.14 (m, 3H), 6.96 (d, $J$ = 7.8, 1H), 6.88 (t, $J$ = 7.4, 1H), 6.73 (s, 1H), 4.30 (dd, $J$ = 15.8, 6.4, 1H), 4.24 (dd, $J$ = 15.8, 6.1, 1H), 3.86 (m, 2H), 3.79 (s, 3H), 2.59 (ddd, $J$ = 12.7, 7.1, 4.3, 1H), 2.12 (dt, $J$ = 12.9, 7.8, 1H). | 3.58 min [341] |
| "A326" | 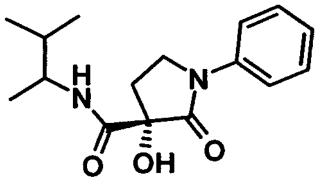 (S)-3-Hydroxy-2-oxo-1-phenyl-pyrrolidin-3-carbonsäure-(1,2-dimethyl-propyl)-amid | - | 3.33 min [291] |

(fortgesetzt)

| Nr. | Struktur / Name | $^1$H NMR (400 MHz, DMSO-$d_6$) δ [ppm] | LC-MS; rt; [M+H$^+$]* |
|---|---|---|---|
| "A327" | 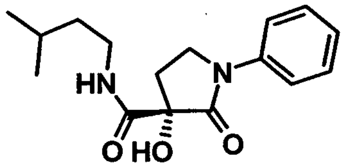 (S)-3-Hydroxy-2-oxo-1-phenyl-pyrrolidin-3-carbonsäure-(3-methylbutyl)-amid | 7.87 (t, *J* = 5.9, 1H), 7.68 (dt, *J* = 8.9, 1.7, 2H), 7.43 - 7.36 (m, 2H), 7.20 - 7.14 (m, 1H), 6.57 (s, 1H), 3.86 (dt, *J* = 8.9, 0.7, 1H), 3.83 - 3.77 (m, 1H), 3.10 (dd, *J* = 14.4, 6.3, 2H), 2.56 - 2.50 (m, 1H), 2.07 (ddd, *J* = 12.9, 8.5, 7.6, 1H), 1.54 (dp, *J* = 13.3, 6.7, 1H), 1.32 (dd, *J* = 14.5, 7.0, 2H), 0.85 (dd, *J* = 6.6, 2.2, 6H). | 3.53 min [291] |
| "A328" | 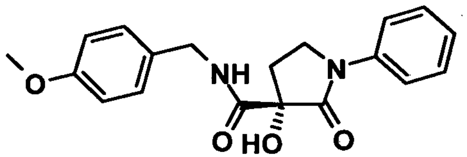 (S)-3-Hydroxy-2-oxo-1-phenyl-pyrrolidin-3-carbonsäure-4-methoxy-benzylamid | 8.41 (t, *J* = 6.3, 1H), 7.69 (dt, *J* = 9.0, 1.7, 2H), 7.44 - 7.34 (m, 2H), 7.23-7.16 (m, 3H), 6.89 - 6.82 (m, 2H), 6.65 (s, 1H), 4.25 (dd, *J* = 14.8, 6.5, 1H), 4.20 (dd, *J* = 14.8, 6.3, 1H), 3.91 - 3.79 (m, 2H), 3.71 (s, 3H), 2.55 (ddd, *J* = 12.8, 7.4, 3.9, 1H), 2.14 - 2.05 (m, 1H). | 3.41 min [341] |
| "A329" | 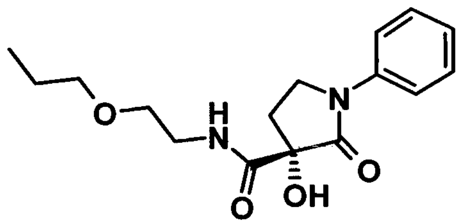 (S)-3-Hydroxy-2-oxo-1-phenyl-pyrrolidin-3-carbonsäure-(2-propoxy-ethyl)-amid | 7.82 (t, *J* = 5.7, 1H), 7.71 - 7.66 (m, 2H), 7.43 - 7.36 (m, 2H), 7.21 - 7.14 (m, 1H), 6.67 (s, 1H), 3.90 - 3.76 (m, 2H), 3.40 (t, *J* = 6.2, 2H), 3.34 (d, *J* = 6.6, 2H), 3.29 (dd, *J* = 9.7, 3.8, 1H), 3.26 - 3.16 (m, 1H), 2.56 - 2.51 (m, 1H), 2.14 - 2.01 (m, 1H), 1.54 - 1.45 (m, 2H), 0.85 (t, *J* = 7.4, 3H). | 3.01 min [307] |
| "A330" | 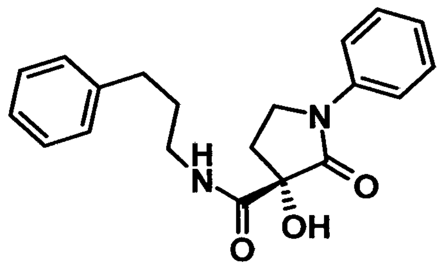 (S)-3-Hydroxy-2-oxo-1-phenyl-pyrrolidin-3-carbonsäure-(3-phenylpropyl)-amid | 8.00 (t, *J* = 5.9, 1H), 7.69 (dd, *J* = 8.7, 1.0, 2H), 7.44 - 7.36 (m, 2H), 7.29-7.23 (m, 2H), 7.22 - 7.11 (m, 4H), 6.60 (s, 1H), 3.93 - 3.76 (m, 2H), 3.18-3.03 (m, 2H), 2.59 - 2.51 (m, 3H), 2.08 (dt, *J* = 12.9, 7.9, 1 H), 1.73 (dt, *J* = 14.7, 7.2, 2H). | 4.01 min [339] |

(fortgesetzt)

| Nr. | Struktur / Name | $^1H$ NMR (400 MHz, DMSO-$d_6$) δ [ppm] | LC-MS; rt; [M+H$^+$]* |
|---|---|---|---|
| "A331" | 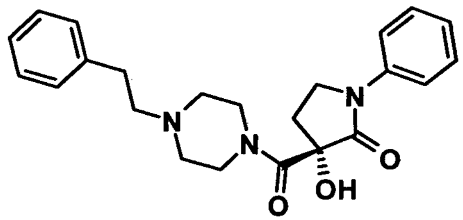 (S)-3-Hydroxy-3-(4-phenethyl-piperazin-1-carbonyl)-1-phenyl-pyrrolidin-2-on | 7.68 (dd, *J* = 8.7, 1.0, 2H), 7.43 - 7.36 (m, 2H), 7.29 - 7.14 (m, 6H), 6.70 (s, 1H), 4.01 (m, 1H), 3.78 (t, *J* = 8.4, 2H), 3.66 (td, *J* = 9.4, 6.7, 2H), 3.54 (m, 1H), 3.37 (m, 1H), 2.78 - 2.69 (m, 2H), 2.69 - 2.60 (m, 1H), 2.57 - 2.50 (m, 2H), 2.47 - 2.27 (m, 3H), 2.11 (dt, *J* = 12.5, 8.9, 1H). | 3.07 min [394] |
| "A332" | 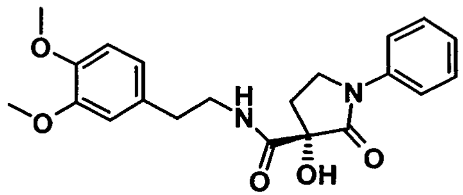 (S)-3-Hydroxy-2-oxo-1-phenyl-pyrrolidin-3-carbonsäure-[2-(3,4-dimethoxy-phenyl)-ethyl]-amid | 7.92 (t, *J* = 6.0, 1H), 7.72 - 7.66 (m, 2H), 7.44 - 7.36 (m, 2H), 7.21 - 7.14 (m, 1H), 6.84 (d, *J* = 8.2, 1H), 6.80 (d, *J* = 1.9, 1H), 6.71 (dd, *J* = 8.1, 1.9, 1H), 6.61 (s, 1H), 3.89 - 3.78 (m, 2H), 3.73 (s, 3H), 3.70 (s, 3H), 3.30 - 3.20 (m, 2H), 2.67 (t, *J* = 7.3, 2H), 2.53 -2.46 (m, 1H), 2.06 (dt, *J* = 12.9, 7.9, 1H). | 3.28 min [385] |
| "A333" | 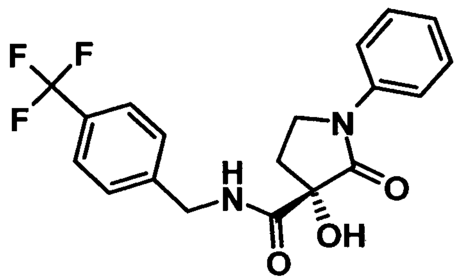 (S)-3-Hydroxy-2-oxo-1-phenyl-pyrrolidin-3-carbonsäure-4-trifluormethyl-benzylamid | 8.71 (t, *J* = 6.4, 1H), 7.72 - 7.64 (m, 4H), 7.48 (d, *J* = 8.0, 2H), 7.44 - 7.37 (m, 2H), 7.17 (t, *J* = 7.4, 1H), 6.73 (s, 1H), 4.42 (dd, *J* = 15.6, 6.6, 1H), 4.34 (dd, *J* = 15.6, 6.1, 1H), 3.88 - 3.82 (m, 2H), 2.58 (ddd, *J* = 12.8, 6.9, 4.7, 1H), 2.12 (dt, *J* = 12.9, 7.7, 1H). | 4.29 min [379] |

(fortgesetzt)

| Nr. | Struktur / Name | $^1H$ NMR (400 MHz, DMSO-$d_6$) δ [ppm] | LC-MS; rt; [M+H$^+$]* |
|---|---|---|---|
| "A334" | 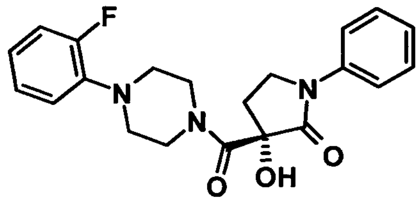 (S)-3-[4-(2-Fluor-phenyl)-piperazin-1-carbonyl]-3-hydroxy-1-phenyl-pyrrolidin-2-on | 7.72 - 7.66 (m, 2H), 7.43 - 7.36 (m, 2H), 7.20 - 6.93 (m, 5H), 6.81 (s, 1H), 4.21 (s, 1H), 3.92 (s, 1H), 3.83 - 3.74 (m, 1H), 3.68 (td, *J* = 9.4, 6.6, 2H), 3.51 (br. m,, 1H), 2.99 (br. m, 4H), 2.75 - 2.67 (m, 1H), 2.15 (dt, *J* = 12.6, 9.0, 1H). | 4.08 min [384 + 385] |
| "A335" | 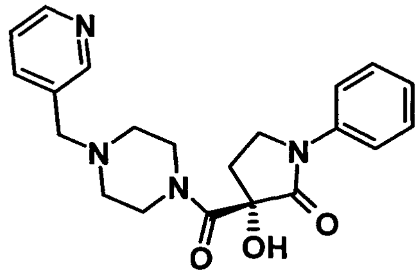 (S)-3-Hydroxy-1-phenyl-3-(4-pyridin-3-ylmethyl-piperazin-1-carbonyl)-pyrrolidin-2-on | 8.50 (d, *J* = 1.6, 1H), 8.46 (dd, *J* = 4.8, 1.7, 1H), 7.71 (dt, *J* = 7.8, 1.9, 1H), 7.68 (d, *J* = 1.1, 1H), 7.66 (d, J = 1.0, 1H), 7.42 - 7.33 (m, 3H), 7.20-7.13 (m, 1H), 7.00 (s, 1H), 6.70 (s, 1H), 4.08 (m, 1H), 3.77 (m, 2H), 3.65 (m, 2H), 3.52 (s, 2H), 3.16 (d, *J* = 5.3, 1H), 2.68 - 2.60 (m, 1H), 2.39 (s, 3H), 2.30 (m, 1H), 2.15 - 2.04 (m, 1H). | 1,99 min [381] |
| "A336" | 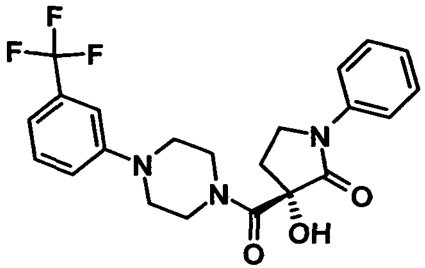 (S)-3-Hydroxy-1-phenyl-3-[4-(3-trifluoromethyl-phenyl)-piperazin-1-carbonyl]-pyrrolidin-2-on | δ 7.71 - 7.67 (m, 2H),7.46 - 7.36 (m, 3H), 7.27-7.22 (m, 1H), 7.21 - 7.13 (m, 2H), 7.09 (d, *J* = 7.6, 1H), 6.81 (s, 1H), 4.22 (m, 1H), 3.93 (m, 1H), 3.80 (t, *J* = 8.4, 1H), 3.68 (td, *J* = 9.4, 6.6, 2H), 3.50 (m, 1H), 3.35 (m, 1H), 3.27 (m, 3H), 2.77 - 2.67 (m, 1H), 2.15 (dt, *J* = 12.6, 8.9, 1H). | 4.81 min [434.0] |

(fortgesetzt)

| Nr. | Struktur / Name | $^1H$ NMR (400 MHz, DMSO-$d_6$) δ [ppm] | LC-MS; rt; [M+H$^+$]* |
|---|---|---|---|
| "A337" | 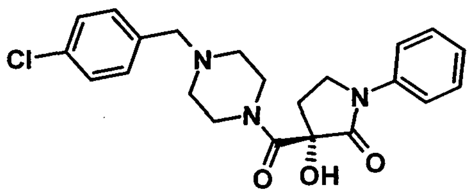 (S)-3-[4-(4-Chlor-benzyl)-piperazin-1-carbonyl]-3-hydroxy-1-phenyl-pyrrolidin-2-on | 7.67 (dd, *J* = 8.7, 1.0, 2H), 7.38 (dt, *J* = 10.6, 2.0, 4H), 7.35 - 7.30 (m, 2H), 7.19 - 7.11 (m, 1H), 6.69 (s, 1H), 4.03 (m, 1H), 3.77 (m, 2H), 3.65 (m, 2H), 3.54 (m, 1H), 3.47 (s, 2H), 2.68 - 2.60 (m, 1H), 2.38 (m, 3H), 2.29 - 2.18 (m, 1H), 2.10 (dt, *J* = 12.6, 8.9, 1H). | 3.20 min [414.0] |
| "A338" | 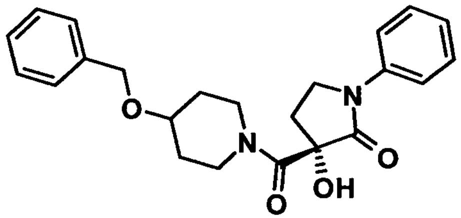 (S)-3-(4-Benzyloxy-piperidin-1-carbonyl)-3-hydroxy-1-phenyl-pyrrolidin-2-on | 7.71 - 7.66 (m, 2H), 7.42 - 7.35 (m, 2H), 7.35 (s, 4H), 7.30 - 7.24 (m, 1H), 7.16 (t, *J*= 7.4, 1H), 6.71 (s, 1H), 4.52 (s, 2H), 4.34 (m, 1H), 4.08 (m, 1H), 3.86 (m, 1H), 3.82 - 3.72 (m, 1H), 3.64 (m 3H), 3.33 - 3.18 (m, 1H), 3.02 (m, 1H), 2.63 (dd, *J* = 12.6, 5.8, 1H), 2.12 (dt, *J* = 12.6, 8.9, 1H), 1.88 (m, 2H), 1.47 (m, 2H). | 4.28 min [395] |
| "A339" | 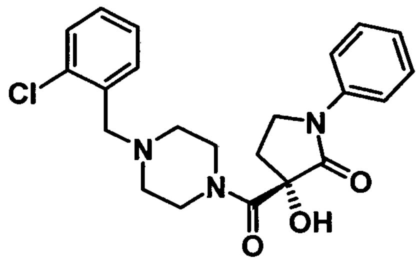 (S)-3-[4-(2-Chlor-benzyl)-piperazin-1-carbonyl]-3-hydroxy-1-phenyl-pyrrolidin-2-on | 7.68 (dd, *J* = 8.7, 1.0, 2H), 7.50 (dd, *J* = 7.4, 1.9, 1H), 7.43 (dd, *J* = 7.7, 1.5, 1H), 7.41 - 7.36 (m, 2H), 7.34 (dd, *J* = 7.4, 1.5, 1H), 7.31 (dd, *J* = 2.9, 2.0, 1H), 7.28 (dd, *J* = 7.5, 1.9, 1H), 7.16 (t, *J* = 7.4, 1H), 6.71 (s, 1H), 4.04 (m, 1H), 3.78 (t, *J* = 8.4, 2H), 3.65 (m, 1H), 3.58 (s, 2H), 3.35 (m, 2H), 2.69 - 2.59 (m, 1H), 2.47 - 2.27 (m, 4H), 2.11 (dt, *J* = 12.6, 8.9, 1H). | 2.98 min [414] |

(fortgesetzt)

| Nr. | Struktur / Name | $^1$H NMR (400 MHz, DMSO-$d_6$) δ [ppm] | LC-MS; rt; [M+H$^+$]* |
|---|---|---|---|
| "A340" | 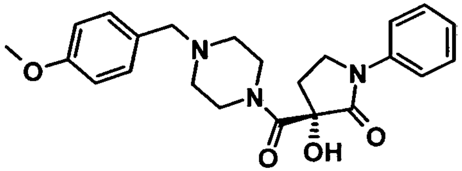 (S)-3-Hydroxy-3-[4-(4-methoxy-benzyl)-piperazin-1-carbonyl]-1-phenyl-pyrrolidin-2-on | 7.69 - 7.64 (m, 2H), 7.42 - 7.35 (m, 2H), 7.21 (dd, *J* = 6.7, 4.8, 2H), 7.16 (qd, *J* = 2.3, 1.3, 1H), 6.90 - 6.85 (m, 2H), 6.68 (s, 1H), 4.01 (m, 1H), 3.77 (m, 1H), 3.72 (s, 3H), 3.65 (m, 2H), 3.54 (m, 1H), 3.40 (s, 2H), 2.68 - 2.59 (m, 1H), 2.30 (m, 4H), 2.10 (dt, *J* = 12.6, 8.8, 1H). | 2.92 min [410] |
| "A341" | 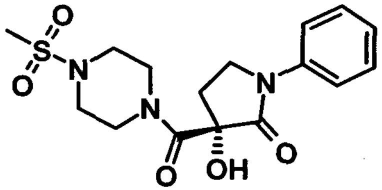 (S)-3-Hydroxy-3-(4-methansulfonyl-piperazin-1-carbonyl)-1-phenyl-pyrrolidin-2-on | 7.71 - 7.66 (m, 2H), 7.42 - 7.36 (m, 2H), 7.19 - 7.14 (m, 1H), 6.84 (s, 1H), 4.20 (m, 1H), 3.79 (t, *J* = 8.5, 3H), 3.66 (td, *J* = 9.4, 6.6, 1H), 3.36 (m, 1H), 3.10 (m, 4H), 2.88 (s, 3H), 2.74 - 2.65 (m, 1H), 2.14 (dt, *J* = 12.7, 9.0, 1H). | 2.68 min [368] |
| "A342" | 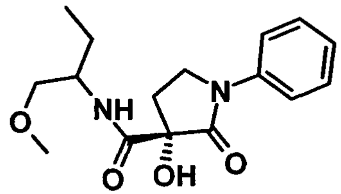 (S)-3-Hydroxy-2-oxo-1-phenyl-pyrrolidin-3-carbonsäure-(1-methoxymethyl-propyl)-amid | 7.69 (dd, *J* = 8.7, 1.0, 2H), 7.51 (t, *J* = 8.5, 1H), 7.40 (t, *J* = 7.7, 2H), 7.17 (t, *J* = 7.4, 1H), 6.67 (d, *J* = 2.6, 1H), 3.91 - 3.68 (m, 3H), 3.38 - 3.32 (m, 1H), 3.30 - 3.25 (m, 1H), 3.24 (d, J = 2.2, 3H), 2.60 - 2.50 (m, 2H), 2.09 (dq, *J* = 12.9, 7.8, 1H), 1.58 - 1.33 (m, 2H), 0.81 (q, *J* = 7.5, 3H). | 2.77 min + 2.83 min [307] |
| "A343" | 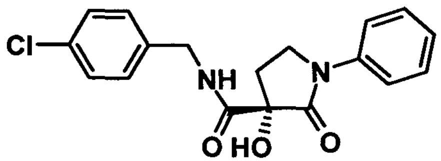 (S)-3-Hydroxy-2-oxo-1-phenyl-pyrrolidin-3-carbonsäure-4-chlor-benzylamid | 8.60 (t, *J* = 6.3, 1H), 7.69 (dt, *J* = 8.9, 1.7, 2H), 7.43 - 7.33 (m, 4H), 7.31 - 7.25 (m, 2H), 7.21 - 7.13 (m, 1H), 6.70 (s, 1H), 4.31 (dd, *J* = 15.2, 6.5, 1H), 4.24 (dd, *J* = 15.2, 6.2, 1H), 3.91 - 3.76 (m, 2H), 2.56 (ddd, *J* = 12.8, 7.1, 4.4, 1H), 2.17 - 2.04 (m, 1H). | 3.93 min [345.0] |

(fortgesetzt)

| Nr. | Struktur / Name | $^1H$ NMR (400 MHz, DMSO-$d_6$) δ [ppm] | LC-MS; rt; [M+H+]* |
|---|---|---|---|
| "A344" | 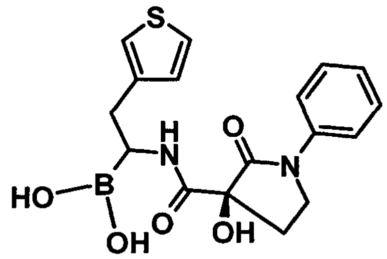 [1-[[(3S)-3-hydroxy-2-oxo-1-phenyl-pyrrolidin-3-carbonyl]amino]-2-(3-thienyl)ethyl]boronsäure | | HPLC 3.53 min |
| "A345" | 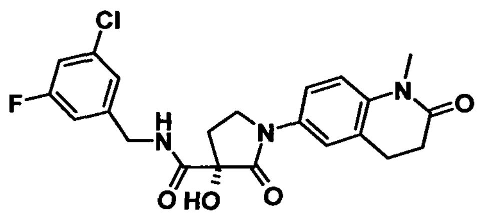 (S)-3-Hydroxy-1-(1-methyl-2-oxo-1,2,3,4-tetrahydro-chinolin-6-yl)-2-oxo-pyrrolidin-3-carbonsäure-3-chlor-5-fluor-benzylamid | 8.72 (t, *J* = 6.3, 1H), 7.58 (dd, *J* = 8.8, 2.4, 1H), 7.54 (s, 1H), 7.30 - 7.24 (m, 1H), 7.20 (s, 1H), 7.11 (d, *J* = 8.7, 2H), 6.73 (s, 1H), 4.38 (dd, *J* = 15.8, 6.7, 1H), 4.24 (dd, *J* = 15.8, 6.0, 1H), 3.83 (t, *J* = 6.8, 2H), 3.24 (s, 3H), 2.87 (t, *J* = 7.3, 2H), 2.63 - 2.51 (m, 3H), 2.13 (dt, *J* = 13.0, 7.6, 1H). | 3.67 min [446] |
| "A346" | 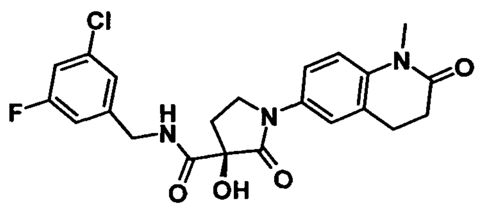 (R)-3-Hydroxy-1-(1-methyl-2-oxo-1,2,3,4-tetrahydro-chinolin-6-yl)-2-oxo-pyrrolidin-3-carbonsäure-3-chlor-5-fluor-benzylamid | | 3.67 min [446] |

LCMS:

Method: A- 0.1% TFA in $H_2O$, B- 0.1% TFA in ACN: Flow - 2.0ml/min.
Column: X Bridge C8 (50x4.6 mm.3.5u) + ve mode

| Time | %B |
|---|---|
| 0 | 05 |
| 8.0 | 100 |
| 8.1 | 100 |
| 8.5 | 05 |
| 10 | 05 |

$

LC-MS-Methode: (Gerät: Agilent 1100 Series)
Säule: Chromolith Speed Rod RP18e-50-4.6
Flussrate: 2.4 ml/min
Solvent A: Wasser + 0,05% HCOOH
Solvent B: Acetonitril + 0,04% HCOOH
WL: 220 nm
Gradient: 0-2.8 min: 4% B auf 100% B, 2.8-3.3 min:100%B.

$$

Method: A- 10 mM $NH_4HCO_3$, B- ACN: Flow - 1.0ml/min.
Column: X Bridge C8 (50x4.6mm.3.5u) - ve mode

| Time | %B |
|---|---|
| 0 | 05 |
| 8.0 | 100 |
| 8.1 | 100 |
| 8.5 | 05 |
| 10 | 05 |

HPLC:

Method: A- 0.1 % TFA in $H_2O$, B- 0.1 % TFA in ACN: Flow - 2.0ml/min.
Column: X Bridge C8 (50x4.6 mm.3.5u) + ve mode

| Time | %B |
|---|---|
| 0 | 5 |
| 8.0 | 100 |
| 8.1 | 100 |
| 8.5 | 5 |
| 10 | 5 |

$$$

Method: Isopropanol: Flow - 0.8 ml/min.
Run time: 20 min
Column: Chiralpak AD

Tabelle 1

| Inhibierung der MetAP-2<br>$IC_{50}$ von erfindungsgemäßen Verbindungen der Formel I | | | |
|---|---|---|---|
| Verbindung Nr. | $IC_{50}$ Enzym | Verbindung Nr. | $IC_{50}$ Enzym |
| "A1" | A | "A16" | B |
| "A2" | C | "A17" | A |
| "A3" | B | "A18" | A |
| "A4" | A | "A19" | B |
| "A5" | A | "A20" | A |
| "A6" | B | "A21" | A |
| "A7" | A | "A22" | A |
| "A8" | B | "A23" | A |
| "A9" | B | "A24" | B |
| "A10" | C | "A25" | C |
| "A11" | A | "A26" | C |
| "A12" | C | "A27" | A |
| "A13" | C | "A28" | A |
| "A14" | C | "A29" | A |
| "A15" | C | "A30" | A |
| | | | |
| "A31" | A | "A46" | A |
| "A32" | A | "A47" | A |
| "A33" | A | "A48" | A |
| "A34" | C | "A49" | A |
| "A35" | C | "A50" | A |
| "A36" | B | "A51" | A |
| "A37" | A | "A52" | A |
| "A38" | A | "A53" | C |
| "A39" | C | "A54" | A |
| "A40" | A | "A55" | A |
| "A41" | B | "A56" | A |
| "A42" | C | "A57" | A |
| "A43" | C | "A58" | A |
| "A44" | B | "A59" | A |
| "A45" | C | "A60" | C |
| | | | |
| "A61" | C | "A71" | A |

(fortgesetzt)

| Verbindung Nr. | $IC_{50}$ Enzym | Verbindung Nr. | $IC_{50}$ Enzym |
|---|---|---|---|
| "A62" | A | "A72" | C |
| "A63" | A | "A73" | B |
| "A64" | B | "A74" | C |
| "A65" | A | "A75" | C |
| "A66" | A | "A76" | C |
| "A67" | B | "A77" | B |
| "A68" | B | "A78" | B |
| "A69" | A | "A79" | C |
| "A70" | C | "A80" | |
| | | | |
| "A81" | | "A231" | B |
| "A82" | | "A233" | A |
| "A83" | | "A240" | A |
| "A84" | | "A250" | A |
| "A162" | A | "A253" | A |
| "A163" | C | "A254" | A |
| "A164" | A | "A260" | A |
| "A165" | C | "A261" | A |
| "A170" | A | "A267" | A |
| "A171" | B | "A270" | A |
| "A172" | A | "A279" | A |
| "A173" | A | "A282" | A |
| "A180" | A | "A283" | A |
| "A181" | A | "A284" | A |
| "A192" | A | "A285" | C |
| "A193" | A | "A286" | A |
| "A200" | A | "A287" | A |
| "A211" | A | "A288" | A |
| "A215" | A | "A290" | A |
| "A220" | B | "A292" | C |
| | | | |
| "A295" | B | "A310" | A |
| "A298" | B | "A316" | B |
| "A300" | C | "A320" | A |
| "A301" | B | "A321" | C |
| "A302" | A | "A323" | B |
| "A303" | C | "A324" | A |
| "A305" | B | "A326" | B |

(fortgesetzt)

| Verbindung Nr. | $IC_{50}$ Enzym | Verbindung Nr. | $IC_{50}$ Enzym |
|---|---|---|---|
| "A307" | 10 | "A329" | B |
| "A308" | C | "A333" | C |
| $IC_{50}$: 10 nM - 1 µM = A<br>1 µM - 10 µM = B<br>> 10 µM = C | | | |

Die nachfolgenden Beispiele betreffen Arzneimittel:

**Beispiel A: Injektionsgläser**

Eine Lösung von 100 g eines Wirkstoffes der Formel I und 5 g Dinatriumhydrogenphosphat wird in 3 l zweifach destilliertem Wasser mit 2 N Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

**Beispiel B: Suppositorien**

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

**Beispiel C: Lösung**

Man bereitet eine Lösung aus 1 g eines Wirkstoffes der Formel I, 9,38 g $NaH_2PO_4 \cdot 2 H_2O$, 28,48 g $Na_2HPO_4 \cdot 12 H_2O$ und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

**Beispiel D: Salbe**

Man mischt 500 mg eines Wirkstoffes der Formel I mit 99,5 g Vaseline unter aseptischen Bedingungen.

**Beispiel E: Tabletten**

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

**Beispiel F: Dragees**

Analog Beispiel E werden Tabletten gepreßt, die anschließend in üblicher Weise mit einem Überzug aus Saccharose, Kartoffelstärke, Talk, Tragant und Farbstoff überzogen werden.

**Beispiel G: Kapseln**

2 kg Wirkstoff der Formel I werden in üblicher Weise in Hartgelatinekapseln gefüllt, so daß jede Kapsel 20 mg des Wirkstoffs enthält.

**Beispiel H: Ampullen**

Eine Lösung von 1 kg Wirkstoff der Formel I in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

**Patentansprüche**

1. Verbindungen der Formel I

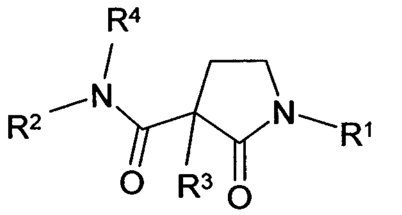

I

worin

$R^1$ unsubstituiertes oder ein-, zwei-, drei-, vier- oder fünffach durch Hal, CN, NHCOA, $NHSO_2A$, $SO_2A$ und/oder $CONH_2$ substituiertes Phenyl, Benzyl, Naphthyl oder Biphenyl; A oder $(CH_2)_nHet$,
$R^2$ $[C(R^4)_2]_nAr^2$, $(CH_2)_nCyc$, $CH[B(OH)_2]CH_2Het$,

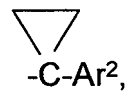

CH(C≡CH)phenyl, A oder $(CH_2)_nHet$,
$R^3$ OH, $N_3$, $NH_2$ oder F,
$R^4$ H oder Alkyl mit 1, 2, 3 oder 4 C-Atomen,
$R^2$ und $R^4$ zusammen auch Alkylen mit 2, 3, 4 oder 5 C-Atomen, wobei eine $CH_2$-Gruppe auch durch NH, NA, N-COA, $N-(CH_2)_nAr^3$, $N-(CH_2)_nHet^2$, CH-A, $CH-O-(CH_2)_nAr^3$, $N-SO_2A$ oder O ersetzt sein kann und/oder durch A substituiert sein kann,
Het unsubstituiertes oder ein-, zwei- oder dreifach durch A, OA, COOA, COA, CHO, $(CH_2)_nCONH_2$, $(CH_2)_nCONHA$, $(CH_2)_nCONA_2$, $SO_2A$, $NHSO_2A$, =O und/oder $Het^1$ substituiertes Pyridazinyl, Pyrazolyl, Benzimidazolyl, Pyridyl, Dibenzofuranyl, Carbazolyl, Indolyl, Dihydro-indolyl, Benzofuranyl, Dihydro-benzofuranyl, 2,3-Dihydrobenzo[1,4]dioxin-yl, Chromanyl, Piperazinyl, Morpholinyl, Tetrahydropyranyl, Chinolinyl, Isochinolinyl, Isoindolyl, Dihydrochinolinyl, Dihydroisochinolinyl, Tetrahydrochinolinyl, Tetrahydroisochinolinyl, Chinazolinyl, Chinoxalinyl, Phthalazinyl, Purinyl, Naphthyridinyl, Pyrimidinyl, Indazolyl, Furyl, Thienyl, Imidazolyl, Pyrrolyl, Oxazolyl, Oxadiazolyl, Isoxazolyl, Thiazolyl, Triazolyl, Tetrazolyl, Thiadiazol, Benzothiazolyl, Imidazo[1,2-a]pyridinyl, 1,3-Benzodioxolyl, Benzoxazolyl, Piperidin-1-yl, Pyrrolidin-1-yl, [1,2]Oxazinan-2-yl, [1,2,5]Oxadiazinan-2-yl, [1,3]Oxazinan-3-yl oder Hexahydropyrimidinyl,
$Het^1$ unsubstituiertes oder ein-, zwei- oder dreifach durch A und/oder OA substituiertes Pyridazinyl, Pyrazolyl, Pyridyl, Piperazinyl, Morpholinyl, Pyrimidinyl, Furyl, Thienyl, Imidazolyl, Pyrrolyl, Oxazolyl, Isoxazolyl, Thiazolyl, Triazolyl, Tetrazolyl, Thiadiazol, Piperidin-1-yl, Pyrrolidin-1-yl, Tetrahydropyranyl, [1,2]Oxazinan-2-yl, [1,2,5]Oxadiazinan-2-yl, [1,3]Oxazinan-3-yl oder Hexahydropyrimidinyl,
$Het^2$ Pyridyl, Pyrimidinyl, Furyl, Thienyl, Imidazolyl, Pyrrolyl, Oxazolyl, Oxadiazolyl, Isoxazolyl, Thiazolyl, Triazolyl, Tetrazolyl oder Thiadiazol,
A unverzweigtes oder verzweigtes Alkyl mit 1-10 C-Atomen, worin 1-7 H-Atome durch F, Cl, Br, OH, CHO, COA, COOA, CN, $CONA_2$, CONHA und/oder $CONH_2$ ersetzt sein können, und/oder worin eine oder zwei nichtbenachbarte CH- und/oder $CH_2$-Gruppen durch O, N und/oder $NR^4$ ersetzt sein können,
oder Cyc,
$Ar^2$ unsubstituiertes oder ein-, zwei-, drei-, vier- oder fünffach durch A, Hal, CN, OH und/oder OA substituiertes Phenyl,
$Ar^3$ unsubstituiertes oder ein-, zwei- oder dreifach durch Hal, OH, OA und/oder A substituiertes Phenyl,
Cyc cyclisches Alkyl mit 3-7 C-Atomen,
Hal F, Cl, Br oder I,
n 0, 1, 2, 3 oder 4,

bedeutet,
sowie ihre pharmazeutisch verwendbaren Salze, Tautomere und Stereoisomere, einschließlich deren Mischungen in allen Verhältnissen.

**2.** Verbindungen nach Anspruch 1, ausgewählt aus der Gruppe

| Verbindung Nr. | Struktur/Name |
|---|---|
| "A1" | 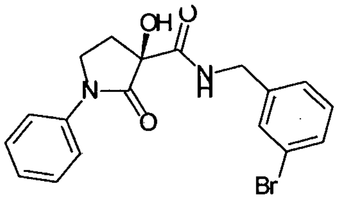 (S)-3-Hydroxy-2-oxo-1-phenyl-pyrrolidin-3-carbonsäure-3-brom-benzylamid |
| "A2" | 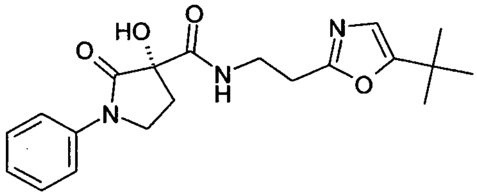 (S)-3-Hydroxy-2-oxo-1-phenyl-pyrrolidin-3-carbonsäure-[2-(5-tert.-butyl-oxazol-2-yl)-ethyl]-amid |
| "A3" | 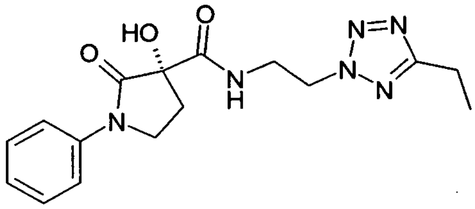 (S)-3-Hydroxy-2-oxo-1-phenyl-pyrrolidin-3-carbonsäure-[2-(5-ethyl-tetrazol-2-yl)-ethyl]-amid |
| "A4" | 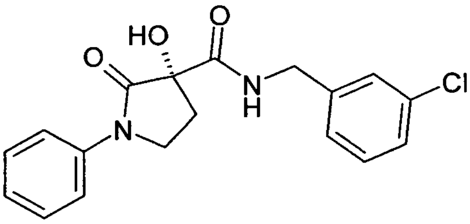 (S)-3-Hydroxy-2-oxo-1-phenyl-pyrrolidin-3-carbonsäure-3-chlor-benzylamid |
| "A5" | 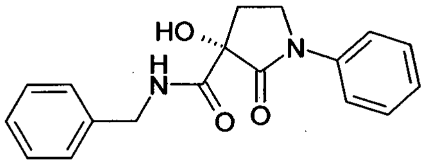 (S)-3-Hydroxy-2-oxo-1-phenyl-pyrrolidin-3-carbonsäure-benzylamid |
| "A6" | 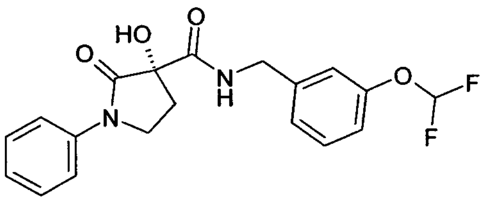 (S)-3-Hydroxy-2-oxo-1-phenyl-pyrrolidin-3-carbonsäure-3-difluormethoxy-benzylamid |

(fortgesetzt)

| Verbindung Nr. | Struktur/Name |
|---|---|
| "A7" | 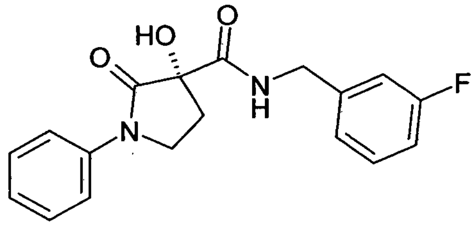 (S)-3-Hydroxy-2-oxo-1-phenyl-pyrrolidin-3-carbonsäure-3-fluor-benzylamid |
| "A8" | 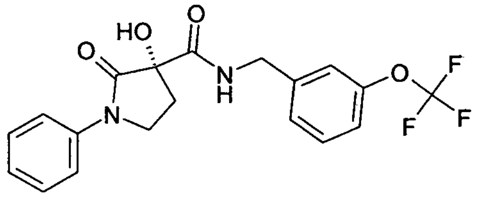 (S)-3-Hydroxy-2-oxo-1-phenyl-pyrrolidin-3-carbonsäure-3-trifluormethoxy-benzylamid |
| "A9" | 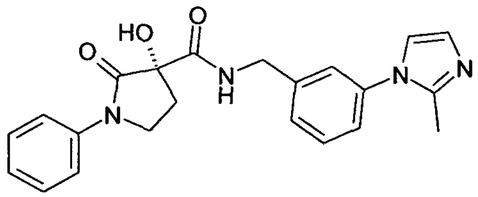 (S)-3-Hydroxy-2-oxo-1-phenyl-pyrrolidin-3-carbonsäure-3-(2-methyl-imidazol-1-yl)-benzylamid |
| "A10" | 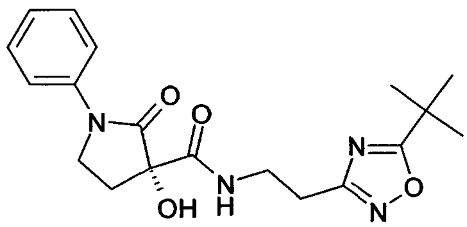 (S)-3-Hydroxy-2-oxo-1-phenyl-pyrrolidin-3-carbonsäure-[2-(5-tert.-butyl-[1,2,4]oxadiazol-3-yl)-ethyl]-amid |
| "A11" | 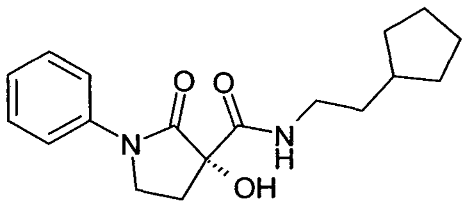 (S)-3-Hydroxy-2-oxo-1-phenyl-pyrrolidin-3-carbonsäure-(2-cyclopentyl-ethyl)-amid |
| "A12" | 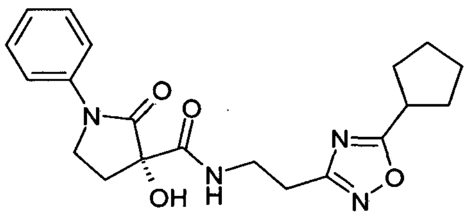 (S)-3-Hydroxy-2-oxo-1-phenyl-pyrrolidin-3-carbonsäure-[2-(5-cyclopentyl-[1,2,4]oxadiazol-3-yl)-ethyl]-amid |

(fortgesetzt)

| Verbindung Nr. | Struktur/Name |
|---|---|
| "A13" | 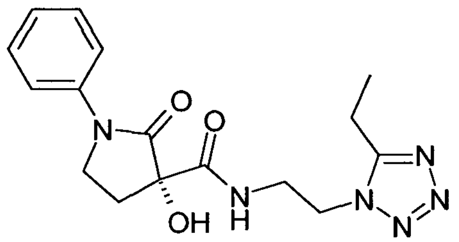<br>(S)-3-Hydroxy-2-oxo-1-phenyl-pyrrolidin-3-carbonsäure-[2-(5-ethyl-tetrazol-1-yl)-ethyl]-amid |
| "A14" | 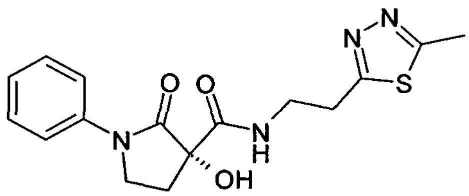<br>(S)-3-Hydroxy-2-oxo-1-phenyl-pyrrolidin-3-carbonsäure-[2-(5-methyl-[1,3,4]thiadiazol-2-yl)-ethyl]-amid |
| "A15" | 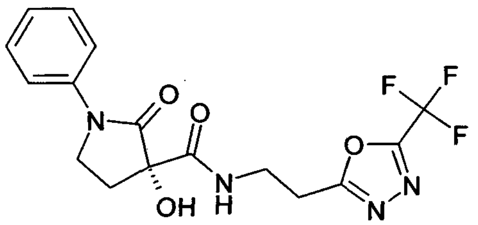<br>(S)-3-Hydroxy-2-oxo-1-phenyl-pyrrolidin-3-carbonsäure-[2-(5-trifluormethyl-[1,3,4]oxadiazol-2-yl)-ethyl]-amid |
| "A16" | 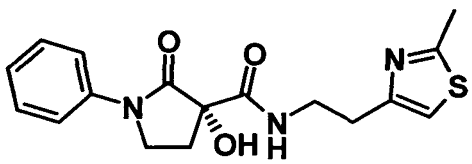<br>(S)-3-Hydraxy-2-oxo-1-phenyl-pyrrolidin-3-carbonsäure-[2-(2-methyl-thiazol-4-yl)-ethyl]-amid |
| "A17" | 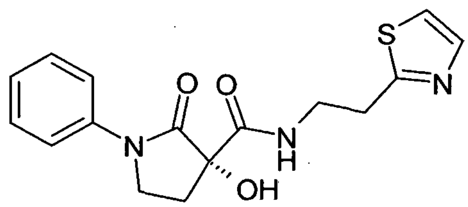<br>(S)-3-Hydroxy-2-oxo-1-phenyl-pyrrolidin-3-carbonsäure-(2-thiazol-2-yl-ethyl)-amid |
| "A18" | 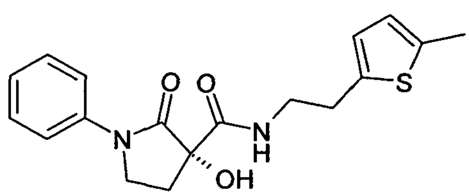<br>(S)-3-Hydroxy-2-oxo-1-phenyl-pyrrolidin-3-carbonsäure-[2-(5-methyl-thiophen-2-yl)-ethyl]-amid |

(fortgesetzt)

| Verbindung Nr. | Struktur/Name |
|---|---|
| "A19" | 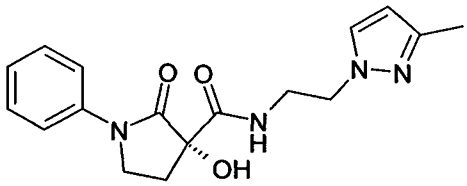 (S)-3-Hydroxy-2-oxo-1-phenyl-pyrrolidin-3-carbonsäure-[2-(3-methyl-pyrazol-1-yl)-ethyl]-amid |
| "A20" | 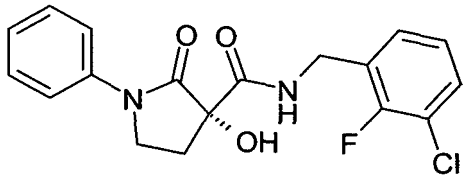 (S)-3-Hydroxy-2-oxo-1-phenyl-pyrrolidin-3-carbonsäure-3-chlor-2-fluor-benzylamid |
| "A21" | 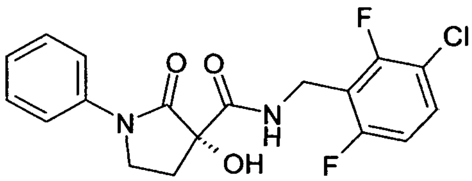 (S)-3-Hydroxy-2-oxo-1-phenyl-pyrrolidin-3-carbonsäure-3-chlor-2,6-difluor-benzylamid |
| "A22" | 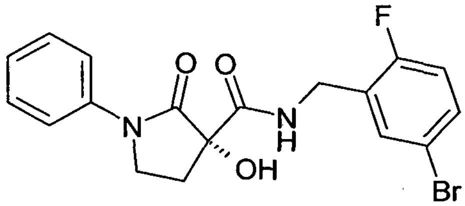 (S)-3-Hydroxy-2-oxo-1-phenyl-pyrrolidin-3-carbonsäure-5-brom-2-fluor-benzylamid |
| "A23" | 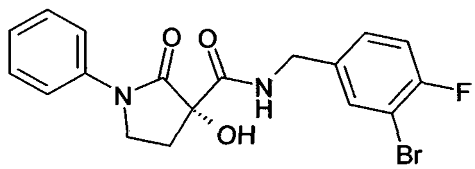 (S)-3-Hydroxy-2-oxo-1-phenyl-pyrrolidin-3-carbonsäure-3-brom-4-fluor-benzylamid |
| "A24" | 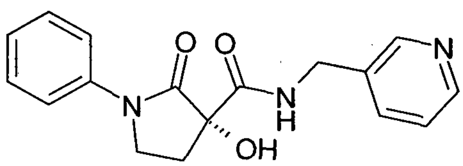 (S)-3-Hydroxy-2-oxo-1-phenyl-pyrrolidin-3-carbonsäure-(pyridin-3-ylmethyl)-amid |
| "A25" | 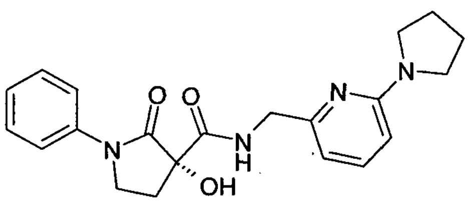 (S)-3-Hydroxy-2-oxo-1-phenyl-pyrrolidin-3-carbonsäure-(6-pyrrolidin-1-yl-pyridin-2-ylmethyl)-amid |

(fortgesetzt)

| Verbindung Nr. | Struktur/Name |
|---|---|
| "A26" | 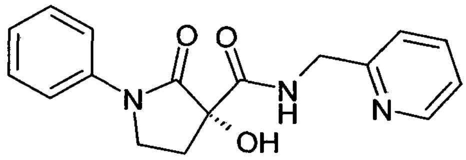 (S)-3-Hydroxy-2-oxo-1-phenyl-pyrrolidin-3-carbonsäure-(pyridin-2-ylmethyl)-amid |
| "A27" | 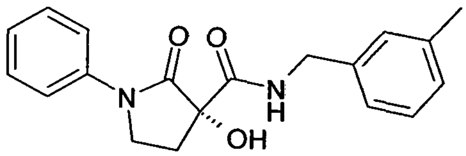 (S)-3-Hydroxy-2-oxo-1-phenyl-pyrrolidin-3-carbonsäure-3-methyl-benzylamid |
| "A28" | 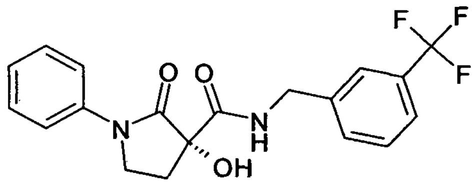 (S)-3-Hydroxy-2-oxo-1-phenyl-pyrrolidin-3-carbonsäure-3-trifluormethyl-benzylamid |
| "A29" | 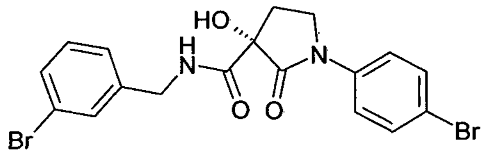 (S)-1-(4-Brom-phenyl)-3-hydroxy-2-oxo-pyrrolidin-3-carbonsäure-3-brom-benzylamid |
| "A30" | 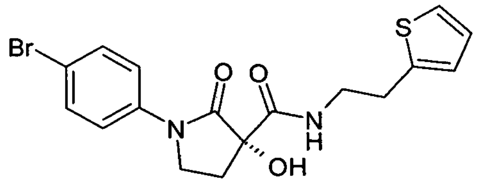 (S)-1-(4-Bromo-phenyl)-3-hydroxy-2-oxo-pyrrolidine-3-carboxylic acid (2-thiophen-2-yl-ethyl)-amide |
| "A31" | 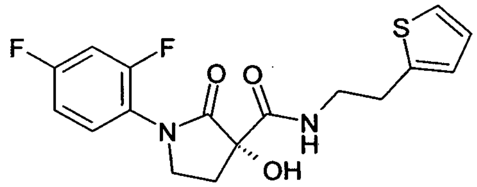 (S)-1-(2,4-Difluor-phenyl)-3-hydroxy-2-oxo-pyrrolidin-3-carbonsäure-(2-thiophen-2-yl-ethyl)-amid |
| "A32" | 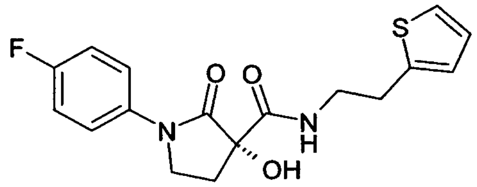 (S)-1-(4-Fluor-phenyl)-3-hydroxy-2-oxo-pyrrolidin-3-carbonsäure-(2-thiophen-2-yl-ethyl)-amid |

(fortgesetzt)

| Verbindung Nr. | Struktur/Name |
|---|---|
| "A33" | 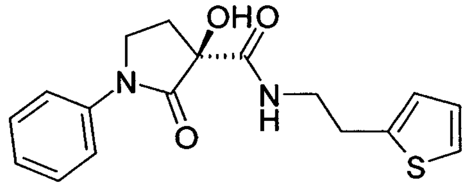 (S)-3-Hydroxy-2-oxo-1-phenyl-pyrrolidin-3-carbonsäure-(2-thiophen-2-yl-ethyl)-amid |
| "A34" | 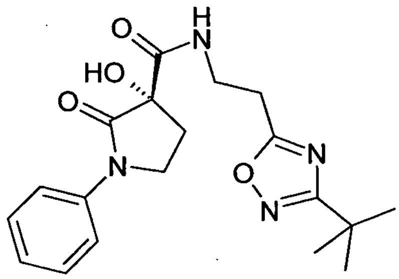 (S)-3-Hydroxy-2-oxo-1-phenyl-pyrrolidin-3-carbonsäure-[2-(3-tert.-butyl-[1,2,4]oxadiazol-5-yl)-ethyl]-amid |
| "A35" | 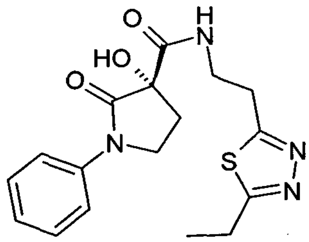 (S)-3-Hydroxy-2-oxo-1-phenyl-pyrrolidin-3-carbonsäure-[2-(5-ethyl-[1,3,4]thiadiazol-2-yl)-ethyl]-amid |
| "A36" | 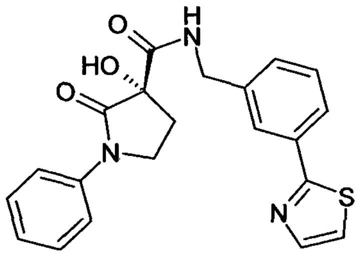 (S)-3-Hydroxy-2-oxo-1-phenyl-pyrrolidin-3-carbonsäure-3-thiazol-2-yl-benzylamid |
| "A37" | 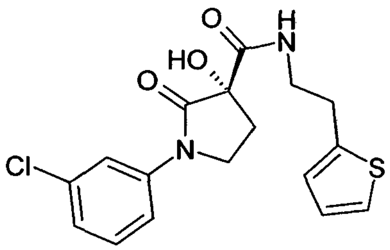 (S)-1-(3-Chlor-phenyl)-3-hydroxy-2-oxo-pyrrolidin-3-carbonsäure-(2-thiophen-2-yl-ethyl)-amid |

(fortgesetzt)

| Verbindung Nr. | Struktur/Name |
|---|---|
| "A38" | 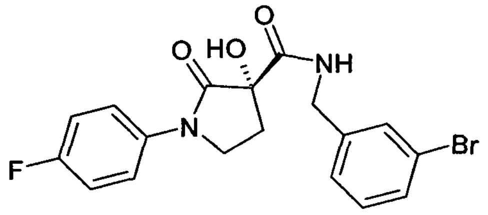 (S)-1-(4-Fluor-phenyl)-3-hydroxy-2-oxo-pyrrolidin-3-carbonsäure-3-brom-benzylamid |
| "A39" | 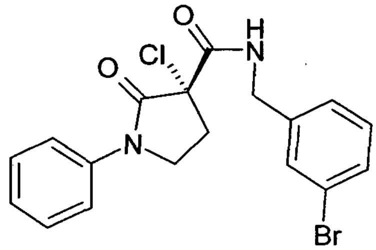 (S)-3-Chlor-2-oxo-1-phenyl-pyrrolidin-3-carbonsäure-3-brom-benzylamid |
| "A40" | 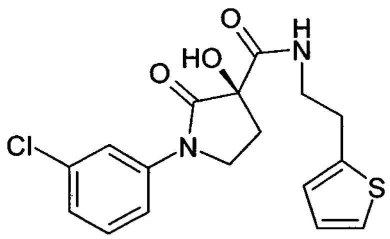 (R)-1-(3-Chlor-phenyl)-3-hydroxy-2-oxo-pyrrolidin-3-carbonsäure-(2-thiophen-2-yl-ethyl)-amid |
| "A41" | 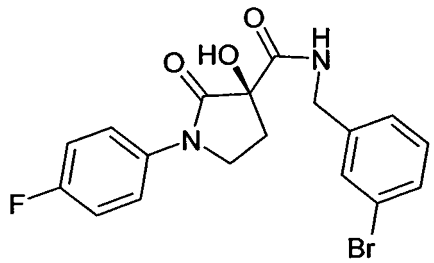 (R)-1-(4-Fluor-phenyl)-3-hydroxy-2-oxo-pyrrolidin-3-carbonsäure-3-brom-benzylamid |
| "A42" | 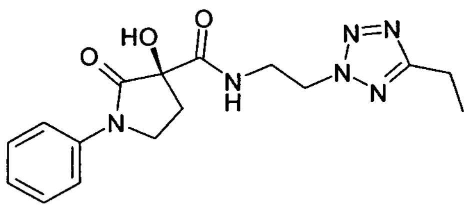 (R)-3-Hydroxy-2-oxo-1-phenyl-pyrrolidin-3-carbonsäure-[2-(5-ethyl-tetrazol-2-yl)-ethyl]-amid |

(fortgesetzt)

| Verbindung Nr. | Struktur/Name |
|---|---|
| "A43" | 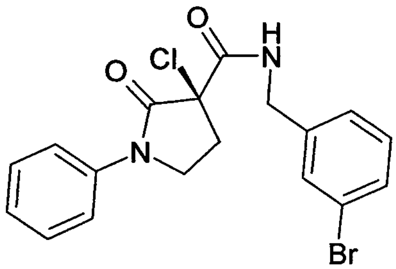 (R)-3-Chlor-2-oxo-1-phenyl-pyrrolidin-3-carbonsäure-3-brom-benzylamid |
| "A44" | 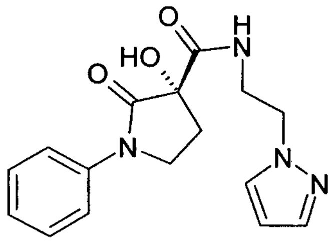 (S)-3-Hydroxy-2-oxo-1-phenyl-pyrrolidin-3-carbonsäure-(2-pyrazol-1-yl-ethyl)-amid |
| "A45" | 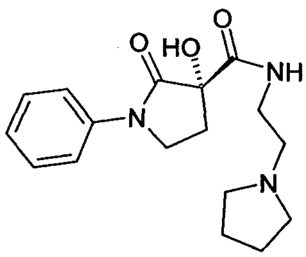 (S)-3-Hydroxy-2-oxo-1-phenyl-pyrrolidin-3-carbonsäure-(2-pyrrolidin-1-yl-ethyl)-amid |
| "A46" | 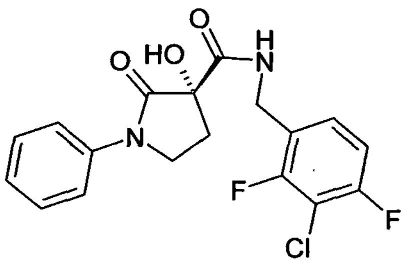 (S)-3-Hydroxy-2-oxo-1-phenyl-pyrrolidin-3-carbonsäure-3-chlor-2,4-difluor-benzylamid |
| "A47" | 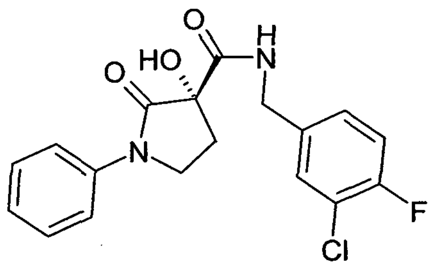 (S)-3-Hydroxy-2-oxo-1-phenyl-pyrrolidin-3-carbonsäure-3-chlor-4-fluor-benzylamid |

(fortgesetzt)

| Verbindung Nr. | Struktur/Name |
|---|---|
| "A48" | 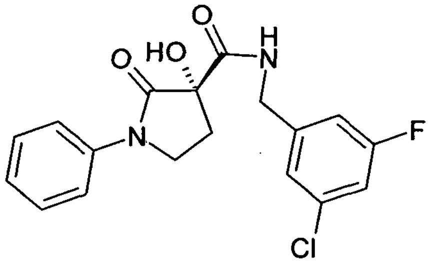<br>(S)-3-Hydroxy-2-oxo-1-phenyl-pyrrolidin-3-carbonsäure-3-chlor-5-fluor-benzylamid |
| "A49" | 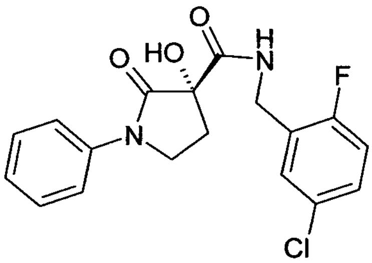<br>(S)-3-Hydroxy-2-oxo-1-phenyl-pyrrolidin-3-carbonsäure-5-chlor-2-fluor-benzylamid |
| "A50" | 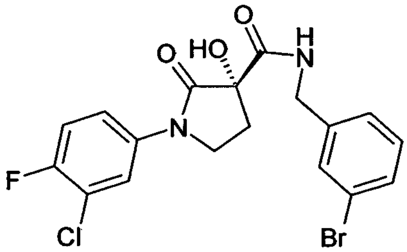<br>(S)-1-(3-Chlor-4-fluor-phenyl)-3-hydroxy-2-oxo-pyrrolidin-3-carbonsäure-3-brom-benzylamid |
| "A51" | 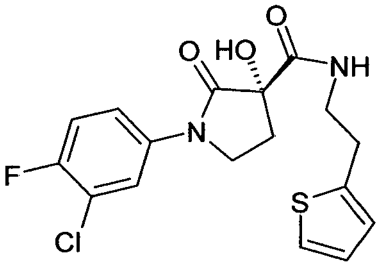<br>(S)-1-(3-Chlor-4-fluor-phenyl)-3-hydroxy-2-oxo-pyrrolidin-3-carbonsäure-(2-thiophen-2-yl-ethyl)-amid |
| "A52" | 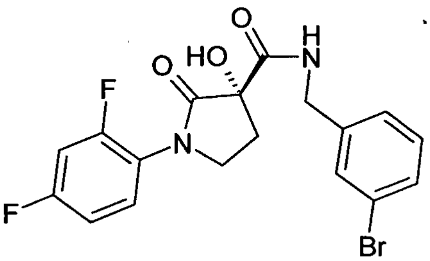<br>(S)-1-(2,4-Difluor-phenyl)-3-hydroxy-2-oxo-pyrrolidin-3-carbonsäure-3-brom-benzylamid |

(fortgesetzt)

| Verbindung Nr. | Struktur/Name |
|---|---|
| "A53" | 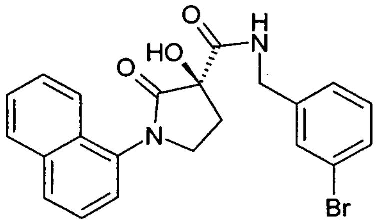 (R)-3-Hydroxy-1-naphthalin-1-yl-2-oxo-pyrrolidin-3-carbonsäure-3-brom-benzylamid |
| "A54" | 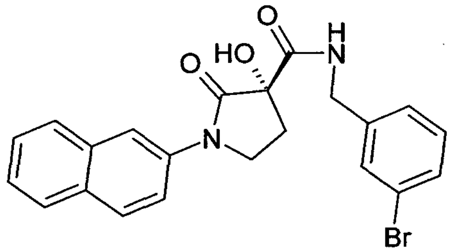 (S)-3-Hydroxy-1-naphthalin-2-yl-2-oxo-pyrrolidin-3-carbonsäure-3-brom-benzylamid |
| "A55" | 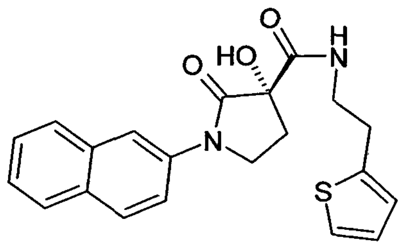 (S)-3-Hydroxy-1-naphthalin-2-yl-2-oxo-pyrrolidin-3-carbonsäure-(2-thiophen-2-yl-ethyl)-amid |
| "A56" | 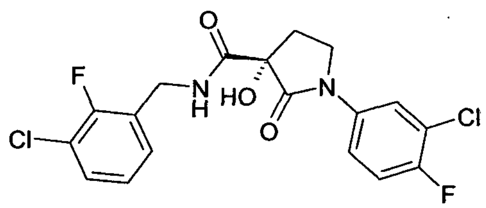 (S)-1-(3-Chlor-4-fluor-phenyl)-3-hydroxy-2-oxo-pyrrolidin-3-carbonsäure-3-chlor-2-fluor-benzylamid |
| "A57" | 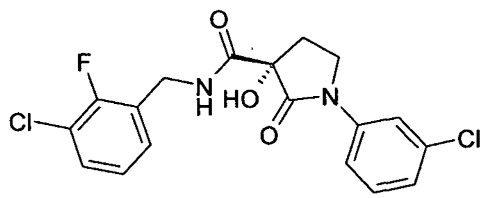 (S)-1-(3-Chlor-phenyl)-3-hydroxy-2-oxo-pyrrolidin-3-carbonsäure-3-chlor-2-fluor-benzylamid |
| "A58" | 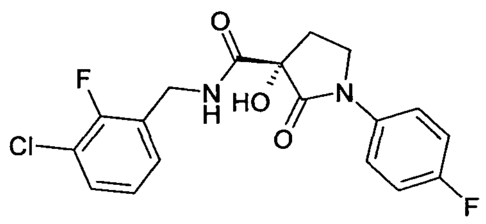 (S)-1-(4-Fluor-phenyl)-3-hydroxy-2-oxo-pyrrolidin-3-carbonsäure-3-chlor-2-fluor-benzylamid |

(fortgesetzt)

| Verbindung Nr. | Struktur/Name |
|---|---|
| "A59" | 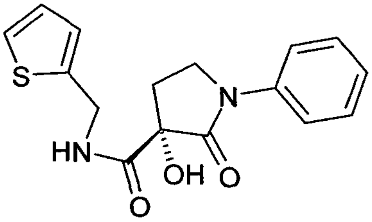 (S)-3-Hydroxy-2-oxo-1-phenyl-pyrrolidin-3-carbonsäure-(thiophen-2-ylmethyl)-amid |
| "A60" | 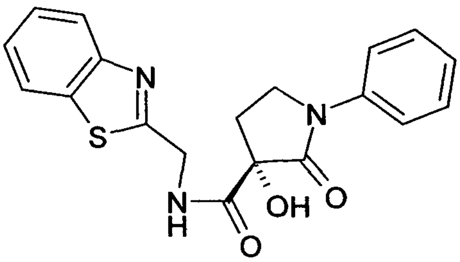 (S)-3-Hydroxy-2-oxo-1-phenyl-pyrrolidin-3-carbonsäure-(benzothiazol-2-ylmethyl)-amid |
| "A61" | 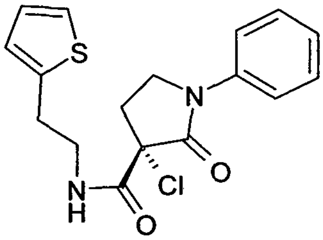 (S)-3-Chlor-2-oxo-1-phenyl-pyrrolidin-3-carbonsäure-(2-thiophen-2-yl-ethyl)-amid |
| "A62" | 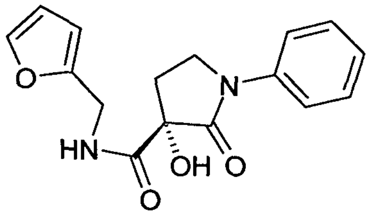 (S)-3-Hydroxy-2-oxo-1-phenyl-pyrrolidin-3-carbonsäure-(furan-2-ylmethyl)-amid |
| "A63" | 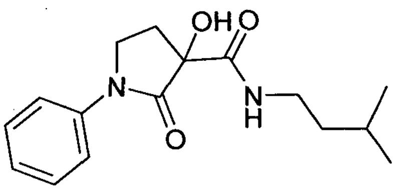 3-Hydroxy-2-oxo-1-phenyl-pyrrolidin-3-carbonsäure-(3-methyl-butyl)-amid |
| "A64" | 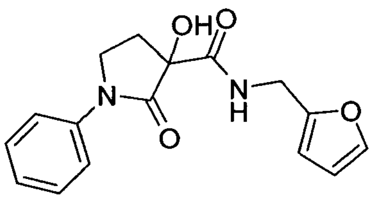 3-Hydroxy-2-oxo-1-phenyl-pyrrolidin-3-carbonsäure-(furan-2-ylmethyl)-amid |

(fortgesetzt)

| Verbindung Nr. | Struktur/Name |
|---|---|
| "A65" | 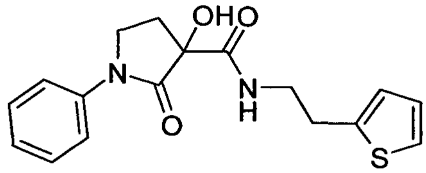 3-Hydroxy-2-oxo-1-phenyl-pyrrolidin-3-carbonsäure-(2-thiophen-2-yl-ethyl)-amid |
| "A66" | 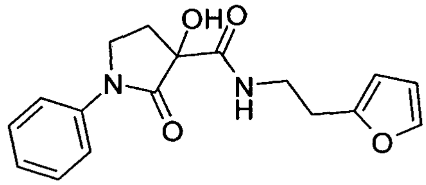 3-Hydroxy-2-oxo-1-phenyl-pyrrolidin-3-carbonsäure-(2-furan-2-yl-ethyl)-amid |
| "A67" | 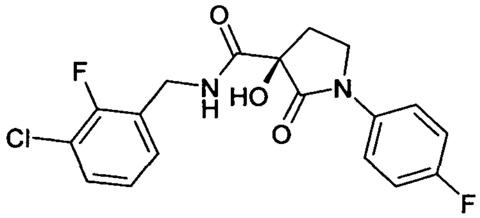 (R)-1-(4-Fluor-phenyl)-3-hydroxy-2-oxo-pyrrolidin-3-carbonsäure-3-chlor-2-fluor-benzylamid |
| "A68" | 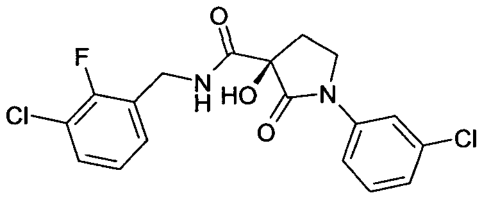 (R)-1-(3-Chlor-phenyl)-3-hydroxy-2-oxo-pyrrolidin-3-carbonsäure-3-chlor-2-fluor-benzylamid |
| "A69" | 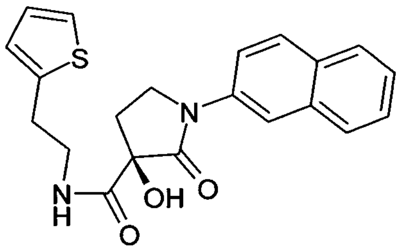 (R)-3-Hydroxy-1-naphthalin-2-yl-2-oxo-pyrrolidin-3-carbonsäure-(2-thiophen-2-yl-ethyl)-amid |
| "A70" | 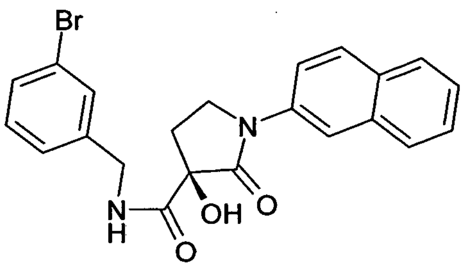 (R)-3-Hydroxy-1-naphthalin-2-yl-2-oxo-pyrrolidin-3-carbonsäure-3-brom-benzylamid |

(fortgesetzt)

| Verbindung Nr. | Struktur/Name |
|---|---|
| "A71" | 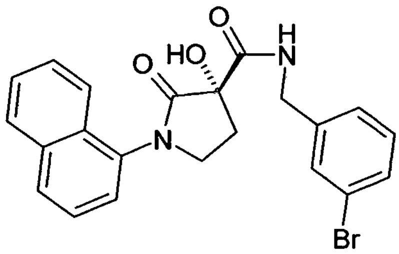<br>(S)-3-Hydroxy-1-naphthalin-1-yl-2-oxo-pyrrolidin-3-carbonsäure-3-brom-benzylamid |
| "A72" | 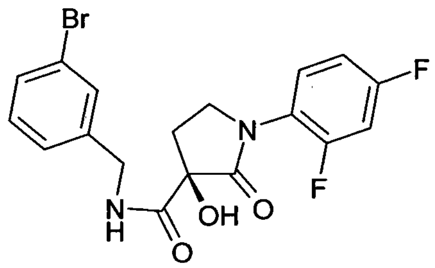<br>(R)-1-(2,4-Difluor-phenyl)-3-hydroxy-2-oxo-pyrrolidin-3-carbonsäure-3-brom-benzylamid |
| "A73" | 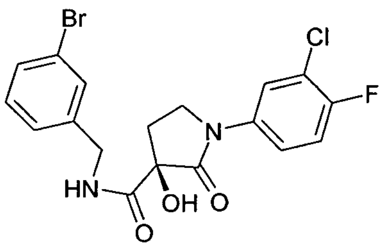<br>(R)-1-(3-Chlor-4-fluor-phenyl)-3-hydroxy-2-oxo-pyrrolidin-3-carbonsäure-3-brom-benzylamid |
| "A74" | 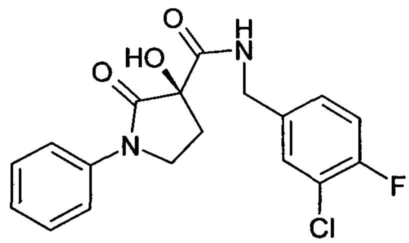<br>(R)-3-Hydroxy-2-oxo-1-phenyl-pyrrolidin-3-carbonsäure-3-chlor-4-fluor-benzylamid |
| "A75" | 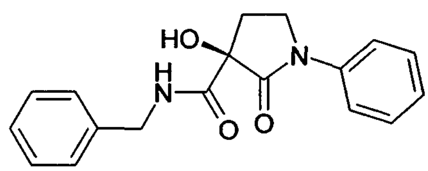<br>(R)-3-Hydroxy-2-oxo-1-phenyl-pyrrolidin-3-carbonsäure-benzylamid |
| "A76" | 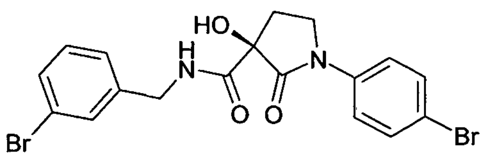<br>(R)-1-(4-Brom-phenyl)-3-hydroxy-2-oxo-pyrrolidin-3-carbonsäure-3-brom-benzylamid |

(fortgesetzt)

| Verbindung Nr. | Struktur/Name |
|---|---|
| "A77" | 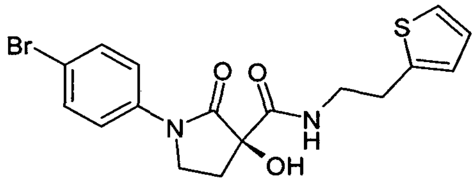 (R)-1-(4-Brom-phenyl)-3-hydroxy-2-oxo-pyrrolidin-3-carbonsäure-(2-thiophen-2-yl-ethyl)-amid |
| "A78" | 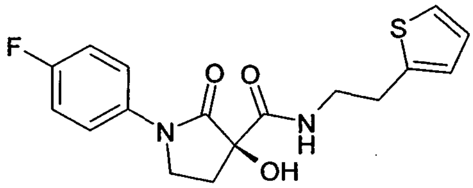 (R)-1-(4-Fluor-phenyl)-3-hydroxy-2-oxo-pyrrolidin-3-carbonsäure-(2-thiophen-2-yl-ethyl)-amid |
| "A79" | 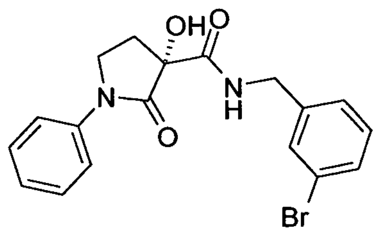 (R)-3-Hyd roxy-2-oxo-1-phenyl-pyrrolidin-3-carbonsäure-3-brom-benzylamid |
| "A80" | 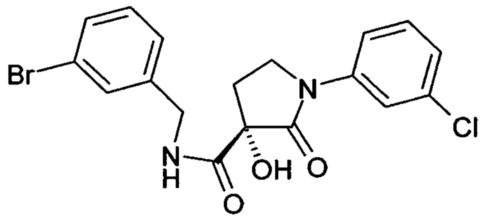 (S)-1-(3-Chlor-phenyl)-3-hydroxy-2-oxo-pyrrolidin-3-carbonsäure-3-brom-benzylamid |
| "A81" | 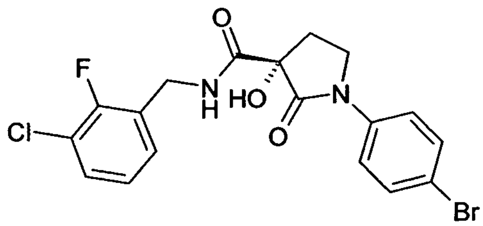 (S)-1-(4-Brom-phenyl)-3-hydroxy-2-oxo-pyrrolidin-3-carbonsäure-3-chlor-2-fluor-benzylamid |
| "A82" | 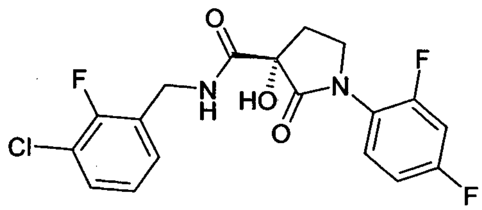 (S)-1-(2,4-Difluor-phenyl)-3-hydroxy-2-oxo-pyrrolidin-3-carbonsäure-3-chlor-2-fluor-benzylamid |

(fortgesetzt)

| Verbindung Nr. | Struktur/Name |
|---|---|
| "A83" | 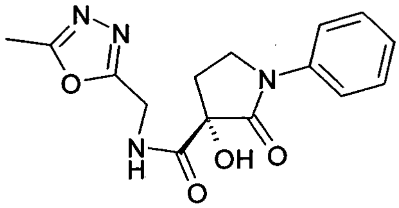 (S)-3-Hydroxy-2-oxo-1-phenyl-pyrrolidin-3-carbonsäure-(5-methyl-[1,3,4]oxadiazol-2-ylmethyl)-amid |
| "A84" | 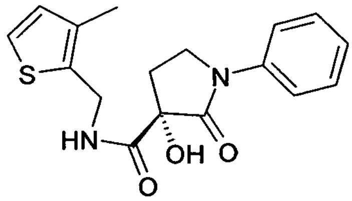 (S)-3-Hydroxy-2-oxo-1-phenyl-pyrrolidin-3-carbonsäure-(3-methyl-thiophen-2-ylmethyl)-amid |
| "A85" | 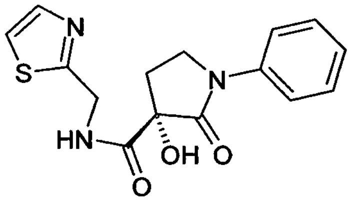 (S)-3-Hydroxy-2-oxo-1-phenyl-pyrrolidin-3-carbonsäure-(thiazol-2-ylmethyl)-amid |
| "A86" | 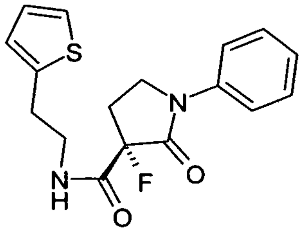 (S)-3-Fluor-2-oxo-1-phenyl-pyrrolidin-3-carbonsäure-(2-thiophen-2-yl-ethyl)-amid |
| "A87" | 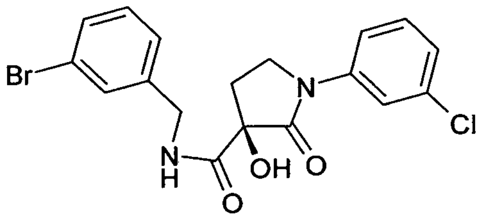 (R)-1-(3-Chlor-phenyl)-3-hydroxy-2-oxo-pyrrolidin-3-carbonsäure-3-brom-benzylamid |
| "A88" | 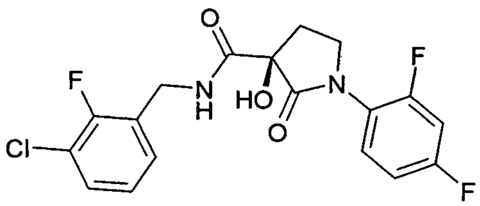 (R)-1-(2,4-Difluor-phenyl)-3-hydroxy-2-oxo-pyrrolidin-3-carbonsäure-3-chlor-2-fluor-benzylamid |

(fortgesetzt)

| Verbindung Nr. | Struktur/Name |
|---|---|
| "A89" | 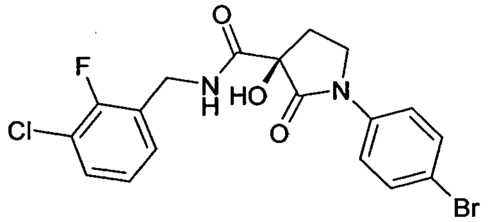 (R)-1-(4-Brom-phenyl)-3-hydroxy-2-oxo-pyrrolidin-3-carbonsäure-3-chlor-2-fluor-benzylamid |
| "A90" | 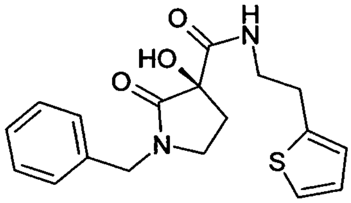 (R)-1-Benzyl-3-hydroxy-2-oxo-pyrrolidin-3-carbonsäure-(2-thiophen-2-yl-ethyl)-amid |
| "A91" | 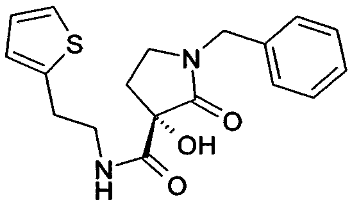 (S)-1-Benzyl-3-hydroxy-2-oxo-pyrrolidin-3-carbonsäure-(2-thiophen-2-yl-ethyl)-amid |
| "A91a" | (S)-3-Amino-2-oxo-1-phenyl-pyrrolidin-3-carbonsäure-(3-chlor-5-fluor-benzyl)-amid |
| "A92" | 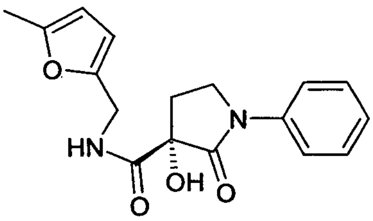 (S)-3-Hydroxy-2-oxo-1-phenyl-pyrrolidin-3-carbonsäure-(5-methyl-furan-2-ylmethyl)-amid |
| "A93" | 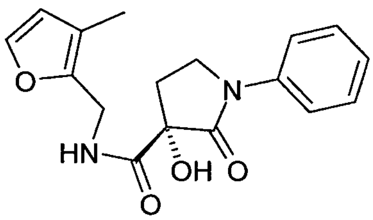 (S)-3-Hydroxy-2-oxo-1-phenyl-pyrrolidin-3-carbonsäure-(3-methyl-furan-2-ylmethyl)-amid |
| "A94" | 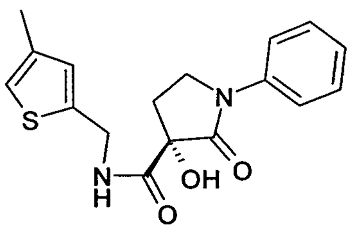 (S)-3-Hydroxy-2-oxo-1-phenyl-pyrrolidin-3-carbonsäure-(4-methyl-thiophen-2-ylmethyl)-amid |

(fortgesetzt)

| Verbindung Nr. | Struktur/Name |
|---|---|
| "A95" | 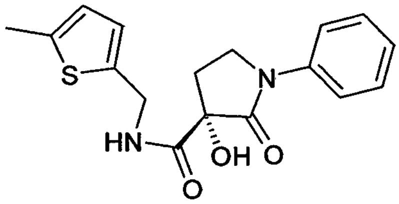 (S)-3-Hydroxy-2-oxo-1-phenyl-pyrrolidin-3-carbonsäure-(5-methyl-thiophen-2-ylmethyl)-amid |
| "A96" | 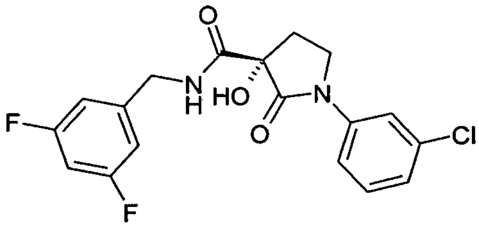 (S)-1-(3-Chlor-phenyl)-3-hydroxy-2-oxo-pyrrolidin-3-carbonsäure-3,5-difluor-benzylamid |
| "A97" | 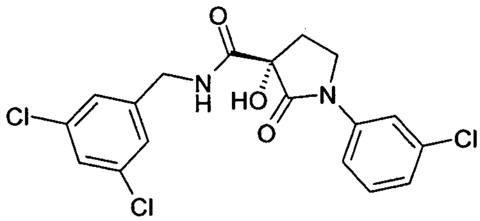 (S)-1-(3-Chlor-phenyl)-3-hydroxy-2-oxo-pyrrolidin-3-carbonsäure-3,5-dichlor-benzylamid |
| "A98" | 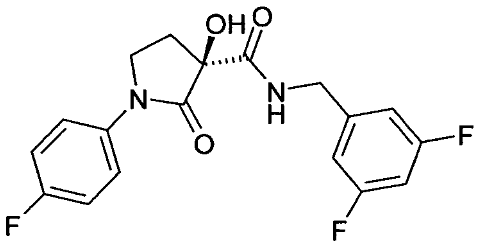 (S)-1-(4-Fluor-phenyl)-3-hydroxy-2-oxo-pyrrolidin-3-carbonsäure-3,5-difluor-benzylamid |
| "A99" | 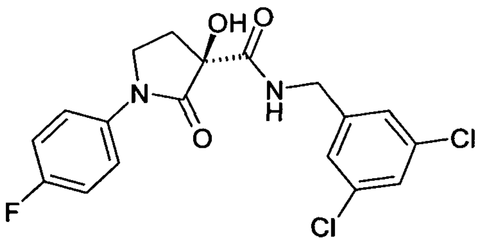 (S)-1-(4-Fluor-phenyl)-3-hydroxy-2-oxo-pyrrolidin-3-carbonsäure-3,5-dichlor-benzylamid |
| "A100" | 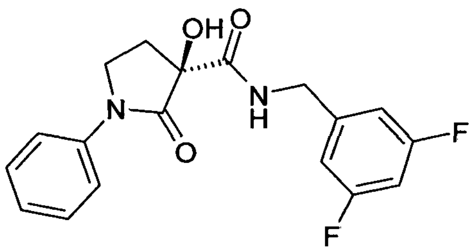 (S)-3-Hydroxy-2-oxo-1-phenyl-pyrrolidin-3-carbonsäure-3,5-difluor-benzylamid |

(fortgesetzt)

| Verbindung Nr. | Struktur/Name |
|---|---|
| "A101" | 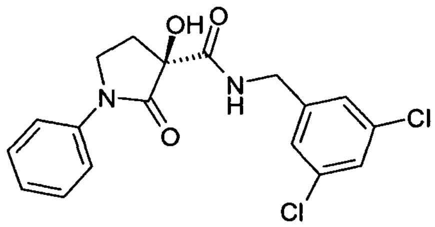<br>(S)-3-Hydroxy-2-oxo-1-phenyl-pyrrolidin-3-carbonsäure-3,5-dichlor-benzylamid |
| "A102" | 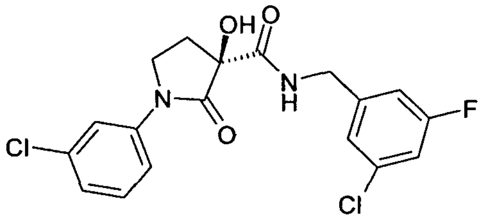<br>(S)-1-(3-Chlor-phenyl)-3-hydroxy-2-oxo-pyrrolidin-3-carbonsäure-3-chlor-5-fluor-benzylamid |
| "A103" | 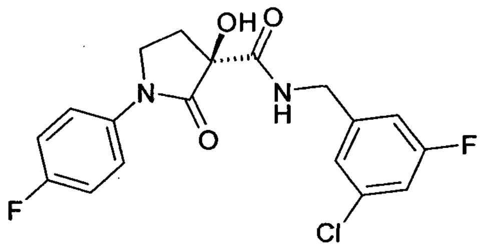<br>(S)-1-(4-Fluor-phenyl)-3-hydroxy-2-oxo-pyrrolidin-3-carbonsäure-3-chlor-5-fluor-benzylamid |
| "A104" | 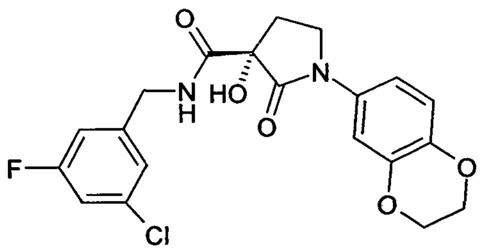<br>(S)-1-(2,3-Dihydro-benzo[1,4]dioxin-6-yl)-3-hydroxy-2-oxo-pyrrolidin-3-carbonsäure-3-chlor-5-fluor-benzylamid |
| "A105" | 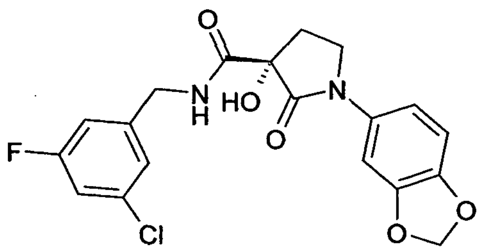<br>(S)-1-Benzo[1,3]dioxol-5-yl-3-hydroxy-2-oxo-pyrrolidin-3-carbonsäure-3-chlor-5-fluor-benzylamid |

(fortgesetzt)

| Verbindung Nr. | Struktur/Name |
|---|---|
| "A106" | 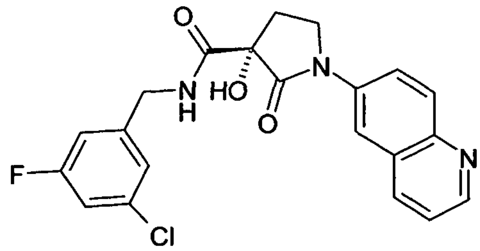 (S)-3-Hydroxy-2-oxo-1-chinolin-6-yl-pyrrolidin-3-carbonsäure-3-chlor-5-fluor-benzylamid |
| "A107" | 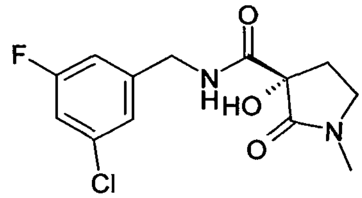 (S)-3-Hydroxy-1-methyl-2-oxo-pyrrolidin-3-carbonsäure-3-chlor-5-fluor-benzylamid |
| "A108" | 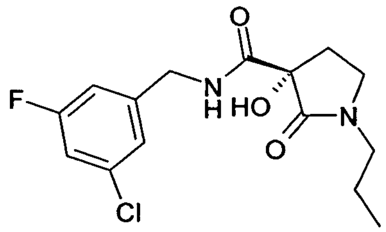 (S)-3-Hydroxy-2-oxo-1-propyl-pyrrolidin-3-carbonsäure-3-chlor-5-fluor-benzylamid |
| "A109" | 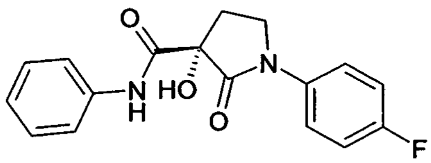 (S)-1-(4-Fluor-phenyl)-3-hydroxy-2-oxo-pyrrolidin-3-carbonsäure-phenylamid |
| "A110" | 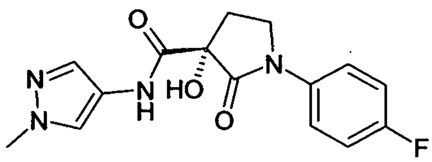 (S)-1-(4-Fluor-phenyl)-3-hydroxy-2-oxo-pyrrolidin-3-carbonsäure-(1-methyl-1H-pyrazol-4-yl)-amid |
| "A111" | 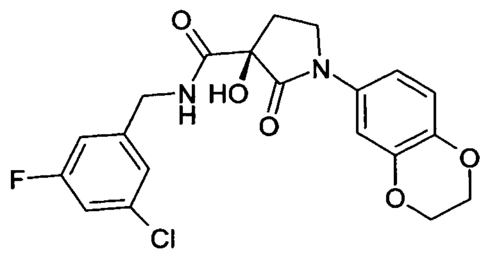 (R)-1-(2,3-Dihydro-benzo[1,4]dioxin-6-yl)-3-hydroxy-2-oxo-pyrrolidin-3-carbonsäure-3-chlor-5-fluor-benzylamid |

(fortgesetzt)

| Verbindung Nr. | Struktur/Name |
|---|---|
| "A112" | 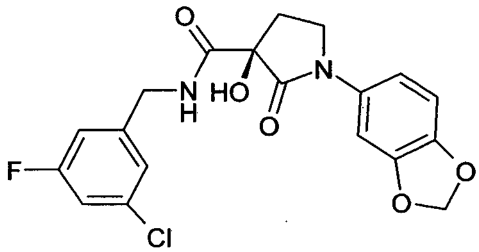 (R)-1-Benzo[1,3]dioxol-5-yl-3-hydroxy-2-oxo-pyrrolidin-3-carbonsäure-3-chlor-5-fluor-benzylamid |
| "A113" | 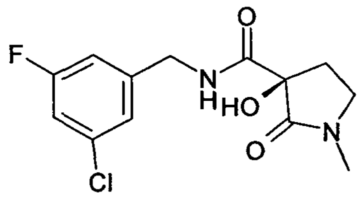 (R)-3-Hydroxy-1-methyl-2-oxo-pyrrolidin-3-carbonsäure-3-chlor-5-fluor-benzylamid |
| "A114" | 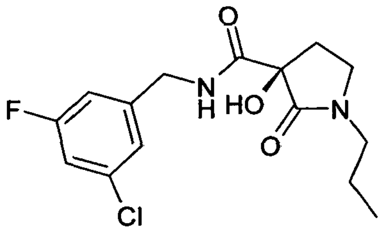 (R)-3-Hydroxy-2-oxo-1-propyl-pyrrolidin-3-carbonsäure-3-chlor-5-fluor-benzylamid |
| "A115" | 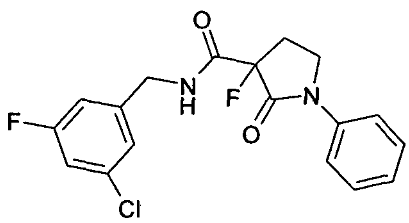 3-Fluor-2-oxo-1-phenyl-pyrrolidin-3-carbonsäure-3-chlor-5-fluor-benzylamid |
| "A116" | 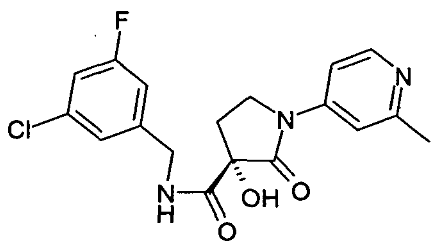 (S)-3-Hydroxy-1-(2-methyl-pyridin-4-yl)-2-oxo-pyrrolidin-3-carbonsäure-3-chlor-5-fluor-benzylamid |
| "A117" | 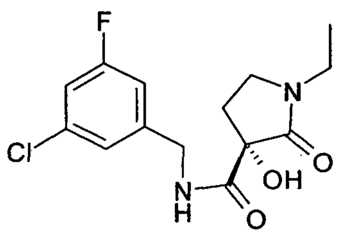 |

(fortgesetzt)

| Verbindung Nr. | Struktur/Name |
|---|---|
| | (S)-1-Ethyl-3-hydroxy-2-oxo-pyrrolidin-3-carbonsäure-3-chlor-5-fluor-benzylamid |
| "A118" | 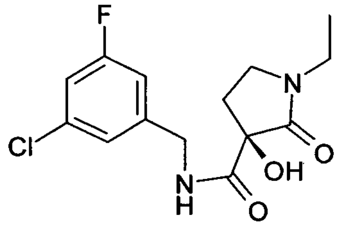<br>(R)-1-Ethyl-3-hydroxy-2-oxo-pyrrolidin-3-carbonsäure-3-chlor-5-fluor-benzylamid |
| "A119" | 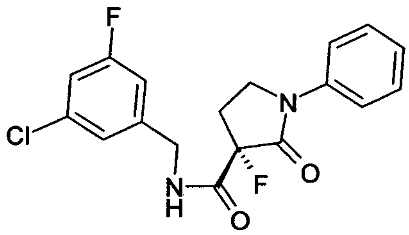<br>(S)-3-Fluor-2-oxo-1-phenyl-pyrrolidin-3-carbonsäure-3-chlor-5-fluor-benzylamid |
| "A120" | 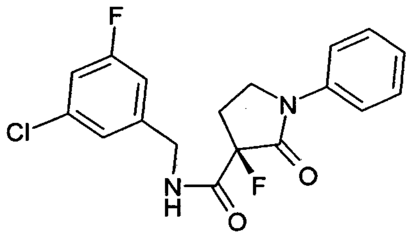<br>(R)-3-Fluor-2-oxo-1-phenyl-pyrrolidin-3-carbonsäure-3-chlor-5-fluor-benzylamid |
| "A150" | 3-Azido-2-oxo-1-phenyl-pyrrolidin-3-carbonsäure-(3-chlor-5-fluor-benzyl)amid |
| "A151" | 3-Amino-2-oxo-1-phenyl-pyrrolidin-3-carbonsäure-(3-chlor-5-fluor-benzyl)amid |
| "A162" | 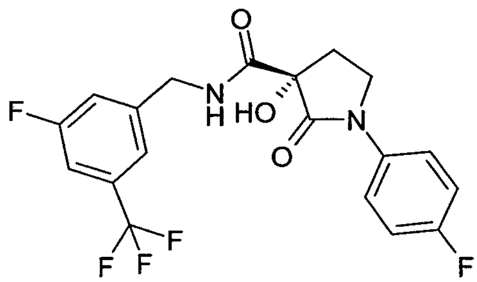<br>(S)-1-(4-Fluor-phenyl)-3-hydroxy-2-oxo-pyrrolidin-3-carbonsäure-3-fluor-5-trifluormethyl-benzylamid |
| "A163" | 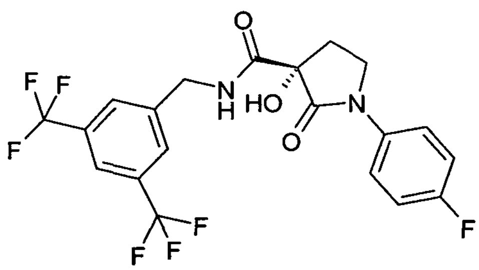<br>(S)-1-(4-Fluor-phenyl)-3-hydroxy-2-oxo-pyrrolidin-3-carbonsäure-3,5-bis-trifluormethyl-benzylamid |

(fortgesetzt)

| Verbindung Nr. | Struktur/Name |
|---|---|
| "A164" | 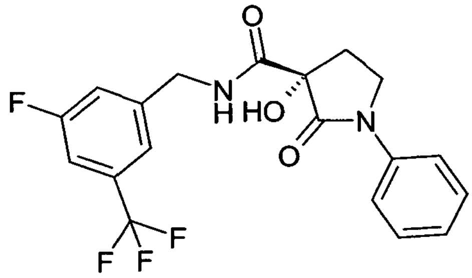 (S)-3-Hydroxy-2-oxo-1-phenyl-pyrrolidin-3-carbonsäure-3-fluor-5-trifluormethyl-benzylamid |
| "A165" | 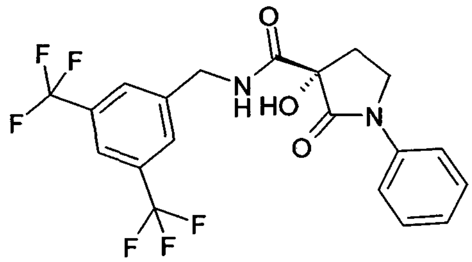 (S)-3-Hydroxy-2-oxo-1-phenyl-pyrrolidin-3-carbonsäure-3,5-bis-trifluormethyl-benzylamid |
| "A166" | 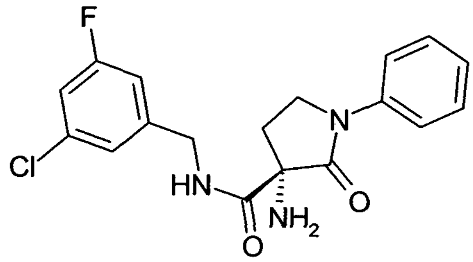 (S)-3-Amino-2-oxo-1-phenyl-pyrrolidin-3-carbonsäure-3-chlor-5-fluor-benzylamid |
| "A167" | 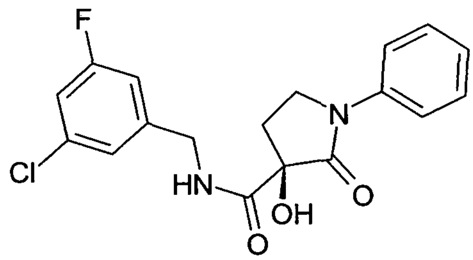 (R)-3-Hydroxy-2-oxo-1-phenyl-pyrrolidin-3-carbonsäure-3-chlor-5-fluor-benzylamid |
| "A168" | 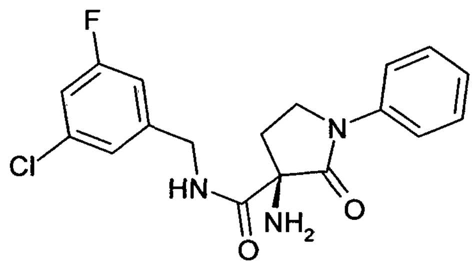 (R)-3-Amino-2-oxo-1-phenyl-pyrrolidin-3-carbonsäure-3-chlor-5-fluor-benzylamid |

(fortgesetzt)

| Verbindung Nr. | Struktur/Name |
|---|---|
| "A169" | 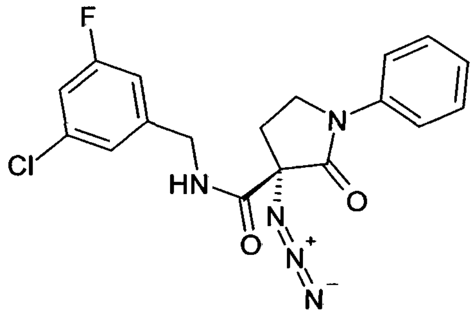<br>(S)-3-Azido-2-oxo-1-phenyl-pyrrolidin-3-carbonsäure-3-chlor-5-fluor-benzylamid |
| "A170" | 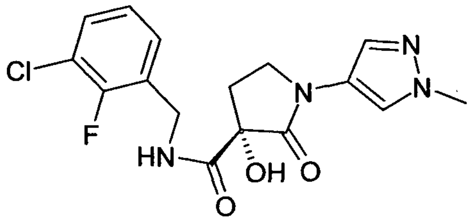<br>(S)-3-Hydroxy-1-(1-methyl-1H-pyrazol-4-yl)-2-oxo-pyrrolidin-3-carbonsäure-3-chlor-2-fluor-benzylamid |
| "A171" | 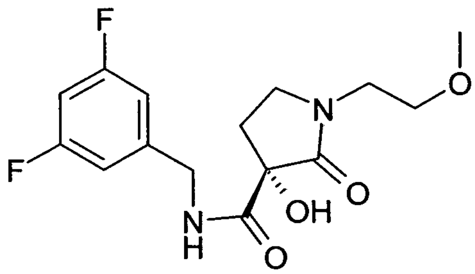<br>(S)-3-Hydroxy-1-(2-methoxy-ethyl)-2-oxo-pyrrolidin-3-carbonsäure-3,5-difluor-benzylamid |
| "A172" | 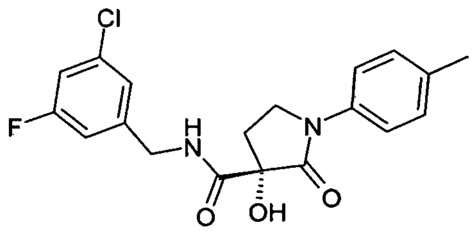<br>(S)-3-Hydroxy-2-oxo-1-p-tolyl-pyrrolidin-3-carbonsäure-3-chlor-5-fluor-benzylamid |
| "A173" | 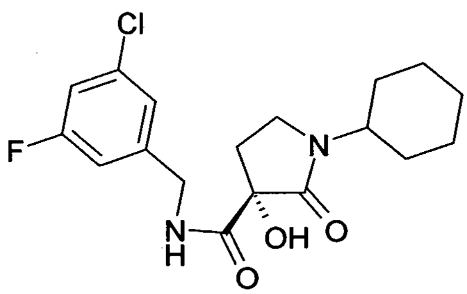<br>(S)-1-Cyclohexyl-3-hyd roxy-2-oxo-pyrrolidin-3-carbonsäure-3-chlor-5-fluor-benzylamid |

(fortgesetzt)

| Verbindung Nr. | Struktur/Name |
|---|---|
| "A174" | 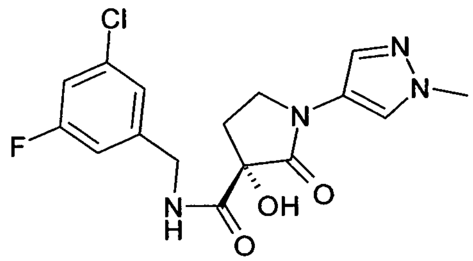 (S)-3-Hydroxy-1-(1-methyl-1H-pyrazol-4-yl)-2-oxo-pyrrolidin-3-carbonsäure-3-chlor-5-fluor-benzylamid |
| "A175" | 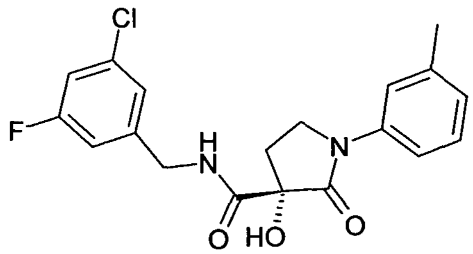 (S)-3-Hydroxy-2-oxo-1-m-tolyl-pyrrolidin-3-carbonsäure-3-chlor-5-fluor-benzylamid |
| "A176" | 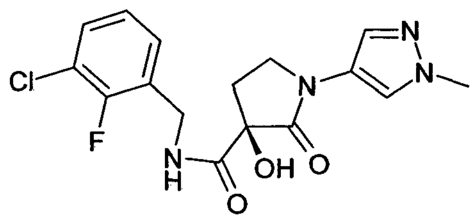 (R)-3-Hydroxy-1-(1-methyl-1H-pyrazol-4-yl)-2-oxo-pyrrolidin-3-carbonsäure-3-chlor-2-fluor-benzylamid |
| "A177" | 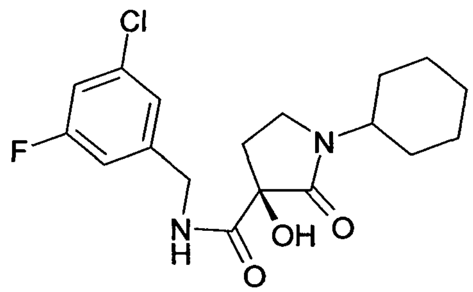 (R)-1-Cyclohexyl-3-hydroxy-2-oxo-pyrrolidin-3-carbonsäure-3-chlor-5-fluor-benzylamid |
| "A178" | 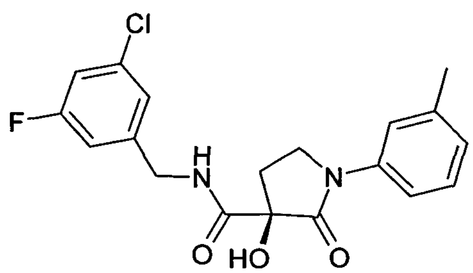 (R)-3-Hydroxy-2-oxo-1-m-tolyl-pyrrolidin-3-carbonsäure-3-chlor-5-fluor-benzylamid |

(fortgesetzt)

| Verbindung Nr. | Struktur/Name |
|---|---|
| "A179" | 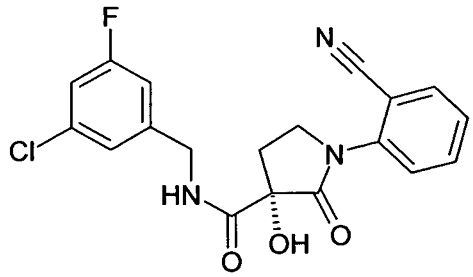 (S)-1-(2-Cyan-phenyl)-3-hydroxy-2-oxo-pyrrolidin-3-carbonsäure-3-chlor-5-fluor-benzylamid |
| "A180" | 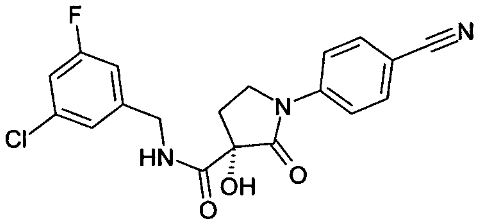 (S)-1-(4-Cyan-phenyl)-3-hydroxy-2-oxo-pyrrolidin-3-carbonsäure-3-chlor-5-fluor-benzylamid |
| "A181" | 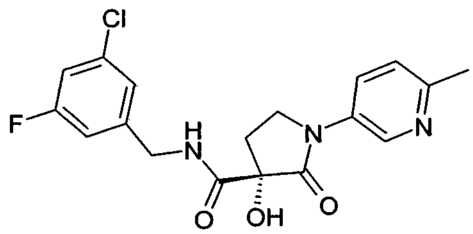 (S)-3-Hydroxy-1-(6-methyl-pyridin-3-yl)-2-oxo-pyrrolidin-3-carbonsäure-3-chlor-5-fluor-benzylamid |
| "A182" | 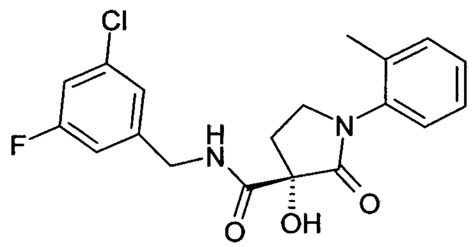 (S)-3-Hydroxy-2-oxo-1-o-tolyl-pyrrolidin-3-carbonsäure-3-chlor-5-fluor-benzylamid |
| "A183" | 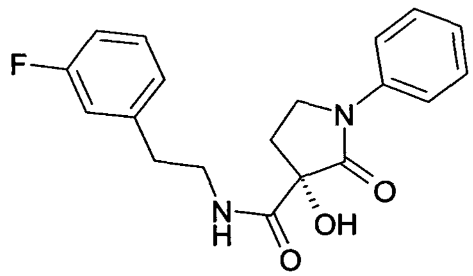 (S)-3-Hydroxy-2-oxo-1-phenyl-pyrrolidin-3-carbonsäure-[2-(3-fluor-phenyl)-ethyl]-amid |

(fortgesetzt)

| Verbindung Nr. | Struktur/Name |
|---|---|
| "A184" | 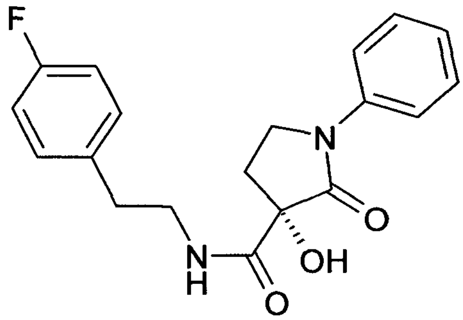<br>(S)-3-Hydroxy-2-oxo-1-phenyl-pyrrolidin-3-carbonsäure-[2-(4-fluor-phenyl)-ethyl]-amid |
| "A185" | 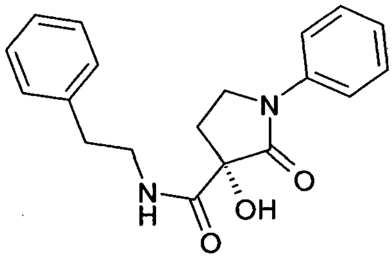<br>(S)-3-Hydroxy-2-oxo-1-phenyl-pyrrolidin-3-carbonsäure-phenethyl-amid |
| "A186" | 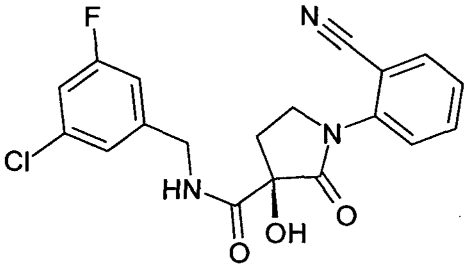<br>(R)-1-(2-Cyan-phenyl)-3-hydroxy-2-oxo-pyrrolidin-3-carbonsäure-3-chlor-5-fluor-benzylamid |
| "A187" | 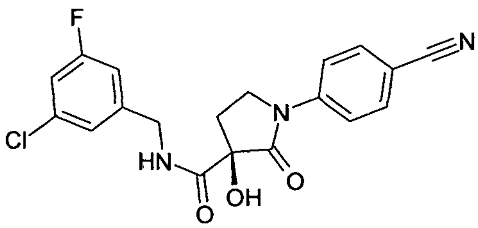<br>(R)-1-(4-Cyan-phenyl)-3-hydroxy-2-oxo-pyrrolidin-3-carbonsäure-3-chlor-5-fluor-benzylamid |
| "A188" | 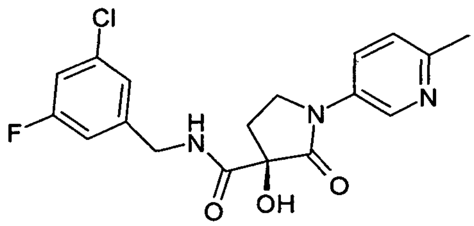<br>(R)-3-Hydroxy-1-(6-methyl-pyridin-3-yl)-2-oxo-pyrrolidin-3-carbonsäure-3-chlor-5-fluor-benzylamid |

(fortgesetzt)

| Verbindung Nr. | Struktur/Name |
|---|---|
| "A189" | 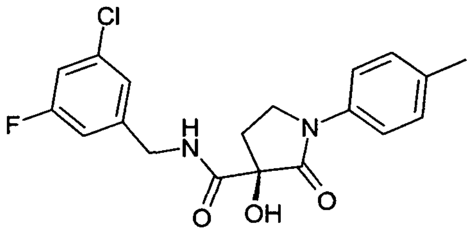 (R)-3-Hydroxy-2-oxo-1-p-tolyl-pyrrolidin-3-carbonsäure-3-chlor-5-fluor-benzylamid |
| "A190" | 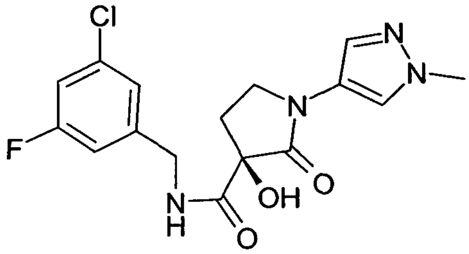 (R)-3-Hydroxy-1-(1-methyl-1 H-pyrazol-4-yl)-2-oxo-pyrrolidin-3-carbonsäure-3-chlor-5-fluor-benzylamid |
| "A191" | 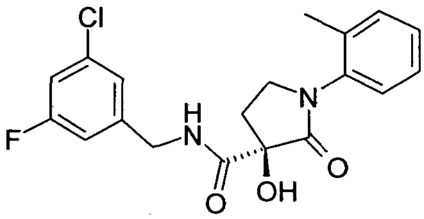 (R)-3-Hydroxy-2-oxo-1-o-tolyl-pyrrolidin-3-carbonsäure-3-chlor-5-fluor-benzylamid |
| "A192" | 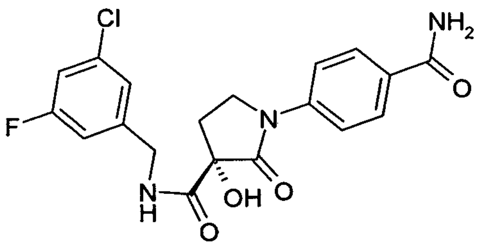 (S)-1-(4-Carbamoyl-phenyl)-3-hydroxy-2-oxo-pyrrolidin-3-carbonsäure-3-chlor-5-fluor-benzylamid |
| "A193" | 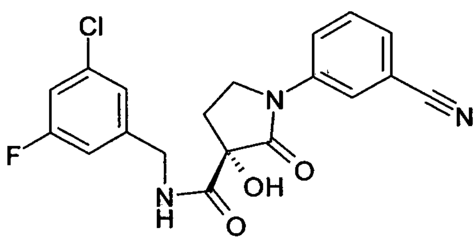 (S)-1-(3-Cyan-phenyl)-3-hydroxy-2-oxo-pyrrolidin-3-carbonsäure-3-chlor-5-fluor-benzylamid |

(fortgesetzt)

| Verbindung Nr. | Struktur/Name |
|---|---|
| "A194" | 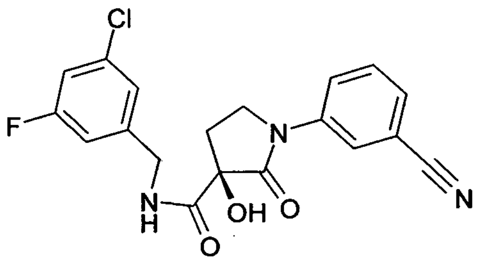 (R)-1-(3-Cyan-phenyl)-3-hydroxy-2-oxo-pyrrolidin-3-carbonsäure-3-chlor-5-fluor-benzylamid |
| "A195" | 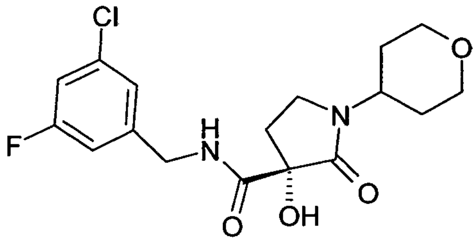 (S)-3-Hydroxy-2-oxo-1-(tetrahydro-pyran-4-yl)-pyrrolidin-3-carbonsäure-3-chlor-5-fluor-benzylamid |
| "A196" | 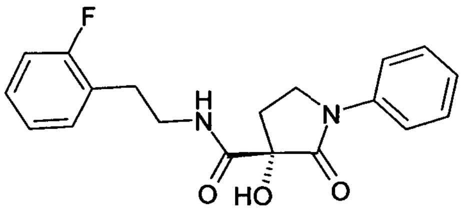 (S)-3-Hydroxy-2-oxo-1-phenyl-pyrrolidin-3-carbonsäure-[2-(2-fluor-phenyl)-ethyl]-amid |
| "A197" | 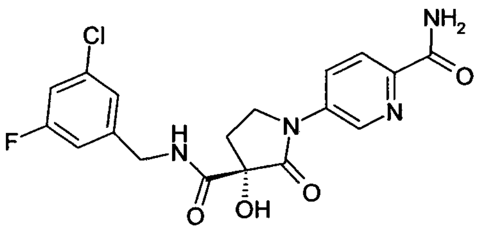 5-[(S)-3-(3-Chlor-5-fluor-benzylcarbamoyl)-3-hydroxy-2-oxo-pyrrolidin-1-yl]-pyridin-2-carbonsäure-amid |
| "A198" | 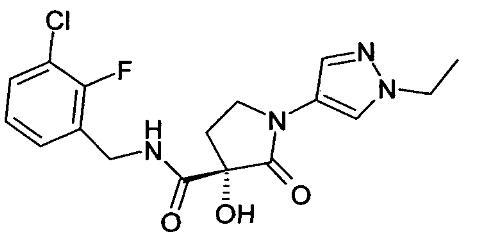 (S)-1-(1-Ethyl-1H-pyrazol-4-yl)-3-hydroxy-2-oxo-pyrrolidin-3-carbonsäure-3-chlor-2-fluor-benzylamid |

(fortgesetzt)

| Verbindung Nr. | Struktur/Name |
|---|---|
| "A199" | 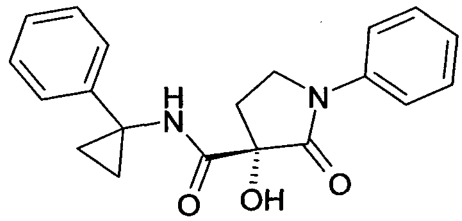<br>(S)-3-Hydroxy-2-oxo-1-phenyl-pyrrolidin-3-carbonsäure-(1-phenyl-cyclopropyl)-amid |
| "A200" | 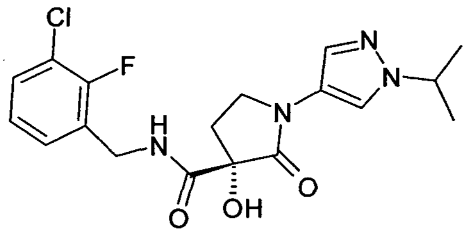<br>(S)-3-Hydroxy-1-(1-isopropyl-1H-pyrazol-4-yl)-2-oxo-pyrrolidin-3-carbonsäure-3-chlor-2-fluor-benzylamid |
| "A201" | 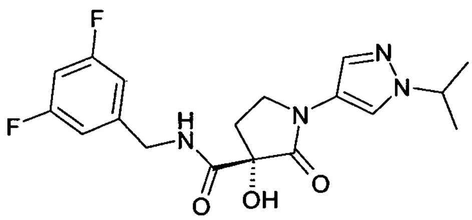<br>(S)-3-Hydroxy-1-(1-isopropyl-1 H-pyrazol-4-yl)-2-oxo-pyrrolidin-3-carbonsäure-3,5-difluor-benzylamid |
| "A202" | 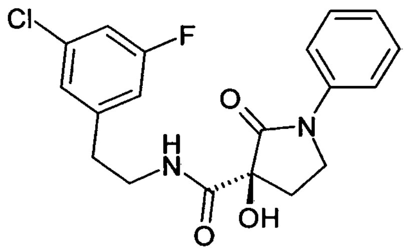<br>(S)-3-Hydroxy-2-oxo-1-phenyl-pyrrolidin-3-carbonsäure-[2-(3-chlor-5-fluor-phenyl)-ethyl]-amid |
| "A203" | 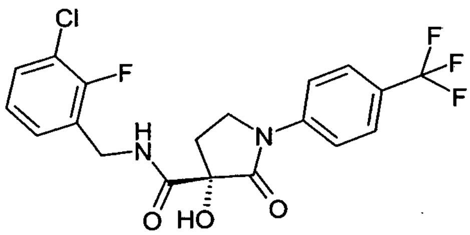<br>(S)-3-Hydroxy-2-oxo-1-(4-trifluoromethyl-phenyl)-pyrrolidin-3-carbonsäure-3-chlor-2-fluor-benzylamid |

(fortgesetzt)

| Verbindung Nr. | Struktur/Name |
|---|---|
| "A204" | 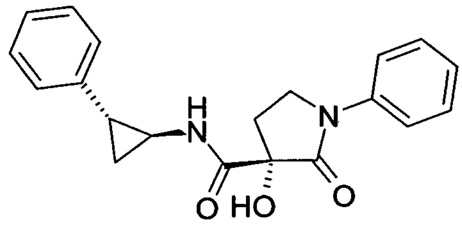 (S)-3-Hydroxy-2-oxo-1-phenyl-pyrrolidin-3-carbonsäure-((1S,2R)-2-phenyl-cyclopropyl)-amid |
| "A205" | 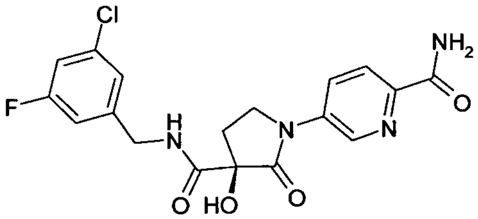 5-[(R)-3-(3-Chlor-5-fluor-benzylcarbamoyl)-3-hydroxy-2-oxo-pyrrolidin-1-yl]-pyridin-2-carbonsäure-amid |
| "A206" | 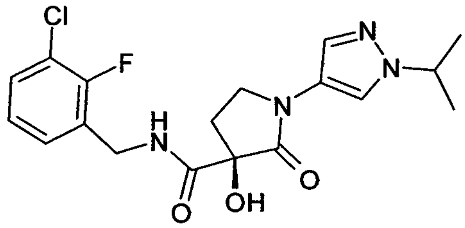 (R)-3-Hydroxy-1-(1-isopropyl-1H-pyrazol-4-yl)-2-oxo-pyrrolidin-3-carbonsäure-3-chlor-2-fluor-benzylamid |
| "A207" | 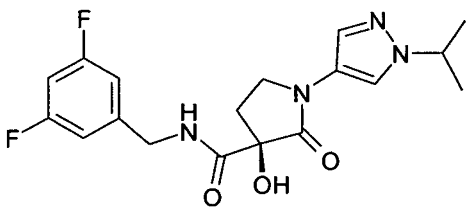 (R)-3-Hydroxy-1-(1-isopropyl-1H-pyrazol-4-yl)-2-oxo-pyrrolidin-3-carbonsäure-3,5-difluor-benzylamid |
| "A208" | 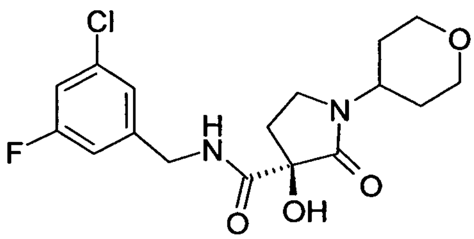 (R)-3-Hydroxy-2-oxo-1-(tetrahydro-pyran-4-yl)-pyrrolidin-3-carbonsäure- 3-chlor-5-fluor-benzylamid |

(fortgesetzt)

| Verbindung Nr. | Struktur/Name |
|---|---|
| "A209" | 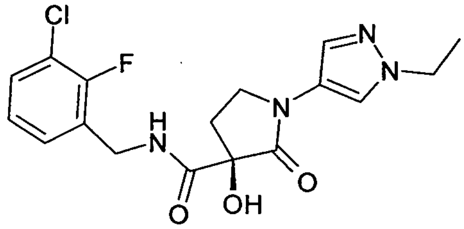 (R)-1-(1-Ethyl-1H-pyrazol-4-yl)-3-hydroxy-2-oxo-pyrrolidin-3-carbonsäure-3-chlor-2-fluor-benzylamid |
| "A210" | 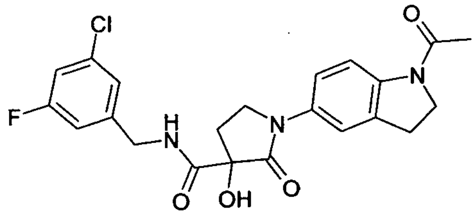 1-(1-Acetyl-2,3-dihydro-1H-indol-5-yl)-3-hydroxy-2-oxo-pyrrolidin-3-carbonsäure-3-chlor-5-fluor-benzylamid |
| "A211" | 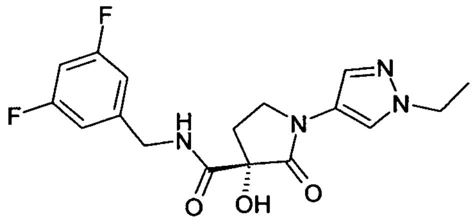 (S)-1-(1-Ethyl-1 H-pyrazol-4-yl)-3-hydroxy-2-oxo-pyrrolidin-3-carbonsäure-3,5-difluor-benzylamid |
| "A212" | 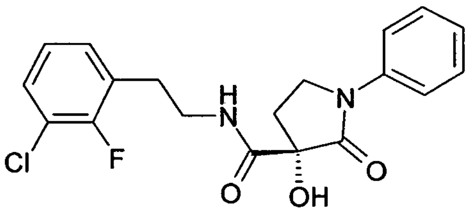 (S)-3-Hydroxy-2-oxo-1-phenyl-pyrrolidin-3-carbonsäure-[2-(3-chlor-2-fluor-phenyl)-ethyl]-amid |
| "A213" | 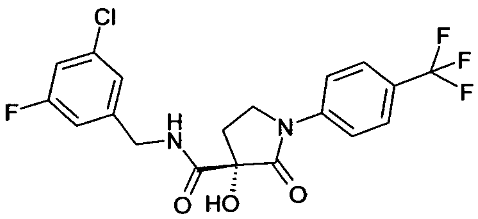 (S)-3-Hydroxy-2-oxo-1-(4-trifluormethyl-phenyl)-pyrrolidin-3-carbonsäure-3-chlor-5-fluor-benzylamid |

(fortgesetzt)

| Verbindung Nr. | Struktur/Name |
|---|---|
| "A214" | 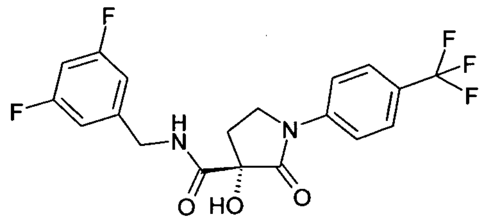 (S)-3-Hydroxy-2-oxo-1-(4-trifluormethyl-phenyl)-pyrrolidin-3-carbonsäure-3,5-difluor-benzylamid |
| "A215" | 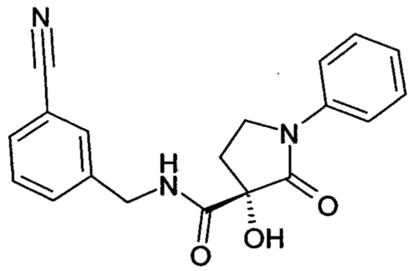 (S)-3-Hydroxy-2-oxo-1-phenyl-pyrrolidin-3-carbonsäure-3-cyan-benzylamid |
| "A216" | 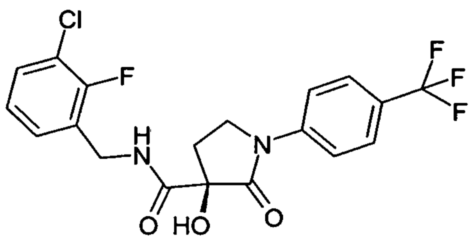 (R)-3-Hydroxy-2-oxo-1-(4-trifluormethyl-phenyl)-pyrrolidin-3-carbonsäure-3-chlor-2-fluor-benzylamid |
| "A217" | 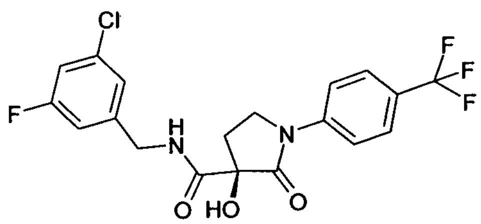 (R)-3-Hydroxy-2-oxo-1-(4-trifluormethyl-phenyl)-pyrrolidin-3-carbonsäure-3-chlor-5-fluor-benzylamid |
| "A218" | 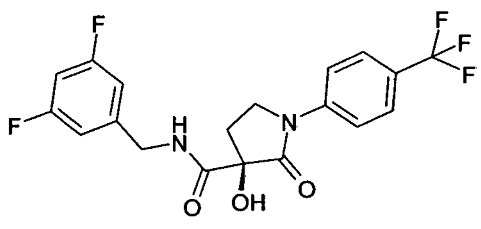 (R)-3-Hydroxy-2-oxo-1-(4-trifluormethyl-phenyl)-pyrrolidin-3-carbonsäure-3,5-difluor-benzylamid |
| "A219" | 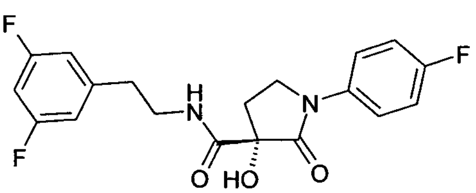 |

(fortgesetzt)

| Verbindung Nr. | Struktur/Name |
|---|---|
| | (S)-1-(4-Fluor-phenyl)-3-hydroxy-2-oxo-pyrrolidin-3-carbonsäure-[2-(3,5-difluorphenyl)-ethyl]-amid |
| "A220" | 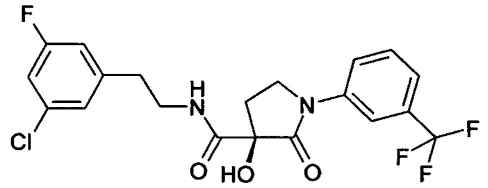 (R)-3-Hydroxy-2-oxo-1-(3-trifluormethyl-phenyl)-pyrrolidin-3-carbonsäure-[2-(3-chlor-5-fluor-phenyl)-ethyl]-amid |
| "A221" | 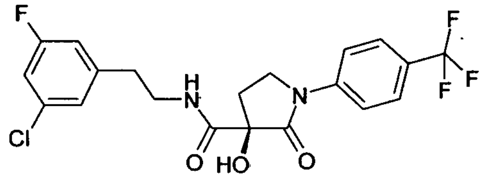 (R)-3-Hydroxy-2-oxo-1-(4-trifluormethyl-phenyl)-pyrrolidin-3-carbonsäure-[2-(3-chlor-5-fluor-phenyl)-ethyl]-amid |
| "A222" | 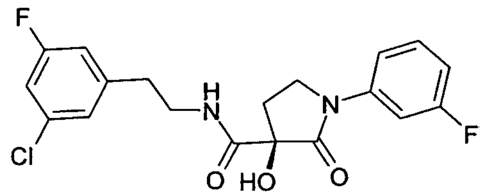 (R)-1-(3-Fluor-phenyl)-3-hydroxy-2-oxo-pyrrolidin-3-carbonsäure-[2-(3-chlor-5-fluor-phenyl)-ethyl]-amid |
| "A223" | 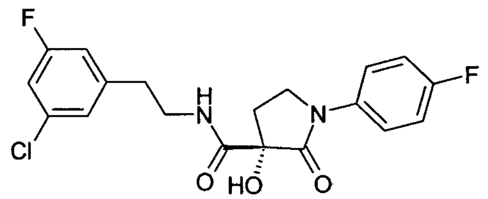 (S)-1-(4-Fluor-phenyl)-3-hydroxy-2-oxo-pyrrolidin-3-carbonsäure-[2-(3-chlor-5-fluor-phenyl)-ethyl]-amid |
| "A224" | 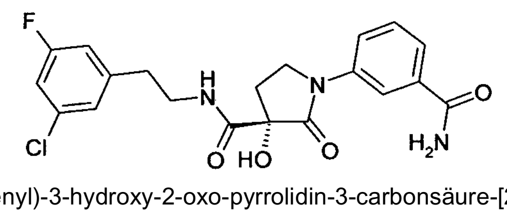 (S)-1-(3-Carbamoyl-phenyl)-3-hydroxy-2-oxo-pyrrolidin-3-carbonsäure-[2-(3-chlor-5-fluor-phenyl)-ethyl]-amid |
| "A225" | 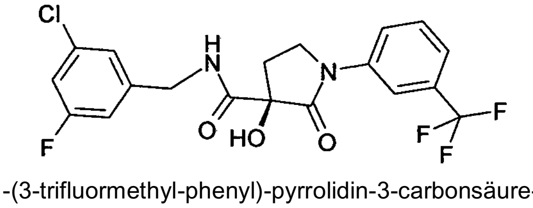 (R)-3-Hydroxy-2-oxo-1-(3-trifluormethyl-phenyl)-pyrrolidin-3-carbonsäure-3-chlor-5-fluor-benzylamid |

(fortgesetzt)

| Verbindung Nr. | Struktur/Name |
|---|---|
| "A226" | 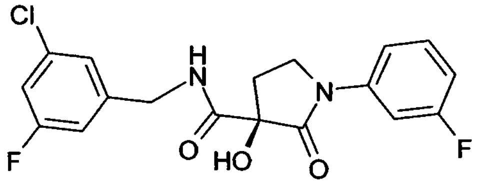 (R)-1-(3-Fluor-phenyl)-3-hydroxy-2-oxo-pyrrolidin-3-carbonsäure-3-chlor-5-fluor-benzylamid |
| "A227" | 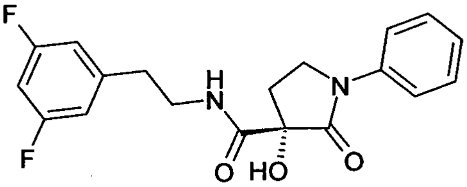 (S)-3-Hydroxy-2-oxo-1-phenyl-pyrrolidin-3-carbonsäure-[2-(3,5-difluor-phenyl)-ethyl]-amid |
| "A228" | 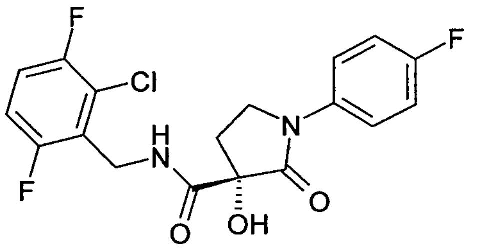 (S)-1-(4-Fluor-phenyl)-3-hydroxy-2-oxo-pyrrolidin-3-carbonsäure-2-chlor-3,6-difluor-benzylamid |
| "A229" | 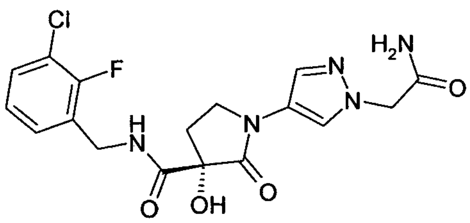 (S)-1-(1-Carbamoylmethyl-1 H-pyrazol-4-yl)-3-hydroxy-2-oxo-pyrrolidin-3-carbonsäure-3-chlor-2-fluor-benzylamid |
| "A230" | 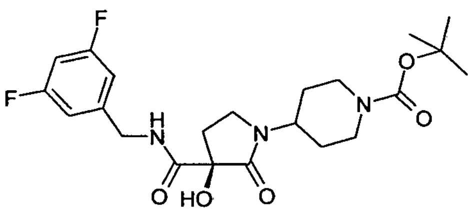 4-[(R)-3-(3,5-Difluor-benzylcarbamoyl)-3-hydroxy-2-oxo-pyrrolidin-1-yl]-piperidin-1-carbonsäure-tert.-butylester |
| "A231" | 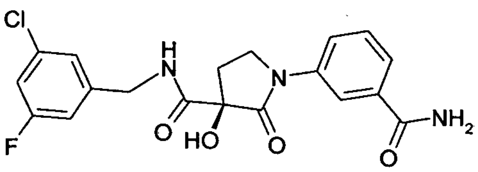 (R)-1-(3-Carbamoyl-phenyl)-3-hydroxy-2-oxo-pyrrolidin-3-carbonsäure-3-chlor-5-fluor-benzylamid |

(fortgesetzt)

| Verbindung Nr. | Struktur/Name |
|---|---|
| "A232" | 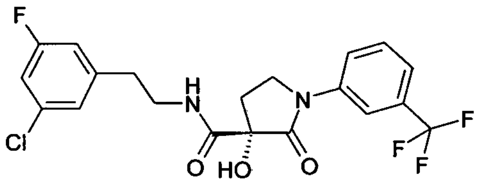 (S)-3-Hydroxy-2-oxo-1-(3-trifluormethyl-phenyl)-pyrrolidin-3-carbonsäure-[2-(3-chlor-5-fluor-phenyl)-ethyl]-amid |
| "A233" | 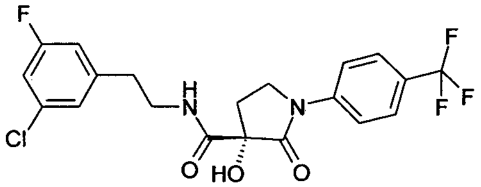 (S)-3-Hydroxy-2-oxo-1-(4-trifluormethyl-phenyl)-pyrrolidin-3-carbonsäure-[2-(3-chlor-5-fluor-phenyl)-ethyl]-amid |
| "A234" | 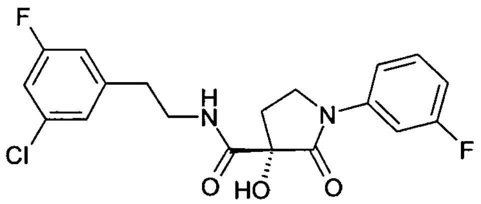 (S)-1-(3-Fluor-phenyl)-3-hydroxy-2-oxo-pyrrolidin-3-carbonsäure-[2-(3-chlor-5-fluor-phenyl)-ethyl]-amid |
| "A235" | 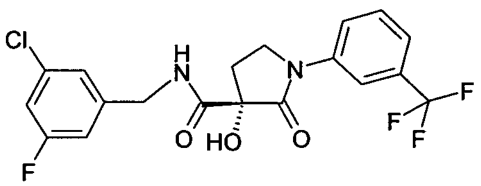 (S)-3-Hydroxy-2-oxo-1-(3-trifluormethyl-phenyl)-pyrrolidin-3-carbonsäure-3-chlor-5-fluor-benzylamid |
| "A236" | 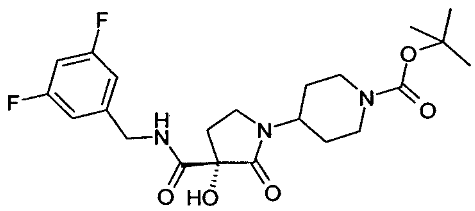 4-[(S)-3-(3,5-Difluor-benzylcarbamoyl)-3-hydroxy-2-oxo-pyrrolidin-1-yl]-piperidin-1-carbonsäure-tert.-butylester |
| "A237" | 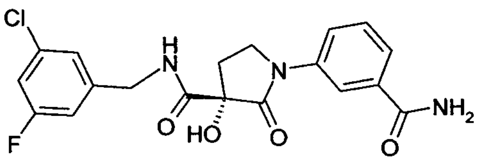 (S)-1-(3-Carbamoyl-phenyl)-3-hydroxy-2-oxo-pyrrolidin-3-carbonsäure-3-chloro-5-fluor-benzylamid |

(fortgesetzt)

| Verbindung Nr. | Struktur/Name |
|---|---|
| "A238" | 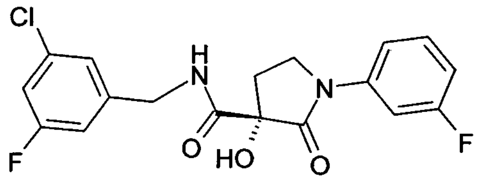 (S)-1-(3-Fluor-phenyl)-3-hydroxy-2-oxo-pyrrolidin-3-carbonsäure-3-chlor-5-fluor-benzylamid |
| "A239" | 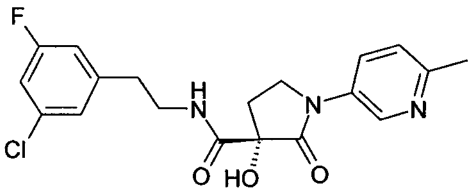 (S)-3-Hydroxy-1-(6-methyl-pyridin-3-yl)-2-oxo-pyrrolidin-3-carbonsäure-[2-(3-chlor-5-fluor-phenyl)-ethyl]-amid |
| "A240" | 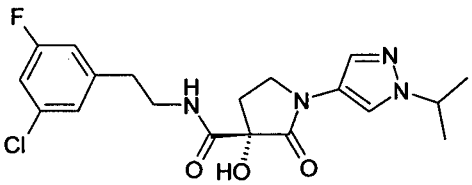 (S)-3-Hydroxy-1-(1-isopropyl-1 H-pyrazol-4-yl)-2-oxo-pyrrolidin-3-carbonsäure-[2-(3-chlor-5-fluor-phenyl)-ethyl]-amid |
| "A241" | 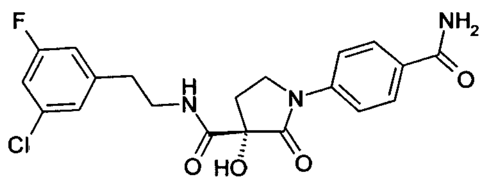 (S)-1-(4-Carbamoyl-phenyl)-3-hydroxy-2-oxo-pyrrolidin-3-carbonsäure-[2-(3-chlor-5-fluor-phenyl)-ethyl]-amid |
| "A242" | 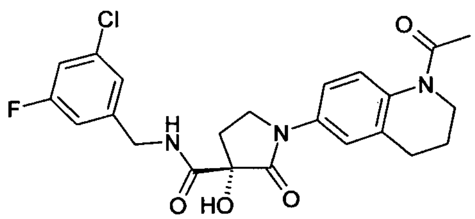 (S)-1-(1-Acetyl-1,2,3,4-tetrahydro-chinolin-6-yl)-3-hydroxy-2-oxo-pyrrolidin-3-carbonsäure-3-chlor-5-fluor-benzylamid |
| "A243" | 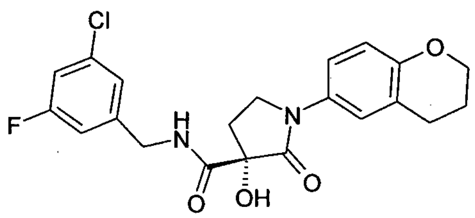 (S)-1-Chroman-6-yl-3-hydroxy-2-oxo-pyrrolidin-3-carbonsäure-3-chlor-5-fluor-benzylamid |

(fortgesetzt)

| Verbindung Nr. | Struktur/Name |
|---|---|
| "A244" | 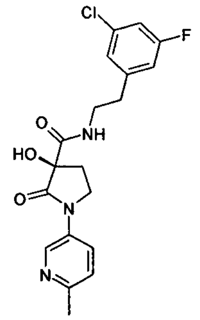 (R)-3-Hydroxy-1-(6-methyl-pyridin-3-yl)-2-oxo-pyrrolidin-3-carbonsäure-[2-(3-chlor-5-fluor-phenyl)-ethyl]-amid |
| "A245" | 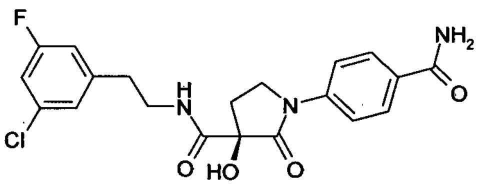 (R)-1-(4-Carbamoyl-phenyl)-3-hydroxy-2-oxo-pyrrolidin-3-carbonsäure-[2-(3-chlor-5-fluor-phenyl)-ethyl]-amid |
| "A246" | 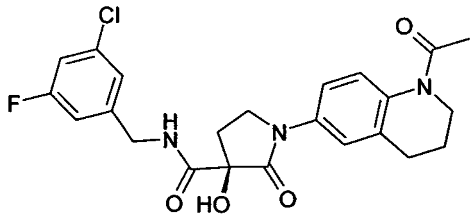 (R)-1-(1-Acetyl-1,2,3,4-tetrahydro-chinolin-6-yl)-3-hydroxy-2-oxo-pyrrolidin-3-carbonsäure-3-chlor-5-fluor-benzylamid |
| "A247" | 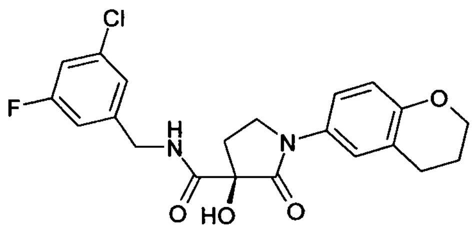 (R)-1-Chroman-6-yl-3-hydroxy-2-oxo-pyrrolidin-3-carbonsäure-3-chlor-5-fluor-benzylamid |
| "A248" | 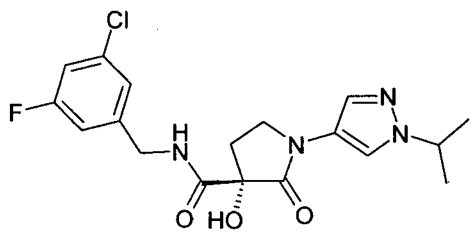 (S)-3-Hydroxy-1-(1-isopropyl-1H-pyrazol-4-yl)-2-oxo-pyrrolidin-3-carbonsäure-3-chlor-5-fluor-benzylamid |

(fortgesetzt)

| Verbindung Nr. | Struktur/Name |
|---|---|
| "A249" | 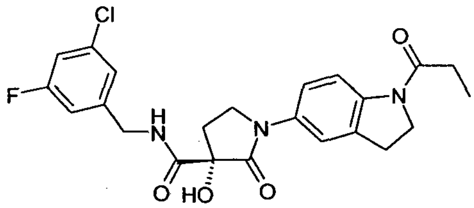 (S)-3-Hydroxy-2-oxo-1-(1-propionyl-2,3-dihydro-1H-indol-5-yl)-pyrrolidin-3-carbonsäure-3-chlor-5-fluor-benzylamid |
| "A250" | 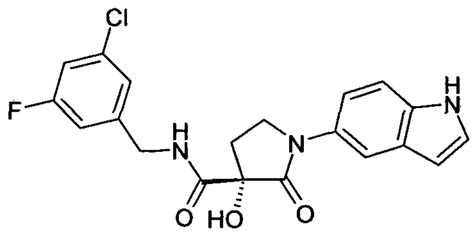 (S)-3-Hydroxy-1-(1H-indol-5-yl)-2-oxo-pyrrolidin-3-carbonsäure-3-chlor-5-fluor-benzylamid |
| "A251" | 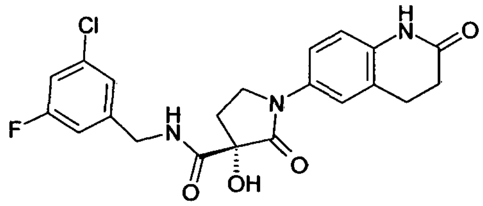 (S)-3-Hydroxy-2-oxo-1-(2-oxo-1,2,3,4-tetrahydro-chinolin-6-yl)-pyrrolidin-3-carbonsäure-3-chlor-5-fluor-benzylamid |
| "A252" | 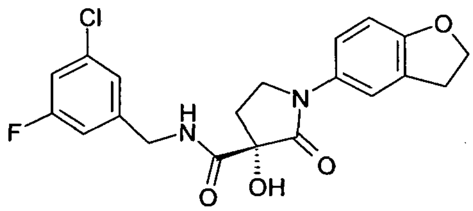 (S)-1-(2,3-Dihydro-benzofuran-5-yl)-3-hydroxy-2-oxo-pyrrolidin-3-carbonsäure-3-chlor-5-fluor-benzylamid |
| "A253" | 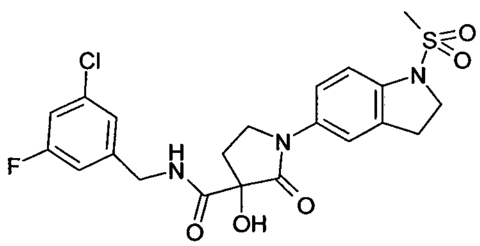 3-Hydroxy-1-(1-methansulfonyl-2,3-dihydro-1H-indol-5-yl)-2-oxo-pyrrolidin-3-carbonsäure-3-chlor-5-fluor-benzylamid |

(fortgesetzt)

| Verbindung Nr. | Struktur/Name |
|---|---|
| "A254" | 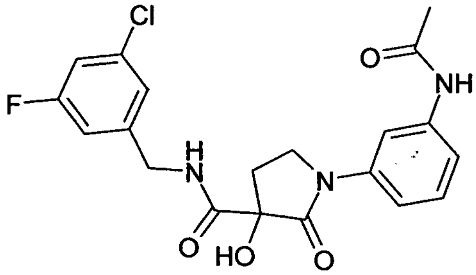<br>1-(3-Acetylamino-phenyl)-3-hydroxy-2-oxo-pyrrolidin-3-carbonsäure-3-chlor-5-fluor-benzylamid |
| "A255" | 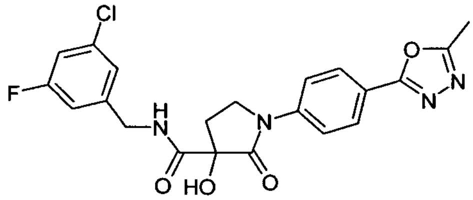<br>3-Hydroxy-1-[4-(5-methyl-[1,3,4]oxadiazol-2-yl)-phenyl]-2-oxo-pyrrolidin-3-carbonsäure-3-chlor-5-fluor-benzylamid |
| "A256" | 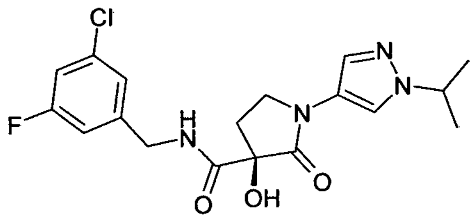<br>(R)-3-Hydroxy-1-(1-isopropyl-1 H-pyrazol-4-yl)-2-oxo-pyrrolidin-3-carbonsäure-3-chlor-5-fluor-benzylamid |
| "A257" | 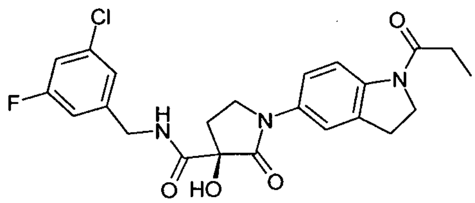 |
| | (R)-3-Hydroxy-2-oxo-1-(1-propionyl-2,3-dihydro-1H-indol-5-yl)-pyrrolidin-3-carbonsäure-3-chlor-5-fluor-benzylamid |
| "A258" | 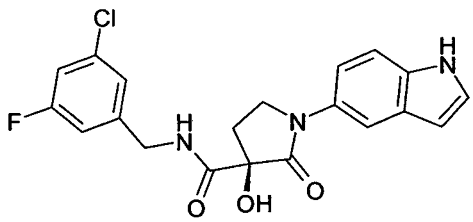<br>(R)-3-Hydroxy-1-(1H-indol-5-yl)-2-oxo-pyrrolidin-3-carbonsäure-3-chlor-5-fluor-benzylamid |

(fortgesetzt)

| Verbindung Nr. | Struktur/Name |
|---|---|
| "A259" | 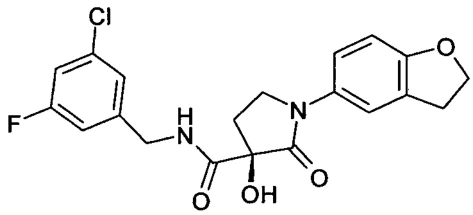<br>(R)-1-(2,3-Dihydro-benzofuran-5-yl)-3-hydroxy-2-oxo-pyrrolidin-3-carbonsäure-3-chlor-5-fluor-benzylamid |
| "A260" | 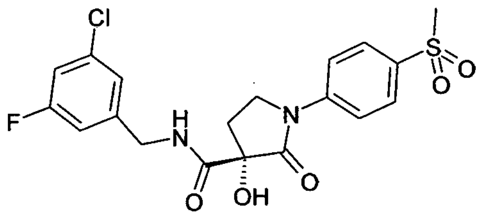<br>(S)-3-Hydroxy-1-(4-methansulfonyl-phenyl)-2-oxo-pyrrolidin-3-carbonsäure-3-chlor-5-fluor-benzylamid |
| "A261" | 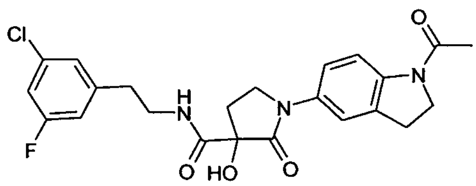<br>1-(1-Acetyl-2,3-dihydro-1H-indol-5-yl)-3-hydroxy-2-oxo-pyrrolidin-3-carbonsäure-[2-(3-chlor-5-fluor-phenyl)-ethyl]-amid |
| "A262" | 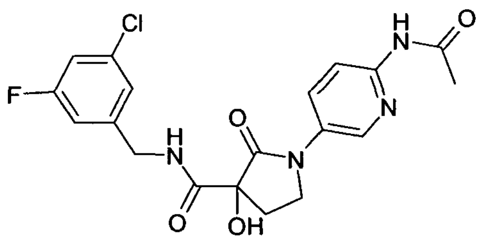<br>1-(6-Acetylamino-pyridin-3-yl)-3-hydroxy-2-oxo-pyrrolidin-3-carbonsäure-3-chlor-5-fluor-benzylamid |
| "A263" | 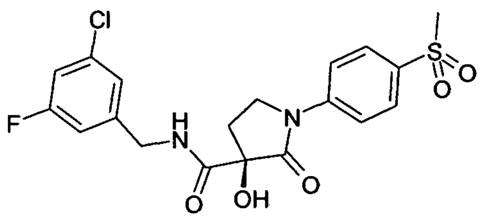<br>(R)-3-Hydroxy-1-(4-methansulfonyl-phenyl)-2-oxo-pyrrolidin-3-carbonsäure-3-chlor-5-fluor-benzylamid |
| "A264" | 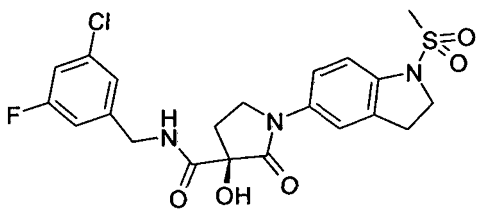 |

(fortgesetzt)

| Verbindung Nr. | Struktur/Name |
|---|---|
| | (R)-3-Hydroxy-1-(1-methansulfonyl-2,3-dihydro-1H-indol-5-yl)-2-oxo-pyrrolidin-3-carbonsäure-3-chlor-5-fluor-benzylamid |
| "A265" | 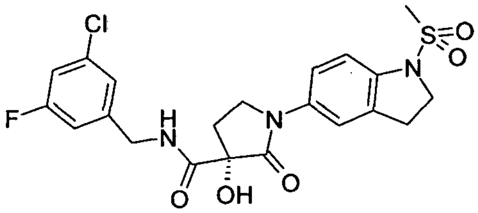 (S)-3-Hydroxy-1-(1-methansulfonyl-2,3-dihydro-1H-indol-5-yl)-2-oxo-pyrrolidin-3-carbonsäure-3-chlor-5-fluor-benzylamid |
| "A266" | 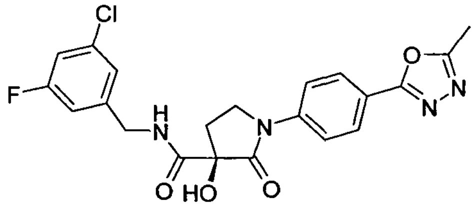 (R)-3-Hydroxy-1-[4-(5-methyl-[1,3,4]oxadiazol-2-yl)-phenyl]-2-oxo-pyrrolidin-3-carbonsäure-3-chlor-5-fluor-benzylamid |
| "A267" | 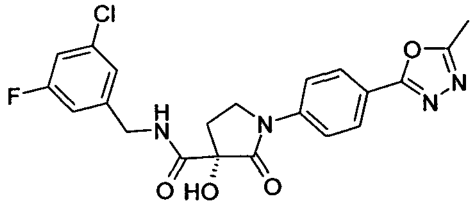 (S)-3-Hydroxy-1-(1-methansulfonyl-2,3-dihydro-1H-indol-5-yl)-2-oxo-pyrrolidin-3-carbonsäure-3-chlor-5-fluor-benzylamid |
| "A268" | 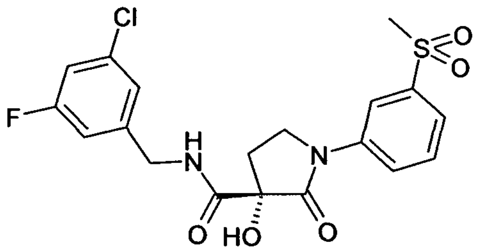 (S)-3-Hydroxy-1-(3-methansulfonyl-phenyl)-2-oxo-pyrrolidin-3-carbonsäure-3-chlor-5-fluor-benzylamid |
| "A269" | 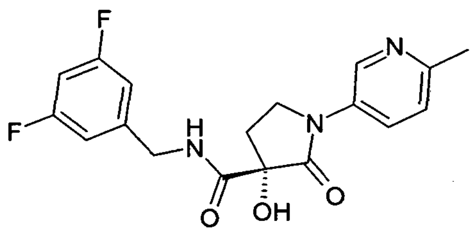 (S)-3-Hydroxy-1-(6-methyl-pyridin-3-yl)-2-oxo-pyrrolidin-3-carbonsäure-3,5-difluor-benzylamid |

(fortgesetzt)

| Verbindung Nr. | Struktur/Name |
|---|---|
| "A270" | 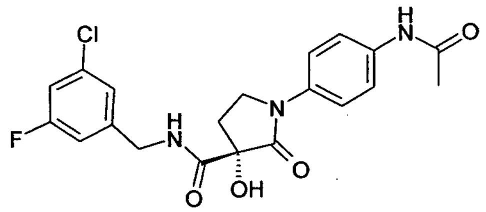 (S)-1-(4-Acetylamino-phenyl)-3-hydroxy-2-oxo-pyrrolidin-3-carbonsäure-3-chlor-5-fluor-benzylamid |
| "A271" | 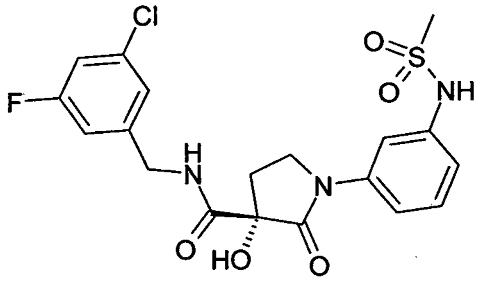 (S)-3-Hydroxy-1-(3-methansulfonylamino-phenyl)-2-oxo-pyrrolidin-3-carbonsäure-3-chlor-5-fluor-benzylamid |
| "A272" | 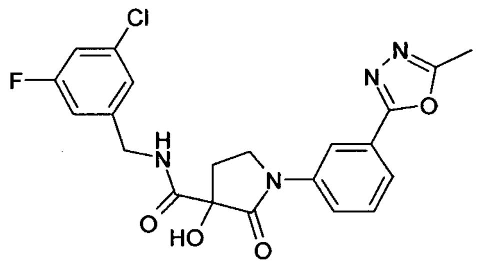 3-Hydroxy-1-[3-(5-methyl-[1,3,4]oxadiazol-2-yl)-phenyl]-2-oxo-pyrrolidin-3-carbonsäure-3-chlor-5-fluor-benzylamid |
| "A273" | 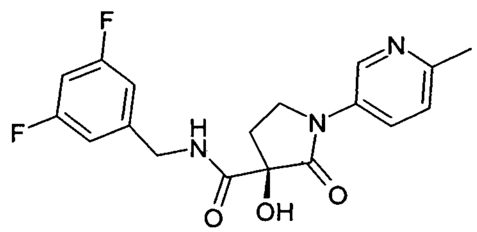 (R)-3-Hydroxy-1-(6-methyl-pyridin-3-yl)-2-oxo-pyrrolidin-3-carbonsäure-3,5-difluor-benzylamid |
| "A274" | 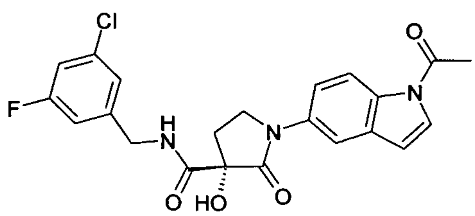 (S)-1-(1-Acetyl-1H-indol-5-yl)-3-hydroxy-2-oxo-pyrrolidin-3-carbonsäure-3-chlor-5-fluor-benzylamid |

(fortgesetzt)

| Verbindung Nr. | Struktur/Name |
|---|---|
| "A275" | 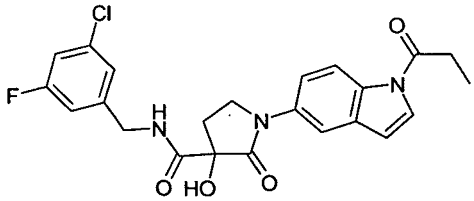 3-Hydroxy-2-oxo-1-(1-propionyl-1H-indol-5-yl)-pyrrolidin-3-carbonsäure-3-chlor-5-fluor-benzylamid |
| "A276" | 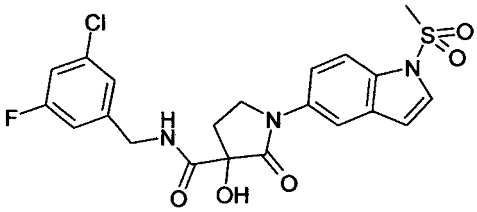 3-Hydroxy-1-(1-methansulfonyl-1H-indol-5-yl)-2-oxo-pyrrolidin-3-carbonsäure-3-chlor-5-fluor-benzylamid |
| "A277" | 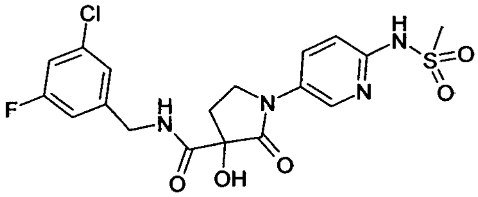 3-Hydroxy-1-(6-methansulfonylamino-pyridin-3-yl)-2-oxo-pyrrolidin-3-carbonsäure-3-chlor-5-fluor-benzylamid |
| "A278" | 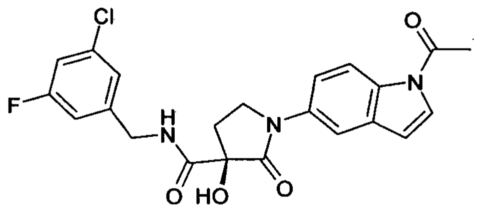 (R)-1-(1-Acetyl-1H-indol-5-yl)-3-hydroxy-2-oxo-pyrrolidin-3-carbonsäure-3-chlor-5-fluor-benzylamid |
| "A279" | 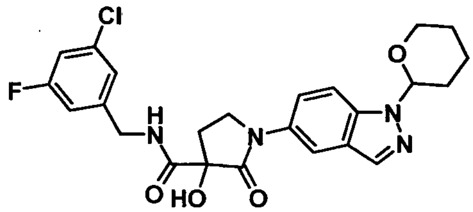 3-Hydroxy-2-oxo-1-[1-(tetrahydro-pyran-2-yl)-1 H-indazol-5-yl]-pyrrolidin-3-carbonsäure-3-chlor-5-fluor-benzylamid |
| "A280" | 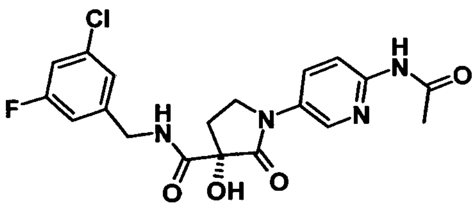 (S)-1-(6-Acetylamino-pyridin-3-yl)-3-hydroxy-2-oxo-pyrrolidin-3-carbonsäure-3-chlor-5-fluor-benzylamid |

(fortgesetzt)

| Verbindung Nr. | Struktur/Name |
|---|---|
| "A281" | 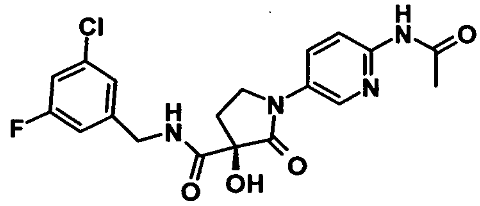 (R)-1-(6-Acetylamino-pyridin-3-yl)-3-hydroxy-2-oxo-pyrrolidin-3-carbonsäure-3-chlor-5-fluor-benzylamid |
| "A282" | 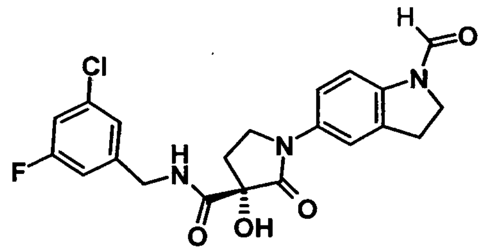 (S)-1-(1-Formyl-2,3-dihydro-1H-indol-5-yl)-3-hydroxy-2-oxo-pyrrolidin-3-carbonsäure-3-chlor-5-fluor-benzylamid |
| "A283" | 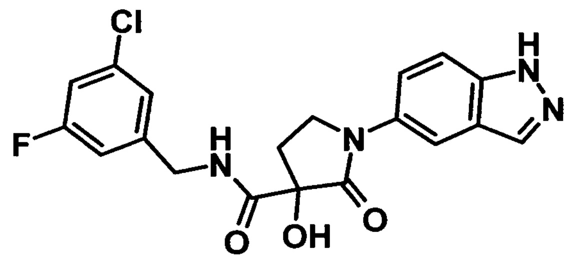 3-Hydroxy-1-(1H-indazol-5-yl)-2-oxo-pyrrolidin-3-carbonsäure-3-chlor-5-fluor-benzylamid |
| "A284" | 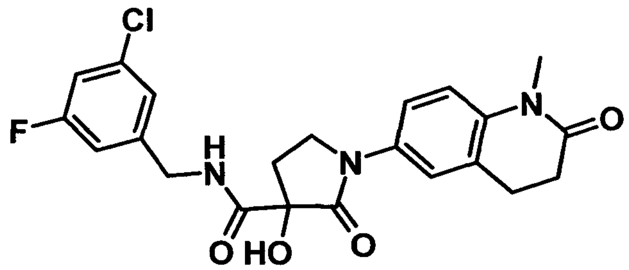 3-Hydroxy-1-(1-methyl-2-oxo-1,2,3,4-tetrahydro-chinolin-6-yl)-2-oxo-pyrrolidin-3-carbonsäure-3-chlor-5-fluor-benzylamid |
| "A285" | 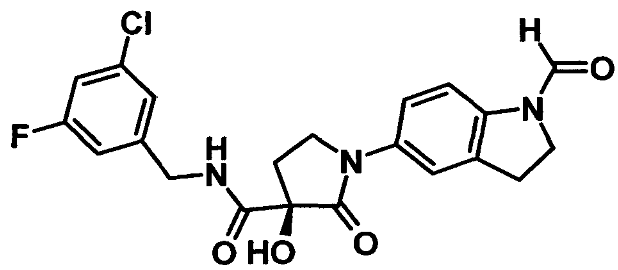 (R)-1-(1-Formyl-2,3-dihydro-1H-indol-5-yl)-3-hydroxy-2-oxo-pyrrolidin-3-carbonsäure-3-chlor-5-fluor-benzylamid |

(fortgesetzt)

| Verbindung Nr. | Struktur/Name |
|---|---|
| "A286" | 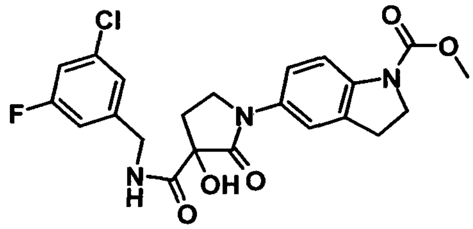<br>5-[3-(3-Chlor-5-fluor-benzylcarbamoyl)-3-hydroxy-2-oxo-pyrrolidin-1-yl]-2,3-dihydro-indol-1-carbonsäuremethylester |
| "A287" | 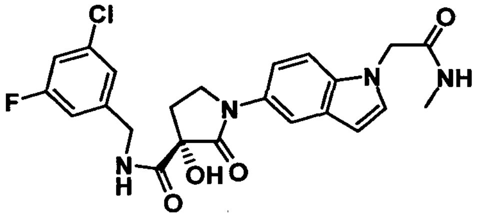<br>(S)-3-Hydroxy-1-(1-methyl-carbamoyl-methyl-1 H-indol-5-yl)-2-oxo-pyrrolidin-3-carbonsäure-3-chlor-5-fluor-benzylamid |
| "A288" | 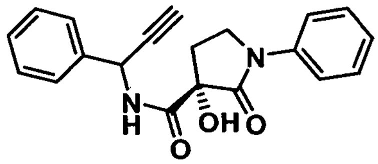<br>(S)-3-Hydroxy-2-oxo-1-phenyl-pyrrolidin-3-carbonsäure-(1-phenyl-prop-2-ynyl)-amid |
| "A289" | 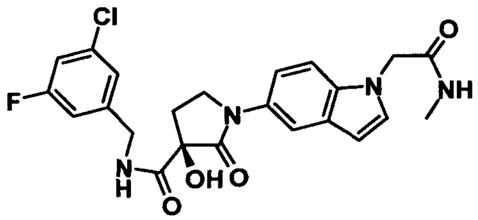<br>(R)-3-Hydroxy-1-(1-methylcarbamoylmethyl-1 H-indol-5-yl)-2-oxo-pyrrolidin-3-carbonsäure-3-chlor-5-fluor-benzylamid |
| "A290" | 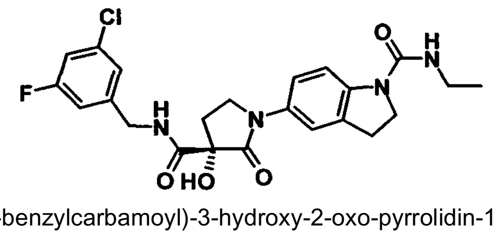<br>5-[(S)-3-(3-Chlor-5-fluor-benzylcarbamoyl)-3-hydroxy-2-oxo-pyrrolidin-1-yl]-2,3-dihydro-indol-1-carbonsäure-ethylamid |
| "A291" | 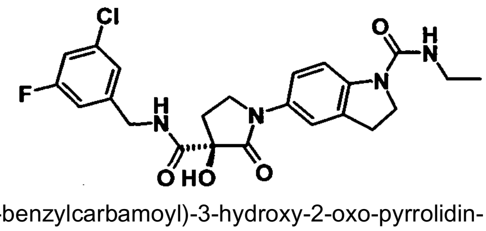<br>5-[(R)-3-(3-Chlor-5-fluor-benzylcarbamoyl)-3-hydroxy-2-oxo-pyrrolidin-1-yl]-2,3-dihydro-indol-1-carbonsäure-ethylamid |

(fortgesetzt)

| Verbindung Nr. | Struktur/Name |
|---|---|
| "A292" | 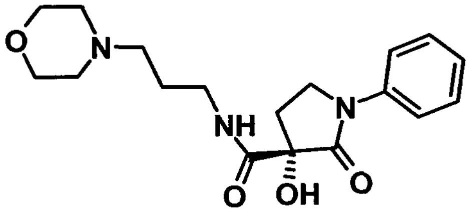 (S)-3-Hydroxy-2-oxo-1-phenyl-pyrrolidin-3-carbonsäure-(3-morpholin-4-yl-propyl)-amid |
| "A293" | 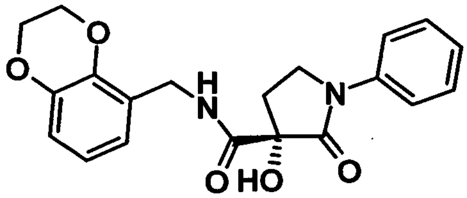 (S)-3-Hydroxy-2-oxo-1-phenyl-pyrrolidin-3-carbonsäure-(2,3-dihydro-benzo[1,4]dioxin-5-ylmethyl)-amid |
| "A294" | 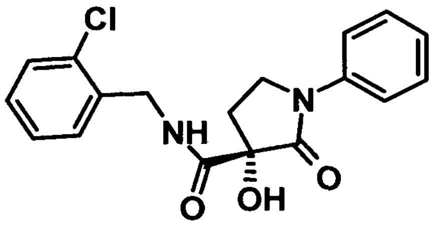 (S)-3-Hydroxy-2-oxo-1-phenyl-pyrrolidin-3-carbonsäure-2-chlor-benzylamid |
| "A295" | 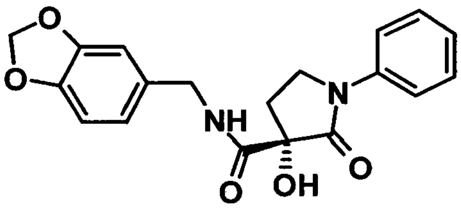 (S)-3-Hydroxy-2-oxo-1-phenyl-pyrrolidin-3-carbonsäure-(benzo[1,3]dioxol-5-ylmethyl)-amid |
| "A296" | 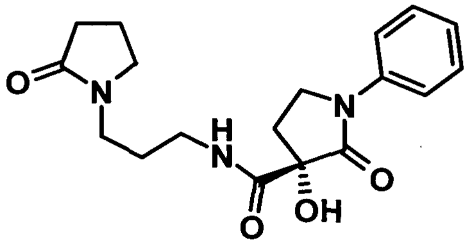 (S)-3-Hydroxy-2-oxo-1-phenyl-pyrrolidin-3-carbonsäure-[3-(2-oxo-pyrrolidin-1-yl)-propyl]-amid |
| "A297" | 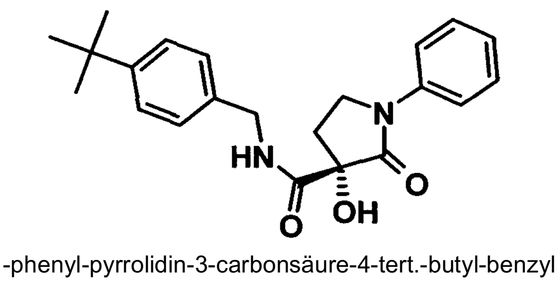 (S)-3-Hydroxy-2-oxo-1-phenyl-pyrrolidin-3-carbonsäure-4-tert.-butyl-benzylamid |

(fortgesetzt)

| Verbindung Nr. | Struktur/Name |
|---|---|
| "A298" | 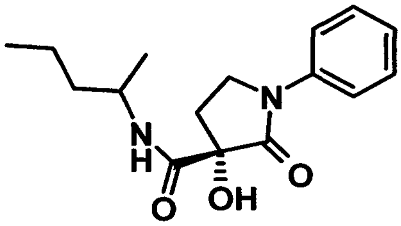 (S)-3-Hydroxy-2-oxo-1-phenyl-pyrrolidin-3-carbonsäure-(1-methyl-butyl)-amid |
| "A299" | 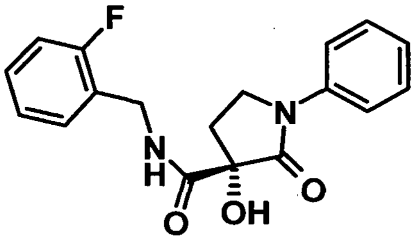 (S)-3-Hydroxy-2-oxo-1-phenyl-pyrrolidine-3-carbonsäure-2-fluor-benzylamid |
| "A300" | 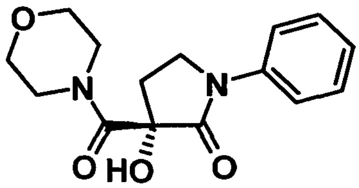 (S)-3-Hydroxy-3-(morpholin-4-carbonyl)-1-phenyl-pyrrolidin-2-on |
| "A301" | 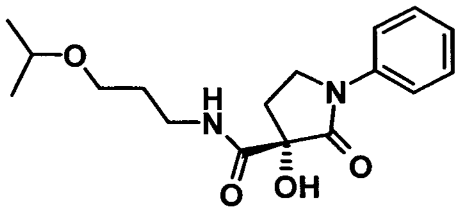 (S)-3-Hydroxy-2-oxo-1-phenyl-pyrrolidin-3-carbonsäure-(3-isopropoxy-propyl)-amid |
| "A302" | 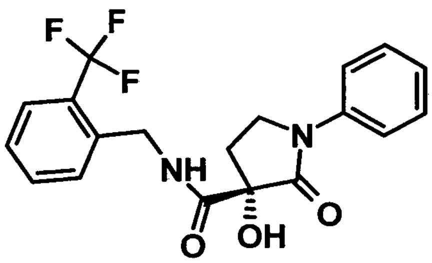 (S)-3-Hydroxy-2-oxo-1-phenyl-pyrrolidin-3-carbonsäure-2-trifluoromethyl-benzylamid |
| "A303" | 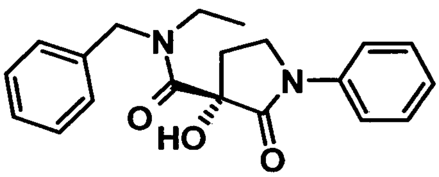 (S)-3-Hydroxy-2-oxo-1-phenyl-pyrrolidin-3-carbonsäure-benzyl-ethyl-amid |

(fortgesetzt)

| Verbindung Nr. | Struktur/Name |
|---|---|
| "A304" | 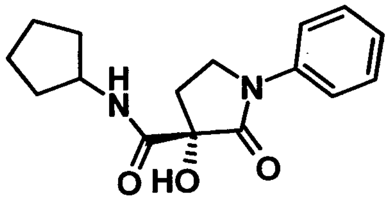 (S)-3-Hydroxy-2-oxo-1-phenyl-pyrrolidine-3-carbonsäure-cyclopentylamid |
| "A305" | 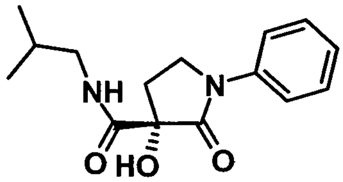 (S)-3-Hydroxy-2-oxo-1-phenyl-pyrrolidine-3-carboxylic acid isobutyl-amid |
| "A306" | 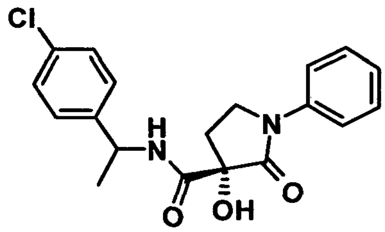 (S)-3-Hydroxy-2-oxo-1-phenyl-pyrrolidine-3-carboxylic acid [1-(4-chloro-phenyl)-ethyl]-amid |
| "A307" | 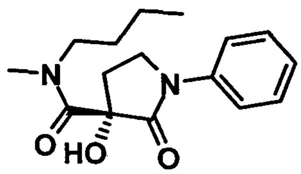 (S)-3-Hydroxy-2-oxo-1-phenyl-pyrrolidin-3-carbonsäure-butyl-methyl-amid |
| "A308" | 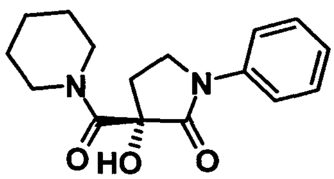 (S)-3-Hydroxy-1-phenyl-3-(piperidin-1-carbonyl)-pyrrolidin-2-on |
| "A309" | 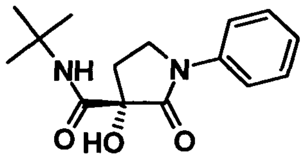 (S)-3-Hydroxy-2-oxo-1-phenyl-pyrrolidine-3-carbonsäure-tert.-butylamid |
| "A310" | 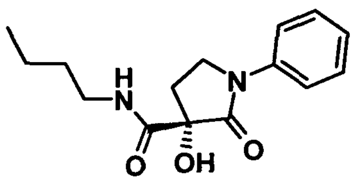 (S)-3-Hydroxy-2-oxo-1-phenyl-pyrrolidin-3-carbonsäure-butylamid |

(fortgesetzt)

| Verbindung Nr. | Struktur/Name |
|---|---|
| "A311" | 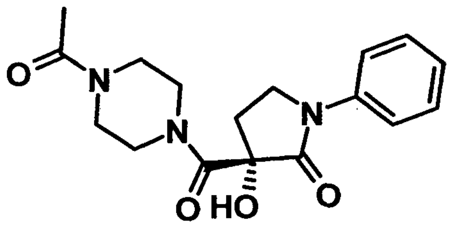 (S)-3-(4-Acetyl-piperazin-1-carbonyl)-3-hydroxy-1-phenyl-pyrrolidin-2-on |
| "A312" | 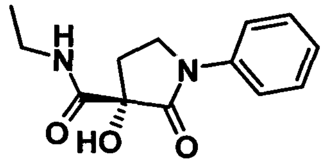 (S)-3-Hydroxy-2-oxo-1-phenyl-pyrrolidin-3-carbonsäure-ethylamid |
| "A313" | 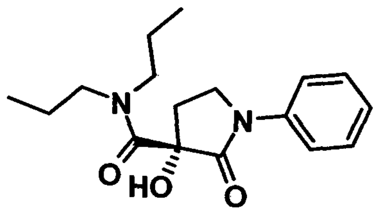 (S)-3-Hydroxy-2-oxo-1-phenyl-pyrrolidin-3-carbonsäure-dipropylamid |
| "A314" | 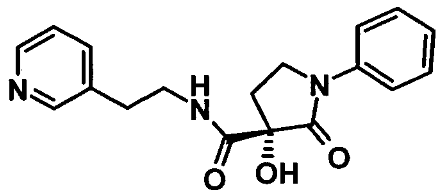 (S)-3-Hydroxy-2-oxo-1-phenyl-pyrrolidin-3-carbonsäure-(2-pyridin-3-yl-ethyl)-amid |
| "A315" | 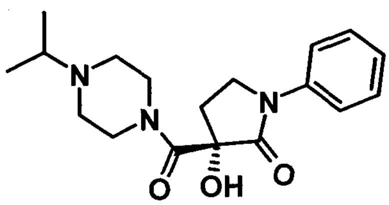 (S)-3-Hydroxy-3-(4-isopropyl-piperazin-1-carbonyl)-1-phenyl-pyrrolidin-2-on |
| "A316" | 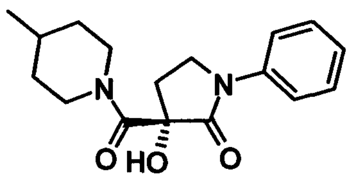 (S)-3-Hydroxy-3-(4-methyl-piperidin-1-carbonyl)-1-phenyl-pyrrolidin-2-on |
| "A317" | 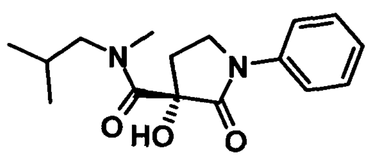 (S)-3-Hydroxy-2-oxo-1-phenyl-pyrrolidin-3-carbonsäure-isobutyl-methyl-amid |

(fortgesetzt)

| Verbindung Nr. | Struktur/Name |
|---|---|
| "A318" | 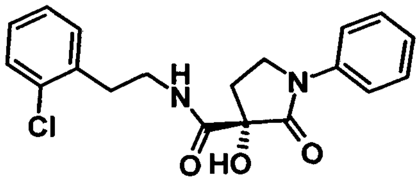 (S)-3-Hydroxy-2-oxo-1-phenyl-pyrrolidin-3-carbonsäure-[2-(2-chlor-phenyl)-ethyl]-amid |
| "A319" | 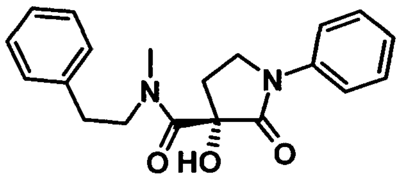 (S)-3-Hydroxy-2-oxo-1-phenyl-pyrrolidin-3-carbonsäure-methyl-phenethyl-amid |
| "A320" | 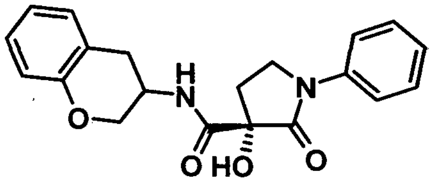 (S)-3-Hydroxy-2-oxo-1-phenyl-pyrrolidin-3-carbonsäure-chroman-3-ylamid |
| "A321" | 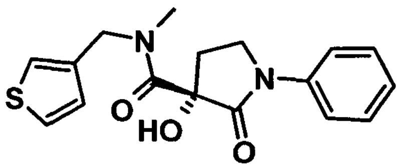 (S)-3-Hydroxy-2-oxo-1-phenyl-pyrrolidin-3-carbonsäure-methyl-thiophen-3-ylmethyl-amid |
| "A322" | 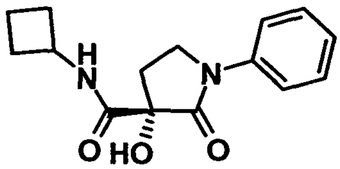 (S)-3-Hydroxy-2-oxo-1-phenyl-pyrrolidin-3-carbonsäure-cyclobutylamid |
| "A323" | 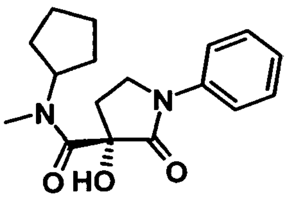 (S)-3-Hydroxy-2-oxo-1-phenyl-pyrrolidin-3-carbonsäure-cyclopentyl-methyl-amid |
| "A324" | 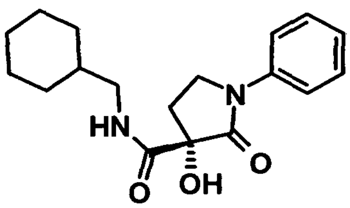 (S)-3-Hydroxy-2-oxo-1-phenyl-pyrrolidin-3-carbonsäure-cyclohexylmethyl-amid |

(fortgesetzt)

| Verbindung Nr. | Struktur/Name |
| --- | --- |
| "A325" | 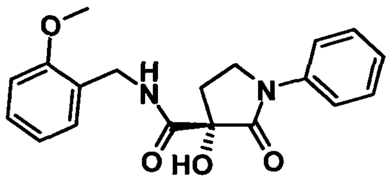 (S)-3-Hydroxy-2-oxo-1-phenyl-pyrrolidin-3-carbonsäure-2-methoxy-benzylamid |
| "A326" | 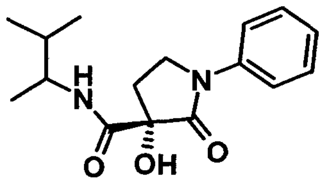 (S)-3-Hydroxy-2-oxo-1-phenyl-pyrrolidin-3-carbonsäure-(1,2-dimethyl-propyl)-amid |
| "A327" | 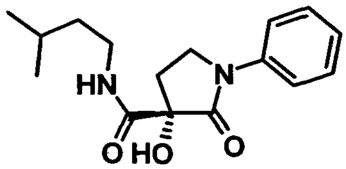 (S)-3-Hydroxy-2-oxo-1-phenyl-pyrrolidin-3-carbonsäure-(3-methyl-butyl)-amid |
| "A328" | 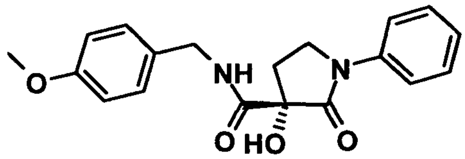 (S)-3-Hydroxy-2-oxo-1-phenyl-pyrrolidin-3-carbonsäure-4-methoxy-benzylamid |
| "A329" | 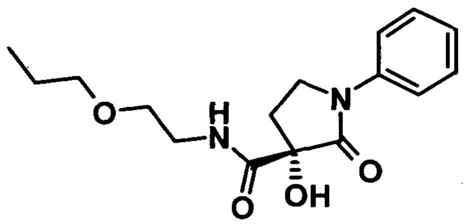 (S)-3-Hydroxy-2-oxo-1-phenyl-pyrrolidin-3-carbonsäure-(2-propoxy-ethyl)-amid |
| "A330" | 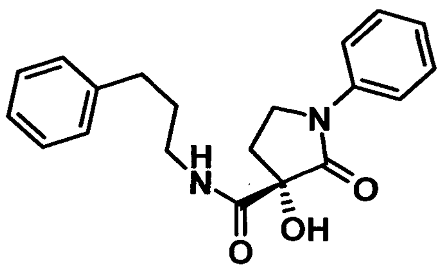 (S)-3-Hydroxy-2-oxo-1-phenyl-pyrrolidin-3-carbonsäure-(3-phenyl-propyl)-amid |

(fortgesetzt)

| Verbindung Nr. | Struktur/Name |
|---|---|
| "A331" | 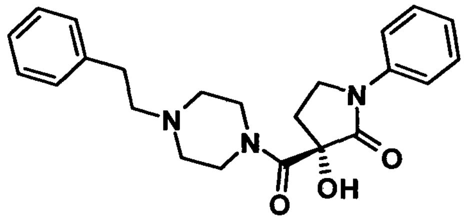 (S)-3-Hydroxy-3-(4-phenethyl-piperazin-1-carbonyl)-1-phenyl-pyrrolidin-2-on |
| "A332" | 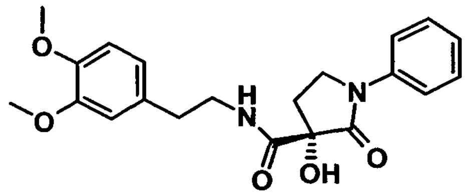 (S)-3-Hydroxy-2-oxo-1-phenyl-pyrrolidin-3-carbonsäure-[2-(3,4-dimethoxy-phenyl)-ethyl]-amid |
| "A333" | 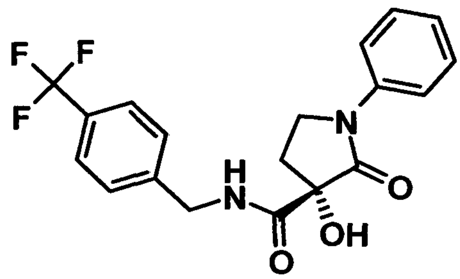 (S)-3-Hydroxy-2-oxo-1-phenyl-pyrrolidin-3-carbonsäure-4-trifluormethyl-benzylamid |
| "A334" | 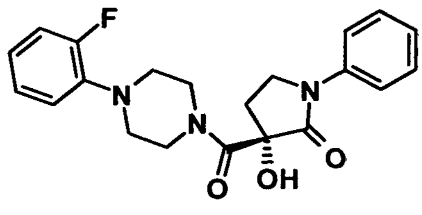 (S)-3-[4-(2-Fluor-phenyl)-piperazin-1-carbonyl]-3-hydroxy-1-phenyl-pyrrolidin-2-on |
| "A335" | 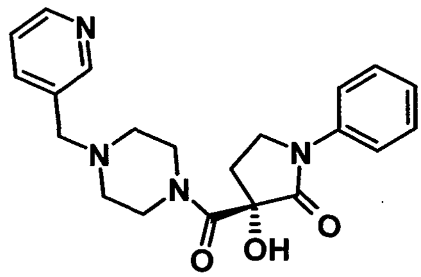 (S)-3-Hydroxy-1-phenyl-3-(4-pyridin-3-ylmethyl-piperazin-1-carbonyl)-pyrrolidin-2-on |

(fortgesetzt)

| Verbindung Nr. | Struktur/Name |
|---|---|
| "A336" | 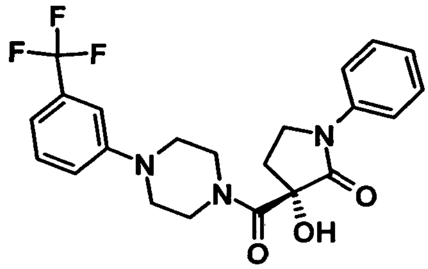 (S)-3-Hydroxy-1-phenyl-3-[4-(3-trifluoromethyl-phenyl)-piperazin-1-carbonyl]-pyrrolidin-2-on |
| "A337" | 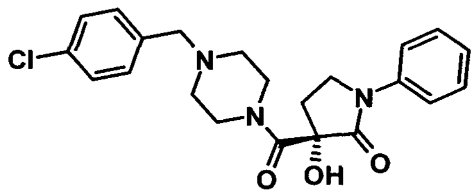 (S)-3-[4-(4-Chlor-benzyl)-piperazin-1-carbonyl]-3-hydroxy-1-phenyl-pyrrolidin-2-on |
| "A338" | 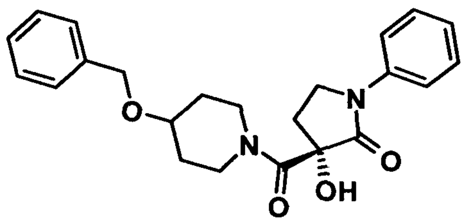 (S)-3-(4-Benzyloxy-piperidin-1-carbonyl)-3-hydroxy-1-phenyl-pyrrolidin-2-on |
| "A339" | 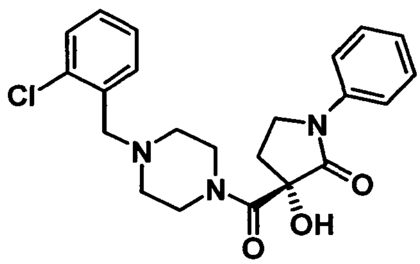 (S)-3-[4-(2-Chlor-benzyl)-piperazin-1-carbonyl]-3-hydroxy-1-phenyl-pyrrolidin-2-on |
| "A340" | 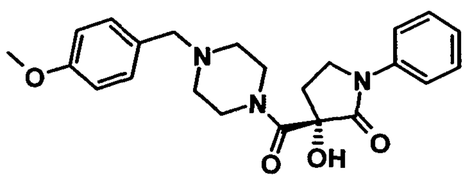 (S)-3-Hydroxy-3-[4-(4-methoxy-benzyl)-piperazin-1-carbonyl]-1-phenyl-pyrrolidin-2-on |
| "A341" | 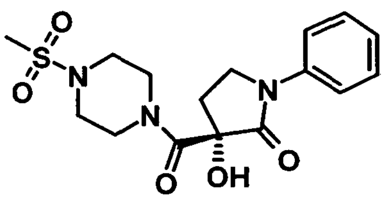 (S)-3-Hydroxy-3-(4-methansulfonyl-piperazin-1-carbonyl)-1-phenyl-pyrrolidin-2-on |

(fortgesetzt)

| Verbindung Nr. | Struktur/Name |
|---|---|
| "A342" | 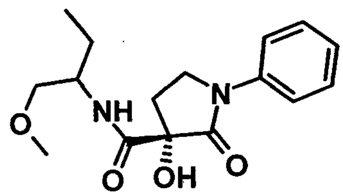 (S)-3-Hydroxy-2-oxo-1-phenyl-pyrrolidin-3-carbonsäure-(1-methoxymethyl-propyl)-amid |
| "A343" | 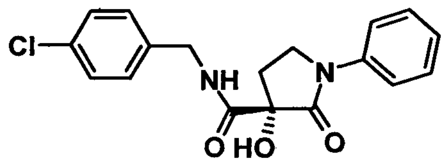 (S)-3-Hydroxy-2-oxo-1-phenyl-pyrrolidin-3-carbonsäure-4-chlor-benzylamid |
| "A344" | 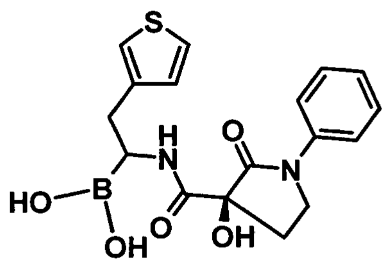 [1-[[(3S)-3-hydroxy-2-oxo-1-phenyl-pyrrolidin-3-carbonyl]amino]-2-(3-thienyl)ethyl]boronsäure |
| "A345" | 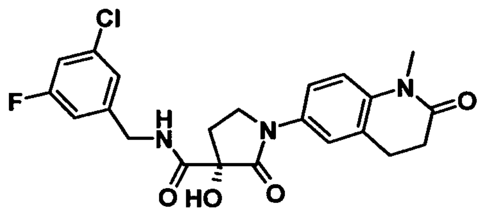 (S)-3-Hydroxy-1-(1-methyl-2-oxo-1,2,3,4-tetrahydro-chinolin-6-yl)-2-oxo-pyrrolidin-3-carbonsäure-3-chlor-5-fluor-benzylamid |
| "A346" | 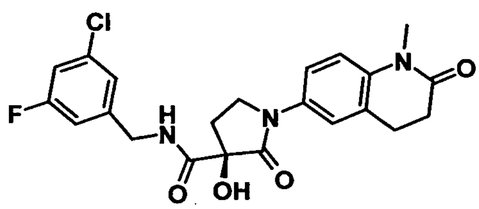 (R)-3-Hydroxy-1-(1-methyl-2-oxo-1,2,3,4-tetrahydro-chinolin-6-yl)-2-oxo-pyrrolidin-3-carbonsäure-3-chlor-5-fluor-benzylamid |

sowie ihre pharmazeutisch verwendbaren Salze, Tautomere und Stereoisomere, einschließlich deren Mischungen in allen Verhältnissen.

**3.** Verfahren zur Herstellung von Verbindungen der Formel I nach den Ansprüchen 1-2 sowie ihrer pharmazeutisch verwendbaren Salze, Tautomeren und Stereoisomeren, **dadurch gekennzeichnet, daß** man

a) eine Verbindung der Formel II

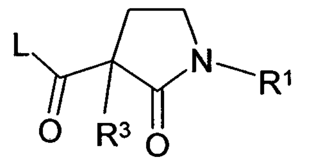

II

worin $R^1$ und $R^3$ die in Anspruch 1 angegebenen Bedeutungen haben, und L Cl, Br, I oder eine freie oder reaktionsfähig funktionell abgewandelte OH-Gruppe bedeutet,
mit einer Verbindung der Formel III

$R^2$-$NHR^4$ III

worin $R^2$ und $R^4$ die in Anspruch 1 angegebenen Bedeutungen haben,
umsetzt,
oder
b) zur Herstellung von Verbindungen der Formel I, worin $R^3$ OH bedeutet,
eine Verbindung der Formel IV

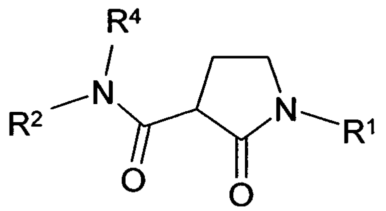

IV

worin $R^1$, $R^2$ und $R^4$ die in Anspruch 1 angegebenen Bedeutungen haben,
oxidiert,
oder
c) einen Rest $R^3$ in einen anderen Rest $R^3$ umwandelt, indem man eine OH-Gruppe gegen ein Halogenatom austauscht,
oder ein Halogenatom gegen $N_3$ austauscht,

und/oder
eine Base oder Säure der Formel I in eines ihrer Salze umwandelt.

**4.** Arzneimittel, enthaltend mindestens eine Verbindung der Formel I nach Anspruch 1-2 und/oder ihre pharmazeutisch verwendbaren Salze, Tautomeren und Stereoisomeren, einschließlich deren Mischungen in allen Verhältnissen, sowie gegebenenfalls Träger- und/oder Hilfsstoffe.

**5.** Verbindungen der Formel I gemäß Anspruch 1-2,
sowie ihrer pharmazeutisch verwendbaren Salze, Tautomeren und Stereoisomeren, einschließlich deren Mischungen in allen Verhältnissen, zur Verwendung zur Behandlung von Tumoren, Tumormetastasen, proliferativer Erkrankungen der Mesangiumzellen, Hämangiom, proliferativer Retinopathie, rheumatoider Arthritis, atherosklerotischer Neovaskularisation, Psoriasis, okularer Neovaskularisation, Osteoporose, Diabetes und Fettleibigkeit, lymphoider Leukämie, Lymphom, Malaria und Prostatahypertrophie.

**6.** Verbindungen nach Anspruch 5, wobei die Tumorerkrankung ausgewählt ist aus der Gruppe
des Plattenepithel, der Blase, des Magens, der Nieren, von Kopf und Hals, des Ösophagus, des Gebärmutterhalses, der Schilddrüse, des Darms, der Leber, des Gehirns, der Prostata, des Urogenitaltrakts, des lymphatischen Systems, des Magens, des Kehlkopfs, der Lunge, der Haut, Monozytenleukämie, Lungenadenokarzinom, kleinzelliges Lungenkarzinom, Bauchspeicheldrüsenkrebs, Glioblastom, Brustkarzinom, akute myelotische Leukämie, chronische myelotische Leukämie, akute lymphatische Leukämie, chronische lymphatische Leukämie, Hodgkin-Lymphom, non-Hodgkin-Lymphom,

**7.** Verbindungen der Formel I gemäß Anspruch 1-2 und/oder ihrer physiologisch unbedenklichen Salze zur Verwendung zur Behandlung von Tumoren, wobei eine therapeutisch wirksame Menge einer Verbindung der Formel I in Kombination mit einer Verbindung aus der Gruppe 1) Östrogenrezeptormodulator, 2) Androgenrezeptormodulator, 3) Retinoidrezeptormodulator, 4) Zytotoxikum, 5) antiproliferatives Mittel, 6) Prenyl-Proteintransferasehemmer, 7) HMG-CoA-Reduktase-Hemmer, 8) HIV-Protease-Hemmer, 9) Reverse-Transkriptase-Hemmer sowie 10) weiterer Angiogenese-Hemmer verabreicht wird.

**8.** Verbindungen der Formel I gemäß Anspruch 1-2 und/oder ihrer physiologisch unbedenklichen Salze zur Verwendung zur Behandlung von Tumoren wobei eine therapeutisch wirksame Menge einer Verbindung der Formel I in Kombination mit Radiotherapie und einer Verbindung aus der Gruppe 1) Östrogenrezeptormodulator, 2) Androgenrezeptormodulator, 3) Retinoidrezeptormodulator, 4) Zytotoxikum, 5) antiproliferatives Mittel, 6) Prenyl-Proteintransferasehemmer, 7) HMG-CoA-Reduktase-Hemmer, 8) HIV-Protease-Hemmer, 9) Reverse-Transkriptase-Hemmer sowie 10) weiterer Angiogenese-Hemmer verabreicht wird.

## Claims

**1.** Compounds of the formula I

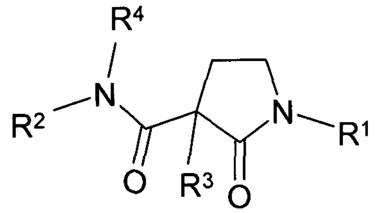

I

in which

$R^1$ denotes phenyl, benzyl, naphthyl or biphenyl, each of which is unsubstituted or mono-, di-, tri-, tetra- or pentasubstituted by Hal, CN, NHCOA, $NHSO_2A$, $SO_2A$ and/or $CONH_2$; A or $(CH_2)_nHet$,
$R^2$ denotes $[C(R^4)_2]_nAr^2$, $(CH_2)_nCyc$, $CH[B(OH)_2]CH_2Het$,

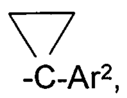

CH(C≡CH)phenyl, A or $(CH_2)_nHet$,
$R^3$ denotes OH, $N_3$, $NH_2$ or F,
$R^4$ denotes H or alkyl having 1, 2, 3 or 4 C atoms,
$R^2$ and $R^4$ together also denote alkylene having 2, 3, 4 or 5 C atoms, where one $CH_2$ group may also be replaced by NH, NA, N-COA, $N-(CH_2)_nAr^3$, $N-(CH_2)_nHet^2$, CH-A, $CH-O-(CH_2)_nAr^3$, $N-SO_2A$ or O and/or may be substituted by A,
Het denotes pyridazinyl, pyrazolyl, benzimidazolyl, pyridyl, dibenzofuranyl, carbazolyl, indolyl, dihydroindolyl, benzofuranyl, dihydrobenzofuranyl, 2,3-dihydrobenzo-1,4-dioxinyl, chromanyl, piperazinyl, morpholinyl, tetrahydropyranyl, quinolinyl, isoquinolinyl, isoindolyl, dihydroquinolinyl, dihydroisoquinolinyl, tetrahydroquinolinyl, tetrahydroiso-quinolinyl, quinazolinyl, quinoxalinyl, phthalazinyl, purinyl, naphthyridinyl, pyrimidinyl, indazolyl, furyl, thienyl, imidazolyl, pyrrolyl, oxazolyl, oxadiazolyl, isoxazolyl, thiazolyl, triazolyl, tetrazolyl, thiadiazole, benzothiazolyl, imidazo[1,2-a]-pyridinyl, 1,3-benzodioxolyl, benzoxazolyl, piperidin-1-yl, pyrrolidin-1-yl, 1,2-oxazinan-2-yl, 1,2,5-oxadiazinan-2-yl, 1,3-oxazinan-3-yl or hexahydropyrimidinyl, each of which is unsubstituted or mono-, di- or trisubstituted by A, OA, COOA, COA, CHO, $(CH_2)_nCONH_2$, $(CH_2)_nCONHA$, $(CH_2)_nCONA_2$, $SO_2A$, $NHSO_2A$, =O and/or $Het^1$,
$Het^1$ denotes pyridazinyl, pyrazolyl, pyridyl, piperazinyl, morpholinyl, pyrimidinyl, furyl, thienyl, imidazolyl, pyrrolyl, oxazolyl, isoxazolyl, thiazolyl, triazolyl, tetrazolyl, thiadiazole, piperidin-1-yl, pyrrolidin-1-yl, tetrahydropyranyl, 1,2-oxazinan-2-yl, 1,2,5-oxadiazinan-2-yl, 1,3-oxazinan-3-yl or hexahydropyrimidinyl, each of which is unsubstituted or mono-, di- or trisubstituted by A and/or OA,
$Het^2$ denotes pyridyl, pyrimidinyl, furyl, thienyl, imidazolyl, pyrrolyl, oxazolyl, oxadiazolyl, isoxazolyl, thiazolyl, triazolyl, tetrazolyl or thiadiazole,
A denotes unbranched or branched alkyl having 1-10 C atoms, in which 1-7 H atoms may be replaced by F, Cl, Br, OH, CHO, COA, COOA, CN, $CONA_2$, CONHA and/or $CONH_2$,

and/or in which one or two non-adjacent CH and/or $CH_2$ groups may be replaced by O, N and/or $NR^4$, or Cyc,
$Ar^2$ denotes phenyl which is unsubstituted or mono-, di-, tri-, tetra- or pentasubstituted by A, Hal, CN, OH and/or OA,
$Ar^3$ denotes phenyl which is unsubstituted or mono-, di- or trisubstituted by Hal, OH, OA and/or A,
Cyc denotes cyclic alkyl having 3-7 C atoms,
Hal denotes F, Cl, Br or I,
n denotes 0, 1, 2, 3 or 4,

and pharmaceutically usable salts, tautomers and stereoisomers thereof, including mixtures thereof in all ratios.

**2.** Compounds according to Claim 1, selected from the group

| Compound No. | Structure/name |
|---|---|
| "A1" | 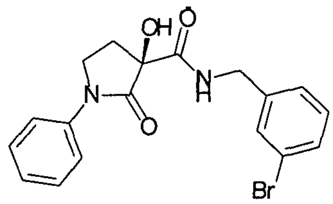 (S)-3-Hydroxy-2-oxo-1-phenylpyrrolidine-3-carboxylic acid 3-bromobenzyl amide |
| "A2" | 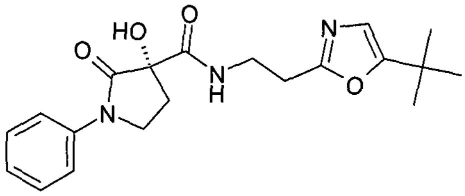 (S)-3-Hydroxy-2-oxo-1-phenylpyrrolidine-3-carboxylic acid [2-(5-tert-butyloxazol-2-yl)ethyl] amide |
| "A3" | 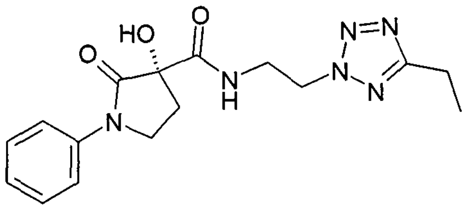 (S)-3-Hydroxy-2-oxo-1-phenylpyrrolidine-3-carboxylic acid [2-(5-ethyltetrazol-2-yl)ethyl] amide |
| "A4" | 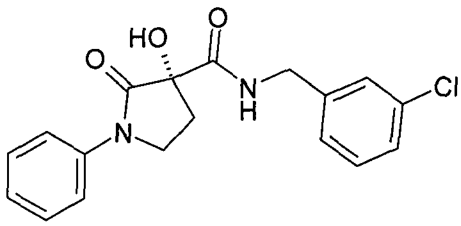 (S)-3-Hydroxy-2-oxo-1-phenylpyrrolidine-3-carboxylic acid 3-chlorobenzyl amide |
| "A5" | 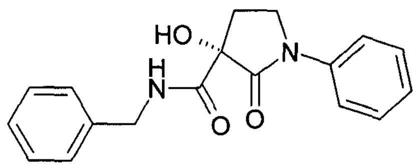 (S)-3-Hydroxy-2-oxo-1-phenylpyrrolidine-3-carboxylic acid benzyl amide |

(continued)

| Compound No. | Structure/name |
|---|---|
| "A6" | 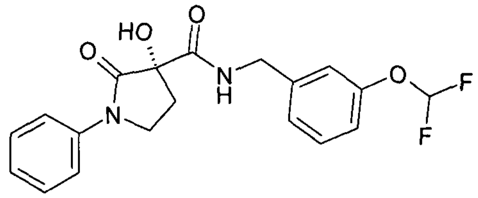 (S)-3-Hydroxy-2-oxo-1-phenylpyrrolidine-3-carboxylic acid 3-difluoromethoxybenzyl amide |
| "A7" | 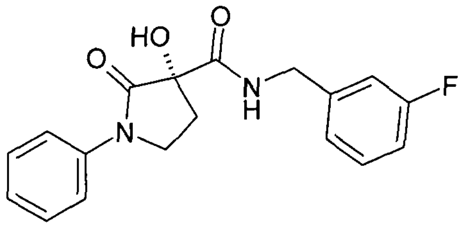 (S)-3-Hydroxy-2-oxo-1-phenylpyrrolidine-3-carboxylic acid 3-fluorobenzyl amide |
| "A8" | 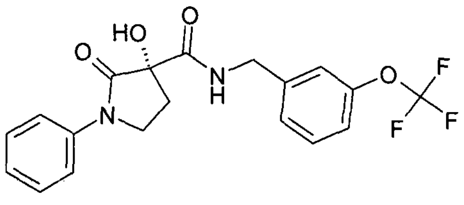 (S)-3-Hydroxy-2-oxo-1-phenylpyrrolidine-3-carboxylic acid 3-trifluoromethoxybenzyl amide |
| "A9" | 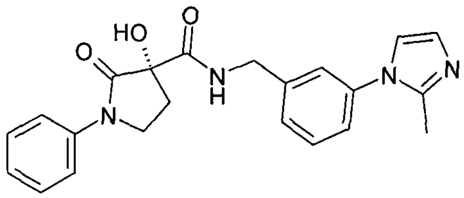 (S)-3-Hydroxy-2-oxo-1-phenylpyrrolidine-3-carboxylic acid 3-(2-methylimidazol-1-yl)benzyl amide |
| "A10" | 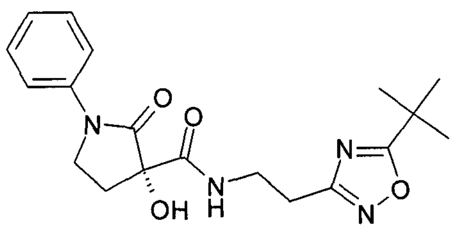 (S)-3-Hydroxy-2-oxo-1-phenylpyrrolidine-3-carboxylic acid [2-(5-tert-butyl-1,2,4-oxadiazol-3-yl)ethyl] amide |
| "A11" | 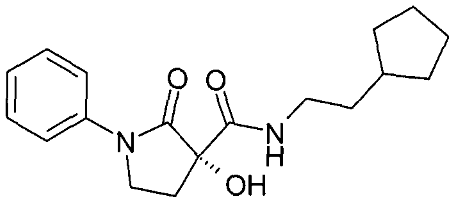 (S)-3-Hydroxy-2-oxo-1-phenylpyrrolidine-3-carboxylic acid (2-cyclopentylethyl) amide |

(continued)

| Compound No. | Structure/name |
|---|---|
| "A12" | 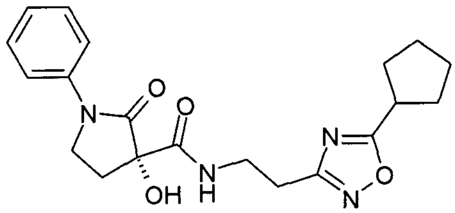 (S)-3-Hydroxy-2-oxo-1-phenylpyrrolidine-3-carboxylic acid [2-(5-cyclopentyl-1,2,4-oxadiazol-3-yl)ethyl] amide |
| "A13" | 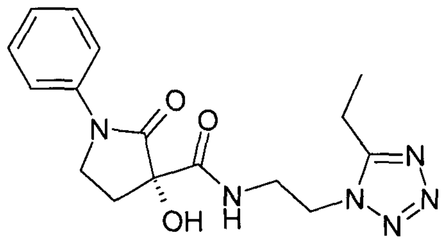 (S)-3-Hydroxy-2-oxo-1-phenylpyrrolidine-3-carboxylic acid [2-(5-ethyltetrazol-1-yl)ethyl] amide |
| "A14" | 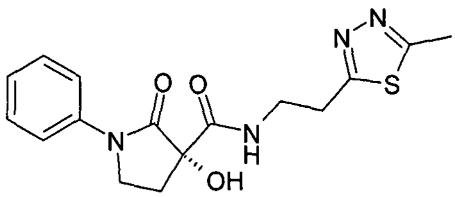 (S)-3-Hydroxy-2-oxo-1-phenylpyrrolidine-3-carboxylic acid [2-(5-methyl-1,3,4-thiadiazol-2-yl)ethyl] amide |
| "A15" | 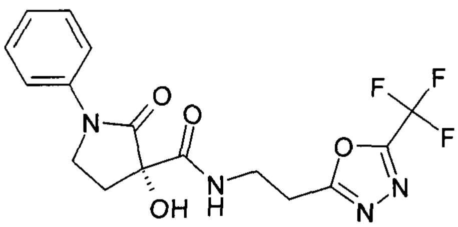 (S)-3-Hydroxy-2-oxo-1-phenylpyrrolidine-3-carboxylic acid [2-(5-trifluoromethyl-1,3,4-oxadiazol-2-yl)ethyl] amide |
| "A16" | 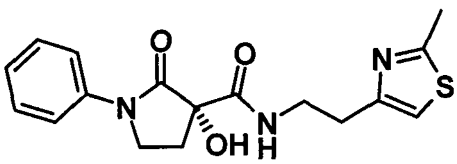 (S)-3-Hydroxy-2-oxo-1-phenylpyrrolidine-3-carboxylic acid [2-(2-methylthiazol-4-yl)ethyl] amide |
| "A17" | 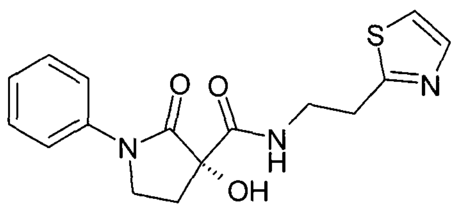 (S)-3-Hydroxy-2-oxo-1-phenylpyrrolidine-3-carboxylic acid (2-thiazol-2-ylethyl) amide |

(continued)

| Compound No. | Structure/name |
|---|---|
| "A18" | 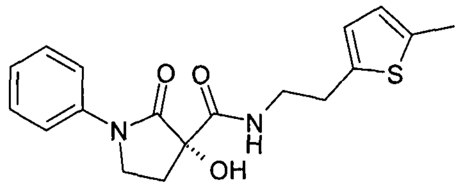<br>(S)-3-Hydroxy-2-oxo-1-phenylpyrrolidine-3-carboxylic acid [2-(5-methylthiophen-2-yl)ethyl] amide |
| "A19" | 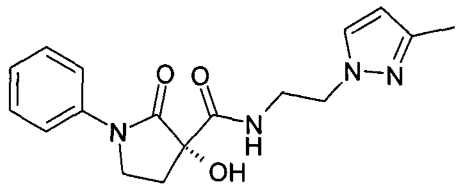<br>(S)-3-Hydroxy-2-oxo-1-phenylpyrrolidine-3-carboxylic acid [2-(3-methylpyrazol-1-yl)ethyl] amide |
| "A20" | 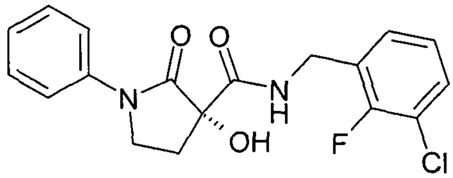<br>(S)-3-Hydroxy-2-oxo-1-phenylpyrrolidine-3-carboxylic acid 3-chloro-2-fluorobenzyl amide |
| "A21" | 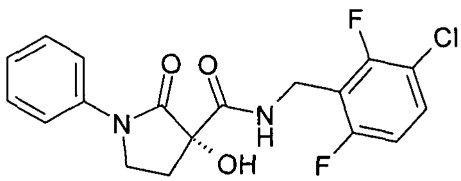<br>(S)-3-Hydroxy-2-oxo-1-phenylpyrrolidine-3-carboxylic acid 3-chloro-2,6-difluorobenzyl amide |
| "A22" | 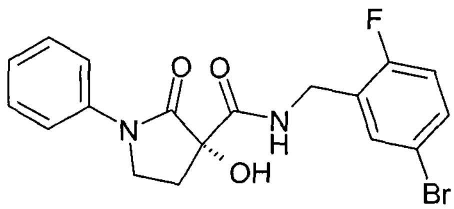<br>(S)-3-Hydroxy-2-oxo-1-phenylpyrrolidine-3-carboxylic acid 5-bromo-2-fluorobenzyl amide |
| "A23" | 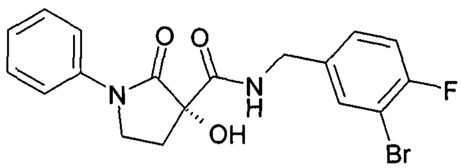<br>(S)-3-Hydroxy-2-oxo-1-phenylpyrrolidine-3-carboxylic acid 3-bromo-4-fluorobenzyl amide |
| "A24" | 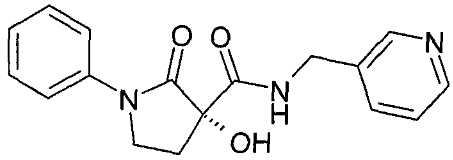<br>(S)-3-Hydroxy-2-oxo-1-phenylpyrrolidine-3-carboxylic acid (pyridin-3-ylmethyl) amide |

(continued)

| Compound No. | Structure/name |
| --- | --- |
| "A25" | 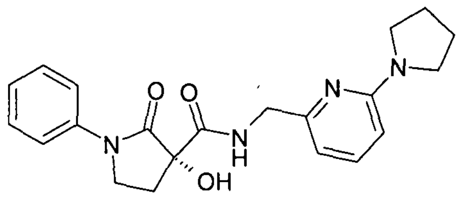 (S)-3-Hydroxy-2-oxo-1-phenylpyrrolidine-3-carboxylic acid (6-pyrrolidin-1-ylpyridin-2-ylmethyl) amide |
| "A26" | 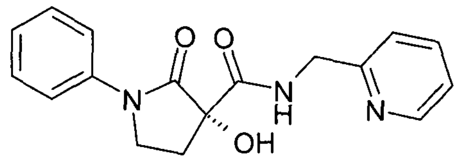 (S)-3-Hydroxy-2-oxo-1-phenylpyrrolidine-3-carboxylic acid (pyridin-2-ylmethyl) amide |
| "A27" | 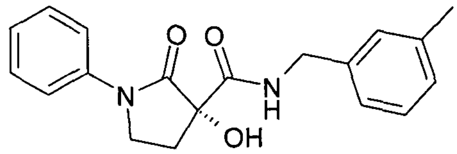 (S)-3-Hydroxy-2-oxo-1-phenylpyrrolidine-3-carboxylic acid 3-methylbenzyl amide |
| "A28" | 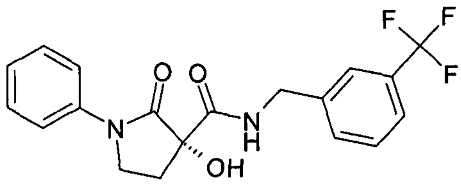 (S)-3-Hydroxy-2-oxo-1-phenylpyrrolidine-3-carboxylic acid 3-trifluoromethylbenzyl amide |
| "A29" | 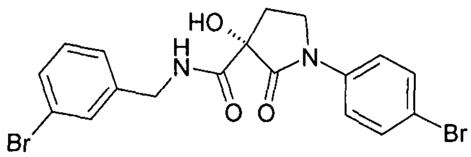 (S)-1-(4-Bromophenyl)-3-hydroxy-2-oxopyrrolidine-3-carboxylic acid 3-bromobenzyl amide |
| "A30" | 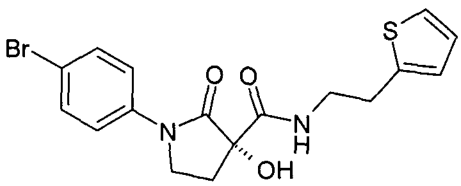 (S)-1-(4-Bromophenyl)-3-hydroxy-2-oxopyrrolidine-3-carboxylic acid (2-thiophen-2-ylethyl) amide |
| "A31" | 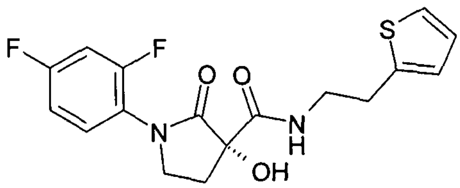 (S)-1-(2,4-Difluorophenyl)-3-hydroxy-2-oxopyrrolidine-3-carboxylic acid (2-thiophen-2-ylethyl) amide |

(continued)

| Compound No. | Structure/name |
|---|---|
| "A32" | 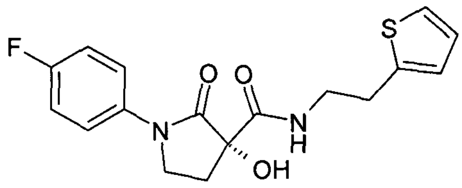<br>(S)-1-(4-Fluorophenyl)-3-hydroxy-2-oxopyrrolidine-3-carboxylic acid (2-thiophen-2-ylethyl) amide |
| "A33" | 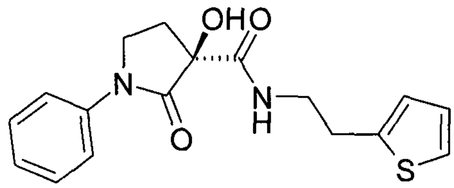<br>(S)-3-Hydroxy-2-oxo-1-phenylpyrrolidine-3-carboxylic acid (2-thiophen-2-ylethyl) amide |
| "A34" | 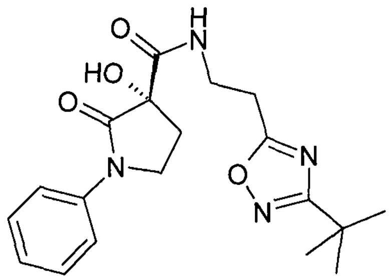<br>(S)-3-Hydroxy-2-oxo-1-phenylpyrrolidine-3-carboxylic acid [2-(3-tert-butyl-1,2,4-oxadiazol-5-yl)ethyl] amide |
| "A35" | 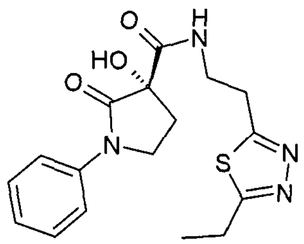<br>(S)-3-Hydroxy-2-oxo-1-phenylpyrrolidine-3-carboxylic acid [2-(5-ethyl-1,3,4-thiadiazol-2-yl)ethyl] amide |
| "A36" | 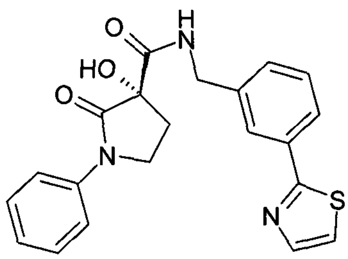<br>(S)-3-Hydroxy-2-oxo-1-phenylpyrrolidine-3-carboxylic acid 3-thiazol-2-ylbenzyl amide |

(continued)

| Compound No. | Structure/name |
|---|---|
| "A37" | 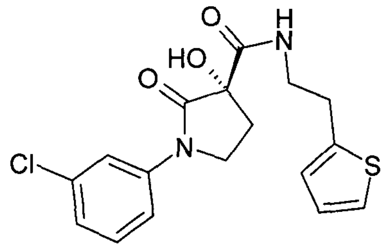<br>(S)-1-(3-Chlorophenyl)-3-hydroxy-2-oxopyrrolidine-3-carboxylic acid (2-thiophen-2-ylethyl) amide |
| "A38" | 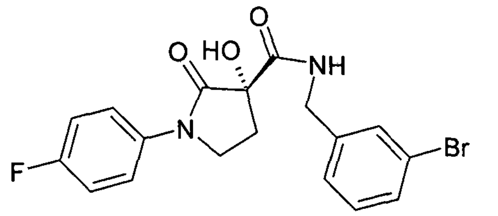<br>(S)-1-(4-Fluorophenyl)-3-hydroxy-2-oxopyrrolidine-3-carboxylic acid 3-bromobenzyl amide |
| "A39" | 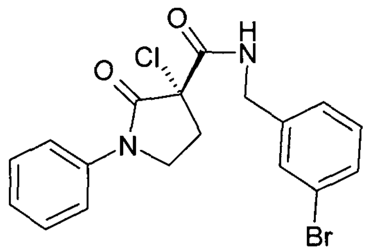<br>(S)-3-Chloro-2-oxo-1-phenylpyrrolidine-3-carboxylic acid 3-bromobenzyl amide |
| "A40" | 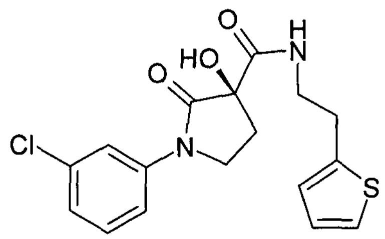<br>(R)-1-(3-Chlorophenyl)-3-hydroxy-2-oxopyrrolidine-3-carboxylic acid (2-thiophen-2-ylethyl) amide |
| "A41" | 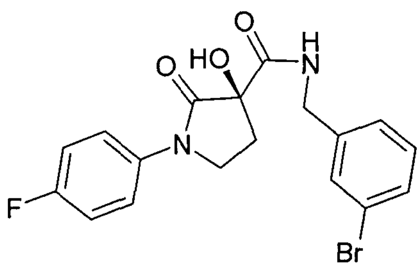<br>(R)-1-(4-Fluorophenyl)-3-hydroxy-2-oxopyrrolidine-3-carboxylic acid 3-bromobenzyl amide |
| "A42" | 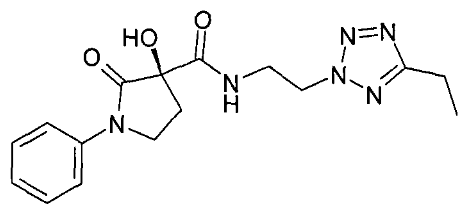 |

(continued)

| Compound No. | Structure/name |
|---|---|
| | (R)-3-Hydroxy-2-oxo-1-phenylpyrrolidine-3-carboxylic acid [2-(5-ethyltetrazol-2-yl)ethyl] amide |
| "A43" | 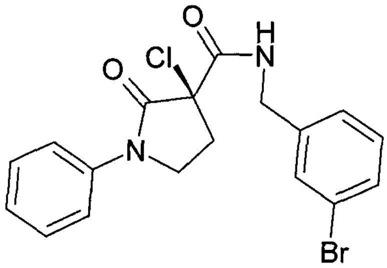 (R)-3-Chloro-2-oxo-1-phenylpyrrolidine-3-carboxylic acid 3-bromobenzyl amide |
| "A44" | 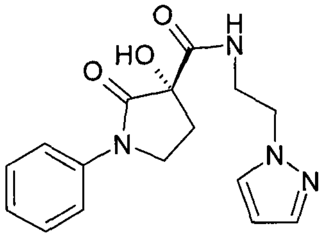 (S)-3-Hydroxy-2-oxo-1-phenylpyrrolidine-3-carboxylic acid (2-pyrazol-1-ylethyl) amide |
| "A45" | 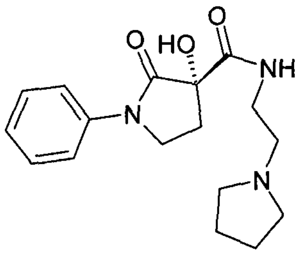 (S)-3-Hydroxy-2-oxo-1-phenylpyrrolidine-3-carboxylic acid (2-pyrrolidin-1-ylethyl) amide |
| "A46" | 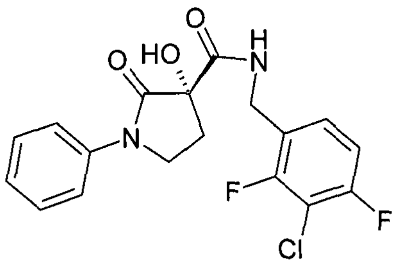 (S)-3-Hydroxy-2-oxo-1-phenylpyrrolidine-3-carboxylic acid 3-chloro-2,4-difluorobenzyl amide |
| "A47" | 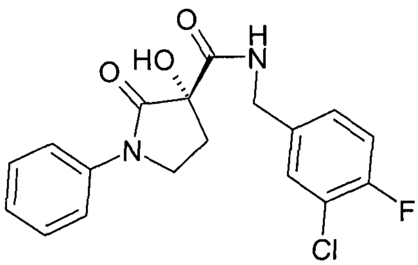 (S)-3-Hydroxy-2-oxo-1-phenylpyrrolidine-3-carboxylic acid 3-chloro-4-fluorobenzyl amide |

(continued)

| Compound No. | Structure/name |
|---|---|
| "A48" | 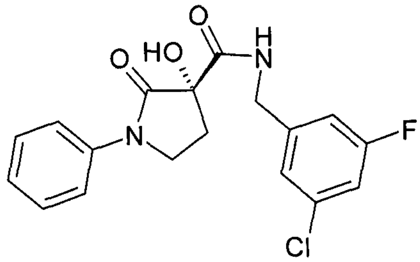 (S)-3-Hydroxy-2-oxo-1-phenylpyrrolidine-3-carboxylic acid 3-chloro-5-fluorobenzyl amide |
| "A49" | 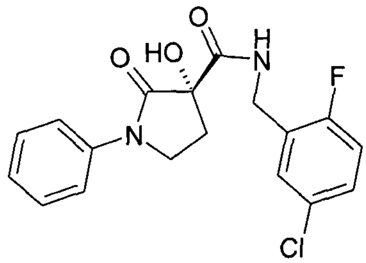 (S)-3-Hydroxy-2-oxo-1-phenylpyrrolidine-3-carboxylic acid 5-chloro-2-fluorobenzyl amide |
| "A50" | 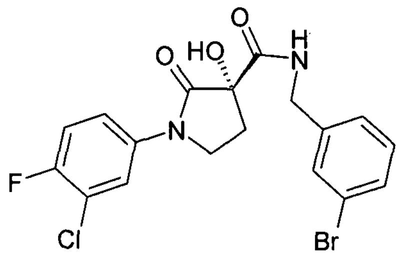 (S)-1-(3-Chloro-4-fluorophenyl)-3-hydroxy-2-oxopyrrolidine-3-carboxylic acid 3-bromobenzyl amide |
| "A51" | 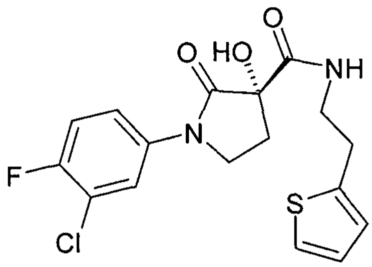 (S)-1-(3-Chloro-4-fluorophenyl)-3-hydroxy-2-oxopyrrolidine-3-carboxylic acid (2-thiophen-2-ylethyl) amide |
| "A52" | 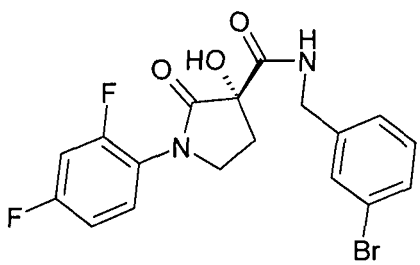 (S)-1-(2,4-Difluorophenyl)-3-hydroxy-2-oxopyrrolidine-3-carboxylic acid 3-bromobenzyl amide |

(continued)

| Compound No. | Structure/name |
|---|---|
| "A53" | 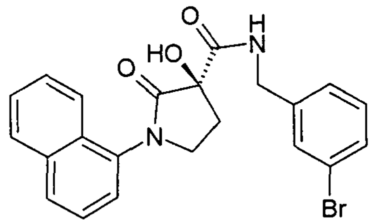 (R)-3-Hydroxy-1-naphthalen-1-yl-2-oxopyrrolidine-3-carboxylic acid 3-bromobenzyl amide |
| "A54" | 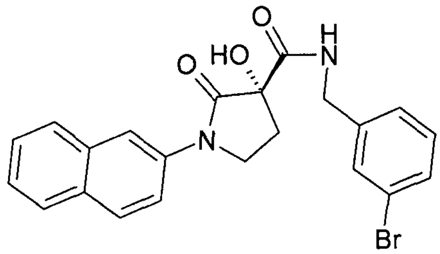 (S)-3-Hydroxy-1-naphthalen-2-yl-2-oxopyrrolidine-3-carboxylic acid 3-bromobenzyl amide |
| "A55" | 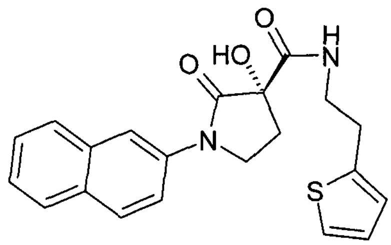 (S)-3-Hydroxy-1-naphthalen-2-yl-2-oxopyrrolidine-3-carboxylic acid (2-thiophen-2-ylethyl) amide |
| "A56" | 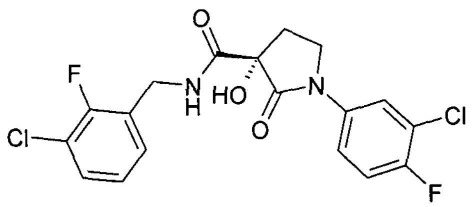 (S)-1-(3-Chloro-4-fluorophenyl)-3-hydroxy-2-oxopyrrolidine-3-carboxylic acid 3-chloro-2-fluorobenzyl amide |
| "A57" | 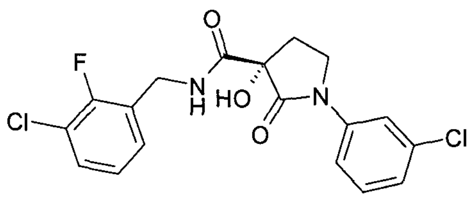 (S)-1-(3-Chlorophenyl)-3-hydroxy-2-oxopyrrolidine-3-carboxylic acid 3-chloro-2-fluorobenzyl amide |
| "A58" | 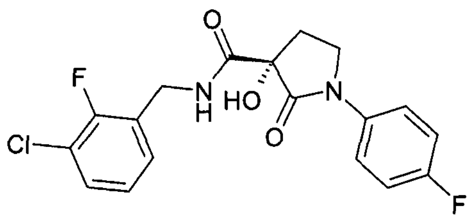 |

(continued)

| Compound No. | Structure/name |
|---|---|
| | (S)-1-(4-Fluorophenyl)-3-hydroxy-2-oxopyrrolidine-3-carboxylic acid 3-chloro-2-fluorobenzyl amide |
| "A59" | 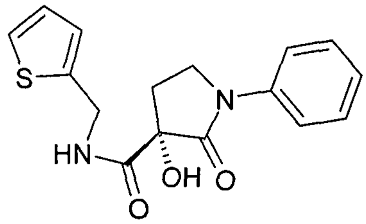 (S)-3-Hydroxy-2-oxo-1-phenylpyrrolidine-3-carboxylic acid (thiophen-2-ylmethyl) amide |
| "A60" | 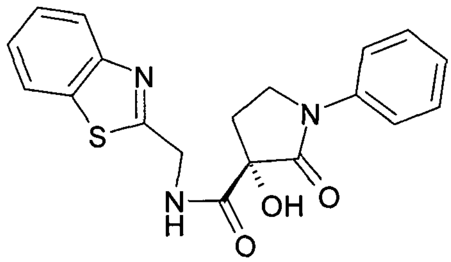 (S)-3-Hydroxy-2-oxo-1-phenylpyrrolidine-3-carboxylic acid (benzothiazol-2-ylmethyl) amide |
| "A61" | 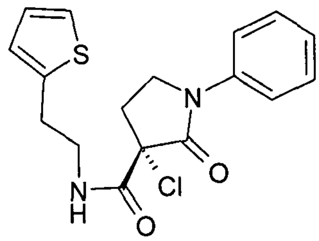 (S)-3-Chloro-2-oxo-1-phenylpyrrolidine-3-carboxylic acid (2-thiophen-2-ylethyl) amide |
| "A62" | 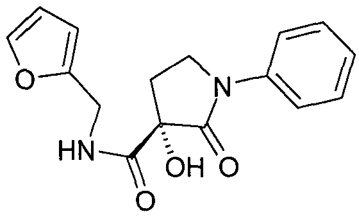 (S)-3-Hydroxy-2-oxo-1-phenylpyrrolidine-3-carboxylic acid (furan-2-ylmethyl) amide |
| "A63" | 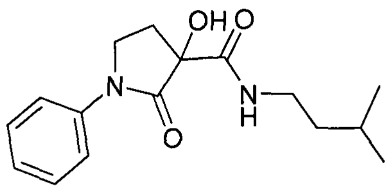 3-Hydroxy-2-oxo-1-phenylpyrrolidine-3-carboxylic acid (3-methylbutyl) amide |
| "A64" | 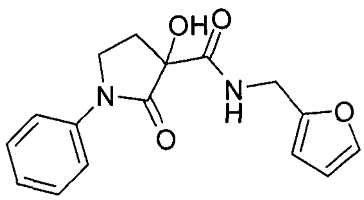 |

(continued)

| Compound No. | Structure/name |
|---|---|
| | 3-Hydroxy-2-oxo-1-phenylpyrrolidine-3-carboxylic acid (furan-2-ylmethyl) amide |
| "A65" | 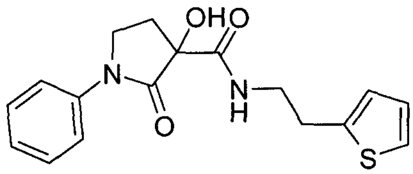 3-Hydroxy-2-oxo-1-phenylpyrrolidine-3-carboxylic acid (2-thiophen-2-ylethyl) amide |
| "A66" | 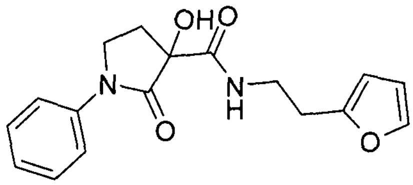 3-Hydroxy-2-oxo-1-phenylpyrrolidine-3-carboxylic acid (2-furan-2-ylethyl) amide |
| "A67" | 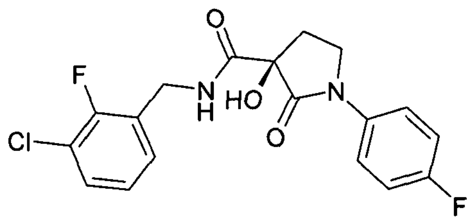 (R)-1-(4-Fluorophenyl)-3-hydroxy-2-oxopyrrolidine-3-carboxylic acid 3-chloro-2-fluorobenzyl amide |
| "A68" | 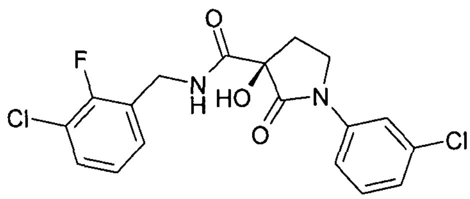 (R)-1-(3-Chlorophenyl)-3-hydroxy-2-oxopyrrolidine-3-carboxylic acid 3-chloro-2-fluorobenzyl amide |
| "A69" | 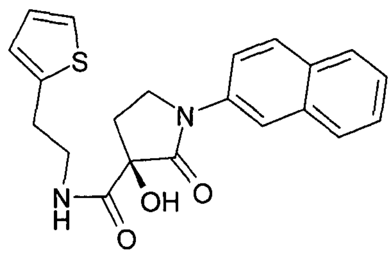 (R)-3-Hydroxy-1-naphthalen-2-yl-2-oxopyrrolidine-3-carboxylic acid (2-thiophen-2-ylethyl) amide |
| "A70" | 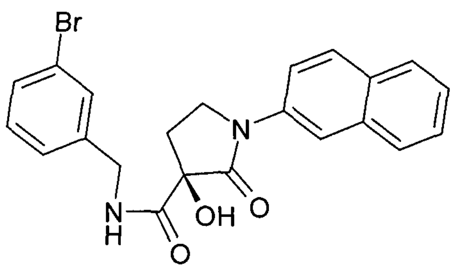 (R)-3-Hydroxy-1-naphthalen-2-yl-2-oxopyrrolidine-3-carboxylic acid 3-bromobenzyl amide |

(continued)

| Compound No. | Structure/name |
|---|---|
| "A71" | 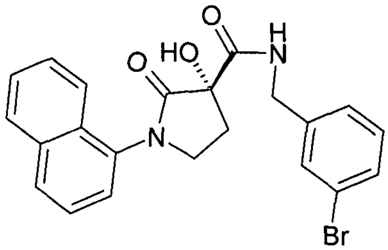<br>(S)-3-Hydroxy-1-naphthalen-1-yl-2-oxopyrrolidine-3-carboxylic acid 3-bromobenzyl amide |
| "A72" | 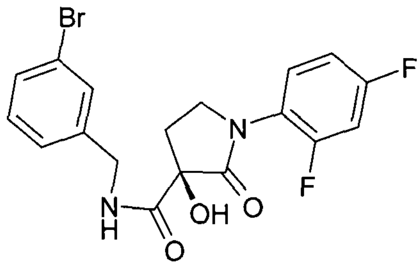<br>(R)-1-(2,4-Difluorophenyl)-3-hydroxy-2-oxopyrrolidine-3-carboxylic acid 3-bromobenzyl amide |
| "A73" | 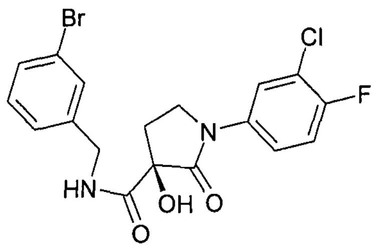<br>(R)-1-(3-Chloro-4-fluorophenyl)-3-hydroxy-2-oxopyrrolidine-3-carboxylic acid 3-bromobenzyl amide |
| "A74" | 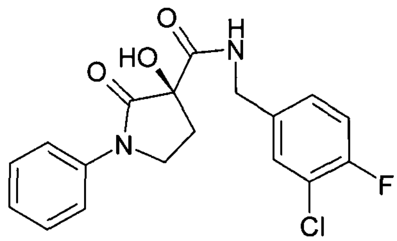<br>(R)-3-Hydroxy-2-oxo-1-phenylpyrrolidine-3-carboxylic acid 3-chloro-4-fluorobenzyl amide |
| "A75" | 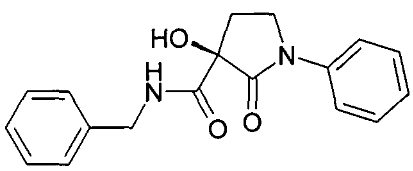<br>(R)-3-Hydroxy-2-oxo-1-phenylpyrrolidine-3-carboxylic acid benzyl amide |
| "A76" | 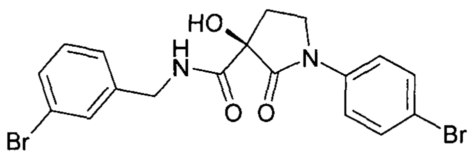<br>(R)-1-(4-Bromophenyl)-3-hydroxy-2-oxopyrrolidine-3-carboxylic acid 3-bromobenzyl amide |

(continued)

| Compound No. | Structure/name |
|---|---|
| "A77" | 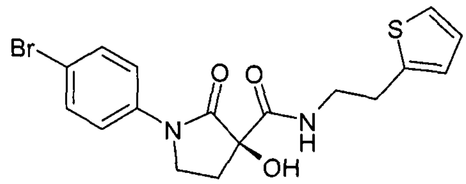 (R)-1-(4-Bromophenyl)-3-hydroxy-2-oxopyrrolidine-3-carboxylic acid (2-thiophen-2-ylethyl) amide |
| "A78" | 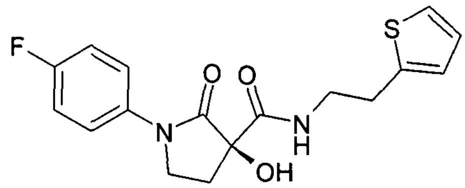 (R)-1-(4-Fluorophenyl)-3-hydroxy-2-oxopyrrolidine-3-carboxylic acid (2-thiophen-2-ylethyl) amide |
| "A79" | 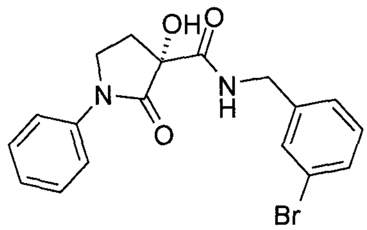 (R)-3-Hydroxy-2-oxo-1-phenylpyrrolidine-3-carboxylic acid 3-bromobenzyl amide |
| "A80" | 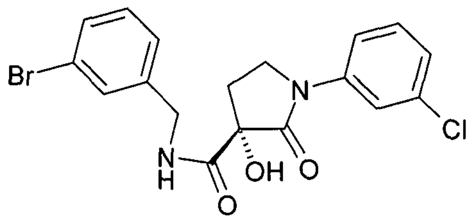 (S)-1-(3-Chlorophenyl)-3-hydroxy-2-oxopyrrolidine-3-carboxylic acid 3-bromobenzyl amide |
| "A81" | 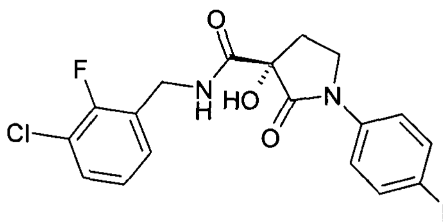 (S)-1-(4-Bromophenyl)-3-hydroxy-2-oxopyrrolidine-3-carboxylic acid 3-chloro-2-fluorobenzyl amide |
| "A82" | 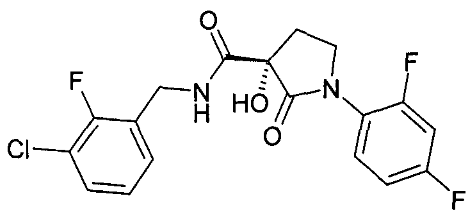 (S)-1-(2,4-Difluorophenyl)-3-hydroxy-2-oxopyrrolidine-3-carboxylic acid 3-chloro-2-fluorobenzyl amide |

(continued)

| Compound No. | Structure/name |
|---|---|
| "A83" | 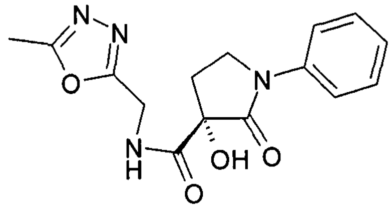 (S)-3-Hydroxy-2-oxo-1-phenylpyrrolidine-3-carboxylic acid (5-methyl-1,3,4-oxadiazol-2-ylmethyl) amide |
| "A84" | 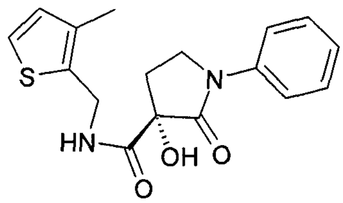 (S)-3-Hydroxy-2-oxo-1-phenylpyrrolidine-3-carboxylic acid (3-methylthiophen-2-ylmethyl) amide |
| "A85" | 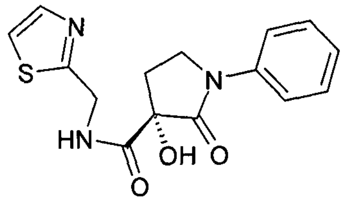 (S)-3-Hydroxy-2-oxo-1-phenylpyrrolidine-3-carboxylic acid (thiazol-2-ylmethyl) amide |
| "A86" | 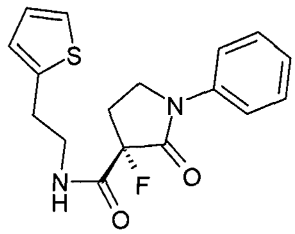 (S)-3-Fluoro-2-oxo-1-phenylpyrrolidine-3-carboxylic acid (2-thiophen-2-ylethyl) amide |
| "A87" | 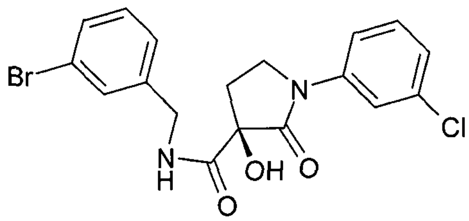 (R)-1-(3-Chlorophenyl)-3-hydroxy-2-oxopyrrolidine-3-carboxylic acid 3-bromobenzyl amide |
| "A88" | 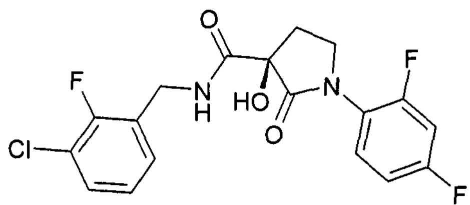 (R)-1-(2,4-Difluorophenyl)-3-hydroxy-2-oxopyrrolidine-3-carboxylic acid 3-chloro-2-fluorobenzyl amide |

(continued)

| Compound No. | Structure/name |
|---|---|
| "A89" | 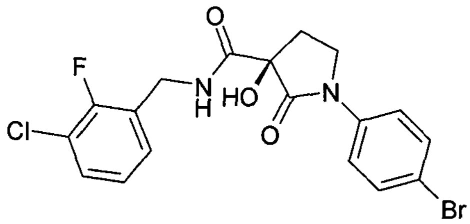 (R)-1-(4-Bromophenyl)-3-hydroxy-2-oxopyrrolidine-3-carboxylic acid 3-chloro-2-fluorobenzyl amide |
| "A90" | 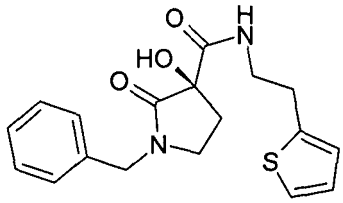 (R)-1-Benzyl-3-hydroxy-2-oxopyrrolidine-3-carboxylic acid (2-thiophen-2-ylethyl) amide |
| "A91" | 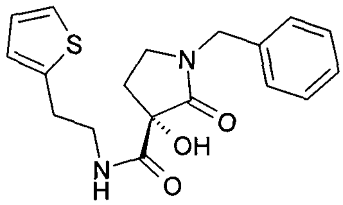 (S)-1-Benzyl-3-hydroxy-2-oxopyrrolidine-3-carboxylic acid (2-thiophen-2-ylethyl) amide |
| "A91a" | (S)-3-Amino-2-oxo-1-phenylpyrrolidine-3-carboxylic acid (3-chloro-5-fluorobenzyl) amide |
| "A92" | 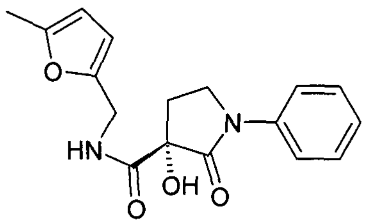 (S)-3-Hydroxy-2-oxo-1-phenylpyrrolidine-3-carboxylic acid (5-methylfuran-2-ylmethyl) amide |
| "A93" | 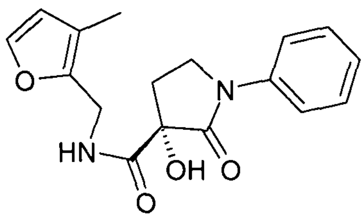 (S)-3-Hydroxy-2-oxo-1-phenylpyrrolidine-3-carboxylic acid (3-methylfuran-2-ylmethyl) amide |
| "A94" | 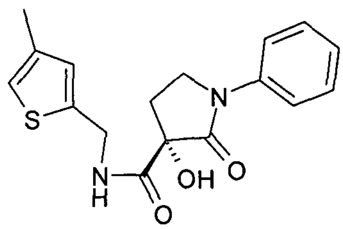 (S)-3-Hydroxy-2-oxo-1-phenylpyrrolidine-3-carboxylic acid (4-methylthiophen-2-ylmethyl) amide |

(continued)

| Compound No. | Structure/name |
|---|---|
| "A95" | 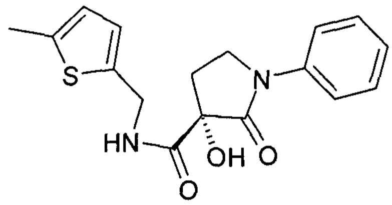<br>(S)-3-Hydroxy-2-oxo-1-phenylpyrrolidine-3-carboxylic acid (5-methylthiophen-2-ylmethyl) amide |
| "A96" | 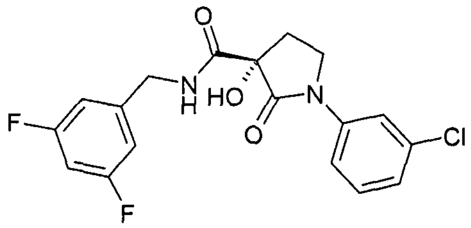<br>(S)-1-(3-Chlorophenyl)-3-hydroxy-2-oxopyrrolidine-3-carboxylic acid 3,5-difluorobenzyl amide |
| "A97" | 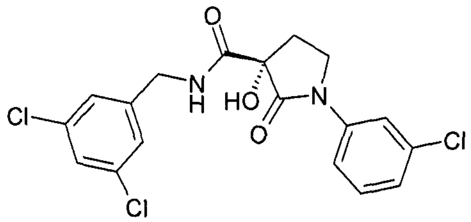<br>(S)-1-(3-Chlorophenyl)-3-hydroxy-2-oxopyrrolidine-3-carboxylic acid 3,5-dichlorobenzyl amide |
| "A98" | 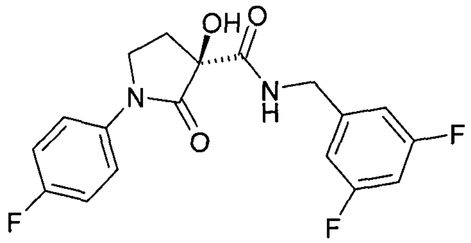<br>(S)-1-(4-Fluorophenyl)-3-hydroxy-2-oxopyrrolidine-3-carboxylic acid 3,5-difluorobenzyl amide |
| "A99" | 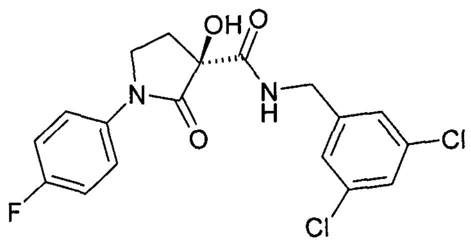<br>(S)-1-(4-Fluorophenyl)-3-hydroxy-2-oxopyrrolidine-3-carboxylic acid 3,5-dichlorobenzyl amide |
| "A100" | 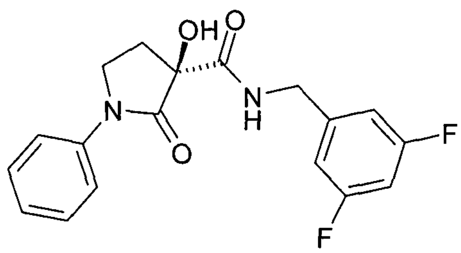<br>(S)-3-Hydroxy-2-oxo-1-phenylpyrrolidine-3-carboxylic acid 3,5-difluorobenzyl amide |

(continued)

| Compound No. | Structure/name |
| --- | --- |
| "A101" | 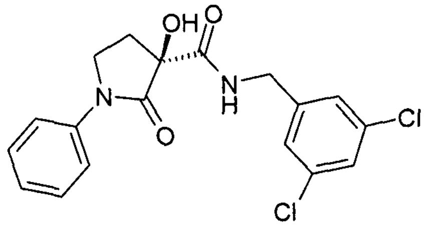 (S)-3-Hydroxy-2-oxo-1-phenylpyrrolidine-3-carboxylic acid 3,5-dichlorobenzyl amide |
| "A102" | 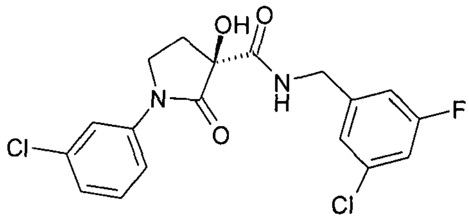 (S)-1-(3-Chlorophenyl)-3-hydroxy-2-oxopyrrolidine-3-carboxylic acid 3-chloro-5-fluorobenzyl amide |
| "A103" | 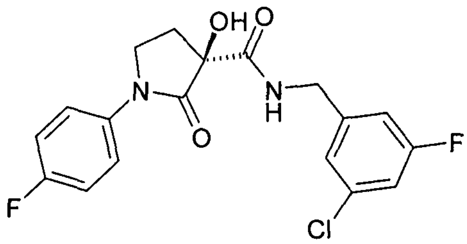 (S)-1-(4-Fluorophenyl)-3-hydroxy-2-oxopyrrolidine-3-carboxylic acid 3-chloro-5-fluorobenzyl amide |
| "A104" | 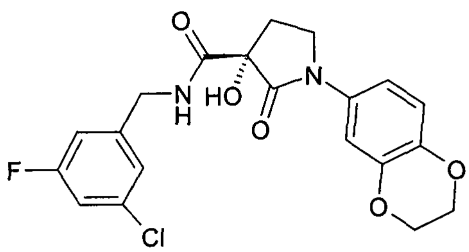 (S)-1-(2,3-Dihydrobenzo-1,4-dioxin-6-yl)-3-hydroxy-2-oxo-pyrrolidine-3-carboxylic acid 3-chloro-5-fluorobenzyl amide |
| "A105" | 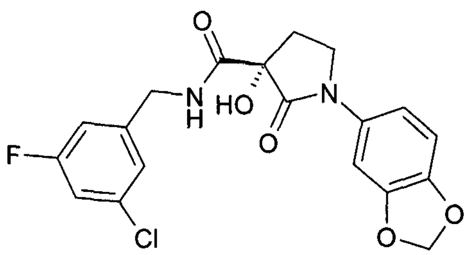 (S)-1-Benzo-1,3-dioxol-5-yl-3-hydroxy-2-oxopyrrolidine-3-carboxylic acid 3-chloro-5-fluorobenzyl amide |

(continued)

| Compound No. | Structure/name |
|---|---|
| "A106" | 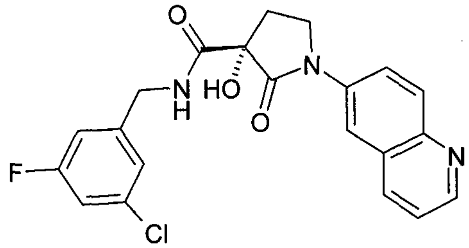<br>(S)-3-Hydroxy-2-oxo-1-quinolin-6-ylpyrrolidine-3-carboxylic acid 3-chloro-5-fluorobenzyl amide |
| "A107" | 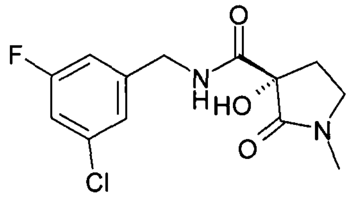<br>(S)-3-Hydroxy-1-methyl-2-oxopyrrolidine-3-carboxylic acid 3-chloro-5-fluorobenzyl amide |
| "A108" | 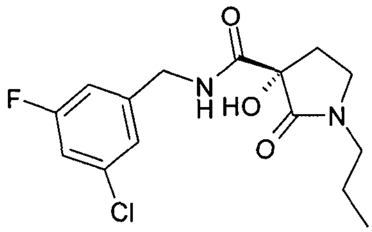<br>(S)-3-Hydroxy-2-oxo-1-propylpyrrolidine-3-carboxylic acid 3-chloro-5-fluorobenzyl amide |
| "A109" | 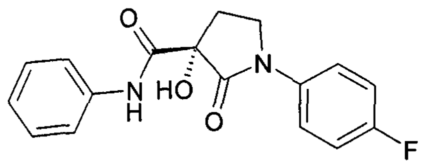<br>(S)-1-(4-Fluorophenyl)-3-hydroxy-2-oxopyrrolidine-3-carboxylic acid phenyl amide |
| "A110" | 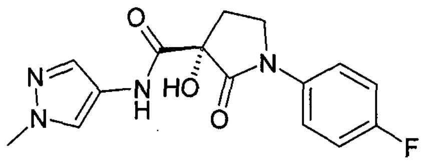<br>(S)-1-(4-Fluorophenyl)-3-hydroxy-2-oxopyrrolidine-3-carboxylic acid (1-methyl-1 H-pyrazol-4-yl) amide |
| "A111" | 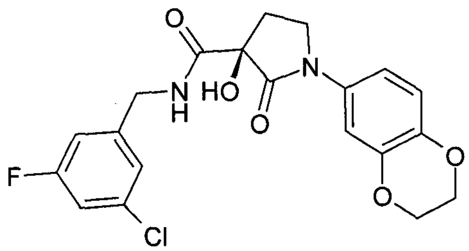<br>(R)-1-(2,3-Dihydrobenzo-1,4-dioxin-6-yl)-3-hydroxy-2-oxo-pyrrolidine-3-carboxylic acid 3-chloro-5-fluorobenzyl amide |

(continued)

| Compound No. | Structure/name |
|---|---|
| "A112" | 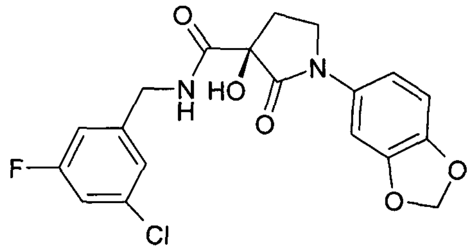 (R)-1-Benzo-1,3-dioxol-5-yl-3-hydroxy-2-oxopyrrolidine-3-carboxylic acid 3-chloro-5-fluorobenzyl amide |
| "A113" | 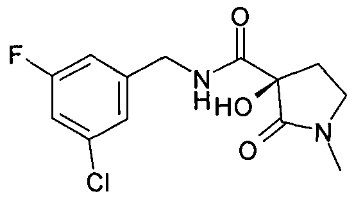 (R)-3-Hydroxy-1-methyl-2-oxopyrrolidine-3-carboxylic acid 3-chloro-5-fluorobenzyl amide |
| "A114" | 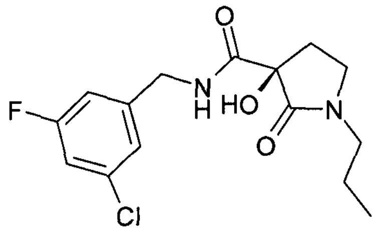 (R)-3-Hydroxy-2-oxo-1-propylpyrrolidine-3-carboxylic acid 3-chloro-5-fluorobenzyl amide |
| "A115" | 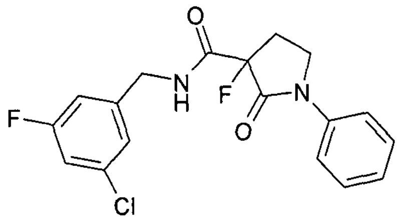 3-Fluoro-2-oxo-1-phenylpyrrolidine-3-carboxylic acid 3-chloro-5-fluorobenzyl amide |
| "A116" | 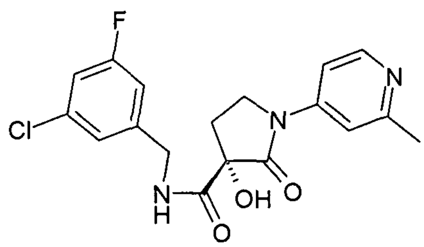 (S)-3-Hydroxy-1-(2-methylpyridin-4-yl)-2-oxopyrrolidine-3-carboxylic acid 3-chloro-5-fluorobenzyl amide |
| "A117" | 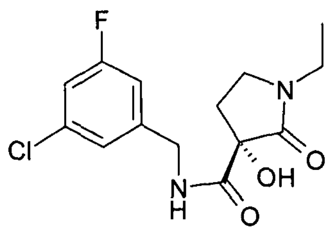 |

(continued)

| Compound No. | Structure/name |
|---|---|
| | (S)-1-Ethyl-3-hydroxy-2-oxopyrrolidine-3-carboxylic acid 3-chloro-5-fluorobenzyl amide |
| "A118" | 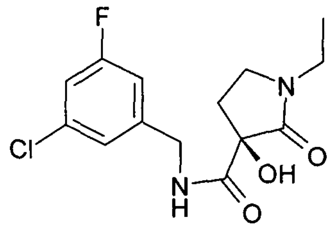 (R)-1-Ethyl-3-hydroxy-2-oxopyrrolidine-3-carboxylic acid 3-chloro-5-fluorobenzyl amide |
| "A119" | 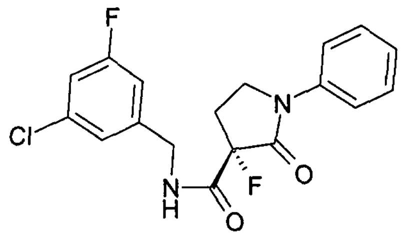 (S)-3-Fluoro-2-oxo-1-phenylpyrrolidine-3-carboxylic acid 3-chloro-5-fluorobenzyl amide |
| "A120" | 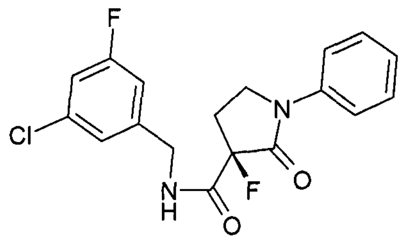 (R)-3-Fluoro-2-oxo-1-phenylpyrrolidine-3-carboxylic acid 3-chloro-5-fluorobenzyl amide |
| "A150" | 3-Azido-2-oxo-1-phenylpyrrolidine-3-carboxylic acid (3-chloro-5-fluorobenzyl) amide |
| "A151" | 3-Amino-2-oxo-1-phenylpyrrolidine-3-carboxylic acid (3-chloro-5-fluorobenzyl) amide |
| "A162" | 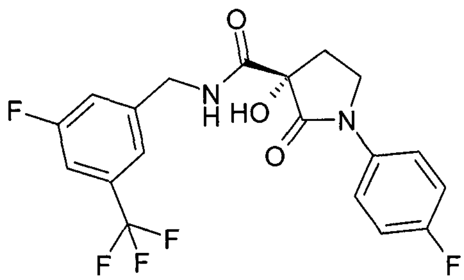 (S)-1-(4-Fluorophenyl)-3-hydroxy-2-oxopyrrolidine-3-carboxylic acid 3-fluoro-5-trifluoromethylbenzyl amide |
| "A163" | 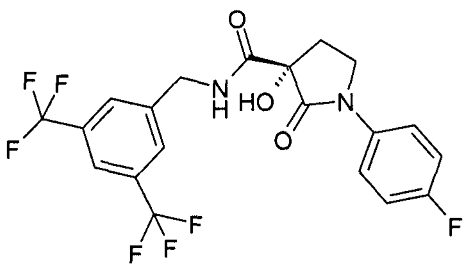 (S)-1-(4-Fluorophenyl)-3-hydroxy-2-oxopyrrolidine-3-carboxylic acid 3,5-bistrifluoromethylbenzyl amide |

(continued)

| Compound No. | Structure/name |
|---|---|
| "A164" | 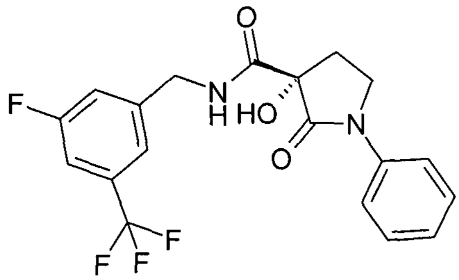 (S)-3-Hydroxy-2-oxo-1-phenylpyrrolidine-3-carboxylic acid 3-fluoro-5-trifluoromethylbenzyl amide |
| "A165" | 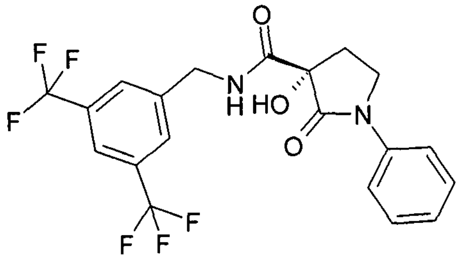 (S)-3-Hydroxy-2-oxo-1-phenylpyrrolidine-3-carboxylic acid 3,5-bistrifluoromethylbenzyl amide |
| "A166" | 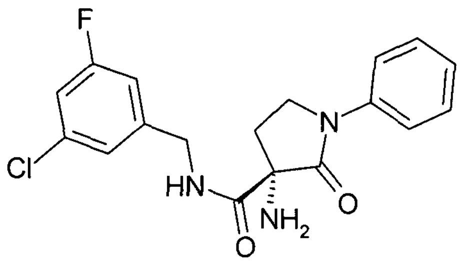 (S)-3-Amino-2-oxo-1-phenylpyrrolidine-3-carboxylic acid 3-chloro-5-fluorobenzyl amide |
| "A167" | 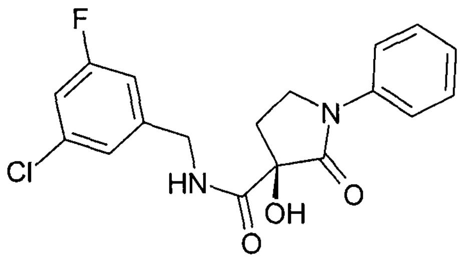 (R)-3-Hydroxy-2-oxo-1-phenylpyrrolidine-3-carboxylic acid 3-chloro-5-fluorobenzyl amide |
| "A168" | 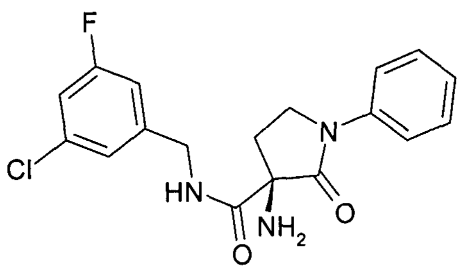 (R)-3-Amino-2-oxo-1-phenylpyrrolidine-3-carboxylic acid 3-chloro-5-fluorobenzyl amide |

(continued)

| Compound No. | Structure/name |
|---|---|
| "A169" | 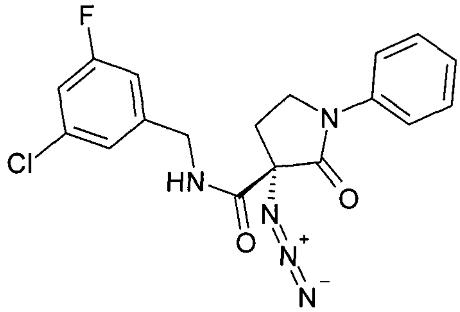 (S)-3-Azido-2-oxo-1-phenylpyrrolidine-3-carboxylic acid 3-chloro-5-fluorobenzyl amide |
| "A170" | 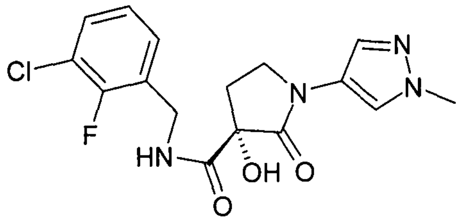 (S)-3-Hydroxy-1-(1-methyl-1H-pyrazol-4-yl)-2-oxo-pyrrolidine-3-carboxylic acid 3-chloro-2-fluorobenzyl amide |
| "A171" | 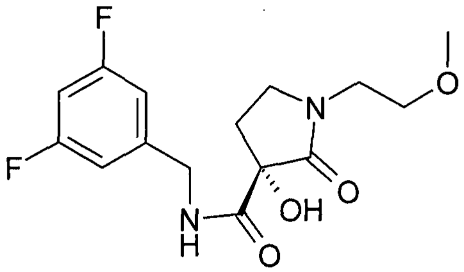 (S)-3-Hydroxy-1-(2-methoxyethyl)-2-oxopyrrolidine-3-carboxylic acid 3,5-difluorobenzyl amide |
| "A172" | 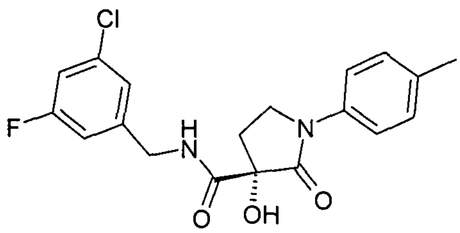 (S)-3-Hydroxy-2-oxo-1-p-tolylpyrrolidine-3-carboxylic acid 3-chloro-5-fluorobenzyl amide |
| "A173" | 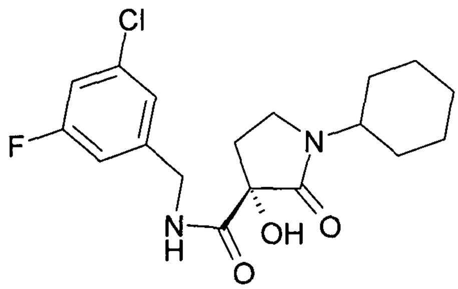 (S)-1-Cyclohexyl-3-hydroxy-2-oxopyrrolidine-3-carboxylic acid 3-chloro-5-fluorobenzyl amide |

(continued)

| Compound No. | Structure/name |
|---|---|
| "A174" | 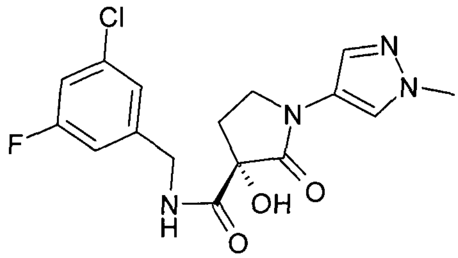 (S)-3-Hydroxy-1-(1-methyl-1H-pyrazol-4-yl)-2-oxo-pyrrolidine-3-carboxylic acid 3-chloro-5-fluorobenzyl amide |
| "A175" | 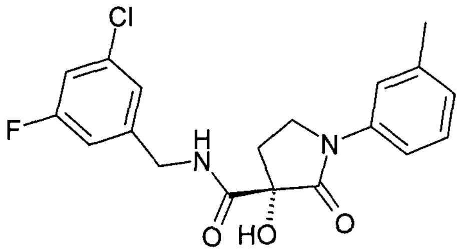 (S)-3-Hydroxy-2-oxo-1-m-tolylpyrrolidine-3-carboxylic acid 3-chloro-5-fluorobenzyl amide |
| "A176" | 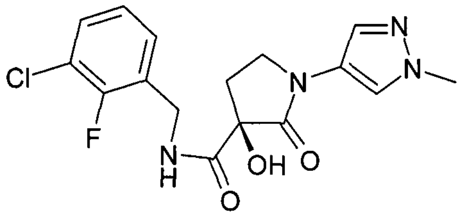 (R)-3-Hydroxy-1-(1-methyl-1H-pyrazol-4-yl)-2-oxo-pyrrolidine-3-carboxylic acid 3-chloro-2-fluorobenzyl amide |
| "A177" | 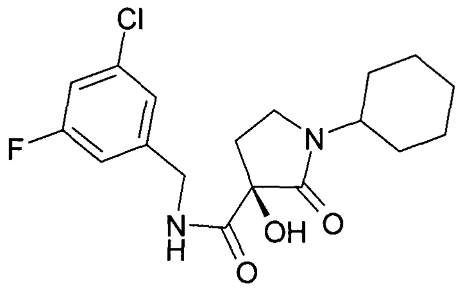 (R)-1-Cyclohexyl-3-hydroxy-2-oxopyrrolidine-3-carboxylic acid 3-chloro-5-fluorobenzyl amide |
| "A178" | 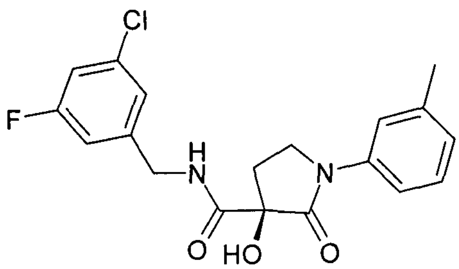 (R)-3-Hydroxy-2-oxo-1-m-tolylpyrrolidine-3-carboxylic acid 3-chloro-5-fluorobenzyl amide |

(continued)

| Compound No. | Structure/name |
|---|---|
| "A179" | 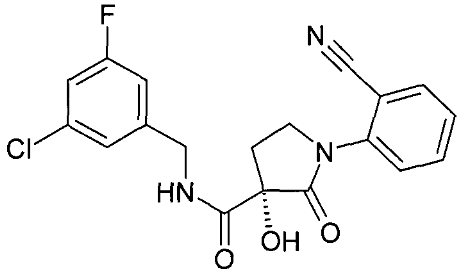 (S)-1-(2-Cyanophenyl)-3-hydroxy-2-oxopyrrolidine-3-carboxylic acid 3-chloro-5-fluorobenzyl amide |
| "A180" | 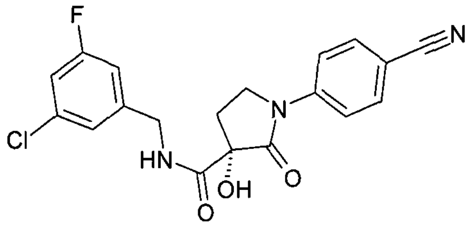 (S)-1-(4-Cyanophenyl)-3-hydroxy-2-oxopyrrolidine-3-carboxylic acid 3-chloro-5-fluorobenzyl amide |
| "A181" | 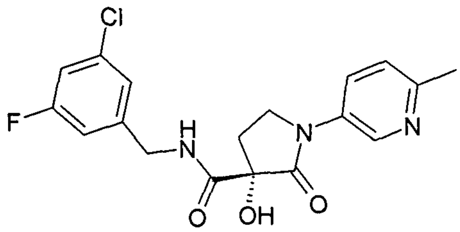 (S)-3-Hydroxy-1-(6-methylpyridin-3-yl)-2-oxopyrrolidine-3-carboxylic acid 3-chloro-5-fluorobenzyl amide |
| "A182" | 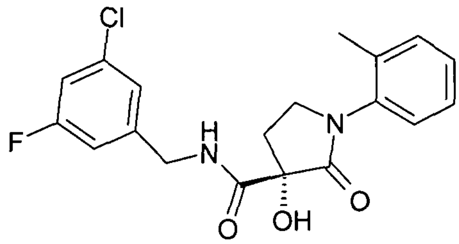 (S)-3-Hydroxy-2-oxo-1-o-tolylpyrrolidine-3-carboxylic acid 3-chloro-5-fluorobenzyl amide |
| "A183" | 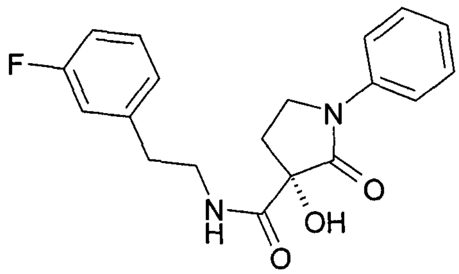 (S)-3-Hydroxy-2-oxo-1-phenylpyrrolidine-3-carboxylic acid [2-(3-fluorophenyl)ethyl] amide |

(continued)

| Compound No. | Structure/name |
|---|---|
| "A184" | 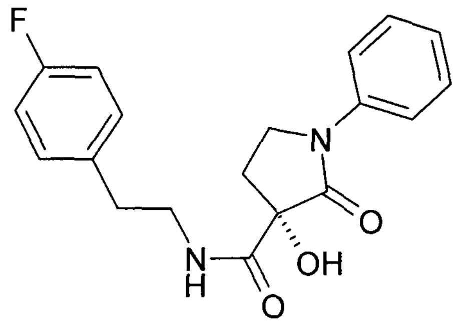<br>(S)-3-Hydroxy-2-oxo-1-phenylpyrrolidine-3-carboxylic acid [2-(4-fluorophenyl)ethyl] amide |
| "A185" | 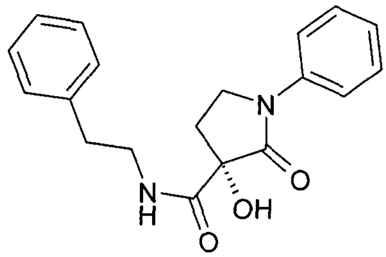<br>(S)-3-Hydroxy-2-oxo-1-phenylpyrrolidine-3-carboxylic acid phenethyl amide |
| "A186" | 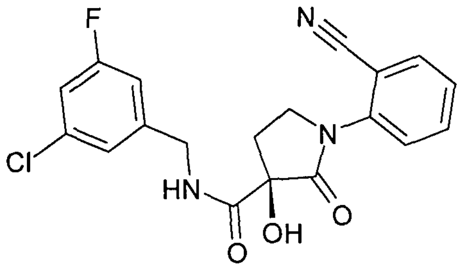<br>(R)-1-(2-Cyanophenyl)-3-hydroxy-2-oxopyrrolidine-3-carboxylic acid 3-chloro-5-fluorobenzyl amide |
| "A187" | 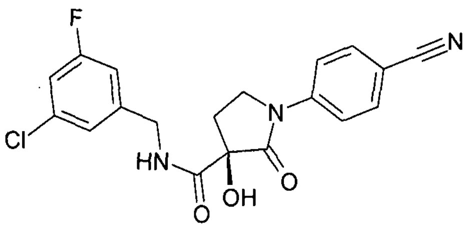<br>(R)-1-(4-Cyanophenyl)-3-hydroxy-2-oxopyrrolidine-3-carboxylic acid 3-chloro-5-fluorobenzyl amide |
| "A188" | 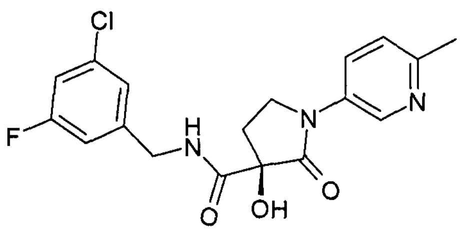<br>(R)-3-Hydroxy-1-(6-methylpyridin-3-yl)-2-oxopyrrolidine-3-carboxylic acid 3-chloro-5-fluorobenzyl amide |

(continued)

| Compound No. | Structure/name |
|---|---|
| "A189" | 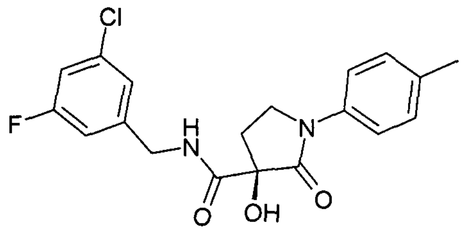<br>(R)-3-Hydroxy-2-oxo-1-p-tolylpyrrolidine-3-carboxylic acid 3-chloro-5-fluorobenzyl amide |
| "A190" | 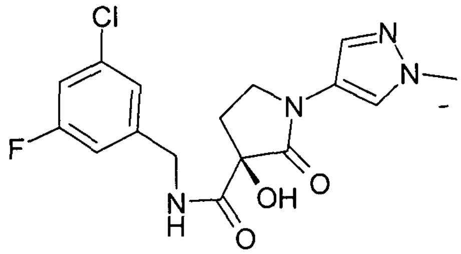<br>(R)-3-Hydroxy-1-(1-methyl-1H-pyrazol-4-yl)-2-oxo-pyrrolidine-3-carboxylic acid 3-chloro-5-fluorobenzyl amide |
| "A191" | 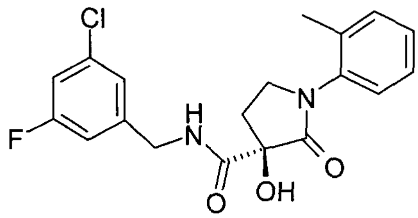<br>(R)-3-Hydroxy-2-oxo-1-o-tolylpyrrolidine-3-carboxylic acid 3-chloro-5-fluorobenzyl amide |
| "A192" | 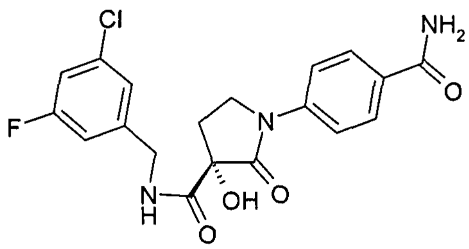<br>(S)-1-(4-Carbamoylphenyl)-3-hydroxy-2-oxopyrrolidine-3-carboxylic acid 3-chloro-5-fluorobenzyl amide |
| "A193" | 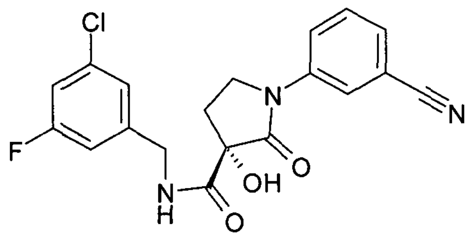<br>(S)-1-(3-Cyanophenyl)-3-hydroxy-2-oxopyrrolidine-3-carboxylic acid 3-chloro-5-fluorobenzyl amide |

(continued)

| Compound No. | Structure/name |
|---|---|
| "A194" | 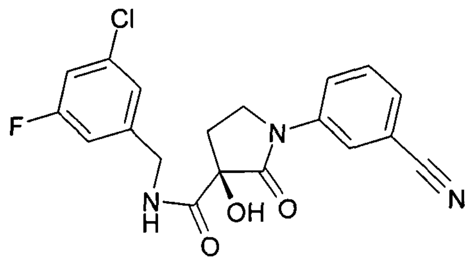 (R)-1-(3-Cyanophenyl)-3-hydroxy-2-oxopyrrolidine-3-carboxylic acid 3-chloro-5-fluorobenzyl amide |
| "A195" | 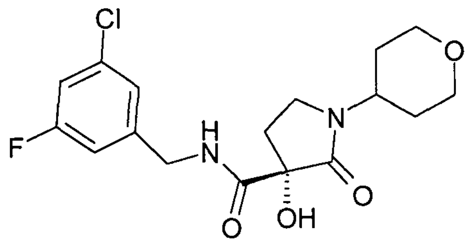 (S)-3-Hydroxy-2-oxo-1-(tetrahydropyran-4-yl)pyrrolidine-3-carboxylic acid 3-chloro-5-fluorobenzyl amide |
| "A196" | 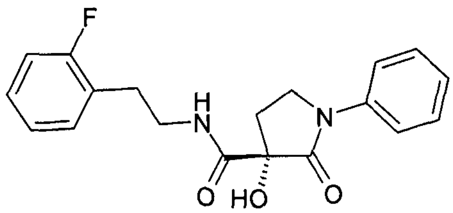 (S)-3-Hydroxy-2-oxo-1-phenylpyrrolidine-3-carboxylic acid [2-(2-fluorophenyl)ethyl] amide |
| "A197" | 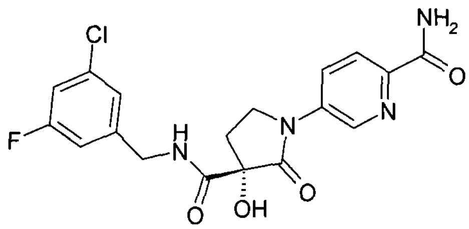 5-[(S)-3-(3-Chloro-5-fluorobenzylcarbamoyl)-3-hydroxy-2-oxopyrrolidin-1-yl]pyridine-2-carboxylic acid amide |
| "A198" | 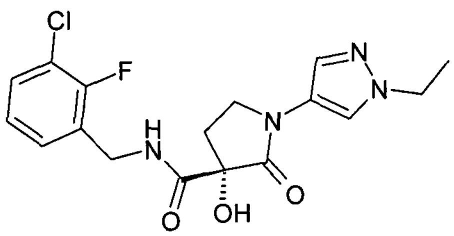 (S)-1-(1-Ethyl-1H-pyrazol-4-yl)-3-hydroxy-2-oxopyrrolidine-3-carboxylic acid 3-chloro-2-fluorobenzyl amide |

(continued)

| Compound No. | Structure/name |
|---|---|
| "A199" | 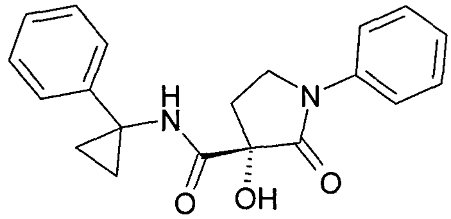 (S)-3-Hydroxy-2-oxo-1-phenylpyrrolidine-3-carboxylic acid (1-phenylcyclopropyl) amide |
| "A200" | 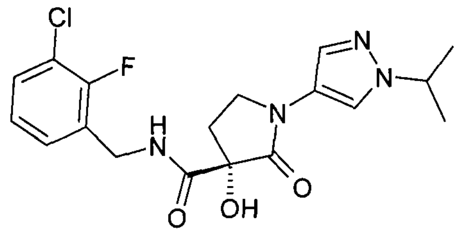 (S)-3-Hydroxy-1-(1-isopropyl-1H-pyrazol-4-yl)-2-oxo-pyrrolidine-3-carboxylic acid 3-chloro-2-fluorobenzyl amide |
| "A201" | 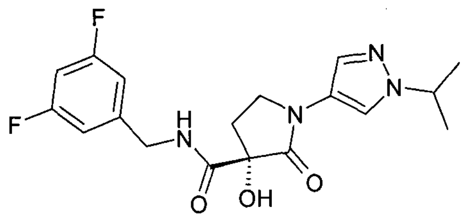 (S)-3-Hydroxy-1-(1-isopropyl-1H-pyrazol-4-yl)-2-oxo-pyrrolidine-3-carboxylic acid 3,5-difluorobenzyl amide |
| "A202" | 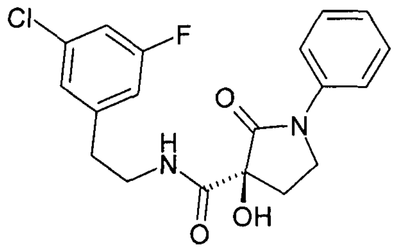 (S)-3-Hydroxy-2-oxo-1-phenylpyrrolidine-3-carboxylic acid [2-(3-chloro-5-fluorophenyl)ethyl] amide |
| "A203" | 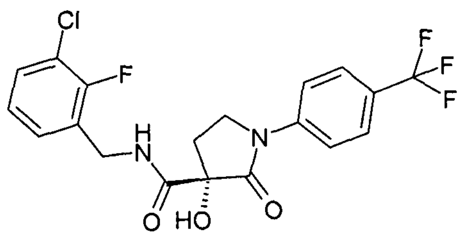 (S)-3-Hydroxy-2-oxo-1-(4-trifluoromethylphenyl)pyrrolidine-3-carboxylic acid 3-chloro-2-fluorobenzyl amide |

(continued)

| Compound No. | Structure/name |
|---|---|
| "A204" | 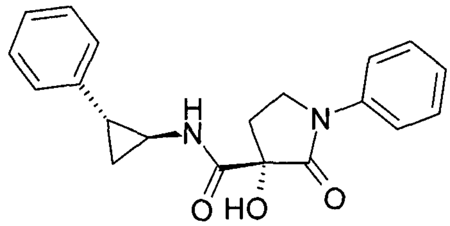 (S)-3-Hydroxy-2-oxo-1-phenylpyrrolidine-3-carboxylic acid ((1S,2R)-2-phenylcyclopropyl) amide |
| "A205" | 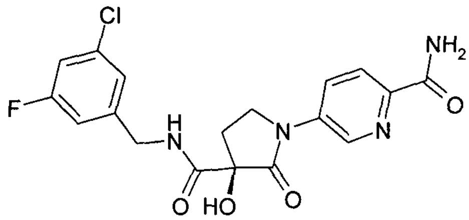 5-[(R)-3-(3-Chloro-5-fluorobenzylcarbamoyl)-3-hydroxy-2-oxopyrrolidin-1-yl]pyridine-2-carboxylic acid amide |
| "A206" | 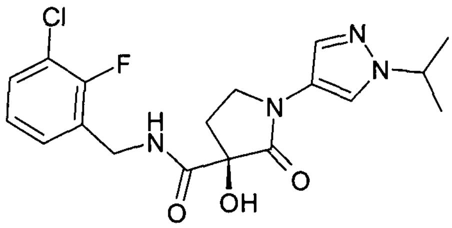 (R)-3-Hydroxy-1-(1-isopropyl-1H-pyrazol-4-yl)-2-oxo-pyrrolidine-3-carboxylic acid 3-chloro-2-fluorobenzyl amide |
| "A207" | 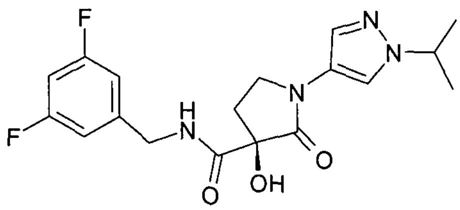 (R)-3-Hydroxy-1-(1-isopropyl-1H-pyrazol-4-yl)-2-oxo-pyrrolidine-3-carboxylic acid 3,5-difluorobenzyl amide |
| "A208" | 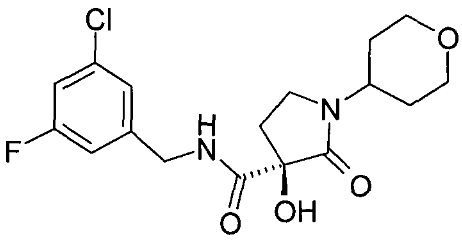 (R)-3-Hydroxy-2-oxo-1-(tetrahydropyran-4-yl)pyrrolidine-3-carboxylic acid 3-chloro-5-fluorobenzyl amide |

(continued)

| Compound No. | Structure/name |
|---|---|
| "A209" | 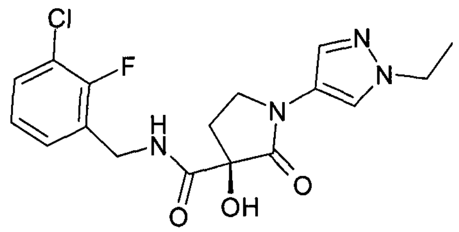 (R)-1-(1-Ethyl-1H-pyrazol-4-yl)-3-hydroxy-2-oxopyrrolidine-3-carboxylic acid 3-chloro-2-fluorobenzyl amide |
| "A210" | 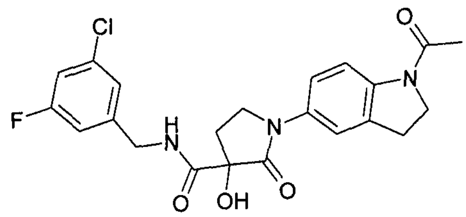 1-(1-Acetyl-2,3-dihydro-1H-indol-5-yl)-3-hydroxy-2-oxo-pyrrolidine-3-carboxylic acid 3-chloro-5-fluorobenzyl amide |
| "A211" | 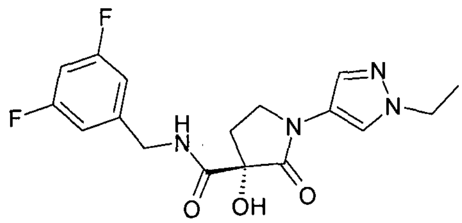 (S)-1-(1-Ethyl-1H-pyrazol-4-yl)-3-hydroxy-2-oxopyrrolidine-3-carboxylic acid 3,5-difluorobenzyl amide |
| "A212" | 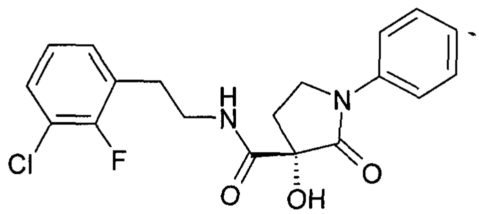 (S)-3-Hydroxy-2-oxo-1-phenylpyrrolidine-3-carboxylic acid [2-(3-chloro-2-fluorophenyl)ethyl] amide |
| "A213" | 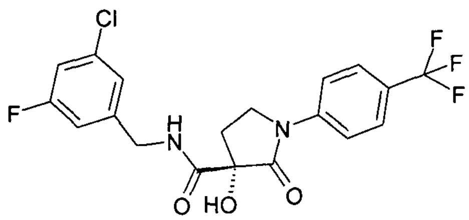 (S)-3-Hydroxy-2-oxo-1-(4-trifluoromethylphenyl)pyrrolidine-3-carboxylic acid 3-chloro-5-fluorobenzyl amide |

(continued)

| Compound No. | Structure/name |
|---|---|
| "A214" | 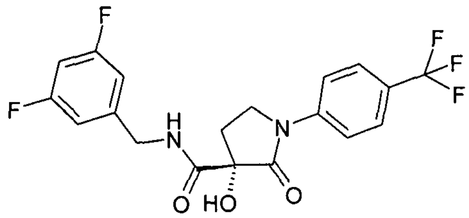 (S)-3-Hydroxy-2-oxo-1-(4-trifluoromethylphenyl)pyrrolidine-3-carboxylic acid 3,5-difluorobenzyl amide |
| "A215" | 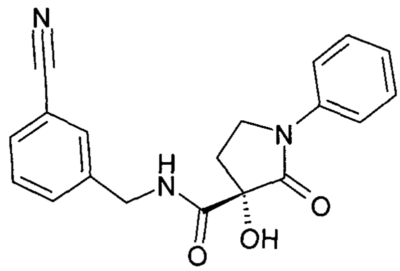 (S)-3-Hydroxy-2-oxo-1-phenylpyrrolidine-3-carboxylic acid 3-cyanobenzyl amide |
| "A216" | 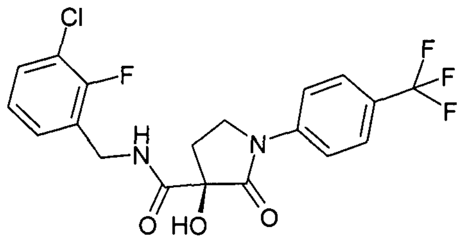 (R)-3-Hydroxy-2-oxo-1-(4-trifluoromethylphenyl)pyrrolidine-3-carboxylic acid 3-chloro-2-fluorobenzyl amide |
| "A217" | 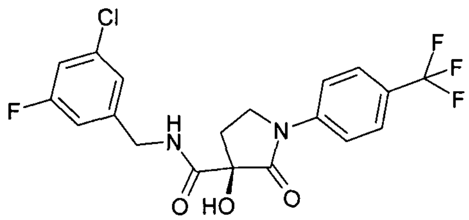 (R)-3-Hydroxy-2-oxo-1-(4-trifluoromethylphenyl)pyrrolidine-3-carboxylic acid 3-chloro-5-fluorobenzyl amide |
| "A218" | 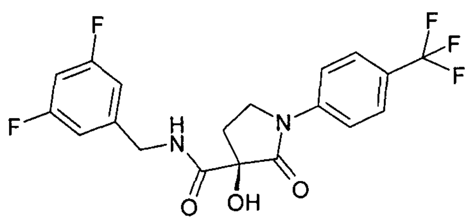 (R)-3-Hydroxy-2-oxo-1-(4-trifluoromethylphenyl)pyrrolidine-3-carboxylic acid 3,5-difluorobenzyl amide |

(continued)

| Compound No. | Structure/name |
|---|---|
| "A219" | 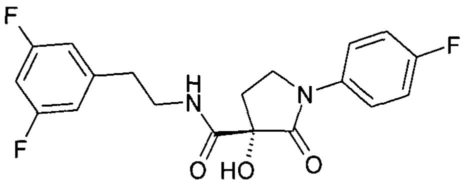 (S)-1-(4-Fluorophenyl)-3-hydroxy-2-oxopyrrolidine-3-carboxylic acid [2-(3,5-difluorophenyl)ethyl] amide |
| "A220" | 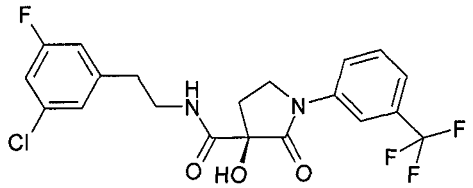 (R)-3-Hydroxy-2-oxo-1-(3-trifluoromethylphenyl)pyrrolidine-3-carboxylic acid [2-(3-chloro-5-fluorophenyl)ethyl] amide |
| "A221" | 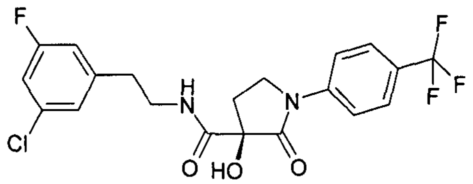 (R)-3-Hydroxy-2-oxo-1-(4-trifluoromethylphenyl)pyrrolidine-'3-carboxylic acid [2-(3-chloro-5-fluorophenyl)ethyl] amide |
| "A222" | 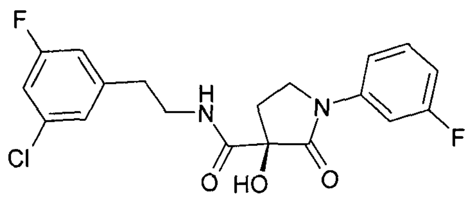 (R)-1-(3-Fluorophenyl)-3-hydroxy-2-oxopyrrolidine-3-carboxylic acid [2-(3-chloro-5-fluorophenyl)ethyl] amide |
| "A223" | 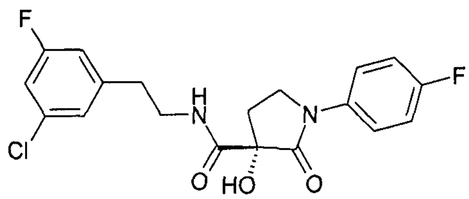 (S)-1-(4-Fluorophenyl)-3-hydroxy-2-oxopyrrolidine-3-carboxylic acid [2-(3-chloro-5-fluorophenyl)ethyl] amide |
| "A224" | 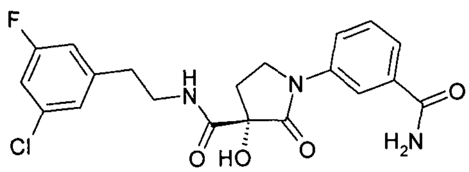 (S)-1-(3-Carbamoylphenyl)-3-hydroxy-2-oxopyrrolidine-3-carboxylic acid [2-(3-chloro-5-fluorophenyl)ethyl] amide |

(continued)

| Compound No. | Structure/name |
|---|---|
| "A225" | 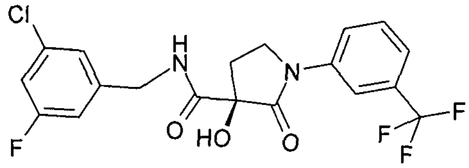 (R)-3-Hydroxy-2-oxo-1-(3-trifluoromethylphenyl)pyrrolidine-3-carboxylic acid 3-chloro-5-fluorobenzyl amide |
| "A226" | 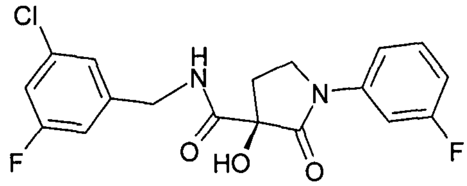 (R)-1-(3-Fluorophenyl)-3-hydroxy-2-oxopyrrolidine-3-carboxylic acid 3-chloro-5-fluorobenzyl amide |
| "A227" | 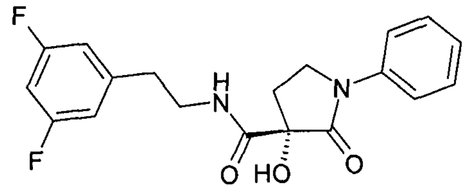 (S)-3-Hydroxy-2-oxo-1-phenylpyrrolidine-3-carboxylic acid [2-(3,5-difluorophenyl)ethyl] amide |
| "A228" | 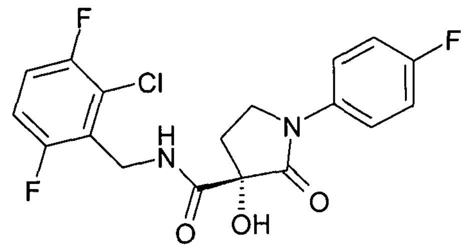 (S)-1-(4-Fluorophenyl)-3-hydroxy-2-oxopyrrolidine-3-carboxylic acid 2-chloro-3,6-difluorobenzyl amide |
| "A229" | 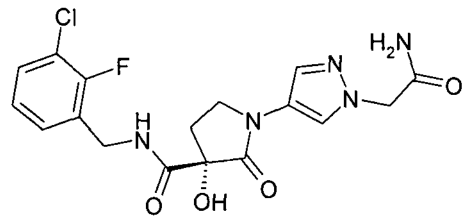 (S)-1-(1-Carbamoylmethyl-1H-pyrazol-4-yl)-3-hydroxy-2-oxopyrrolidine-3-carboxylic acid 3-chloro-2-fluorobenzyl amide |
| "A230" | 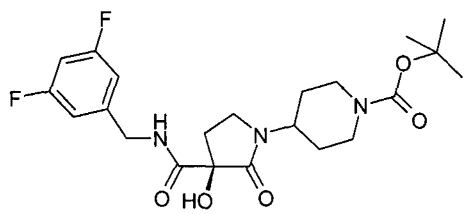 Tert-Butyl 4-[(R)-3-(3,5-difluorobenzylcarbamoyl)-3-hydroxy-2-oxopyrrolidin-1-yl]piperidine-1-carboxylate |

(continued)

| Compound No. | Structure/name |
|---|---|
| "A231" | 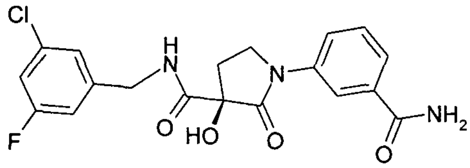<br>(R)-1-(3-Carbamoylphenyl)-3-hydroxy-2-oxopyrrolidine-3-carboxylic acid 3-chloro-5-fluorobenzyl amide |
| "A232" | 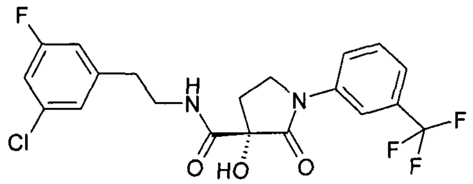<br>(S)-3-Hydroxy-2-oxo-1-(3-trifluoromethylphenyl)pyrrolidine-3-carboxylic acid [2-(3-chloro-5-fluorophenyl)ethyl] amide |
| "A233" | 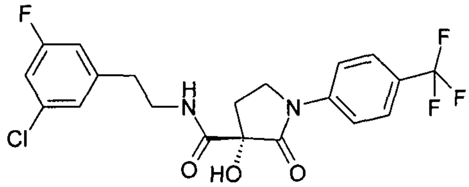<br>(S)-3-Hydroxy-2-oxo-1-(4-trifluoromethylphenyl)pyrrolidine-3-carboxylic acid [2-(3-chloro-5-fluorophenyl)ethyl] amide |
| "A234" | 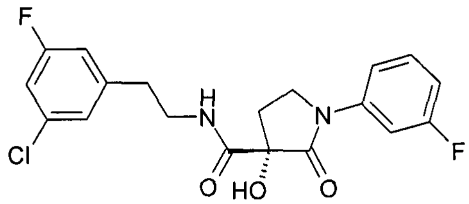<br>(S)-1-(3-Fluorophenyl)-3-hydroxy-2-oxopyrrolidine-3-carboxylic acid [2-(3-chloro-5-fluorophenyl)ethyl] amide |
| "A235" | 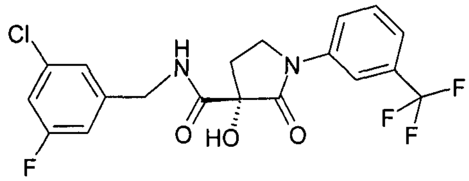<br>(S)-3-Hydroxy-2-oxo-1-(3-trifluoromethylphenyl)pyrrolidine-3-carboxylic acid 3-chloro-5-fluorobenzyl amide |
| "A236" | 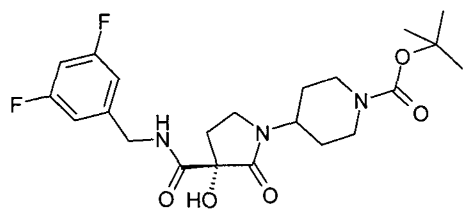<br>Tert-Butyl 4-[(S)-3-(3,5-difluorobenzylcarbamoyl)-3-hydroxy-2-oxopyrrolidin-1-yl]piperidine-1-carboxylate |

(continued)

| Compound No. | Structure/name |
|---|---|
| "A237" | 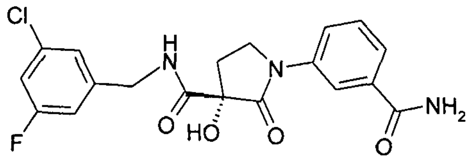 (S)-1-(3-Carbamoylphenyl)-3-hydroxy-2-oxopyrrolidine-3-carboxylic acid 3-chloro-5-fluorobenzyl amide |
| "A238" | 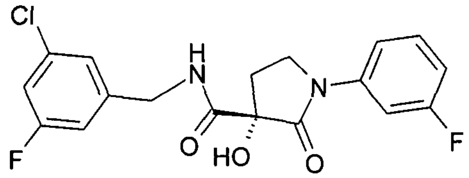 (S)-1-(3-Fluorophenyl)-3-hydroxy-2-oxopyrrolidine-3-carboxylic acid 3-chloro-5-fluorobenzyl amide |
| "A239" | 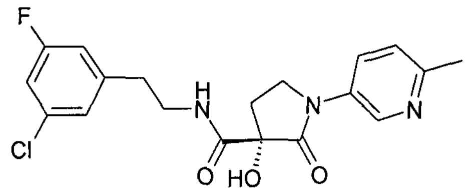 (S)-3-Hydroxy-1-(6-methylpyridin-3-yl)-2-oxopyrrolidine-3-carboxylic acid [2-(3-chloro-5-fluorophenyl)ethyl] amide |
| "A240" | 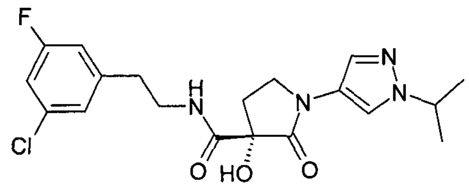 (S)-3-Hydroxy-1-(1-isopropyl-1H-pyrazol-4-yl)-2-oxo-pyrrolidine-3-carboxylic acid [2-(3-chloro-5-fluorophenyl)-ethyl] amide |
| "A241" | 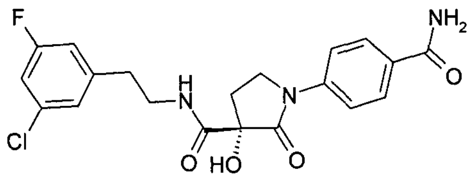 (S)-1-(4-Carbamoylphenyl)-3-hydroxy-2-oxopyrrolidine-3-carboxylic acid [2-(3-chloro-5-fluorophenyl)ethyl] amide |
| "A242" | 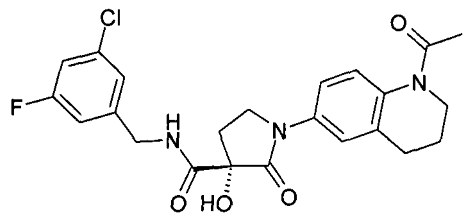 (S)-1-(1-Acetyl-1,2,3,4-tetrahydroquinolin-6-yl)-3-hydroxy-2-oxopyrrolidine-3-carboxylic acid 3-chloro-5-fluorobenzyl amide |

(continued)

| Compound No. | Structure/name |
|---|---|
| "A243" | 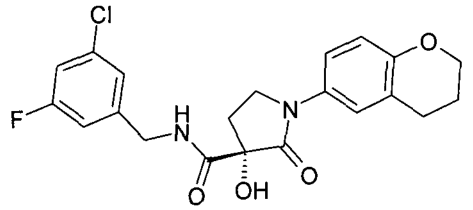 (S)-1-Chroman-6-yl-3-hydroxy-2-oxopyrrolidine-3-carboxylic acid 3-chloro-5-fluorobenzyl amide |
| "A244" | 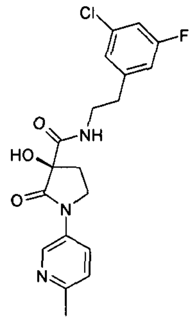 (R)-3-Hydroxy-1-(6-methylpyridin-3-yl)-2-oxopyrrolidine-3-carboxylic acid [2-(3-chloro-5-fluorophenyl)ethyl] amide |
| "A245" | 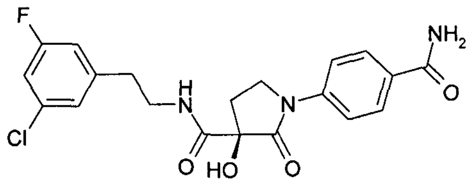 (R)-1-(4-Carbamoylphenyl)-3-hydroxy-2-oxopyrrolidine-3-carboxylic acid [2-(3-chloro-5-fluorophenyl)ethyl] amide |
| "A246" | 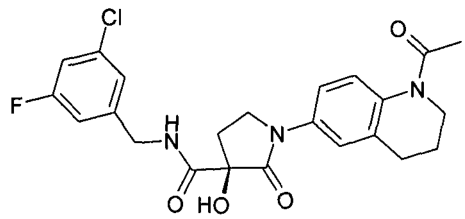 (R)-1-(1-Acetyl-1,2,3,4-tetrahydroquinolin-6-yl)-3-hydroxy-2-oxopyrrolidine-3-carboxylic acid 3-chloro-5-fluorobenzyl amide |
| "A247" | 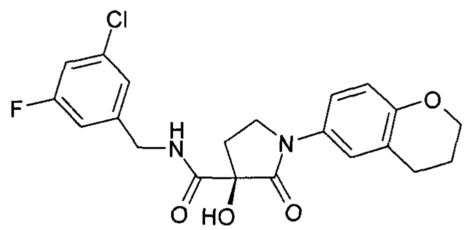 (R)-1-Chroman-6-yl-3-hydroxy-2-oxopyrrolidine-3-carboxylic acid 3-chloro-5-fluorobenzyl amide |

(continued)

| Compound No. | Structure/name |
|---|---|
| "A248" | 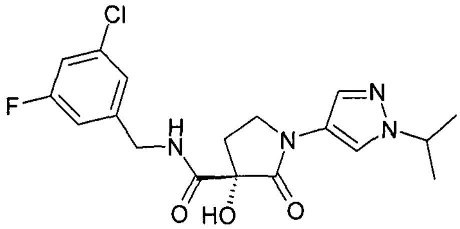<br>(S)-3-Hydroxy-1-(1-isopropyl-1H-pyrazol-4-yl)-2-oxo-pyrrolidine-3-carboxylic acid 3-chloro-5-fluorobenzyl amide |
| "A249" | 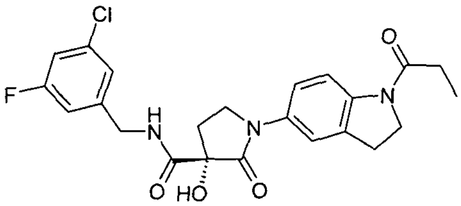<br>(S)-3-Hydroxy-2-oxo-1-(1-propionyl-2,3-dihydro-1H-indol-5-yl)pyrrolidine-3-carboxylic acid 3-chloro-5-fluorobenzyl amide |
| "A250" | 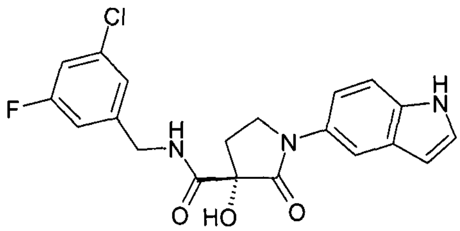<br>(S)-3-Hydroxy-1-(1H-indol-5-yl)-2-oxopyrrolidine-3-carboxylic acid 3-chloro-5-fluorobenzyl amide |
| "A251" | 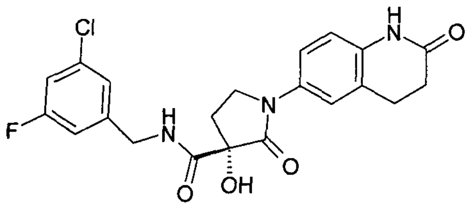<br>(S)-3-Hydroxy-2-oxo-1-(2-oxo-1,2,3,4-tetrahydroquinolin-6-yl)pyrrolidine-3-carboxylic acid 3-chloro-5-fluorobenzyl amide |
| "A252" | 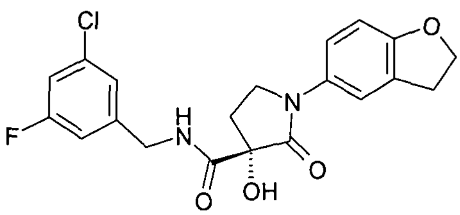<br>(S)-1-(2,3-Dihydrobenzofuran-5-yl)-3-hydroxy-2-oxo-pyrrolidine-3-carboxylic acid 3-chloro-5-fluorobenzyl amide |

(continued)

| Compound No. | Structure/name |
|---|---|
| "A253" | 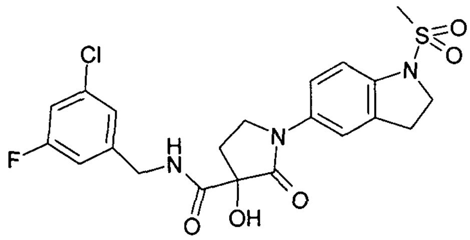 3-Hydroxy-1-(1-methanesulfonyl-2,3-dihydro-1H-indol-5-yl)-2-oxopyrrolidine-3-carboxylic acid 3-chloro-5-fluorobenzyl amide |
| "A254" | 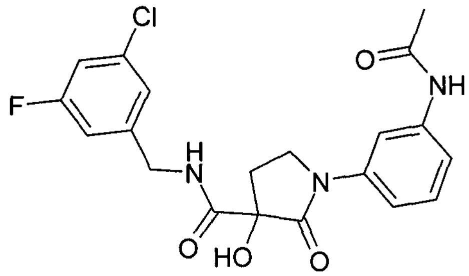 1-(3-Acetylaminophenyl)-3-hydroxy-2-oxopyrrolidine-3-carboxylic acid 3-chloro-5-fluorobenzyl amide |
| "A255" | 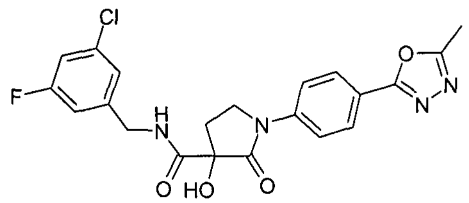 3-Hydroxy-1-[4-(5-methyl-1,3,4-oxadiazol-2-yl)-phenyl]-2-oxopyrrolidine-3-carboxylic acid 3-chloro-5-fluorobenzyl amide |
| "A256" | 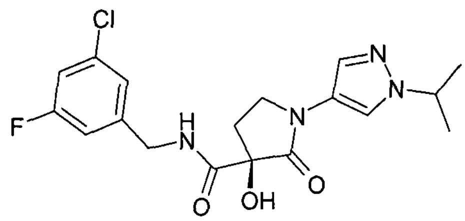 (R)-3-Hydroxy-1-(1-isopropyl-1H-pyrazol-4-yl)-2-oxo-pyrrolidine-3-carboxylic acid 3-chloro-5-fluorobenzyl amide |
| "A257" | 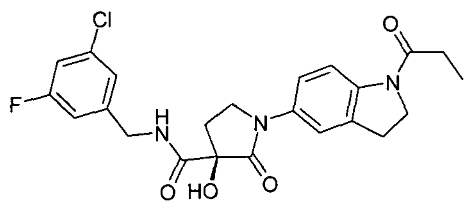 (R)-3-Hydroxy-2-oxo-1-(1-propionyl-2,3-dihydro-1H-indol-5-yl)pyrrolidine-3-carboxylic acid 3-chloro-5-fluorobenzyl amide |

(continued)

| Compound No. | Structure/name |
|---|---|
| "A258" | 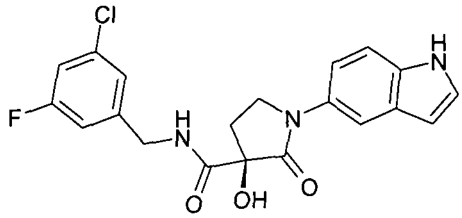<br>(R)-3-Hydroxy-1-(1H-indol-5-yl)-2-oxopyrrolidine-3-carboxylic acid 3-chloro-5-fluorobenzyl amide |
| "A259" | 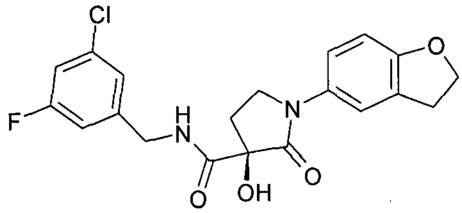<br>(R)-1-(2,3-Dihydrobenzofuran-5-yl)-3-hydroxy-2-oxo-pyrrolidine-3-carboxylic acid 3-chloro-5-fluorobenzyl amide |
| "A260" | 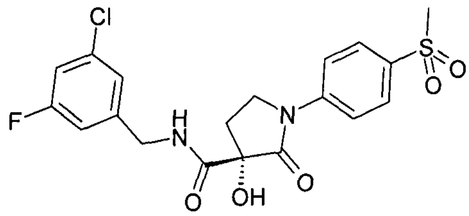<br>(S)-3-Hydroxy-1-(4-methanesulfonylphenyl)-2-oxo-pyrrolidine-3-carboxylic acid 3-chloro-5-fluorobenzyl amide |
| "A261" | 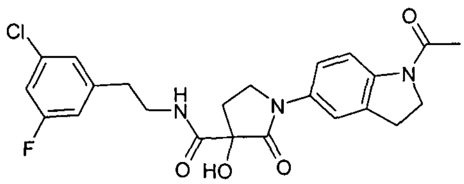<br>1-(1-Acetyl-2,3-dihydro-1H-indol-5-yl)-3-hydroxy-2-oxo-pyrrolidine-3-carboxylic acid [2-(3-chloro-5-fluorophenyl)-ethyl] amide |
| "A262" | 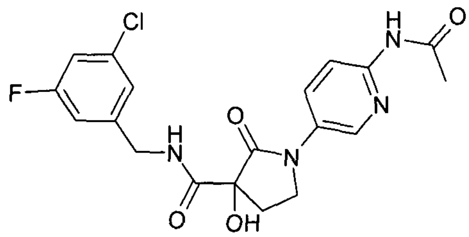<br>1-(6-Acetylaminopyridin-3-yl)-3-hydroxy-2-oxopyrrolidine-3-carboxylic acid 3-chloro-5-fluorobenzyl amide |
| "A263" | 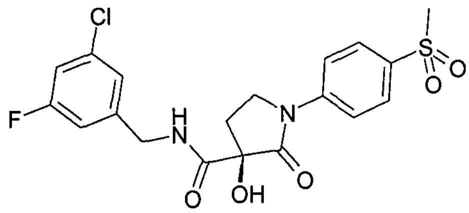 |

(continued)

| Compound No. | Structure/name |
|---|---|
| | (R)-3-Hydroxy-1-(4-methanesulfonylphenyl)-2-oxo-pyrrolidine-3-carboxylic acid 3-chloro-5-fluorobenzyl amide |
| "A264" | 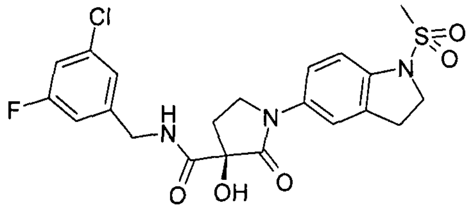 (R)-3-Hydroxy-1-(1-methanesulfonyl-2,3-dihydro-1H-indol-5-yl)-2-oxopyrrolidine-3-carboxylic acid 3-chloro-5-fluorobenzyl amide |
| "A265" | 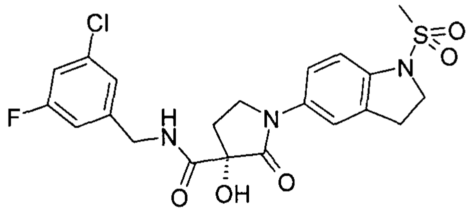 (S)-3-Hydroxy-1-(1-methanesulfonyl-2,3-dihydro-1H-indol-5-yl)-2-oxopyrrolidine-3-carboxylic acid 3-chloro-5-fluorobenzyl amide |
| "A266" | 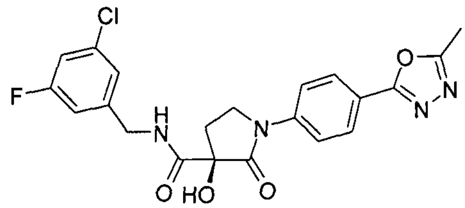 (R)-3-Hydroxy-1-[4-(5-methyl-1,3,4-oxadiazol-2-yl)-phenyl]-2-oxopyrrolidine-3-carboxylic acid 3-chloro-5-fluorobenzyl amide |
| "A267" | 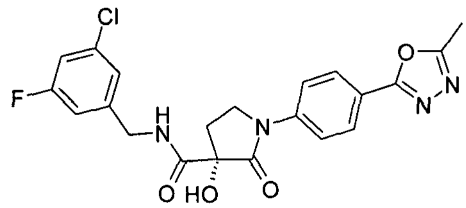 (S)-3-Hydroxy-1-(1-methanesulfonyl-2,3-dihydro-1H-indol-5-yl)-2-oxopyrrolidine-3-carboxylic acid 3-chloro-5-fluorobenzyl amide |
| "A268" | 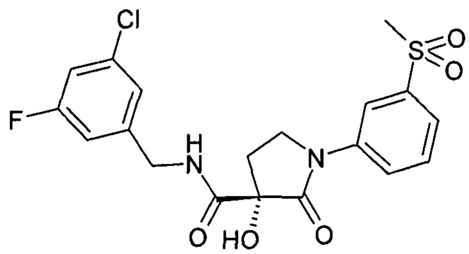 (S)-3-Hydroxy-1-(3-methanesulfonylphenyl)-2-oxo-pyrrolidine-3-carboxylic acid 3-chloro-5-fluorobenzyl amide |

(continued)

| Compound No. | Structure/name |
|---|---|
| "A269" | 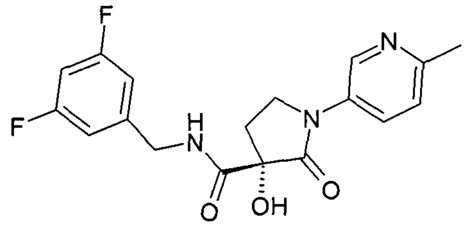 (S)-3-Hydroxy-1-(6-methylpyridin-3-yl)-2-oxopyrrolidine-3-carboxylic acid 3,5-difluorobenzyl amide |
| "A270" | 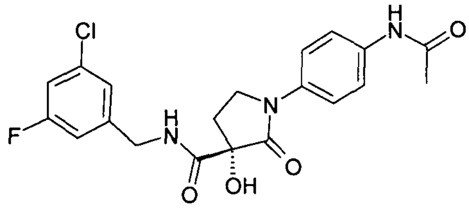 (S)-1-(4-Acetylaminophenyl)-3-hydroxy-2-oxopyrrolidine-3-carboxylic acid 3-chloro-5-fluorobenzyl amide |
| "A271" | 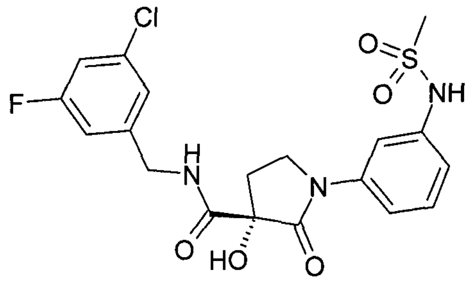 (S)-3-Hydroxy-1-(3-methanesulfonylaminophenyl)-2-oxo-pyrrolidine-3-carboxylic acid 3-chloro-5-fluorobenzyl amide |
| "A272" | 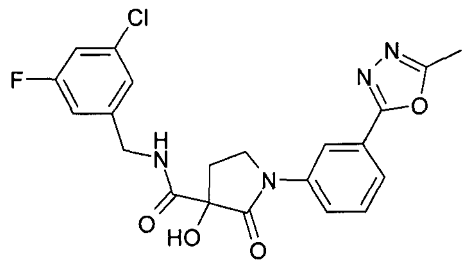 3-Hydroxy-1-[3-(5-methyl-1,3,4-oxadiazol-2-yl)-phenyl]-2-oxopyrrolidine-3-carboxylic acid 3-chloro-5-fluorobenzyl amide |
| "A273" | 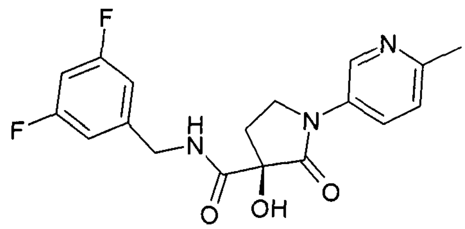 (R)-3-Hydroxy-1-(6-methylpyridin-3-yl)-2-oxopyrrolidine-3-carboxylic acid 3,5-difluorobenzyl amide |

(continued)

| Compound No. | Structure/name |
|---|---|
| "A274" | 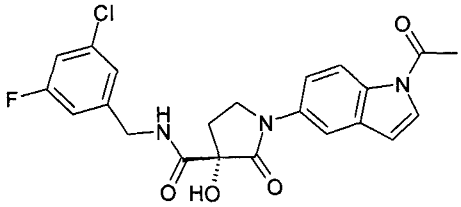 (S)-1-(1-Acetyl-1H-indol-5-yl)-3-hydroxy-2-oxopyrrolidine-3-carboxylic acid 3-chloro-5-fluorobenzyl amide |
| "A275" | 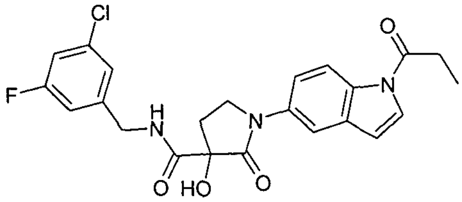 3-Hydroxy-2-oxo-1-(1-propionyl-1H-indol-5-yl)pyrrolidine-3-carboxylic acid 3-chloro-5-fluorobenzyl amide |
| "A276" | 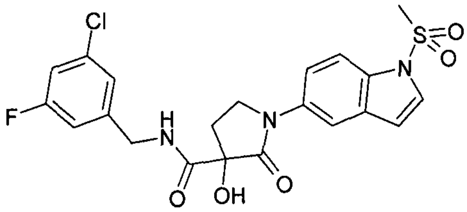 3-Hydroxy-1-(1-methanesulfonyl-1H-indol-5-yl)-2-oxo-pyrrolidine-3-carboxylic acid 3-chloro-5-fluorobenzyl amide |
| "A277" | 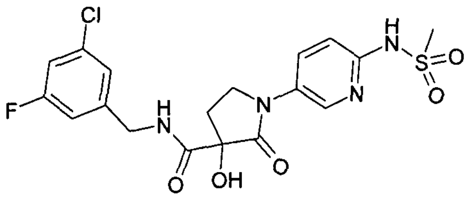 3-Hydroxy-1-(6-methanesulfonylaminopyridin-3-yl)-2-oxo-pyrrolidine-3-carboxylic acid 3-chloro-5-fluorobenzyl amide |
| "A278" | 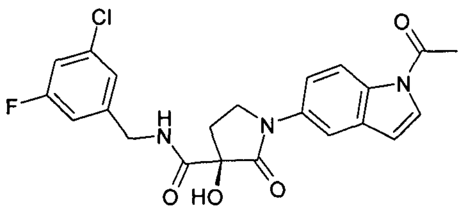 (R)-1-(1-Acetyl-1H-indol-5-yl)-3-hydroxy-2-oxopyrrolidine-3-carboxylic acid 3-chloro-5-fluorobenzyl amide |
| "A279" | 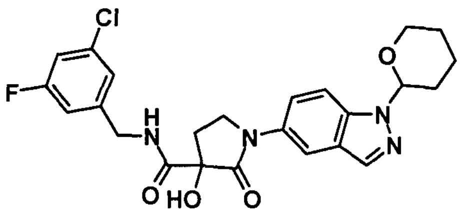 |

(continued)

| Compound No. | Structure/name |
|---|---|
| | 3-Hydroxy-2-oxo-1-[1-(tetrahydropyran-2-yl)-1H-indazol-5-yl]pyrrolidine-3-carboxylic acid 3-chloro-5-fluorobenzyl amide |
| "A280" | 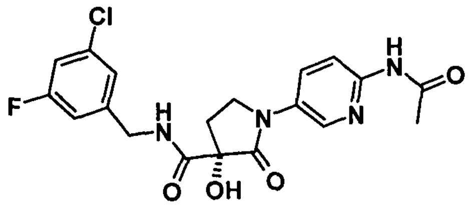 (S)-1-(6-Acetylaminopyridin-3-yl)-3-hydroxy-2-oxo-pyrrolidine-3-carboxylic acid 3-chloro-5-fluorobenzyl amide |
| "A281" | 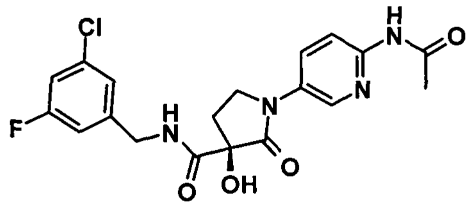 (R)-1-(6-Acetylaminopyridin-3-yl)-3-hydroxy-2-oxo-pyrrolidine-3-carboxylic acid 3-chloro-5-fluorobenzyl amide |
| "A282" | 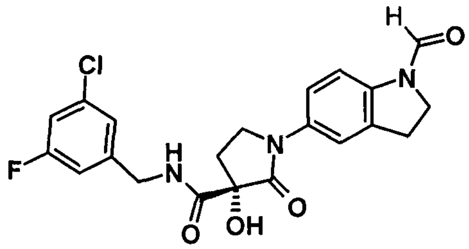 (S)-1-(1-Formyl-2,3-dihydro-1H-indol-5-yl)-3-hydroxy-2-oxopyrrolidine-3-carboxylic acid 3-chloro-5-fluorobenzyl amide |
| "A283" | 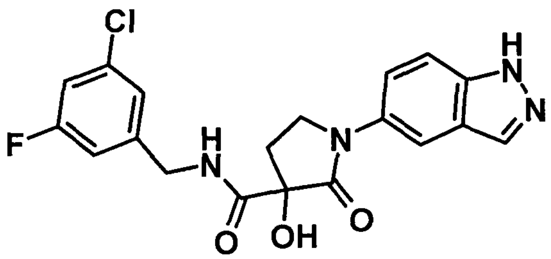 3-Hydroxy-1-(1H-indazol-5-yl)-2-oxopyrrolidine-3-carboxylic acid 3-chloro-5-fluorobenzyl amide |
| "A284" | 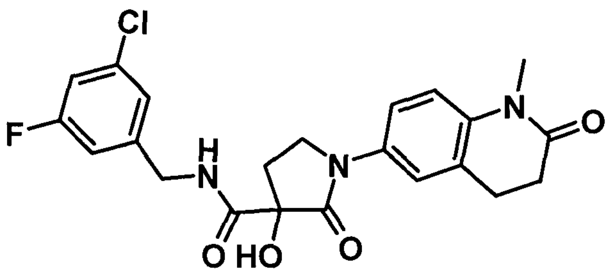 3-Hydroxy-1-(1-methyl-2-oxo-1,2,3,4-tetrahydroquinolin-6-yl)-2-oxopyrrolidine-3-carboxylic acid 3-chloro-5-fluorobenzyl amide |

(continued)

| Compound No. | Structure/name |
|---|---|
| "A285" | 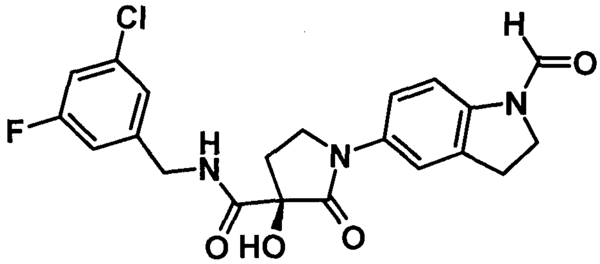 (R)-1-(1-Formyl-2,3-dihydro-1H-indol-5-yl)-3-hydroxy-2-oxopyrrolidine-3-carboxylic acid 3-chloro-5-fluorobenzyl amide |
| "A286" | 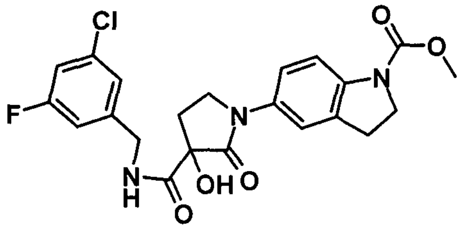 Methyl 5-[3-(3-chloro-5-fluorobenzylcarbamoyl)-3-hydroxy-2-oxopyrrolidin-1-yl]-2,3-dihydroindole-1-carboxylate |
| "A287" | 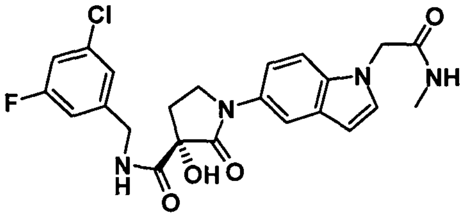 (S)-3-Hydroxy-1-(1-methylcarbamoylmethyl-1H-indol-5-yl)-2-oxopyrrolidine-3-carboxylic acid 3-chloro-5-fluorobenzyl amide |
| "A288" | 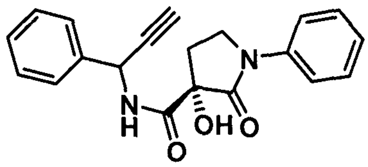 (S)-3-Hydroxy-2-oxo-1-phenylpyrrolidine-3-carboxylic acid (1-phenylprop-2-ynyl) amide |
| "A289" | 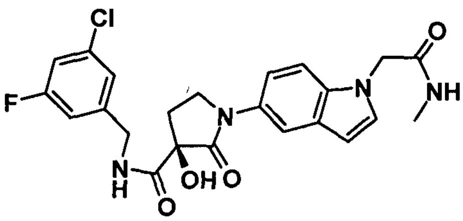 (R)-3-Hydroxy-1-(1-methylcarbamoylmethyl-1H-indol-5-yl)-2-oxopyrrolidine-3-carboxylic acid 3-chloro-5-fluorobenzyl amide |
| "A290" | 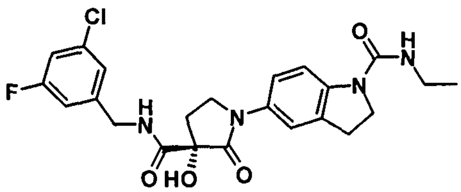 |

(continued)

| Compound No. | Structure/name |
|---|---|
| | 5-[(S)-3-(3-Chloro-5-fluorobenzylcarbamoyl)-3-hydroxy-2-oxopyrrolidin-1-yl]-2,3-dihydroindole-1-carboxylic acid ethyl amide |
| "A291" | 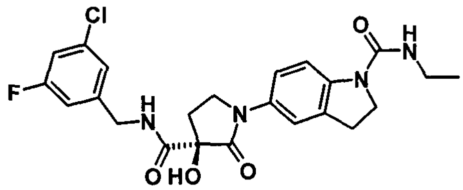 5-[(R)-3-(3-Chloro-5-fluorobenzylcarbamoyl)-3-hydroxy-2-oxopyrrolidin-1-yl]-2,3-dihydroindole-1-carboxylic acid ethyl amide |
| "A292" | 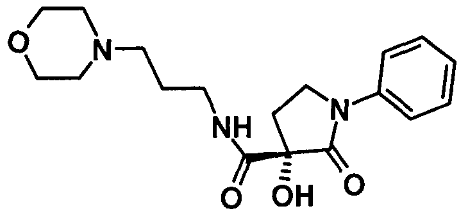 (S)-3-Hydroxy-2-oxo-1-phenylpyrrolidine-3-carboxylic acid (3-morpholin-4-ylpropyl) amide |
| "A293" | 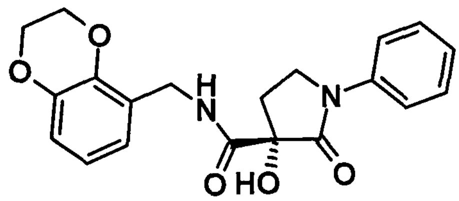 (S)-3-Hydroxy-2-oxo-1-phenylpyrrolidine-3-carboxylic acid (2,3-dihydrobenzo-1,4-dioxin-5-ylmethyl) amide |
| "A294" | 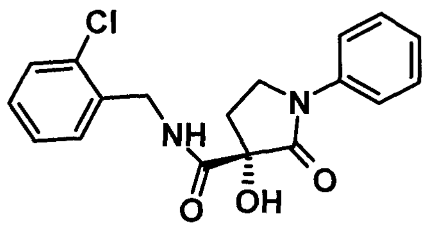 (S)-3-Hydroxy-2-oxo-1-phenylpyrrolidine-3-carboxylic acid 2-chlorobenzyl amide |
| "A295" | 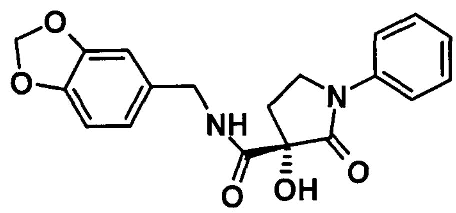 (S)-3-Hydroxy-2-oxo-1-phenylpyrrolidine-3-carboxylic acid (benzo-1,3-dioxol-5-ylmethyl) amide |
| "A296" | 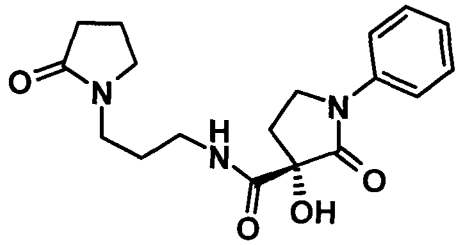 |

(continued)

| Compound No. | Structure/name |
|---|---|
| | (S)-3-Hydroxy-2-oxo-1-phenylpyrrolidine-3-carboxylic acid [3-(2-oxopyrrolidin-1-yl)-propyl] amide |
| "A297" | 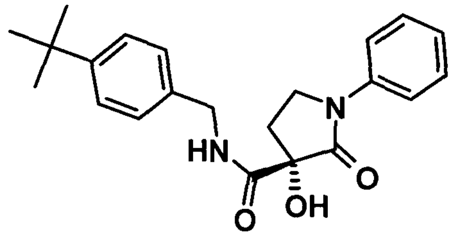 (S)-3-Hydroxy-2-oxo-1-phenylpyrrolidine-3-carboxylic acid 4-tert-butylbenzyl amide |
| "A298" | 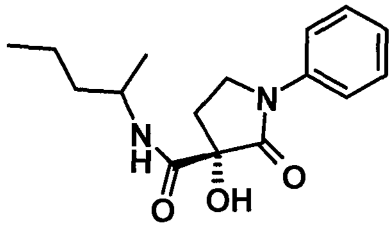 (S)-3-Hydroxy-2-oxo-1-phenylpyrrolidine-3-carboxylic acid (1-methylbutyl) amide |
| "A299" | 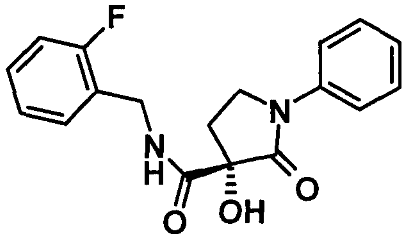 (S)-3-Hydroxy-2-oxo-1-phenylpyrrolidine-3-carboxylic acid 2-fluorobenzyl amide |
| "A300" | 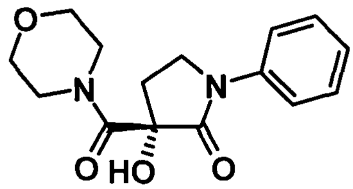 (S)-3-Hydroxy-3-(morpholin-4-carbonyl)-1-phenylpyrrolidin-2-one |
| "A301" | 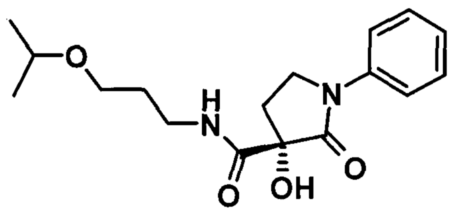 (S)-3-Hydroxy-2-oxo-1-phenylpyrrolidine-3-carboxylic acid (3-isopropoxypropyl) amide |
| "A302" | 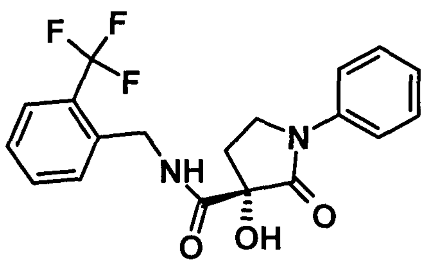 |

(continued)

| Compound No. | Structure/name |
|---|---|
| | (S)-3-Hydroxy-2-oxo-1-phenylpyrrolidine-3-carboxylic acid 2-trifluoromethylbenzyl amide |
| "A303" | 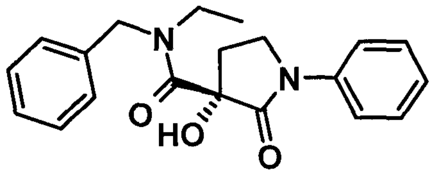 (S)-3-Hydroxy-2-oxo-1-phenylpyrrolidine-3-carboxylic acid benzyl ethyl amide |
| "A304" | 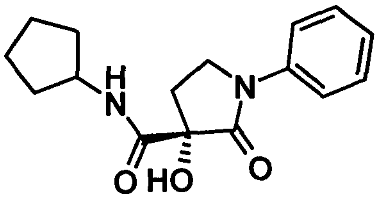 (S)-3-Hydroxy-2-oxo-1-phenylpyrrolidine-3-carboxylic acid cyclopentyl amide |
| "A305" | 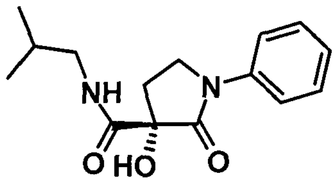 (S)-3-Hydroxy-2-oxo-1-phenylpyrrolidine-3-carboxylic acid isobutyl amide |
| "A306" | 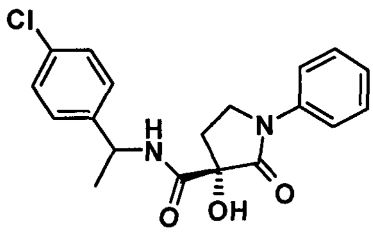 (S)-3-Hydroxy-2-oxo-1-phenylpyrrolidine-3-carboxylic acid [1-(4-chlorophenyl)ethyl] amide |
| "A307" | 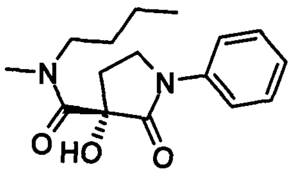 (S)-3-Hydroxy-2-oxo-1-phenylpyrrolidine-3-carboxylic acid butyl methyl amide |
| "A308" | 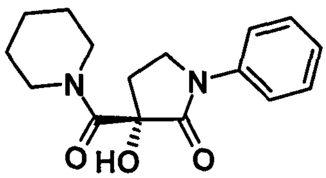 (S)-3-Hydroxy-1-phenyl-3-(piperidine-1-carbonyl)pyrrolidin-2-one |
| "A309" | 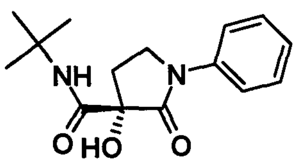 (S)-3-Hydroxy-2-oxo-1-phenylpyrrolidine-3-carboxylic acid tert-butyl amide |

(continued)

| Compound No. | Structure/name |
|---|---|
| "A310" | 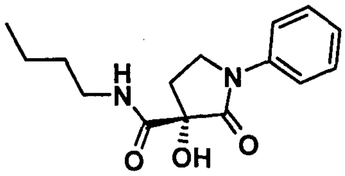<br>(S)-3-Hydroxy-2-oxo-1-phenylpyrrolidine-3-carboxylic acid butyl amide |
| "A311" | 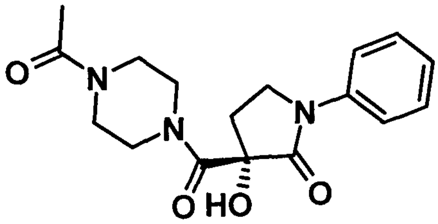<br>(S)-3-(4-Acetylpiperazine-1-carbonyl)-3-hydroxy-1-phenyl-pyrrolidin-2-one |
| "A312" | 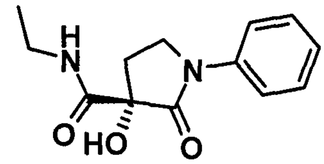<br>(S)-3-Hydroxy-2-oxo-1-phenylpyrrolidine-3-carboxylic acid ethyl amide |
| "A313" | 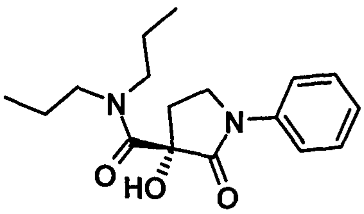<br>(S)-3-Hydroxy-2-oxo-1-phenylpyrrolidine-3-carboxylic acid dipropyl amide |
| "A314" | 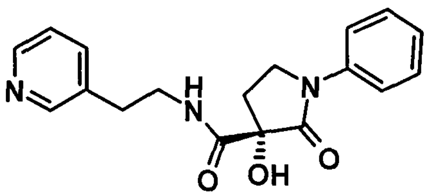<br>(S)-3-Hydroxy-2-oxo-1-phenylpyrrolidine-3-carboxylic acid (2-pyridin-3-ylethyl) amide |
| "A315" | 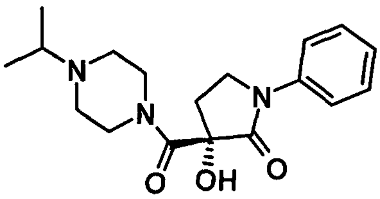<br>(S)-3-Hydroxy-3-(4-isopropylpiperazine-1-carbonyl)-1-phenylpyrrolidin-2-one |
| "A316" | 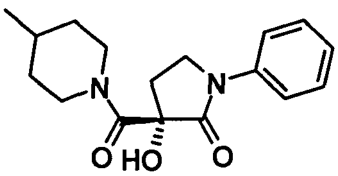 |

(continued)

| Compound No. | Structure/name |
|---|---|
| | (S)-3-Hydroxy-3-(4-methylpiperidine-1-carbonyl)-1-phenyl-pyrrolidin-2-one |
| "A317" | 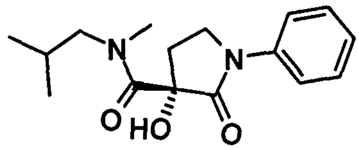 (S)-3-Hydroxy-2-oxo-1-phenylpyrrolidine-3-carboxylic acid isobutyl methyl amide |
| "A318" | 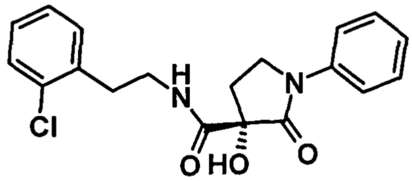 (S)-3-Hydroxy-2-oxo-1-phenylpyrrolidine-3-carboxylic acid [2-(2-chlorophenyl)ethyl] amide |
| "A319" | 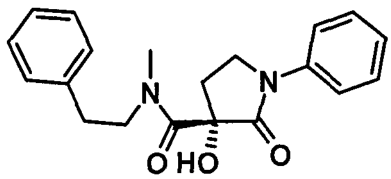 (S)-3-Hydroxy-2-oxo-1-phenylpyrrolidine-3-carboxylic acid methyl phenethyl amide |
| "A320" | 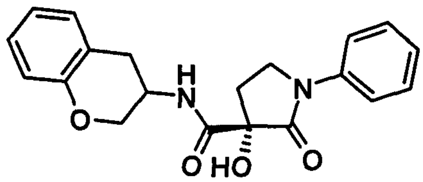 (S)-3-Hydroxy-2-oxo-1-phenylpyrrolidine-3-carboxylic acid chroman-3-yl amide |
| "A321" | 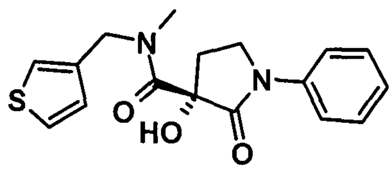 (S)-3-Hydroxy-2-oxo-1-phenylpyrrolidine-3-carboxylic acid methyl thiophen-3-ylmethyl amide |
| "A322" | 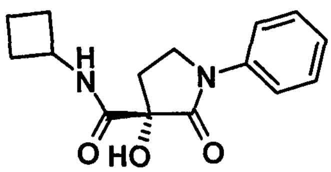 (S)-3-Hydroxy-2-oxo-1-phenylpyrrolidine-3-carboxylic acid cyclobutyl amide |
| "A323" | 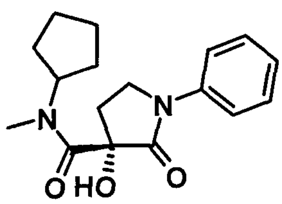 (S)-3-Hydroxy-2-oxo-1-phenylpyrrolidine-3-carboxylic acid cyclopentyl methyl amide |

(continued)

| Compound No. | Structure/name |
|---|---|
| "A324" | 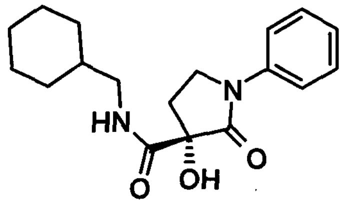 (S)-3-Hydroxy-2-oxo-1-phenylpyrrolidine-3-carboxylic acid cyclohexylmethyl amide |
| "A325" | 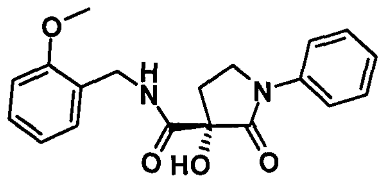 (S)-3-Hydroxy-2-oxo-1-phenylpyrrolidine-3-carboxylic acid 2-methoxybenzyl amide |
| "A326" | 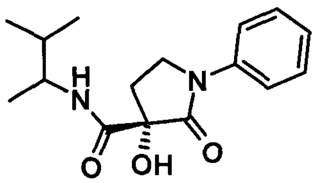 (S)-3-Hydroxy-2-oxo-1-phenylpyrrolidine-3-carboxylic acid (1,2-dimethylpropyl) amide |
| "A327" | 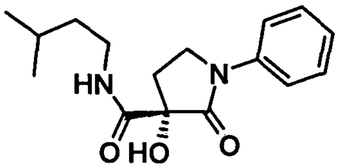 (S)-3-Hydroxy-2-oxo-1-phenylpyrrolidine-3-carboxylic acid (3-methylbutyl) amide |
| "A328" | 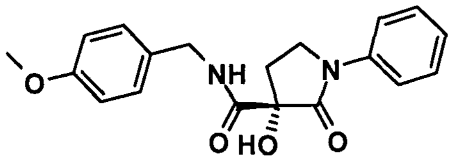 (S)-3-Hydroxy-2-oxo-1-phenylpyrrolidine-3-carboxylic acid 4-methoxybenzyl amide |
| "A329" | 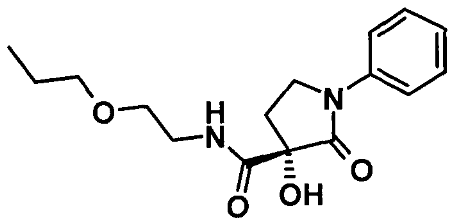 (S)-3-Hydroxy-2-oxo-1-phenylpyrrolidine-3-carboxylic acid (2-propoxyethyl) amide |

(continued)

| Compound No. | Structure/name |
| --- | --- |
| "A330" | 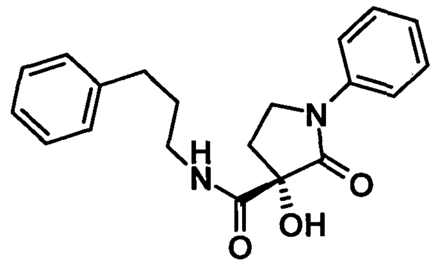<br>(S)-3-Hydroxy-2-oxo-1-phenylpyrrolidine-3-carboxylic acid (3-phenylpropyl) amide |
| "A331" | 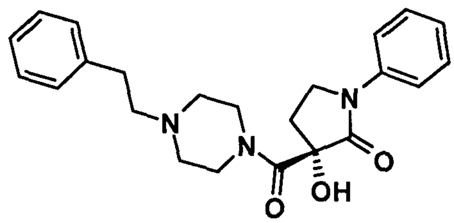<br>(S)-3-Hydroxy-3-(4-phenethylpiperazine-1-carbonyl)-1-phenylpyrrolidin-2-one |
| "A332" | 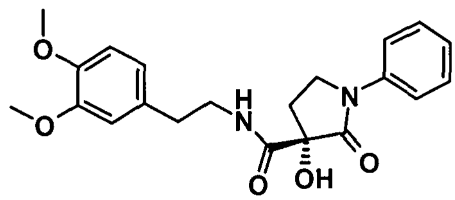<br>(S)-3-Hydroxy-2-oxo-1-phenylpyrrolidine-3-carboxylic acid [2-(3,4-dimethoxyphenyl)ethyl] amide |
| "A333" | 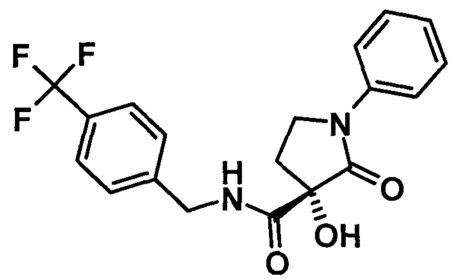<br>(S)-3-Hydroxy-2-oxo-1-phenylpyrrolidine-3-carboxylic acid 4-trifluoromethylbenzyl amide |
| "A334" | 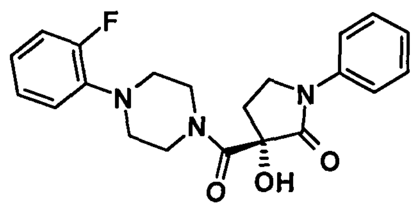<br>(S)-3-[4-(2-Fluorophenyl)piperazine-1-carbonyl]-3-hydroxy-1-phenylpyrrolidin-2-one |

(continued)

| Compound No. | Structure/name |
|---|---|
| "A335" | 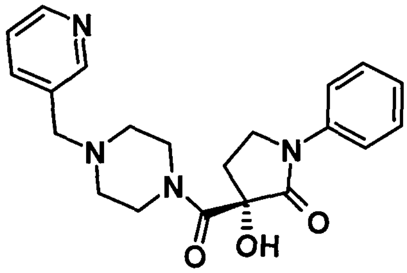 (S)-3-Hydroxy-1-phenyl-3-(4-pyridin-3-ylmethylpiperazine-1-carbonyl)pyrrolidin-2-one |
| "A336" | 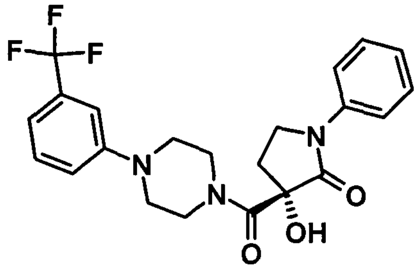 (S)-3-Hydroxy-1-phenyl-3-[4-(3-trifluoromethylphenyl)-piperazine-1-carbonyl]pyrrolidin-2-one |
| "A337" | 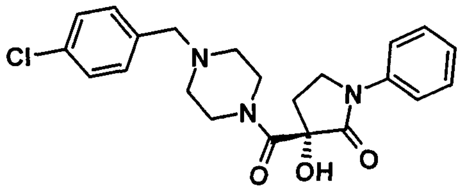 (S)-3-[4-(4-Chlorobenzyl)piperazine-1-carbonyl]-3-hydroxy-1-phenylpyrrolidin-2-one |
| "A338" | 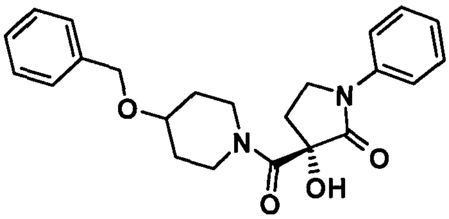 (S)-3-(4-Benzyloxypiperidine-1-carbonyl)-3-hydroxy-1-phenylpyrrolidin-2-one |
| "A339" | 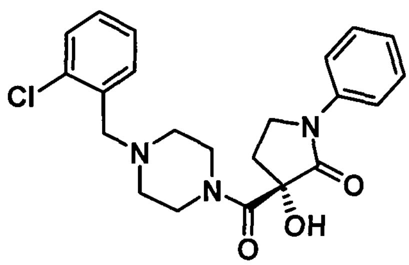 (S)-3-[4-(2-Chlorobenzyl)piperazine-1-carbonyl]-3-hydroxy-1-phenylpyrrolidin-2-one |
| "A340" | 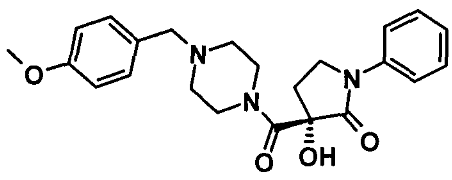 (S)-3-Hydroxy-3-[4-(4-methoxybenzyl)piperazine-1-carbonyl]-1-phenylpyrrolidin-2-one |

(continued)

| Compound No. | Structure/name |
|---|---|
| "A341" | 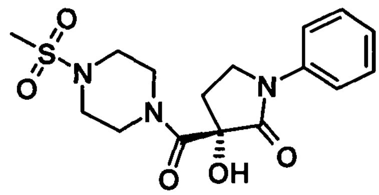 (S)-3-Hydroxy-3-(4-methanesulfonylpiperazine-1-carbonyl)-1-phenylpyrrolidin-2-on |
| "A342" | 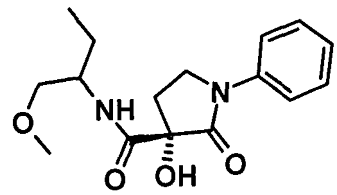 (S)-3-Hydroxy-2-oxo-1-phenylpyrrolidine-3-carboxylic acid (1-methoxymethylpropyl) amide |
| "A343" | 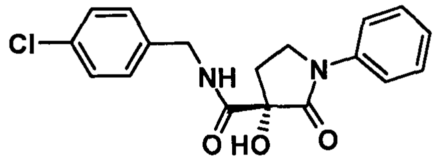 (S)-3-Hydroxy-2-oxo-1-phenylpyrrolidine-3-carboxylic acid 4-chlorobenzyl amide |
| "A344" | 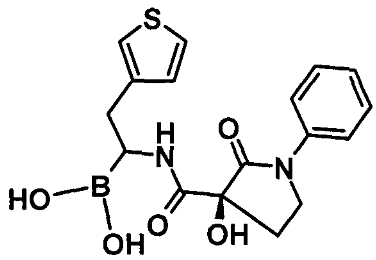 [1-[[(3S)-3-hydroxy-2-oxo-1-phenylpyrrolidine-3-carbonyl]-amino]-2-(3-thienyl)ethyl]boronic acid |
| "A345" | 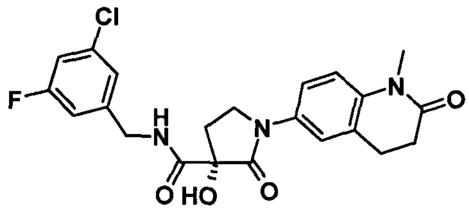 (S)-3-Hydroxy-1-(1-methyl-2-oxo-1,2,3,4-tetrahydro-quinolin-6-y!)-2-oxopyrrolidine-3-carboxylic acid 3-chloro-5-fluorobenzyl amide |
| "A346" | 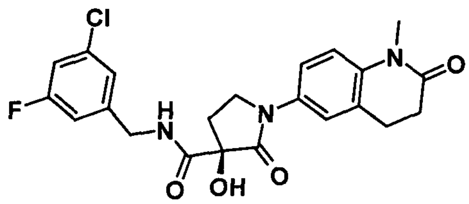 (R)-3-Hydroxy-1-(1-methyl-2-oxo-1,2,3,4-tetrahydro-quinolin-6-yl)-2-oxopyrrolidine-3-carboxylic acid 3-chloro-5-fluorobenzyl amide |

and pharmaceutically usable salts, tautomers and stereoisomers thereof, including mixtures thereof in all ratios.

3. Process for the preparation of compounds of the formula I according to Claims 1-2 and pharmaceutically usable salts, tautomers and stereoisomers thereof, **characterised in that**

a) a compound of the formula II

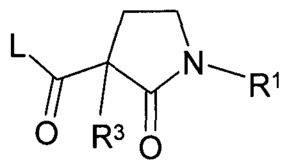

II

in which $R^1$ and $R^3$ have the meanings indicated in Claim 1,
and L denotes Cl, Br, I or a free or reactively functionally modified OH group,
is reacted with a compound of the formula III

$R^2$-$NHR^4$ III

in which $R^2$ and $R^4$ have the meanings indicated in Claim 1,
or
b) for the preparation of compounds of the formula I in which $R^3$ denotes OH,
a compound of the formula IV

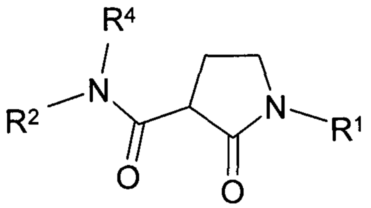

IV

in which $R^1$, $R^2$ and $R^4$ have the meanings indicated in Claim 1,
is oxidised,
or
c) a radical $R^3$ is converted into another radical $R^3$ by replacing an OH group with a halogen atom, or replacing a halogen atom with $N_3$,

and/or
a base or acid of the formula I is converted into one of its salts.

4. Medicaments comprising at least one compound of the formula I according to Claims 1-2 and/or pharmaceutically usable salts, tautomers and stereoisomers thereof, including mixtures thereof in all ratios, and optionally excipients and/or adjuvants.

5. Compounds of the formula I according to Claims 1-2,
and pharmaceutically usable salts, tautomers and stereoisomers thereof, including mixtures thereof in all ratios, for use for the treatment of tumours, tumour metastases, proliferative diseases of the mesangial cells, haemangioma, proliferative retinopathy, rheumatoid arthritis, atherosclerotic neovascularisation, psoriasis, ocular neovascularisation, osteoporosis, diabetes and obesity, lymphoid leukaemia, lymphoma, malaria and prostate hypertrophy.

6. Compounds according to Claim 5, where the tumour disease is selected from the group
of the squamous epithelium, of the bladder, of the stomach, of the kidneys, of head and neck, of the oesophagus, of the cervix, of the thyroid, of the intestine, of the liver, of the brain, of the prostate, of the urogenital tract, of the lymphatic system, of the stomach, of the larynx, of the lung, of the skin, monocytic leukaemia, lung adenocarcinoma, small-cell lung carcinoma, pancreatic cancer, glioblastoma, breast carcinoma, acute myeloid leukaemia, chronic myeloid leukaemia, acute lymphatic leukaemia, chronic lymphatic leukaemia, Hodgkin's lymphoma, non-Hodgkin's lymphoma.

7. Compounds of the formula I according to Claims 1-2 and/or physiologically acceptable salts thereof for use for the treatment of tumours, where a therapeutically effective amount of a compound of the formula I is administered in combination with a compound from the group 1) oestrogen receptor modulator, 2) androgen receptor modulator, 3) retinoid receptor modulator, 4) cytotoxic agent, 5) antiproliferative agent, 6) prenyl-protein transferase inhibitor, 7) HMG-CoA reductase inhibitor, 8) HIV protease inhibitor, 9) reverse transcriptase inhibitor and 10) further angiogenesis inhibitors.

8. Compounds of the formula I according to Claims 1-2 and/or physiologically acceptable salts thereof for use for the treatment of tumours, where a therapeutically effective amount of a compound of the formula I is administered in combination with radiotherapy and a compound from the group 1) oestrogen receptor modulator, 2) androgen receptor modulator, 3) retinoid receptor modulator, 4) cytotoxic agent, 5) antiproliferative agent, 6) prenyl-protein transferase inhibitor, 7) HMG-CoA reductase inhibitor, 8) HIV protease inhibitor, 9) reverse transcriptase inhibitor and 10) further angiogenesis inhibitors.

## Revendications

1. Composés de formule I

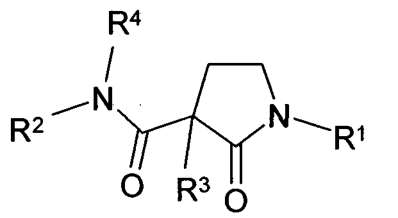

I

dans laquelle

$R^1$ désigne phényle, benzyle, naphtyle ou biphényle, chacun d'entre eux étant non substitué ou mono-, di-, tri-, tétra- ou penta-substitué par Hal, CN, NHCOA, $NHSO_2A$, $SO_2A$ et/ou $CONH_2$;
A ou $(CH_2)_n$Hét,
$R^2$ désigne $[C(R^4)_2]_nAr^2$, $(CH_2)_n$Cyc, $CH[B(OH)_2]CH_2$Hét,

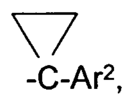

CH(C≡CH)phényle, A ou $(CH_2)_n$Hét,
$R^3$ désigne OH, $N_3$, $NH_2$ ou F,
$R^4$ désigne H ou alkyle ayant 1, 2, 3 ou 4 atomes de C,
$R^2$ et $R^4$ pris ensemble, désignent aussi alkylène ayant 2, 3, 4 ou 5 atomes de C, où un groupement $CH_2$ peut aussi être remplacé par NH, NA, N-COA, $N-(CH_2)_nAr^3$, $N-(CH_2)_n$Hét$^2$, CH-A, $CH-O-(CH_2)_nAr^3$, $N-SO_2A$ ou O et/ou peut être substitué par A,
Hét désigne pyridazinyle, pyrazolyle, benzimidazolyle, pyridyle, dibenzofuranyle, carbazolyle, indolyle, dihydroindolyle, benzofuranyle, dihydrobenzofuranyle, 2,3-dihydrobenzo-1,4-dioxinyle, chromanyle, pipérazinyle, morpholinyle, tétrahydropyranyle, quinoléinyle, isoquinoléinyle, isoindolyle, dihydroquinoléinyle, dihydroisoquinoléinyle, tétrahydroquinoléinyle, tétrahydroisoquinoléinyle, quinazolinyle, quinoxalinyle, phtalazinyle, purinyle, naphtyridinyle, pyrimidinyle, indazolyle, furyle, thiényle, imidazolyle, pyrrolyle, oxazolyle, oxadiazolyle, isoxazolyle, thiazolyle, triazolyle, tétrazolyle, thiadiazole, benzothiazolyle, imidazo[1,2-a]pyridinyle, 1,3-benzodioxolyle, benzoxazolyle, pipéridin-1-yle, pyrrolidin-1-yle, 1,2-oxazinan-2-yle, 1,2,5-oxadiazinan-2-yle, 1,3-oxa-zinan-3-yle ou hexahydropyrimidinyle, chacun d'entre eux étant non substitué ou mono-, di- ou trisubstitué par A, OA, COOA, COA, CHO, $(CH_2)_nCONH_2$, $(CH_2)_n$CONHA, $(CH_2)_nCONA_2$, $SO_2A$, $NHSO_2A$, =O et/ou Hét$^1$,
Hét$^1$ désigne pyridazinyle, pyrazolyle, pyridyle, pipérazinyle, morpholinyle, pyrimidinyle, furyle, thiényle, imidazolyle, pyrrolyle, oxazolyle, isoxazolyle, thiazolyle, triazolyle, tétrazolyle, thiadiazole, pipéridin-1-yle, pyrrolidin-1-yle, tétrahydropyranyle, 1,2-oxazinan-2-yle, 1,2,5-oxadiazinan-2-yle, 1,3-oxazinan-3-yle ou hexahydropyrimidinyle, chacun d'entre eux étant non substitué ou mono-, di- ou trisubstitué par A et/ou OA,
Hét$^2$ désigne pyridyle, pyrimidinyle, furyle, thiényle, imidazolyle, pyrrolyle, oxazolyle, oxadiazolyle, isoxazolyle,

thiazolyle, triazolyle, tétrazolyle ou thiadiazole,

A désigne alkyle ramifié ou non ramifié ayant 1-10 atomes de C, où 1-7 atomes de H peuvent être remplacés par F, Cl, Br, OH, CHO, COA, COOA, CN, $CONA_2$, CONHA et/ou $CONH_2$,

et/ou où un ou deux groupements CH et/ou $CH_2$ non adjacents peuvent être remplacés par O, N et/ou $NR^4$, ou Cyc,

$Ar^2$ désigne phényle qui est non substitué ou mono-, di-, tri-, tétra- ou penta-substitué par A, Hal, CN, OH et/ou OA,

$Ar^3$ désigne phényle qui est non substitué ou mono-, di- ou trisubstitué par Hal, OH, OA et/ou A,

Cyc désigne alkyle cyclique ayant 3-7 atomes de C,

Hal désigne F, Cl, Br ou I,

n désigne 0, 1, 2, 3 ou 4,

ainsi que les sels, tautomères et stéréoisomères pharmaceutiquement utilisables de ceux-ci, y compris des mélanges de ceux-ci en toutes proportions.

**2.** Composés selon la revendication 1, choisis dans le groupe constitué par

| Composé n° | Structure/nom |
|---|---|
| « A1 » | 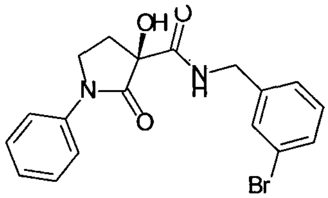 Acide (S)-3-hydroxy-2-oxo-1-phénylpyrrolidine-3-carboxylique 3-bromobenzylamide |
| « A2 » | 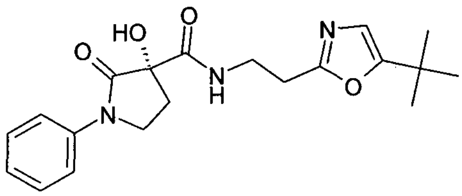 Acide (S)-3-hydroxy-2-oxo-1-phénylpyrrolidine-3-carboxylique [2-(5-tertio-butyloxazol-2-yl)éthyl]amide |
| « A3 » | 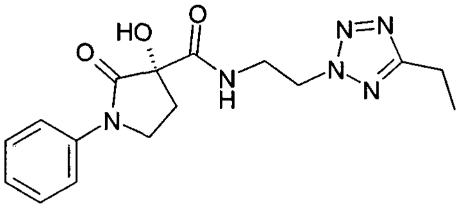 Acide (S)-3-hydroxy-2-oxo-1-phénylpyrrolidine-3-carboxylique [2-(5-éthyltétrazol-2-yl)éthyl]amide |
| « A4 » | 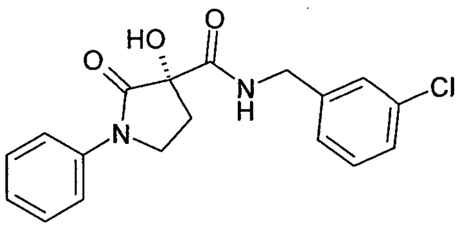 Acide (S)-3-hydroxy-2-oxo-1-phénylpyrrolidine-3-carboxylique 3-chlorobenzylamide |
| « A5 » | 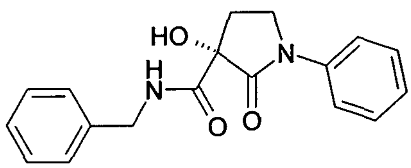 |

(suite)

| Composé n° | Structure/nom |
|---|---|
| | Acide (S)-3-hydroxy-2-oxo-1-phénylpyrrolidine-3-carboxylique benzylamide |
| « A6 » | 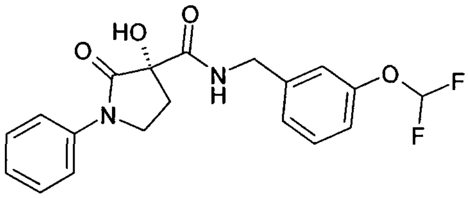<br>Acide (S)-3-hydroxy-2-oxo-1-phénylpyrrolidine-3-carboxylique 3-difluorométhoxybenzylamide |
| « A7 » | 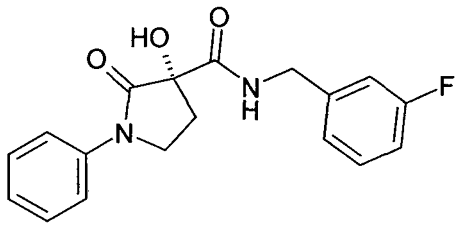<br>Acide (S)-3-hydroxy-2-oxo-1-phénylpyrrolidine-3-carboxylique 3-fluorobenzylamide |
| « A8 » | 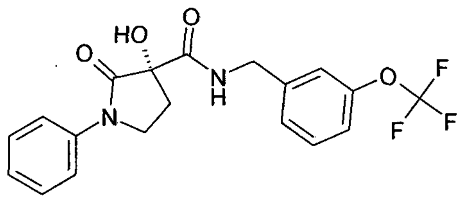<br>Acide (S)-3-hydroxy-2-oxo-1-phénylpyrrolidine-3-carboxylique 3-trifluorométhoxybenzylamide |
| « A9 » | 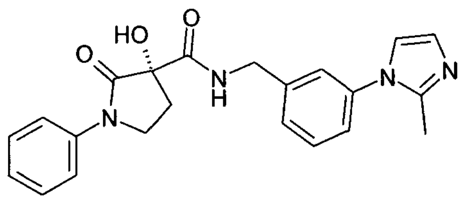<br>Acide (S)-3-hydroxy-2-oxo-1-phénylpyrrolidine-3-carboxylique 3-(2-méthylimidazol-1-yl)benzylamide |
| « A10 » | 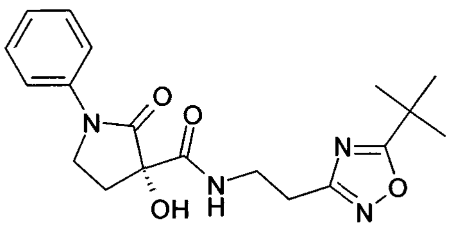<br>Acide (S)-3-hydroxy-2-oxo-1-phénylpyrrolidine-3-carboxylique [2-(5-tertio-butyl-1,2,4-oxadiazol-3-yl)-éthyl]amide |
| « A11 » | 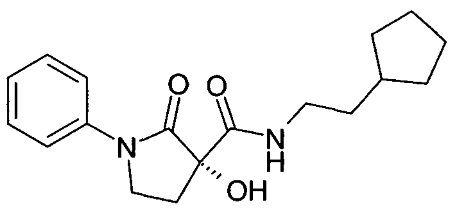<br>Acide (S)-3-hydroxy-2-oxo-1-phénylpyrrolidine-3-carboxylique (2-cyclopentyléthyl)amide |

(suite)

| Composé n° | Structure/nom |
|---|---|
| «A12» | 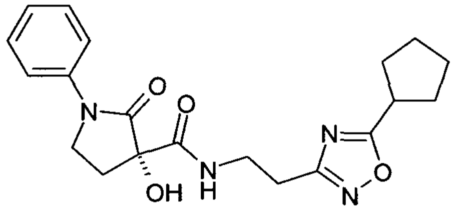 Acide (S)-3-hydroxy-2-oxo-1-phénylpyrrolidine-3-carboxylique [2-(5-cyclopentyl-1,2,4-oxadiazol-3-yl)-éthyl]amide |
| « A13 » | 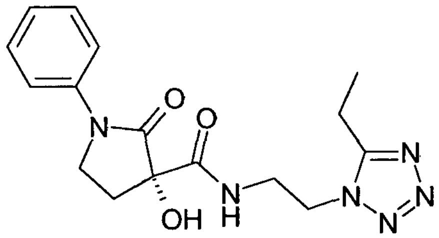 Acide (S)-3-hydroxy-2-oxo-1-phénylpyrrolidine-3-carboxylique [2-(5-éthyltétrazol-1-yl)éthyl]amide |
| « A14 » | 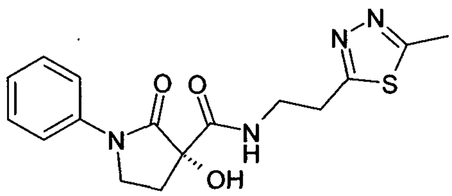 Acide (S)-3-hydroxy-2-oxo-1-phénylpyrrolidine-3-carboxylique [2-(5-méthyl-1,3,4-thiadiazol-2-yl)éthyl]amide |
| « A15 » | 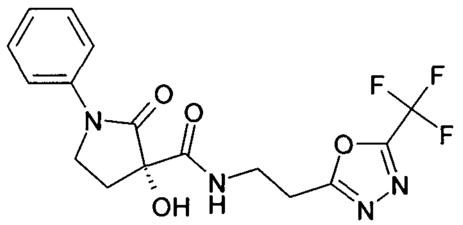 Acide (S)-3-hydroxy-2-oxo-1-phénylpyrrolidine-3-carboxylique [2-(5-trifluorométhyl-1,3,4-oxadiazol-2-yl)-éthyl]amide |
| « A16 » | 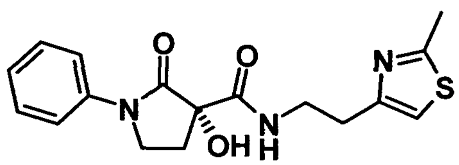 Acide (S)-3-hydroxy-2-oxo-1-phénylpyrrolidine-3-carboxylique [2-(2-méthylthiazol-4-yl)éthyl]amide |
| « A17 » | 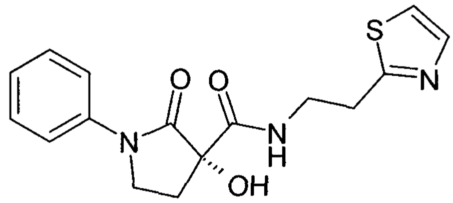 Acide (S)-3-hydroxy-2-oxo-1-phénylpyrrolidine-3-carboxylique (2-thiazol-2-yléthyl)amide |

(suite)

| Composé n° | Structure/nom |
|---|---|
| « A18 » | 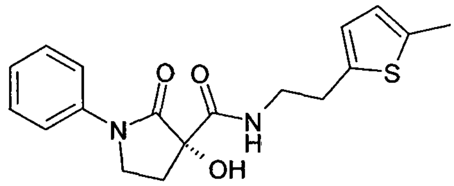 Acide (S)-3-hydroxy-2-oxo-1-phénylpyrrolidine-3-carboxylique [2-(5-méthylthiophén-2-yl)éthyl]amide |
| « A19 » | 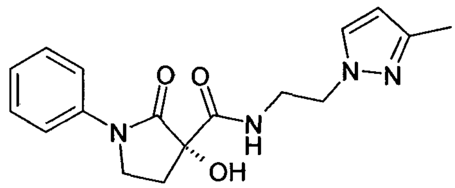 Acide (S)-3-hydroxy-2-oxo-1-phénylpyrrolidine-3-carboxylique [2-(3-méthylpyrazol-1-yl)éthylJamide |
| « A20 » | 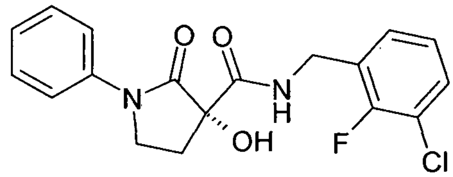 Acide (S)-3-hydroxy-2-oxo-1-phénylpyrrolidine-3-carboxylique 3-chloro-2-fluorobenzylamide |
| « A21 » | 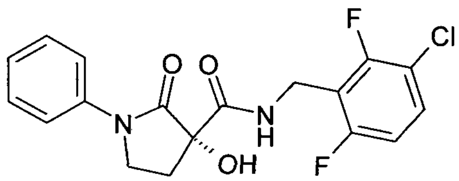 Acide (S)-3-hydroxy-2-oxo-1-phénylpyrrolidine-3-carboxylique 3-chloro-2,6-difluorobenzylamide |
| « A22 » | 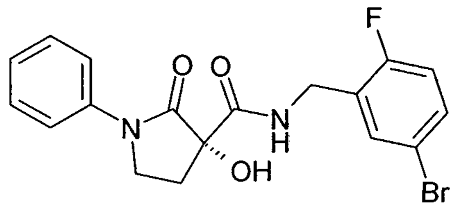 Acide (S)-3-hydroxy-2-oxo-1-phénylpyrrolidine-3-carboxylique 5-bromo-2-fluorobenzylamide |
| « A23 » | 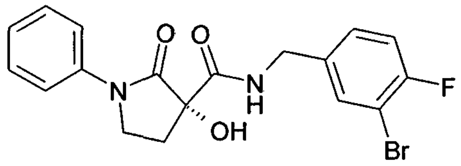 Acide (S)-3-hydroxy-2-oxo-1-phénylpyrrolidine-3-carboxylique 3-bromo-4-fluorobenzylamide |
| « A24 » | 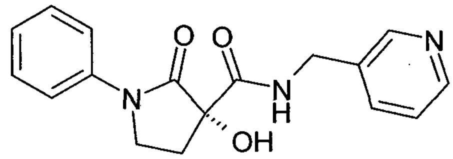 Acide (S)-3-hydroxy-2-oxo-1-phénylpyrrolidine-3-carboxylique (pyridin-3-ylméthyl)amide |

(suite)

| Composé n° | Structure/nom |
|---|---|
| « A25 » | 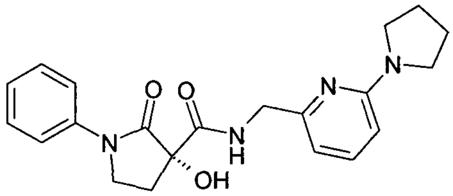 Acide (S)-3-hydroxy-2-oxo-1-phénylpyrrolidine-3-carboxylique (6-pyrrolidin-1-ylpyridin-2-ylméthyl)amide |
| « A26 » | 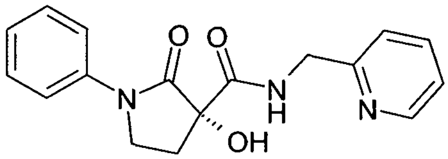 Acide (S)-3-hydroxy-2-oxo-1-phénylpyrrolidine-3-carboxylique (pyridin-2-ylméthyl)amide |
| « A27 » | 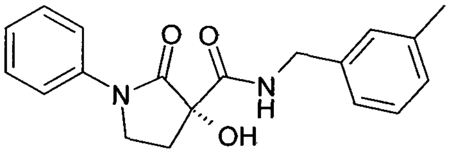 Acide (S)-3-hydroxy-2-oxo-1-phénylpyrrolidine-3-carboxylique 3-méthylbenzylamide |
| « A28 » | 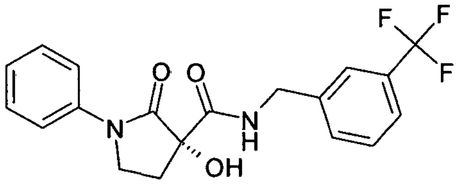 Acide (S)-3-hydroxy-2-oxo-1-phénylpyrrolidine-3-carboxylique 3-trifluorométhylbenzylamide |
| « A29 » | 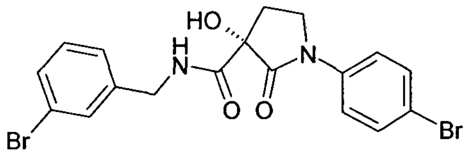 Acide (S)-1-(4-bromophényl)-3-hydroxy-2-oxopyrrolidine-3-carboxylique 3-bromobenzylamide |
| « A30 » | 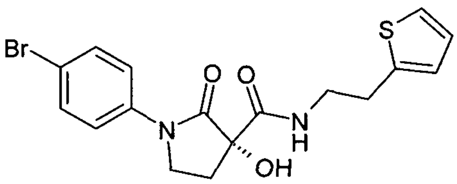 Acide (S)-1-(4-bromophényl)-3-hydroxy-2-oxopyrrolidine-3-carboxylique (2-thiophén-2-yléthyl)amide |
| « A31 » | 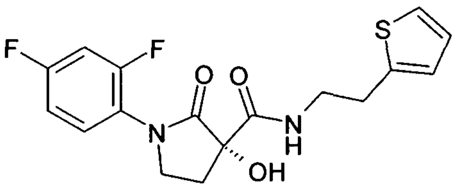 Acide (S)-1-(2,4-difluorophényl)-3-hydroxy-2-oxopyrrolidine-3-carboxylique (2-thiophén-2-yléthyl)amide |

(suite)

| Composé n° | Structure/nom |
|---|---|
| « A32 » | 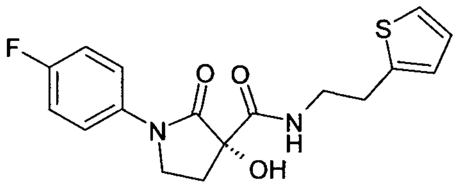 Acide (S)-1-(4-fluorophényl)-3-hydroxy-2-oxopyrrolidine-3-carboxylique (2-thiophén-2-yléthyl)amide |
| « A33 » | 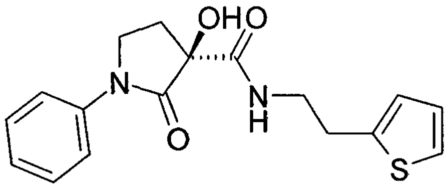 Acide (S)-3-hydroxy-2-oxo-1-phénylpyrrolidine-3-carboxylique (2-thiophén-2-yléthyl)amide |
| « A34 » | 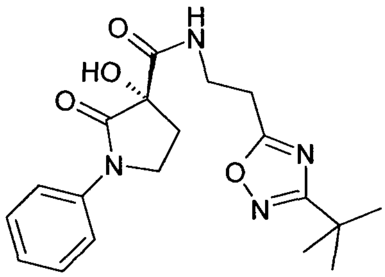 Acide (S)-3-hydroxy-2-oxo-1-phénylpyrrolidine-3-carboxylique [2-(3-tertio-butyl-1,2,4-oxadiazol-5-yl)-éthyl]amide |
| « A35 » | 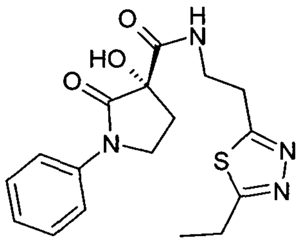 Acide (S)-3-hydroxy-2-oxo-1-phénylpyrrolidine-3-carboxylique [2-(5-éthyl-1,3,4-thiadiazol-2-yl)éthyl]amide |
| « A36 » | 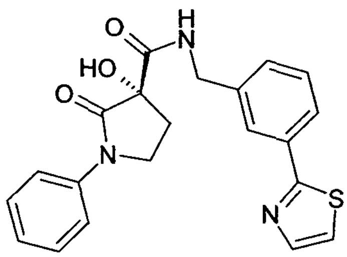 Acide (S)-3-hydroxy-2-oxo-1-phénylpyrrolidine-3-carboxylique 3-thiazol-2-ylbenzylamide |

(suite)

| Composé n° | Structure/nom |
|---|---|
| « A37 » | 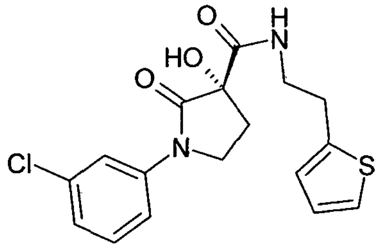<br>Acide (S)-1-(3-chlorophényl)-3-hydroxy-2-oxopyrrolidine-3-carboxylique (2-thiophén-2-yléthyl)amide |
| « A38 » | 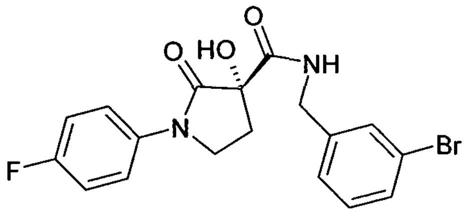<br>Acide (S)-1-(4-fluorophényl)-3-hydroxy-2-oxopyrrolidine-3-carboxylique 3-bromobenzylamide |
| « A39 » | 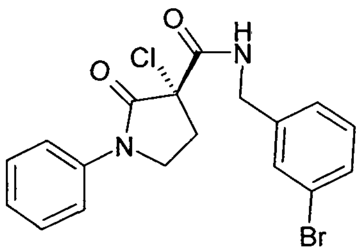<br>Acide (S)-3-chloro-2-oxo-1-phénylpyrrolidine-3-carboxylique 3-bromobenzylamide |
| « A40 » | 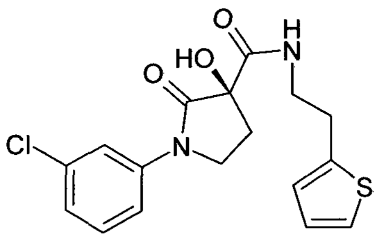<br>Acide (R)-1-(3-chlorophényl)-3-hydroxy-2-oxopyrrolidine-3-carboxylique (2-thiophén-2-yléthyl)amide |
| « A41 » | 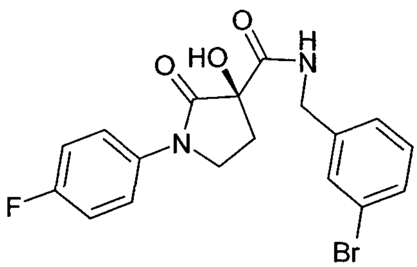<br>Acide (R)-1-(4-fluorophényl)-3-hydroxy-2-oxopyrrolidine-3-carboxylique 3-bromobenzylamide |

(suite)

| Composé n° | Structure/nom |
|---|---|
| « A42 » | 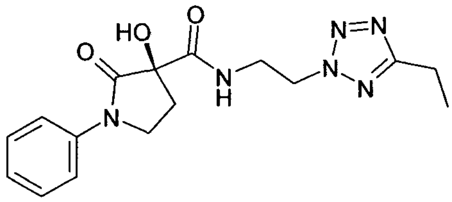<br>Acide (R)-3-hydroxy-2-oxo-1-phénylpyrrolidine-3-carboxylique [2-(5-éthyltétrazol-2-yl)éthyl]amide |
| « A43 » | 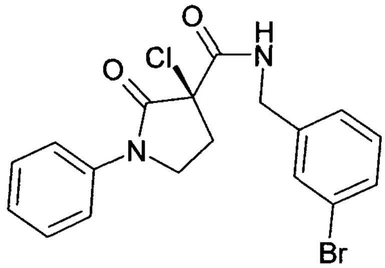<br>Acide (R)-3-chloro-2-oxo-1-phénylpyrrolidine-3-carboxylique 3-bromobenzylamide |
| « A44 » | 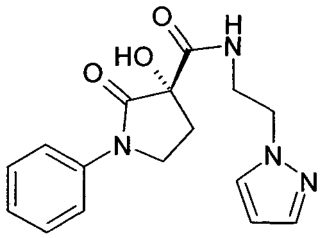<br>Acide (S)-3-hydroxy-2-oxo-1-phénylpyrrolidine-3-carboxylique (2-pyrazol-1-yléthyl)amide |
| « A45 » | 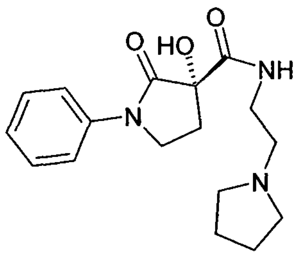<br>Acide (S)-3-hydroxy-2-oxo-1-phénylpyrrolidine-3-carboxylique (2-pyrrolidin-1-yléthyl)amide |
| « A46 » | 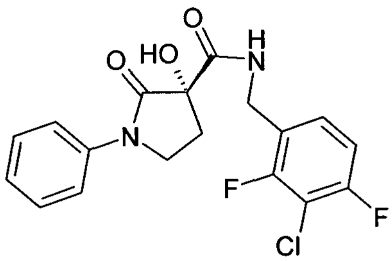<br>Acide (S)-3-hydroxy-2-oxo-1-phénylpyrrolidine-3-carboxylique 3-chloro-2,4-difluorobenzylamide |

(suite)

| Composé n° | Structure/nom |
|---|---|
| « A47 » | 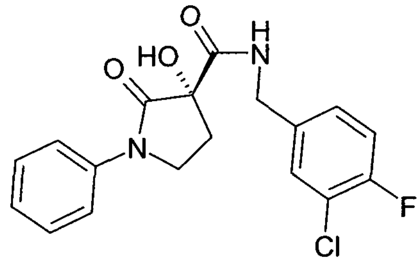<br>Acide (S)-3-hydroxy-2-oxo-1-phénylpyrrolidine-3-carboxylique 3-chloro-4-fluorobenzylamide |
| « A48 » | 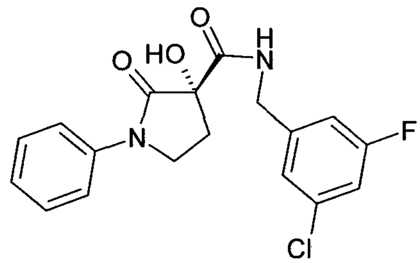<br>Acide (S)-3-hydroxy-2-oxo-1-phénylpyrrolidine-3-carboxylique 3-chloro-5-fluorobenzylamide |
| « A49 » | 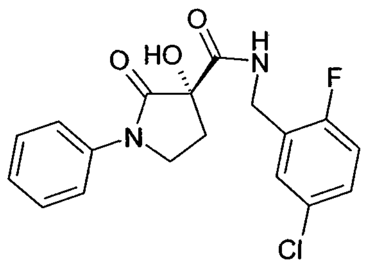<br>Acide (S)-3-hydroxy-2-oxo-1-phénylpyrrolidine-3-carboxylique 5-chloro-2-fluorobenzylamide |
| « A50 » | 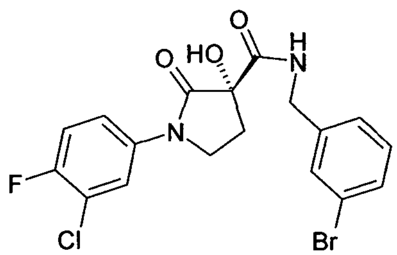<br>Acide (S)-1-(3-chloro-4-fluorophényl)-3-hydroxy-2-oxopyrrolidine-3-carboxylique 3-bromobenzylamide |
| « A51 » | 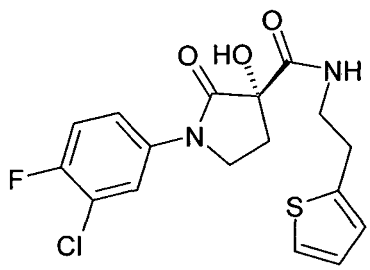<br>Acide (S)-1-(3-chloro-4-fluorophényl)-3-hydroxy-2-oxopyrrolidine-3-carboxylique (2-thiophén-2-yléthyl)amide |

(suite)

| Composé n° | Structure/nom |
|---|---|
| « A52 » | 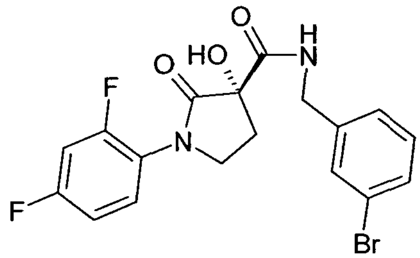 Acide (S)-1-(2,4-difluorophényl)-3-hydroxy-2-oxopyrrolidine-3-carboxylique 3-bromobenzylamide |
| « A53 » | 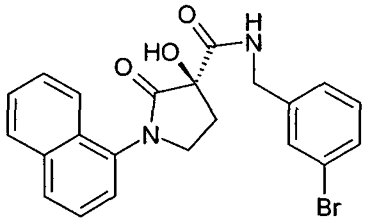 Acide (R)-3-hydroxy-1-naphtalén-1-yl-2-oxopyrrolidine-3-carboxylique 3-bromobenzylamide |
| « A54 » | 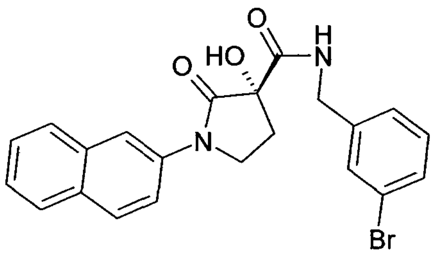 Acide (S)-3-hydroxy-1-naphtalén-2-yl-2-oxopyrrolidine-3-carboxylique 3-bromobenzylamide |
| « A55 » | 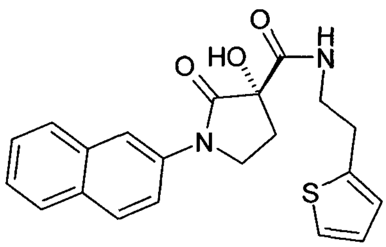 Acide (S)-3-hydroxy-1-naphtalén-2-yl-2-oxopyrrolidine-3-carboxylique (2-thiophén-2-yléthyl)amide |
| « A56 » | 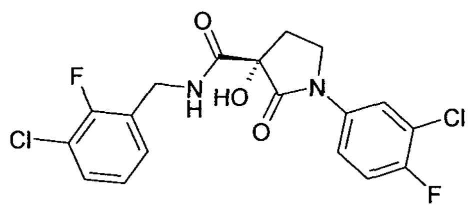 Acide (S)-1-(3-chloro-4-fluorophényl)-3-hydroxy-2-oxopyrrolidine-3-carboxylique 3-chloro-2-fluorobenzylamide |
| « A57 » | 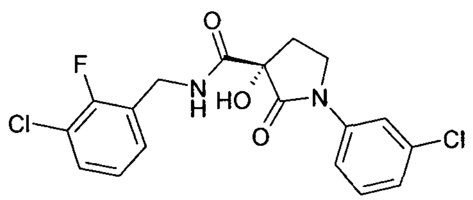 |

(suite)

| Composé n° | Structure/nom |
|---|---|
| | Acide (S)-1-(3-chlorophényl)-3-hydroxy-2-oxopyrrolidine-3-carboxylique 3-chloro-2-fluorobenzylamide |
| « A58 » | 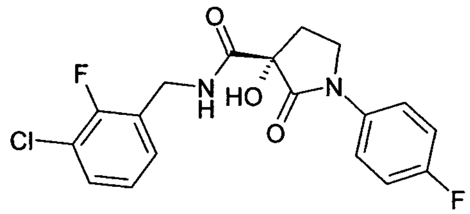<br>Acide (S)-1-(4-fluorophényl)-3-hydroxy-2-oxopyrrolidine-3-carboxylique 3-chloro-2-fluorobenzylamide |
| « A59 » | 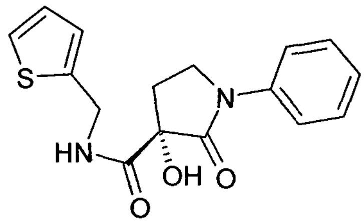<br>Acide (S)-3-hydroxy-2-oxo-1-phénylpyrrolidine-3-carboxylique (thiophén-2-ylméthyl)amide |
| « A60 » | 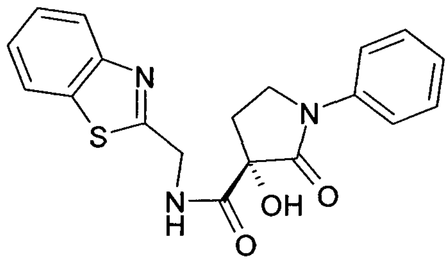<br>Acide (S)-3-hydroxy-2-oxo-1-phénylpyrrolidine-3-carboxylique (benzothiazol-2-yiméthyl)amide |
| « A61 » | 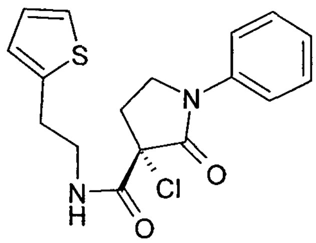<br>Acide (S)-3-chloro-2-oxo-1-phénylpyrrolidine-3-carboxylique (2-thiophén-2-yléthyl)amide |
| « A62 » | 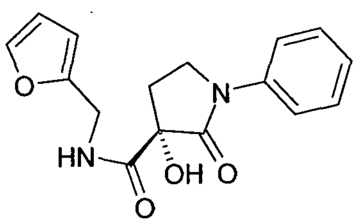<br>Acide (S)-3-hydroxy-2-oxo-1-phénylpyrrolidine-3-carboxylique (furan-2-ylméthyl)amide |

(suite)

| Composé n° | Structure/nom |
|---|---|
| « A63 » | 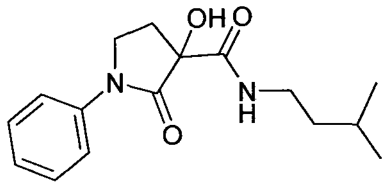<br>Acide 3-hydroxy-2-oxo-1-phénylpyrrolidine-3-carboxylique (3-méthylbutyl)amide |
| « A64 » | 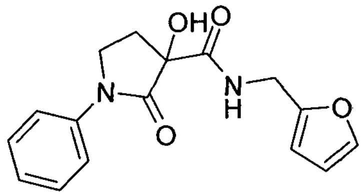<br>Acide 3-hydroxy-2-oxo-1-phénylpyrrolidine-3-carboxylique (furan-2-ylméthyl)amide |
| « A65 » | 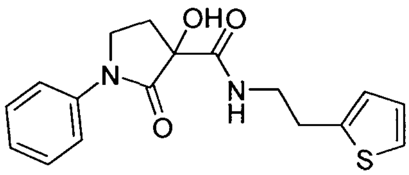<br>Acide 3-hydroxy-2-oxo-1-phénylpyrrolidine-3-carboxylique (2-thiophén-2-yléthyl)amide |
| « A66 » | 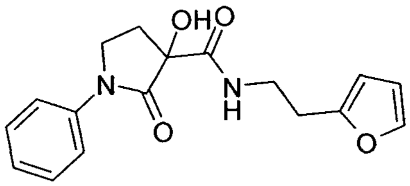<br>Acide 3-hydroxy-2-oxo-1-phénylpyrrolidine-3-carboxylique (2-furan-2-yléthyl)amide |
| « A67 » | 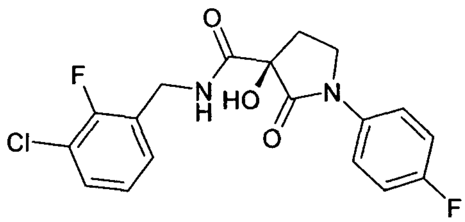<br>Acide (R)-1-(4-fluorophényl)-3-hydroxy-2-oxopyrrolidine-3-carboxylique 3-chloro-2-fluorobenzylamide |
| « A68 » | 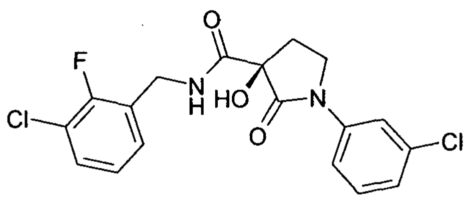<br>Acide (R)-1-(3-chlorophényl)-3-hydroxy-2-oxopyrrolidine-3-carboxylique 3-chloro-2-fluorobenzylamide |

(suite)

| Composé n° | Structure/nom |
|---|---|
| « A69 » | 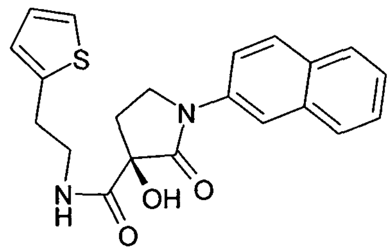<br>Acide (R)-3-hydroxy-1-naphtalén-2-yl-2-oxopyrrolidine-3-carboxylique (2-thiophén-2-yléthyl)amide |
| « A70 » | 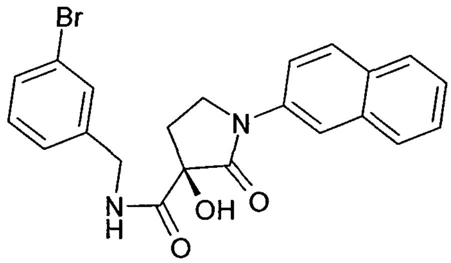<br>Acide (R)-3-hydroxy-1-naphtalén-2-yl-2-oxopyrrolidine-3-carboxylique 3-bromobenzylamide |
| « A71 » | 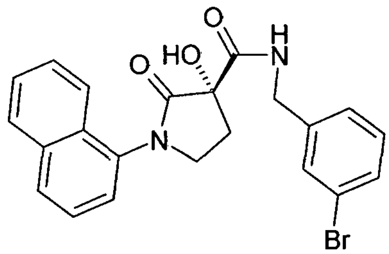<br>Acide (S)-3-hydroxy-1-naphtalén-1-yl-2-oxopyrrolidine-3-carboxylique 3-bromobenzylamide |
| « A72 » | 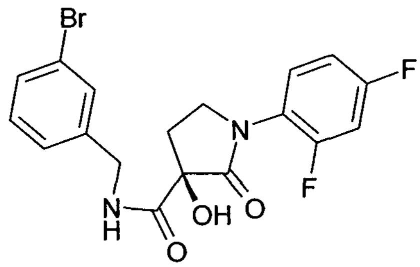<br>Acide (R)-1-(2,4-difluorophényl)-3-hydroxy-2-oxopyrrolidine-3-carboxylique 3-bromobenzylamide |
| « A73 » | 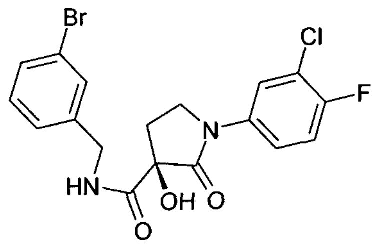<br>Acide (R)-1-(3-chloro-4-fluorophényl)-3-hydroxy-2-oxopyrrolidine-3-carboxylique 3-bromobenzylamide |

(suite)

| Composé n° | Structure/nom |
|---|---|
| « A74 » | 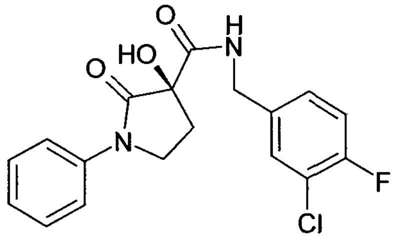 Acide (R)-3-hydroxy-2-oxo-1-phénylpyrrolidine-3-carboxylique 3-chloro-4-fluorobenzylamide |
| « A75 » | 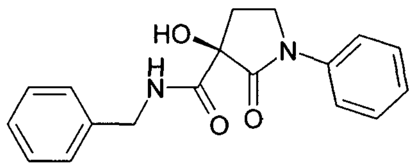 Acide (R)-3-hydroxy-2-oxo-1-phénylpyrrolidine-3-carboxylique benzylamide |
| « A76 » | 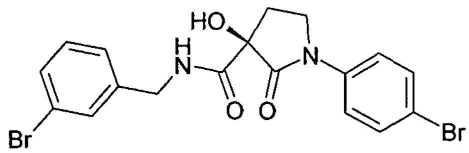 Acide (R)-1-(4-bromophényl)-3-hydroxy-2-oxopyrrolidine-3-carboxylique 3-bromobenzylamide |
| « A77 » | 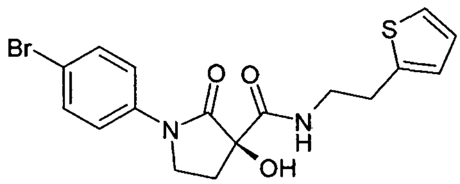 Acide (R)-1-(4-bromophényl)-3-hydroxy-2-oxopyrrolidine-3-carboxylique (2-thiophén-2-yléthyl)amide |
| « A78 » | 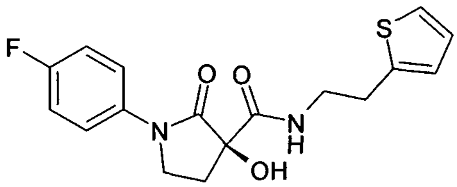 Acide (R)-1-(4-fluorophényl)-3-hydroxy-2-oxopyrrolidine-3-carboxylique (2-thiophén-2-yléthyl)amide |
| « A79 » | 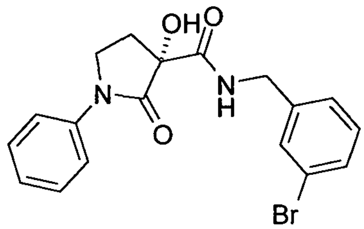 Acide (R)-3-hydroxy-2-oxo-1-phénylpyrrolidine-3-carboxylique 3-bromobenzylamide |

(suite)

| Composé n° | Structure/nom |
|---|---|
| « A80 » | 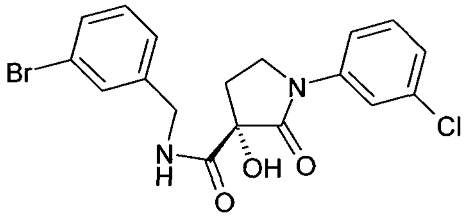<br>Acide (S)-1-(3-chlorophényl)-3-hydroxy-2-oxopyrrolidine-3-carboxylique 3-bromobenzylamide |
| « A81 » | 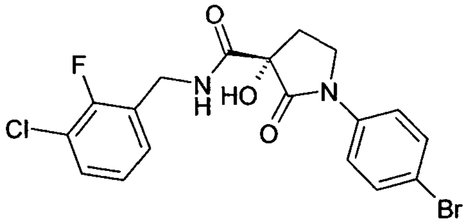<br>Acide (S)-1-(4-bromophényl)-3-hydroxy-2-oxopyrrolidine-3-carboxylique 3-chloro-2-fluorobenzylamide |
| « A82 » | 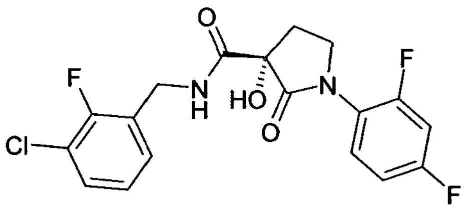<br>Acide (S)-1-(2,4-difluorophényl)-3-hydroxy-2-oxopyrrolidine-3-carboxylique 3-chloro-2-fluorobenzylamide |
| « A83 » | 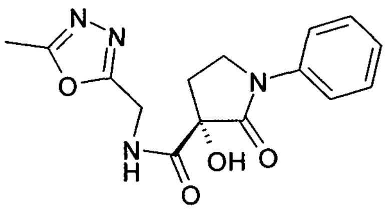<br>Acide (S)-3-hydroxy-2-oxo-1-phénylpyrrolidine-3-carboxylique (5-méthyl-1,3,4-oxadiazol-2-ylméthyl)amide |
| « A84 » | 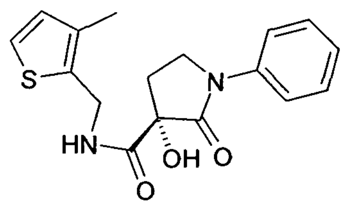<br>Acide (S)-3-hydroxy-2-oxo-1-phénylpyrrolidine-3-carboxylique (3-méthylthiophén-2-ylméthyl)amide |
| « A85 » | 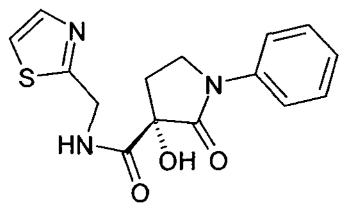<br>Acide (S)-3-hydroxy-2-oxo-1-phénylpyrrolidine-3-carboxylique (thiazol-2-ylméthyl)amide |

(suite)

| Composé n° | Structure/nom |
|---|---|
| « A86 » | 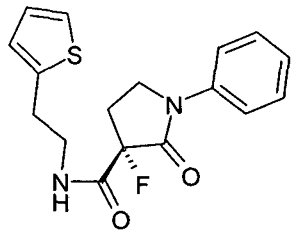<br>Acide (S)-3-fluoro-2-oxo-1-phénylpyrrolidine-3-carboxylique (2-thiophén-2-yléthyl)amide |
| « A87 » | 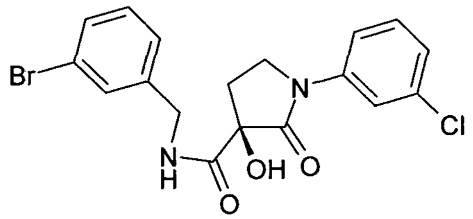<br>Acide (R)-1-(3-chlorophényl)-3-hydroxy-2-oxopyrrolidine-3-carboxylique 3-bromobenzylamide |
| « A88 » | 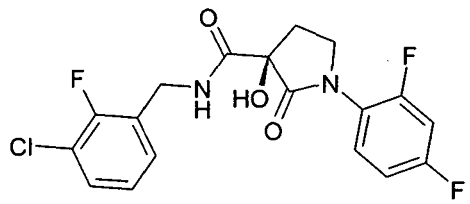<br>Acide (R)-1-(2,4-difluorophényl)-3-hydroxy-2-oxopyrrolidine-3-carboxylique 3-chloro-2-fluorobenzylamide |
| « A89 » | 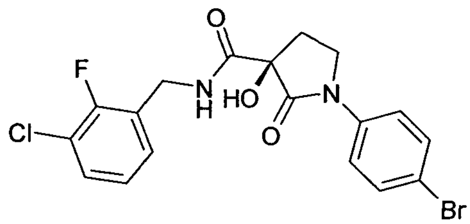<br>Acide (R)-1-(4-bromophényl)-3-hydroxy-2-oxopyrrolidine-3-carboxylique 3-chloro-2-fluorobenzylamide |
| « A90 » | 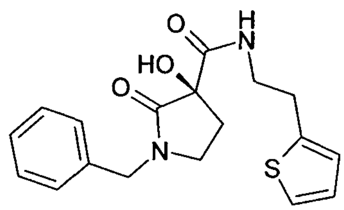<br>Acide (R)-1-benzyl-3-hydroxy-2-oxopyrrolidine-3-carboxylique (2-thiophén-2-yléthyl)amide |
| « A91 » | 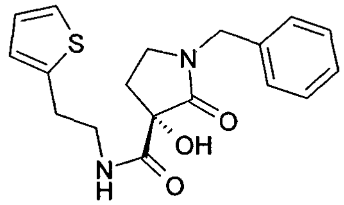<br>Acide (S)-1-benzyl-3-hydroxy-2-oxopyrrolidine-3-carboxylique (2-thiophén-2-yléthyl)amide |

(suite)

| Composé n° | Structure/nom |
|---|---|
| « A91a » | Acide (S)-3-amino-2-oxo-1-phénylpyrrolidine-3-carboxylique (3-chloro-5-fluorobenzyl)amide |
| « A92 » | 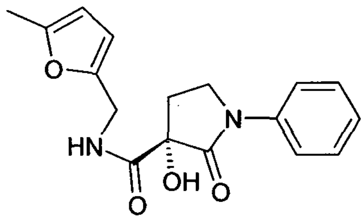 Acide (S)-3-hydroxy-2-oxo-1-phénylpyrrolidine-3-carboxylique (5-méthylfuran-2-ylméthyl)amide |
| « A93 » | 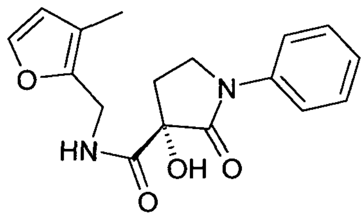 Acide (S)-3-hydroxy-2-oxo-1-phénylpyrrolidine-3-carboxylique (3-méthylfuran-2-ylméthyl)amide |
| « A94 » | 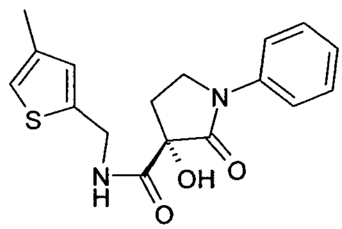 Acide (S)-3-hydroxy-2-oxo-1-phénylpyrrolidine-3-carboxylique (4-méthylthiophén-2-ylméthyl)amide |
| « A95 » | 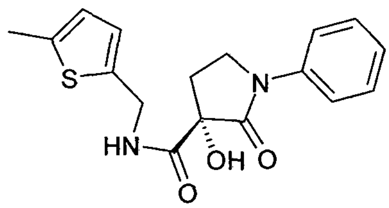 Acide (S)-3-hydroxy-2-oxo-1-phénylpyrrolidine-3-carboxylique (5-méthylthiophén-2-ylméthyl)amide |
| « A96 » | 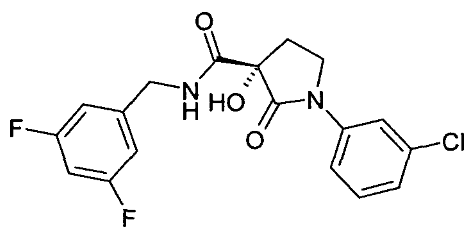 Acide (S)-1-(3-chlorophényl)-3-hydroxy-2-oxopyrrolidine-3-carboxylique 3,5-difluorobenzylamide |
| « A97 » | 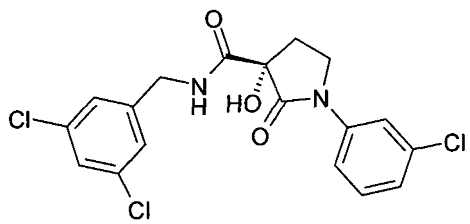 Acide (S)-1-(3-chlorophényl)-3-hydroxy-2-oxopyrrolidine-3-carboxylique 3,5-dichlorobenzylamide |

(suite)

| Composé n° | Structure/nom |
|---|---|
| « A98 » | 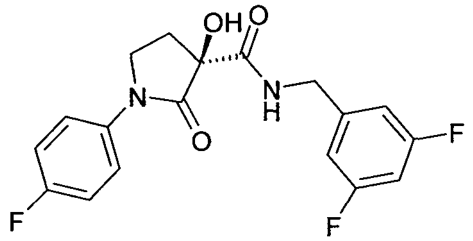<br>Acide (S)-1-(4-fluorophényl)-3-hydroxy-2-oxopyrrolidine-3-carboxylique 3,5-difluorobenzylamide |
| « A99 » | 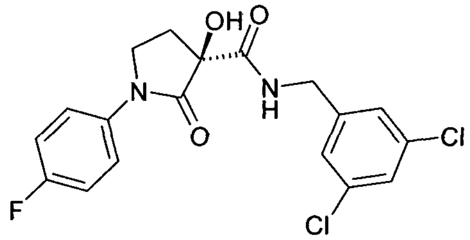<br>Acide (S)-1-(4-fluorophényl)-3-hydroxy-2-oxopyrrolidine-3-carboxylique 3,5-dichlorobenzylamide |
| « A100 » | 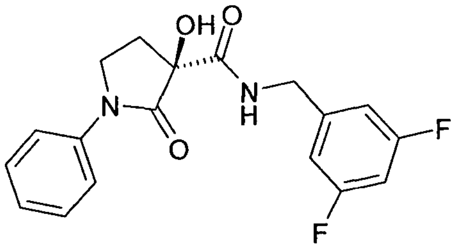<br>Acide (S)-3-hydroxy-2-oxo-1-phénylpyrrolidine-3-carboxylique 3,5-difluorobenzylamide |
| « A101 » | 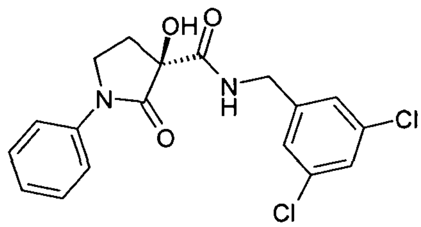<br>Acide (S)-3-hydroxy-2-oxo-1-phénylpyrrolidine-3-carboxylique 3,5-dichlorobenzylamide |
| « A102 » | 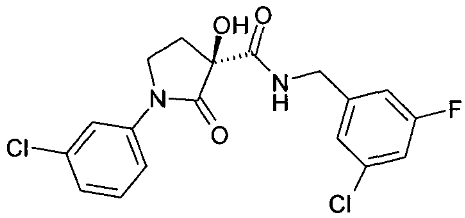<br>Acide (S)-1-(3-chlorophényl)-3-hydroxy-2-oxopyrrolidine-3-carboxylique 3-chloro-5-fluorobenzylamide |

(suite)

| Composé n° | Structure/nom |
|---|---|
| « A103 » | 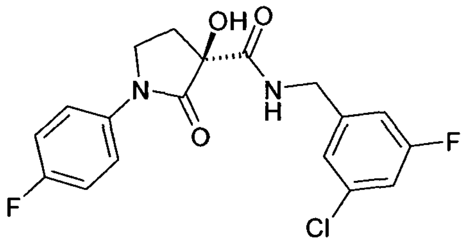 Acide (S)-1-(4-fluorophényl)-3-hydroxy-2-oxopyrrolidine-3-carboxylique 3-chloro-5-fluorobenzylamide |
| « A104 » | 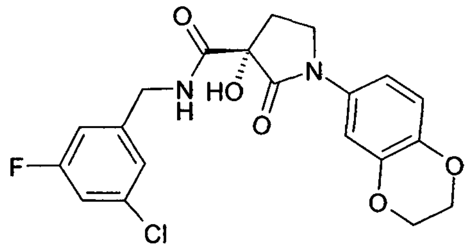 Acide (S)-1-(2,3-dihydrobenzo-1,4-dioxin-6-yl)-3-hydroxy-2-oxopyrrolidine-3-carboxylique 3-chloro-5-fluorobenzylamide |
| « A105 » | 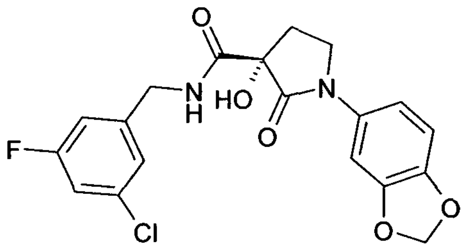 Acide (S)-1-benzo-1,3-dioxol-5-yl-3-hydroxy-2-oxopyrrolidine-3-carboxylique 3-chloro-5-fluorobenzylamide |
| « A106 » | 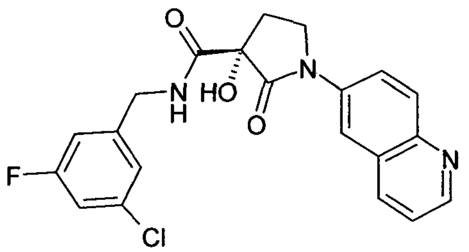 Acide (S)-3-hydroxy-2-oxo-1-quinoléin-6-ylpyrrolidine-3-carboxylique 3-chloro-5-fluorobenzylamide |
| « A107 » | 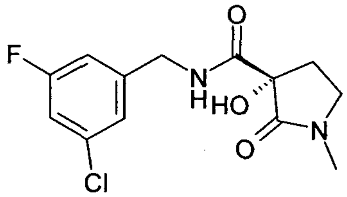 Acide (S)-3-hydroxy-1-méthyl-2-oxopyrrolidine-3-carboxylique 3-chloro-5-fluorobenzylamide |

(suite)

| Composé n° | Structure/nom |
|---|---|
| « A108 » | 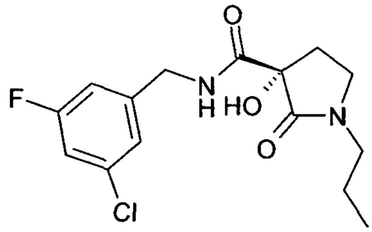 Acide (S)-3-hydroxy-2-oxo-1-propylpyrrolidine-3-carboxylique 3-chloro-5-fluorobenzylamide |
| « A109 » | 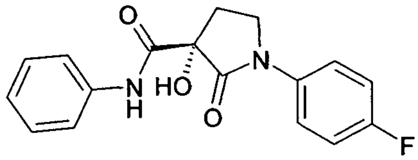 Acide (S)-1-(4-fluorophényl)-3-hydroxy-2-oxopyrrolidine-3-carboxylique phénylamide |
| « A110 » | 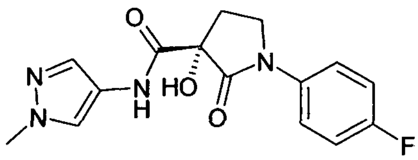 Acide (S)-1-(4-fluorophényl)-3-hydroxy-2-oxopyrrolidine-3-carboxylique (1-méthyl-1H-pyrazol-4-yl)amide |
| « A111 » | 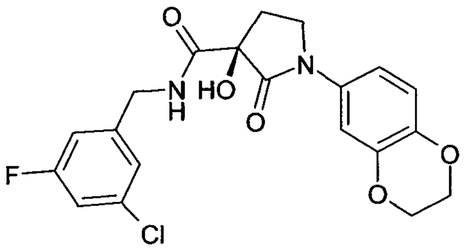 Acide (R)-1-(2,3-dihydrobenzo-1,4-dioxin-6-yl)-3-hydroxy-2-oxopyrrolidine-3-carboxylique 3-chloro-5-fluorobenzylamide |
| « A112 » | 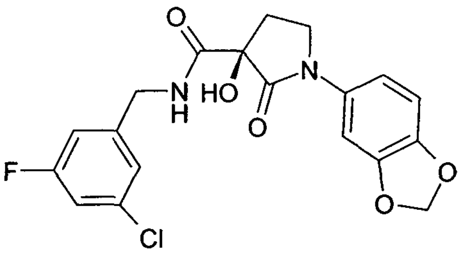 Acide (R)-1-benzo-1,3-dioxol-5-yl-3-hydroxy-2-oxopyrrolidine-3-carboxylique 3-chloro-5-fluorobenzylamide |
| « A113 » | 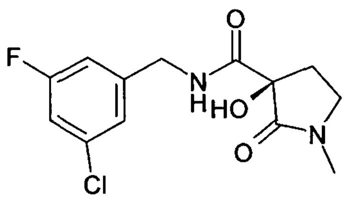 Acide (R)-3-hydroxy-1-méthyl-2-oxopyrrolidine-3-carboxylique 3-chloro-5-fluorobenzylamide |

(suite)

| Composé n° | Structure/nom |
|---|---|
| « A114 » | 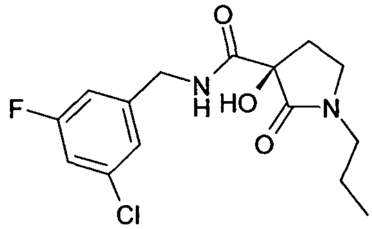<br>Acide (R)-3-hydroxy-2-oxo-1-propylpyrrolidine-3-carboxylique 3-chloro-5-fluorobenzylamide |
| « A115 » | 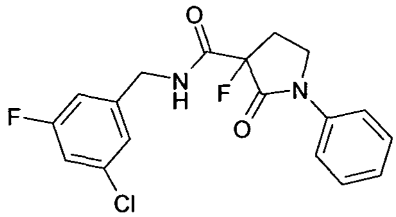<br>Acide 3-fluoro-2-oxo-1-phénylpyrrolidine-3-carboxylique 3-chloro-5-fluorobenzylamide |
| « A116 » | 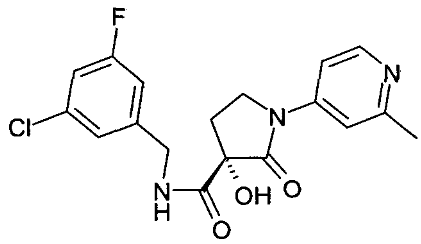<br>Acide (S)-3-hydroxy-1-(2-méthylpyridin-4-yl)-2-oxopyrrolidine-3-carboxylique 3-chloro-5-fluorobenzylamide |
| « A117 » | 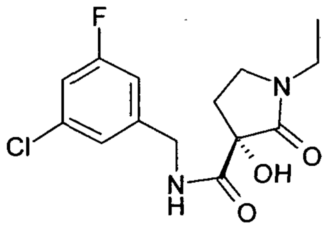<br>Acide (S)-1-éthyl-3-hydroxy-2-oxopyrrolidine-3-carboxylique 3-chloro-5-fluorobenzylamide |
| « A118 » | 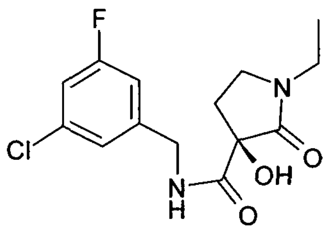<br>Acide (R)-1-éthyl-3-hydroxy-2-oxopyrrolidine-3-carboxylique 3-chloro-5-fluorobenzylamide |
| « A119 » | 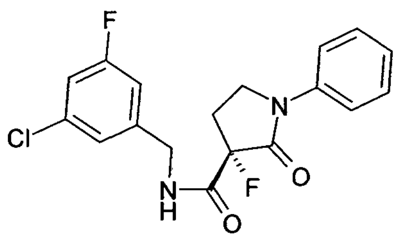 |

(suite)

| Composé n° | Structure/nom |
|---|---|
| | Acide (S)-3-fluoro-2-oxo-1-phénylpyrrolidine-3-carboxylique 3-chloro-5-fluorobenzylamide |
| « A120 » | 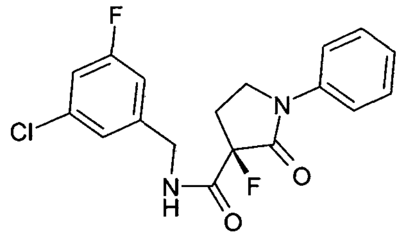<br>Acide (R)-3-fluoro-2-oxo-1-phénylpyrrolidine-3-carboxylique 3-chloro-5-fluorobenzylamide |
| « A150 » | Acide 3-azido-2-oxo-1-phénylpyrrolidine-3-carboxylique (3-chloro-5-fluorobenzyl)amide |
| « A151 » | Acide 3-amino-2-oxo-1-phénylpyrrolidine-3-carboxylique (3-chloro-5-fluorobenzyl)amide |
| «A162» | 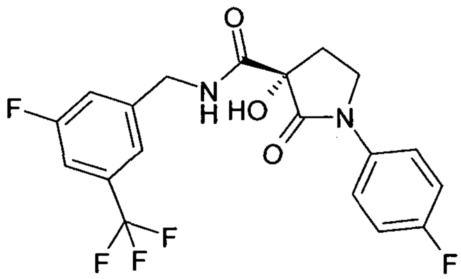<br>Acide (S)-1-(4-fluorophényl)-3-hydroxy-2-oxopyrrolidine-3-carboxylique 3-fluoro-5-trifluorométhylbenzylamide |
| « A163 » | 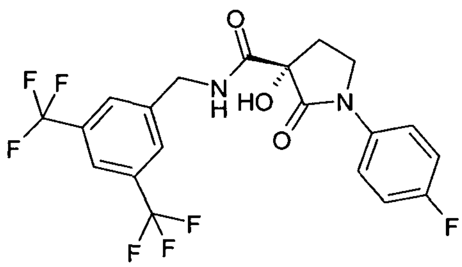<br>Acide (S)-1-(4-fluorophényl)-3-hydroxy-2-oxopyrrolidine-3-carboxylique 3,5-bis-trifluorométhylbenzylamide |
| « A164 » | 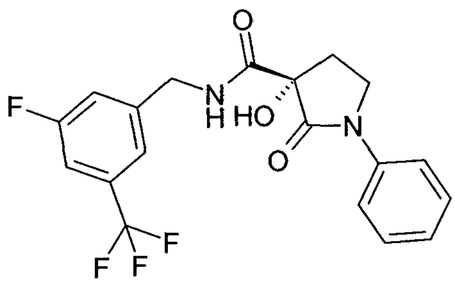<br>Acide (S)-3-hydroxy-2-oxo-1-phénylpyrrolidine-3-carboxylique 3-fluoro-5-trifluorométhylbenzylamide |

(suite)

| Composé n° | Structure/nom |
|---|---|
| « A165 » | 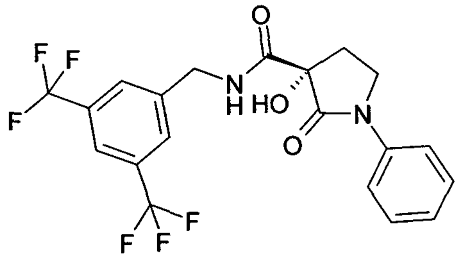<br>Acide (S)-3-hydroxy-2-oxo-1-phénylpyrrolidine-3-carboxylique 3,5-bis-trifluorométhylbenzylamide |
| « A166 » | 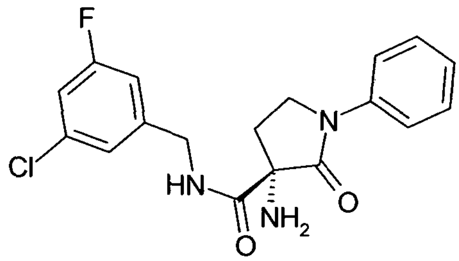<br>Acide (S)-3-amino-2-oxo-1-phénylpyrrolidine-3-carboxylique 3-chloro-5-fluorobenzylamide |
| « A167 » | 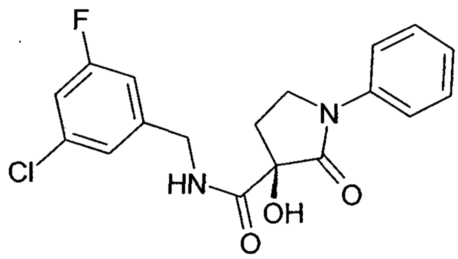<br>Acide (R)-3-hydroxy-2-oxo-1-phénylpyrrolidine-3-carboxylique 3-chloro-5-fluorobenzylamide |
| « A168 » | 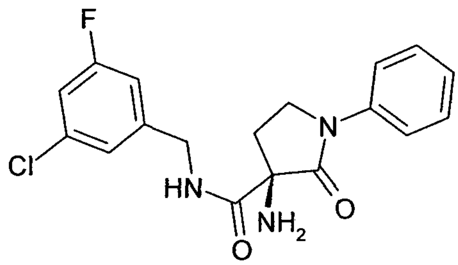<br>Acide (R)-3-amino-2-oxo-1-phénylpyrrolidine-3-carboxylique 3-chloro-5-fluorobenzylamide |
| « A169 » | 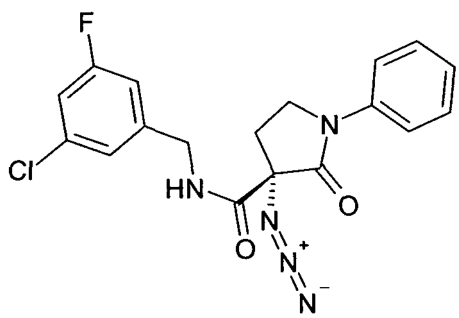<br>Acide (S)-3-azido-2-oxo-1-phénylpyrrolidine-3-carboxylique 3-chloro-5-fluorobenzylamide |

(suite)

| Composé n° | Structure/nom |
|---|---|
| « A170 » | 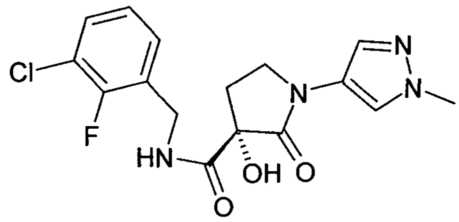 Acide (S)-3-hydroxy-1-(1-méthyl-1H-pyrazol-4-yl)-2-oxopyrrolidine-3-carboxylique 3-chloro-2-fluorobenzylamide |
| « A171 » | 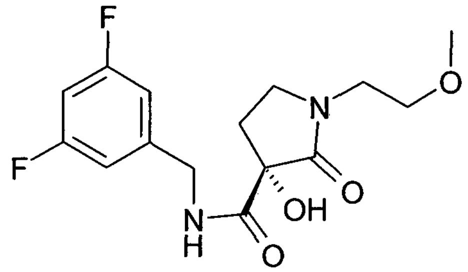 Acide (S)-3-hydroxy-1-(2-méthoxyéthyl)-2-oxopyrrolidine-3-carboxylique 3,5-difluorobenzylamide |
| « A172 » | 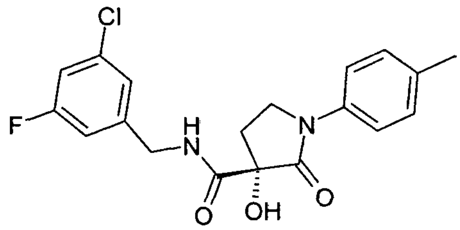 Acide (S)-3-hydroxy-2-oxo-1-p-tolylpyrrolidine-3-carboxylique 3-chloro-5-fluorobenzylamide |
| « A173 » | 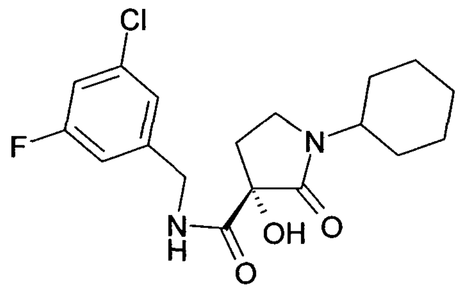 Acide (S)-1-cyclohexyl-3-hydroxy-2-oxopyrrolidine-3-carboxylique 3-chloro-5-fluorobenzylamide |
| « A174 » | 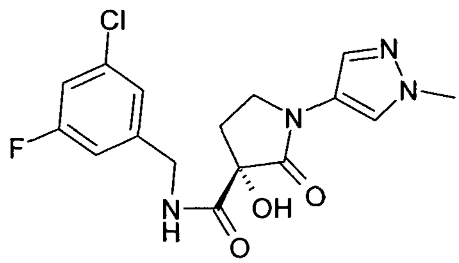 Acide (S)-3-hydroxy-1-(1-méthyl-1H-pyrazol-4-yl)-2-oxopyrrolidine-3-carboxylique 3-chloro-5-fluorobenzylamide |

(suite)

| Composé n° | Structure/nom |
|---|---|
| « A175 » | 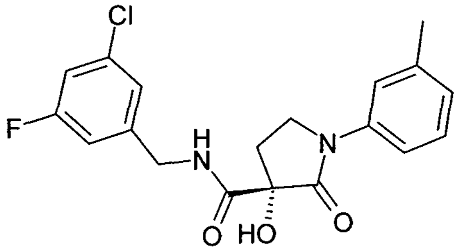<br>Acide (S)-3-hydroxy-2-oxo-1-m-tolylpyrrolidine-3-carboxylique 3-chloro-5-fluorobenzylamide |
| « A176 » | 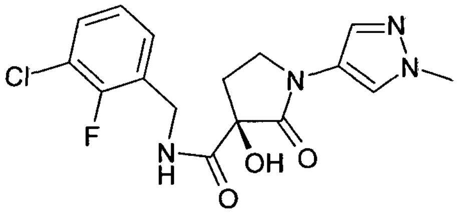<br>Acide (R)-3-hydroxy-1-(1-méthyl-1H-pyrazol-4-yl)-2-oxopyrrolidine-3-carboxylique 3-chloro-2-fluorobenzylamide |
| « A177» | 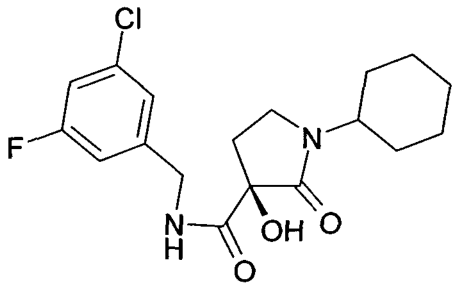<br>Acide (R)-1-cyclohexyl-3-hydroxy-2-oxopyrrolidine-3-carboxylique 3-chloro-5-fluorobenzylamide |
| « A178 » | 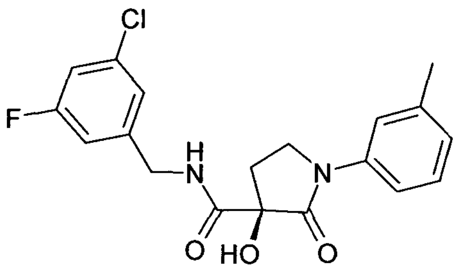<br>Acide (R)-3-hydroxy-2-oxo-1-m-tolylpyrrolidine-3-carboxylique 3-chloro-5-fluorobenzylamide |
| « A179 » | 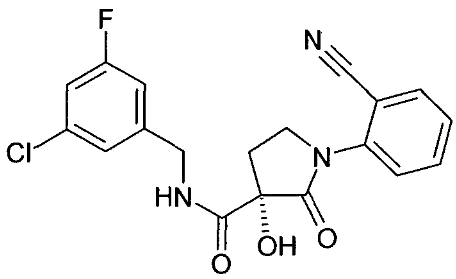<br>Acide (S)-1-(2-cyanophényl)-3-hydroxy-2-oxopyrrolidine-3-carboxylique 3-chloro-5-fluorobenzylamide |

(suite)

| Composé n° | Structure/nom |
|---|---|
| « A180 » | 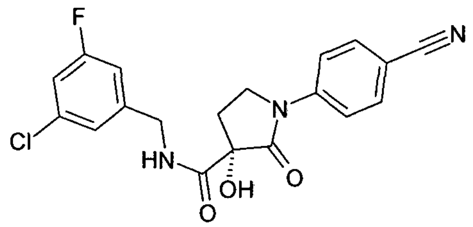 Acide (S)-1-(4-cyanophényl)-3-hydroxy-2-oxopyrrolidine-3-carboxylique 3-chloro-5-fluorobenzylamide |
| « A181 » | 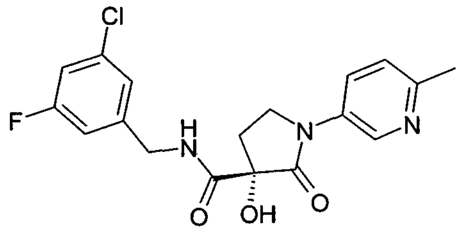 Acide (S)-3-hydroxy-1-(6-méthylpyridin-3-yl)-2-oxo-pyrrolidine-3-carboxylique 3-chloro-5-fluorobenzylamide |
| « A182 » | 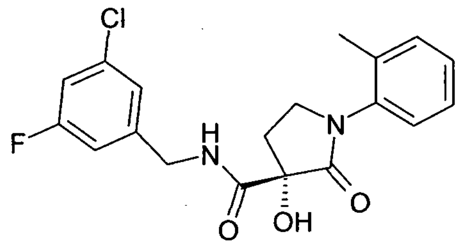 Acide (S)-3-hydroxy-2-oxo-1-o-tolylpyrrolidine-3-carboxylique 3-chloro-5-fluorobenzylamide |
| « A183 » | 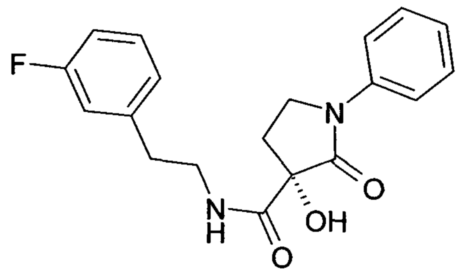 Acide (S)-3-hydroxy-2-oxo-1-phénylpyrrolidine-3-carboxylique [2-(3-fluorophényl)éthyl]amide |
| « A184 » | 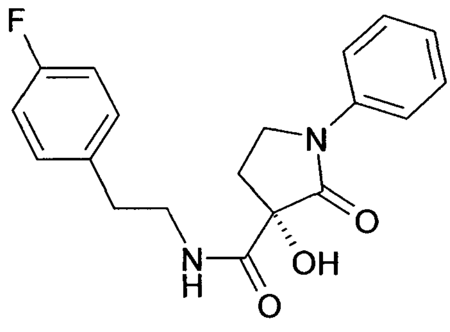 Acide (S)-3-hydroxy-2-oxo-1-phénylpyrrolidine-3-carboxylique [2-(4-fluorophényl)éthyl]amide |

(suite)

| Composé n° | Structure/nom |
|---|---|
| « A185 » | 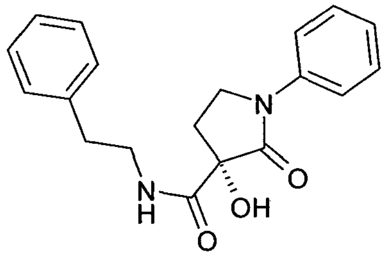<br>Acide (S)-3-hydroxy-2-oxo-1-phénylpyrrolidine-3-carboxylique phénéthylamide |
| « A186 » | 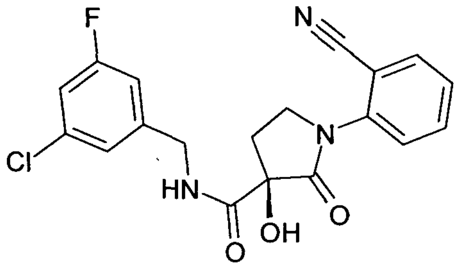<br>Acide (R)-1-(2-cyanophényl)-3-hydroxy-2-oxopyrrolidine-3-carboxylique 3-chloro-5-fluorobenzylamide |
| « A187 » | 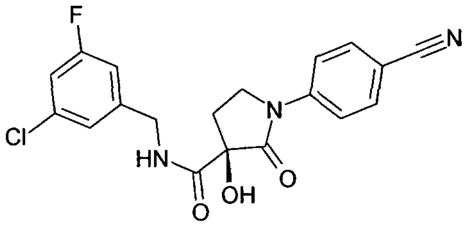<br>Acide (R)-1-(4-cyanophényl)-3-hydroxy-2-oxopyrrolidine-3-carboxylique 3-chloro-5-fluorobenzylamide |
| « A188 » | 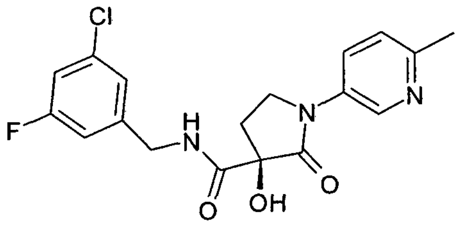<br>Acide (R)-3-hydroxy-1-(6-méthylpyridin-3-yl)-2-oxopyrrolidine-3-carboxylique 3-chloro-5-fluorobenzylamide |
| « A189 » | 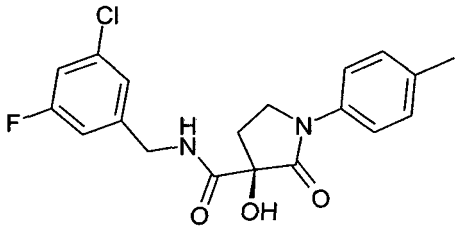<br>Acide (R)-3-hydroxy-2-oxo-1-p-tolylpyrrolidine-3-carboxylique 3-chloro-5-fluorobenzylamide |

(suite)

| Composé n° | Structure/nom |
|---|---|
| « A190 » | 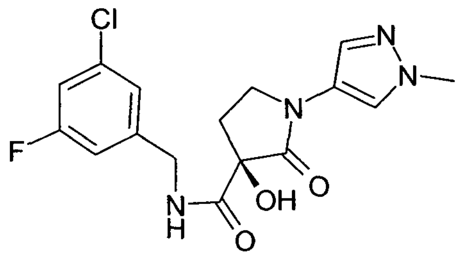 Acide (R)-3-hydroxy-1-(1-méthyl-1H-pyrazol-4-yl)-2-oxopyrrolidine-3-carboxylique 3-chloro-5-fluorobenzylamide |
| « A191 » | 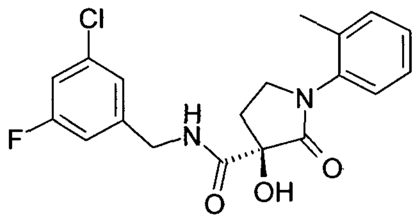 Acide (R)-3-hydroxy-2-oxo-1-o-tolylpyrrolidine-3-carboxylique 3-chloro-5-fluorobenzylamide |
| « A192 » | 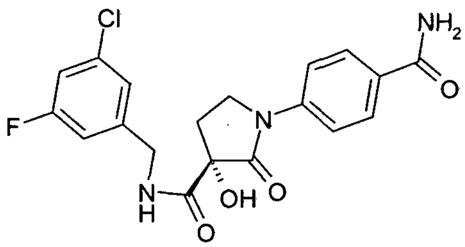 Acide (S)-1-(4-carbamoylphényl)-3-hydroxy-2-oxo-pyrrolidine-3-carboxylique 3-chloro-5-fluorobenzylamide |
| « A193 » | 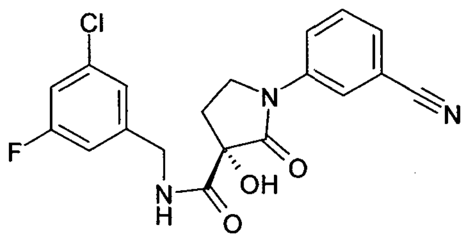 Acide (S)-1-(3-cyanophényl)-3-hydroxy-2-oxopyrrolidine-3-carboxylique 3-chloro-5-fluorobenzylamide |
| « A194 » | 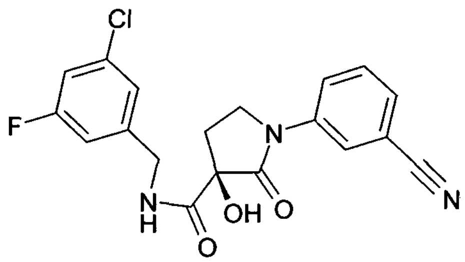 Acide (R)-1-(3-cyanophényl)-3-hydroxy-2-oxopyrrolidine-3-carboxylique 3-chloro-5-fluorobenzylamide |

(suite)

| Composé n° | Structure/nom |
|---|---|
| « A195 » | 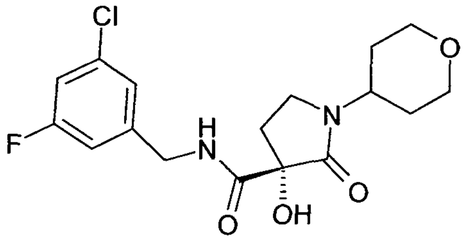<br>Acide (S)-3-hydroxy-2-oxo-1-(tétrahydropyran-4-yl)-pyrrolidine-3-carboxylique 3-chloro-5-fluorobenzylamide |
| « A196 » | 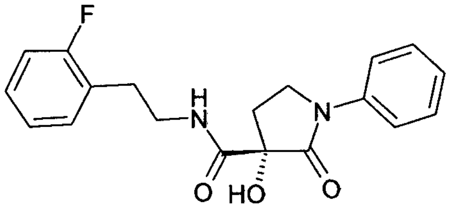<br>Acide (S)-3-hydroxy-2-oxo-1-phénylpyrrolidine-3-carboxylique [2-(2-fluorophényl)éthyl]amide |
| « A197 » | 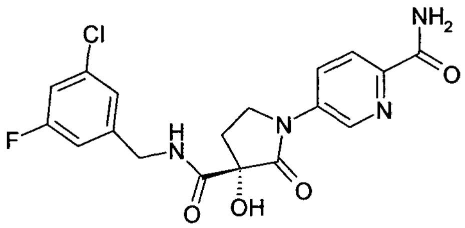<br>Acide 5-[(S)-3-(3-chloro-5-fluorobenzylcarbamoyl)-3-hydroxy-2-oxopyrrolidin-1-yl]pyridine-2-carboxylique amide |
| « A198 » | 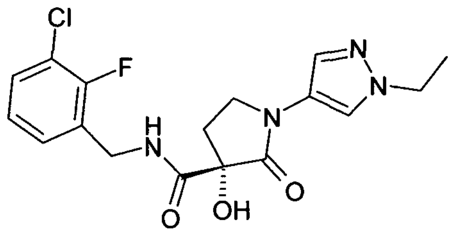<br>Acide (S)-1-(1-éthyl-1H-pyrazol-4-yl)-3-hydroxy-2-oxo-pyrrolidine-3-carboxylique 3-chloro-2-fluorobenzylamide |
| « A199 » | 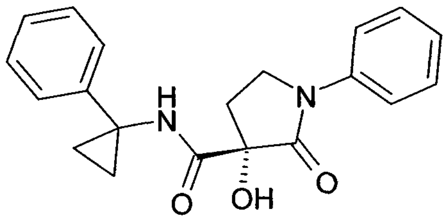<br>Acide (S)-3-hydroxy-2-oxo-1-phénylpyrrolidine-3-carboxylique (1-phénylcyclopropyl)amide |

(suite)

| Composé n° | Structure/nom |
|---|---|
| « A200 » | 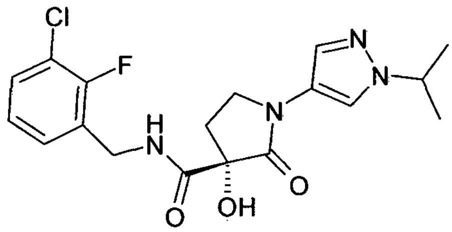 Acide (S)-3-hydroxy-1-(1-isopropyl-1H-pyrazol-4-yl)-2-oxopyrrolidine-3-carboxylique 3-chloro-2-fluorobenzylamide |
| « A201 » | 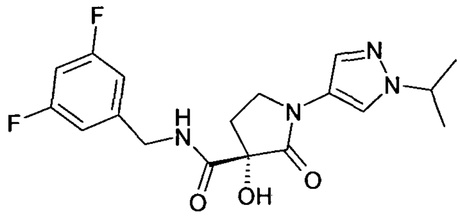 Acide (S)-3-hydroxy-1-(1-isopropyl-1H-pyrazol-4-yl)-2-oxopyrrolidine-3-carboxylique 3,5-difluorobenzylamide |
| « A202 » | 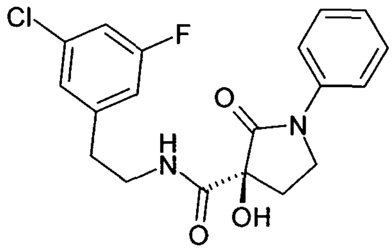 Acide (S)-3-hydroxy-2-oxo-1-phénylpyrrolidine-3-carboxylique [2-(3-chloro-5-fluorophényl)éthyl]amide |
| « A203 » | 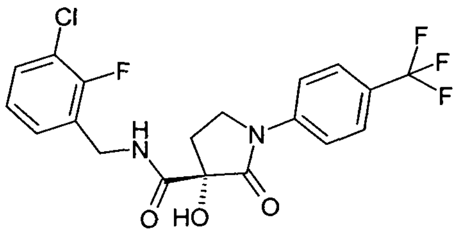 Acide (S)-3-hydroxy-2-oxo-1-(4-trifluorométhylphényl)-pyrrolidine-3-carboxylique 3-chloro-2-fluorobenzylamide |
| « A204 » | 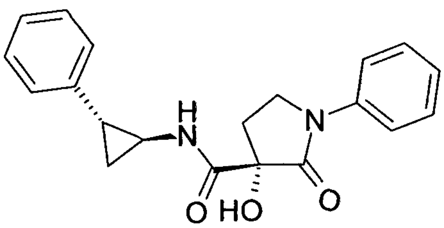 Acide (S)-3-hydroxy-2-oxo-1-phénylpyrrolidine-3-carboxylique ((1S,2R)-2-phénylcyclopropyl)amide |

(suite)

| Composé n° | Structure/nom |
|---|---|
| « A205 » | 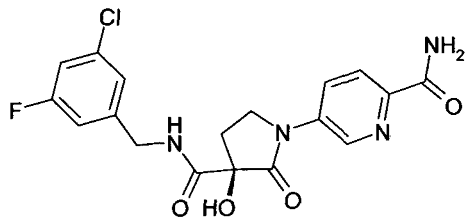 Acide 5-[(R)-3-(3-chloro-5-fluorobenzylcarbamoyl)-3-hydroxy-2-oxopyrrolidin-1-yl]pyridine-2-carboxylique amide |
| « A206 » | 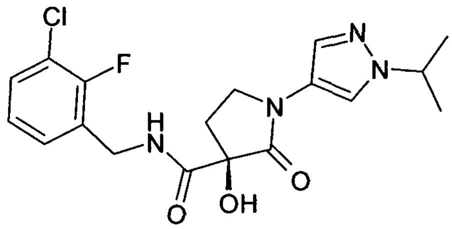 Acide (R)-3-hydroxy-1-(1-isopropyl-1H-pyrazol-4-yl)-2-oxopyrrolidine-3-carboxylique 3-chloro-2-fluorobenzylamide |
| « A207 » | 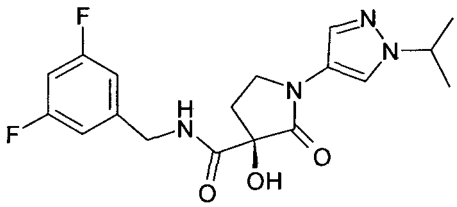 Acide (R)-3-hydroxy-1-(1-isopropyl-1H-pyrazol-4-yl)-2-oxopyrrolidine-3-carboxylique 3,5-difluorobenzylamide |
| « A208 » | 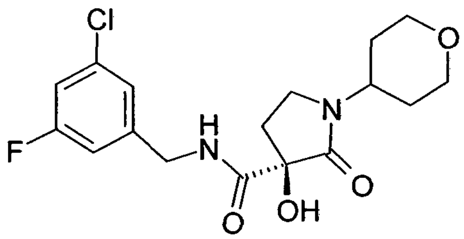 Acide (R)-3-hydroxy-2-oxo-1-(tétrahydropyran-4-yl)-pyrrolidine-3-carboxylique 3-chloro-5-fluorobenzylamide |
| « A209 » | 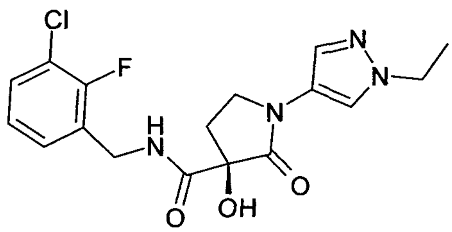 Acide (R)-1-(1-éthyl-1H-pyrazol-4-yl)-3-hydroxy-2-oxo-pyrrolidine-3-carboxylique 3-chloro-2-fluorobenzylamide |

(suite)

| Composé n° | Structure/nom |
|---|---|
| « A210 » | 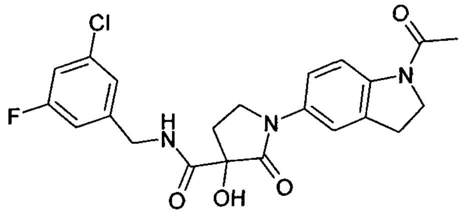<br>Acide 1-(1-acétyl-2,3-dihydro-1H-indol-5-yl)-3-hydroxy-2-oxopyrrolidine-3-carboxylique 3-chloro-5-fluorobenzylamide |
| « A211 » | 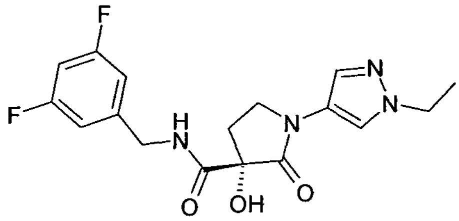<br>Acide (S)-1-(1-éthyl-1H-pyrazol-4-yl)-3-hydroxy-2-oxo-pyrrolidine-3-carboxylique 3,5-difluorobenzylamide |
| « A212 » | 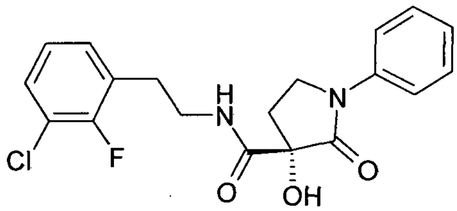<br>Acide (S)-3-hydroxy-2-oxo-1-phénylpyrrolidine-3-carboxylique [2-(3-chloro-2-fluorophényl)éthyl]amide |
| « A213 » | 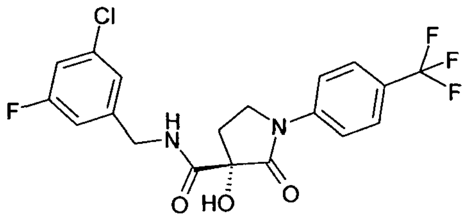<br>Acide (S)-3-hydroxy-2-oxo-1-(4-trifluorométhylphényl)-pyrrolidine-3-carboxylique 3-chloro-5-fluorobenzylamide |
| « A214 » | 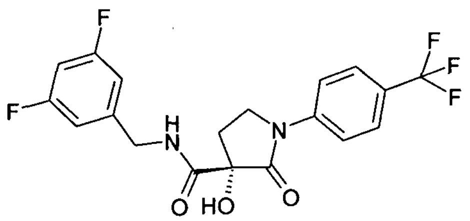<br>Acide (S)-3-hydroxy-2-oxo-1-(4-trifluorométhylphényl)-pyrrolidine-3-carboxylique 3,5-difluorobenzylamide |

(suite)

| Composé n° | Structure/nom |
|---|---|
| « A215 » | 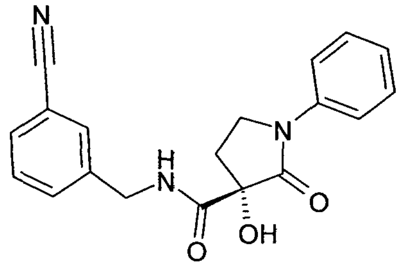<br>Acide (S)-3-hydroxy-2-oxo-1-phénylpyrrolidine-3-carboxylique 3-cyanobenzylamide |
| « A216 » | 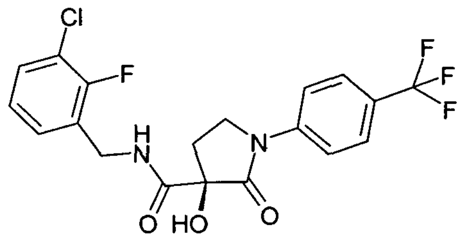<br>Acide (R)-3-hydroxy-2-oxo-1-(4-trifluorométhylphényl)-pyrrolidine-3-carboxylique 3-chloro-2-fluorobenzylamide |
| « A217 » | 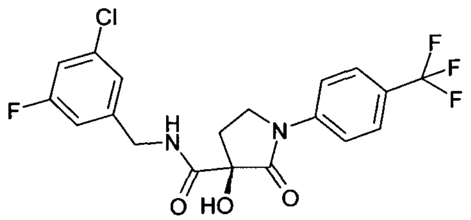<br>Acide (R)-3-hydroxy-2-oxo-1-(4-trifluorométhylphényl)-pyrrolidine-3-carboxylique 3-chloro-5-fluorobenzylamide |
| « A218 » | 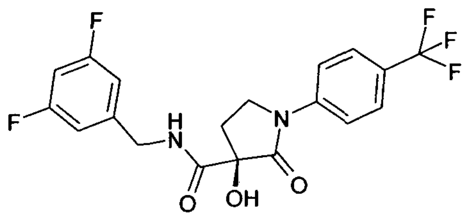<br>Acide (R)-3-hydroxy-2-oxo-1-(4-trifluorométhylphényl)-pyrrolidine-3-carboxylique 3,5-difluorobenzylamide |
| « A219 » | 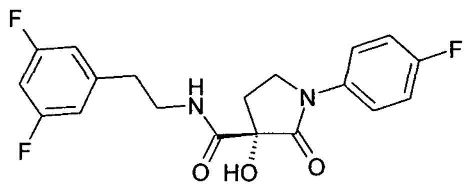<br>Acide (S)-1-(4-fluorophényl)-3-hydroxy-2-oxopyrrolidine-3-carboxylique [2-(3,5-difluorophényl)éthyl]amide |

(suite)

| Composé n° | Structure/nom |
|---|---|
| « A220 » | 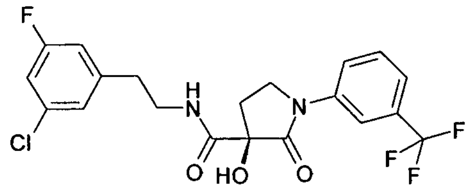<br>Acide (R)-3-hydroxy-2-oxo-1-(3-trifluorométhylphényl)-pyrrolidine-3-carboxylique [2-(3-chloro-5-fluorophényl)-éthyl]amide |
| « A221 » | 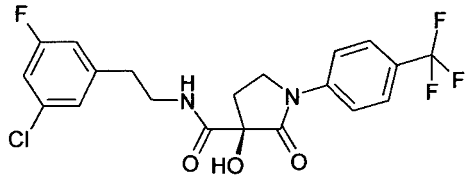<br>Acide (R)-3-hydroxy-2-oxo-1-(4-trifluorométhylphényl)-pyrrolidine-3-carboxylique [2-(3-chloro-5-fluorophényl)-éthyl]amide |
| « A222 » | 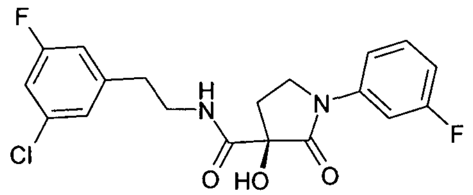<br>Acide (R)-1-(3-fluorophényl)-3-hydroxy-2-oxopyrrolidine-3-carboxylique [2-(3-chloro-5-fluorophényl)éthyl]amide |
| « A223 » | 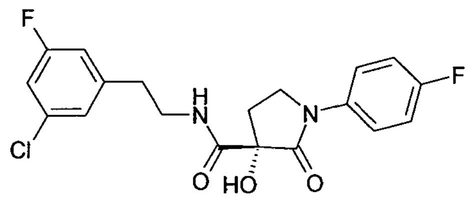<br>Acide (S)-1-(4-fluorophényl)-3-hydroxy-2-oxopyrrolidine-3-carboxylique [2-(3-chloro-5-fluorophényl)éthyl]amide |
| « A224 » | 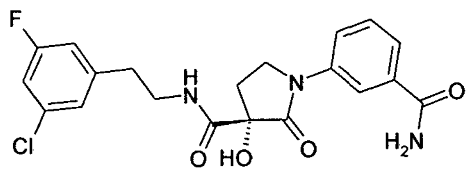<br>Acide (S)-1-(3-carbamoylphényl)-3-hydroxy-2-oxo-pyrrolidine-3-carboxylique [2-(3-chloro-5-fluorophényl)-éthyl]amide |
| « A225 » | 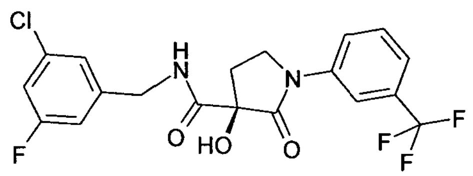<br>Acide (R)-3-hydroxy-2-oxo-1-(3-trifluorométhylphényl)-pyrrolidine-3-carboxylique 3-chloro-5-fluorobenzylamide |

(suite)

| Composé n° | Structure/nom |
|---|---|
| « A226 » | 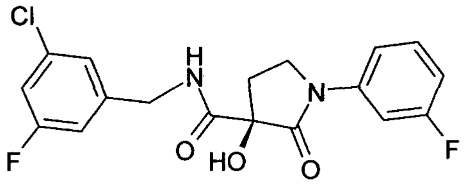<br>Acide (R)-1-(3-fluorophényl)-3-hydroxy-2-oxopyrrolidine-3-carboxylique 3-chloro-5-fluorobenzylamide |
| « A227 » | 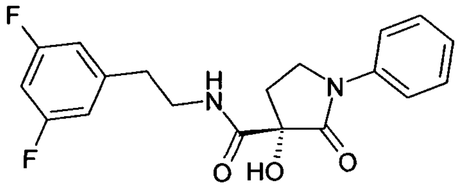<br>Acide (S)-3-hydroxy-2-oxo-1-phénylpyrrolidine-3-carboxylique [2-(3,5-difluorophényl)éthyl]amide |
| « A228 » | 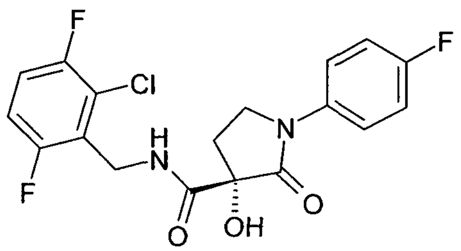<br>Acide (S)-1-(4-fluorophényl)-3-hydroxy-2-oxopyrrolidine-3-carboxylique 2-chloro-3,6-difluorobenzylamide |
| « A229 » | 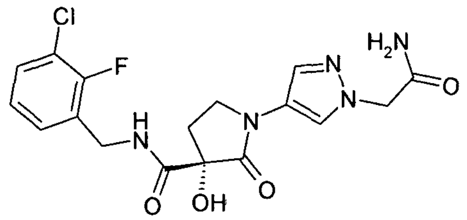<br>Acide (S)-1-(1-carbamoylméthyl-1H-pyrazol-4-yl)-3-hydroxy-2-oxopyrrolidine-3-carboxylique 3-chloro-2-fluorobenzylamide |
| « A230 » | 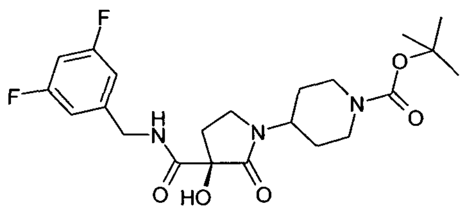<br>4-[(R)-3-(3,5-Difluorobenzylcarbamoyl)-3-hydroxy-2-oxopyrrolidin-1-yl]pipéridine-1-carboxylate de tertio-butyle |
| « A231 » | 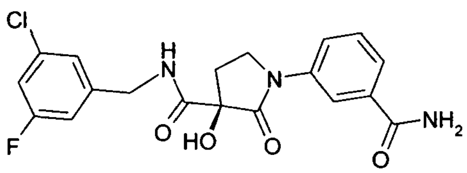<br>Acide (R)-1-(3-carbamoylphényl)-3-hydroxy-2-oxo-pyrrolidine-3-carboxylique 3-chloro-5-fluorobenzylamide |

(suite)

| Composé n° | Structure/nom |
|---|---|
| « A232 » | 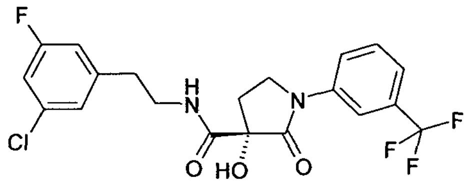<br>Acide (S)-3-hydroxy-2-oxo-1-(3-trifluorométhylphényl)-pyrrolidine-3-carboxylique [2-(3-chloro-5-fluorophényl)-éthyl]amide |
| « A233 » | 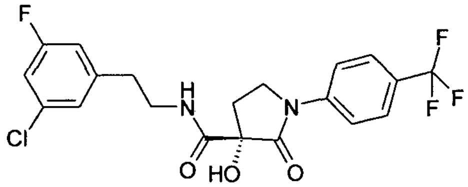<br>Acide (S)-3-hydroxy-2-oxo-1-(4-trifluorométhylphényl)-pyrrolidine-3-carboxylique [2-(3-chloro-5-fluorophényl)-éthyl]amide |
| « A234 » | 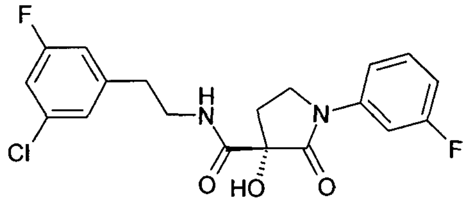<br>Acide (S)-1-(3-fluorophényl)-3-hydroxy-2-oxopyrrolidine-3-carboxylique [2-(3-chloro-5-fluorophényl)éthyl]amide |
| « A235 » | 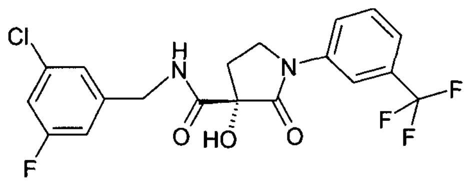<br>Acide (S)-3-hydroxy-2-oxo-1-(3-trifluorométhylphényl)-pyrrolidine-3-carboxylique 3-chloro-5-fluorobenzylamide |
| « A236 » | 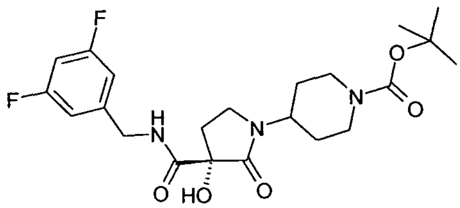<br>4-[(S)-3-(3,5-Difluorobenzylcarbamoyl)-3-hydroxy-2-oxo-pyrrolidin-1-yl]pipéridine-1-carboxylate de tertio-butyle |
| « A237 » | 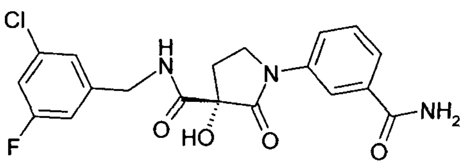<br>Acide (S)-1-(3-carbamoylphényl)-3-hydroxy-2-oxo-pyrrolidine-3-carboxylique 3-chloro-5-fluorobenzylamide |

(suite)

| Composé n° | Structure/nom |
|---|---|
| « A238 » | 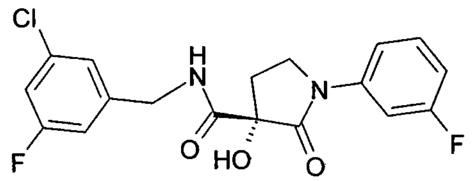<br>Acide (S)-1-(3-fluorophényl)-3-hydroxy-2-oxopyrrolidine-3-carboxylique 3-chloro-5-fluorobenzylamide |
| « A239 » | 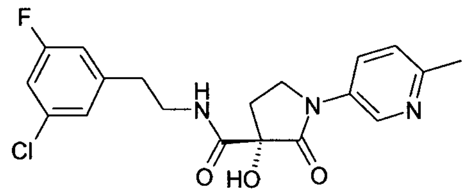<br>Acide (S)-3-hydroxy-1-(6-méthylpyridin-3-yl)-2-oxo-pyrrolidine-3-carboxylique [2-(3-chloro-5-fluorophényl)-éthyl]amide |
| « A240 » | 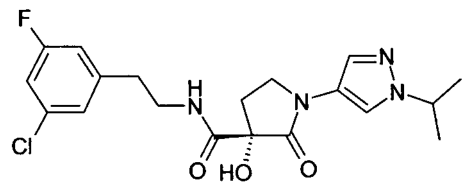<br>Acide (S)-3-hydroxy-1-(1-isopropyl-1H-pyrazol-4-yl)-2-oxopyrrolidine-3-carboxylique [2-(3-chloro-5-fluorophényl)-éthyl]amide |
| « A241 » | 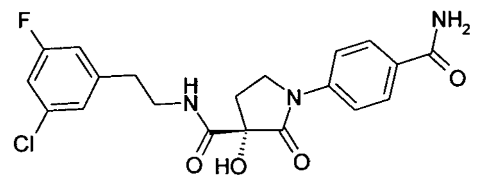<br>Acide (S)-1-(4-carbamoylphényl)-3-hydroxy-2-oxo-pyrrolidine-3-carboxylique [2-(3-chloro-5-fluorophényl)-éthyl]amide |
| « A242 » | 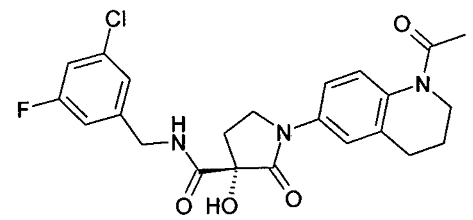<br>Acide (S)-1-(1-acétyl-1,2,3,4-tétrahydroquinoléin-6-yl)-3-hydroxy-2-oxopyrrolidine-3-carboxylique 3-chloro-5-fluorobenzylamide |
| « A243 » | 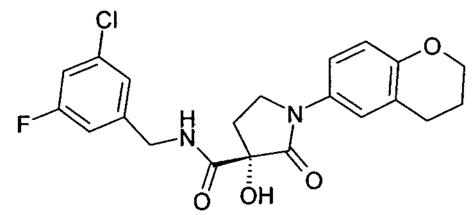<br>Acide (S)-1-chroman-6-yl-3-hydroxy-2-oxopyrrolidine-3-carboxylique 3-chloro-5-fluorobenzylamide |

(suite)

| Composé n° | Structure/nom |
|---|---|
| « A244 » | 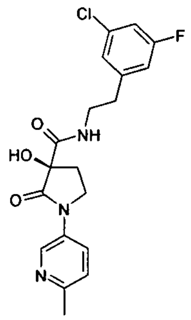 Acide (R)-3-hydroxy-1-(6-méthylpyridin-3-yl)-2-oxo-pyrrolidine-3-carboxylique [2-(3-chloro-5-fluorophényl)-éthyl]amide |
| « A245 » | 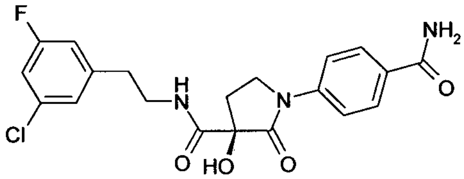 Acide (R)-1-(4-carbamoylphényl)-3-hydroxy-2-oxo-pyrrolidine-3-carboxylique [2-(3-chloro-5-fluorophényl)-éthyl]amide |
| « A246 » | 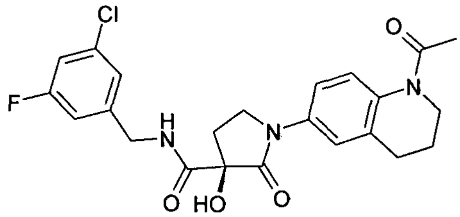 Acide (R)-1-(1-acétyl-1,2,3,4-tétrahydroquinoléin-6-yl)-3-hydroxy-2-oxopyrrolidine-3-carboxylique 3-chloro-5-fluorobenzylamide |
| « A247 » | 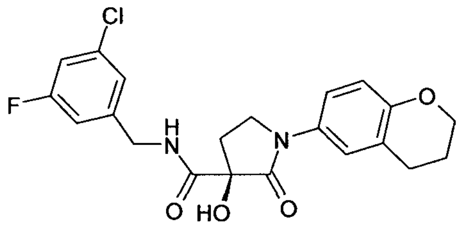 Acide (R)-1-chroman-6-yl-3-hydroxy-2-oxopyrrolidine-3-carboxylique 3-chloro-5-fluorobenzylamide |
| « A248 » | 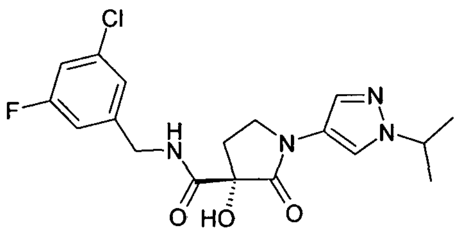 Acide (S)-3-hydroxy-1-(1-isopropyl-1H-pyrazol-4-yl)-2-oxopyrrolidine-3-carboxylique 3-chloro-5-fluorobenzylamide |

(suite)

| Composé n° | Structure/nom |
|---|---|
| « A249 » | 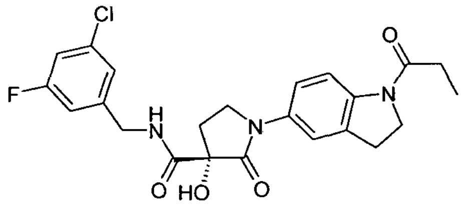 Acide (S)-3-hydroxy-2-oxo-1-(1-propionyl-2,3-dihydro-1H-indol-5-yl)pyrrolidine-3-carboxylique 3-chloro-5-fluorobenzylamide |
| « A250 » | 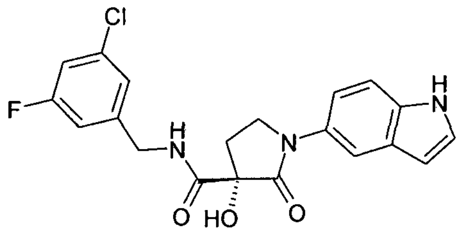 Acide (S)-3-hydroxy-1-(1H-indol-5-yl)-2-oxopyrrolidine-3-carboxylique 3-chloro-5-fluorobenzylamide |
| « A251 » | 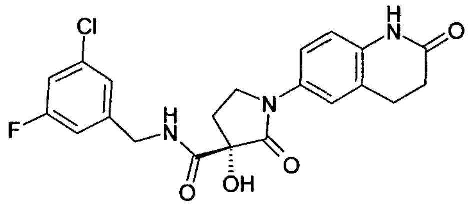 Acide (S)-3-hydroxy-2-oxo-1-(2-oxo-1,2,3,4-tétrahydro-quinoléin-6-yl)pyrrolidine-3-carboxylique 3-chloro-5-fluorobenzylamide |
| « A252 » | 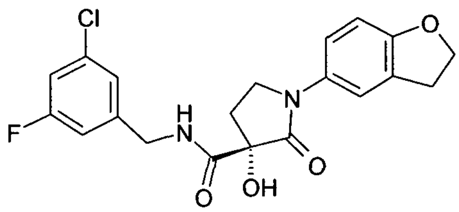 Acide (S)-1-(2,3-dihydrobenzofuran-5-yl)-3-hydroxy-2-oxopyrrolidine-3-carboxylique 3-chloro-5-fluorobenzylamide |
| « A253 » | 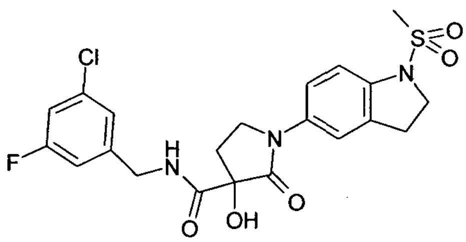 Acide 3-hydroxy-1-(1-méthanesulfonyl-2,3-dihydro-1H-indol-5-yl)-2-oxopyrrolidine-3-carboxylique 3-chloro-5-fluorobenzylamide |

(suite)

| Composé n° | Structure/nom |
|---|---|
| « A254 » | 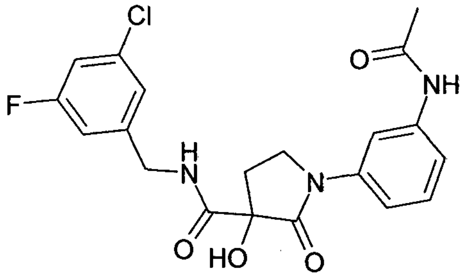<br>Acide 1-(3-acétylaminophényl)-3-hydroxy-2-oxopyrrolidine-3-carboxylique 3-chloro-5-fluorobenzylamide |
| « A255 » | 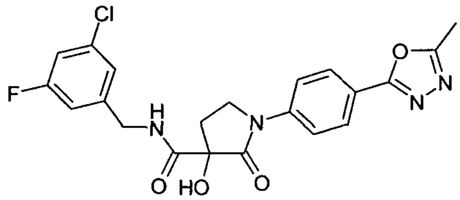<br>Acide 3-hydroxy-1-[4-(5-méthyl-1,3,4-oxadiazol-2-yl)-phényl]-2-oxopyrrolidine-3-carboxylique 3-chloro-5-fluorobenzylamide |
| « A256 » | 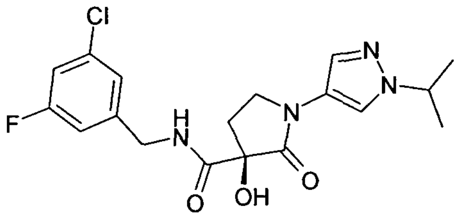<br>Acide (R)-3-hydroxy-1-(1-isopropyl-1H-pyrazol-4-yl)-2-oxopyrrolidine-3-carboxylique 3-chloro-5-fluorobenzylamide |
| « A257 » | 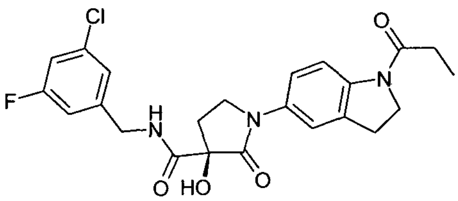<br>Acide (R)-3-hydroxy-2-oxo-1-(1-propionyl-2,3-dihydro-1H-indol-5-yl)pyrrolidine-3-carboxylique 3-chloro-5-fluorobenzylamide |
| « A258 » | 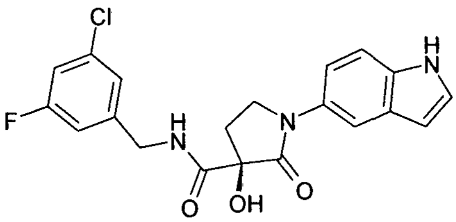<br>Acide (R)-3-hydroxy-1-(1H-indol-5-yl)-2-oxopyrrolidine-3-carboxylique 3-chloro-5-fluorobenzylamide |

(suite)

| Composé n° | Structure/nom |
|---|---|
| « A259 » | 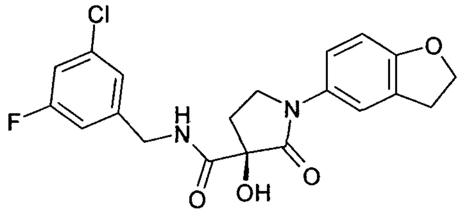<br>Acide (R)-1-(2,3-dihydrobenzofuran-5-yl)-3-hydroxy-2-oxopyrrolidine-3-carboxylique 3-chloro-5-fluorobenzylamide |
| « A260 » | 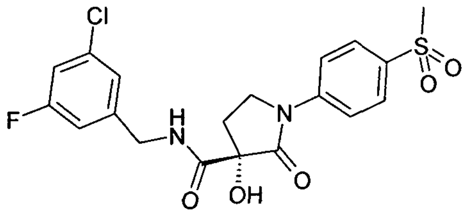<br>Acide (S)-3-hydroxy-1-(4-méthanesulfonylphényl)-2-oxopyrrolidine-3-carboxylique 3-chloro-5-fluorobenzylamide |
| « A261 » | 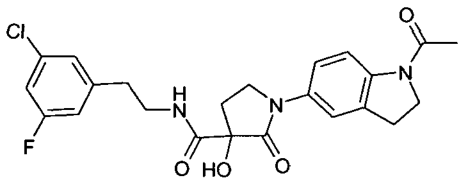<br>Acide 1-(1-acétyl-2,3-dihydro-1H-indol-5-yl)-3-hydroxy-2-oxopyrrolidine-3-carboxylique [2-(3-chloro-5-fluorophényl)-éthyl]amide |
| « A262 » | 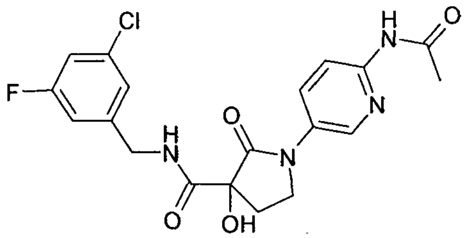<br>Acide 1-(6-acétylaminopyridin-3-yl)-3-hydroxy-2-oxo-pyrrolidine-3-carboxylique 3-chloro-5-fluorobenzylamide |
| « A263 » | 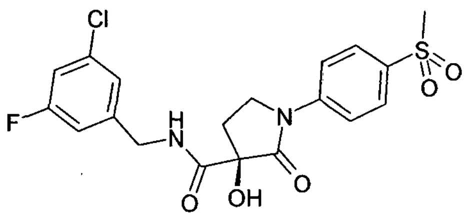<br>Acide (R)-3-hydroxy-1-(4-méthanesulfonylphényl)-2-oxo-pyrrolidine-3-carboxylique 3-chloro-5-fluorobenzylamide |
| « A264 » | 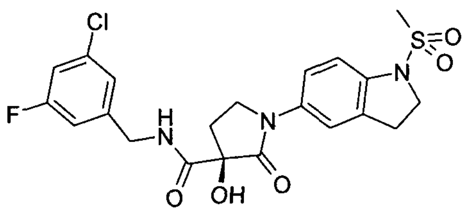 |

(suite)

| Composé n° | Structure/nom |
|---|---|
| | Acide (R)-3-hydroxy-1-(1-méthanesulfonyl-2,3-dihydro-1H-indol-5-yl)-2-oxopyrrolidine-3-carboxylique 3-chloro-5-fluorobenzylamide |
| « A265 » | 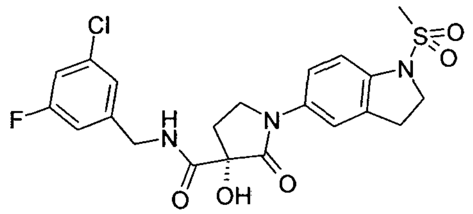<br>Acide (S)-3-hydroxy-1-(1-méthanesulfonyl-2,3-dihydro-1H-indol-5-yl)-2-oxopyrrolidine-3-carboxylique 3-chloro-5-fluorobenzylamide |
| « A266 » | 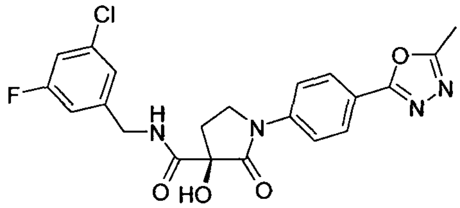<br>Acide (R)-3-hydroxy-1-[4-(5-méthyl-1,3,4-oxadiazol-2-yl)-phényl]-2-oxopyrrolidine-3-carboxylique 3-chloro-5-fluorobenzylamide |
| « A267 » | 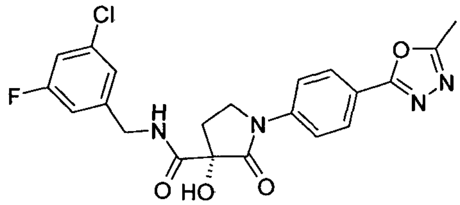<br>Acide (S)-3-hydroxy-1-(1-méthanesulfonyl-2,3-dihydro-1H-indol-5-yl)-2-oxopyrrolidine-3-carboxylique 3-chloro-5-fluorobenzylamide |
| « A268 » | 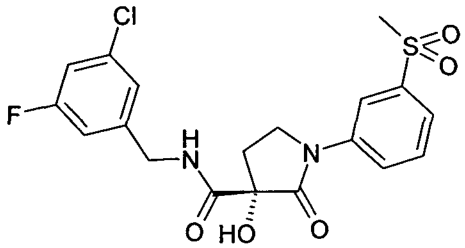<br>Acide (S)-3-hydroxy-1-(3-méthanesulfonylphényl)-2-oxopyrrolidine-3-carboxylique 3-chloro-5-fluorobenzylamide |
| « A269 » | 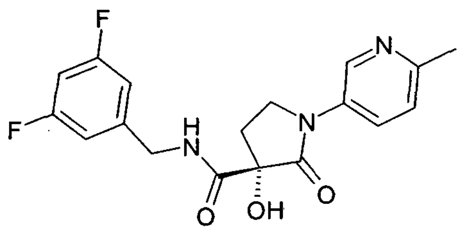<br>Acide (S)-3-hydroxy-1-(6-méthylpyridin-3-yl)-2-oxo-pyrrolidine-3-carboxylique 3,5-difluorobenzylamide |

(suite)

| Composé n° | Structure/nom |
|---|---|
| « A270 » | 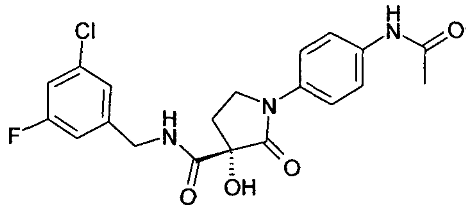 Acide (S)-1-(4-acétylaminophényl)-3-hydroxy-2-oxo-pyrrolidine-3-carboxylique 3-chloro-5-fluorobenzylamide |
| « A271 » | 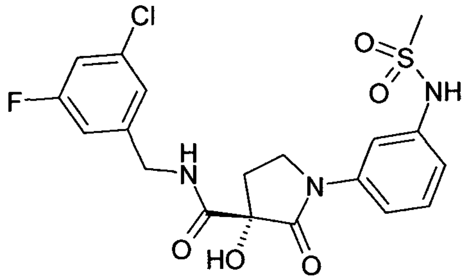 Acide (S)-3-hydroxy-1-(3-méthanesulfonylaminophényl)-2-oxopyrrolidine-3-carboxylique 3-chloro-5-fluorobenzylamide |
| « A272 » | 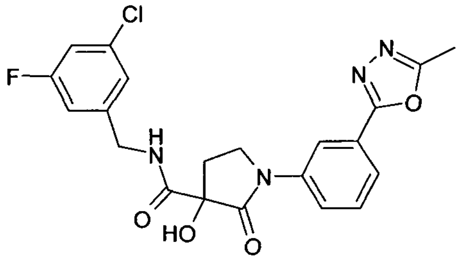 Acide 3-hydroxy-1-[3-(5-méthyl-1,3,4-oxadiazol-2-yl)-phényl]-2-oxopyrrolidine-3-carboxylique 3-chloro-5-fluorobenzylamide |
| « A273 » | 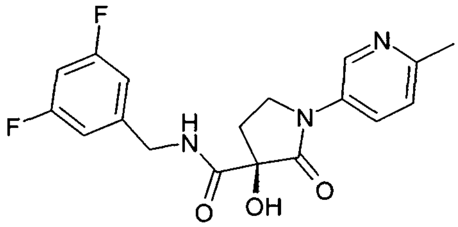 Acide (R)-3-hydroxy-1-(6-méthylpyridin-3-yl)-2-oxo-pyrrolidine-3-carboxylique 3,5-difluorobenzylamide |
| « A274 » | 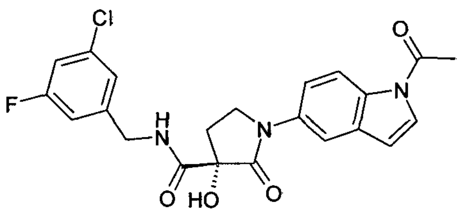 Acide (S)-1-(1-acétyl-1H-indol-5-yl)-3-hydroxy-2-oxo-pyrrolidine-3-carboxylique 3-chloro-5-fluorobenzylamide |

(suite)

| Composé n° | Structure/nom |
|---|---|
| « A275 » | 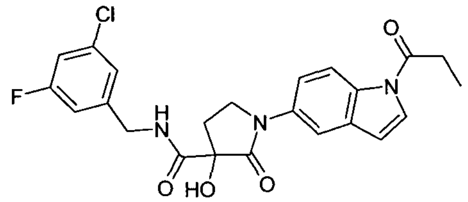 Acide 3-hydroxy-2-oxo-1-(1-propionyl-1H-indol-5-yl)-pyrrolidine-3-carboxylique 3-chloro-5-fluorobenzylamide |
| « A276 » | 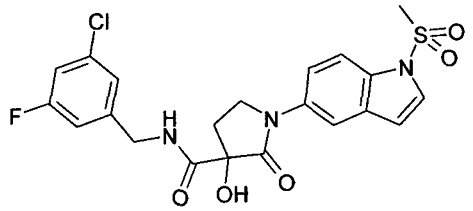 Acide 3-hydroxy-1-(1-méthanesulfonyl-1 H-indol-5-yl)-2-oxopyrrolidine-3-carboxylique 3-chloro-5-fluorobenzylamide |
| « A277 » | 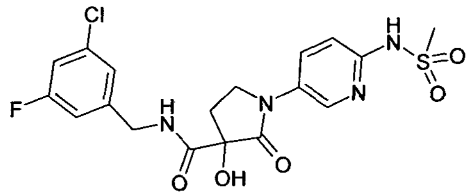 Acide 3-hydroxy-1-(6-méthanesulfonylaminopyridin-3-yl)-2-oxopyrrolidine-3-carboxylique 3-chloro-5-fluorobenzylamide |
| « A278 » | 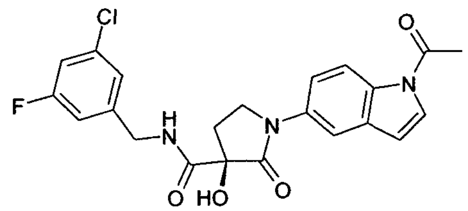 Acide (R)-1-(1-acétyl-1H-indol-5-yl)-3-hydroxy-2-oxo-pyrrolidine-3-carboxylique 3-chloro-5-fluorobenzylamide |
| « A279 » | 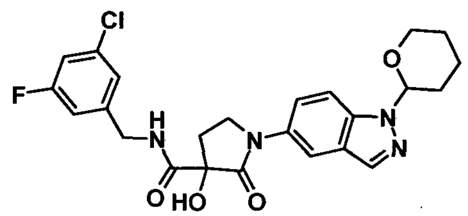 Acide 3-hydroxy-2-oxo-1-[1-(tétrahydropyran-2-yl)-1H-indazol-5-yl]pyrrolidine-3-carboxylique 3-chloro-5-fluorobenzylamide |
| « A280 » | 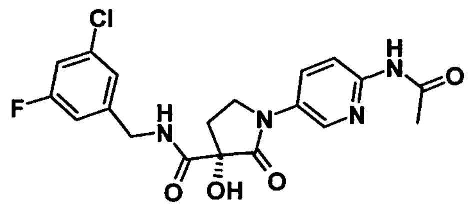 |

(suite)

| Composé n° | Structure/nom |
|---|---|
| | Acide (S)-1-(6-acétylaminopyridin-3-yl)-3-hydroxy-2-oxo-pyrrolidine-3-carboxylique 3-chloro-5-fluorobenzylamide |
| « A281 » | 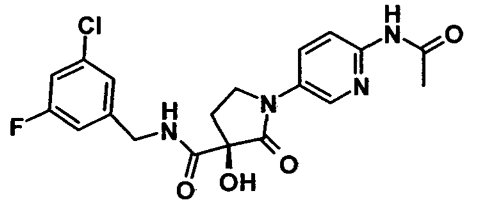 Acide (R)-1-(6-acétylaminopyridin-3-yl)-3-hydroxy-2-oxo-pyrrolidine-3-carboxylique 3-chloro-5-fluorobenzylamide |
| « A282 » | 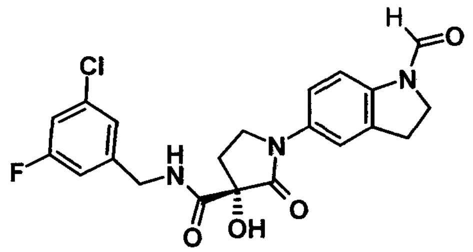 Acide (S)-1-(1-formyl-2,3-dihydro-1H-indol-5-yl)-3-hydroxy-2-oxopyrrolidine-3-carboxylique 3-chloro-5-fluorobenzylamide |
| « A283 » | 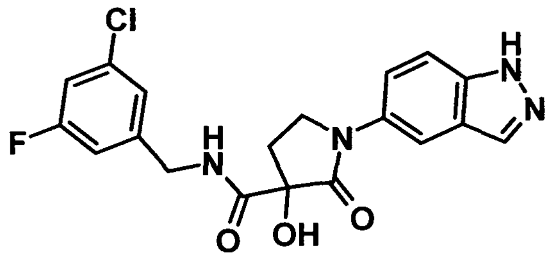 Acide 3-hydroxy-1-(1H-indazol-5-yl)-2-oxopyrrolidine-3-carboxylique 3-chloro-5-fluorobenzylamide |
| « A284 » | 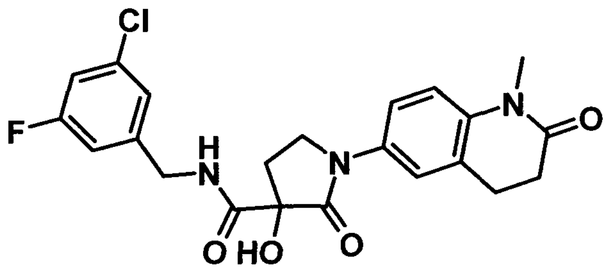 Acide 3-hydroxy-1-(1-méthyl-2-oxo-1,2,3,4-tétrahydro-quinoléin-6-yl)-2-oxopyrrolidine-3-carboxylique 3-chloro-5-fluorobenzylamide |
| « A285 » | 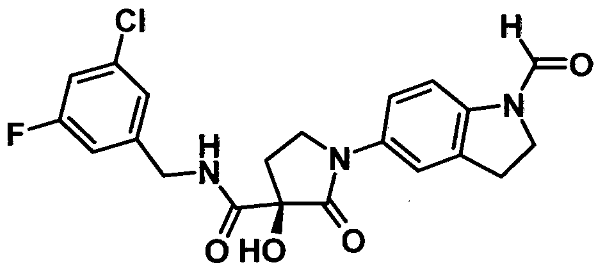 Acide (R)-1-(1-formyl-2,3-dihydro-1H-indol-5-yl)-3-hydroxy-2-oxopyrrolidine-3-carboxylique 3-chloro-5-fluorobenzylamide |

(suite)

| Composé n° | Structure/nom |
|---|---|
| « A286 » | 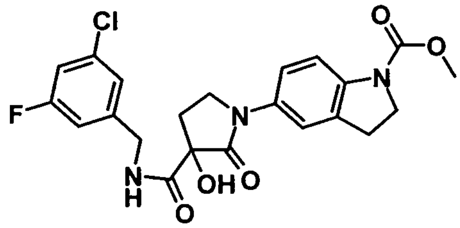 5-[3-(3-Chloro-5-fluorobenzylcarbamoyl)-3-hydroxy-2-oxopyrrolidin-1-yl]-2,3-dihydroindole-1-carboxylate de méthyle |
| « A287 » | 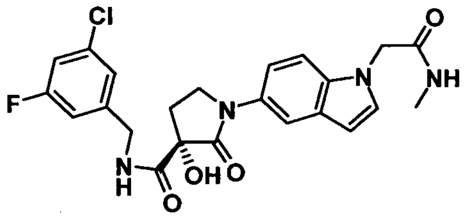 Acide (S)-3-hydroxy-1-(1-méthylcarbamoylméthyl-1H-indol-5-yl)-2-oxopyrrolidine-3-carboxylique 3-chloro-5-fluorobenzylamide |
| « A288 » | 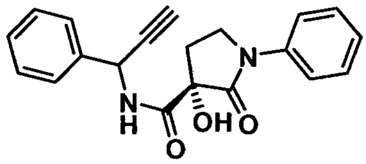 Acide (S)-3-hydroxy-2-oxo-1-phénylpyrrolidine-3-carboxylique (1-phénylprop-2-ynyl)amide |
| « A289 » | 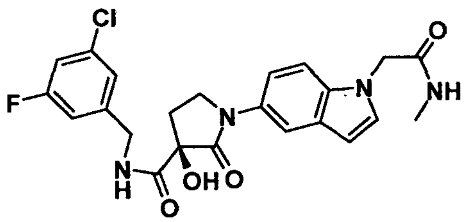 Acide (R)-3-hydroxy-1-(1-méthylcarbamoylméthyl-1H-indol-5-yl)-2-oxopyrrolidine-3-carboxylique 3-chloro-5-fluoro-benzylamide |
| « A290 » | 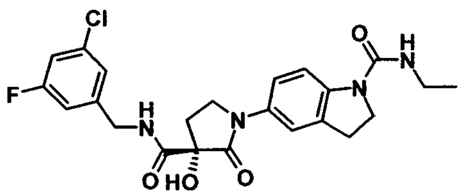 Acide 5-[(S)-3-(3-chloro-5-fluorobenzylcarbamoyl)-3-hydroxy-2-oxopyrrolidin-1-yl]-2,3-dihydroindole-1-carboxylique éthylamide |
| « A291 » | 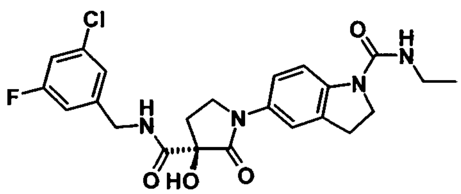 Acide 5-[(R)-3-(3-chloro-5-fluorobenzylcarbamoyl)-3-hydroxy-2-oxopyrrolidin-1-yl]-2,3-dihydroindole-1-carboxylique éthylamide |

(suite)

| Composé n° | Structure/nom |
|---|---|
| « A292 » | 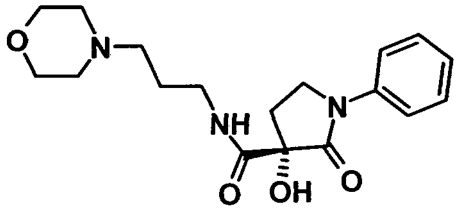 Acide (S)-3-hydroxy-2-oxo-1-phénylpyrrolidine-3-carboxylique (3-morpholin-4-ylpropyl)amide |
| « A293 » | 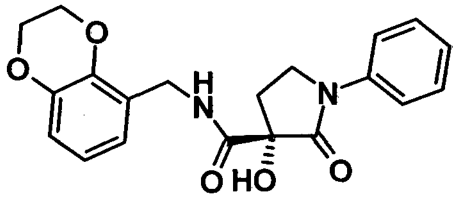 Acide (S)-3-hydroxy-2-oxo-1-phénylpyrrolidine-3-carboxylique (2,3-dihydrobenzo-1,4-dioxin-5-ylméthyl)amide |
| « A294 » | 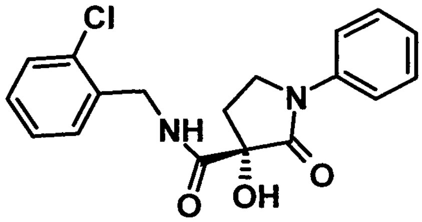 Acide (S)-3-hydroxy-2-oxo-1-phénylpyrrolidine-3-carboxylique 2-chlorobenzylamide |
| « A295 » | 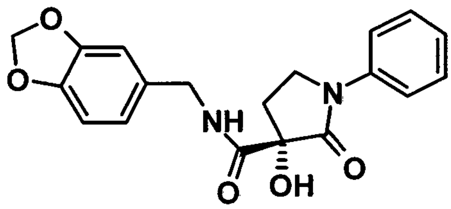 Acide (S)-3-hydroxy-2-oxo-1-phénylpyrrolidine-3-carboxylique (benzo-1,3-dioxol-5-ylméthyl)amide |
| « A296 » | 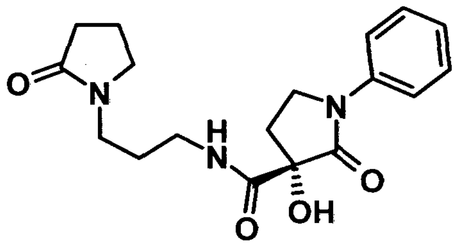 Acide (S)-3-hydroxy-2-oxo-1-phénylpyrrolidine-3-carboxylique [3-(2-oxopyrrolidin-1-yl)-propyl]amide |
| « A297 » | 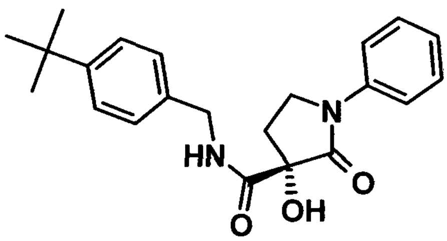 Acide (S)-3-hydroxy-2-oxo-1-phénylpyrrolidine-3-carboxylique 4-tertio-butylbenzylamide |

(suite)

| Composé n° | Structure/nom |
|---|---|
| « A298 » | 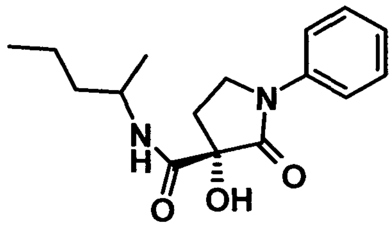 Acide (S)-3-hydroxy-2-oxo-1-phénylpyrrolidine-3-carboxylique (1-méthylbutyl)amide |
| « A299 » | 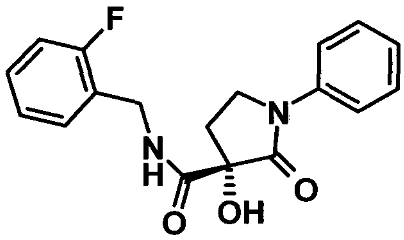 Acide (S)-3-hydroxy-2-oxo-1-phénylpyrrolidine-3-carboxylique 2-fluorobenzylamide |
| « A300 » | 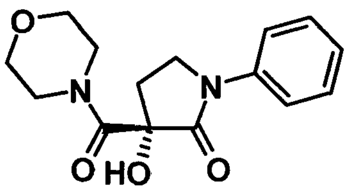 (S)-3-Hydroxy-3-(morpholino-4-carbonyl)-1-phényl-pyrrolidin-2-one |
| « A301 » | 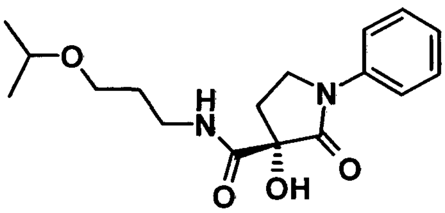 Acide (S)-3-hydroxy-2-oxo-1-phénylpyrrolidine-3-carboxylique (3-isopropoxypropyl)amide |
| « A302 » | 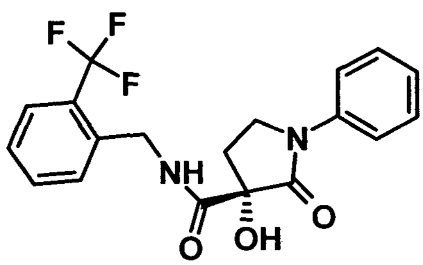 Acide (S)-3-hydroxy-2-oxo-1-phénylpyrrolidine-3-carboxylique 2-trifluorométhylbenzylamide |
| « A303 » | 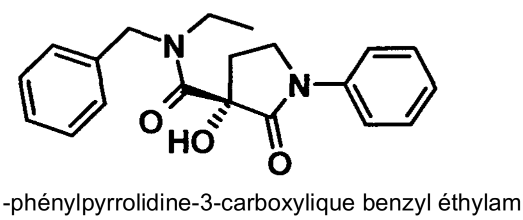 Acide (S)-3-hydroxy-2-oxo-1-phénylpyrrolidine-3-carboxylique benzyl éthylamide |

(suite)

| Composé n° | Structure/nom |
|---|---|
| « A304 » | 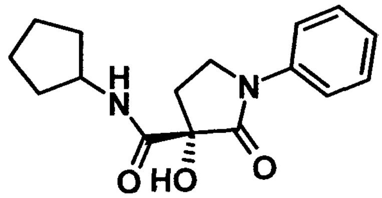 Acide (S)-3-hydroxy-2-oxo-1-phénylpyrrolidine-3-carboxylique cyclopentylamide |
| « A305 » | 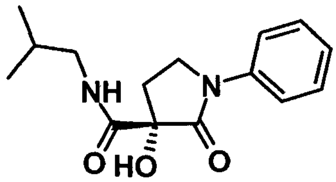 Acide (S)-3-hydroxy-2-oxo-1-phénylpyrrolidine-3-carboxylique isobutylamide |
| « A306 » | 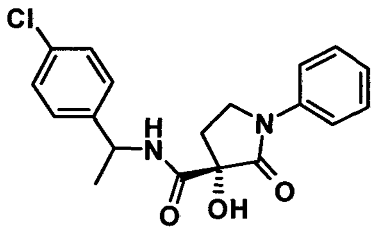 Acide (S)-3-hydroxy-2-oxo-1-phénylpyrrolidine-3-carboxylique [1-(4-chlorophényl)éthyl]amide |
| « A307 » | 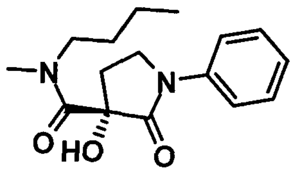 Acide (S)-3-hydroxy-2-oxo-1-phénylpyrrolidine-3-carboxylique butyl méthylamide |
| « A308 » | 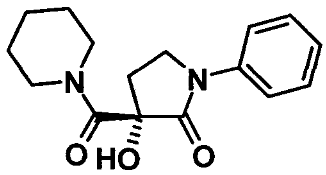 (S)-3-Hydroxy-1-phényl-3-(pipéridine-1-carbonyl)pyrrolidin-2-one |
| « A309 » | 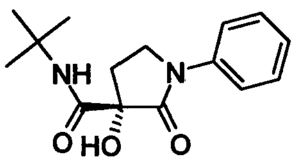 Acide (S)-3-hydroxy-2-oxo-1-phénylpyrrolidine-3-carboxylique tertio-butylamide |
| « A310 » | 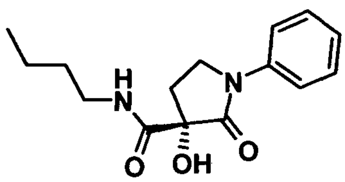 Acide (S)-3-hydroxy-2-oxo-1-phénylpyrrolidine-3-carboxylique butylamide |

(suite)

| Composé n° | Structure/nom |
|---|---|
| « A311 » | 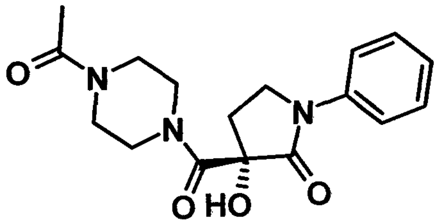<br>(S)-3-(4-Acétylpipérazine-1'-carbonyl)-3-hydroxy-1-phénylpyrrolidin-2-one |
| « A312 » | 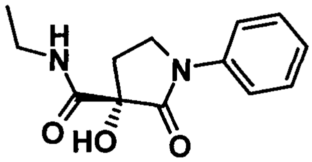<br>Acide (S)-3-hydroxy-2-oxo-1-phénylpyrrolidine-3-carboxylique éthylamide |
| « A313 » | 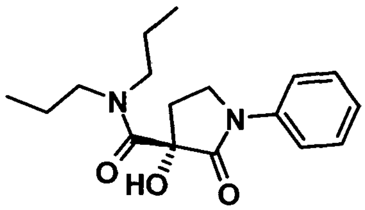<br>Acide (S)-3-hydroxy-2-oxo-1-phénylpyrrolidine-3-carboxylique dipropylamide |
| « A314 » | 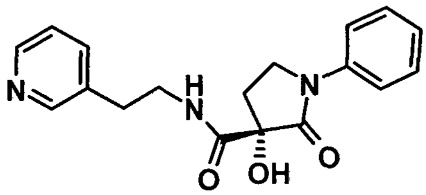<br>Acide (S)-3-hydroxy-2-oxo-1-phénylpyrrolidine-3-carboxylique (2-pyridin-3-yléthyl)amide |
| « A315 » | 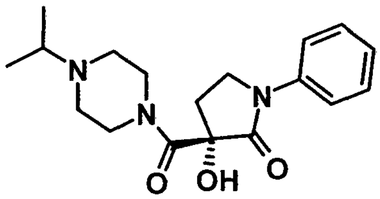<br>(S)-3-Hydroxy-3-(4-isopropylpipérazine-1-carbonyl)-1-phénylpyrrolidin-2-one |
| « A316 » | 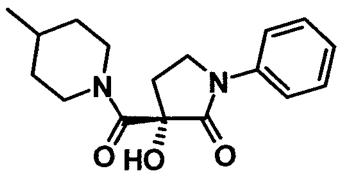<br>(S)-3-Hydroxy-3-(4-méthylpipéridine-1-carbonyl)-1-phénylpyrrolidin-2-one |
| « A317 » | 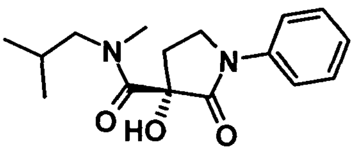<br>Acide (S)-3-hydroxy-2-oxo-1-phénylpyrrolidine-3-carboxylique isobutyl méthylamide |

(suite)

| Composé n° | Structure/nom |
|---|---|
| « A318 » | 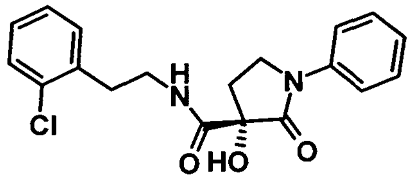 Acide (S)-3-hydroxy-2-oxo-1-phénylpyrrolidine-3-carboxylique [2-(2-chlorophényl)éthyl]amide |
| « A319 » | 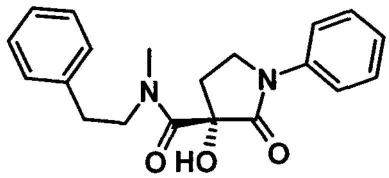 Acide (S)-3-hydroxy-2-oxo-1-phénylpyrrolidine-3-carboxylique méthyl phénéthylamide |
| « A320 » | 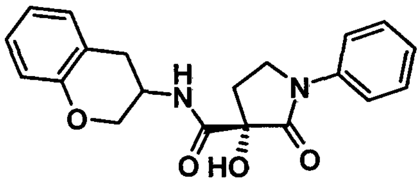 Acide (S)-3-hydroxy-2-oxo-1-phénylpyrrolidine-3-carboxylique chroman-3-ylamide |
| « A321 » | 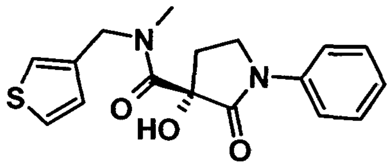 Acide (S)-3-hydroxy-2-oxo-1-phénylpyrrolidine-3-carboxylique méthyl thiophén-3-ylméthylamide |
| « A322 » | 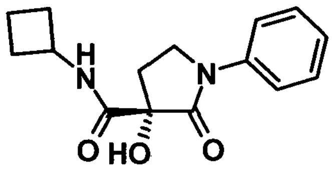 Acide (S)-3-hydroxy-2-oxo-1-phénylpyrrolidine-3-carboxylique cyclobutylamide |
| « A323 » | 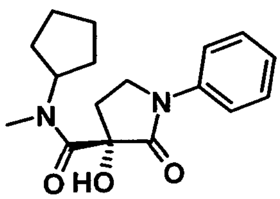 Acide (S)-3-hydroxy-2-oxo-1-phénylpyrrolidine-3-carboxylique cyclopentyl méthylamide |
| « A324 » | 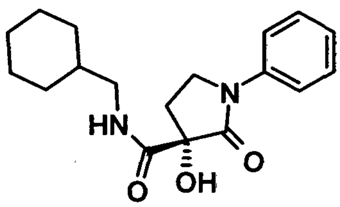 Acide (S)-3-hydroxy-2-oxo-1-phénylpyrrolidine-3-carboxylique cyclohexylméthylamide |

(suite)

| Composé n° | Structure/nom |
|---|---|
| « A325 » | 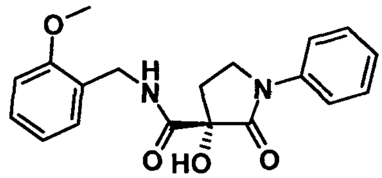<br>Acide (S)-3-hydroxy-2-oxo-1-phénylpyrrolidine-3-carboxylique 2-méthoxybenzylamide |
| « A326 » | 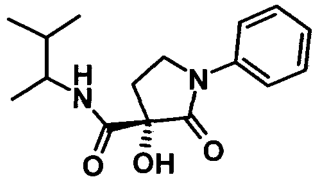<br>Acide (S)-3-hydroxy-2-oxo-1-phénylpyrrolidine-3-carboxylique (1,2-diméthylpropyl)amide |
| « A327 » | 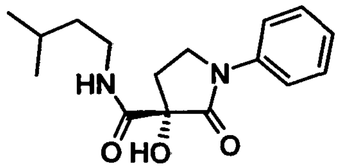<br>Acide (S)-3-hydroxy-2-oxo-1-phénylpyrrolidine-3-carboxylique (3-méthylbutyl)amide |
| « A328 » | 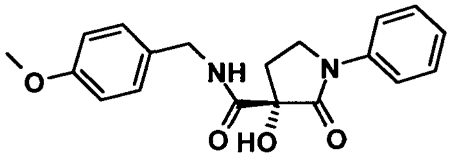<br>Acide (S)-3-hydroxy-2-oxo-1-phénylpyrrolidine-3-carboxylique 4-méthoxybenzylamide |
| « A329 » | 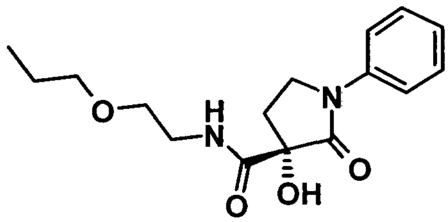<br>Acide (S)-3-hydroxy-2-oxo-1-phénylpyrrolidine-3-carboxylique (2-propoxyéthyl)amide |
| « A330 » | 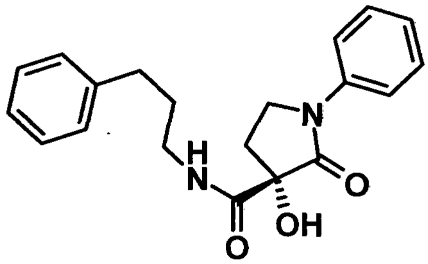<br>Acide (S)-3-hydroxy-2-oxo-1-phénylpyrrolidine-3-carboxylique (3-phénylpropyl)amide |

(suite)

| Composé n° | Structure/nom |
|---|---|
| « A331 » | 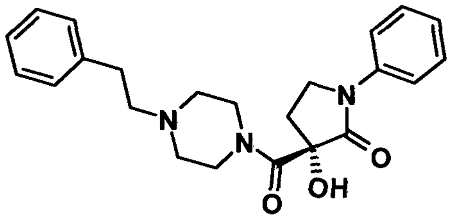<br>(S)-3-Hydroxy-3-(4-phénéthylpipérazine-1-carbonyl)-1-phénylpyrrolidin-2-one |
| « A332 » | 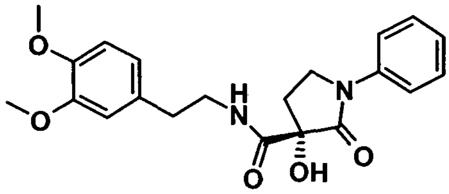<br>Acide (S)-3-hydroxy-2-oxo-1-phénylpyrrolidine-3-carboxylique [2-(3,4-diméthoxyphényl)éthyl]amide |
| « A333 » | 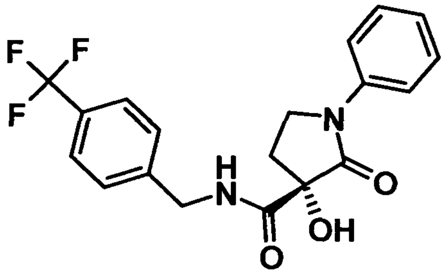<br>Acide (S)-3-hydroxy-2-oxo-1-phénylpyrrolidine-3-carboxylique 4-trifluorométhylbenzylamide |
| « A334 » | 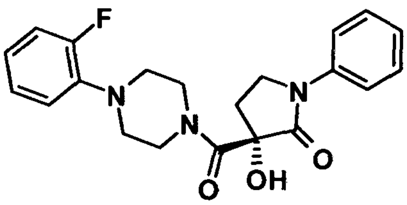<br>(S)-3-[4-(2-Fluorophényl)pipérazine-1-carbonyl]-3-hydroxy-1-phénylpyrrolidin-2-one |
| « A335 » | 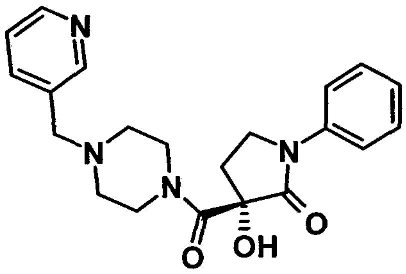<br>(S)-3-Hydroxy-1-phényl-3-(4-pyridin-3-ylméthylpipérazine-1-carbonyl)pyrrolidin-2-one |

(suite)

| Composé n° | Structure/nom |
|---|---|
| « A336 » | 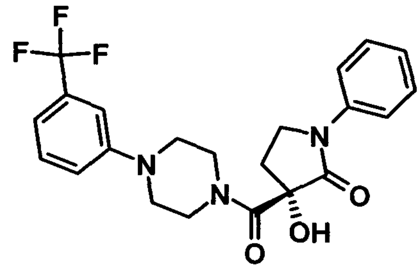<br>(S)-3-Hydroxy-1-phényl-3-[4-(3-trifluorométhylphényl)-pipérazine-1-carbonyl]pyrrolidin-2-one |
| « A337 » | 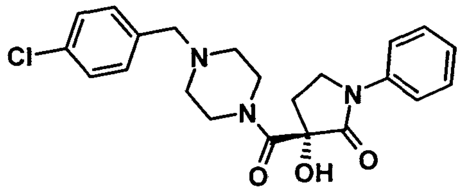<br>(S)-3-[4-(4-Chlorobenzyl)pipérazine-1-carbonyl]-3-hydroxy-1-phénylpyrrolidin-2-one |
| « A338 » | 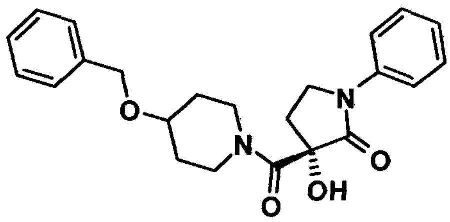<br>(S)-3-(4-Benzyloxypipéridine-1-carbonyl)-3-hydroxy-1-phénylpyrrolidin-2-one |
| « A339 » | 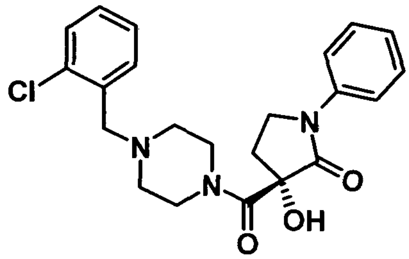<br>(S)-3-[4-(2-Chlorobenzyl)pipérazine-1-carbonyl]-3-hydroxy-1-phénylpyrrolidin-2-one |
| « A340 » | 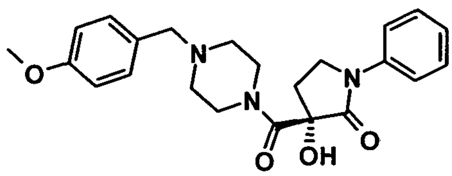<br>(S)-3-Hydroxy-3-[4-(4-méthoxybenzyl)pipérazine-1-carbonyl]-1-phénylpyrrolidin-2-one |
| « A341 » | 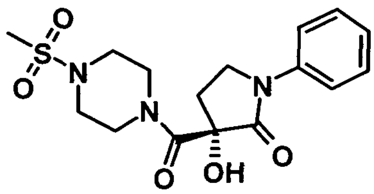<br>(S)-3-Hydroxy-3-(4-méthanesulfonylpipérazine-1-carbonyl)-1-phénylpyrrolidin-2-one |

(suite)

| Composé n° | Structure/nom |
|---|---|
| « A342 » | 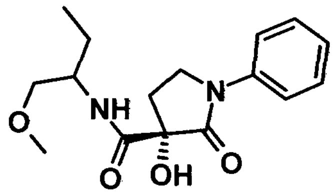 Acide (S)-3-hydroxy-2-oxo-1-phénylpyrrolidine-3-carboxylique (1-méthoxyméthylpropyl)amide |
| « A343 » | 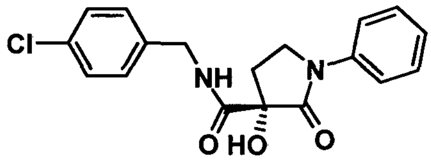 Acide (S)-3-hydroxy-2-oxo-1-phénylpyrrolidine-3-carboxylique 4-chlorobenzylamide |
| « A344 » | 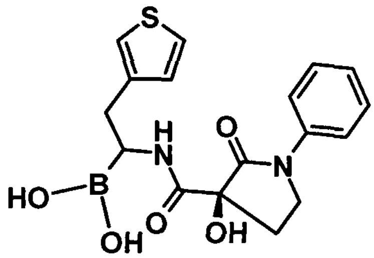 Acide [1-[[(3S)-3-hydroxy-2-oxo-1-phénylpyrrolidine-3-carbonyl]amino]-2-(3-thiényl)éthyl]boronique |
| « A345 » | 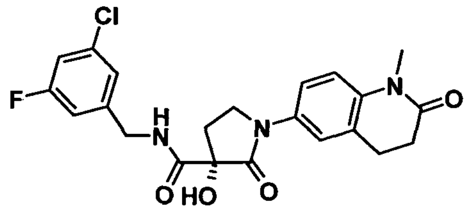 Acide (S)-3-hydroxy-1-(1-méthyl-2-oxo-1,2,3,4-tétrahydro-quinoléin-6-yl)-2-oxopyrrolidine-3-carboxylique 3-chloro-5-fluorobenzylamide |
| « A346 » | 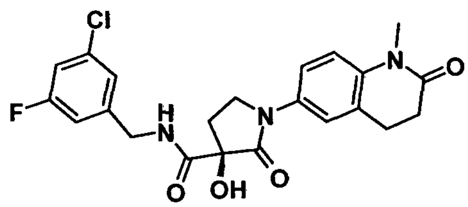 Acide (R)-3-hydroxy-1-(1-méthyl-2-oxo-1,2,3,4-tétrahydro-quinoléin-6-yl)-2-oxopyrrolidine-3-carboxylique 3-chloro-5-fluorobenzylamide |

ainsi que les sels, tautomères et stéréoisomères pharmaceutiquement utilisables de ceux-ci, y compris des mélanges de ceux-ci en toutes proportions.

**3.** Procédé de préparation de composés de formule I selon les revendications 1-2 ainsi que de sels, tautomères et stéréoisomères pharmaceutiquement utilisables de ceux-ci, **caractérisé en ce que**

a) un composé de formule II

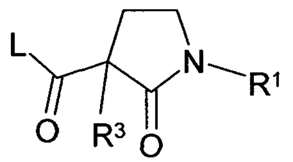

II

dans laquelle $R^1$ et $R^3$ revêtent les significations indiquées selon la revendication 1, et L désigne Cl, Br, I ou un groupement OH libre ou modifié de façon fonctionnelle et réactive, est réagi avec un composé de formule III

$R^2$-$NHR^4$ III

dans laquelle $R^2$ et $R^4$ revêtent les significations indiquées selon la revendication 1,
ou
b) pour la préparation de composés de formule I dans laquelle $R^3$ désigne OH,
un composé de formule IV

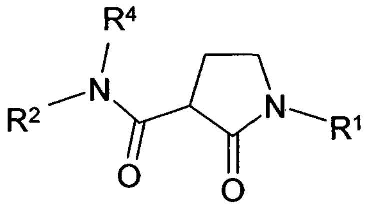

IV

dans laquelle $R^1$, $R^2$ et $R^4$ revêtent les significations indiquées selon la revendication 1,
est oxydé,
ou
c) un radical $R^3$ est converti en un autre radical $R^3$ en remplaçant un groupement OH par un atome d'halogène, ou en remplaçant un atome d'halogène par $N_3$,

et/ou
une base ou un acide de formule I est converti(e) en l'un de ses sels.

**4.** Médicaments comprenant au moins un composé de formule I selon les revendications 1-2 et/ou des sels, tautomères et stéréoisomères pharmaceutiquement utilisables de celui-ci, y compris des mélanges de ceux-ci en toutes proportions, et éventuellement des excipients et/ou des adjuvants.

**5.** Composés de formule I selon les revendications 1-2,
et des sels, tautomères et stéréoisomères pharmaceutiquement utilisables de ceux-ci, y compris des mélanges de ceux-ci en toutes proportions, pour une utilisation dans le traitement de tumeurs, de métastases tumorales, de maladies prolifératives des cellules mésangiales, de l'hémangiome, de la rétinopathie proliférative, de la polyarthrite rhumatoïde, de la néovascularisation athérosclérotique, du psoriasis, de la néovascularisation oculaire, de l'ostéoporose, du diabète et de l'obésité, de la leucémie lymphoïde, du lymphome, du paludisme et de l'hypertrophie de la prostate.

**6.** Composés selon la revendication 5, **caractérisés en ce que** la maladie tumorale est choisie dans le groupe de l'épithélium squameux, de la vessie, de l'estomac, des reins, de la tête et du cou, de l'oesophage, du col de l'utérus, de la thyroïde, des intestins, du foie, du cerveau, de la prostate, du tractus urogénital , du système lymphatique, de l'estomac, du larynx, du poumon, de la peau, la leucémie monocytique, l'adénocarcinome du poumon, le carcinome du poumon à petites cellules, le cancer du pancréas, le glioblastome, le carcinome du sein, la leucémie myéloïde aiguë, la leucémie myéloïde chronique, la leucémie lymphoïde aiguë, la leucémie lymphoïde chronique, le lymphome hodgkinien, le lymphome non hodgkinien.

**7.** Composés de formule I selon les revendications 1-2,
et/ou des sels physiologiquement acceptables de ceux-ci, pour une utilisation dans le traitement de tumeurs, où une quantité thérapeutiquement efficace d'un composé de formule I est administrée en association avec un composé

issu du groupe constitué par 1) un modulateur du récepteur des oestrogènes, 2) un modulateur du récepteur des androgènes, 3) un modulateur du récepteur des rétinoïdes, 4) un agent cytotoxique, 5) un agent antiprolifératif, 6) un inhibiteur de la prényl-protéine transférase, 7) un inhibiteur de la HMG-CoA réductase, 8) un inhibiteur de la protéase du VIH, 9) un inhibiteur de la transcriptase inverse et 10) d'autres inhibiteurs de l'angiogenèse.

**8.** Composés de formule I selon les revendications 1-2,
et/ou des sels physiologiquement acceptables de ceux-ci, pour une utilisation dans le traitement de tumeurs, où une quantité thérapeutiquement efficace d'un composé de formule I est administrée en association avec une radiothérapie et un composé issu du groupe constitué par 1) un modulateur du récepteur des oestrogènes, 2) un modulateur du récepteur des androgènes, 3) un modulateur du récepteur des rétinoïdes, 4) un agent cytotoxique, 5) un agent antiprolifératif, 6) un inhibiteur de la prényl-protéine transférase, 7) un inhibiteur de la HMG-CoA réductase, 8) un inhibiteur de la protéase du VIH, 9) un inhibiteur de la transcriptase inverse et 10) d'autres inhibiteurs de l'angiogenèse.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 0179157 A
- WO 02081415 A
- WO 2008011114 A
- WO 2011085201 A1
- WO 2011085198 A1
- WO 0050032 A
- US 6069134 A


### In der Beschreibung aufgeführte Nicht-Patentliteratur


- **NATURFORSCHUNG, B.** *Chemical Sciences,* 1994, vol. 49 (11), 1586-95
- *Analytica Chimica Acta,* 1987, vol. 202, 167-74
- *Journal of Electroanalytical Chemistry and Interfacial Electrochemistry,* 1988, vol. 239 (1-2), 161-73
- *Naturfor.Teil B: Anorg. Chem. Org. Chem,* 1978, vol. 33B (12), 1540-6
- *J. Chem. Soc.,* Oktober 1965, 5556-62
- *J. Chem. Soc.,* Oktober 1965, 5551-6
- **F. SPINELLA et al.** *J. Cardiovasc. Pharmacol.,* 2004, vol. 44, 140
- **A. MORABITO et al.** *Crit. Rev. Oncol./Hematol.,* 2004, vol. 49, 91
- **B. NICHOLSON et al.** *Cancer Metastas. Rev.,* 2001, vol. 20, 297
- **E. NG et al.** *Can. J. Ophthalmol.,* 2005, vol. 23, 3706
- **HENRI R. LIJNEN et al.** *Obesity,* 2010, vol. 18 (12), 2241-2246
- **X. CHEM et al.** *Chemistry & Biology,* 2009, vol. 16, 193-202
- *Int. J. Pharm.,* 1995, vol. 115, 61-67
- *Angew. Chem.,* 1980, vol. 92, 129
- *Pharmaceutical Research,* 1986, vol. 3 (6), 318